(19) **Europäisches Patentamt / European Patent Office / Office européen des brevets**

(11) **EP 4 545 568 A2**

(12) **EUROPEAN PATENT APPLICATION**

(43) Date of publication:
**30.04.2025   Bulletin 2025/18**

(21) Application number: **25160693.5**

(22) Date of filing: **29.06.2021**

(51) International Patent Classification (IPC):
**C07K 16/46** $^{(2006.01)}$

(52) Cooperative Patent Classification (CPC):
**A61P 35/00; C07K 16/2809; C07K 16/32; C07K 16/468;** A61K 38/00; A61K 2039/505; C07K 16/2818; C07K 16/2827; C07K 16/2878; C07K 2317/21; C07K 2317/24; C07K 2317/30; C07K 2317/31; C07K 2317/33; C07K 2317/35; (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2020   CN 202010618158**

(62) Document number(s) of the earlier application(s) in accordance with Art. 76 EPC:
**21833501.6 / 4 154 910**

(71) Applicant: **Harbour Biomed (Shanghai) Co., Ltd Shanghai 201203 (CN)**

(72) Inventors:
- **HE, Yun**
  **Shanghai, 201203 (CN)**
- **SHI, Lei**
  **Shanghai, 201203 (CN)**
- **LUO, Haishan**
  **Shanghai, 201203 (CN)**
- **LV, Qiang**
  **Shanghai, 201203 (CN)**
- **HE, Jinqiu**
  **Shanghai, 201203 (CN)**
- **PEI, Zenglin**
  **Shanghai, 201203 (CN)**
- **WANG, Yongqiang**
  **Shanghai, 201203 (CN)**
- **ZHONG, Chen**
  **Shanghai, 201203 (CN)**
- **HUANG, Bing**
  **Shanghai, 201203 (CN)**
- **ZHAO, Jianxun**
  **Shanghai, 201203 (CN)**
- **JIA, Xingxing**
  **Shanghai, 201203 (CN)**
- **ZHANG, Xuekun**
  **Shanghai, 201203 (CN)**
- **ZHAO, Chuchu**
  **Shanghai, 201203 (CN)**
- **CHEN, Fei**
  **Shanghai, 201203 (CN)**
- **NIU, Lei**
  **Shanghai, 201203 (CN)**
- **ZHOU, Wei**
  **Shanghai, 201203 (CN)**
- **TAN, Xiangyang**
  **Shanghai, 201203 (CN)**
- **RONG, Yiping**
  **Shanghai, 201203 (CN)**

(74) Representative: **Maiwald GmbH Elisenhof Elisenstraße 3 80335 München (DE)**

Remarks:
•The complete document including Reference Table(s) and the Sequence Listing(s) can be downloaded from the EPO website
•This application was filed on 27-02-2025 as a divisional application to the application mentioned under INID code 62.

(54) **BINDING PROTEIN HAVING H2L2 AND HCAB STRUCTURES**

(57)   Disclosed is a binding protein having at least two protein functional regions, the binding protein comprising a protein functional region A and a protein functional region B; the protein functional region A and the protein functional region B target different antigens or epitopes, the protein functional region A is a Fab or a scFv structure, the protein functional region B is a VH structure, and there are one or more of each of the protein functional region A and the protein functional region B. A multi-specific binding protein has a smaller molecular weight, fewer polypeptide chains, and a simpler structure. By means of different structure types, relative positions, binding valence and other parameters, the functional activities for different targets can be adjusted, and then different combinations of activities can be designed so as to meet the needs of different dose combinations of

clinical combinations.

(52) Cooperative Patent Classification (CPC): (Cont.)
C07K 2317/51; C07K 2317/52; C07K 2317/55;
C07K 2317/569; C07K 2317/60; C07K 2317/622;
C07K 2317/64; C07K 2317/66; C07K 2317/70;
C07K 2317/73; C07K 2317/732; C07K 2317/76;
C07K 2317/77; C07K 2317/92; C07K 2317/94;
C07K 2319/00

**Description**

## TECHNICAL FIELD

The present invention relates to the field of biomedicine, in particular to a bispecific or multispecific binding protein with H2L2 and HCAb structures and use thereof.

## BACKGROUND

Antibodies of most species are of tetrameric structures comprising two identical heavy chains and two identical light chains, which are also referred to as "H2L2". A heavy-chain antibody (HCAb) lacking light chains is naturally present in the serum of Camelidae species and sharks. The variable region of the heavy-chain antibody (VHH) fragment is similar to the Fab of the H2L2 antibody, with a stable structure and specific antigen-binding activity, but has a molecular weight of only about 13 kDa, and is therefore also referred to as a nanobody or a single-domain antibody. Human antibody has a natural structure of "H2L2", so it is impossible to obtain useful human heavy-chain antibodies from natural sources. However, Frank Grosveld et al. proposed a method for obtaining a fully human heavy-chain antibody using a transgenic animal (WO2007/096779), and a stable and soluble fully human VH single-domain antibody can be obtained by this method.

Bispecific antibodies and multispecific antibodies are a class of artificial antibodies prepared by the protein engineering technology on the basis of natural monoclonal antibodies, which can bind to two or more different antigens or different epitopes on the same antigen, and can achieve mechanism of action and functional effects that cannot be achieved by some monospecific antibodies.

The structural design of the bispecific antibodies is very important. The most significant challenge in generating bispecific antibodies from two different H2L2 antibodies is how to obtain functional bispecific antibody molecules with the correct chain combinations from more than 10 different combinations of heavy and light chains, that is, how to solve the problem of mismatching of chains. Scientists have developed a number of strategies to try to solve this problem: for example, fusing VH and VL together using the structure of a single-chain variable fragment (scFv); alternatively, promoting the heterodimerization of heavy chains using the knob-into-hole (KiH) technique or other mutants with complementary spatial structures; alternatively, reducing the number of different polypeptide chains using the "consensus light chain" or "consensus heavy chain" technique, and so on. However, various technical approaches have their limitations, for example, scFv structures are often unstable and prone to aggregation; the "consensus light chain" technique requires the use of a very complex screening process to obtain VLs that can be paired with different VHs; and FIT-Ig and other techniques will produce bispecific antibody molecules with relatively large size (about 250 kDa), which may affect the ability of the bispecific antibody molecules to penetrate cells or tissues. Therefore, there is still an urgent need to develop new structures of bispecific antibodies and techniques for preparing bispecific antibodies.

Heavy-chain antibodies and single-domain antibodies derived therefrom have unique advantages in the construction of bispecific or even multispecific antibodies. The heavy-chain antibody has an antigen-binding domain that is only one quarter the size of the Fab of a conventional antibody, and does not have light chain, so that the problem of mismatching of light chains is avoided. Therefore, bispecific or even multispecific antibodies with smaller molecular weight, less polypeptide chains and simpler structures can be constructed using heavy-chain antibodies and single-domain antibodies derived therefrom. However, the potential immunogenic risk of a non-human heavy-chain antibody may limit its therapeutic use. Therefore, compared with the nanobody of Camelidae species, the construction of bispecific or multispecific antibodies using the fully human heavy-chain antibody has more advantages in immunogenicity and druggability.

## SUMMARY

In order to overcome the defects in the prior art of the lack of bispecific or multispecific binding proteins with good therapeutic effect, stability and convenient production, the present invention provides a bispecific or multispecific binding protein with H2L2 and HCAb structures and a method for preparing and using the binding protein, which utilizes multivalent and multispecific binding proteins constructed by fully human heavy-chain antibodies and single-domain antibodies derived therefrom. Compared with the multivalent and multispecific binding proteins constructed using conventional IgG antibodies, such binding protein has many advantages and is more flexible in terms of adjusting specificity and binding valence. The multivalent and multispecific binding proteins provided herein comprise at least one heavy chain variable domain VH derived from a human heavy-chain antibody, and are capable of binding to two or more antigens, two or more epitopes on the same antigen or two or more copies on the same epitope.

In order to solve the above technical problems, a first technical solution of the present invention is as follows: provided is a binding protein comprising at least two protein functional regions, wherein the binding protein comprises a protein functional region A and a protein functional region B, wherein the protein functional region A and the protein functional region B target different antigens or antigenic epitopes, the protein functional region A is of a Fab or scFv structure, the protein functional region B is of a VH structure, and the number of each of the protein functional region A and the protein functional region B is one or more. Preferably, the binding protein further comprises Fc, wherein the number of the Fc is two, thereby forming an Fc dimer. Preferably, the binding protein is of a symmetric or asymmetric structure, and/or the Fc dimer is as a homodimer or a heterodimer.

In some specific embodiments, (I) the binding protein is of a symmetric structure, and the symmetric structure is a left-right symmetric IgG-like structure, wherein the number of the protein functional region A is two, and the number of the protein functional region B is two or four. The two protein functional regions A form an IgG structure with the two Fc. In the left-right symmetric IgG-like structure, the left and right are only spatially relative positions, and the structure may be up-down symmetric or front-back symmetric. The left-right symmetric IgG-like structure means that extra protein functional regions are left-right symmetrically linked to the antibody of the IgG structure, and these extra protein functional regions can be linked to the C-terminus of CH3 of an Fc functional region or the N-terminus of a VH or VL, or linked in an embedded form between the variable region and the Fc functional region of the IgG structure. As used herein, "extra" refers to structures or functional regions other than the IgG structure.

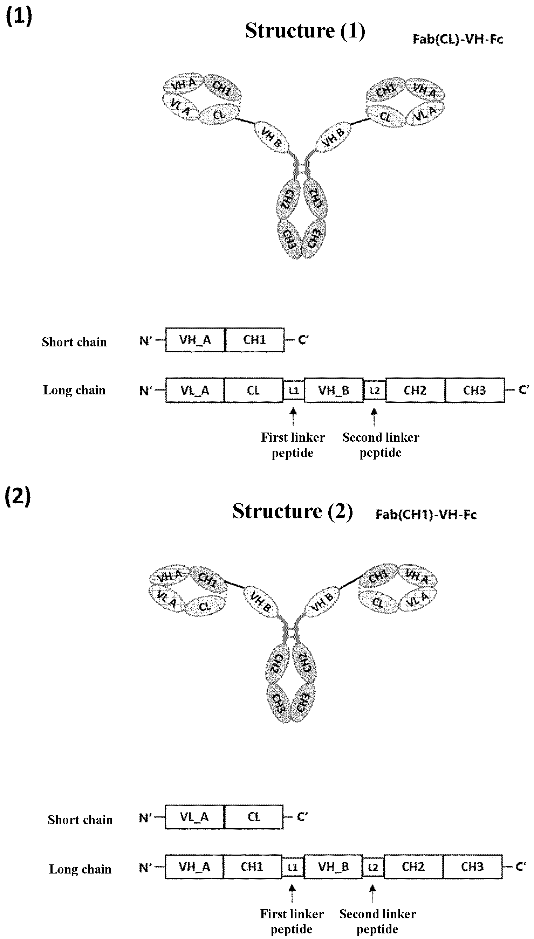

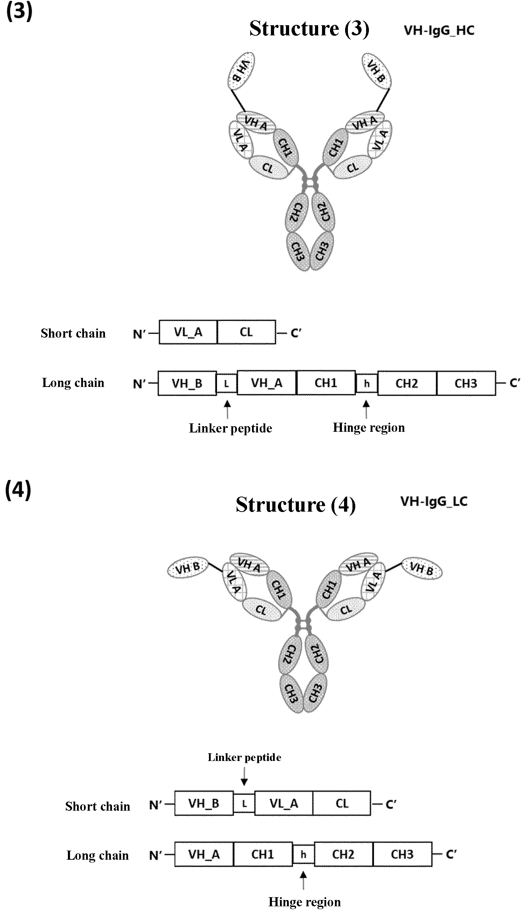

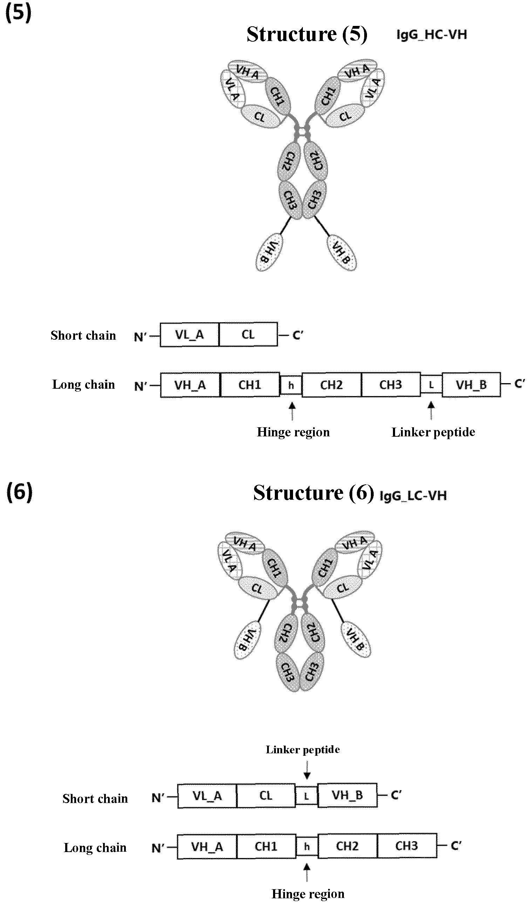

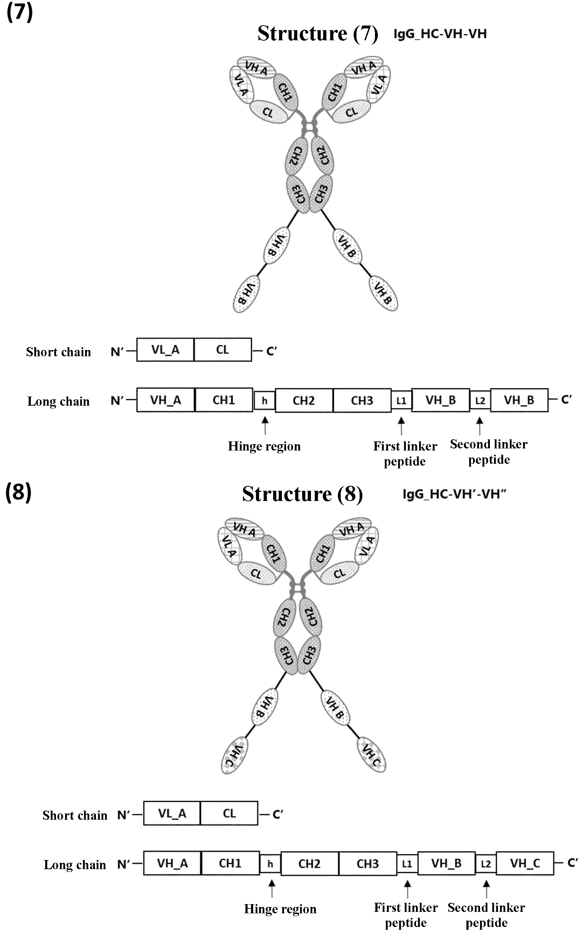

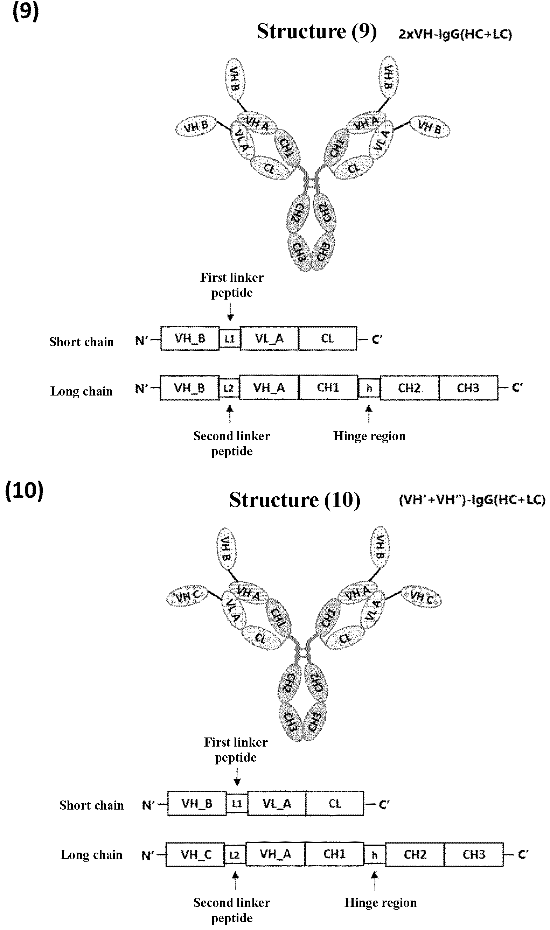

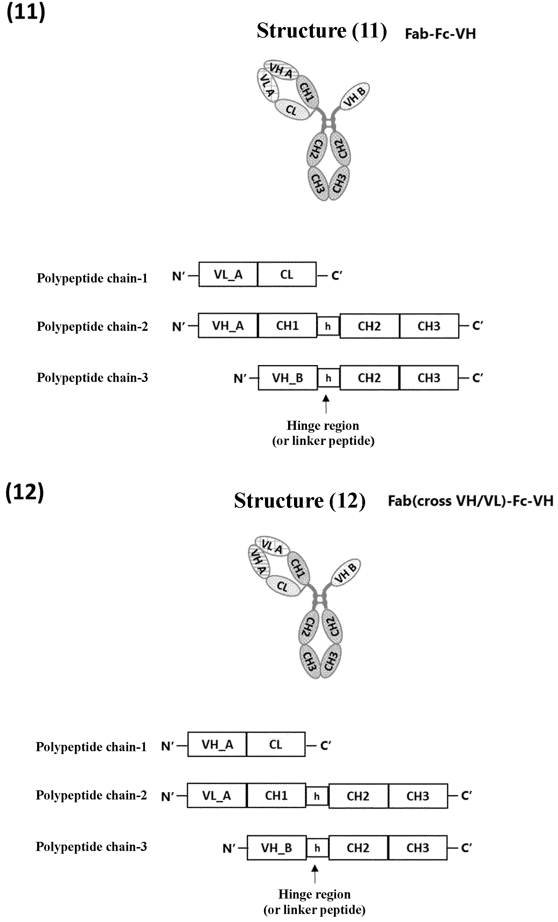

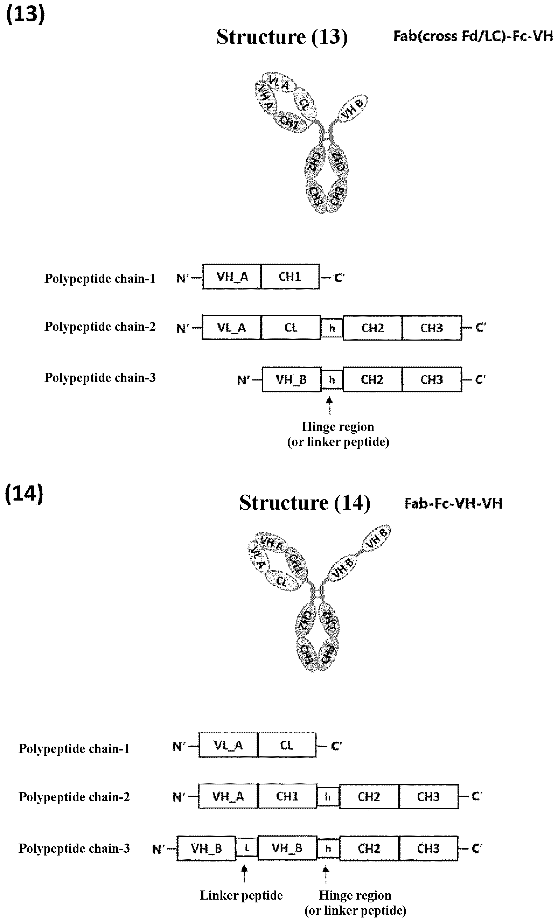

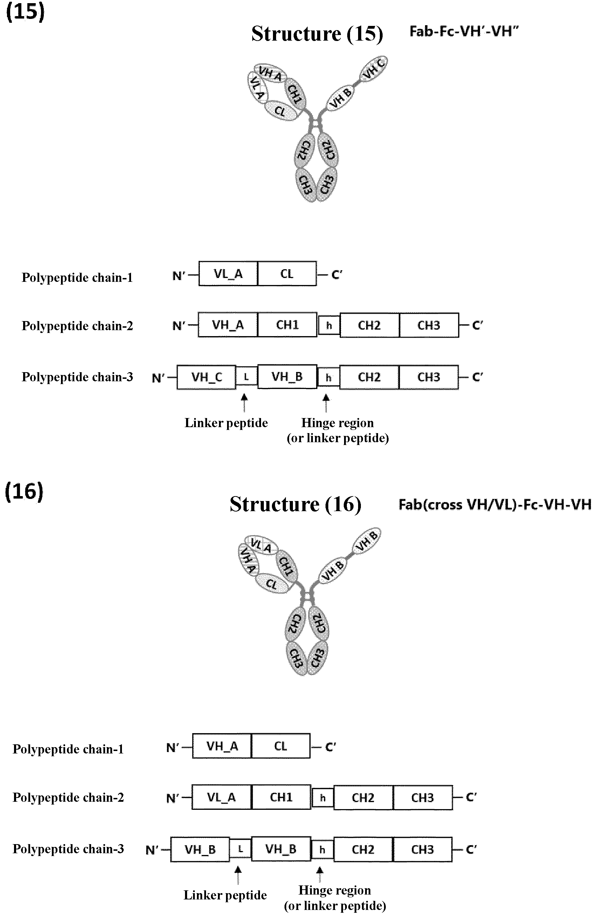

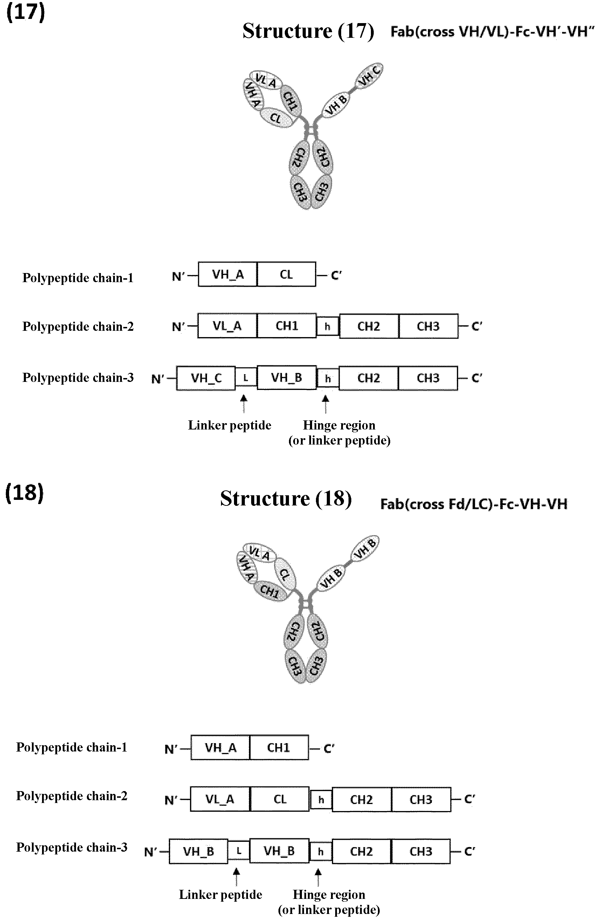

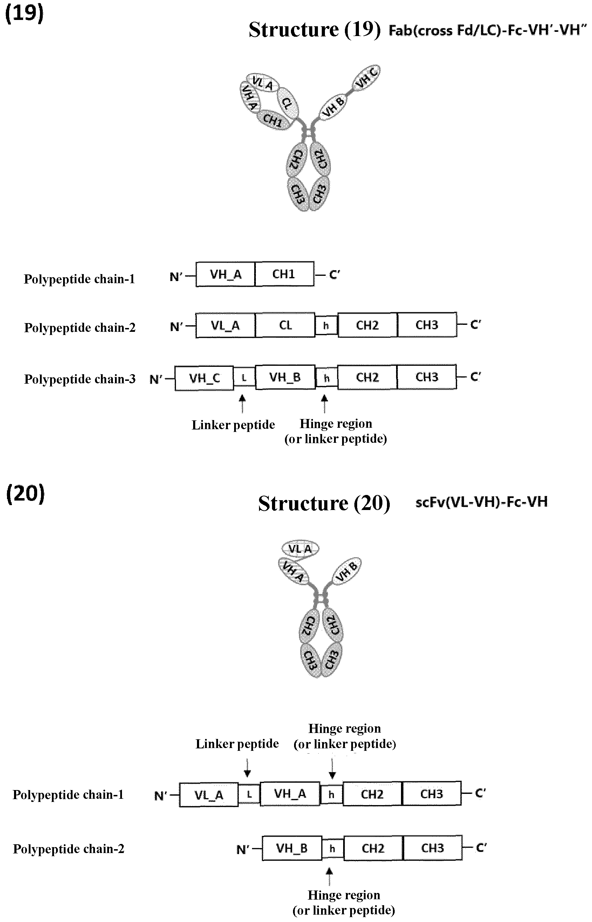

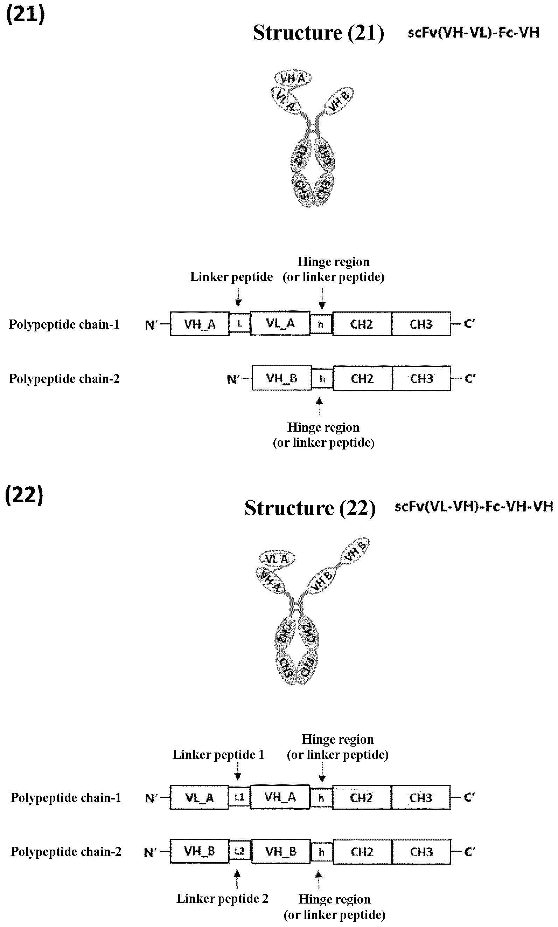

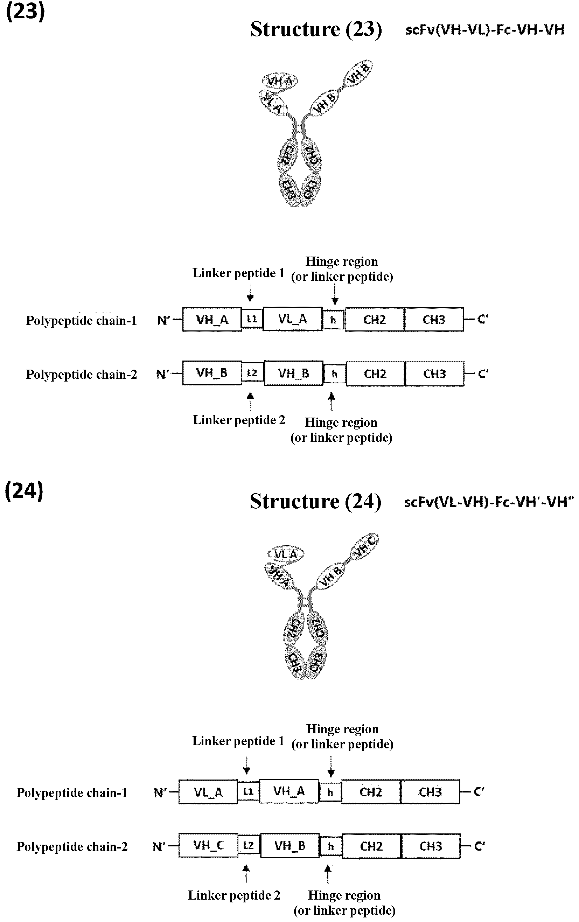

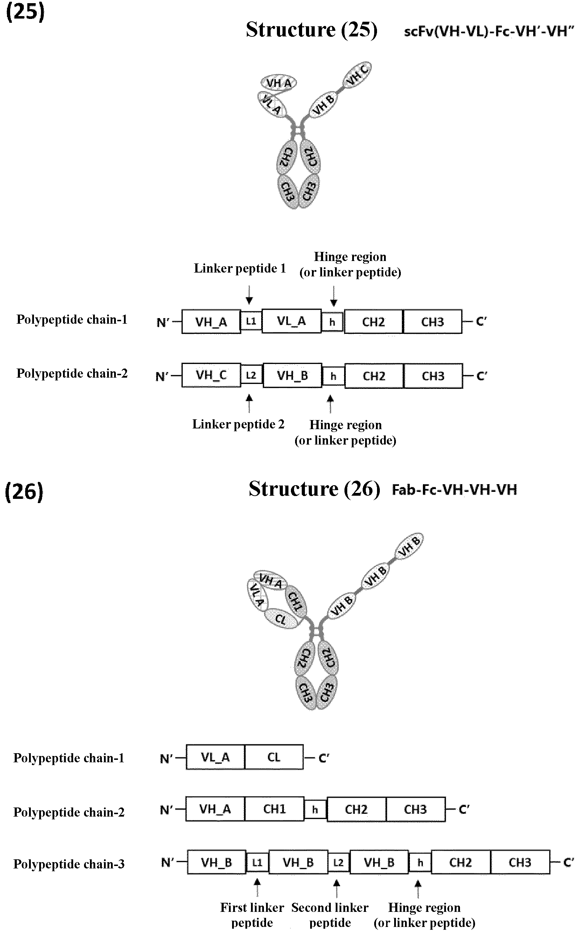

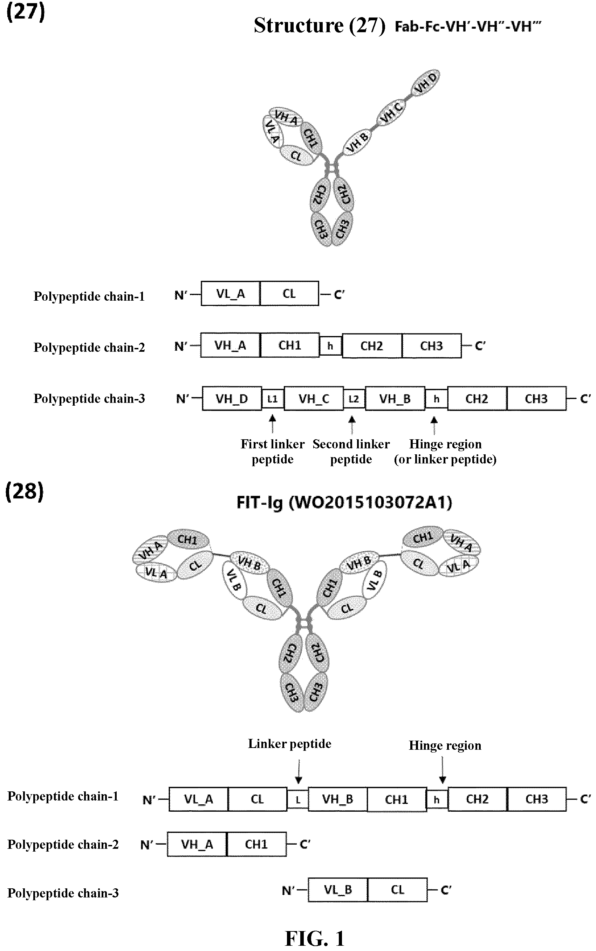

Preferably, when the number of the protein functional region B is two, the binding protein is a tetravalent binding protein specifically comprising a structure described as follows: (a) a protein functional region A, a protein functional region B and Fc are arranged sequentially from the N-terminus to the C-terminus of the binding protein, wherein the protein functional region A is linked to the protein functional region B via a first linker peptide (L1), and the protein functional region B is linked to the Fc via a second linker peptide (L2); in this embodiment, the two protein functional regions B form a symmetric dimer of a single chain antibody with the Fc, and the protein functional region A is linked to the N-terminus of the dimer of the single chain antibody, in which case the protein functional region A may be linked to the N-terminus of the protein functional region B via its CH1 (FIG. 1 (2)) or CL (FIG. 1 (1)); or, (b) a protein functional region B, a protein functional region A and Fc are arranged sequentially from the N-terminus to the C-terminus of the binding protein, wherein the protein functional region B is linked to the protein functional region A via a linker peptide, and the protein functional region A is linked to the Fc via a hinge region; in this embodiment, the two protein functional regions A form a symmetric IgG structure with the Fc, and the protein functional region B is linked to the N-terminus of the IgG structure, in which case the protein functional region B may be linked to the N-terminus of VH (FIG. 1 (3)) or VL (FIG. 1 (4)) of the protein functional region A; or, (c) a protein functional region A, Fc and a protein functional region B are arranged sequentially from the N-terminus to the C-terminus of the binding protein, wherein the protein functional region A is linked to the Fc via a hinge region, and the Fc is linked to the protein functional region B via a linker peptide; in this embodiment, the two protein functional regions A form a symmetric IgG structure with the Fc, and the protein functional region B is linked to the C-terminus of the IgG structure, in which case the protein functional region B may be linked to the C-terminus of CH3 of the Fc (FIG. 1 (5)) or CL (FIG. 1 (6)) of the protein functional region A;

Preferably, when the number of the protein functional region B is four, the binding protein is a hexavalent binding protein specifically comprising a structure described as follows: two additional protein functional regions B are further linked to the N-terminus or the C-terminus of the binding protein described in the (a), (b) or (c); preferably linked to the C-terminus of the Fc or the C-terminus of the original protein functional region B of the binding protein described in the (c), or linked to the N-terminus of the protein functional region A together with the original protein functional region B of the binding protein described in the (b). The "additional" described herein is merely for convenience, to distinguish from the above protein functional region B, i.e., the "original" protein functional region, and there is no precedence distinction between the additional protein functional region and the original protein functional region in the actual design or production of the antibody. In this embodiment, the two protein functional regions A form a symmetric IgG structure with the Fc, and the protein functional region B is linked to the C-terminus of the IgG structure, in which case two of the protein functional regions B may be linked to the CH3s of the Fc via first linker peptides, and the other two of the protein functional regions B are linked to the above two protein functional regions B via second linker peptides (FIG. 1 (7)); or two of the protein functional regions B may be linked to the N-termini of the VHs of the protein functional regions A via first linker peptides, and the other two of the protein functional regions B may be linked to the N-termini of the VLs of the protein functional regions A via second linker peptides (FIG. 1 (9)).

The above binding protein with two different protein functional regions is also referred to as a bispecific binding protein. More preferably, the binding protein further comprises a protein functional region C, and the binding protein is a hexavalent or octavalent trispecific binding protein specifically comprises a structure described as follows: the protein functional region C targets different antigens or antigenic epitopes with the protein functional region A and the protein functional region B, and the protein functional region C is linked to the N-terminus or the C-terminus of the antibody; preferably, the number of the protein functional region C is two, and the protein functional region C is linked to the C-terminus of the binding protein described in the (c), or linked to the N-terminus of the protein functional region A as the protein functional region B of the binding protein described in the (b). In this embodiment, the two protein functional regions C are further linked to the C-termini of structure (5) (FIG. 1 (8)) or the N-termini of structure (3) or (4) via second linker peptides (FIG. 1 (10) shows the case of linking to the N-termini of structure (3)).

Even more preferably, the binding protein has four polypeptide chains, including two identical short chains (or referred to as "polypeptide chains 1") and two identical long chains (or referred to as "polypeptide chains 2"), wherein (1) the short chain comprises VH_A-CH1 sequentially from the N-terminus to the C-terminus, and the long chain comprises VL_A-CL-L1-VH_B-L2-CH2-CH3 sequentially from the N-terminus to the C-terminus; or (2) the short chain comprises VL_A-CL sequentially from the N-terminus to the C-terminus, and the long chain comprises VH_A-CH1-L1-VH_B-L2-CH2-CH3 sequentially from the N-terminus to the C-terminus; or (3) the short chain comprises VL_A-CL sequentially from the N-terminus to the C-terminus, and the long chain comprises VH_B-L-VH_A-CH1-h-CH2-CH3 sequentially from the N-terminus to the C-terminus; or (4) the short chain comprises VH_B-L-VL_A-CL sequentially from the N-terminus to the C-terminus, and the long chain comprises VH_A-CH1-h-CH2-CH3 sequentially from the N-terminus to the C-terminus; or (5) the short chain comprises VL_A-CL sequentially from the N-terminus to the C-terminus, and the long chain comprises VH_A-CH1-h-CH2-CH3-L-VH_B sequentially from the N-terminus to the C-terminus; or (6) the short chain comprises VL_A-CL-L-VH_B sequentially from the N-terminus to the C-terminus, and the long chain comprises VH_A-CH1-h-CH2-CH3 sequentially from the N-terminus to the C-terminus; or (7) the short chain comprises VL_A-CL sequentially from the N-terminus to the C-terminus, and the long chain comprises VH_A-CH1-h-CH2-CH3-L1-VH_B-L2-VH_B sequentially from the N-terminus to the C-terminus; or (8) the short chain comprises VL_A-CL sequentially from the N-terminus to the C-terminus, and the long chain comprises VH_A-CH1-h-CH2-CH3-L1-VH_B-L2-VH_C sequentially from the N-terminus to the C-terminus; or (9) the short chain comprises VH_B-L1-VL_A-CL sequentially from the N-terminus to the C-terminus, and the long chain comprises VH_B-L2-VH_A-CH1-h-CH2-CH3 sequentially from the N-terminus to the C-terminus; or (10) the short chain comprises VH_B-L1-VL_A-CL sequentially from the N-terminus to the C-terminus, and the long chain comprises VH_C-L2-VH_A-CH1-h-CH2-CH3 sequentially from the N-terminus to the C-terminus; wherein the L, L1 and L2 are linker peptides, and the h is a hinge region or linker peptide such as "-", GS or an amino acid sequence as set forth in any one of SEQ ID NOs: 495-519. The VL, VH, CL and CH herein all have conventional meanings in the art and represent light chain variable region, heavy chain variable region, light chain constant region and heavy chain constant region, respectively, wherein the CH includes CH1, CH2 and CH3; the _A, _B and _C represent the functional regions as protein functional region A, protein functional region B, and protein functional region C or compositions thereof, respectively; the "-" represents a polypeptide bond linking different structural regions or used to separate different structural regions; the C-terminus is the carboxyl-terminus of the peptide chain (which may also be written as "C'"), and the N-terminus is the amino-terminus of the peptide chain (which may also be written as "N'"). The different protein functional regions are fused on the same polypeptide chain, so that mismatched byproducts can be avoided. In some embodiments, L, L1 and L2 may be identical sequences. In other embodiments, L, L1 and L2 may be different sequences. The protein functional region of the present invention may be Fab, scFv or VH in some cases, or $F(ab)_2$ or a full-length antibody in other cases, and is also referred to as an antibody, an antigen-binding protein or a binding protein in certain cases. In some specific embodiments, the protein functional region A is also referred to as an antibody A against a first antigen or a first antigen-binding domain; the protein functional region B is also referred to as an antibody B against a second antigen or a second antigen-binding domain; the protein functional region C is also referred to as an antibody C against a third antigen or a third antigen-binding domain; the protein functional region D is also referred to as an antibody D against a fourth antigen or a fourth antigen-binding domain, and so on.

In some specific embodiments, (II) the binding protein is of an asymmetric structure, and the asymmetric structure is a left-right asymmetric IgG-like structure, wherein a left arm is a protein functional region A of a Fab or scFv structure, a right arm is a protein functional region B of a VH structure, and the protein functional region A and the protein functional region B are each linked to one Fc; preferably, the number of the protein functional region A is one, and the number of the protein functional regions B is one, two or three.

Preferably, when the number of the protein functional region B is one, the binding protein is a bivalent binding protein comprising a structure described as follows: the protein functional region A is of (d) a conventional Fab structure, or (e) a Fab (cross VH/VL) structure, or (f) a Fab (cross Fd/LC) structure; when the number of the protein functional region B is two, the binding protein is a trivalent binding protein comprising a structure described as follows: the protein functional region A of the left arm is of the (d), (e) or (f) structure, and a second protein functional region B is linked to the N-terminus or C-terminus of the protein functional region A of the left arm, or the N-terminus of the first protein functional region B of the right arm, or the C-terminus of the Fc, preferably, the second protein functional region B is linked to the N-terminus of the first protein functional region B of the right arm; when the number of the protein functional region B is three, the binding protein is a tetravalent binding protein specifically comprising a structure described as follows: the protein functional region A of the left arm is of the (d), (e) or (f) structure, and a third protein functional region B is further linked to the N-terminus or C-terminus of the protein functional region A of the left arm, or the N-terminus or C-terminus of a second protein functional region B of the left arm, or the N-terminus of a first or second protein functional region B of the right arm, or the C-terminus of the Fc, preferably, the second protein functional region B is linked to the N-terminus of the first protein functional region B of the right arm, and the third protein functional region B is linked to the N-terminus of the second protein functional region B.

When the number of the protein functional region B is one, the binding protein is a bivalent binding protein comprising a structure described as follows: the protein functional region A of the left arm is of (g) an scFv structure, wherein the scFv structure is linked to Fc via the terminus of VH or the terminus of VL; when the number of the protein functional region B is two, the binding protein is a trivalent binding protein specifically comprising a structure described as follows: the protein functional region A of the left arm is of the (g) structure, and a second protein functional region B is linked to the N-terminus of the protein functional region A of the left arm, or the N-terminus of the original protein functional region B of the right arm, or the C-terminus of the Fc, preferably, the second protein functional region B is linked to the N-terminus of a first protein functional region B of the right arm; when the number of the protein functional regions B is three, the binding protein is a tetravalent binding protein specifically comprising a structure described as follows: the protein functional region A of the left arm is of the (g) structure, a third protein functional region B is further linked to the N-terminus of the protein functional region A of the left arm, or the N-terminus or the C-terminus of a second protein functional region B, or the C-terminus of the Fc, preferably, the second protein functional region B is linked to the N-terminus of the first protein functional region B of the right arm, and the third protein functional region B is linked to the N-terminus of the second protein functional region B.

More preferably, the binding protein further comprises a protein functional region C, and the binding protein is a trivalent or multivalent multispecific binding protein, wherein the protein functional region C targets different antigens or antigenic epitopes with the protein functional region A and the protein functional region B, and the protein functional region C is linked to the N-terminus or C-terminus of the above binding protein; preferably, the protein functional region C is linked to the N-terminus of the protein functional region B of the above binding protein described in the (d), (e), (f) or (g); and

even more preferably, the binding protein further comprises a protein functional region D, and the binding protein is a tetravalent or multivalent multispecific binding protein, wherein specifically: the protein functional region D targets different antigens or antigenic epitopes with the protein functional region A, the protein functional region B and the protein functional region C, and the protein functional region D is linked to the N-terminus or C-terminus of the above binding protein; preferably, the protein functional region D is linked to the N-terminus of the protein functional region B of the above binding protein described in the (d), (e), (f) or (g).

Even more preferably, the binding protein has three polypeptide chains, including one polypeptide chain 1, one polypeptide chain 2 and one polypeptide chain 3. In the present invention, the polypeptide chain 1 is also referred to as a first polypeptide chain, the polypeptide chain 2 is also referred to as a second polypeptide chain, and the polypeptide chain 3 is also referred to as a third polypeptide chain, wherein (11) the polypeptide chain 1 comprises VL_A-CL sequentially from the N-terminus to the C-terminus, the polypeptide chain 2 comprises VH_A-CH1-h-CH2-CH3 sequentially from the N-terminus to the C-terminus, and the polypeptide chain 3 comprises VH_B-h-CH2-CH3 sequentially from the N-terminus to the C-terminus; or (12) the polypeptide chain 1 comprises VH_A-CL sequentially from the N-terminus to the C-terminus, the polypeptide chain 2 comprises VL_A-CH1-h-CH2-CH3 sequentially from the N-terminus to the C-terminus, and the polypeptide chain 3 comprises VH_B-h-CH2-CH3 sequentially from the N-terminus to the C-terminus; or (13) the polypeptide chain 1 comprises VH_A-CH1 sequentially from the N-terminus to the C-terminus, the polypeptide chain 2 comprises VL_A-CL-h-CH2-CH3 sequentially from the N-terminus to the C-terminus, and the polypeptide chain 3 comprises VH_B-h-CH2-CH3 sequentially from the N-terminus to the C-terminus; or (14) the polypeptide chain 1 comprises VL_A-CL sequentially from the N-terminus to the C-terminus, the polypeptide chain 2 comprises VH_A-CH1-h-CH2-CH3 sequentially from the N-terminus to the C-terminus, and the polypeptide chain 3 comprises VH_B-L-VH_B-h-CH2-CH3 sequentially from the N-terminus to the C-terminus; or (15) the polypeptide chain 1 comprises VL_A-CL sequentially from the N-terminus to the C-terminus, the polypeptide chain 2 comprises VH_A-CH1-h-CH2-CH3 sequentially from the N-terminus to the C-terminus, and the polypeptide chain 3 comprises VH_C-L-VH_B-h-CH2-CH3 sequentially from the N-terminus to the C-terminus; or (16) the polypeptide chain 1 comprises VH_A-CL sequentially from the N-terminus to the C-terminus, the polypeptide chain 2 comprises VL_A-CH1-h-CH2-CH3 sequentially from the N-terminus to the C-terminus, and the polypeptide chain 3 comprises VH_B-L-VH_B-h-CH2-CH3 sequentially from the N-terminus to the C-terminus; or (17) the polypeptide chain 1 comprises VH_A-CL sequentially from the N-terminus to the C-terminus, the polypeptide chain 2 comprises VL_A-CH1-h-CH2-CH3 sequentially from the N-terminus to the C-terminus, and the polypeptide chain 3 comprises VH_C-L-VH_B-h-CH2-CH3 sequentially from the N-terminus to the C-terminus; or (18) the polypeptide chain 1 comprises VH_A-CH1 sequentially from the N-terminus to the C-terminus, the polypeptide chain 2 comprises VL_A-CL-h-CH2-CH3 sequentially from the N-terminus to the C-terminus, and the polypeptide chain 3 comprises VH_B-L-VH_B-h-CH2-CH3 sequentially from the N-terminus to the C-terminus; or (19) the polypeptide chain 1 comprises VH_A-CH1 sequentially from the N-terminus to the C-terminus, the polypeptide chain 2 comprises VL_A-CL-h-CH2-CH3 sequentially from the N-terminus to the C-terminus, and the polypeptide chain 3 comprises VH_C-L-VH_B-h-CH2-CH3 sequentially from the N-terminus to the C-terminus; or (26) the polypeptide chain 1 comprises VL_A-CL sequentially from the N-terminus to the C-terminus, the polypeptide chain 2 comprises VH_A-CH1-h-CH2-CH3 sequentially from the N-terminus to the C-terminus, and the polypeptide chain 3 comprises VH_B-L1-VH_B-L2-VH_B-h-CH2-CH3 sequentially from the N-terminus to the C-terminus; or (27) the polypeptide chain 1 comprises VL_A-CL sequentially from the N-terminus to the C-terminus, the polypeptide chain 2 comprises VH_A-CH1-h-CH2-CH3 sequentially from the N-terminus to the C-terminus, and the polypeptide chain 3 comprises VH _D-L1-VH_C-L2-VH_B-h-CH2-CH3 sequentially from the N-terminus to the C-terminus; wherein the L, L1 and L2 are linker peptides, and the h is a hinge region or linker peptide such as "-", GS or an amino acid sequence as set forth in any one of SEQ ID NOs: 495-519.

Even more preferably, the binding protein has two polypeptide chains, including one polypeptide chain 1 and one polypeptide chain 2. Likewise, the polypeptide 1 is also referred to as a first polypeptide chain and the polypeptide 2 is also referred to as a second polypeptide chain, wherein (20) the polypeptide chain 1 comprises VL_A-L-VH_A-h-CH2-CH3 sequentially from the N-terminus to the C-terminus, and the polypeptide chain 2 comprises VH_B-h-CH2-CH3 sequentially from the N-terminus to the C-terminus; or (21) the polypeptide chain 1 comprises VH_A-L-VL_A-h-CH2-CH3 sequentially from the N-terminus to the C-terminus, and the polypeptide chain 2 comprises VH_B-h-CH2-CH3 sequentially from the N-terminus to the C-terminus; or (22) the polypeptide chain 1 comprises VL_A-L1-VH_A-h-CH2-CH3 sequentially from the N-terminus to the C-terminus, and the polypeptide chain 2 comprises VH_B-L2-VH_B-h-CH2-CH3 sequentially from the N-terminus to the C-terminus; or (23) the polypeptide chain 1 comprises VH_A-L1-VL_A-h-CH2-CH3 sequentially from the N-terminus to the C-terminus, and the polypeptide chain 2 comprises VH_B-L2-VH_B-h-CH2-CH3 sequentially from the N-terminus to the C-terminus; or (24) the polypeptide chain 1 comprises VL_A-L1-VH_A-h-CH2-CH3 sequentially from the N-terminus to the C-terminus, and the polypeptide chain 2 comprises VH_C-L2-VH_B-CH2-CH3 sequentially from the N-terminus to the C-terminus; or (25) the polypeptide chain 1 comprises VH_A-L1-VL_A-h-CH2-CH3 sequentially from the N-terminus to the C-terminus, and the polypeptide chain 2 comprises VH_C-L2-VH_B-h-CH2-CH3 sequentially from the N-terminus to the C-terminus; wherein the L, L1 and L2 are linker peptides, and the h is a hinge region or linker peptide such as "-", GS or an amino acid sequence as set forth in any one of SEQ ID NOs: 495-519.

In some specific embodiments, the binding protein comprises at least two protein functional regions, namely protein functional region A and protein functional region B, wherein the protein functional region A and the protein functional region B are each one of a PD-L1 antibody, an HER2 antibody, a B7H4 antibody, a CD3 antibody, a CTLA4 antibody, a 4-1BB antibody and a BCMA antibody. Preferably, the protein functional region A is a PD-L1 antibody, an HER2 antibody, a B7H4 antibody or a CD3 antibody, and the protein functional region B is a CTLA4 antibody, a 4-1BB antibody or a BCMA antibody. More preferably, the protein functional region A is a PD-L1 antibody, and the protein functional region B is a CTLA4 antibody; or, the protein functional region A is a PD-L1 antibody, and the protein functional region B is a 4-1BB antibody; or, the protein functional region A is an HER2 antibody, and the protein functional region B is a CTLA4 antibody; or, the protein functional region A is a B7H4 antibody, and the protein functional region B is a 4-1BB antibody; or, the protein functional region A is a CD3 antibody, and the protein functional region B is a BCMA antibody.

In some specific embodiments, the PD-L1 antibody comprises a light chain variable region and a heavy chain variable region, wherein the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 62 and 122, respectively; or, the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 69 and 122, respectively. The amino acid sequences of the listed CDRs are shown according to the Chothia scheme. Preferably, the light chain variable region VL comprises an amino acid sequence as set forth in SEQ ID NO: 282; and the heavy chain variable region VH comprises an amino acid sequence as set forth in SEQ ID NO: 233; or, the light chain variable region VL comprises an amino acid sequence as set forth in SEQ ID NO: 282; and the heavy chain variable region VH comprises an amino acid sequence as set forth in SEQ ID NO: 240. More preferably, the PD-L1 antibody comprises two polypeptide chains; wherein a first polypeptide chain comprises an amino acid sequence as set forth in SEQ ID NO: 347, and a second polypeptide chain comprises an amino acid sequence as set forth in SEQ ID NO: 300; or, a first polypeptide chain comprises an amino acid sequence as set forth in SEQ ID NO: 353, and a second polypeptide chain comprises an amino acid sequence as set forth in SEQ ID NO: 308; or, a first polypeptide chain comprises an amino acid sequence as set forth in SEQ ID NO: 353, and a second polypeptide chain comprises an amino acid sequence as set forth in SEQ ID NO: 310.

In some specific embodiments, the PD-L1 antibody has an amino acid sequence as shown in that of atezolizumab known in the art.

In some specific embodiments, the HER2 antibody has an amino acid sequence as shown in that of trastuzumab or pertuzumab known in the art.

In some specific embodiments, the B7H4 antibody comprises a light chain variable region and a heavy chain variable region, wherein the light chain variable region VL comprises LCDR1, LCDR2 and LCDR3 with amino acid sequences as set forth in SEQ ID NOs: 180, 191 and 225, respectively; and the heavy chain variable region VH comprises HCDR1, HCDR2 and HCDR3 with amino acid sequences as set forth in SEQ ID NOs: 32, 87 and 143, respectively; or, the light chain variable region VL comprises LCDR1, LCDR2 and LCDR3 with amino acid sequences as set forth in SEQ ID NOs: 177, 191 and 226, respectively; and the heavy chain variable region VH comprises HCDR1, HCDR2 and HCDR3 with amino acid sequences as set forth in SEQ ID NOs: 33, 88 and 144, respectively. The amino acid sequences of the listed CDRs are shown according to the Chothia scheme. Preferably, the light chain variable region VL comprises an amino acid sequence as set forth in SEQ ID NO: 298; and the heavy chain variable region VH comprises an amino acid sequence as set forth in SEQ ID NO: 261; or, the light chain variable region VL comprises an amino acid sequence as set forth in SEQ ID NO: 299; and the heavy chain variable region VH comprises an amino acid sequence as set forth in SEQ ID NO: 262. More preferably, the B7H4 antibody comprises two polypeptide chains; wherein a first polypeptide chain comprises an amino acid sequence as set forth in SEQ ID NO: 360, and a second polypeptide chain comprises an amino acid sequence as set forth in SEQ ID NO: 326; or, a first polypeptide chain comprises an amino acid sequence as set forth in SEQ ID NO: 361, and a second polypeptide chain comprises an amino acid sequence as set forth in SEQ ID NO: 327.

In some specific embodiments, the CTLA4 antibody comprises a heavy chain variable region, wherein the heavy chain variable region VH comprises HCDR1, HCDR2 and HCDR3 with amino acid sequences as set forth in SEQ ID NOs: 17, 65 and 125, respectively. The amino acid sequences of the listed CDRs are shown according to the Chothia scheme. Preferably, the heavy chain variable region VH comprises an amino acid sequence as set forth in SEQ ID NO: 236. More preferably, the CTLA4 antibody comprises one polypeptide chain, wherein the polypeptide chain comprises an amino acid sequence as set forth in SEQ ID NO: 303 or an amino acid sequence as set forth in SEQ ID NO: 306.

In some specific embodiments, the 4-1BB antibody comprises a light chain variable region and a heavy chain variable region, wherein the light chain variable region VL comprises LCDR1, LCDR2 and LCDR3 with amino acid sequences as set forth in SEQ ID NOs: 170, 191 and 214, respectively; and the heavy chain variable region VH comprises HCDR1, HCDR2 and HCDR3 with amino acid sequences as set forth in SEQ ID NOs: 18, 66 and 126, respectively; or, the light chain variable region VL comprises LCDR1, LCDR2 and LCDR3 with amino acid sequences as set forth in SEQ ID NOs: 170, 191 and 214, respectively; and the heavy chain variable region VH comprises HCDR1, HCDR2 and HCDR3 with amino acid sequences as set forth in SEQ ID NOs: 18, 71 and 126, respectively. The amino acid sequences of the listed CDRs are shown according to the Chothia scheme. Preferably, the light chain variable region VL comprises an amino acid sequence as set forth in SEQ ID NO: 285; and the heavy chain variable region VH comprises an amino acid sequence as set forth in SEQ ID NO: 237; or, the light chain variable region VL comprises an amino acid sequence as set forth in SEQ ID NO: 289; and the heavy chain variable region VH comprises an amino acid sequence as set forth in SEQ ID NO: 242. More preferably, the 4-1BB antibody comprises two polypeptide chains; wherein a first polypeptide chain comprises an amino acid sequence as set forth in SEQ ID NO: 350, and a second polypeptide chain comprises an amino acid sequence as set forth in SEQ ID NO: 304; or, a first polypeptide chain comprises an amino acid sequence as set forth in SEQ ID NO: 355, and a second polypeptide chain comprises an amino acid sequence as set forth in SEQ ID NO: 311.

In some specific embodiments, the 4-1BB antibody comprises a heavy chain variable region, wherein the heavy chain variable region VH comprises HCDR1, HCDR2 and HCDR3 with amino acid sequences as set forth in SEQ ID NOs: 27, 79 and 137, respectively; or, the heavy chain variable region VH comprises HCDR1, HCDR2 and HCDR3 with amino acid sequences as set forth in SEQ ID NOs: 28, 80 and 138, respectively; or, the heavy chain variable region VH comprises HCDR1, HCDR2 and HCDR3 with amino acid sequences as set forth in SEQ ID NOs: 29, 82 and 138, respectively; or, the heavy chain variable region VH comprises HCDR1, HCDR2 and HCDR3 with amino acid sequences as set forth in SEQ ID NOs: 28, 89 and 145, respectively; or, the heavy chain variable region VH comprises HCDR1, HCDR2 and HCDR3 with amino acid sequences as set forth in SEQ ID NO: 28, 81 and 139, respectively; or, the heavy chain variable region VH comprises HCDR1, HCDR2 and HCDR3 with amino acid sequences as set forth in SEQ ID NOs: 15, 83 and 140, respectively. The amino acid sequences of the listed CDRs are shown according to the Chothia scheme. Preferably, the heavy chain variable region VH comprises an amino acid sequence as set forth in SEQ ID NO: 252; or, the heavy chain variable region VH comprises an amino acid sequence as set forth in SEQ ID NO: 253; or, the heavy chain variable region VH comprises an amino acid sequence as set forth in SEQ ID NO: 255; or, the heavy chain variable region VH comprises an amino acid sequence as set forth in SEQ ID NO: 264; or, the heavy chain variable region VH comprises an amino acid sequence as set forth in SEQ ID NO: 254; or, the heavy chain variable region VH comprises an amino acid sequence as set forth in SEQ ID NO: 256. More preferably, the 4-1BB antibody comprises one polypeptide chain, wherein the polypeptide chain comprises an amino acid sequence as set forth in SEQ ID NO: 320, an amino acid sequence as set forth in SEQ ID NO: 321, an amino acid sequence as set forth in SEQ ID NO: 323, an amino acid sequence as set forth in SEQ ID NO: 322, an amino acid sequence as set forth in SEQ ID NO: 324 or an amino acid sequence as set forth in SEQ ID NO: 329.

In some specific embodiments, the CD3 antibody comprises a light chain variable region and a heavy chain variable region, wherein the light chain variable region VL comprises LCDR1, LCDR2 and LCDR3 with amino acid sequences as set forth in SEQ ID NOs: 177, 191 and 221, respectively; and the heavy chain variable region VH comprises HCDR1, HCDR2 and HCDR3 with amino acid sequences as set forth in SEQ ID NOs: 15, 84 and 141, respectively; or, the light chain variable region VL comprises LCDR1, LCDR2 and LCDR3 with amino acid sequences as set forth in SEQ ID NOs: 178, 197 and 222, respectively; and the heavy chain variable region VH comprises HCDR1, HCDR2 and HCDR3 with amino acid sequences as set forth in SEQ ID NOs: 31, 85 and 142, respectively; or, the light chain variable region VL comprises LCDR1, LCDR2 and LCDR3 with amino acid sequences as set forth in SEQ ID NOs: 177, 191 and 223, respectively; and the heavy chain variable region VH comprises HCDR1, HCDR2 and HCDR3 with amino acid sequences as set forth in SEQ ID NOs: 31, 86 and 141, respectively; or, the light chain variable region VL comprises LCDR1, LCDR2 and LCDR3 with amino acid sequences as set forth in SEQ ID NOs: 179, 198 and 224, respectively; and the heavy chain variable region VH comprises HCDR1, HCDR2 and HCDR3 with amino acid sequences as set forth in SEQ ID NOs: 31, 85 and 142, respectively. The amino acid sequences of the listed CDRs are shown according to the Chothia scheme.

Preferably, the light chain variable region VL comprises an amino acid sequence as set forth in SEQ ID NO: 294; and the heavy chain variable region VH comprises an amino acid sequence as set forth in SEQ ID NO: 258; or, the light chain variable region VL comprises an amino acid sequence as set forth in SEQ ID NO: 295; and the heavy chain variable region VH comprises an amino acid sequence as set forth in SEQ ID NO: 259; or, the light chain variable region VL comprises an amino acid sequence as set forth in SEQ ID NO: 296; and the heavy chain variable region VH comprises an amino acid sequence as set forth in SEQ ID NO: 260; or, the light chain variable region VL comprises an amino acid sequence as set forth in SEQ ID NO: 297; and the heavy chain variable region VH comprises an amino acid sequence as set forth in SEQ ID NO: 259.

In some specific embodiments, the CD3 antibody comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; the heavy chain variable region VH comprises HCDR1, HCDR2 and HCDR3 with amino acid sequences as set forth in SEQ ID NOs: 20, 68 and 128, respectively; or, the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively. The amino acid sequences of the listed CDRs are shown according to the Chothia scheme. Preferably, the light chain variable region VL comprises an amino acid sequence as set forth in SEQ ID NO: 291; and the heavy chain variable region VH comprises an amino acid sequence as set forth in SEQ ID NO: 245; or, the light chain variable region VL comprises an amino acid sequence as set forth in SEQ ID NO: 291; and the heavy chain variable region VH comprises an amino acid sequence as set forth in SEQ ID NO: 257; or, the light chain variable region VL comprises an amino acid sequence as set forth in SEQ ID NO: 291; and the heavy chain variable region VH comprises an amino acid sequence as set forth in SEQ ID NO: 263; or, the light chain variable region VL comprises an amino acid sequence as set forth in SEQ ID NO: 291; and the heavy chain variable region VH comprises an amino acid sequence as set forth in SEQ ID NO: 281; or, the light chain variable region VL comprises an amino acid sequence as set forth in SEQ ID NO: 291; and the heavy chain variable region VH comprises an amino acid sequence as set forth in SEQ ID NO: 244.

In some specific embodiments, the CD3 antibody comprises two polypeptide chains, wherein a first polypeptide chain (VL) comprises an amino acid sequence as set forth in SEQ ID NO: 357, and a second polypeptide chain (VH) comprises an amino acid sequence as set forth in SEQ ID NO: 313; or, a first polypeptide chain comprises an amino acid sequence as set forth in SEQ ID NO: 357, and a second polypeptide chain comprises an amino acid sequence as set forth in SEQ ID NO: 325; or, a first polypeptide chain comprises an amino acid sequence as set forth in SEQ ID NO: 357, and a second polypeptide chain comprises an amino acid sequence as set forth in SEQ ID NO: 328; or, a first polypeptide chain comprises an amino acid sequence as set forth in SEQ ID NO: 357, and a second polypeptide chain comprises an amino acid sequence as set forth in SEQ ID NO: 346.

In some specific embodiments, the CD3 antibody comprises one polypeptide chain, wherein the polypeptide chain comprises an amino acid sequence as set forth in SEQ ID NO: 489, an amino acid sequence as set forth in SEQ ID NO: 490, an amino acid sequence as set forth in SEQ ID NO: 491, an amino acid sequence as set forth in SEQ ID NO: 492 or an amino acid sequence as set forth in SEQ ID NO: 493.

In some specific embodiments, the BCMA antibody comprises a heavy chain variable region, wherein the heavy chain variable region VH comprises HCDR1, HCDR2 and HCDR3 with amino acid sequences as set forth in SEQ ID NOs: 15, 75 and 133, respectively; or, the heavy chain variable region VH comprises HCDR1, HCDR2 and HCDR3 with amino acid sequences as set forth in SEQ ID NOs: 24, 76 and 134, respectively; or, the heavy chain variable region VH comprises HCDR1, HCDR2 and HCDR3 with amino acid sequences as set forth in SEQ ID NOs: 25, 77 and 135, respectively; or, the heavy chain variable region VH comprises HCDR1, HCDR2 and HCDR3 with amino acid sequences as set forth in SEQ ID NOs: 26, 78 and 136, respectively. The amino acid sequences of the listed CDRs are shown according to the Chothia scheme. Preferably, the heavy chain variable region VH comprises an amino acid sequence as set forth in SEQ ID NO: 248; or, the heavy chain variable region VH comprises an amino acid sequence as set forth in SEQ ID NO: 249; or, the heavy chain variable region VH comprises an amino acid sequence as set forth in SEQ ID NO: 250; or, the heavy chain variable region VH comprises an amino acid sequence as set forth in SEQ ID NO: 251. More preferably, the BCMA antibody comprises one polypeptide chain, wherein the polypeptide chain comprises an amino acid sequence as set forth in SEQ ID NO: 316, an amino acid sequence as set forth in SEQ ID NO: 317, an amino acid sequence as set forth in SEQ ID NO: 318 or an amino acid sequence as set forth in SEQ ID NO: 319.

In some specific embodiments, the BCMA antibody comprises a heavy chain variable region, wherein the heavy chain variable region VH comprises HCDR1, HCDR2 and HCDR3 with amino acid sequences as set forth in SEQ ID NOs: 26, 90 and 136, respectively; or, the heavy chain variable region VH comprises HCDR1, HCDR2 and HCDR3 with amino acid sequences as set forth in SEQ ID NOs: 34, 78 and 146, respectively; or, the heavy chain variable region VH comprises HCDR1, HCDR2 and HCDR3 with amino acid sequences as set forth in SEQ ID NOs: 35, 78 and 147, respectively; or, the heavy chain variable region VH comprises HCDR1, HCDR2 and HCDR3 with amino acid sequences as set forth in SEQ ID NOs: 35, 90 and 147, respectively; or, the heavy chain variable region VH comprises HCDR1, HCDR2 and HCDR3 with amino acid sequences as set forth in SEQ ID NOs: 36, 90 and 146, respectively; or, the heavy chain variable region VH comprises HCDR1, HCDR2 and HCDR3 with amino acid sequences as set forth in SEQ ID NOs: 35, 76 and 147, respectively; or, the heavy chain variable region VH comprises HCDR1, HCDR2 and HCDR3 with amino acid sequences as set forth in SEQ ID NOs: 34, 78 and 148, respectively; or, the heavy chain variable region VH comprises HCDR1, HCDR2 and HCDR3 with amino acid sequences as set forth in SEQ ID NOs: 35, 76 and 136, respectively; or, the heavy chain variable region VH comprises HCDR1, HCDR2 and HCDR3 with amino acid sequences as set forth in SEQ ID NOs: 35, 90 and 146, respectively; or, the heavy chain variable region VH comprises HCDR1, HCDR2 and HCDR3 with amino acid sequences as set forth in SEQ ID NOs: 34, 76 and 136, respectively; or, the heavy chain variable region VH comprises HCDR1, HCDR2 and HCDR3 with amino acid sequences as set forth in SEQ ID NOs: 35, 76 and 146, respectively; or, the heavy chain variable region VH comprises HCDR1, HCDR2 and HCDR3 with amino acid sequences as set forth in SEQ ID NOs: 34, 76 and 146, respectively; or, the heavy chain variable region VH comprises HCDR1, HCDR2 and HCDR3 with amino acid sequences as set forth in SEQ ID NOs: 35, 90 and 148, respectively; or, the heavy chain variable region VH comprises HCDR1, HCDR2 and HCDR3 with amino acid sequences as set forth in SEQ ID NOs: 35, 90 and 136, respectively. The amino acid sequences of the listed CDRs are shown according to the Chothia scheme. Preferably, the heavy chain variable region VH comprises an amino acid sequence as set forth in SEQ ID NO: 265; or, the heavy chain variable region VH comprises an amino acid sequence as set forth in SEQ ID NO: 266; or, the heavy chain variable region VH comprises an amino acid sequence as set forth in SEQ ID NO: 267; or, the heavy chain variable region VH comprises an amino acid sequence as set forth in SEQ ID NO: 268; or, the heavy chain variable region VH comprises an amino acid sequence as set forth in SEQ ID NO: 269; or, the heavy chain variable region VH comprises an amino acid sequence as set forth in SEQ ID NO: 270; or, the heavy chain variable region VH comprises an amino acid sequence as set forth in SEQ ID NO: 271; or, the heavy chain variable region VH comprises an amino acid sequence as set forth in SEQ ID NO: 272; or, the heavy chain variable region VH comprises an amino acid sequence as set forth in SEQ ID NO: 273; or, the heavy chain variable region VH comprises an amino acid sequence as set forth in SEQ ID NO: 274; or, the heavy chain variable region VH comprises an amino acid sequence as set forth in SEQ ID NO: 275; or, the heavy chain variable region VH comprises an amino acid sequence as set forth in SEQ ID NO: 276; or, the heavy chain variable region VH comprises an amino acid sequence as set forth in SEQ ID NO: 277; or, the heavy chain variable region VH comprises an amino acid sequence as set forth in SEQ ID NO: 278; or, the heavy chain variable region VH comprises an amino acid sequence as set forth in SEQ ID NO: 279; or, the heavy chain variable region VH comprises an amino acid sequence as set forth in SEQ ID NO: 280. More preferably, the BCMA antibody comprises one polypeptide chain, wherein the polypeptide chain comprises an amino acid sequence as set forth in SEQ ID NO: 330; or, an amino acid sequence as set forth in SEQ ID NO: 331; or, an amino acid sequence as set forth in SEQ ID NO: 332; or, an amino acid sequence as set forth in SEQ ID NO: 333; or, an amino acid sequence as set forth in SEQ ID NO: 334; or, an amino acid sequence as set forth in SEQ ID NO: 335; or, an amino acid sequence as set forth in SEQ ID NO: 336; or, an amino acid sequence as set forth in SEQ ID NO: 337; or, an amino acid sequence as set forth in SEQ ID NO: 338; or, an amino acid sequence as set forth in SEQ ID NO: 339; or, an amino acid sequence as set forth in SEQ ID NO: 340; or, an amino acid sequence as set forth in SEQ ID NO: 341; or, an amino acid sequence as set forth in SEQ ID NO: 342; or, an amino acid sequence as set forth in SEQ ID NO: 343; or, an amino acid sequence as set forth in SEQ ID NO: 344; or, an amino acid sequence as set forth in SEQ ID NO: 345.

In some specific embodiments, the protein functional region C is a heavy chain variable region of the BCMA heavy-chain antibody, wherein the heavy chain variable region VH comprises HCDR1, HCDR2 and HCDR3 with amino acid sequences as set forth in SEQ ID NOs: 24, 76 and 134, respectively; or, the heavy chain variable region VH comprises HCDR1, HCDR2 and HCDR3 with amino acid sequences as set forth in SEQ ID NOs: 26, 78 and 136, respectively. Preferably, the heavy chain variable region VH comprises an amino acid sequence as set forth in SEQ ID NO: 249; or, the heavy chain variable region VH comprises an amino acid sequence as set forth in SEQ ID NO: 251.

In the above specific embodiments, the binding protein may further comprise a light chain constant region, wherein the light chain constant region is preferably a human light chain constant region, and more preferably a human light chain constant region Cκ or Cλ.

In the above specific embodiments, the binding protein may further comprise a heavy chain constant region (CH1, CH2 and/or CH3), wherein the heavy chain constant region is preferably a human heavy chain constant region, and more preferably a human IgG1, IgG2, IgG3 or IgG4 heavy chain constant region. In some specific embodiments, the IgG1 heavy chain constant region has one or more of the mutations of C220S, N297A, L234A, L235A, P329G, S239D, I332E, S354C, T366W, Y349C, T366S, L368A, Y407V, M252Y, S254T, T256E and others on the Fc, and sites of the mutations are numbered according to an EU numbering scheme.

In some preferred embodiments, the binding protein comprises a protein functional region A and a protein functional region B, wherein: the protein functional region A comprises a light chain variable region and a heavy chain variable region, wherein the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 69 and 122, respectively; and the protein functional region B comprises a heavy chain variable region, wherein the heavy chain variable region VH comprises HCDR1, HCDR2 and HCDR3 with amino acid sequences as set forth in SEQ ID NOs: 17, 65 and 125, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 62 and 122, respectively; and the protein functional region B comprises a heavy chain variable region, wherein the heavy chain variable region VH comprises HCDR1, HCDR2 and HCDR3 with amino acid sequences as set forth in SEQ ID NOs: 17, 65 and 125, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 171, 190 and 215, respectively; and the heavy chain variable region VH comprises HCDR1, HCDR2 and HCDR3 with amino acid sequences as set forth in SEQ ID NOs: 19, 67 and 127, respectively; and the protein functional region B comprises a heavy chain variable region, wherein the heavy chain variable region VH comprises HCDR1, HCDR2 and HCDR3 with amino acid sequences as set forth in SEQ ID NOs: 17, 65 and 125, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 176, 196 and 220, respectively; and the heavy chain variable region VH comprises HCDR1, HCDR2 and HCDR3 with amino acid sequences as set forth in SEQ ID NOs: 23, 74 and 132, respectively; and the protein functional region B comprises a heavy chain variable region, wherein the heavy chain variable region VH comprises HCDR1, HCDR2 and HCDR3 with amino acid sequences as set forth in SEQ ID NOs: 17, 65 and 125, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 69 and 122, respectively; and the protein functional region B comprises a heavy chain variable region, wherein the heavy chain variable region VH comprises HCDR1, HCDR2 and HCDR3 with amino acid sequences as set forth in SEQ ID NOs: 28, 80 and 138, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 69 and 122, respectively; and the protein functional region B comprises a heavy chain variable region, wherein the heavy chain variable region VH comprises HCDR1, HCDR2 and HCDR3 with amino acid sequences as set forth in SEQ ID NOs: 27, 79 and 137, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 69 and 122, respectively; and the protein functional region B comprises a heavy chain variable region, wherein the heavy chain variable region VH comprises HCDR1, HCDR2 and HCDR3 with amino acid sequences as set forth in SEQ ID NOs: 29, 82 and 138, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 180, 191 and 225, respectively; and the heavy chain variable region VH comprises HCDR1, HCDR2 and HCDR3 with amino acid sequences as set forth in SEQ ID NOs: 32, 87 and 143, respectively; and the protein functional region B comprises a heavy chain variable region, wherein the heavy chain variable region VH comprises HCDR1, HCDR2 and HCDR3 with amino acid sequences as set forth in SEQ ID NOs: 28, 80 and 138, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 180, 191 and 225, respectively; and the heavy chain variable region VH comprises HCDR1, HCDR2 and HCDR3 with amino acid sequences as set forth in SEQ ID NOs: 32, 87 and 143, respectively; and the protein functional region B comprises a heavy chain variable region, wherein the heavy chain variable region VH comprises HCDR1, HCDR2 and HCDR3 with amino acid sequences as set forth in SEQ ID NOs: 27, 79 and 137, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 180, 191 and 225, respectively; and the heavy chain variable region VH comprises HCDR1, HCDR2 and HCDR3 with amino acid sequences as set forth in SEQ ID NOs: 32, 87 and 143, respectively; and the protein functional region B comprises a heavy chain variable region, wherein the heavy chain variable region VH comprises HCDR1, HCDR2 and HCDR3 with amino acid sequences as set forth in SEQ ID NOs: 28, 89 and 145, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 177, 191 and 226, respectively; and the heavy chain variable region VH comprises HCDR1, HCDR2 and HCDR3 with amino acid sequences as set forth in SEQ ID NOs: 33, 88 and 144, respectively; and the protein functional region B comprises a heavy chain variable region, wherein the heavy chain variable region VH comprises HCDR1, HCDR2 and HCDR3 with amino acid sequences as set forth in SEQ ID NOs: 28, 80 and 138, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 177, 191 and 226, respectively; and the heavy chain variable region VH comprises HCDR1, HCDR2 and HCDR3 with amino acid sequences as set forth in SEQ ID NOs: 33, 88 and 144, respectively; and the protein functional region B comprises a heavy chain variable region, wherein the heavy chain variable region VH comprises HCDR1, HCDR2 and HCDR3 with amino acid sequences as set forth in SEQ ID NOs: 28, 81 and 139, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 177, 191 and 226, respectively; and the heavy chain variable region VH comprises HCDR1, HCDR2 and HCDR3 with amino acid sequences as set forth in SEQ ID NOs: 33, 88 and 144, respectively; and the protein functional region B comprises a heavy chain variable region, wherein the heavy chain variable region VH comprises HCDR1, HCDR2 and HCDR3 with amino acid sequences as set forth in SEQ ID NOs: 15, 83 and 140, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 20, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 24, 76 and 134, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 20, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 20, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 15, 75 and 133, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 15, 75 and 133, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 26, 90 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 36, 90 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 76 and 147, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 34, 78 and 148, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 76 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 90 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 34, 76 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 76 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 34, 76 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 90 and 148, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 90 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 34, 78 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 78 and 147, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 90 and 147, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 177, 191 and 221, respectively; and the heavy chain variable region VH comprises HCDR1, HCDR2 and HCDR3 with amino acid sequences as set forth in SEQ ID NOs: 15, 84 and 141, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 178, 197 and 222, respectively; and the heavy chain variable region VH comprises HCDR1, HCDR2 and HCDR3 with amino acid sequences as set forth in SEQ ID NOs: 31, 85 and 142, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 177, 191 and 223, respectively; and the heavy chain variable region VH comprises HCDR1, HCDR2 and HCDR3 with amino acid sequences as set forth in SEQ ID NOs: 31, 86 and 141, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 179, 198 and 224, respectively; and the heavy chain variable region VH comprises HCDR1, HCDR2 and HCDR3 with amino acid sequences as set forth in SEQ ID NOs: 31, 85 and 142, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

in some preferred embodiments, the binding protein comprises a protein functional region A, a protein functional region B and a protein functional region C, wherein: the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; the protein functional region B comprises a heavy chain variable region, wherein the heavy chain variable region VH comprises HCDR1, HCDR2 and HCDR3 with amino acid sequences as set forth in SEQ ID NOs: 15, 75 and 133, respectively; and the protein functional region C comprises a heavy chain variable region, wherein the heavy chain variable region VH comprises HCDR1, HCDR2 and HCDR3 with amino acid sequences as set forth in SEQ ID NOs: 24, 76 and 134, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; the protein functional region B comprises a heavy chain variable region, wherein the heavy chain variable region VH comprises HCDR1, HCDR2 and HCDR3 with amino acid sequences as set forth in SEQ ID NOs: 25, 77 and 135, respectively; and the protein functional region C comprises a heavy chain variable region, wherein the heavy chain variable region VH comprises HCDR1, HCDR2 and HCDR3 with amino acid sequences as set forth in SEQ ID NOs: 26, 78 and 136, respectively. The amino acid sequences of the listed CDRs are shown according to the Chothia scheme.

In some more preferred embodiments, the binding protein comprises a protein functional region A and a protein functional region B, wherein: the protein functional region A comprises a light chain variable region and a heavy chain variable region, wherein the light chain variable region VL comprises an amino acid sequence as set forth in SEQ ID NO: 282; and the heavy chain variable region VH comprises an amino acid sequence as set forth in SEQ ID NO: 240; and the protein functional region B comprises a heavy chain variable region, wherein the heavy chain variable region VH comprises an amino acid sequence as set forth in SEQ ID NO: 236; or,

the protein functional region A comprises a light chain variable region and a heavy chain variable region, wherein the light chain variable region VL comprises an amino acid sequence as set forth in SEQ ID NO: 282; and the heavy chain variable region VH comprises an amino acid sequence as set forth in SEQ ID NO: 233; and the protein functional region B comprises a heavy chain variable region, wherein the heavy chain variable region VH comprises an amino acid sequence as set forth in SEQ ID NO: 236; or,

the protein functional region A comprises a light chain variable region and a heavy chain variable region, wherein the light chain variable region VL comprises an amino acid sequence as set forth in SEQ ID NO: 286; and the heavy chain variable region VH comprises an amino acid sequence as set forth in SEQ ID NO: 238; and the protein functional region B comprises a heavy chain variable region, wherein the heavy chain variable region VH comprises an amino acid sequence as set forth in SEQ ID NO: 236; or,

the protein functional region A comprises a light chain variable region and a heavy chain variable region, wherein the light chain variable region VL comprises an amino acid sequence as set forth in SEQ ID NO: 293; and the heavy chain variable region VH comprises an amino acid sequence as set forth in SEQ ID NO: 247; and the protein functional region B comprises a heavy chain variable region, wherein the heavy chain variable region VH comprises an amino acid sequence as set forth in SEQ ID NO: 236; or,

the protein functional region A comprises a light chain variable region and a heavy chain variable region, wherein the light chain variable region VL comprises an amino acid sequence as set forth in SEQ ID NO: 282; and the heavy chain variable region VH comprises an amino acid sequence as set forth in SEQ ID NO: 240; and the protein functional region B comprises a heavy chain variable region, wherein the heavy chain variable region VH comprises an amino acid sequence as set forth in SEQ ID NO: 253; or,

the protein functional region A comprises a light chain variable region and a heavy chain variable region, wherein the light chain variable region VL comprises an amino acid sequence as set forth in SEQ ID NO: 282; and the heavy chain variable region VH comprises an amino acid sequence as set forth in SEQ ID NO: 240; and the protein functional region B comprises a heavy chain variable region, wherein the heavy chain variable region VH comprises an amino acid sequence as set forth in SEQ ID NO: 252; or,

the protein functional region A comprises a light chain variable region and a heavy chain variable region, wherein the light chain variable region VL comprises an amino acid sequence as set forth in SEQ ID NO: 282; and the heavy chain variable region VH comprises an amino acid sequence as set forth in SEQ ID NO: 240; and the protein functional region B comprises a heavy chain variable region, wherein the heavy chain variable region VH comprises an amino acid sequence as set forth in SEQ ID NO: 255; or,

the protein functional region A comprises a light chain variable region and a heavy chain variable region, wherein the light chain variable region VL comprises an amino acid sequence as set forth in SEQ ID NO: 298; and the heavy chain variable region VH comprises an amino acid sequence as set forth in SEQ ID NO: 261; and the protein functional region B comprises a heavy chain variable region, wherein the heavy chain variable region VH comprises an amino acid sequence as set forth in SEQ ID NO: 253; or,

the protein functional region A comprises a light chain variable region and a heavy chain variable region, wherein the light chain variable region VL comprises an amino acid sequence as set forth in SEQ ID NO: 298; and the heavy chain variable region VH comprises an amino acid sequence as set forth in SEQ ID NO: 261; and the protein functional region B comprises a heavy chain variable region, wherein the heavy chain variable region VH comprises an amino acid sequence as set forth in SEQ ID NO: 252; or,

the protein functional region A comprises a light chain variable region and a heavy chain variable region, wherein the light chain variable region VL comprises an amino acid sequence as set forth in SEQ ID NO: 298; and the heavy chain variable region VH comprises an amino acid sequence as set forth in SEQ ID NO: 261; and the protein functional region B comprises a heavy chain variable region, wherein the heavy chain variable region VH comprises an amino acid sequence as set forth in SEQ ID NO: 264; or,

the protein functional region A comprises a light chain variable region and a heavy chain variable region, wherein the light chain variable region VL comprises an amino acid sequence as set forth in SEQ ID NO: 299; and the heavy chain variable region VH comprises an amino acid sequence as set forth in SEQ ID NO: 262; and the protein functional region B comprises a heavy chain variable region, wherein the heavy chain variable region VH comprises an amino acid sequence as set forth in SEQ ID NO: 253; or,

the protein functional region A comprises a light chain variable region and a heavy chain variable region, wherein the light chain variable region VL comprises an amino acid sequence as set forth in SEQ ID NO: 299; and the heavy chain variable region VH comprises an amino acid sequence as set forth in SEQ ID NO: 262; and the protein functional region B comprises a heavy chain variable region, wherein the heavy chain variable region VH comprises an amino acid sequence as set forth in SEQ ID NO: 254; or,

the protein functional region A comprises a light chain variable region and a heavy chain variable region, wherein the light chain variable region VL comprises an amino acid sequence as set forth in SEQ ID NO: 299; and the heavy chain variable region VH comprises an amino acid sequence as set forth in SEQ ID NO: 262; and the protein functional region B comprises a heavy chain variable region, wherein the heavy chain variable region VH comprises an amino acid sequence as set forth in SEQ ID NO: 256; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 245; and the protein functional region B comprises a heavy chain variable region, wherein the heavy chain variable region VH comprises an amino acid sequence as set forth in SEQ ID NO: 249; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 245; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 245; and the protein functional region B comprises a heavy chain variable region, wherein the heavy chain variable region VH comprises an amino acid sequence as set forth in SEQ ID NO: 248; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 257; and the protein functional region B comprises a heavy chain variable region, wherein the heavy chain variable region VH comprises an amino acid sequence as set forth in SEQ ID NO: 248; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 257; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 281; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 265; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 269; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 270; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 271; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 272; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 273; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 274; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 275; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 276; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 277; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 278; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 279; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 280; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 266; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 267; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 268; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 244; and the protein functional region B comprises a heavy chain variable region, wherein the heavy chain variable region VH comprises an amino acid sequence as set forth in SEQ ID NO: 249; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 244; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 294; and the heavy chain variable region VH comprises an amino acid sequence as set forth in SEQ ID NO: 258; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 295; and the heavy chain variable region VH comprises an amino acid sequence as set forth in SEQ ID NO: 259; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 296; and the heavy chain variable region VH comprises an amino acid sequence as set forth in SEQ ID NO: 260; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 297; and the heavy chain variable region VH comprises an amino acid sequence as set forth in SEQ ID NO: 259; and the protein functional region B comprises a heavy chain variable region, wherein the heavy chain variable region VH comprises an amino acid sequence as set forth in SEQ ID NO: 251; or,

in some more preferred embodiments, the binding protein comprises a protein functional region A, a protein functional region B and a protein functional region C, wherein the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 257; and the protein functional region B comprises a heavy chain variable region, wherein the heavy chain variable region VH comprises an amino acid sequence as set forth in SEQ ID NO: 248. The protein functional region C comprises a heavy chain variable region, wherein the heavy chain variable region VH comprises an amino acid sequence as set forth in SEQ ID NO: 249; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 257; the protein functional region B comprises a heavy chain variable region, wherein the heavy chain variable region VH comprises an amino acid sequence as set forth in SEQ ID NO: 250; and the protein functional region C comprises a heavy chain variable region, wherein the heavy chain variable region VH comprises an amino acid sequence as set forth in SEQ ID NO: 251.

In some more preferred embodiments, the binding protein comprises two polypeptide chains, wherein a first polypeptide chain comprises an amino acid sequence as set forth in SEQ ID NO: 371; and a second polypeptide chain comprises an amino acid sequence as set forth in SEQ ID NO: 372; or,

a first polypeptide chain comprises an amino acid sequence as set forth in SEQ ID NO: 371; and a second polypeptide chain comprises an amino acid sequence as set forth in SEQ ID NO: 373; or,

a first polypeptide chain comprises an amino acid sequence as set forth in SEQ ID NO: 353; and a second polypeptide chain comprises an amino acid sequence as set forth in SEQ ID NO: 362; or,

a first polypeptide chain comprises an amino acid sequence as set forth in SEQ ID NO: 363; and a second polypeptide chain comprises an amino acid sequence as set forth in SEQ ID NO: 364; or,

a first polypeptide chain comprises an amino acid sequence as set forth in SEQ ID NO: 365; and a second polypeptide chain comprises an amino acid sequence as set forth in SEQ ID NO: 364; or,

a first polypeptide chain comprises an amino acid sequence as set forth in SEQ ID NO: 353; and a second polypeptide chain comprises an amino acid sequence as set forth in SEQ ID NO: 366; or,

a first polypeptide chain comprises an amino acid sequence as set forth in SEQ ID NO: 353; and a second polypeptide chain comprises an amino acid sequence as set forth in SEQ ID NO: 369; or,

a first polypeptide chain comprises an amino acid sequence as set forth in SEQ ID NO: 353; and a second polypeptide chain comprises an amino acid sequence as set forth in SEQ ID NO: 370; or,

a first polypeptide chain comprises an amino acid sequence as set forth in SEQ ID NO: 353; and a second polypeptide chain comprises an amino acid sequence as set forth in SEQ ID NO: 394; or,

a first polypeptide chain comprises an amino acid sequence as set forth in SEQ ID NO: 353; and a second polypeptide chain comprises an amino acid sequence as set forth in SEQ ID NO: 395; or,

a first polypeptide chain comprises an amino acid sequence as set forth in SEQ ID NO: 363; and a second polypeptide chain comprises an amino acid sequence as set forth in SEQ ID NO: 310; or,

a first polypeptide chain comprises an amino acid sequence as set forth in SEQ ID NO: 363; and a second polypeptide chain comprises an amino acid sequence as set forth in SEQ ID NO: 396; or,

a first polypeptide chain comprises an amino acid sequence as set forth in SEQ ID NO: 363; and a second polypeptide chain comprises an amino acid sequence as set forth in SEQ ID NO: 362; or,

a first polypeptide chain comprises an amino acid sequence as set forth in SEQ ID NO: 363; and a second polypeptide chain comprises an amino acid sequence as set forth in SEQ ID NO: 394; or,

a first polypeptide chain comprises an amino acid sequence as set forth in SEQ ID NO: 363; and a second polypeptide chain comprises an amino acid sequence as set forth in SEQ ID NO: 395; or,

a first polypeptide chain comprises an amino acid sequence as set forth in SEQ ID NO: 367; and a second polypeptide chain comprises an amino acid sequence as set forth in SEQ ID NO: 368; or,

a first polypeptide chain comprises an amino acid sequence as set forth in SEQ ID NO: 367; and a second polypeptide chain comprises an amino acid sequence as set forth in SEQ ID NO: 378; or,

a first polypeptide chain comprises an amino acid sequence as set forth in SEQ ID NO: 367; and a second polypeptide chain comprises an amino acid sequence as set forth in SEQ ID NO: 379; or,

a first polypeptide chain comprises an amino acid sequence as set forth in SEQ ID NO: 367; and a second polypeptide chain comprises an amino acid sequence as set forth in SEQ ID NO: 380; or,

a first polypeptide chain comprises an amino acid sequence as set forth in SEQ ID NO: 367; and a second polypeptide chain comprises an amino acid sequence as set forth in SEQ ID NO: 381; or,

a first polypeptide chain comprises an amino acid sequence as set forth in SEQ ID NO: 367; and a second polypeptide chain comprises an amino acid sequence as set forth in SEQ ID NO: 382; or,

a first polypeptide chain comprises an amino acid sequence as set forth in SEQ ID NO: 367; and a second polypeptide chain comprises an amino acid sequence as set forth in SEQ ID NO: 383; or,

a first polypeptide chain comprises an amino acid sequence as set forth in SEQ ID NO: 367; and a second polypeptide chain comprises an amino acid sequence as set forth in SEQ ID NO: 385; or,

a first polypeptide chain comprises an amino acid sequence as set forth in SEQ ID NO: 367; and a second polypeptide chain comprises an amino acid sequence as set forth in SEQ ID NO: 388; or,

a first polypeptide chain comprises an amino acid sequence as set forth in SEQ ID NO: 367; and a second polypeptide chain comprises an amino acid sequence as set forth in SEQ ID NO: 389; or,

a first polypeptide chain comprises an amino acid sequence as set forth in SEQ ID NO: 351; and a second polypeptide chain comprises an amino acid sequence as set forth in SEQ ID NO: 374; or,

a first polypeptide chain comprises an amino acid sequence as set forth in SEQ ID NO: 351; and a second polypeptide chain comprises an amino acid sequence as set forth in SEQ ID NO: 375; or,

a first polypeptide chain comprises an amino acid sequence as set forth in SEQ ID NO: 351; and a second polypeptide chain comprises an amino acid sequence as set forth in SEQ ID NO: 386; or,

a first polypeptide chain comprises an amino acid sequence as set forth in SEQ ID NO: 351; and a second polypeptide chain comprises an amino acid sequence as set forth in SEQ ID NO: 387; or,

a first polypeptide chain comprises an amino acid sequence as set forth in SEQ ID NO: 351; and a second polypeptide chain comprises an amino acid sequence as set forth in SEQ ID NO: 401; or,

a first polypeptide chain comprises an amino acid sequence as set forth in SEQ ID NO: 402; and a second polypeptide chain comprises an amino acid sequence as set forth in SEQ ID NO: 305; or,

a first polypeptide chain comprises an amino acid sequence as set forth in SEQ ID NO: 351; and a second polypeptide chain comprises an amino acid sequence as set forth in SEQ ID NO: 403; or,

a first polypeptide chain comprises an amino acid sequence as set forth in SEQ ID NO: 402; and a second polypeptide chain comprises an amino acid sequence as set forth in SEQ ID NO: 409; or,

a first polypeptide chain comprises an amino acid sequence as set forth in SEQ ID NO: 359; and a second polypeptide chain comprises an amino acid sequence as set forth in SEQ ID NO: 404; or,

a first polypeptide chain comprises an amino acid sequence as set forth in SEQ ID NO: 405; and a second polypeptide chain comprises an amino acid sequence as set forth in SEQ ID NO: 315; or,

a first polypeptide chain comprises an amino acid sequence as set forth in SEQ ID NO: 359; and a second polypeptide chain comprises an amino acid sequence as set forth in SEQ ID NO: 406; or,

a first polypeptide chain comprises an amino acid sequence as set forth in SEQ ID NO: 351; and a second polypeptide chain comprises an amino acid sequence as set forth in SEQ ID NO: 376; or,

a first polypeptide chain comprises an amino acid sequence as set forth in SEQ ID NO: 351; and a second polypeptide chain comprises an amino acid sequence as set forth in SEQ ID NO: 377; or,

a first polypeptide chain comprises an amino acid sequence as set forth in SEQ ID NO: 351; and a second polypeptide chain comprises an amino acid sequence as set forth in SEQ ID NO: 397; or,

a first polypeptide chain comprises an amino acid sequence as set forth in SEQ ID NO: 351; and a second polypeptide chain comprises an amino acid sequence as set forth in SEQ ID NO: 398; or,

a first polypeptide chain comprises an amino acid sequence as set forth in SEQ ID NO: 351; and a second polypeptide chain comprises an amino acid sequence as set forth in SEQ ID NO: 399; or,

a first polypeptide chain comprises an amino acid sequence as set forth in SEQ ID NO: 351; and a second polypeptide chain comprises an amino acid sequence as set forth in SEQ ID NO: 400; or,

a first polypeptide chain comprises an amino acid sequence as set forth in SEQ ID NO: 402; and a second polypeptide chain comprises an amino acid sequence as set forth in SEQ ID NO: 401; or,

a first polypeptide chain comprises an amino acid sequence as set forth in SEQ ID NO: 402; and a second polypeptide chain comprises an amino acid sequence as set forth in SEQ ID NO: 403; or,

a first polypeptide chain comprises an amino acid sequence as set forth in SEQ ID NO: 405; and a second polypeptide chain comprises an amino acid sequence as set forth in SEQ ID NO: 404; or,

a first polypeptide chain comprises an amino acid sequence as set forth in SEQ ID NO: 405; and a second polypeptide chain comprises an amino acid sequence as set forth in SEQ ID NO: 406; or,

a first polypeptide chain comprises an amino acid sequence as set forth in SEQ ID NO: 371; and a second polypeptide chain comprises an amino acid sequence as set forth in SEQ ID NO: 486; or,

a first polypeptide chain comprises an amino acid sequence as set forth in SEQ ID NO: 353; and a second polypeptide chain comprises an amino acid sequence as set forth in SEQ ID NO: 460; or,

a first polypeptide chain comprises an amino acid sequence as set forth in SEQ ID NO: 353; and a second polypeptide chain comprises an amino acid sequence as set forth in SEQ ID NO: 461; or,

a first polypeptide chain comprises an amino acid sequence as set forth in SEQ ID NO: 353; and a second polypeptide chain comprises an amino acid sequence as set forth in SEQ ID NO: 462; or,

a first polypeptide chain comprises an amino acid sequence as set forth in SEQ ID NO: 487; and a second polypeptide chain comprises an amino acid sequence as set forth in SEQ ID NO: 488; or,

a first polypeptide chain comprises an amino acid sequence as set forth in SEQ ID NO: 360; and a second polypeptide chain comprises an amino acid sequence as set forth in SEQ ID NO: 455; or,

a first polypeptide chain comprises an amino acid sequence as set forth in SEQ ID NO: 360; and a second polypeptide chain comprises an amino acid sequence as set forth in SEQ ID NO: 456; or,

a first polypeptide chain comprises an amino acid sequence as set forth in SEQ ID NO: 360; and a second polypeptide chain comprises an amino acid sequence as set forth in SEQ ID NO: 457; or,

a first polypeptide chain comprises an amino acid sequence as set forth in SEQ ID NO: 360; and a second polypeptide chain comprises an amino acid sequence as set forth in SEQ ID NO: 458; or,

a first polypeptide chain comprises an amino acid sequence as set forth in SEQ ID NO: 360; and a second polypeptide chain comprises an amino acid sequence as set forth in SEQ ID NO: 459; or,

a first polypeptide chain comprises an amino acid sequence as set forth in SEQ ID NO: 419; and a second polypeptide chain comprises an amino acid sequence as set forth in SEQ ID NO: 412; or,

a first polypeptide chain comprises an amino acid sequence as set forth in SEQ ID NO: 419; and a second polypeptide chain comprises an amino acid sequence as set forth in SEQ ID NO: 414; or,

a first polypeptide chain comprises an amino acid sequence as set forth in SEQ ID NO: 440; and a second polypeptide chain comprises an amino acid sequence as set forth in SEQ ID NO: 428; or,

a first polypeptide chain comprises an amino acid sequence as set forth in SEQ ID NO: 441; and a second polypeptide chain comprises an amino acid sequence as set forth in SEQ ID NO: 428; or,

a first polypeptide chain comprises an amino acid sequence as set forth in SEQ ID NO: 442; and a second polypeptide chain comprises an amino acid sequence as set forth in SEQ ID NO: 428; or,

a first polypeptide chain comprises an amino acid sequence as set forth in SEQ ID NO: 443; and a second polypeptide chain comprises an amino acid sequence as set forth in SEQ ID NO: 428; or,

a first polypeptide chain comprises an amino acid sequence as set forth in SEQ ID NO: 487; and a second polypeptide chain comprises an amino acid sequence as set forth in SEQ ID NO: 520; or,

a first polypeptide chain comprises an amino acid sequence as set forth in SEQ ID NO: 353; and a second polypeptide chain comprises an amino acid sequence as set forth in SEQ ID NO: 521; or,

a first polypeptide chain comprises an amino acid sequence as set forth in SEQ ID NO: 371; and a second polypeptide chain comprises an amino acid sequence as set forth in SEQ ID NO: 522; or,

a first polypeptide chain comprises an amino acid sequence as set forth in SEQ ID NO: 360; and a second polypeptide chain comprises an amino acid sequence as set forth in SEQ ID NO: 523.

In some more preferred embodiments, the binding protein comprises three polypeptide chains, wherein a first polypeptide chain comprises an amino acid sequence as set forth in SEQ ID NO: 353; a second polypeptide chain comprises an amino acid sequence as set forth in SEQ ID NO: 407; and a third polypeptide chain comprises an amino acid sequence as set forth in SEQ ID NO: 390; or,

a first polypeptide chain comprises an amino acid sequence as set forth in SEQ ID NO: 353; a second polypeptide chain comprises an amino acid sequence as set forth in SEQ ID NO: 408; and a third polypeptide chain comprises an amino acid sequence as set forth in SEQ ID NO: 392; or,

a first polypeptide chain comprises an amino acid sequence as set forth in SEQ ID NO: 351; a second polypeptide chain comprises an amino acid sequence as set forth in SEQ ID NO: 391; and a third polypeptide chain comprises an amino acid sequence as set forth in SEQ ID NO: 390; or,

a first polypeptide chain comprises an amino acid sequence as set forth in SEQ ID NO: 351; a second polypeptide chain comprises an amino acid sequence as set forth in SEQ ID NO: 393; and a third polypeptide chain comprises an amino acid sequence as set forth in SEQ ID NO: 392; or,

a first polypeptide chain comprises an amino acid sequence as set forth in SEQ ID NO: 351; a second polypeptide chain comprises an amino acid sequence as set forth in SEQ ID NO: 391; and a third polypeptide chain comprises an amino acid sequence as set forth in SEQ ID NO: 434; or,

a first polypeptide chain comprises an amino acid sequence as set forth in SEQ ID NO: 351; a second polypeptide chain comprises an amino acid sequence as set forth in SEQ ID NO: 391; and a third polypeptide chain comprises an amino acid sequence as set forth in SEQ ID NO: 435; or,

a first polypeptide chain comprises an amino acid sequence as set forth in SEQ ID NO: 351; a second polypeptide chain comprises an amino acid sequence as set forth in SEQ ID NO: 393; and a third polypeptide chain comprises an amino acid sequence as set forth in SEQ ID NO: 436; or,

a first polypeptide chain comprises an amino acid sequence as set forth in SEQ ID NO: 351; a second polypeptide chain comprises an amino acid sequence as set forth in SEQ ID NO: 393; and a third polypeptide chain comprises an amino acid sequence as set forth in SEQ ID NO: 437; or,

a first polypeptide chain comprises an amino acid sequence as set forth in SEQ ID NO: 367; a second polypeptide chain comprises an amino acid sequence as set forth in SEQ ID NO: 438; and a third polypeptide chain comprises an amino acid sequence as set forth in SEQ ID NO: 434; or,

a first polypeptide chain comprises an amino acid sequence as set forth in SEQ ID NO: 367; a second polypeptide chain comprises an amino acid sequence as set forth in SEQ ID NO: 438; and a third polypeptide chain comprises an amino acid sequence as set forth in SEQ ID NO: 435; or,

a first polypeptide chain comprises an amino acid sequence as set forth in SEQ ID NO: 367; a second polypeptide chain comprises an amino acid sequence as set forth in SEQ ID NO: 439; and a third polypeptide chain comprises an amino acid sequence as set forth in SEQ ID NO: 436; or,

a first polypeptide chain comprises an amino acid sequence as set forth in SEQ ID NO: 367; a second polypeptide chain comprises an amino acid sequence as set forth in SEQ ID NO: 439; and a third polypeptide chain comprises an amino acid sequence as set forth in SEQ ID NO: 437; or,

a first polypeptide chain comprises an amino acid sequence as set forth in SEQ ID NO: 361; a second polypeptide chain comprises an amino acid sequence as set forth in SEQ ID NO: 481; and a third polypeptide chain comprises an amino acid sequence as set forth in SEQ ID NO: 482; or,

a first polypeptide chain comprises an amino acid sequence as set forth in SEQ ID NO: 361; a second polypeptide chain comprises an amino acid sequence as set forth in SEQ ID NO: 481; and a third polypeptide chain comprises an amino acid sequence as set forth in SEQ ID NO: 483; or,

a first polypeptide chain comprises an amino acid sequence as set forth in SEQ ID NO: 361; a second polypeptide chain comprises an amino acid sequence as set forth in SEQ ID NO: 481; and a third polypeptide chain comprises an amino acid sequence as set forth in SEQ ID NO: 484; or,

a first polypeptide chain comprises an amino acid sequence as set forth in SEQ ID NO: 361; a second polypeptide chain comprises an amino acid sequence as set forth in SEQ ID NO: 481; and a third polypeptide chain comprises an amino acid sequence as set forth in SEQ ID NO: 485; or,

a first polypeptide chain comprises an amino acid sequence as set forth in SEQ ID NO: 357; a second polypeptide chain comprises an amino acid sequence as set forth in SEQ ID NO: 411; and a third polypeptide chain comprises an amino acid sequence as set forth in SEQ ID NO: 412; or,

a first polypeptide chain comprises an amino acid sequence as set forth in SEQ ID NO: 357; a second polypeptide chain comprises an amino acid sequence as set forth in SEQ ID NO: 411; and a third polypeptide chain comprises an amino acid sequence as set forth in SEQ ID NO: 414; or,

a first polypeptide chain comprises an amino acid sequence as set forth in SEQ ID NO: 415; a second polypeptide chain comprises an amino acid sequence as set forth in SEQ ID NO: 416; and a third polypeptide chain comprises an amino acid sequence as set forth in SEQ ID NO: 410; or,

a first polypeptide chain comprises an amino acid sequence as set forth in SEQ ID NO: 415; a second polypeptide chain comprises an amino acid sequence as set forth in SEQ ID NO: 416; and a third polypeptide chain comprises an amino acid sequence as set forth in SEQ ID NO: 412; or,

a first polypeptide chain comprises an amino acid sequence as set forth in SEQ ID NO: 415; a second polypeptide chain comprises an amino acid sequence as set forth in SEQ ID NO: 416; and a third polypeptide chain comprises an amino acid sequence as set forth in SEQ ID NO: 414; or,

a first polypeptide chain comprises an amino acid sequence as set forth in SEQ ID NO: 417; a second polypeptide chain comprises an amino acid sequence as set forth in SEQ ID NO: 418; and a third polypeptide chain comprises an amino acid sequence as set forth in SEQ ID NO: 412; or,

a first polypeptide chain comprises an amino acid sequence as set forth in SEQ ID NO: 417; a second polypeptide chain comprises an amino acid sequence as set forth in SEQ ID NO: 418; and a third polypeptide chain comprises an amino acid sequence as set forth in SEQ ID NO: 414; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 426; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 427; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 428; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 429; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 449; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 449; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 445; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 446; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 445; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 446; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 447; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 448; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 447; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 448; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 445; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 446; or,

a first polypeptide chain comprises an amino acid sequence as set forth in SEQ ID NO: 357; a second polypeptide chain comprises an amino acid sequence as set forth in SEQ ID NO: 463; and a third polypeptide chain comprises an amino acid sequence as set forth in SEQ ID NO: 464; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 465; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 466; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 467; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 468; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 469; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 470; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 471; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 472; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 473; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 474; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 475; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 476; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 477; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 478; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 479; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 480; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 430; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 431; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 432; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 433.

In order to solve the above technical problems, a second technical solution of the present invention is as follows: provided is an isolated nucleic acid encoding the binding protein described above.

In order to solve the above technical problems, a third technical solution of the present invention is as follows: provided is an expression vector comprising the isolated nucleic acid described above.

In order to solve the above technical problems, a fourth technical solution of the present invention is as follows: provided is a host cell comprising the expression vector described above, wherein the host cell is a prokaryotic cell or a eukaryotic cell.

In order to solve the above technical problems, a fifth technical solution of the present invention is as follows: provided is a method for preparing the binding protein described above, which comprises the following steps: culturing the host cell described above, and obtaining the binding protein from a culture.

In order to solve the above technical problems, a sixth technical solution of the present invention is as follows: provided is a pharmaceutical composition comprising the binding protein described above.

In order to solve the above technical problems, a seventh technical solution of the present invention is as follows: provided is a combination of kits comprising a kit I and a kit I, wherein the kit I comprises the binding protein or the pharmaceutical composition described above, and the second kit comprises an additional antibody or pharmaceutical composition for treating cancer.

In order to solve the above technical problems, an eighth technical solution of the present invention is as follows: provided is use of the binding protein or the pharmaceutical

composition described above in the preparation of a drug for treating and/or preventing cancer. The cancer is preferably breast cancer, ovarian cancer, endometrial cancer, renal cancer, melanoma, lung cancer, gastric cancer, liver cancer, esophageal cancer, cervical cancer, head and neck tumor, cholangiocarcinoma, gallbladder cancer, bladder cancer, sarcoma, colorectal cancer, lymphoma or multiple myeloma.

In order to solve the above technical problems, a ninth technical solution of the present invention is as follows: provided is a method for treating cancer, which comprises administering to a subject in need thereof the binding protein according to the first technical solution of the present invention, the pharmaceutical composition according to the sixth technical solution of the present invention or the combination of kits according to the seventh technical solution of the present invention; preferably, further comprises administering an additional antibody for treating cancer, such as an immune checkpoint antibody and/or a chemotherapeutic agent.

The cancer is preferably breast cancer, ovarian cancer, endometrial cancer, renal cancer, melanoma, lung cancer, gastric cancer, liver cancer, esophageal cancer, cervical cancer, head and neck tumor, cholangiocarcinoma, gallbladder cancer, bladder cancer, sarcoma, colorectal cancer, lymphoma or multiple myeloma.

In order to solve the above technical problems, a tenth technical solution of the present invention is as follows: provided is a CD3 antibody.

The CD3 antibody comprises a light chain variable region and a heavy chain variable region, wherein the light chain variable region VL comprises LCDR1, LCDR2 and LCDR3 with amino acid sequences as set forth in SEQ ID NOs: 177, 191 and 221, respectively; and the heavy chain variable region VH comprises HCDR1, HCDR2 and HCDR3 with amino acid sequences as set forth in SEQ ID NOs: 15, 84 and 141, respectively; or, the light chain variable region VL comprises LCDR1, LCDR2 and LCDR3 with amino acid sequences as set forth in SEQ ID NOs: 178, 197 and 222, respectively; and the heavy chain variable region VH comprises HCDR1, HCDR2 and HCDR3 with amino acid sequences as set forth in SEQ ID NOs: 31, 85 and 142, respectively; or, the light chain variable region VL comprises LCDR1, LCDR2 and LCDR3 with amino acid sequences as set forth in SEQ ID NOs: 177, 191 and 223, respectively; and the heavy chain variable region VH comprises HCDR1, HCDR2 and HCDR3 with amino acid sequences as set forth in SEQ ID NOs: 31, 86 and 141, respectively; or, the light chain variable region VL comprises LCDR1, LCDR2 and LCDR3 with amino acid sequences as set forth in SEQ ID NOs: 179, 198 and 224, respectively; and the heavy chain variable region VH comprises HCDR1, HCDR2 and HCDR3 with amino acid sequences as set forth in SEQ ID NOs: 31, 85 and 142, respectively. The amino acid sequences of the listed CDRs are shown according to the Chothia scheme. Preferably, the light chain variable region VL comprises an amino acid sequence as set forth in SEQ ID NO: 294; and the heavy chain variable region VH comprises an amino acid sequence as set forth in SEQ ID NO: 258; or, the light chain variable region VL comprises an amino acid sequence as set forth in SEQ ID NO: 295; and the heavy chain variable region VH comprises an amino acid sequence as set forth in SEQ ID NO: 259; or, the light chain variable region VL comprises an amino acid sequence as set forth in SEQ ID NO: 296; and the heavy chain variable region VH comprises an amino acid sequence as set forth in SEQ ID NO: 260; or, the light chain variable region VL comprises an amino acid sequence as set forth in SEQ ID NO: 297; and the heavy chain variable region VH comprises an amino acid sequence as set forth in SEQ ID NO: 259.

Alternatively, the CD3 antibody comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 20, 68 and 128, respectively; or, the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively. The amino acid sequences of the listed CDRs are shown according to the Chothia scheme. Preferably, the light chain variable region VL comprises an amino acid sequence as set forth in SEQ ID NO: 291; and the heavy chain variable region VH comprises an amino acid sequence as set forth in SEQ ID NO: 245; or, the light chain variable region VL comprises an amino acid sequence as set forth in SEQ ID NO: 291; and the heavy chain variable region VH comprises an amino acid sequence as set forth in SEQ ID NO: 257; or, the light chain variable region VL comprises an amino acid sequence as set forth in SEQ ID NO: 291; and the heavy chain variable region VH comprises an amino acid sequence as set forth in SEQ ID NO: 263; or, the light chain variable region VL comprises an amino acid sequence as set forth in SEQ ID NO: 291; and the heavy chain variable region VH comprises an amino acid sequence as set forth in SEQ ID NO: 281; or, the light chain variable region VL comprises an amino acid sequence as set forth in SEQ ID NO: 291; and the heavy chain variable region VH comprises an amino acid sequence as set forth in SEQ ID NO: 244.

In some specific embodiments, the CD3 antibody comprises two polypeptide chains, wherein a first polypeptide chain comprises an amino acid sequence as set forth in SEQ ID NO: 357, and a second polypeptide chain comprises an amino acid sequence as set forth in SEQ ID NO: 313; or, a first polypeptide chain comprises an amino acid sequence as set forth in SEQ ID NO: 357, and a second polypeptide chain comprises an amino acid sequence as set forth in SEQ ID NO: 325; or, a first polypeptide chain comprises an amino acid sequence as set forth in SEQ ID NO: 357, and a second polypeptide chain comprises an amino acid sequence as set forth in SEQ ID NO: 328; or, a first polypeptide chain comprises an amino acid sequence as set forth in SEQ ID NO: 357, and a second polypeptide chain comprises an amino acid sequence as set forth in SEQ ID NO: 346.

In some specific embodiments, the CD3 antibody comprises one polypeptide chain, wherein the polypeptide chain comprises an amino acid sequence as set forth in SEQ ID NO: 489, an amino acid sequence as set forth in SEQ ID NO: 490, an amino acid sequence as set forth in SEQ ID NO: 491, an amino acid sequence as set forth in SEQ ID NO: 492 or an amino acid sequence as set forth in SEQ ID NO: 493.

In order to solve the above technical problems, an eleventh technical solution of the present invention is as follows: provided is a BCMA antibody.

The BCMA antibody comprises a heavy chain variable region, wherein the heavy chain variable region VH comprises HCDR1, HCDR2 and HCDR3 with amino acid sequences as set forth in SEQ ID NOs: 15, 75 and 133, respectively; or, the heavy chain variable region VH comprises HCDR1, HCDR2 and HCDR3 with amino acid sequences as set forth in SEQ ID NOs: 24, 76 and 134, respectively; or, the heavy chain variable region VH comprises HCDR1, HCDR2 and HCDR3 with amino acid sequences as set forth in SEQ ID NOs: 25, 77 and 135, respectively; or, the heavy chain variable region VH comprises HCDR1, HCDR2 and HCDR3 with amino acid sequences as set forth in SEQ ID NOs: 26, 78 and 136, respectively. The amino acid sequences of the listed CDRs are shown according to the Chothia scheme. Preferably, the heavy chain variable region VH comprises an amino acid sequence as set forth in SEQ ID NO: 248; or, the heavy chain variable region VH comprises an amino acid sequence as set forth in SEQ ID NO: 249; or, the heavy chain variable region VH comprises an amino acid sequence as set forth in SEQ ID NO: 250; or, the heavy chain variable region VH comprises an amino acid sequence as set forth in SEQ ID NO: 251. More preferably, the BCMA antibody comprises one polypeptide chain, wherein the polypeptide chain comprises an amino acid sequence as set forth in SEQ ID NO: 316, an amino acid sequence as set forth in SEQ ID NO: 317, an amino acid sequence as set forth in SEQ ID NO: 318 or an amino acid sequence as set forth in SEQ ID NO: 319.

Alternatively, the BCMA antibody comprises a heavy chain variable region, wherein the heavy chain variable region VH comprises HCDR1, HCDR2, and HCDR3 with amino acid sequences as set forth in SEQ ID NOs: 26, 90 and 136, respectively; or, the heavy chain variable region VH comprises HCDR1, HCDR2 and HCDR3 with amino acid sequences as set forth in SEQ ID NOs: 34, 78 and 146, respectively; or, the heavy chain variable region VH comprises HCDR1, HCDR2 and HCDR3 with amino acid sequences as set forth in SEQ ID NOs: 35, 78 and 147, respectively; or, the heavy chain variable region VH comprises HCDR1, HCDR2 and HCDR3 with amino acid sequences as set forth in SEQ ID NOs: 35, 90 and 147, respectively; or, the heavy chain variable region VH comprises HCDR1, HCDR2 and HCDR3 with amino acid sequences as set forth in SEQ ID NOs: 36, 90 and 146, respectively; or, the heavy chain variable region VH comprises HCDR1, HCDR2 and HCDR3 with amino acid sequences as set forth in SEQ ID NOs: 35, 76 and 147, respectively; or, the heavy chain variable region VH comprises HCDR1, HCDR2 and HCDR3 with amino acid sequences as set forth in SEQ ID NOs: 34, 78 and 148, respectively; or, the heavy chain variable region VH comprises HCDR1, HCDR2 and HCDR3 with amino acid sequences as set forth in SEQ ID NOs: 35, 76 and 136, respectively; or, the heavy chain variable region VH comprises HCDR1, HCDR2 and HCDR3 with amino acid sequences as set forth in SEQ ID NOs: 35, 90 and 146, respectively; or, the heavy chain variable region VH comprises HCDR1, HCDR2 and HCDR3 with amino acid sequences as set forth in SEQ ID NOs: 34, 76 and 136, respectively; or, the heavy chain variable region VH comprises HCDR1, HCDR2 and HCDR3 with amino acid sequences as set forth in SEQ ID NOs: 35, 76 and 146, respectively; or, the heavy chain variable region VH comprises HCDR1, HCDR2 and HCDR3 with amino acid sequences as set forth in SEQ ID NOs: 34, 76 and 146, respectively; or, the heavy chain variable region VH comprises HCDR1, HCDR2 and HCDR3 with amino acid sequences as set forth in SEQ ID NOs: 35, 90 and 148, respectively; or, the heavy chain variable region VH comprises HCDR1, HCDR2 and HCDR3 with amino acid sequences as set forth in SEQ ID NOs: 35, 90 and 136, respectively. The amino acid sequences of the listed CDRs are shown according to the Chothia scheme.

In some specific embodiments, the heavy chain variable region VH comprises an amino acid sequence as set forth in SEQ ID NO: 265; or, the heavy chain variable region VH comprises an amino acid sequence as set forth in SEQ ID NO: 266; or, the heavy chain variable region VH comprises an amino acid sequence as set forth in SEQ ID NO: 267; or, the heavy chain variable region VH comprises an amino acid sequence as set forth in SEQ ID NO: 268; or, the heavy chain variable region VH comprises an amino acid sequence as set forth in SEQ ID NO: 269; or, the heavy chain variable region VH comprises an amino acid sequence as set forth in SEQ ID NO: 270; or, the heavy chain variable region VH comprises an amino acid sequence as set forth in SEQ ID NO: 271; or, the heavy chain variable region VH comprises an amino acid sequence as set forth in SEQ ID NO: 272; or, the heavy chain variable region VH comprises an amino acid sequence as set forth in SEQ ID NO: 273; or, the heavy chain variable region VH comprises an amino acid sequence as set forth in SEQ ID NO: 274; or, the heavy chain variable region VH comprises an amino acid sequence as set forth in SEQ ID NO: 275; or, the heavy chain variable region VH comprises an amino acid sequence as set forth in SEQ ID NO: 276; or, the heavy chain variable region VH comprises an amino acid sequence as set forth in SEQ ID NO: 277; or, the heavy chain variable region VH comprises an amino acid sequence as set forth in SEQ ID NO: 278; or, the heavy chain variable region VH comprises an amino acid sequence as set forth in SEQ ID NO: 279; or, the heavy chain variable region VH comprises an amino acid sequence as set forth in SEQ ID NO: 280.

In some specific embodiments, the BCMA antibody comprises one polypeptide chain, wherein the polypeptide chain comprises an amino acid sequence as set forth in SEQ ID NO: 330; or, an amino acid sequence as set forth in SEQ ID NO: 331; or, an amino acid sequence as set forth in SEQ ID NO: 332; or, an amino acid sequence as set forth in SEQ ID NO: 333; or, an amino acid sequence as set forth in SEQ ID NO: 334; or, an amino acid sequence as set forth in SEQ ID NO: 335; or, an amino acid sequence as set forth in SEQ ID NO: 336; or, an amino acid sequence as set forth in SEQ ID NO: 337; or, an amino acid sequence as set forth in SEQ ID NO: 338; or, an amino acid sequence as set forth in SEQ ID NO: 339; or, an amino acid sequence as set forth in SEQ ID NO: 340; or, an amino acid sequence as set forth in SEQ ID NO: 341; or, an amino acid sequence as set forth in SEQ ID NO: 342; or, an amino acid sequence as set forth in SEQ ID NO: 343; or, an amino acid sequence as set forth in SEQ ID NO: 344; or, an amino acid sequence as set forth in SEQ ID NO: 345.

The beneficial effects of the present invention are as follows:

The present invention provides bivalent to multivalent and bispecific or multispecific binding proteins constructed using fully human heavy-chain antibodies and single-domain antibodies derived therefrom, as well as a method for preparing and using the binding proteins. Compared with the bivalent to multivalent and bispecific or multispecific binding proteins constructed using conventional IgG antibodies, such binding proteins have many advantages and is more flexible in terms of adjusting specificity and binding valence, so that multispecific binding proteins with smaller molecular weight, less polypeptide chains and simpler structure can be constructed, and different biological functions can be achieved through different structures.

In some specific embodiments, by regulating the functional activity of different targets through different structure types, relative positions, binding valence and other parameters, different activity combinations were designed to meet the requirements of dose combinations of combination drugs in the clinic. In some specific embodiments, the VH domains of heavy-chain antibodies can be conveniently connected in tandem to form multivalent structures, which can facilitate crosslinking of the targets by multivalent binding to the targets, and further enhance biological functions induced by crosslinking of the targets. In some specific embodiments, asymmetric tetravalent structures such as "1 + 3" may be achieved using the VH domains of heavy-chain antibodies, while similar structures are difficult to achieve using conventional IgG antibody-derived structures.

## BRIEF DESCRIPTION OF THE DRAWINGS

FIG. 1 shows the schematic diagrams of molecular structures.

FIG. 2 shows the binding activity of PD-L1 ×CTLA4 bispecific antibody molecules to an hCTLA4-His protein.

FIG. 3 shows the binding activity of PD-L1 ×CTLA4 bispecific antibody molecules to CHO-K1/hCTLA4 cells.

FIG. 4 shows the binding activity of PD-L1 ×CTLA4 bispecific antibody molecules to HEK293/hCTLA4 cells.

FIG. 5 shows the activity of PD-L1 ×CTLA4 bispecific antibody molecules blocking the binding of human CTLA4 proteins to its ligand protein B7-1.

FIG. 6 shows the activity of PD-L1 ×CTLA4 bispecific antibody molecules blocking the binding of CHO-K1/hCTLA4 cells to its ligand protein B7-1.

FIG. 7 shows the binding activity of PD-L1 ×CTLA4 bispecific antibody molecules to an hPDL1-His protein.

FIG. 8 shows the binding activity of PD-L1 ×CTLA4 bispecific antibody molecules to CHO-K1/hPDL1 cells.

FIG. 9 shows the binding activity of PD-L1 ×CTLA4 bispecific antibody molecules to MDA-MB-231 cells highly expressing human PD-L1.

FIG. 10 shows the activity of PD-L1 ×CTLA4 bispecific antibody molecules blocking the binding of CHO-K1/hPDL1 cells to its ligand protein PD-1.

FIG. 11 shows the killing ability of PD-L1 ×CTLA4 bispecific antibody molecules to CTLA4[+] target cells by ADCC effect.

FIG. 12 shows the T cell activation ability of PD-L1×CTLA4 bispecific antibodies of IgG-VH structures in an SEB stimulation assay.

FIG. 13 shows the T cell activation ability of PD-L1×CTLA4 bispecific antibodies of Fab-HCAb structures in an SEB stimulation assay.

FIG. 14 shows that in the SEB stimulation assay, when VH is at the N-terminus of IgG HC or LC, the PD-L1×CTLA4 bispecific antibody has comparable or even stronger T cell activation ability than the 1:1 dose combination of the corresponding monoclonal antibodies.

FIG. 15 shows that in the SEB stimulation assay, the CTLA4 end has weakened activity in IgG_HC-VH (VH at the C-terminus of IgG HC) and Fab-HCAb structures as compared to its presence in IgG or HCAb bivalent structures, so that the its dose-related toxicity can be reduced.

FIG. 16 shows that in the mixed lymphocyte reaction assay, when VH is at the N-terminus of IgG HC or LC, the PD-L1×CTLA4 bispecific antibody has stronger T cell activation ability than the 1:1 dose combination of the corresponding monoclonal antibodies.

FIG. 17 shows that in the mixed lymphocyte reaction assay, when VH is at the C-terminus of IgG HC or LC, the PD-L1×CTLA4 bispecific antibody has stronger T cell activation ability than the PD-L1 monoclonal antibody.

FIG. 18 shows the binding activity of HER2×CTLA4 bispecific antibody molecules to CHO-K1/hCTLA4 cells.

FIG. 19 shows the activity of HER2×CTLA4 bispecific antibody molecules blocking the binding of human CTLA4 proteins to its ligand protein B7-1.

FIG. 20 shows the activity of HER2×CTLA4 bispecific antibody molecules blocking the binding of CHO-K1/hCTLA4 cells to its ligand protein B7-1.

FIG. 21 shows the binding activity of HER2×CTLA4 bispecific antibody molecules to SK-BR-3 cells.

FIG. 22 shows the killing ability of HER2×CTLA4 bispecific antibody molecules to HER2[+] target cells by ADCC effect.

FIG. 23 shows the inhibition ability of HER2×CTLA4 bispecific antibody molecules to the proliferation of HER2[+] target cell SK-BR-3.

FIG. 24 shows the binding ability of HER2×CTLA4 bispecific antibody molecules to CTLA4[+] cells and HER2[+] cells simultaneously.

FIG. 25 shows the T cell activation ability of HER2×CTLA4 bispecific antibody molecules in the SEB stimulation assay.

FIG. 26 shows the pharmacokinetics of HER2×CTLA4 bispecific antibody molecules in mice.

FIG. 27 shows the binding activity of PD-L1×4-1BB bispecific antibody molecules to CHO-K1/hPDL1 cells.

FIG. 28 shows the binding activity of 4-1BB antibody or PD-L1 ×4-1BB bispecific antibody molecules to CHO-K1/hu 4-1BB cells.

FIG. 29 shows the binding activity of PD-L1 ×4-1BB bispecific antibody molecules to CHO-K1/cyno 4-1BB cells.

FIG. 30 shows the specific T cell activation mediated by PD-L1×4-1BB bispecific antibody molecules via CHO-K1/hPDL1 cells.

FIG. 31 shows the specific T cell activation mediated by PD-L1×4-1BB bispecific antibody molecules via MDA-MB-231 cells.

FIG. 32 shows that in the mixed lymphocyte reaction assay, PD-L1×4-1BB bispecific antibody molecules of IgG-VH structures have stronger T cell activation ability than the bispecific antibody molecules of FIT-Ig structures.

FIG. 33 shows that in the mixed lymphocyte reaction assay, PD-L1×4-1BB bispecific antibody molecules of IgG-VH structures and Fab-HCAb structures have stronger T cell activation ability than the bispecific antibody molecules of FIT-Ig structures.

FIG. 34 shows the pharmacokinetics of PD-L1 ×4-1BB bispecific antibody molecules in mice.

FIG. 35 shows the binding activity of B7H4×4-1BB bispecific antibody molecules to SK-BR-3 cells.

FIG. 36 shows the binding activity of B7H4×4-1BB bispecific antibody molecules to CHO-K1/hu 4-1BB cells.

FIG. 37 shows the specific T cell activation (INFγ release) mediated by B7H4×4-1BB bispecific antibody molecules via SK-BR-3.

FIG. 38 shows the specific T cell activation (IL-2 release) mediated by B7H4×4-1BB bispecific antibody molecules via SK-BR-3.

FIG. 39 shows that the T cell activation by B7H4×4-1BB bispecific antibody molecules is specifically dependent on the expression of B7H4.

FIG. 40 shows the serum stability of B7H4×4-1BB bispecific antibody molecule PR003334.

FIG. 41 shows the serum stability of B7H4×4-1BB bispecific antibody molecule PR003335.

FIG. 42 shows the pharmacokinetics of B7H4×4-1BB bispecific antibody molecules in mice.

FIG. 43 shows the anti-tumor effect of B7H4×4-1BB bispecific antibody molecules in a mouse tumor model.

FIG. 44 shows the binding activity of BCMA HCAb antibodies to HEK293T/hBCMA cells.

FIG. 45 shows the binding activity of BCMA HCAb antibodies to HEK293T/cyno BCMA cells.

FIG. 46 shows the binding activity of BCMA HCAb antibodies to NCI-H929 cells.

FIG. 47 shows the internalization ability of BCMA HCAb antibodies to HEK293T/hBCMA cells.

FIG. 48 shows the affinity of BCMA HCAb antibodies to human BCMA (BLI method).

FIG. 49 shows the binding activity of variant molecules derived from the BCMA HCAb antibody PR001046 to NCI-H929 cells.

FIG. 50 shows the binding activity of BCMA×CD3 bispecific antibody molecules to HEK293T/hBCMA cells.

FIG. 51 shows the binding activity of BCMA×CD3 bispecific antibody molecules to HEK293T/cyno BCMA cells.

FIG. 52 shows the binding activity of BCMA×CD3 bispecific antibody molecules to NCI-H929 cells.

FIG. 53 shows the binding activity of BCMA×CD3 bispecific antibody molecules to human T cells.

FIG. 54 shows the binding activity of BCMA×CD3 bispecific antibody molecules to cynomolgus monkey T cells.

FIG. 55 shows the killing ability of BCMA×CD3 bispecific antibody molecules of scFv-Fc-VH (2) asymmetric structures to NCI-H929 cells.

FIG. 56 shows the killing ability of BCMA×CD3 bispecific antibody molecules of scFv-Fc-VH (1) asymmetric structures to NCI-H929 cells.

FIG. 57 shows the killing ability of BCMA×CD3 bispecific antibody molecules of Fab-Fc-VH (1) asymmetric structures to NCI-H929 cells.

FIG. 58 shows the killing ability of BCMA×CD3 bispecific antibody molecules of Fab-Fc-VH (2) asymmetric structures to NCI-H929 cells.

FIG. 59 shows the specific killing of effector cells on NCI-H929 cells mediated by BCMA×CD3 bispecific antibody molecules (PR001990 and PR002309) and determination of release of cytokines.

FIG. 60 shows the specific killing of effector cells on NCI-H929 cells mediated by BCMA×CD3 bispecific antibody molecules (PR002895, PR002953, PR003178) and determination of release of cytokines.

FIG. 61 shows the influence of soluble APRIL or BAFF on the target cell killing effect of BCMA×CD3 bispecific antibody molecules.

FIG. 62 shows the influence of soluble BCMA on the target cell killing effect of BCMA×CD3 bispecific antibody molecules.

FIG. 63 shows the pharmacokinetics of BCMA×CD3 bispecific antibody molecules in mice or rats.

FIG. 64 shows the anti-tumor effect of BCMA×CD3 bispecific antibody molecules in a mouse tumor model.

FIG. 65 shows the analysis results of SDS-PAGE and SEC-HPLC of protein samples obtained by transient transfection and expression and one-step affinity purification of the bispecific antibody molecules.

FIG. 66 shows the binding activity of CD3 antibodies to human T cells.

FIG. 67 shows the anti-tumor effect of PD-L1 ×CTLA4 bispecific antibody molecules in a mouse tumor model.

## DETAILED DESCRIPTION

The embodiments of the present invention are described below with reference to specific examples, and other advantages and effects of the present invention will be readily apparent to those skilled in the art from the disclosure of the present specification.

In the present application, the term "antibody" or "H2L2" generally refers to a conventional four-chain antibody. Antibodies of most species exhibit a "Y"-type tetrameric structure comprising two identical heavy chains (H chains) and two identical light chains (L chains), which are also referred to as "H2L2". The heavy chain comprises a heavy chain variable region (VH) near the N-terminus and a heavy chain constant region (CH) near the C-terminus; and the light chain comprises a light chain variable region (VL) near the N-terminus and a light chain constant region (CL) near the C-terminus. The heavy chain constant region of IgG antibody has 3 domains, namely CH1, CH2 and CH3; and has a hinge region between CH1 and CH2. According to the types $\kappa$ and $\lambda$ of the light chains, the light chain variable regions are further divided into V$\kappa$ and V$\lambda$, and the corresponding light chain constant regions are C$\kappa$ and C$\lambda$, respectively. The variable region of the antibody is the primary site where it recognizes and binds to the antigen; and the variable domains VH and VL and the constant domains CH1 and CL of the antibody together constitute the antigen-binding fragment (Fab). The CH2 and CH3 constitute the fragment crystallizable (Fc), which is the main region that exerts the effector functions of the antibody such as complement-dependent cytotoxicity (CDC) and antibody-dependent cell-mediated cytotoxicity (ADCC) effects, and affects the serum half-life of the antibody.

In the present application, the term "heavy-chain antibody" or "HCAb" generally refers to a class of antibodies that contain only a heavy chain dimer. A heavy-chain antibody (HCAb) lacking light chains is naturally present in the sera of Camelidae species and sharks. The heavy-chain antibody derived from Camelidae species has no CH1 region between the heavy chain variable region and the hinge region thereof, and contains only one heavy chain variable region (VHH) and two heavy chain constant domains (CH2 and CH3), in addition to the lack of light chains, as compared to the conventional H2L2 antibodies; and the basic structure thereof is a heavy chain dimer. The VHH fragment of the heavy-chain antibody of Camelidae species has different characteristics from the VH of the conventional antibodies, and the VHH structure cloned and expressed separately has structural stability and antigen-binding activity that are comparable to the original heavy-chain antibody, but has a molecular weight of only about 13 kDa, and is therefore also referred to as a nanobody or a single-domain antibody.

In the present application, the term "binding protein" or "antigen-binding protein" generally refers to a protein comprising an antigen-binding moiety, and optionally a scaffold or framework moiety that allows the antigen-binding moiety to adopt a conformation that facilitates the binding of the antigen-binding protein to the antigen. An antibody may typically comprise an antibody light chain variable region (VL) or an antibody heavy chain variable region (VH), or both. The VH and VL regions can be further divided into hypervariable regions termed complementarity determining regions (CDRs), which are scattered over more conserved regions termed framework regions (FRs). Each VH and VL can consist of three CDR regions and four FR regions arranged from amino-terminus to carboxyl-terminus in the following order: FR-1, CDR1, FR-2, CDR2, FR-3, CDR3 and FR-4. The variable regions of the heavy and light chains comprise binding domains that interact with antigens. The three CDRs of VH are denoted as HCDR1, HCDR2 and HCDR3, respectively, and may also be denoted as VH CDR1, VH CDR2 and VH CDR3, respectively; and the three CDRs of VL are denoted as LCDR1, LCDR2 and LCDR3, respectively, and may also be denoted as VL CDR1, VL CDR2 and VL CDR3, respectively. Examples of the antigen-binding proteins include, but are not limited to, antibodies, antigen-binding fragments (Fab, Fab', $F(ab)_2$, Fv fragment, $F(ab')_2$, scFv, di-scFv and/or dAb), immunoconjugates, multispecific antibodies (e.g., bispecific antibodies), antibody fragments, antibody derivatives, antibody analogs or fusion proteins, as long as they exhibit the desired antigen-binding activity.

In the present application, the amino acid sequences of the CDRs are shown according to the Chothia scheme. However, it is well known to those skilled in the art that the CDRs of an antibody can be defined in the art using a variety of methods, such as the Kabat scheme based on sequence variability (see Kabat et al., Sequences of Proteins of Immunological Interest, Fifth Edition, National Institutes of Health (U.S.), Bethesda, Maryland (1991)), and the Chothia scheme based on the location of the structural loop regions (see J Mol Biol 273: 927-48, 1997). In the technical solution of the present invention, the Combined scheme comprising the Kabat scheme and the Chothia scheme can also be used to determine the amino acid residues in a variable domain sequence. The Combined scheme combines the Kabat scheme with the Chothia scheme to obtain a larger range. See the table below for details. It will be understood by those skilled in the art that unless otherwise specified, the terms "CDR" and "complementary determining region" of a given antibody or a region (e.g., variable region) thereof are construed as encompassing complementary determining regions as defined by any one of the above known schemes described herein. Although the scope claimed in the present invention is the sequences shown based on the Chothia scheme, the amino acid sequences corresponding to the other schemes for numbering CDRs shall also fall within the scope of the present invention.

Table-I. The schemes for numbering the CDRs of the antibody of the present invention

|  | Kabat | Chothia | Combined |
| --- | --- | --- | --- |
| LCDR1 | L24--L34 | L24--L34 | L24-L34 |
| LCDR2 | L50--L56 | L50--L56 | L50-L56 |
| LCDR3 | L89--L97 | L89--L97 | L89-L97 |
| HCDR1 | H31--H35 | H26--H32 | H26-H35 |
| HCDR2 | H50--H65 | H52--H56 | H50-H65 |
| HCDR3 | H95--H102 | H95--H102 | H95-H102 |

Laa-Lbb can refer to an amino acid sequence from position aa (the Chothia scheme) to position bb (the Chothia scheme) beginning at the N-terminus of the light chain of the antibody; and Haa-Hbb can refer to an amino acid sequence from position aa (the Chothia scheme) to position bb (the Chothia scheme) beginning at the N-terminus of the heavy chain of the antibody. For example, L24-L34 can refer to the amino acid sequence from position 24 to position 34 according to the Chothia scheme beginning at the N-terminus of the light chain of the antibody; H26-H32 can refer to the amino acid sequence from position 26 to position 32 according to the Chothia scheme beginning at the N-terminus of the heavy chain of the antibody. It should be known to those skilled in the art that there are positions where insertion sites are present in numbering CDRs with the Chothia scheme (see http://bioinf.org.uk/abs/).

In the present application, the term "monoclonal antibody" generally refers to an antibody obtained from a population of substantially homogeneous antibodies, that is, the individual antibodies in the population are identical except for a small amount of natural mutations that may exist. Monoclonal antibodies are generally highly specific for a single antigenic site. Moreover, unlike conventional polyclonal antibody preparations (which generally have different antibodies directed against different determinants), each monoclonal antibody is directed against a single determinant on the antigen. In addition to their specificity, monoclonal antibodies have the advantage that they can be synthesized by hybridoma culture without contamination by other immunoglobulins. The modifier "monoclonal" indicates the characteristic of the antibody obtained from a population of substantially homogeneous antibodies, and is not to be construed as requiring production of the antibody by any particular method. For example, monoclonal antibodies used according to the present invention can be prepared in hybridoma cells or can be prepared by the recombinant DNA method.

In this application, the term "fully human antibody" generally refers to an antibody that is expressed by a genetically engineered antibody gene-deleted animal into which the entire gene that encodes an antibody in human is transferred. All parts of the antibody (including the variable and constant regions of the antibody) are encoded by genes of human origin. The fully human antibody can greatly reduce the immune side effects caused in the human body by the heterologous antibody. Methods for obtaining fully human antibodies in the art can include phage display, transgenic mice, and the like.

In the present application, the term "fully human heavy-chain antibody" generally refers to a heavy-chain antibody having a human antibody variable region VH obtained using Harbour HCAb transgenic mice (Patent Application WO2007/096779). The endogenous antibody heavy chain locus and light chain locus of the transgenic mice are knocked out or inactivated, making it impossible to produce mouse antibodies; then, the human antibody heavy chain gene fragments (V, D, J fragments) are transferred into the mice, and antibodies with human antibody gene sequences are generated by the rearrangement and mutation mechanism of the mice, the variable regions of which are human VHs. The human VH and the human heavy chain constant region Fc are fused and recombined to obtain the fully human heavy-chain antibody.

In the present application, the term "specifically bind to" generally refers to that an antibody binds to an epitope via its antigen-binding domain, and that the binding requires some complementarity between the antigen-binding domain and the epitope. According to this definition, an antibody is said to "specifically bind to" an antigen when the antibody more easily binds to an epitope via its antigen-binding domain than binds to a random, unrelated epitope. "Epitope" refers to a specific atomic group (e.g., saccharide side chain, phosphoryl, sulfonyl) or an amino acid on an antigen that binds to an antigen-binding protein (e.g., an antibody).

In the present application, the term "Fab" generally refers to the portion of a conventional antibody (e.g., IgG) that binds to an antigen, including the heavy chain variable region VH, the light chain variable region VL, the heavy chain constant region domain CH1 and the light chain constant region CL of the antibody. In conventional antibodies, the C-terminus of VH is linked to the N-terminus of CH1 to form a heavy chain Fd fragment, the C-terminus of VL is linked to the N-terminus of CL to form a light chain, and the C-terminus of CH1 is further linked to the hinge region and other constant region domains of the heavy chain to form a heavy chain. In some embodiments, "Fab" also refers to a variant structure of the Fab. For example, in certain embodiments, the C-terminus of VH is linked to the N-terminus of CL to form one polypeptide chain, and the C-terminus of VL is linked to the N-terminus of CH1 to form another polypeptide chain, in which case an Fab (cross VH/VL) structure is formed; in certain embodiments, CH1 of the Fab is not linked to the hinge region, but rather the C-terminus of CL is linked to the hinge region of the heavy chain, in which case an Fab (cross Fd/LC) structure is formed.

In the present application, the term "VH" generally refers to the heavy chain variable region VH domain of an antibody, i.e., the heavy chain variable region VH of a conventional antibody (H2L2 structure) from human or other animals, the heavy chain variable region VHH of a heavy-chain antibody (HCAb structure) from animals such as those of Camelidae species, or the heavy chain variable region VH of a fully human heavy-chain antibody (HCAb structure) produced using a Harbour HCAb transgenic mouse.

In the present application, the term "binding domain" generally refers to any protein functional region that can specifically bind to the antigen, either "Fab" or "VH", or other antigen-binding forms (e.g., derived protein structures such as lipocalins, neuronal cell adhesion molecules (NCAMs), fibronectins, designed ankyrin repeat proteins (DARPins) and the like).

In the present application, the term "binding valence" generally refers to the number of "binding domains" in a binding protein, and also refers to the maximum number of antigen molecules or epitopes to which the binding protein can bind. For example, a conventional IgG antibody has a binding valence of two because it is capable of binding to two identical antigen molecules simultaneously; whereas a Fab antibody has a binding valence of one.

In the present application, the term "bispecific binding protein" generally refers to a binding protein having two antigen-binding specificities. The two antigen-binding specificities may refer to binding to two different antigens or to two different epitopes on the same antigen. Bispecific binding proteins may typically include bispecific antibodies, derivatives thereof and the like.

In the present application, the term "multispecific binding protein" generally refers to a binding protein having two or more antigen-binding specificities. Multispecific binding proteins may typically include multispecific antibodies, derivatives thereof and the like.

In the present application, the term "MFI" (mean fluorescence intensity) generally refers to the fluorescence intensity signal and its mathematical mean value analyzed in flow cytometry FACS, which can generally be obtained by processing and analyzing data generated by flow cytometer with specialized software such as FlowJo (FlowJo, LLC).

In the present application, the term "PD-L1" generally refers to the programmed death ligand 1 protein, a functional variant thereof and/or a functional fragment thereof. PD-L1 is also known as cluster of differentiation 274 (CD274) or B7 homologue 1 (B7-H1) and is a protein encoded by the CD274 gene (in human). The sequence of PD-L1 is known in the art. For example, the amino acid sequence of an exemplary full-length human PD-L1 protein can be found under NCBI accession No. NP_054862 or UniProt accession No. Q9NZQ7; and the sequence of an exemplary full-length cynomolgus monkey PD-L1 protein can be found under NCBI accession No. XP_005581836 or Uniprot accession No. G7PSE7.

In the present application, the term "PD-1" generally refers to the programmed death 1 receptor (also known as CD279), a functional variant thereof and/or a functional fragment thereof. The sequence of PD-1 is known in the art. For example, the sequence of an exemplary full-length human PD-1 protein can be found under NCBI accession No. NP_005009; and the sequence of an exemplary full-length cynomolgus monkey PD-1 protein can be found under NCBI accession No. NP_001271065 or Uniprot accession No. B0LAJ3.

In the present application, the term "CD80" generally refers to the cluster of differentiation 80 (also known as B7-1), a functional variant thereof and/or a functional fragment thereof. The sequence of CD80 is known in the art. For example, the sequence of an exemplary full-length human CD80 can be found under Uniprot accession No. P33681.

In the present application, the term "CTLA4" generally refers to the cytotoxic T lymphocyte-associated antigen-4 (also known as CD152), a functional variant thereof and/or a functional fragment thereof. The sequence of CTLA4 is known in the art. For example, the sequence of an exemplary full-length human CTLA4 can be found under Uniprot accession No. P16410; and the sequence of an exemplary full-length cynomolgus monkey CTLA4 can be found under Uniprot accession No. G7PL88.

In the present application, the term "HER2" generally refers to the receptor tyrosine kinase erbB-2 (also known as ERBB2), a functional variant thereof and/or a functional fragment thereof. The sequence of HER2 is known in the art. For example, the sequence of an exemplary full-length human HER2 can be found under Uniprot accession No. P04626; and the sequence of an exemplary full-length cynomolgus monkey HER2 can be found under NCBI accession No. XP_005584091.

In the present application, the term "B7H4" generally refers to the V-Set domain-containing T-cell activation inhibitor 1 (also known as VTCN1), a functional variant thereof and/or a functional fragment thereof. The sequence of B7H4 is known in the art. For example, the sequence of an exemplary full-length human B7H4 can be found under Uniprot accession No. Q7Z7D3; the sequence of an exemplary full-length cynomolgus monkey B7H4 can be found under NCBI accession No. XP_005542249; and the sequence of an exemplary full-length mouse B7H4 can be found under Uniprot accession No. Q7TSP5.

In the present application, the term "4-1BB" generally refers to the tumor necrosis factor receptor superfamily member 9 (also known as CD137, 4-1BBL receptor), a functional variant thereof and/or a functional fragment thereof. The sequence of 4-1BB is known in the art. For example, the sequence of an exemplary full-length human 4-1BB can be found under Uniprot accession No. Q07011; and the sequence of an exemplary full-length cynomolgus monkey 4-1BB can be found under NCBI accession No. XP_005544945.

In the present application, the term "BCMA" generally refers to the tumor necrosis factor receptor superfamily member 17 (also known as CD269, B cell maturation protein), a functional variant thereof and/or a functional fragment thereof. The sequence of BCMA is known in the art. For example, the sequence of an exemplary full-length human BCMA can be found under Uniprot accession No. Q02223; and the sequence of an exemplary full-length cynomolgus monkey BCMA can be found under NCBI accession No. XP_005591343.

In the present application, the term "BAFF" generally refers to the tumor necrosis factor ligand superfamily member 13B (also known as CD257, B cell activating factor), a functional variant thereof and/or a functional fragment thereof. The sequence of BAFF is known in the art. For example, the sequence of an exemplary full-length human BAFF can be found under Uniprot accession No. Q9Y275.

In the present application, the term "APRIL" generally refers to the tumor necrosis factor ligand superfamily member 13 (also known as CD256, proliferation-inducing ligand), a functional variant thereof and/or a functional fragment thereof. The sequence of APRIL is known in the art. For example, the sequence of an exemplary full-length human APRIL can be found under Uniprot accession No. O75888.

In the present application, the term "CD3" generally refers to the TCR/CD3 receptor protein complex on T cells. The specificity of the T cell response is mediated by the recognition of pMHC by the molecular complex of TCR and CD3. TCR is a heterodimer formed by two different transmembrane polypeptide chains, and the peptide chains comprise four types: $\alpha$, $\beta$, $\gamma$ and $\delta$. According to the different combinations of the peptide chains, TCR is divided into TCR$\alpha\beta$ and TCR$\gamma\delta$. CD3 has different transmembrane polypeptide chains, i.e., $\gamma$, $\delta$, $\varepsilon$ and $\zeta$, which interact to form homodimers or heterodimers as part of the TCR-CD3 complex. Since the cytoplasmic region of the TCR peptide chain is very short, it is believed that the activation signal generated by the recognition of antigen by TCR is transduced into T cells by the CD3 peptide chain.

In the present application, the term "CD3E" generally refers to the $\varepsilon$ peptide chain of "CD3". The sequence of CD3E is known in the art. For example, the sequence of an exemplary full-length human CD3E can be found under Uniprot accession No. P07766; and the sequence of an exemplary full-length cynomolgus monkey CD3E can be found under Uniprot accession No. Q95LI5.

**Examples**

The present invention is further illustrated by the following examples, which are not intended to limit the present invention. The examples do not include detailed descriptions of conventional methods, such as those methods for constructing vectors and plasmids, methods for inserting genes encoding proteins into such vectors and plasmids, or methods for introducing plasmids into host cells. Such methods are well known to those of ordinary skill in the art and are described in numerous publications. Experimental procedures without specified conditions in the following examples are performed in accordance with conventional procedures and conditions, or in accordance with instructions.

**Example 1. Structural Design of Multispecific Binding Proteins based on HCAb**

In this example, the structures of several Fc-containing, symmetric or asymmetric, multivalent and multispecific binding proteins constructed using fully human heavy-chain antibodies (HCAbs) and single-domain antibodies (sdAbs) derived therefrom were listed. In some structures, the domains are linked via linker peptides. In some structures, amino acid mutations were introduced into the Fc region of the heavy chain to alter its binding to Fc receptors, thereby altering the associated effector functions or other properties. In some structures, different amino acid mutations were introduced into the Fc regions of the two heavy chains to reduce the homodimerization of heavy chains.

The molecular structures of the multispecific binding proteins included in the present application are listed in Table 1-1 and FIG. 1, each of which will be further described below. In this example and other parts of the present application, when referring to the number of polypeptide chains contained in a molecular structure, it generally refers to the number of "different polypeptide chains". For example, a conventional IgG antibody has two different polypeptide chains, i.e., a heavy chain and a light chain, and although the IgG antibody molecule itself is a tetrapeptide chain protein molecule containing two identical heavy chains and two identical light chains, its structural characteristics are described with specific reference to its two different polypeptide chains. Binding valence refers to the number of antigen-binding sites in the molecular structure. For example, a conventional IgG antibody can bind to two identical antigen molecules simultaneously, with a binding valence of two. The sequences of the linker peptides that may be used in the structural design of the present application are listed in Table 1-2.

**Table 1-1. Molecular structures of multispecific binding proteins based on HCAb listed in the present application**

| Structure No. | Structure mode | Structure type | Binding valence | Symmetry | Number of different polypeptide chains |
|---|---|---|---|---|---|
| 1 | Fab(CL)-VH-Fc | Fab-HCAb | Tetravalent | Symmetric | 2 |
| 2 | Fab(CH1)-VH-Fc | Fab-HCAb | Tetravalent | Symmetric | 2 |
| 3 | VH-IgG_HC | IgG-VH | Tetravalent | Symmetric | 2 |
| 4 | VH-IgG_LC | IgG-VH | Tetravalent | Symmetric | 2 |
| 5 | IgG_HC-VH | IgG-VH | Tetravalent | Symmetric | 2 |
| 6 | IgG_LC-VH | IgG-VH | Tetravalent | Symmetric | 2 |
| 7 | IgG_HC-VH-VH | IgG-VH(2) | Hexavalent | Symmetric | 2 |
| 8 | IgG_HC-VH'-VH" | IgG-VH(2) | Hexavalent | Symmetric | 2 |
| 9 | 2xVH-IgG(HC+LC) | 2xVH-IgG | Hexavalent | Symmetric | 2 |
| 10 | (VH'+VH")-IgG(HC+LC) | 2xVH-IgG | Hexavalent | Symmetric | 2 |
| 11 | Fab-Fc-VH | Fab-Fc-VH | Bivalent | Asymmetric | 3 |
| 12 | Fab(cross VH/VL)-Fc-VH | Fab-Fc-VH | Bivalent | Asymmetric | 3 |
| 13 | Fab(cross Fd/LC)-Fc-VH | Fab-Fc-VH | Bivalent | Asymmetric | 3 |
| 14 | Fab-Fc-VH-VH | Fab-Fc-VH(2) | Trivalent | Asymmetric | 3 |

(continued)

| Structure No. | Structure mode | Structure type | Binding valence | Symmetry | Number of different polypeptide chains |
|---|---|---|---|---|---|
| 15 | Fab-Fc-VH' -VH" | Fab-Fc-VH(2) | Trivalent | Asymmetric | 3 |
| 16 | Fab(cross VH/VL)-Fc-VH-VH | Fab-Fc-VH(2) | Trivalent | Asymmetric | 3 |
| 17 | Fab(cross VH/VL)-Fc-VH'-VH" | Fab-Fc-VH(2) | Trivalent | Asymmetric | 3 |
| 18 | Fab(cross Fd/LC)-Fc-VH-VH | Fab-Fc-VH(2) | Trivalent | Asymmetric | 3 |
| 19 | Fab(cross Fd/LC)-Fc-VH'-VH" | Fab-Fc-VH(2) | Trivalent | Asymmetric | 3 |
| 20 | scFv(VL-VH)-Fc-VH | scFv-Fc-VH | Bivalent | Asymmetric | 2 |
| 21 | scFv(VH-VL)-Fc-VH | scFv-Fc-VH | Bivalent | Asymmetric | 2 |
| 22 | scFv(VL-VH)-Fc-VH-VH | scFv-Fc-VH(2) | Trivalent | Asymmetric | 2 |
| 23 | scFv(VH-VL)-Fc-VH-VH | scFv-Fc-VH(2) | Trivalent | Asymmetric | 2 |
| 24 | scFv(VL-VH)-Fc-VH'-VH" | scFv-Fc-VH(2) | Trivalent | Asymmetric | 2 |
| 25 | scFv(VH-VL)-Fc-VH'-VH" | scFv-Fc-VH(2) | Trivalent | Asymmetric | 2 |
| 26 | Fab-Fc-VH-VH-VH | Fab-Fc-VH(3) | Tetravalent | Asymmetric | 3 |
| 27 | Fab-Fc-VH'-VH"-VH‴ | Fab-Fc-VH(3) | Tetravalent | Asymmetric | 3 |

**Table 1-2. Sequences of linker peptides**

| Name of linker peptide | Length | Sequence of linker peptide | SEQ ID NO |
|---|---|---|---|
| GS_2 | 2 | GS | None |
| GS_4 | 4 | GSGS | 495 |
| GS_5 | 5 | GGGGS | 496 |
| GS_6 | 6 | GGSGGS | 497 |
| GS_7 | 7 | GGGGSGS | 498 |
| GS_15 | 15 | GGGGSGGGGSGGGGS | 499 |
| GS_20 | 20 | GGGGSGGGGSGGGGSGGGGS | 500 |
| GS_25 | 25 | GGGGSGGGGSGGGGSGGGGSGGGGS | 501 |
| H1_15 | 15 | EPKSSDKTHTPPPPP | 502 |
| UH1 | 6 | EPKSSD | 503 |
| LH1 | 10 | DKTHTCPPCP | 504 |
| G5-LH | 15 | GGGGGDKTHTCPPCP | 505 |
| H1_15-RT | 17 | EPKSSDKTHTPPPPPRT | 506 |
| AS-GS_15 | 17 | ASGGGGSGGGGSGGGGS | 507 |
| L-GS_15-RT | 18 | LGGGGSGGGGSGGGGSRT | 508 |
| L-H1_15-RT | 18 | LEPKSSDKTHTPPPPPRT | 509 |
| KL-H1_15-RT | 19 | KLEPKSSDKTHTPPPPPRT | 510 |
| KL-H1_15-AS | 19 | KLEPKSSDKTHTPPPPPAS | 511 |
| RT-GS_5-KL | 9 | RTGGGGSKL | 512 |
| RT-GS_15-KL | 19 | RTGGGGSGGGGSGGGGSKL | 513 |
| RT-GS_25-KL | 29 | RTGGGGSGGGGSGGGGSGGGGSGGGGSKL | 514 |

(continued)

| Name of linker peptide | Length | Sequence of linker peptide | SEQ ID NO |
|---|---|---|---|
| Human IgG1 hinge | 15 | EPKSCDKTHTCPPCP | 515 |
| Human IgG1 hinge (C220S) | 15 | EPKSSDKTHTCPPCP | 516 |
| Human IgG2 hinge | 12 | ERKCCVECPPCP | 517 |
| Human IgG4 hinge | 12 | ESKYGPPCPSCP | 518 |
| Human IgG4 hinge (S228P) | 12 | ESKYGPPCPPCP | 519 |

**Example 1.1. Fab-HCAb symmetric structure**

The present invention provides a method for constructing a bispecific binding protein using two parent monoclonal antibodies: a conventional antibody A binding to a first antigen and a heavy-chain antibody B binding to a second antigen.

As shown in FIG. 1 (1-2), the Fab end is derived from the conventional antibody A, and VH_A and VL_A are the heavy chain variable region and the light chain variable region of the antibody A, respectively. The VH end is derived from the heavy-chain antibody B, and VH_B is the heavy chain variable region of the heavy-chain antibody B. CL is the light chain constant region domain. CH1, CH2 and CH3 are the first, second and third domains of the heavy chain constant region, respectively. L1 and L2 are the first and second linker peptides, respectively.

**Example 1.1.1.** Structure (1): Fab(CL)-VH-Fc

The binding protein of structure (1) comprises two different polypeptide chains: a polypeptide chain 1 or a first polypeptide chain, also known as a short chain, comprising VH_A-CH1 from the amino-terminus to the carboxyl-terminus; and a polypeptide chain 2 or a second polypeptide chain, also known as a long chain, comprising VL_A-CL-L1-VH_B-L2-CH2-CH3 from the amino-terminus to the carboxyl-terminus. In structure (1), VL_A of the antibody A and VH_B of the heavy-chain antibody B are fused on the same polypeptide chain, so that the mismatched byproducts generated by the association of VL_A and VH_B can be avoided.

VH_B of the polypeptide chain 2 is linked to CH2 via a linker peptide L2; L2 may be a hinge region or a hinge region-derived linker peptide sequence of IgG or the sequence listed in Table 1-2, preferably the sequence of human IgG1 hinge region, human IgG1 hinge (C220S) or G5-LH.

In one embodiment, CL of the polypeptide chain 2 is fusion-linked directly to VH_B, i.e., L1 is 0 in length. In another embodiment, CL of the polypeptide chain 2 is linked to VH_B via a linker peptide L1; and L1 may be the sequence listed in Table 1-2.

**Example 1.1.2. Structure (2): Fab(CH1)-VH-Fc**

The binding protein of structure (2) comprises two different polypeptide chains: a polypeptide chain 1 or a first polypeptide chain, also known as a short chain, comprising VL_A-CL from the amino-terminus to the carboxyl-terminus; and a polypeptide chain 2 or a second polypeptide chain, also known as a long chain, comprising VH_A-CH1-L1-VH_B-L2-CH2-CH3 from the amino-terminus to the carboxyl-terminus.

VH_B of the polypeptide chain 2 is linked to CH2 via a linker peptide L2; L2 may be a hinge region or a hinge region-derived linker peptide sequence of IgG or the sequence listed in Table 1-2, preferably the sequence of human IgG1 hinge region, human IgG1 hinge (C220S) or G5-LH.

In one embodiment, CH1 of the polypeptide chain 2 is fusion-linked directly to VH_B, i.e., L1 is 0 in length. In another embodiment, CH1 of the polypeptide chain 2 is linked to VH_B via a linker peptide L1; and L1 may be the sequence listed in Table 1-2.

**Example 1.2. IgG-VH tetravalent symmetric structure**

The present invention provides a method for constructing a bispecific binding protein using two parent monoclonal antibodies: a conventional antibody A binding to a first antigen and a heavy-chain antibody B binding to a second antigen.

As shown in FIG. 1 (3-6), the Fab end is derived from the conventional antibody A, and VH_A and VL_A are the heavy chain variable region and the light chain variable region of the antibody A, respectively. The VH end is derived from the heavy-chain antibody B, and VH_B is the heavy chain variable region of the heavy-chain antibody B. CL is the light chain constant region domain. CH1, CH2 and CH3 are the first, second and third domains of the heavy chain constant region, respectively. L is a linker peptide, and h is a hinge region or derived sequence of an IgG antibody.

**Example 1.2.1. Structure (3): VH-IgG_HC**

The binding protein of structure (3) comprises two different polypeptide chains: a polypeptide chain 1 or a first polypeptide chain, also known as a short chain, comprising VL_A-CL from the amino-terminus to the carboxyl-terminus; and a polypeptide chain 2 or a second polypeptide chain, also known as a long chain, comprising VH_B-L-VH_A-CH1-h-CH2-CH3 from the amino-terminus to the carboxyl-terminus.

In one embodiment, VH_B of the polypeptide chain 2 is fusion-linked directly to VH_A, i.e., L is 0 in length. In another embodiment, VH_B of the polypeptide chain 2 is linked to VH_A via a linker peptide L; and L may be the sequence listed in Table 1-2.

**Example 1.2.2. Structure (4): VH-IgG_LC**

The binding protein of structure (4) comprises two different polypeptide chains: a polypeptide chain 1 or a first polypeptide chain, also known as a short chain, comprising VH_B-L-VL_A-CL from the amino-terminus to the carboxyl-terminus; and a polypeptide chain 2 or a second polypeptide chain, also known as a long chain, comprising VH_A-CH1-h-CH2-CH3 from the amino-terminus to the carboxyl-terminus.

In one embodiment, VH_B of the polypeptide chain 1 is fusion-linked directly to VL_A, i.e., L is 0 in length. In another embodiment, VH_B of the polypeptide chain 1 is linked to VL_A via linker peptide L; and L may be the sequence listed in Table 1-2.

**Example 1.2.3. Structure (5): IgG_HC-VH**

The binding protein of structure (5) comprises two different polypeptide chains: a polypeptide chain 1 or a first polypeptide chain, also known as a short chain, comprising VL_A-CL from the amino-terminus to the carboxyl-terminus; and a polypeptide chain 2 or a second polypeptide chain, also known as a long chain, comprising VH_A-CH1-h-CH2-CH3-L-VH_B from the amino-terminus to the carboxyl-terminus.

In one embodiment, CH3 of the polypeptide chain 2 is fusion-linked directly to VH_B, i.e., L is 0 in length. In another embodiment, CH3 of the polypeptide chain 2 is linked to VH_B via a linker peptide L; and L may be the sequence listed in Table 1-2.

**Example 1.2.4. Structure (6): IgG_LC-VH**

The binding protein of structure (6) comprises two different polypeptide chains: a polypeptide chain 1 or a first polypeptide chain, also known as a short chain, comprising VL_A-CL-L-VH_B from the amino-terminus to the carboxyl-terminus; and a polypeptide chain 2 or a second polypeptide chain, also known as a long chain, comprising VH_A-CH1-h-CH2-CH3 from the amino-terminus to the carboxyl-terminus.

In one embodiment, CL of the polypeptide chain 1 is fusion-linked directly to VH_B, i.e., L is 0 in length. In another embodiment, CL of the polypeptide chain 1 is linked to VH_B via a linker peptide L; and L may be the sequence listed in Table 1-2.

**Example 1.3. IgG-VH(2) hexavalent symmetric structure**

The present invention provides a method for constructing a bispecific binding protein using two parent monoclonal antibodies: a conventional antibody A binding to a first antigen and a heavy-chain antibody B binding to a second antigen.

The present invention further provides a method for constructing a trispecific binding protein using three parent monoclonal antibodies: a conventional antibody A binding to a first antigen, a heavy-chain antibody B binding to a second antigen, and a heavy-chain antibody C binding to a third antigen.

As shown in FIG. 1 (7-8), the Fab end is derived from the conventional antibody A, and VH_A and VL_A are the heavy chain variable region and the light chain variable region of the antibody A, respectively. The VH end is derived from the heavy-chain antibody B or the heavy-chain antibody C, VH_B is the heavy chain variable region of the heavy-chain antibody B, and VH_C is the heavy chain variable region of the heavy-chain antibody C. CL is the light chain constant region domain. CH1, CH2 and CH3 are the first, second and third domains of the heavy chain constant region, respectively. L1 and L2 are the first and second linker peptides, respectively, and h is a hinge region or derived sequence of an IgG antibody.

### Example 1.3.1. Structure (7): IgG_HC-VH-VH

Structure (7) represents a bispecific binding protein comprising two different polypeptide chains: a polypeptide chain 1 or a first polypeptide chain, also known as a short chain, comprising VL_A-CL from the amino-terminus to the carboxyl-terminus; and a polypeptide chain 2 or a second polypeptide chain, also known as a long chain, comprising VH_A-CH1-h-CH2-CH3-L1-VH_B-L2-VH_B from the amino-terminus to the carboxyl-terminus.

In one embodiment, CH3 of the polypeptide chain 2 is fusion-linked directly to VH_B, i.e., L1 is 0 in length. In another embodiment, CH3 of the polypeptide chain 2 is linked to VH_B via a linker peptide L1; and L1 may be the sequence listed in Table 1-2.

In one embodiment, a first VH_B of the polypeptide chain 2 is fusion-linked directly to a second VH_B, i.e., L2 is 0 in length. In another embodiment, a first VH_B of the polypeptide chain 2 is linked to a second VH_B via a linker peptide L2; and L2 may be the sequence listed in Table 1-2.

### Example 1.3.2. Structure (8): IgG_HC-VH'-VH"

Structure (8) represents a trispecific binding protein comprising two different polypeptide chains: a polypeptide chain 1 or a first polypeptide chain, also known as a short chain, comprising VL_A-CL from the amino-terminus to the carboxyl-terminus; and a polypeptide chain 2 or a second polypeptide chain, also known as a long chain, comprising VH_A-CH1-h-CH2-CH3-L1-VH_B-L2-VH_C from the amino-terminus to the carboxyl-terminus.

In one embodiment, CH3 of the polypeptide chain 2 is fusion-linked directly to VH_B, i.e., L1 is 0 in length. In another embodiment, CH3 of the polypeptide chain 2 is linked to VH_B via a linker peptide L1; and L1 may be the sequence listed in Table 1-2.

In one embodiment, VH_B of the polypeptide chain 2 is fusion-linked directly to VH_C, i.e., L2 is 0 in length. In another embodiment, VH_B of the polypeptide chain 2 is linked to VH_C via a linker peptide L2; and L2 may be the sequence listed in Table 1-2.

### Example 1.4. 2×VH-IgG hexavalent symmetric structure

The present invention provides a method for constructing a bispecific binding protein using two parent monoclonal antibodies: a conventional antibody A binding to a first antigen and a heavy-chain antibody B binding to a second antigen.

The present invention further provides a method for constructing a trispecific binding protein using three parent monoclonal antibodies: a conventional antibody A binding to a first antigen, a heavy-chain antibody B binding to a second antigen, and a heavy-chain antibody C binding to a third antigen.

As shown in FIG. 1 (9-10), the Fab end is derived from the conventional antibody A, and VH_A and VL_A are the heavy chain variable region and the light chain variable region of the antibody A, respectively. The VH end is derived from the heavy-chain antibody B or the heavy-chain antibody C, VH_B is the heavy chain variable region of the heavy-chain antibody B, and VH_C is the heavy chain variable region of the heavy-chain antibody C. CL is the light chain constant region domain. CH1, CH2 and CH3 are the first, second and third domains of the heavy chain constant region, respectively. L1 and L2 are the first and second linker peptides, respectively, and h is a hinge region or derived sequence of an IgG antibody.

### Example 1.4.1. Structure (9): 2×VH-IgG(HC+LC)

Structure (9) represents a bispecific binding protein comprising two different polypeptide chains: a polypeptide chain 1 or a first polypeptide chain, also known as a short chain, comprising VH_B-L1-VL_A-CL from the amino-terminus to the carboxyl-terminus; and a polypeptide chain 2 or a second polypeptide chain, also known as a long chain, comprising VH_B-L2-VH_A-CH1-h-CH2-CH3 from the amino-terminus to the carboxyl-terminus.

In one embodiment, VH_B of the polypeptide chain 1 is fusion-linked directly to VL_A, i.e., L1 is 0 in length. In another embodiment, VH_B of the polypeptide chain 1 is linked to VL_A via a linker peptide L1; and L1 may be the sequence listed in Table 1-2.

In one embodiment, VH_B of the polypeptide chain 2 is fusion-linked directly to VH_A, i.e., L2 is 0 in length. In another embodiment, VH_B of the polypeptide chain 2 is linked to VH_A via a linker peptide L2; and L2 may be the sequence listed in Table 1-2.

In one embodiment, L1 and L2 may be identical sequences. In another embodiment, L1 and L2 may be different sequences.

**Example 1.4.2. Structure (10): (VH'+VH")-IgG(HC+LC)**

Structure (10) represents a trispecific binding protein comprising two different polypeptide chains: a polypeptide chain 1 or a first polypeptide chain, also known as a short chain, comprising VH_B-L1-VL_A-CL from the amino-terminus to the carboxyl-terminus; and a polypeptide chain 2 or a second polypeptide chain, also known as a long chain, comprising VH _C-L2-VH_A-CH1-h-CH2-CH3 from the amino-terminus to the carboxyl-terminus.

In one embodiment, VH_B of the polypeptide chain 1 is fusion-linked directly to VL_A, i.e., L1 is 0 in length. In another embodiment, VH_B of the polypeptide chain 1 is linked to VL_A via a linker peptide L1; and L1 may be the sequence listed in Table 1-2.

In one embodiment, VH_C of the polypeptide chain 2 is fusion-linked directly to VH_A, i.e., L2 is 0 in length. In another embodiment, VH_C of the polypeptide chain 2 is linked to VH_A via a linker peptide L2; and L2 may be the sequence listed in Table 1-2.

In one embodiment, L1 and L2 may be identical sequences. In another embodiment, L1 and L2 may be different sequences.

**Example 1.5. Fab-Fc-VH bivalent asymmetric structure**

The present invention provides a method for constructing a bispecific binding protein using two parent monoclonal antibodies: a conventional antibody A binding to a first antigen and a heavy-chain antibody B binding to a second antigen.

As shown in FIG. 1 (11-13), the Fab end is derived from the conventional antibody A, and VH_A and VL_A are the heavy chain variable region and the light chain variable region of the antibody A, respectively. The VH end is derived from the heavy-chain antibody B, and VH_B is the heavy chain variable region of the heavy-chain antibody B. CL is the light chain constant region domain. CH1, CH2 and CH3 are the first, second and third domains of the heavy chain constant region, respectively. h is a hinge region or derived sequence of an IgG antibody. Different amino acid mutations are introduced into the Fc regions of the two heavy chains to reduce the homodimerization of heavy chains.

In one embodiment, h in the polypeptide chain containing VH_B may be a hinge region or a hinge region-derived linker peptide sequence of IgG, such as the sequence of human IgG1 hinge (C220S) or G5-LH in Table 1-2.

**Example 1.5.1. Structure (11): Fab-Fc-VH**

The binding protein of structure (11) comprises three different polypeptide chains: a polypeptide chain 1 or a first polypeptide chain, comprising VL_A-CL from the amino-terminus to the carboxyl-terminus; a polypeptide chain 2 or a second polypeptide chain, comprising VH_A-CH1-h-CH2-CH3 from the amino-terminus to the carboxyl-terminus; and a polypeptide chain 3, comprising VH_B-h-CH2-CH3 from the amino-terminus to the carboxyl-terminus.

**Example 1.5.2. Structure (12): Fab(cross VH/VL)-Fc-VH**

The binding protein of structure (12) comprises three different polypeptide chains: a polypeptide chain 1 or a first polypeptide chain, comprising VH_A-CL from the amino-terminus to the carboxyl-terminus; a polypeptide chain 2 or a second polypeptide chain, comprising VL_A-CH1-h-CH2-CH3 from the amino-terminus to the carboxyl-terminus; and a polypeptide chain 3, comprising VH_B-h-CH2-CH3 from the amino-terminus to the carboxyl-terminus.

**Example 1.5.3. Structure (13): Fab(cross Fd/LC)-Fc-VH**

The binding protein of structure (13) comprises three different polypeptide chains: a polypeptide chain 1 or a first polypeptide chain, comprising VH_A-CH1 from the amino-terminus to the carboxyl-terminus; a polypeptide chain 2 or a second polypeptide chain, comprising VL_A-CL-h-CH2-CH3 from the amino-terminus to the carboxyl-terminus; and a polypeptide chain 3 or a third polypeptide chain, comprising VH_B-h-CH2-CH3 from the amino-terminus to the carboxyl-terminus.

In one embodiment, h in the polypeptide chain 2 is a hinge region or a linker peptide, the sequence of which may be the sequence of LH1 in Table 1-2.

**Example 1.6. Fab-Fc-VH(2) trivalent asymmetric structure**

The present invention provides a method for constructing a bispecific binding protein using two parent monoclonal antibodies: a conventional antibody A binding to a first antigen and a heavy-chain antibody B binding to a second antigen.

The present invention further provides a method for constructing a trispecific binding protein using three parent monoclonal antibodies: a conventional antibody A binding to a first antigen, a heavy-chain antibody B binding to a second antigen, and a heavy-chain antibody C binding to a third antigen.

As shown in FIG. 1 (14-19), the Fab end is derived from the conventional antibody A, and VH_A and VL_A are the heavy chain variable region and the light chain variable region of the antibody A, respectively. The VH end is derived from the heavy-chain antibody B or the heavy-chain antibody C, VH_B is the heavy chain variable region of the heavy-chain antibody B, and VH_C is the heavy chain variable region of the heavy-chain antibody C. CL is the light chain constant region domain. CH1, CH2 and CH3 are the first, second and third domains of the heavy chain constant region, respectively. L is a linker peptide, and h is a hinge region or derived sequence of an IgG antibody. Different amino acid mutations are introduced into the Fc regions of the two heavy chains to reduce the homodimerization of heavy chains.

In one embodiment, h in the polypeptide chain containing VH_B may be a hinge region or a hinge region-derived linker peptide sequence of IgG, such as the sequence of human IgG1 hinge (C220S) or G5-LH in Table 1-2.

**Example 1.6.1. Structure (14): Fab-Fc-VH-VH**

Structure (14) represents a bispecific binding protein comprising three different polypeptide chains: a polypeptide chain 1 or a first polypeptide chain, comprising VL_A-CL from the amino-terminus to the carboxyl-terminus; a polypeptide chain 2 or a second polypeptide chain, comprising VH_A-CH1-h-CH2-CH3 from the amino-terminus to the carboxyl-terminus; and a polypeptide chain 3 or a third polypeptide chain, comprising VH_B-L-VH_B-h-CH2-CH3 from the amino-terminus to the carboxyl-terminus.

In one embodiment, the first VH_B of the polypeptide chain 3 is fusion-linked directly to the second VH_B, i.e., L is 0 in length. In another embodiment, the first VH_B of the polypeptide chain 3 is linked to the second VH_B via a linker peptide L; and L may be the sequence listed in Table 1-2.

**Example 1.6.2. Structure (15): Fab-Fc-VH'-VH"**

Structure (15) represents a trispecific binding protein comprising three different polypeptide chains: a polypeptide chain 1 or a first polypeptide chain, comprising VL_A-CL from the amino-terminus to the carboxyl-terminus; a polypeptide chain 2 or a second polypeptide chain, comprising VH_A-CH1-h-CH2-CH3 from the amino-terminus to the carboxyl-terminus; and a polypeptide chain 3 or a third polypeptide chain, comprising VH_C-L-VH_B-h-CH2-CH3 from the amino-terminus to the carboxyl-terminus.

In one embodiment, VH_C of the polypeptide chain 3 is fusion-linked directly to VH_B, i.e., L is 0 in length. In another embodiment, VH_C of the polypeptide chain 3 is linked to VH_B via linker peptide L; L may be the sequence listed in Table 1-2.

**Example 1.6.3. Structure (16): Fab(cross VH/VL)-Fc-VH-VH**

Structure (16) represents a bispecific binding protein comprising three different polypeptide chains: a polypeptide chain 1 or a first polypeptide chain, comprising VH_A-CL from the amino-terminus to the carboxyl-terminus; a polypeptide chain 2 or a second polypeptide chain, comprising VL_A-CH1-h-CH2-CH3 from the amino-terminus to the carboxyl-terminus; and a polypeptide chain 3 or a third polypeptide chain, comprising VH_B-L-VH_B-h-CH2-CH3 from the amino-terminus to the carboxyl-terminus.

In one embodiment, the first VH_B of the polypeptide chain 3 is fusion-linked directly to the second VH_B, i.e., L is 0 in length. In another embodiment, the first VH_B of the polypeptide chain 3 is linked to the second VH_B via a linker peptide L; and L may be the sequence listed in Table 1-2.

**Example 1.6.4. Structure (17): Fab(cross VH/VL)-Fc-VH'-VH"**

Structure (17) represents a trispecific binding protein comprising three different polypeptide chains: a polypeptide chain 1 or a first polypeptide chain, comprising VH_A-CL from the amino-terminus to the carboxyl-terminus; a polypeptide chain 2 or a second polypeptide chain, comprising VL_A-CH1-h-CH2-CH3 from the amino-terminus to the carboxyl-terminus; and a polypeptide chain 3 or a third polypeptide chain, comprising VH_C-L-VH_B-h-CH2-CH3 from the amino-terminus to the carboxyl-terminus.

In one embodiment, VH_C of the polypeptide chain 3 is fusion-linked directly to VH_B, i.e., L is 0 in length. In another embodiment, VH_C of the polypeptide chain 3 is linked to VH_B via linker peptide L; L may be the sequence listed in Table 1-2.

**Example 1.6.5. Structure (18): Fab(cross Fd/LC)-Fc-VH-VH**

Structure (18) represents a bispecific binding protein comprising three different polypeptide chains: a polypeptide chain 1 or a first polypeptide chain, comprising VH_A-CH1 from the amino-terminus to the carboxyl-terminus; a polypeptide chain 2 or a second polypeptide chain, comprising VL_A-CL-h-CH2-CH3 from the amino-terminus to the carboxyl-terminus; and a polypeptide chain 3 or a third polypeptide chain, comprising VH_B-L-VH_B-h-CH2-CH3 from the amino-terminus to the carboxyl-terminus.

In one embodiment, the first VH_B of the polypeptide chain 3 is fusion-linked directly to the second VH_B, i.e., L is 0 in length. In another embodiment, the first VH_B of the polypeptide chain 3 is linked to the second VH_B via a linker peptide L; and L may be the sequence listed in Table 1-2.

In one embodiment, h in the polypeptide chain 2 is a hinge region or a linker peptide, the sequence of which may be the sequence of LH1 in Table 1-2.

**Example 1.6.6. Structure (19): Fab(cross Fd/LC)-Fc-VH'-VH"**

Structure (19) represents a trispecific binding protein comprising three different polypeptide chains: a polypeptide chain 1 or a first polypeptide chain, comprising VH_A-CH1 from the amino-terminus to the carboxyl-terminus; a polypeptide chain 2 or a second polypeptide chain, comprising VL_A-CL-h-CH2-CH3 from the amino-terminus to the carboxyl-terminus; and a polypeptide chain 3 or a third polypeptide chain, comprising VH_C-L-VH_B-h-CH2-CH3 from the amino-terminus to the carboxyl-terminus.

In one embodiment, VH_C of the polypeptide chain 3 is fusion-linked directly to VH_B, i.e., L is 0 in length. In another embodiment, VH_C of the polypeptide chain 3 is linked to VH_B via linker peptide L; L may be the sequence listed in Table 1-2.

In one embodiment, h in the polypeptide chain 2 is a hinge region or a linker peptide, the sequence of which may be the sequence of LH1 in Table 1-2.

**Example 1.7. scFv-Fc-VH bivalent asymmetric structure**

The present invention provides a method for constructing a bispecific binding protein using two parent monoclonal antibodies: a conventional antibody A binding to a first antigen and a heavy-chain antibody B binding to a second antigen.

As shown in FIG. 1 (20-21), the scFv end is derived from the conventional antibody A, and VH_A and VL_A are the heavy chain variable region and the light chain variable region of the antibody A, respectively. The VH end is derived from the heavy-chain antibody B, and VH_B is the heavy chain variable region of the heavy-chain antibody B. CH2 and CH3 are the second and third domains of the heavy chain constant region, respectively. L is a linker peptide, and h is a hinge region or derived sequence of an IgG antibody. Different amino acid mutations are introduced into the Fc regions of the two heavy chains to reduce the homodimerization of heavy chains.

In one embodiment, the sequence of the scFv may be VH-linker peptide-VL. In another embodiment, the sequence of the scFv may be VL-linker peptide-VH.

In one embodiment, L in the polypeptide chain is a linker peptide, and may be the sequence listed in Table 1-2, preferably the sequence of GS_15 or GS_20.

In one embodiment, h in the polypeptide chain is a hinge region or derived sequence of an IgG antibody, and may be the sequence listed in Table 1-2, preferably the sequence of human IgG1 hinge (C220S) or G5-LH.

**Example 1.7.1. Structure (20): scFv(VL-VH)-Fc-VH**

The binding protein of structure (20) comprises two different polypeptide chains: a polypeptide chain 1 or a first polypeptide chain, comprising VL_A-L-VH_A-h-CH2-CH3 from the amino-terminus to the carboxyl-terminus; and a polypeptide chain 2 or a second polypeptide chain, comprising VH_B-h-CH2-CH3 from the amino-terminus to the carboxyl-terminus.

**Example 1.7.2. Structure (21): scFv(VH-VL)-Fc-VH**

The binding protein of structure (21) comprises two different polypeptide chains: a polypeptide chain 1 or a first polypeptide chain, comprising VH_A-L-VL_A-h-CH2-CH3 from the amino-terminus to the carboxyl-terminus; and a polypeptide chain 2 or a second polypeptide chain, comprising VH_B-h-CH2-CH3 from the amino-terminus to the carboxyl-terminus.

## Example 1.8. scFv-Fc-VH(2) trivalent asymmetric structure

The present invention provides a method for constructing a bispecific binding protein using two parent monoclonal antibodies: a conventional antibody A binding to a first antigen and a heavy-chain antibody B binding to a second antigen.

The present invention further provides a method for constructing a trispecific binding protein using three parent monoclonal antibodies: a conventional antibody A binding to a first antigen, a heavy-chain antibody B binding to a second antigen, and a heavy-chain antibody C binding to a third antigen.

As shown in FIG. 1 (22-25), the scFv end is derived from the conventional antibody A, and VH_A and VL_A are the heavy chain variable region and the light chain variable region of the antibody A, respectively. The VH end is derived from the heavy-chain antibody B or the heavy-chain antibody C, VH_B is the heavy chain variable region of the heavy-chain antibody B, and VH_C is the heavy chain variable region of the heavy-chain antibody C. CH2 and CH3 are the second and third domains of the heavy chain constant region, respectively. L1 and L2 are the first and second linker peptides, respectively, and h is a hinge region or derived sequence of an IgG antibody. Different amino acid mutations are introduced into the Fc regions of the two heavy chains to reduce the homodimerization of heavy chains.

In one embodiment, the sequence of the scFv may be VH-linker peptide-VL. In another embodiment, the sequence of the scFv may be VL-linker peptide-VH.

In one embodiment, L1 in the polypeptide chain is a linker peptide linking VH and VL in the scFv, and may be the sequence listed in Table 1-2, preferably the sequence of GS_15 or GS_20.

In one embodiment, L2 in the polypeptide chain is a linker peptide linking the heavy chain variable regions of the two heavy-chain antibodies, and may be the sequence listed in Table 1-2. In another embodiment, the heavy chain variable regions of the two heavy-chain antibodies are directly fused, i.e., L2 is 0 in length.

In one embodiment, h in the polypeptide chain is a hinge region or derived sequence of an IgG antibody, and may be the sequence listed in Table 1-2, preferably the sequence of human IgG1 hinge (C220S) or G5-LH.

## Example 1.8.1. Structure (22): scFv(VL-VH)-Fc-VH-VH

Structure (22) represents a bispecific binding protein comprising two different polypeptide chains: a polypeptide chain 1 or a first polypeptide chain, comprising VL_A-L1-VH_A-h-CH2-CH3 from the amino-terminus to the carboxyl-terminus; and a polypeptide chain 2 or a second polypeptide chain, comprising VH_B-L2-VH_B-h-CH2-CH3 from the amino-terminus to the carboxyl-terminus.

In one embodiment, a first VH_B of the polypeptide chain 2 is fusion-linked directly to a second VH_B, i.e., L2 is 0 in length. In another embodiment, a first VH_B of the polypeptide chain 2 is linked to a second VH_B via a linker peptide L2; and L2 may be the sequence listed in Table 1-2.

## Example 1.8.2. Structure (23): scFv(VH-VL)-Fc-VH-VH

Structure (23) represents a bispecific binding protein comprising two different polypeptide chains: a polypeptide chain 1 or a first polypeptide chain, comprising VH_A-L1-VL_A-h-CH2-CH3 from the amino-terminus to the carboxyl-terminus; and a polypeptide chain 2 or a second polypeptide chain, comprising VH_B-L2-VH_B-h-CH2-CH3 from the amino-terminus to the carboxyl-terminus.

In one embodiment, a first VH_B of the polypeptide chain 2 is fusion-linked directly to a second VH_B, i.e., L2 is 0 in length. In another embodiment, a first VH_B of the polypeptide chain 2 is linked to a second VH_B via a linker peptide L2; and L2 may be the sequence listed in Table 1-2.

## Example 1.8.3. Structure (24): scFv(VL-VH)-Fc-VH'-VH"

Structure (24) represents a trispecific binding protein comprising two different polypeptide chains: a polypeptide chain 1 or a first polypeptide chain, comprising VL_A-L1-VH_A-h-CH2-CH3 from the amino-terminus to the carboxyl-terminus; and a polypeptide chain 2 or a second polypeptide chain, comprising VH_C-L2-VH_B-h-CH2-CH3 from the amino-terminus to the carboxyl-terminus.

In one embodiment, VH_C of the polypeptide chain 2 is fusion-linked directly to VH_B, i.e., L2 is 0 in length. In another embodiment, VH_C of the polypeptide chain 2 is linked to VH_B via a linker peptide L2; and L2 may be the sequence listed in Table 1-2.

## Example 1.8.4. Structure (25): scFv(VH-VL)-Fc-VH'-VH"

Structure (25) represents a trispecific binding protein comprising two different polypeptide chains: a polypeptide chain 1 or a first polypeptide chain, comprising VH_A-L1-VL_A-h-CH2-CH3 from the amino-terminus to the carboxyl-terminus; a polypeptide chain 2 or a second polypeptide chain, comprising VH_C-L2-VH_B-h-CH2-CH3 from the amino-terminus to the carboxyl-terminus.

In one embodiment, VH_C of the polypeptide chain 2 is fusion-linked directly to VH_B, i.e., L2 is 0 in length. In another embodiment, VH_C of the polypeptide chain 2 is linked to VH_B via a linker peptide L2; and L2 may be the sequence listed in Table 1-2.

### Example 1.9. Fab-Fc-VH(3) tetravalent asymmetric structure

The present invention provides a method for constructing a bispecific binding protein using two parent monoclonal antibodies: a conventional antibody A binding to a first antigen and a heavy-chain antibody B binding to a second antigen.

The present invention further provides a method for constructing a trispecific binding protein using three parent monoclonal antibodies: a conventional antibody A binding to a first antigen, a heavy-chain antibody B binding to a second antigen, and a heavy-chain antibody C binding to a third antigen.

The present invention further provides a method for constructing a tetraspecific binding protein using four parent monoclonal antibodies: a conventional antibody A binding to a first antigen, a heavy-chain antibody B binding to a second antigen, a heavy-chain antibody C binding to a third antigen, and a heavy-chain antibody D binding to a fourth antigen.

As shown in FIG. 1 (26-27), the Fab end is derived from the conventional antibody A, and VH_A and VL_A are the heavy chain variable region and the light chain variable region of the antibody A, respectively. The VH end is derived from the heavy-chain antibody B, the heavy-chain antibody C or the heavy-chain antibody D, VH_B is the heavy chain variable region of the heavy-chain antibody B, VH_C is the heavy chain variable region of the heavy-chain antibody C, and VH_D is the heavy chain variable region of the heavy-chain antibody D. CL is the light chain constant region domain. CH1, CH2 and CH3 are the first, second and third domains of the heavy chain constant region, respectively. L1 and L2 are linker peptides, and h is a hinge region or derived sequence of an IgG antibody. Different amino acid mutations are introduced into the Fc regions of the two heavy chains to reduce the homodimerization of heavy chains.

In one embodiment, h in the polypeptide chain containing VH_B may be a hinge region or a hinge region-derived linker peptide sequence of IgG, such as the sequence of human IgG1 hinge (C220S) or G5-LH in Table 1-2.

### Example 1.9.1. Structure (26): Fab-Fc-VH-VH-VH

Structure (26) represents a bispecific binding protein comprising three different polypeptide chains: a polypeptide chain 1 or a first polypeptide chain, comprising VL_A-CL from the amino-terminus to the carboxyl-terminus; a polypeptide chain 2 or a second polypeptide chain, comprising VH_A-CH1-h-CH2-CH3 from the amino-terminus to the carboxyl-terminus; and a polypeptide chain 3 or a third polypeptide chain, comprising VH_B-L1-VH_B-L2-VH_B-h-CH2-CH3 from the amino-terminus to the carboxyl-terminus.

In one embodiment, the first VH_B of the polypeptide chain 3 is fusion-linked directly to the second VH_B, i.e., L1 is 0 in length. In another embodiment, a first VH_B of the polypeptide chain 3 is linked to a second VH_B via a linker peptide L1; and L1 may be the sequence listed in Table 1-2.

In one embodiment, a second VH_B of the polypeptide chain 3 is fusion-linked directly to a third VH_B, i.e., L2 is 0 in length. In another embodiment, a second VH_B of the polypeptide chain 3 is linked to a third VH_B via a linker peptide L2; and L2 may be the sequence listed in Table 1-2.

### Example 1.9.2. Structure (27): Fab-Fc-VH'-VH"-VH‴

Structure (27) represents a tetraspecific binding protein comprising three different polypeptide chains: a polypeptide chain 1 or a first polypeptide chain, comprising VL_A-CL from the amino-terminus to the carboxyl-terminus; a polypeptide chain 2 or a second polypeptide chain, comprising VH_A-CH1-h-CH2-CH3 from the amino-terminus to the carboxyl-terminus; and a polypeptide chain 3 or a third polypeptide chain, comprising VH_D-L1-VH_C-L2-VH_B-h-CH2-CH3 from the amino-terminus to the carboxyl-terminus.

In one embodiment, VH_D of the polypeptide chain 3 is fusion-linked directly to VH_C, i.e., L1 is 0 in length. In another embodiment, VH_D of the polypeptide chain 3 is linked to VH_C via a linker peptide L1; and L1 may be the sequence listed in Table 1-2.

In one embodiment, VH_C of the polypeptide chain 3 is fusion-linked directly to VH_B, i.e., L2 is 0 in length. In another embodiment, VH_C of the polypeptide chain 3 is linked to VH_B via linker peptide L2; and L2 may be the sequence listed in Table 1-2.

**Example 2. Sequence Analysis, Expression and Purification, and Characterization and Analysis of Physicochemical Properties of Antibodies**

**Example 2.1. Sequence analysis and optimization of antibodies**

The sequences of the heavy chain variable domain of the antibody are derived from events such as gene rearrangements of germline gene V, D and J segments of heavy chain gene clusters and somatic hypermutations on chromosomes; the sequences of the light chain variable domain are derived from the events such as gene rearrangements of germline gene V, D and J segments of light chain gene clusters and somatic hypermutations. Gene rearrangement and somatic hypermutation are major factors in increasing antibody diversity. Antibodies derived from the same germline V gene segment may also produce different sequences, but with relatively high similarity overall. The germline gene segments that are likely to undergo gene rearrangement can be deduced from the antibody variable domain sequences using algorithms such as IMGT/DomainGapAlign (http://imgt.org/3Dstructure-DB/cgi/DomainGapAlign.cgi) or NCBI/Ig-BLAST (https://www.ncbi.nlm.nih.gov/igblast/).

Chemical modifications, sometimes introduced after amino acid chains of a protein or polypeptide is translated and synthesized in a cell, are called post-translational modifications (PTMs). For antibodies, some PTM sites are very conservative. For example, the conservative amino acid asparagine (Asn) at position 297 (EU numbering) of the constant domain of the human IgG1 antibody is often glycosylated to form a saccharide chain whose structure is critical for antibody structure and associated effector functions. However, PTMs may have a greater effect on antigen binding or result in changes in the physicochemical properties of the antibody, if they are present in the variable domains, particularly in the antigen binding regions (e.g., CDRs) of an antibody. For example, glycosylation, deamidation, isomerization, oxidation, and the like may increase the instability or heterogeneity of antibody molecules, thereby increasing the difficulty and risk of antibody development. Thus, it is very important for the development of therapeutic antibodies to avoid some potential PTMs. As experience has accumulated, it has been found that some PTMs are highly correlated with the composition of amino acid sequences, especially the "pattern" of the composition of adjacent amino acids, which makes it possible to predict potential PTMs from the primary amino acid sequences of a protein. For example, it can be predicted that there is an N-linked glycosylation site from the N-x-S/T sequence pattern (asparagine at the first position, any amino acid other than non-proline at the second position, and serine or threonine at the third position). The amino acid sequence patterns leading to PTMs may be derived from germline gene sequences, e.g., the human germline gene fragment IGHV3-33 naturally having a glycosylation pattern NST in the FR3 region; or they may also be derived from somatic hypermutations. For example, NGS or NLT may be a glycosylation site, NS may be a deamidation site, and DG may cause isomerization of aspartic acid.

The amino acid sequence patterns of PTMs may be disrupted by amino acid mutations, thereby reducing or eliminating the formation of specific PTMs. There are different methods for designing mutations depending on the antibody sequences and PTM sequence patterns. One method is to replace a "hot spot" amino acid (e.g., N or S in the NS pattern) with an amino acid with similar physicochemical properties (e.g., to mutate N into Q). If the PTM sequence pattern is derived from somatic hypermutations and is not present in the germline gene sequence, the other method can be to replace the sequence pattern with the corresponding germline gene sequence. In practice, a variety of methods for designing mutations may be used for the same PTM sequence pattern.

**Example 2.2. Expression and purification of antibodies**

In this example, a general method for preparing antibodies in mammalian host cells (e.g., human embryonic kidney cell HEK293 or Chinese hamster ovary CHO cells and derived cells thereof) by such techniques as transient transfection and expression, and affinity capture and separation is described. This method is applicable to an antibody of interest comprising an Fc region. The antibody of interest may consist of one or more protein polypeptide chains, and may be derived from one or more expression plasmids.

The amino acid sequences of the polypeptide chains of the antibody were converted into nucleotide sequences by codon optimization. The encoding nucleotide sequences were synthesized and cloned into expression vectors compatible with the host cell. The mammalian host cells were transfected simultaneously with plasmids encoding the polypeptide chains of the antibody in a particular ratio, and the recombinant antibody with correct folding and assembly of polypeptide chains could be obtained by the conventional recombinant protein expression and purification techniques. Specifically, FreeStyle™ 293-F cells (Thermo, #R79007) were expanded in FreeStyle™ F17 Expression Medium (Thermo, #A1383504). Before transient transfection, the cells were adjusted to a concentration of 6-8×10⁵ cells/mL, and cultured in a shaker at 37 °C with 8% $CO_2$ for 24 h to make a concentration of $1.2×10^6$ cells/mL. 30 mL of cultured cells were taken. Plasmids encoding the polypeptide chains of the antibody were mixed in a certain ratio, and a total of 30 μg of the plasmids (the ratio of the plasmids to cells was 1 μg : 1 mL) were dissolved in 1.5 mL of Opti-MEM reduced serum medium (Thermo, #31985088). The resulting mixture was filtered through a 0.22 μm filter membrane for sterilization. Then, 1.5 mL of Opti-MEM was dissolved in 120 μL of 1 mg/mL PEI (Polysciences, #23966-2), and the mixture was left to stand for 5 min. PEI was slowly added to the plasmids, and the mixture was incubated at room temperature for 10 min. The mixed solution of plasmids and PEI was slowly added dropwise while shaking the culture flask, and the cells were cultured in a shaker at 37 °C with 8% $CO_2$ for 5 days. Cell viability was measured after 5 days. The culture was collected and centrifuged at 3300 g for 10 min, and then the supernatant was collected and centrifuged at high speed to remove impurities. A gravity column (Bio-Rad, #7311550) containing MabSelect™ (GE Healthcare, #71-5020-91) was equilibrated with a PBS buffer (pH 7.4) and rinsed with 2-5 column volumes of PBS. The column was loaded with the supernatant sample, and rinsed with 5-10 column volumes of PBS buffer, followed by 0.1 M glycine at pH 3.5 to elute the target protein. The eluate was adjusted to neutrality with Tris-HCl at pH 8.0, and concentrated and buffer exchanged into PBS buffer or a buffer with other components with an ultrafiltration tube (Millipore, #UFC901024) to obtain a purified solution of the recombinant antibody. Finally, the purified antibody solution was determined for concentration using NanoDrop (Thermo, NanoDrop™ One), subpackaged and stored for later use.

### Example 2.3. Analysis of protein purity and polymers by SEC-HPLC

In this example, analytical size-exclusion chromatography (SEC) was used to analyze the protein sample for purity and polymer form. An analytical chromatography column TSKgel G3000SW×I (Tosoh Bioscience, #08541, 5 μm, 7.8 mm × 30 cm) was connected to a highpressure liquid chromatograph HPLC (Agilent Technologies, Agilent 1260 Infinity II) and equilibrated with a PBS buffer at room temperature for at least 1 h. A proper amount of the protein sample (at least 10 μg) was filtered through a 0.22 μm filter membrane and then injected into the system, and an HPLC program was set: the sample was passed through the chromatography column with a PBS buffer at a flow rate of 1.0 mL/min for a maximum of 25 min. An analysis report was generated by the HPLC, with the retention time of the components with different molecular sizes in the sample reported.

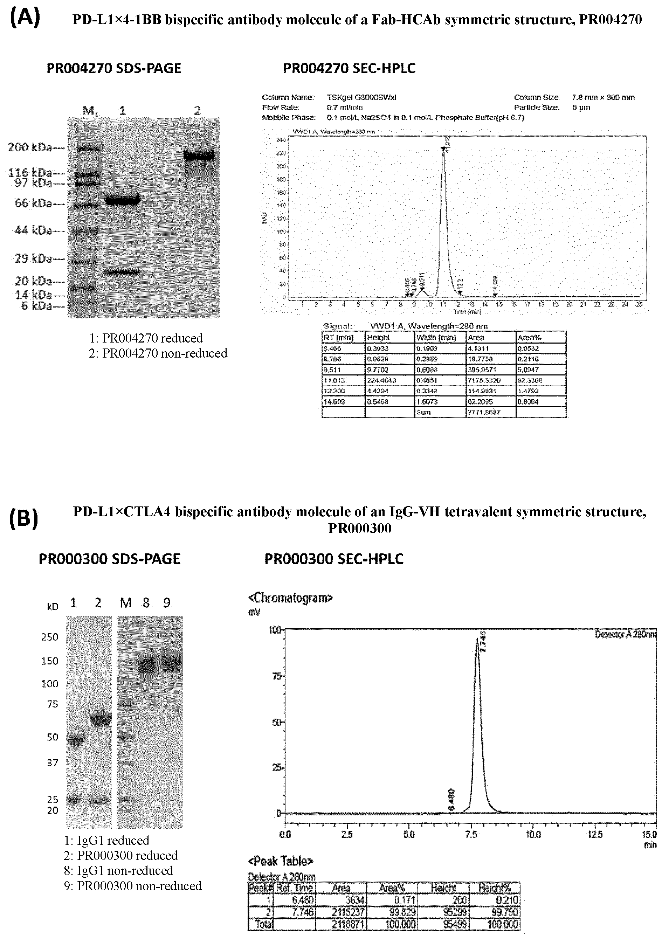

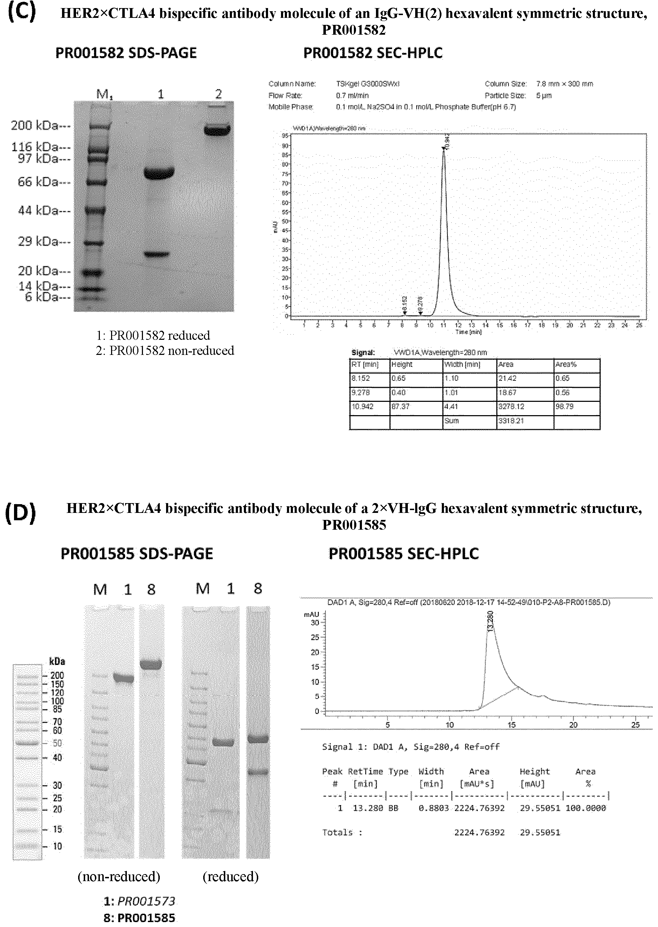

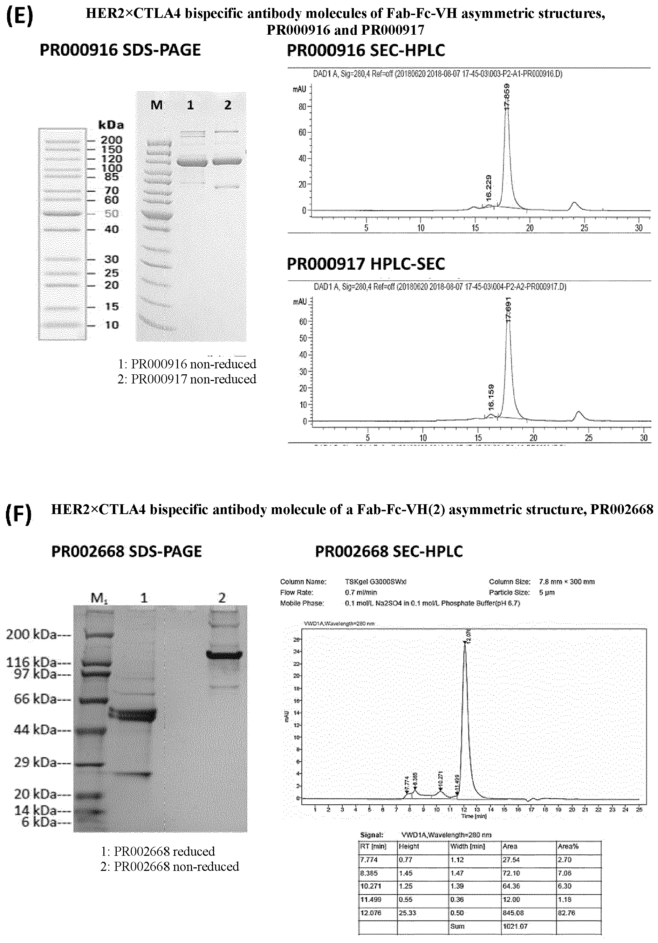

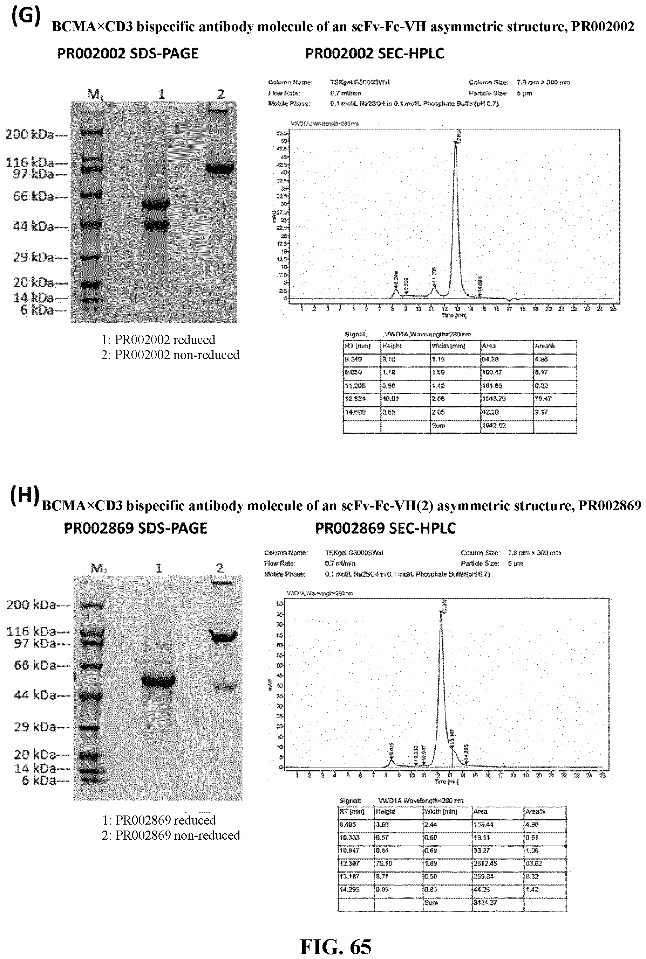

FIG. 65 showed the analysis results of SDS-PAGE and SEC-HPLC of protein samples obtained by transient transfection and expression and one-step affinity purification of several bispecific antibody molecules of different structures in the present application. All of those bispecific antibody molecules showed good yield and purity.

### Example 2.4. Determination of thermostability of protein molecules by DSF

Differential scanning fluorimetry (DSF) is a commonly used high-throughput method for determining the thermostability of proteins. In this method, changes in the fluorescence intensity of the dye that binds to unfolded protein molecules were monitored using a real-time quantitative fluorescence PCR instrument to reflect the denaturation process of the protein and thus to reflect the thermostability of the protein. In this example, the thermal denaturation temperature (Tm) of a protein molecule was measured by DSF. 10 μg of protein was added to a 96-well PCR plate (Thermo, #AB-0700/W), followed by the addition of 2 μL of 100× diluted dye SYPROTM (Invitrogen, #2008138), and then the mixture in each well was brought to a final volume of 40 μL by adding buffer. The PCR plate was sealed, placed in a real-time quantitative fluorescence PCR instrument (Bio-Rad CFX96 PCR System), and incubated at 25 °C for 5 min, then at a temperature gradually increased from 25 °C to 95 °C at a gradient of 0.2 °C/0.2 min, and at a temperature decreased to 25 °C at the end of the test. The FRET scanning mode was used and data analysis was performed using Bio-Rad CFX Maestro software to calculate the Tm of the sample.

### Example 2.5. Determination of molecular stability and molecular aggregation of proteins by Uncle

Uncle (Unchained Labs) is a multifunctional one-stop protein stability analysis platform that characterizes protein stability by total fluorescence, static light scattering (SLS) and dynamic light scattering (DLS) assay methods. The parameters of melting temperature (Tm), aggregation temperature (Tagg) and particle size (diameter) can be obtained simultaneously for the same group of samples. In this example, a "Tm & Tagg with optional DLS" application of Uncle was selected for the determination. 9 μL of sample was added to a Uni tube, and the temperature was set to gradually increase from 25 °C to 95 °C at a gradient of 0.3 °C/min. Four acquisitions of data were performed at the beginning and end of DLS assay, each for 5 s. After the experimental run was finished, the Tm value of each sample was calculated according to a barycentric mean (BCM) formula using the Uncle analysis software, the Tagg value was calculated from a curve (aggregation curve) of fluorescence intensity of SLS at the wavelength of 266 nm or 473 nm, and the particle size and dispersion of the samples were calculated from DLS related functions.

### Example 3. PD-L1×CTLA4 bispecific antibodies

**Example 3.1. Background**

Programmed death receptor 1 (PD-1) is mainly expressed in immune cells such as T cells. It has two ligands, i.e., programmed death ligand 1 (PD-L1) and PD-L2. PD-L1 is mainly expressed in antigen-presenting cells and a variety of tumor cells. The interaction between PD-L1 and PD-1 can down-regulate the activity of T cells, reduce the secretion of cytokines and play a role in immunosuppression. The expression of the PD-L1 protein can be detected in many human tumor tissues. The microenvironment at the tumor site can induce the expression of PD-L1 on tumor cells, and the expressed PD-L1 facilitates the occurrence and growth of tumors, induces the apoptosis of anti-tumor T cells and further protects the tumor cells from immune attack. The PD-1/PD-L1 pathway inhibitor can block the binding of PD-1 to PD-L1, block negative regulation signals, restore the activity of T cells, and play a role in killing tumor cells, thereby inhibiting tumor growth. Therefore, the immunoregulation targeting PD-1/PD-L1 is of great significance for tumor growth inhibition.

Cytotoxic T lymphocyte-associated antigen 4 (CTLA4) is a negative regulator expressed on T cells. After binding to CD80 or CD86 on antigen presenting cells, the CTLA4 blocks the co-stimulatory signal of CD28 and simultaneously down-regulates the activity of T cells, thereby playing a role in immunosuppression. CTLA4-mediated inhibition mechanisms are often responsible for the escape of tumor cells from the immune system. By blocking the interaction between CTLA4 and its ligand, the activity of T cells can be restored, and the anti-tumor ability can be enhanced.

At present, blocking antibodies against PD-1, PD-L1 and CTLA4 all show excellent anti-tumor effects in the clinic, and a plurality of antibody drugs are approved for marketing. Nevertheless, those drugs and treatments still face many clinical challenges, such as low response rates. Therefore, many clinical trials based on the combination regimens of blocking antibodies such as PD-1, PD-L1 and CTLA4 are actively underway. Since the CTLA4 inhibitor shows significant toxic and side effects, in the existing combination regimens of the PD-1/PD-L1 inhibitor and the CTLA4 inhibitor, the CTLA4 inhibitor is usually selected at a lower dose. For example, in a clinical trial of a combination of the anti-PD-1 antibody nivolumab and the anti-CTLA-4 antibody ipilimumab in the treatment of colorectal cancer and renal cell carcinoma, nivolumab and ipilimumab are at doses of 3 mg/kg and 1 mg/kg, respectively. In a clinical trial of a combination of the anti-PD-L1 antibody durvalumab, and the anti-CTLA-4 antibody tremelimumab in the treatment of non-small cell lung cancer, durvalumab and tremelimumab are at doses of 10-20 mg/kg and 1 mg/kg, respectively.

In another aspect, there is a growing understanding of the biological mechanisms related to tumor immunity. It has been found that blocking the CTLA4 signaling pathway will lead to high expression of PD-1 on tumor infiltrating lymphocytes (TILs), and blocking the PD-1 signaling pathway will up-regulate the expression of CTLA4 on TILs (Rev Assoc Med Bras 2017; 63(9): 814-823). This suggests that there may be a drug resistance mechanism induced by blocking a single co-inhibitory signaling pathway, whereas by combining those antibodies to block multiple co-inhibitory signaling pathways simultaneously, there may be a synergistic effect on T cell activation. Moreover, the latest study reported a new mechanism of interaction between PD-1 and CTLA4 signaling pathways, illustrating a synergistic effect of PD-L1 and CTLA4 antibodies (Zhao et al, 2019, Immunity 51, 1059-1073).

Based on the existing knowledge, we believe that bispecific antibodies targeting both PD-L1 and CTLA4 can improve anti-tumor efficacy and safety by one or more mechanisms of action. Firstly, the PD-L1 ×CTLA4 bispecific antibody activates T cells at different stages by blocking the CTLA4 signaling pathway and PD-1/PD-L1 signaling pathway; the cis interaction of PD-L1 and CD80 allows for a better synergy with CTLA4. Secondly, PD-L1 is highly expressed in tumor tissues, and the PD-L1 ×CTLA4 bispecific antibody can specifically remove CTLA4 inhibition signals in a tumor microenvironment to activate T cells and reduce toxic and side effects caused by non-specific activation of CTLA4 monoclonal antibodies in a peripheral system. Thirdly, the PD-L1 ×CTLA4 bispecific antibody can selectively retain Fc effector function (e.g., ADCC), thereby specifically killing inhibitory T cells highly expressing CTLA4 such as Treg cells by CTLA4 or specifically killing tumor cells highly expressing PD-L1 by PD-L1 in a tumor microenvironment. In addition, the bispecific antibody, as a drug product, is more advantageous in terms of economy and convenience of administration than the combination of two drug products.

**Example 3.2. Acquisition of anti-PD-L1 IgG antibodies and anti-CTLA4 HCAb antibodies**

**Example 3.2.1. Acquisition of fully human anti-PD-L1 IgG antibodies**

The Harbour H2L2 mouse (Harbour Antibodies BV) is a transgenic mouse carrying an immune repertoire of human immunoglobulins that produces antibodies with intact human antibody variable domains and rat constant domains.

Harbour H2L2 mice were subjected to multiple rounds of immunization with a soluble recombinant human PD-L1 protein (NovoProtein, #CM06). When the titer of the PD-L1-specific antibody in the serum of mice was detected to reach a certain level, spleen cells of the mice were taken and fused with a myeloma cell line to obtain hybridoma cells. After multiple rounds of screening and cloning of the hybridoma cells, several monoclonal antibody molecules specifically recognizing PD-L1 were identified. Those monoclonal antibodies were further identified, and several candidate antibody molecules were preferentially selected according to parameters such as the binding ability to human PD-L1, the binding ability to cynomolgus monkey PD-L1, and the ability to inhibit the binding of PD-L1 to PD-1. The candidate antibody molecules were then subjected to sequence analysis and optimization to obtain several variant sequences. The VL and VH sequences of the antibody were fused to the corresponding human κ light chain constant region and IgG1 heavy chain constant region sequences and expressed to obtain recombinant fully human antibody molecules. The recombinant fully human anti-PD-L1 IgG antibodies are listed in Table 3-9.

### Example 3.2.2. Acquisition of fully human anti-CTLA4 HCAb antibodies

The Harbour HCAb mouse (Harbour Antibodies BV, WO2010/109165A2) is a transgenic mouse carrying an immune repertoire of human immunoglobulins, capable of producing heavy chain-only antibodies that are only half the size of conventional IgG antibodies. The antibodies produced have only human antibody heavy chain variable domains and mouse Fc constant domains.

Harbour HCAb mice were subjected to multiple rounds of immunization with a soluble recombinant human CTLA4 protein (ACRO Biosystems, #CT4-H5229). When the titer of the CTLA4-specific antibody in the serum of mice was detected to reach a certain level, spleen cells of the mice were taken, from which B cells were isolated, and the CD138-positive plasma cells were sorted using a mouse plasma cell isolation kit (Miltenyi, #130-092-530). The human VH gene was amplified from plasma cells using conventional molecular biology techniques, and the amplified human VH gene fragments were constructed into mammalian cell expression plasmid pCAG vectors encoding the sequence of the heavy chain Fc region of the human IgG1 antibody. Mammal host cells (e.g., human embryonic kidney cell HEK293) were transfected with the plasmids and allowed to express antibodies to obtain a supernatant with fully human HCAb antibodies. Positive HCAb antibodies were identified by testing the supernatant with HCAb antibodies for binding to recombinant human CTLA4 proteins by ELISA. Those HCAb antibodies were further identified, and several candidate HCAb antibody molecules were preferentially selected according to parameters such as the binding ability to human CTLA4, the binding ability to cynomolgus monkey CTLA4, and ability to inhibit the binding of CTLA4 to B7-1. The candidate HCAb antibody molecules were then subjected to sequence analysis and optimization to obtain several variant sequences. The VH sequence of the HCAb antibody and the Fc sequence of the heavy chain of human IgG1 were fused and expressed to obtain fully human recombinant HCAb antibody molecules. The recombinant fully human anti-CTLA4 HCAb antibodies are listed in Table 3-9.

### Example 3.3. Construction of bispecific antibody molecules using anti-PD-L1 IgG antibodies and anti-CTLA4 HCAb antibodies

In this example, PD-L1 ×CTLA4 bispecific antibody molecules of a variety of structures were constructed using the antigen-binding domain Fab of the anti-PD-L1 IgG antibody PR000070 or PR000265 and the antigen-binding domain VH of the anti-CTLA4 HCAb antibody PR000184.

In this and subsequent examples, the anti-PD-L1 IgG monoclonal antibody PR000070, PR000416 or PR000265 was used as an anti-PD-L1 positive control molecule, and was also the parent monoclonal antibody of the PD-L1 end of the PD-L1 ×CTLA4 bispecific antibody molecule. PR000070, PR000265 and PR000416 are all derived from the same anti-PD-L1 monoclonal antibody. PR000070 and PR000265 are both human IgG1 subtypes and both have an N297A mutation in their Fc regions, the only difference between which was that PR000265 had one amino acid mutation in the heavy chain variable region VH. PR000265 and PR000416 are both human IgG1 subtypes and have identical Fab structures, the only difference between which was that PR000265 had an N297A mutation in the Fc region.

In this and subsequent examples, the anti-CTLA4 HCAb monoclonal antibody PR000184 was used as an anti-CTLA4 positive control molecule, and was also the parent monoclonal antibody of the CTLA4 end of the PD-L1×CTLA4 bispecific antibody molecule.

### Example 3.3.1. Construction of molecules of Fab-HCAb symmetric structures

PD-L1×CTLA4 bispecific antibody molecules of Fab-HCAb symmetric structures were designed according to the structures described in Example 1.1 using anti-PD-L1 IgG antibodies and anti-CTLA4 heavy-chain antibodies, with the results summarized in Table 3-1. The antibody molecule samples were prepared and analyzed according to the method described in Example 2, with the results summarized in Table 3-2.

**Table 3-1. PD-L1×CTLA4 bispecific antibody molecules of Fab-HCAb symmetric structures**

| Structure No. | Bispecific antibody molecules | PD-L1 Antibody (Fab) | CTLA4 Antibody (VH_B) | First linker peptide (between Fab and VH_B) | Second linker peptide (between VH_B and CH2) | Fc type (mutation) |
|---|---|---|---|---|---|---|
| 1 | PR000403 | PR000265 | PR000184 | None | Human IgG1 hinge | Human IgG1 (L234A, L235A, and P329G) |
| 1 | PR000404 | PR000265 | PR000184 | None | Human IgG1 hinge | Human IgG1 |

**Table 3-2. Expression of PD-L1×CTLA4 bispecific antibody molecule proteins of Fab-HCAb symmetric structures**

| Structure No. | Bispecific antibody molecules | Expression system and volume | Plasmid transfection ratio(short chain:long chain) | Yield (mg/L) after first purification | SEC-HPLC purity (%) |
|---|---|---|---|---|---|
| 1 | PR000403 | HEK293-F (30ml) | 3 : 2 | 32.00 | 97.57 |
| 1 | PR000404 | HEK293-F (30ml) | 3 : 2 | 19.67 | 97.02 |

**Example 3.3.2. Construction of molecules of IgG-VH tetravalent symmetric structure**

PD-L1 ×CTLA4 bispecific antibody molecules of IgG-VH tetravalent symmetric structures were designed according to the structures described in Example 1.2 using anti-PD-L1 IgG antibodies and anti-CTLA4 heavy-chain antibodies, with the results summarized in Table 3-3. The antibody molecule samples were prepared and analyzed according to the method described in Example 2, with the results summarized in Table 3-4.

**Table 3-3. PD-L1×CTLA4 bispecific antibody molecules of IgG-VH tetravalent symmetric structures**

| Structure No. | Bispecific antibody molecules | PD-L1 Antibody (IgG) | CTLA4 Antibody (VH_B) | VH_B position relative to IgG | Linker peptide | Fc type (mutation) |
|---|---|---|---|---|---|---|
| 3 | PR000300 | PR000070 | PR000184 | N-terminus of heavy chain | KL-H1_15-RT | Human IgG1 |
| 4 | PR000301 | PR000070 | PR000184 | N-terminus of light chain | KL-H1_15-RT | Human IgG1 |
| 6 | PR000302 | PR000070 | PR000184 | C-terminus of light chain | H1_15 | Human IgG1 |
| 5 | PR000303 | PR000070 | PR000184 | C-terminus of heavy chain | L-H1_15-RT | Human IgG1 |
| 5 | PR000401 | PR000265 | PR000184 | C-terminus of heavy chain | L-H1_15-RT | Human IgG1 (L234A, L235A, P329G) |
| 5 | PR000402 | PR000265 | PR000184 | C-terminus of heavy chain | L-GS_15-RT | Human IgG1 (L234A, L235A, P329G) |
| 3 | PR001573 | PR000265 | PR000184 | N-terminus of heavy chain | KL-H1_15-RT | Human IgG1 |

(continued)

| Structure No. | Bispecific antibody molecules | PD-L1 Antibody (IgG) | CTLA4 Antibody (VH_B) | VH_B position relative to IgG | Linker peptide | Fc type (mutation) |
|---|---|---|---|---|---|---|
| 3 | PR001576 | PR000265 | PR000184 | N-terminus of heavy chain | KL-H1_15-RT | Human IgG1 (S239D, I332E) |
| 4 | PR001574 | PR000265 | PR000184 | N-terminus of light chain | KL-H1_15-RT | Human IgG1 |
| 4 | PR001577 | PR000265 | PR000184 | N-terminus of light chain | KL-H1_15-RT | Human IgG1 (S239D, I332E) |

**Table 3-4. Expression of PD-L1×CTLA4 bispecific antibody molecule proteins of IgG-VH tetravalent symmetric structures**

| Structure No. | Bispecific antibody molecules | Expression system and volume | Yield (mg/L) after first purification | SEC-HPLC purity (%) |
|---|---|---|---|---|
| 3 | PR000300 | HEK293-F (30ml) | 27.33 | 100.0 |
| 4 | PR000301 | HEK293-F (30ml) | 20.64 | 100.0 |
| 6 | PR000302 | HEK293-F (30ml) | 29.11 | 100.0 |
| 5 | PR000303 | HEK293-F (30ml) | 42.57 | 100.0 |
| 5 | PR000401 | HEK293-F (30ml) | 2.67 | 96.77 |
| 5 | PR000402 | HEK293-F (30ml) | 5.33 | 96.41 |
| 3 | PR001573 | HEK293-F (50ml) | 20.00 | 100.0 |
| 3 | PR001576 | HEK293-F (50ml) | 0.4 | n.d. |
| 4 | PR001574 | HEK293-F (30ml) | 20.00 | 100.0 |
| 4 | PR001577 | HEK293-F (30ml) | 26.00 | 100.0 |

**Example 3.3.3. Construction of molecules of 2×VH-IgG hexavalent symmetric structures**

PD-L1×CTLA4 bispecific antibody molecules of 2×VH-IgG hexavalent symmetric structures were designed according to the structures described in Example 1.4 using anti-PD-L1 IgG antibodies and anti-CTLA4 heavy-chain antibodies, with the results summarized in Table 3-5. The antibody molecule samples were prepared and analyzed according to the method described in Example 2, with the results summarized in Table 3-6.

**Table 3-5. PD-L1×CTLA4 bispecific antibody molecules of 2×VH-IgG hexavalent symmetric structures**

| Structure No. | Bispecific antibody molecules | PD-L1 Antibody (IgG) | CTLA4 Antibody (VH_B) | First linker peptide (between VH_B and VL_A) | Second linker peptide (between VH_B and VH_A) | Fc type (mutation) |
|---|---|---|---|---|---|---|
| 9 | PR001572 | PR000070 | PR000184 | KL-H1_15-RT | KL-H1_15-RT | Human IgG1 |
| 9 | PR001575 | PR000265 | PR000184 | KL-H1_15-RT | KL-H1_15-RT | Human IgG1 |
| 9 | PR001578 | PR000265 | PR000184 | KL-H1_15-RT | KL-H1_15-RT | Human IgG1 (S239D, I332E) |

**Table 3-6. Expression of PD-L1×CTLA4 bispecific antibody molecule proteins of 2×VH-IgG hexavalent symmetric structures**

| Structure No. | Bispecific antibody molecules | Expression system and volume | Yield (mg/L) after first purification | SEC-HPLC purity (%) |
|---|---|---|---|---|
| 9 | PR001572 | HEK293-F (30ml) | 3.7 | n.d. |
| 9 | PR001575 | HEK293-F (30ml) | 5 | 100 |
| 9 | PR001578 | HEK293-F (30ml) | 4.3 | 100 |

**Example 3.3.4. Construction of molecules of Fab-Fc-VH bivalent asymmetric structures**

PD-L1×CTLA4 bispecific antibody molecules of Fab-Fc-VH bivalent asymmetric structures were designed according to the structures described in Example 1.4 using anti-PD-L1 IgG antibodies and anti-CTLA4 heavy-chain antibodies, with the results summarized in Table 3-7. The antibody molecule samples were prepared and analyzed according to the method described in Example 2, with the results summarized in Table 3-8.

**Table 3-7. PD-L1×CTLA4 bispecific antibody molecules of Fab-Fc-VH bivalent asymmetric structures**

| Structure No. | Bispecific antibody molecules | PD-L1 Antibody (Fab) | CTLA4 Antibody (VH_B) | Structure of Fab end | Fc type of Fab end (mutation) | Fc type of VH_B end (mutation) |
|---|---|---|---|---|---|---|
| 11 | PR001609 | PR000265 | PR000184 | Normal | Human IgG1 (knob) | Human IgG1 (hole) |
| 11 | PR001610 | PR000265 | PR000184 | Normal | Human IgG1 (knob, DE) | Human IgG1 (hole, DE) |

(mutation code: **Knob:** S354C, T366W; **Hole:** Y349C, T366S, L368A, Y407V; **DE:** S239D, I332E.)

**Table 3-8. Expression of PD-L1×CTLA4 bispecific antibody molecule proteins of Fab-Fc-VH bivalent asymmetric structures**

| Structure No. | Bispecific antibody molecules | Expression system and volume | Plasmid transfection ratio (chain 1:2:3) | Yield (mg/L) after first purification | SEC-HPLC purity (%) |
|---|---|---|---|---|---|
| 11 | PR001609 | HEK293-F (30ml) | 1 : 1 : 1 | 40 | 98.27 |
| 11 | PR001610 | HEK293-F (30ml) | 1 : 1 : 1 | 12.33 | 95.68 |

**Example 3.3.5. Sequences of PD-L1×CTLA4 bispecific antibody molecules and control molecules**

The sequence numbers corresponding to the sequences of the PD-L1 ×CTLA4 bispecific antibody molecules constructed in this example, the corresponding parent monoclonal antibody molecules such as the PD-L1 monoclonal antibody and the CTLA4 monoclonal antibody, and the control molecules are listed in Table 3-9, Table 3-10 and Table 3-11. The structure numbers in Table 3-11 correspond to those in Table 1-1 and FIG. 1.The sequence numbers of the corresponding CDR sequences of the first and second antigen-binding domains of the bispecific antibody molecules are listed in Table 3-12.

**Table 3-9. Control molecules and parent monoclonal antibodies**

| Antibody No. | Antibody |
|---|---|
| PR000070 | Anti-PD-L1 91G3H5H3, hIgG1(N297A) |
| PR000265 | Anti-PD-L1 91G3H5H3(D54E), hIgG1(N297A) |
| PR000416 | Anti-PD-L1 91G3H5H3(D54E), hIgG1 |

(continued)

| Antibody No. | Antibody |
|---|---|
| PR000184 | Anti-CTLA4 heavy-chain antibody CL5v3 |
| PR000149 | Anti-CTLA4 monoclonal antibody iplimumab analog, hIgG1 |
| PR000151 | Anti-PD-L1 monoclonal antibody atezolizumab analog, hIgG1(N297A) |

**Table 3-10. Sequence numbers of sequences and CDR sequences of control molecules and parent monoclonal antibodies**

| Antibody No. | Light chain | Heavy chain | VL | VH | LCDR1 | LCDR2 | LCDR3 | HCDR1 | HCDR2 | HCDR3 |
|---|---|---|---|---|---|---|---|---|---|---|
| PR000070 | 347 | 300 | 282 | 233 | 167 | 188 | 211 | 15 | 62 | 122 |
| PR000265 | 353 | 308 | 282 | 240 | 167 | 188 | 211 | 15 | 69 | 122 |
| PR000416 | 353 | 310 | 282 | 240 | 167 | 188 | 211 | 15 | 69 | 122 |
| PR000184 | None | 303 | None | 236 | None | None | None | 17 | 65 | 125 |
| PR000149 | 348 | 301 | 283 | 234 | 168 | 189 | 212 | 15 | 63 | 123 |
| PR000151 | 349 | 302 | 284 | 235 | 169 | 190 | 213 | 16 | 64 | 124 |

**Table 3-11. Sequence numbers of PD-L1×CTLA4 bispecific antibody molecules of this example**

| Structure No. | Antibody No. | Polypeptide chain 1 | Polypeptide chain 2 | Polypeptide chain 3 |
|---|---|---|---|---|
| 3 | PR000300 | 353 | 362 | None |
| 4 | PR000301 | 363 | 364 | None |
| 6 | PR000302 | 365 | 364 | None |
| 5 | PR000303 | 353 | 366 | None |
| 5 | PR000401 | 353 | 369 | None |
| 5 | PR000402 | 353 | 370 | None |
| 1 | PR000403 | 371 | 372 | None |
| 1 | PR000404 | 371 | 373 | None |
| 9 | PR001572 | 363 | 362 | None |
| 3 | PR001573 | 353 | 394 | None |
| 4 | PR001574 | 363 | 310 | None |
| 9 | PR001575 | 363 | 394 | None |
| 3 | PR001576 | 353 | 395 | None |
| 4 | PR001577 | 363 | 396 | None |
| 9 | PR001578 | 363 | 395 | None |
| 11 | PR001609 | 353 | 407 | 390 |
| 11 | PR001610 | 353 | 408 | 392 |

**Table 3-12. Sequence numbers of CDRs of antigen-binding domains of PD-L1×CTLA4 bispecific antibody molecules**

| Structure No. | Antibody No. | Antigen-binding domain No. | LCDR1 | LCDR2 | LCDR3 | HCDR1 | HCDR2 | HCDR3 |
|---|---|---|---|---|---|---|---|---|
| 1 | PR000403, PR000404 | #1 | 167 | 188 | 211 | 15 | 69 | 122 |
| | | #2 | None | None | None | 17 | 65 | 125 |
| 3 | PR000300 | #1 | 167 | 188 | 211 | 15 | 62 | 122 |
| | | #2 | None | None | None | 17 | 65 | 125 |
| 3 | PR001573, PR001576 | #1 | 167 | 188 | 211 | 15 | 69 | 122 |
| | | #2 | None | None | None | 17 | 65 | 125 |
| 4 | PR000301 | #1 | 167 | 188 | 211 | 15 | 62 | 122 |
| | | #2 | None | None | None | 17 | 65 | 125 |
| 4 | PR001574, PR001577 | #1 | 167 | 188 | 211 | 15 | 69 | 122 |
| | | #2 | None | None | None | 17 | 65 | 125 |
| 5 | PR000303 | #1 | 167 | 188 | 211 | 15 | 62 | 122 |
| | | #2 | None | None | None | 17 | 65 | 125 |
| 5 | PR000401, PR000402 | #1 | 167 | 188 | 211 | 15 | 69 | 122 |
| | | #2 | None | None | None | 17 | 65 | 125 |
| 6 | PR000302 | #1 | 167 | 188 | 211 | 15 | 62 | 122 |
| | | #2 | None | None | None | 17 | 65 | 125 |
| 9 | PR001572 | #1 | 167 | 188 | 211 | 15 | 62 | 122 |
| | | #2 | None | None | None | 17 | 65 | 125 |
| 9 | PR001575, PR001578 | #1 | 167 | 188 | 211 | 15 | 69 | 122 |
| | | #2 | None | None | None | 17 | 65 | 125 |
| 11 | PR001609, PR001610 | #1 | 167 | 188 | 211 | 15 | 69 | 122 |
| | | #2 | None | None | None | 17 | 65 | 125 |

## Example 3.4. Binding to CTLA4

This example is intended to investigate the binding activity of the PD-L1×CTLA4 bispecific antibody molecules to CTLA4.

## Example 3.4.1. Binding to human CTLA4 extracellular domain recombinant protein

The binding ability of the antibody molecules to human CTLA4 recombinant protein was determined by enzyme-linked immunosorbent assay (ELISA). Specifically, a 96-well plate was firstly coated with 2 μg/mL human CTLA4-His protein (ACRO Biosystems, #CT4-H5229) at 100 μL/well and incubated at 4 °C overnight. The plate was then rinsed 3 times with a PBST buffer (a PBS buffer containing 0.05% Tween-20), added with a blocking buffer (a PBS buffer containing 5% skim milk powder), and incubated at 37 °C for 1 h. The plate was then rinsed 3 times with a PBST buffer. Then, the antibody molecules at a total of 8 concentrations obtained by a 5-fold gradient dilution with the highest final concentration of 30 nM were added at 100 μL/well. The mixture was mixed well and incubated at 37 °C for 1 h. hIgG1 iso (CrownBio, #C0001) was used as an isotype control. The plate was then rinsed 3 times with a PBST buffer. Then, the HRP-labeled goat anti-human IgG Fc secondary antibody (Sigma, #A0170, diluted in a 1:5000 ratio) was added at 100 μL/well, and the mixture was incubated at 37 °C for 0.5 h. The plate was then rinsed 3 times with a PBST buffer. Then, TMB chromogenic solution was added at 100 μL/well for reaction for 15 min. Finally, a stop buffer was added to stop the reaction. The absorbance value (OD value) was read at 450 nM using an Enspire™ multifunctional microplate reader (Perkin Elmer, Inc.). The data were processed and analyzed by plotting using GraphPad Prism 8 software, and binding curves, $EC_{50}$ values and other parameters were obtained through four-parameter nonlinear fitting.

In this example, the anti-CTLA4 HCAb monoclonal antibody PR000184 was used as a positive control molecule, and was also the parent monoclonal antibody of the CTLA4 end of the PD-L1×CTLA4 bispecific antibody molecule.

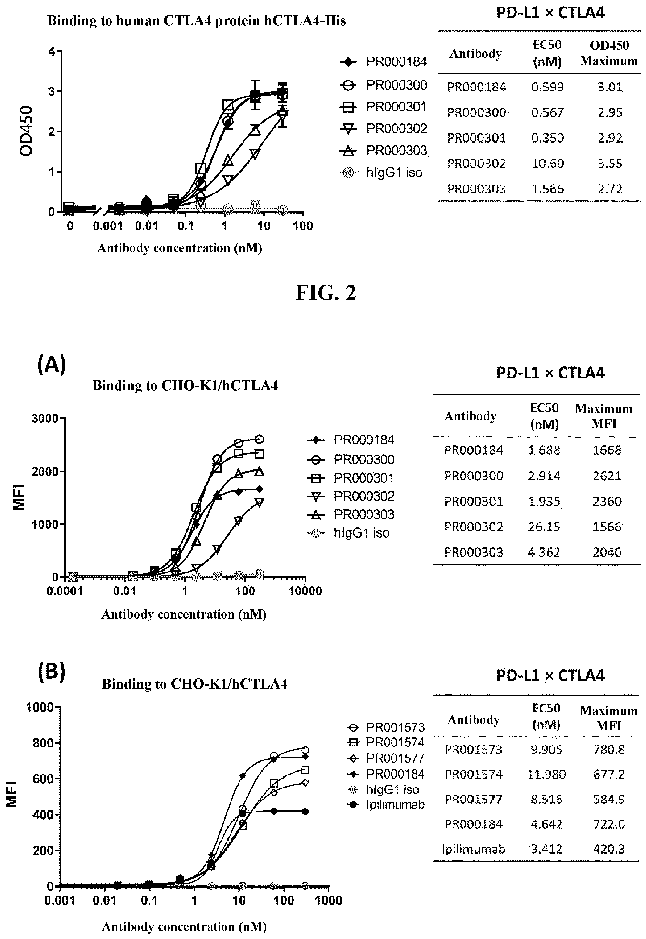

As shown in FIG. 2 and Table 3-13, the binding ability of the bispecific antibody molecules of IgG-VH tetravalent symmetric structures to CTLA4 was related to the position of the VH end of anti-CTLA4 relative to anti-PD-L1 IgG in the bispecific antibody structure. When VH was at the N-terminus of the heavy chain (PR000300) or the N-terminus of the light chain (PR000301) of IgG, the bispecific antibody molecule had a similar or even slightly superior $EC_{50}$ value for binding to CTLA4 to the parent monoclonal antibody PR000184; when VH was at the C-terminus of the heavy chain (PR000303) of IgG, the bispecific antibody molecule had an $EC_{50}$ value for binding to CTLA4 that was 2.6 times poorer than the parent monoclonal antibody PR000184; when VH was at the C-terminus of the light chain (PR000302) of IgG, the bispecific antibody molecule had significantly weakened binding ability to CTLA4. This indicates that the binding ability of the VH to the target can be modulated by adjusting the relative position of the VH on IgG.

### Table 3-13. Binding to human CTLA4 protein

| Antibody | $EC_{50}$ (nM) | Maximum OD450 |
|---|---|---|
| PR000184 | 0.599 | 3.01 |
| PR000300 | 0.567 | 2.95 |
| PR000301 | 0.35 | 2.92 |
| PR000302 | 10.6 | 3.55 |
| PR000303 | 1.566 | 2.72 |

**Example 3.4.2. Binding to CHO-K1 cells CHO-K1/hCTLA4 highly expressing human CTLA4 or HEK293 cells HEK293/hCTLA4 highly expressing human CTLA4**

The binding ability of antibody molecules to cells such as a CHO-K1 cell line CHO-K1/hCTLA4 (KYinno, KC-1406) highly expressing human CTLA4 or an HEK293T cell line HEK293/hCTLA4 (KYinno, KC-0209) highly expressing human CTLA4 was determined by flow cytometry FACS. Specifically, CHO-K1/hCTLA4 and HEK293/hCTLA4 cells were digested and resuspended in an F12K medium and a DMEM medium, respectively, with the cell density adjusted to $1×10^6$ cells/mL. Thereafter, the cells were seeded in a 96-well V-bottom plate (Corning, #3894) at 100 µL/well and centrifuged at 4 °C for 5 min, and the supernatant was discarded. Then, the antibody molecules at a total of 8 concentrations obtained by a 5-fold gradient dilution from the highest final concentration of 300 nM, or a total of 8 concentrations obtained by a 4-fold gradient dilution from the highest final concentration of 100 nM were added to the 96-well plate at 100 µL/well, and the mixture was mixed well. hIgG1 iso (CrownBio, #C0001) was used as an isotype control. The cells were incubated at 4 °C for 1 h away from light. Then, the cells in each well were rinsed twice with 100 µL of pre-cooled FACS buffer (PBS buffer containing 0.5% BSA) and centrifuged at 500 g at 4 °C for 5 min, and then the supernatant was discarded. Thereafter, a fluorescent secondary antibody (Alexa Fluor 488 anti-human IgG Fc, Biolegend, #409322, diluted in a 1:1000 ratio) was added at 100 µL/well, and the plate was incubated at 4 °C for 1 h away from light. Then, the cells in each well were then rinsed twice with 200 µL of pre-cooled FACS buffer and centrifuged at 500 g at 4 °C for 5 min, and then the supernatant was discarded. Finally, a pre-cooled FACS buffer was added at 200 µL/well to resuspend the cells. Fluorescence signal values were read using a BD FACS CANTOII flow cytometer or an ACEA NovoCyte flow cytometer, and the data were processed and analyzed using FlowJo v10 (FlowJo, LLC) software. The data were processed and analyzed by plotting using GraphPad Prism 8 software, and binding curves of the antibodies to target cells, $EC_{50}$ values and other parameters were obtained through four-parameter nonlinear fitting.

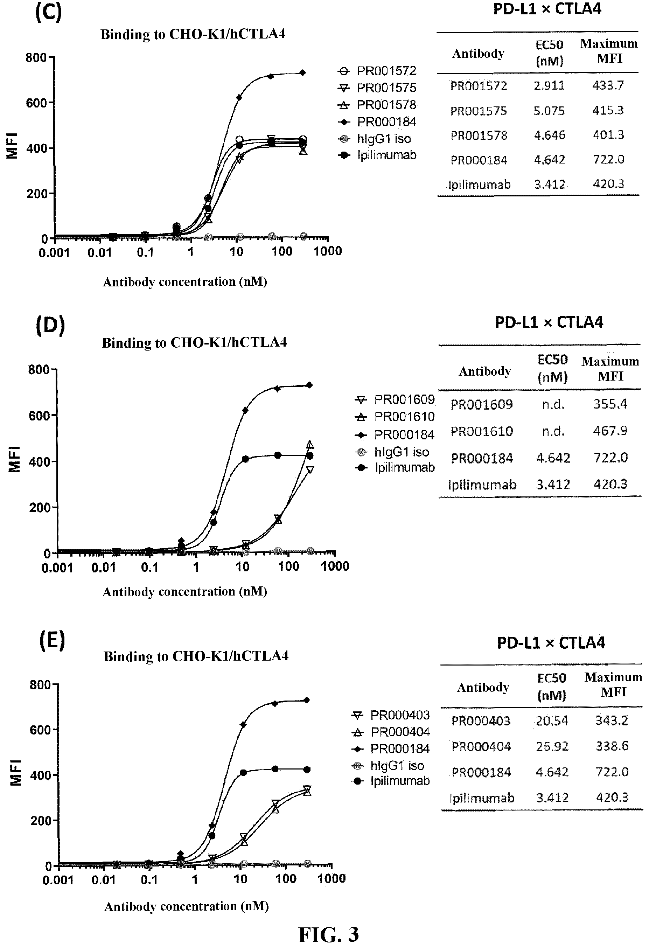

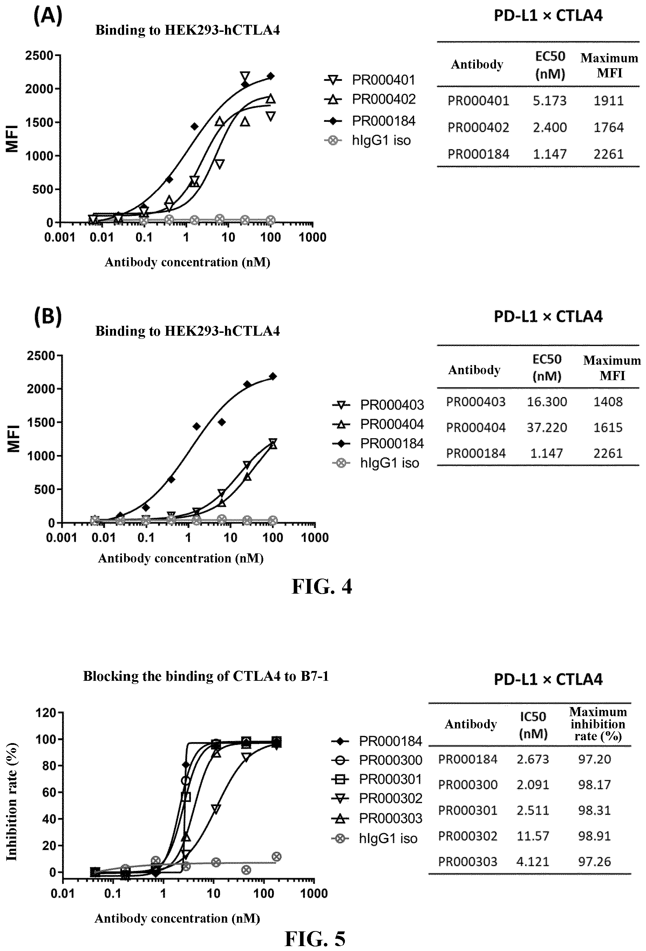

In this example, the anti-CTLA4 HCAb monoclonal antibody PR000184 was used as a positive control molecule, and was also the parent monoclonal antibody of the CTLA4 end of the PD-L1×CTLA4 bispecific antibody molecule. The Ipilimumab analog was also used as a positive control molecule. The results are shown in FIG. 3, FIG. 4, Table 3-14 and Table 3-15.

As shown in FIG. 3 (A-B) and FIG. 4 (A), the binding ability of the bispecific antibody molecules of IgG-VH tetravalent symmetric structures to CTLA4 was related to the position of the VH end of anti-CTLA4 relative to anti-PD-L1 IgG in the bispecific antibody structure. When VH was at the N-terminus of the heavy chain (PR000300 and PR001573) or the N-terminus of the light chain (PR000301, PR001574, and PR001577) of IgG, the bispecific antibody molecules had similar or slightly poorer $EC_{50}$ values for binding to CTLA4 than the parent monoclonal antibody PR000184, but had higher maximum MFIs on FACS than the parent monoclonal antibody PR000184 or the positive control Ipilimumab. When VH was at the C-terminus of the heavy chain (PR000303, PR000401, and PR000402) of IgG, the bispecific antibody molecule had a slightly poorer $EC_{50}$ value for binding to CTLA4 than the parent monoclonal antibody PR000184. When VH was at the C-terminus of the light chain (PR000302) of IgG, the bispecific antibody molecule had significantly weakened binding ability to CTLA4. This indicates that the binding ability of the VH to the target can be modulated by adjusting the relative position of the VH on IgG.

As shown in FIG. 3 (C), the bispecific antibody molecules of 2×VH-IgG hexavalent symmetric structures had similar $EC_{50}$ values for binding to CTLA4 to the parent monoclonal antibody PR000184, but had lower maximum MFIs than the parent monoclonal antibody PR000184. This is probably because the 2×VH-IgG hexavalent symmetric structure contains 4 binding sites for CTLA4, which is twice as many as the CTLA4-binding sites of the parent monoclonal antibody PR000184, so that the amount of bispecific antibody molecules required to bind to the same number of CTLA4 molecules is less than that of the parent monoclonal antibody, and the MFI value is related to the number of fluorescent bispecific antibodies that bind to the Fc region of the bispecific antibody molecules, so that it is reflected in that the maximum MFI of the bispecific antibody molecule is lower than that of the parent monoclonal antibody.

As shown in FIG. 3 (D), the bispecific antibody molecules of Fab-Fc-VH asymmetric structures PR001609 and PR001610 contained only one CTLA4-binding domain, and thus their binding ability to CTLA4 was weaker than that of the bivalent parent monoclonal antibody PR000184.

As shown in FIG. 3 (E) and FIG. 4 (B), the bispecific antibody molecules of Fab-HCAb symmetric structures had weaker binding ability to CTLA4 than the bivalent parent monoclonal antibody PR000184, but had similar maximum MFIs to the positive control Ipilimumab.

**Table 3-14. Binding to CHO-K1/hCTLA4**

| Antibody | $EC_{50}$ (nM) | Maximum MFI | Antibody | $EC_{50}$ (nM) | Maximum MFI | Antibody | $EC_{50}$ (nM) | Maximum MFI |
|---|---|---|---|---|---|---|---|---|
| PR000184 | 1.688 | 1668 | PR001573 | 9.905 | 780.8 | PR001609 | n.d. | 355.4 |
| PR000300 | 2.914 | 2621 | PR001574 | 11.98 | 677.2 | PR001610 | n.d. | 467.9 |
| PR000301 | 1.935 | 2360 | PR001577 | 8.516 | 584.9 | PR000403 | 20.54 | 343.2 |
| PR000302 | 26.15 | 1566 | PR001572 | 2.911 | 433.7 | PR000404 | 26.92 | 338.6 |
| PR000303 | 4.362 | 2040 | PR001575 | 5.075 | 415.3 | PR000184 | 4.642 | 722 |
| | | | PR001578 | 4.646 | 401.3 | Ipilimumab | 3.412 | 420.3 |
| **Test 1** | | | **Test 2** | | | | | |

**Table 3-15. Binding to HEK293/hCTLA4**

| Antibody | $EC_{50}$ (nM) | Maximum MFI |
|---|---|---|
| PR000401 | 5.173 | 1911 |
| PR000402 | 2.4 | 1764 |
| PR000403 | 16.3 | 1408 |
| PR000404 | 37.22 | 1615 |
| PR000184 | 1.147 | 2261 |

**Example 3.5. Blocking the binding of CTLA4 to its ligand**

This example is intended to investigate the inhibitory activity of the PD-L1 ×CTLA4 bispecific antibody molecules for the binding of CTLA4 to its ligand B7-1/CD80.

**Example 3.5.1. Blocking the binding of human CTLA4 protein to its ligand protein**

The inhibitory activity of the antibody molecules for the binding of human CTLA4 protein to its ligand B7-1/CD80 was determined by enzyme-linked immunosorbent assay (ELISA). Specifically, a 96-well plate was firstly coated with 2 μg/mL protein human B7-1-Fc (ACRO Biosystems, #B71-H5259) at 100 μL/well and placed at 4 °C overnight. The plate was then rinsed 3 times with a PBST buffer (a PBS buffer containing 0.05% Tween-20), added with a blocking buffer (a PBS buffer containing 5% skim milk powder), and incubated at 37 °C for 1 h. Then, the antibody molecules at a total of 7 concentrations obtained by a 4-fold gradient dilution with the highest final concentration of 180 nM were added at 90

μL/well. The mixture was mixed well and incubated at 37 °C for 20 min. hIgG1 iso (CrownBio, #C0001) was used as an isotype control. Then, biotinylated human CTLA4-Fc protein (ACRO Biosystems, # CT4-H82F3) was added at 10 μL/well to make a final concentration of 0.25 μg/mL, and the mixture was incubated at 37 °C for 1 h. The plate was then rinsed 3 times with a PBST buffer. Then, a label Precision Protein™ StrepTactin-HRP conjugate (Bio-RAD, #1610380, diluted in a 1:4000 ratio) was added at 100 μL/well, and the mixture was incubated at 37 °C for 0.5 h. The plate was then rinsed 3 times with a PBST buffer. Then, TMB chromogenic solution was added at 100 μL/well for reaction for 15 min. Finally, a stop buffer was added to stop the reaction. The absorbance value (OD value) was read at 490 nM using an Enspire™ multifunctional microplate reader (Perkin Elmer, Inc.). The data were processed and analyzed by plotting using GraphPad Prism 8 software to convert the OD value into the inhibition rate, and inhibition curves, $IC_{50}$ values, the maximum inhibition rates and other parameters were obtained through a four-parameter nonlinear fitting.

In this example, the anti-CTLA4 HCAb monoclonal antibody PR000184 was used as a positive control molecule, and was also the parent monoclonal antibody of the CTLA4 end of the PD-L1×CTLA4 bispecific antibody molecule. The results are shown in FIG. 5 and Table 3-16.

As shown in FIG. 5, the bispecific antibody molecules of IgG-VH tetravalent symmetric structures all had the ability (inhibitory activity) to block the binding of CTLA4 protein to its ligand protein with an inhibition rate of nearly 100%, but their $IC_{50}$ values for the inhibitory activity were related to the position of the VH end of anti-CTLA4 relative to anti-PD-L1 IgG in the bispecific antibody structure. When VH was at the N-terminus of the heavy chain (PR000300) or the N-terminus of the light chain (PR000301) of IgG, the bispecific antibody molecule had similar inhibitory activity for CTLA4 to the parent monoclonal antibody PR000184. When VH was at the C-terminus of the heavy chain (PR000303) of IgG, the bispecific antibody molecule had slightly weaker inhibitory activity for CTLA4 than the parent monoclonal antibody PR000184. When VH was at the C-terminus of the light chain (PR000302) of IgG, the bispecific antibody molecule had an $IC_{50}$ value for the inhibitory activity that was 4 times weaker than the parent monoclonal antibody PR000184. This indicates that the ability of the VH to block the target can be modulated by adjusting the relative position of the VH on IgG.

**Table 3-16. Blocking the binding of human CTLA4 protein to B7-1 protein**

| Antibody | $IC_{50}$ (nM) | Maximum inhibition rate (%) |
|----------|------------|------------------------------|
| PR000184 | 2.673 | 97.20 |
| PR000300 | 2.091 | 98.17 |
| PR000301 | 2.511 | 98.31 |
| PR000302 | 11.57 | 98.91 |
| PR000303 | 4.121 | 97.26 |

**Example 3.5.2. Blocking the binding of human CTLA4 cell to its ligand protein**

The inhibitory activity of the antibody molecules for the binding of cells expressing CTLA4 to the ligand B7-1/CD80 of CTLA4 was determined by flow cytometry FACS. Specifically, a CHO-K1 cell line CHO-K1/hCTLA4 (KYinno, KC-1406) highly expressing human CTLA4 was digested and resuspended in an F12K medium, with the cell density adjusted to $1\times10^6$ cells/mL. The cells were incubated in an FACS buffer (a PBS buffer containing 2% FBS) at 37 °C for 15 min. The FACS buffer was added to a 96-well plate at 200 μL/well for blocking. After incubation at 37 °C for 1 h, the blocking buffer in the wells was discarded. Thereafter, the CHO-K1/hCTLA4 cells were seeded in the 96-well plate at 200 μL/well ($2\times10^5$ cells/well) and centrifuged at 500 g at 4 °C for 5 min, and the supernatant was discarded. Then, the antibody molecules at a total of 8 concentrations obtained by a 3-fold gradient dilution from the highest final concentration of 200 nM, or a total of 8 concentrations obtained by a 5-fold gradient dilution from the highest final concentration of 400 nM were added to the 96-well plate at 100 μL/well, and the mixture was mixed well. hIgG1 iso (CrownBio, #C0001) was used as an isotype control. Then, 1 μg/mL biotinylated ligand protein human B7-1-Fc (ACRO Biosystems, #B71-H82F2) was added to the 96-well plate at 100 μL/well, and the mixture was mixed well. The 96-well plate was incubated at 4 °C for 1.5 h away from light. Then, the cells in each well were rinsed twice with 200 μL of pre-cooled FACS buffer and centrifuged at 500 g at 4 °C for 5 min, and then the supernatant was discarded. Thereafter, a fluorescent label (Streptavidin, Alexa Fluor™ 488 conjugate, Thermo, #S11223, diluted in a 1:500 ratio) was added at 100 μL/well, and the plate was incubated at 4 °C for 1 h away from light. Then, the cells in each well were rinsed three times with 200 μL of pre-cooled FACS buffer and centrifuged at 500 g at 4 °C for 5 min, and then the supernatant was discarded. Finally, a pre-cooled FACS buffer was added at 200 μL/well to resuspend the cells. Fluorescence signal values were read using a BD FACS CANTOII flow cytometer or an ACEA NovoCyte flow cytometer, and the data were processed and analyzed using FlowJo v10 (FlowJo, LLC) software. The data were processed and analyzed by plotting using GraphPad Prism 8 software to convert the fluorescence signal (MFI) into the inhibition rate, and inhibition curves, $IC_{50}$ values, the maximum inhibition rates and other parameters were obtained through four-parameter nonlinear fitting.

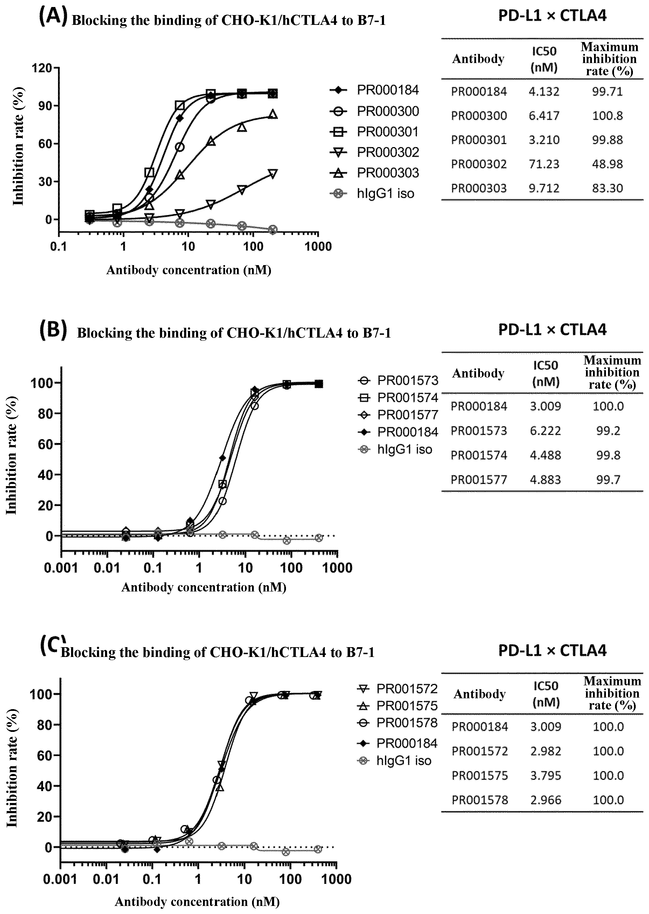

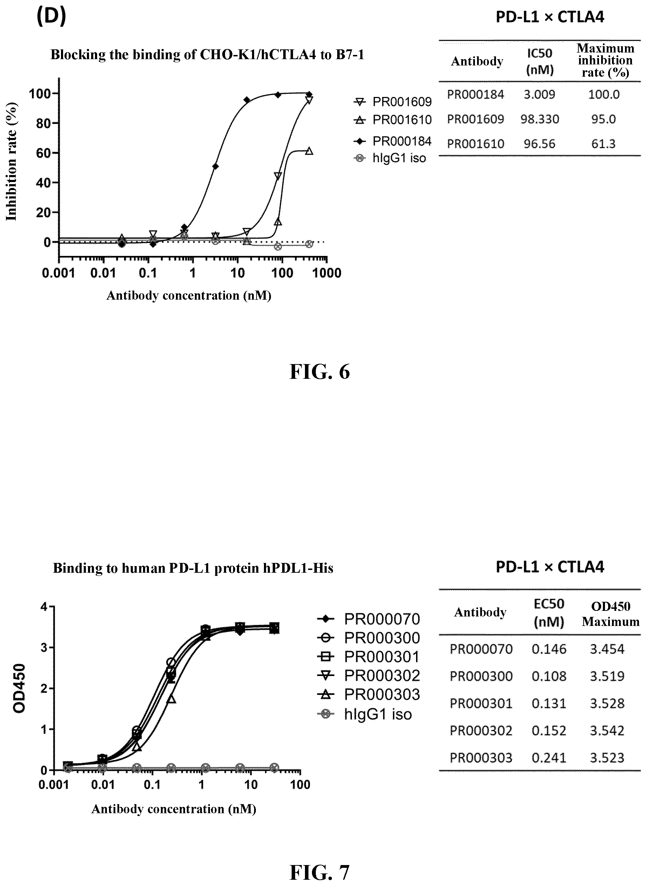

In this example, the anti-CTLA4 HCAb monoclonal antibody PR000184 was used as a positive control molecule, and was also the parent monoclonal antibody of the CTLA4 end of the PD-L1×CTLA4 bispecific antibody molecule. The results are shown in FIG. 6 and Table 3-17.

As shown in FIG. 6 (A-B), the inhibitory activity of the bispecific antibody molecules of IgG-VH tetravalent symmetric structures for CTLA4 was related to the position of the VH end of anti-CTLA4 relative to anti-PD-L1 IgG in the bispecific antibody structure. When VH was at the N-terminus of the heavy chain (PR000300 and PR001573) or the N-terminus of the light chain (PR000301, PR001574, and PR001577) of IgG, the bispecific antibody molecules all had a similar $IC_{50}$ value for the inhibitory activity to the parent monoclonal antibody PR000184, with an inhibition rate of nearly 100%, and PR000301 even had a higher inhibition rate than the parent monoclonal antibody PR000184. When VH was at the C-terminus of the heavy chain (PR000303) of IgG, the bispecific antibody molecule had a slightly weaker $IC_{50}$ value for the inhibitory activity than the parent monoclonal antibody PR000184, with the maximum inhibition rate of about 83%. When VH was at the C-terminus of the light chain (PR000302) of IgG, the bispecific antibody molecule had significantly weakened inhibitory activity, with a maximum inhibition rate of only 49%. This indicates that the ability of the VH to block the target can be modulated by adjusting the relative position of the VH on IgG. In another aspect, the results of different inhibitory activity of the same bispecific antibody molecule in the blocking experiment of CTLA4 cells and in the blocking experiment of CTLA4 protein may reflect the effect of steric hindrance of the protein on the cell membrane.

As shown in FIG. 6 (C), the bispecific antibody molecules of 2×VH-IgG hexavalent symmetric structures had similar inhibitory activity for CTLA4 to the parent monoclonal antibody PR000184.

As shown in FIG. 6 (D), the bispecific antibody molecules PR001609 and PR001610 of Fab-Fc-VH asymmetric structures both contained only one CTLA4-binding domain, so that their inhibitory activity for CTLA4 was significantly weaker than that of the bivalent parent monoclonal antibody PR000184.

**Table 3-17. Blocking the binding of CHO-K1/hCTLA4 cell to B7-1 protein**

| Antibody | $IC_{50}$ (nM) | Maximum inhibition rate (%) | Antibody | $IC_{50}$ (nM) | Maximum inhibition rate (%) | Antibody | $IC_{50}$ (nM) | Maximum inhibition rate (%) |
|---|---|---|---|---|---|---|---|---|
| PR000184 | 4.132 | 99.71 | PR001573 | 6.222 | 99.2 | PR001578 | 2.966 | 100 |
| PR000300 | 6.417 | 100.8 | PR001574 | 4.488 | 99.8 | PR001609 | 98.33 | 95 |
| PR000301 | 3.21 | 99.88 | PR001577 | 4.883 | 99.7 | PR001610 | 96.56 | 61.3 |
| PR000302 | 71.23 | 48.98 | PR001572 | 2.982 | 100 | | | |
| PR000303 | 9.712 | 83.3 | PR001575 | 3.795 | 100 | PR000184 | 3.009 | 100 |
| **Experiment 1** | | | **Experiment 2** | | | | | |

**Example 3.6. ADCC effect mediated by CTLA4**

This example is intended to investigate the antibody-dependent cell-mediated cytotoxicity (ADCC) of PD-L1×CTLA4 bispecific antibody molecules to 293F-hCTLA4 cells (ChemPartner) highly expressing human CTLA4. In the first step, target cells were labeled with DELFIA BATDA (Perkin Elmer, #C136-100). The specific labeling method was as follows. $1×10^6$ target cells were labeled with 2 μL of DELFIA BATDA reagent; the cells were incubated in an incubator at 37 °C with $CO_2$ for 20 min, washed 4 times with PBS, and centrifuged at 1000 rpm for 5 min; after the last wash, the precipitate was resuspended in a complete medium, with the cell density adjusted to $1×105$/mL. In the second step, the labeled target cells were seeded in a 96-well plate (Corning, #3599) at 100 μL/well. Then, the antibody molecules at a total of 7 concentrations obtained by a 5-fold gradient dilution with the highest final concentration of 0.8 nM were added at 50 μL/well. The mixture was mixed well. hIgG1 iso (CrownBio, #C0001) was used as an isotype control. The cells were incubated in an incubator at 37 °C with 5% $CO_2$ for 10 min. In the third step, the NK-92 MI/CD16a cells (ChemPartner) were collected as effector cells, and the cell density of NK-92 MI/CD16a was adjusted to $6×10^5$/mL. Then, the cells were added to the 96-well plate at 50 μL/well in an effect-to-target ratio of 3:1. Thereafter, the cells were incubated in an incubator at 37 °C with 5% $CO_2$ for 2 h. In the fourth step, the cells were centrifuged at 500 g for 5 min, and then 25 μL of supernatant from each well was added to a new 96-well assay plate. Then, a DELFIA® Europium solution (Perkin Elmer, #C135-100) was added at 200 μL/well, and the plate was shaken at 250 rpm at room temperature for 15 min. Finally, fluorescence values were measured using an EnVision® 2015 multifunctional microplate reader (Perkin Elmer, Inc.).

The killing rate was calculated according to the following formula:

$$\text{Kill rate (\%)} = (ER - ETSR)/(TMR - ETSR) \times 100\%$$

wherein:

ER = experimental well (antibody + effector cell + target cell); ETSR = spontaneous release well for mixed effector cell and target cell (effector cell + target cell); TMR = maximum release well for target cell (target cell + lysis buffer)

The data were processed and analyzed by plotting using GraphPad Prism 8 software, and killing rate curves of the antibodies to target cells, $EC_{50}$ values, maximum killing rates and other parameters were obtained through four-parameter nonlinear fitting.

In this example, the anti-CTLA4 HCAb monoclonal antibody PR000184 (hIgG1 subtype) was used as a positive control molecule, and was also the parent monoclonal antibody of the CTLA4 end of the PD-L1 ×CTLA4 bispecific antibody molecule. The anti-PD-L1 monoclonal antibody PR000416 (hIgG1 subtype) was used as a negative control molecule.

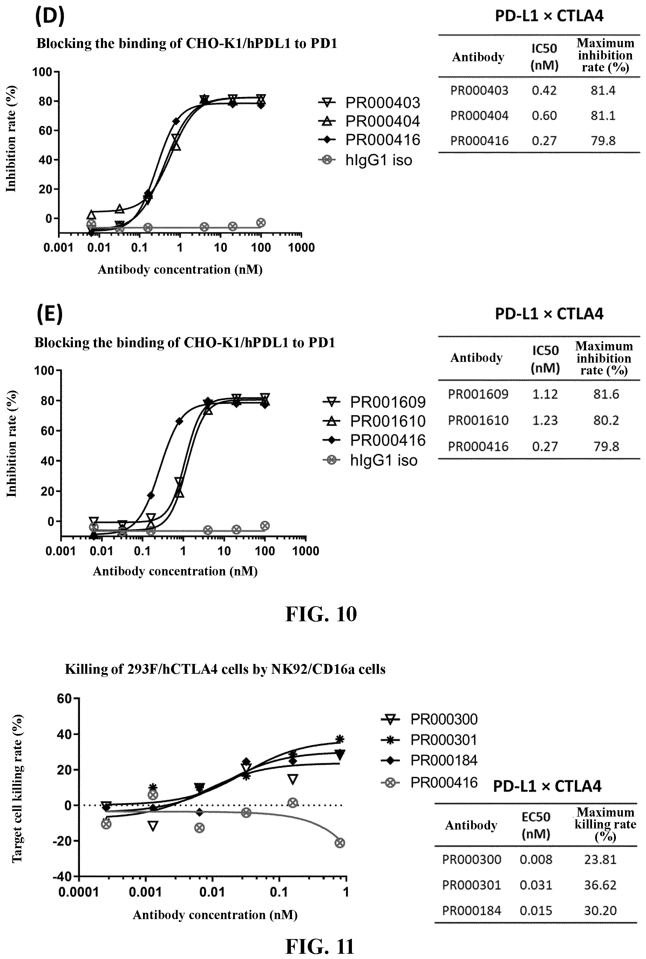

As shown in FIG. 11 and Table 3-18, the bispecific antibody molecules PR000300 and PR000301 had similar ADCC effect on CTLA4 to the parent monoclonal antibody PR000184.

**Table 3-18. Killing target cells by ADCC effect**

| Antibody | $EC_{50}$ (nM) | Maximum killing rate (%) |
|----------|----------------|--------------------------|
| PR000300 | 0.008 | 23.81 |
| PR000301 | 0.031 | 36.62 |
| PR000184 | 0.015 | 30.20 |

## Example 3.7. Binding to PD-L1

This example is intended to investigate the binding activity of the PD-L1 ×CTLA4 bispecific antibody molecules to PD-L1.

## Example 3.7.1. Binding to human PD-L1 extracellular domain recombinant protein

The binding ability of the antibody molecules to PD-L1 recombinant protein was determined by enzyme-linked immunosorbent assay (ELISA). Specifically, a 96-well plate (Corning, #9018) was firstly coated with 2 μg/mL human PD-L1-His protein (ACRO Biosystems, #PD1-H5229) at 100 μL/well and placed at 4 °C overnight. Then, the protein-coated plate was rinsed 3 times with a PBST buffer (a PBS buffer containing 0.05% Tween-20), added with a blocking buffer containing 2% BSA, and incubated at 37 °C for 1 h. The blocking buffer was discarded, and the plate was rinsed 3 times with a PBST buffer. Then, the antibody molecules at a total of 8 concentrations obtained by a 5-fold gradient dilution with the highest final concentration of 30 nM were added at 100 μL/well. The mixture was mixed well and incubated at 37 °C for 1 h. hIgG1 iso (CrownBio, #C0001) was used as an isotype control. The plate was then rinsed 3 times with a PBST buffer. Then, the HRP-labeled goat anti-human IgG Fc secondary antibody (Sigma, #A0170, diluted in a 1:5000 ratio) was added at 100 μL/well, and the mixture was incubated at 37 °C for 0.5 h. The plate was then rinsed 3 times with a PBST buffer. Then, TMB chromogenic solution was added at 100 μL/well for reaction for 15 min. Finally, a stop buffer was added to stop the reaction. The absorbance value (OD value) was read at 450 nM using an Enspire™ multifunctional microplate reader (Perkin Elmer, Inc.).

The data were processed and analyzed by plotting using GraphPad Prism 8 software, and binding curves, $EC_{50}$ values and other parameters were obtained through four-parameter nonlinear fitting.

In this example, the anti-PD-L1 monoclonal antibody PR000070 was used as a positive control molecule, and was also the parent monoclonal antibody of the PD-L1 end of the PD-L1×CTLA4 bispecific antibody molecule.

As shown in FIG. 7 and Table 3-19, the bispecific antibody molecules of IgG-VH tetravalent symmetric structures had similar binding ability to PD-L1 to the parent monoclonal antibody PR000070. This indicates that the VH domain of anti-CTLA4 has no significant effect on the Fab domain of anti-PD-L1.

**Table 3-19. Binding to human PDL1 protein**

| Antibody | $EC_{50}$ (nM) | Maximum OD450 |
|----------|----------------|---------------|
| PR000070 | 0.146 | 3.454 |
| PR000300 | 0.108 | 3.519 |

(continued)

| Antibody | EC$_{50}$ (nM) | Maximum OD450 |
|----------|---------------|---------------|
| PR000301 | 0.131 | 3.528 |
| PR000302 | 0.152 | 3.542 |
| PR000303 | 0.241 | 3.523 |

**Example 3.7.2. Binding to CHO-K1 cells CHO-K1/hPDL1 highly expressing human PD-L1 or tumor cells MDA-MB-231 highly expressing human PD-L1**

The binding ability of the antibody molecules to a CHO-K1 cell line CHO-K1/hPDL1 (GenScript, M00543) highly expressing human PD-L1 or a tumor cell line MDA-MB-231 (ATCC, HTB-26) highly expressing human PD-L1 and other cells was determined by flow cytometry FACS. Specifically, the cells were digested and resuspended in a complete medium, with the cell density adjusted to $1\times10^6$ cells/mL. Thereafter, the cells were seeded in a 96-well V-bottom plate (Corning, #3894) at 100 μL/well and centrifuged at 4 °C for 5 min, and the supernatant was discarded. Then, the antibody molecules at a total of 8 concentrations obtained by a 5-fold gradient dilution from the highest final concentration of 60 nM, or a total of 8 concentrations obtained by a 5-fold gradient dilution from the highest final concentration of 300 nM, or a total of 8 concentrations obtained by a 4-fold gradient dilution from the highest final concentration of 100 nM were added to the 96-well plate at 100 μL/well, and the mixture was mixed well. hIgG1 iso (CrownBio, #C0001) was used as an isotype control. The cells were incubated at 4 °C for 1 h away from light. Then, the cells in each well were rinsed twice with 100 μL of pre-cooled FACS buffer (PBS buffer containing 0.5% BSA) and centrifuged at 500 g at 4 °C for 5 min, and then the supernatant was discarded. Thereafter, a fluorescent secondary antibody (Goat human IgG (H + L) Alexa Fluor 488 conjugation, Thermo, #A11013, 1:1000 dilution) was added at 100 μL/well, and the plate was incubated at 4 °C for 1 h away from light. Then, the cells in each well were then rinsed twice with 200 μL of pre-cooled FACS buffer and centrifuged at 500 g at 4 °C for 5 min, and then the supernatant was discarded. Finally, a pre-cooled FACS buffer was added at 200 μL/well to resuspend the cells. Fluorescence signal values were read using a BD FACS CANTOII flow cytometer or an ACEA NovoCyte flow cytometer, and the data were processed and analyzed using FlowJo v10 (FlowJo, LLC) software. The data were processed and analyzed by plotting using GraphPad Prism 8 software, and binding curves of the antibodies to target cells, EC$_{50}$ values and other parameters were obtained through four-parameter nonlinear fitting.

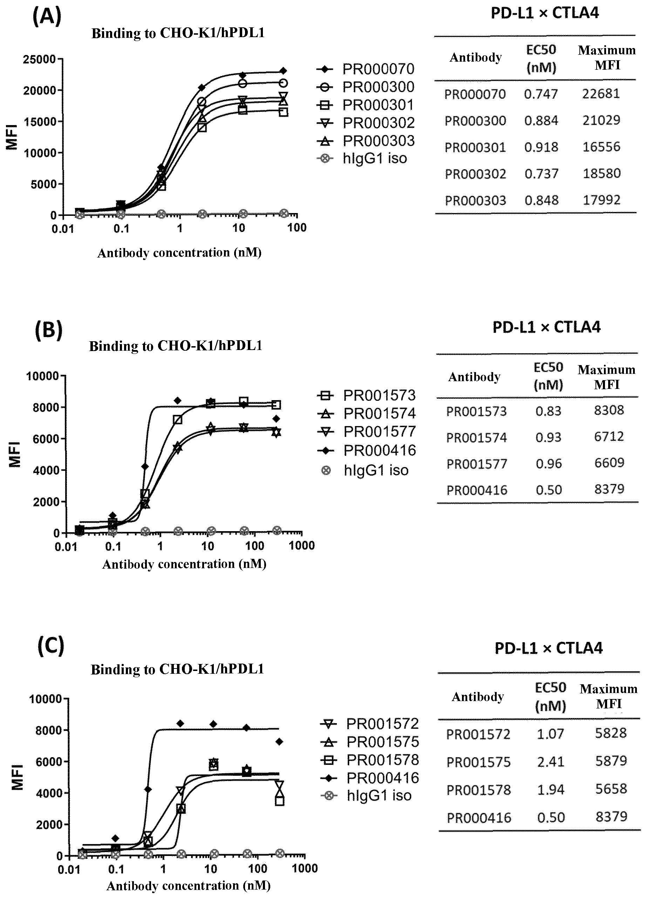

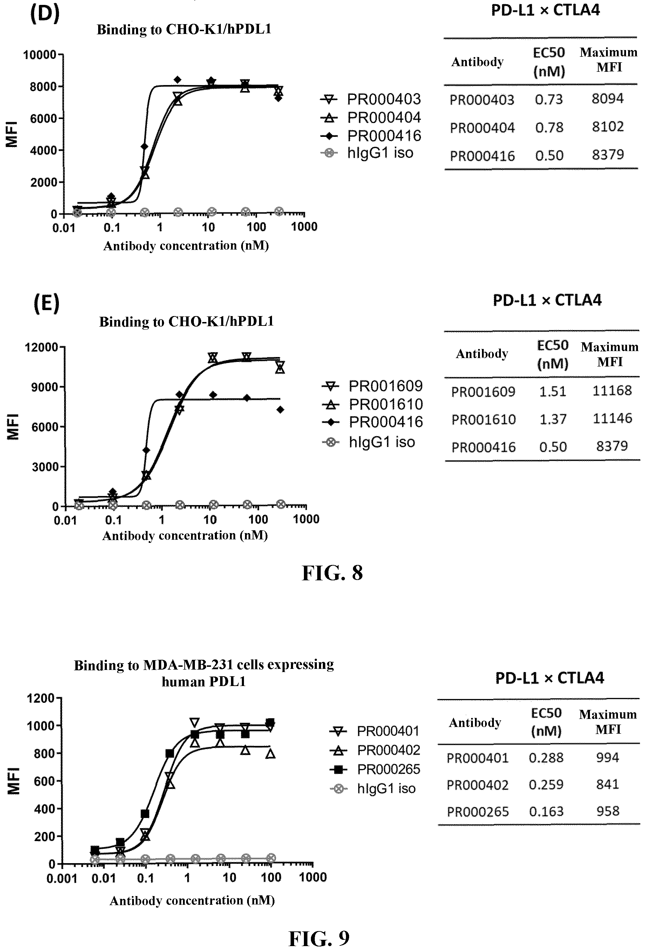

In this example, the anti-PD-L1 monoclonal antibody PR000070 or PR000416 (or PR000265) was used as a positive control molecule, and was also the parent monoclonal antibody of the PD-L1 end of the PD-L1 ×CTLA4 bispecific antibody molecule. The results are shown in FIG. 8, FIG. 9, Table 3-20 and Table 3-21.

As shown in FIG. 8 (A-B) and FIG. 9, the bispecific antibody molecules of IgG-VH tetravalent symmetric structures had similar binding ability to PD-L1 to the corresponding parent monoclonal antibody PR000070 or PR000416, and their EC$_{50}$ values for binding to PD-L1 are very close. Only when VH was at the N-terminus of the light chain (PR000301, PR001574, and PR001577) of IgG, the bispecific antibody molecules had slightly lower maximum MFIs than the parent monoclonal antibody.

As shown in FIG. 8 (C), the bispecific antibody molecules of 2×VH-IgG hexavalent symmetric structures had significantly reduced binding ability to PD-L1 as compared to the parent monoclonal antibody PR000416, indicating that when VH is linked to the N-terminus of both the heavy and light chains of IgG, the function of Fab end of IgG is significantly affected.

As shown in FIG. 8 (D), the bispecific antibody molecules of Fab-HCAb symmetric structures had similar binding ability to PD-L1 to the parent monoclonal antibody PR000416, and they have similar EC$_{50}$ values and maximum MFIs. This indicates that the Fab end structure of anti-PD-L1 of the bispecific antibody molecule of a Fab-HCAb symmetric structure can well retain the binding ability to PD-L1.

As shown in FIG. 8 (E), the PD-L1-binding domains of the bispecific antibody molecules PR001609 and PR001610 of Fab-Fc-VH asymmetric structures were monovalent Fab structures, but those bispecific antibody molecules had similar binding ability to PD-L1 to the parent monoclonal antibody of a bivalent structure, and had higher maximum MFIs than the parent monoclonal antibody.

**Table 3-20. Binding to CHO-K1/hPDL1 cells**

| Antibody | EC$_{50}$ (nM) | Maximum MFI | Antibody | EC$_{50}$ (nM) | Maximum MFI | Antibody | EC$_{50}$ (nM) | Maximum MFI |
|----------|---------------|-------------|----------|---------------|-------------|----------|---------------|-------------|
| PR000070 | 0.747 | 22681 | PR001573 | 0.83 | 8308 | PR001609 | 1.51 | 11168 |
| PR000300 | 0.884 | 21029 | PR001574 | 0.93 | 6712 | PR001610 | 1.37 | 11146 |

(continued)

| Antibody | EC$_{50}$ (nM) | Maximum MFI | Antibody | EC$_{50}$ (nM) | Maximum MFI | Antibody | EC$_{50}$ (nM) | Maximum MFI |
|---|---|---|---|---|---|---|---|---|
| PR000301 | 0.918 | 16556 | PR001577 | 0.96 | 6609 | PR000403 | 0.73 | 8094 |
| PR000302 | 0.737 | 18580 | PR001572 | 1.07 | 5828 | PR000404 | 0.78 | 8102 |
| PR000303 | 0.848 | 17992 | PR001575 | 2.41 | 5879 | PR000416 | 0.5 | 8379 |
| | | | PR001578 | 1.94 | 5658 | | | |
| Experiment 1 | | | Experiment 2 | | | | | |

**Table 3-21. Binding to MDA-MB-231 cells**

| Antibody | EC$_{50}$ (nM) | Maximum MFI |
|---|---|---|
| PR000401 | 0.288 | 994 |
| PR000402 | 0.259 | 841 |
| PR000265 | 0.163 | 958 |

## Example 3.8. Blocking the binding of PD-L1 to its ligand

This example is intended to investigate the inhibitory activity of the PD-L1 ×CTLA4 bispecific antibody molecules for the binding of PD-L1 to its ligand PD-1.

## Example 3.8.1. Blocking the binding of human PD-L1 cell to its ligand protein

The inhibitory activity of the antibody molecules for the binding of cells expressing PD-L1 to the ligand PD-1 of PD-L1 was determined by flow cytometry FACS. Specifically, a CHO-K1 cell line CHO-K1/hPDL1 (GenScript, M00543) highly expressing human PD-L1 was digested and resuspended in an F12K medium, with the cell density adjusted to $2\times10^6$ cells/mL. The cells were incubated in an FACS buffer (a PBS buffer containing 1% BSA) at 37 °C for 15 min. A blocking buffer was added to a 96-well plate at 200 µL/well. After incubation at 37 °C for 1 h, the blocking buffer in the wells was discarded. Thereafter, the CHO-K1/hPDL1 cells were seeded in the 96-well plate ($1\times10^5$ cells/well) at 50 µL/well. Then, the antibody molecules at a total of 8 concentrations obtained by a 5-fold gradient dilution from the highest final concentration of 100 nM were added to the 96-well plate at 100 µL/well. hIgG1 iso (CrownBio, #C0001) was used as an isotype control. The cells were incubated at 4 °C for 0.5 h away from light. Then, a PBS buffer was added at 100 µL/well, the mixture was centrifuged at 500 g at 4 °C for 5 min, and the supernatant was discarded. Then, 1 µg/mL biotinylated ligand protein human PD-1 protein (ACRO Biosystems, #PD1-H82F2) was added to the 96-well plate at 50 µL/well, and the mixture was mixed well. The 96-well plate was incubated at 4 °C for 0.5 h away from light. Then, the cells in each well were then rinsed twice with 100 µL of pre-cooled PBS buffer and centrifuged at 500 g at 4 °C for 5 min, and then the supernatant was discarded. Thereafter, a fluorescent secondary antibody (PE Streptavidin, BD Biosciences, #554061, 1:200 dilution) was added at 100 µL/well, and the plate was incubated at 4 °C for 0.5 h away from light. Then, the cells in each well were then rinsed twice with 200 µL of pre-cooled PBS buffer and centrifuged at 500 g at 4 °C for 5 min, and then the supernatant was discarded. Finally, a pre-cooled FACS buffer was added at 200 µL/well to resuspend the cells. Fluorescence signal values were read using a BD FACS CANTOII flow cytometer or an ACEA NovoCyte flow cytometer, and the data were processed and analyzed using FlowJo v10 (FlowJo, LLC) software. The data were processed and analyzed by plotting using GraphPad Prism 8 software to convert the fluorescence signal (MFI) into the inhibition rate, and inhibition curves, IC$_{50}$ values, the maximum inhibition rates and other parameters were obtained through four-parameter nonlinear fitting.

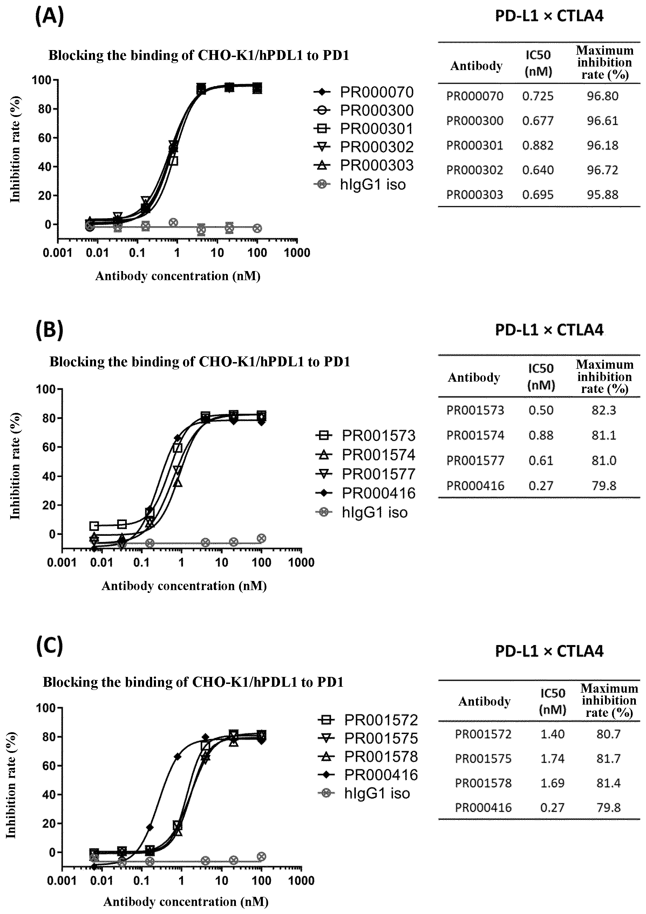

In this example, the anti-PD-L1 monoclonal antibody PR000070 or PR000416 (or PR000265) was used as a positive control molecule, and was also the parent monoclonal antibody of the PD-L1 end of the PD-L1 ×CTLA4 bispecific antibody molecule. The results are shown in FIG. 10 and Table 3-22.

As shown in FIG. 10 (A-B), the bispecific antibody molecules of IgG-VH tetravalent symmetric structures had similar inhibition ability to PD-L1 to the corresponding parent monoclonal antibody PR000070 or PR000416, and had similar IC$_{50}$ value and maximum inhibition rate to the parent monoclonal antibody. Only when VH was at the N-terminus of the light chain (PR001574 and PR001577) of IgG, the bispecific antibody molecules had an IC$_{50}$ value that was 2-3 times poorer than the parent monoclonal antibody.

As shown in FIG. 10 (C), the bispecific antibody molecules of $2\times$VH-IgG hexavalent symmetric structures had weaker inhibitory activity for PD-L1 than the parent monoclonal antibody; and they achieved a similar maximum inhibition rate to the parent monoclonal antibody although their corresponding $IC_{50}$ values were 4-6 times poorer than that of the parent monoclonal antibody.

As shown in FIG. 10 (D), the bispecific antibody molecules of Fab-HCAb symmetric structures had similar inhibitory activity for PD-L1 to the parent monoclonal antibody, and had similar $IC_{50}$ value and maximum inhibition rate to the parent monoclonal antibody.

As shown in FIG. 10 (E), the PD-L1-binding domains of the bispecific antibody molecules PR001609 and PR001610 of Fab-Fc-VH asymmetric structures were monovalent Fab structures, and those bispecific antibody molecules had weaker inhibitory activity for PD-L1 than the bivalent parent monoclonal antibody; and they achieved a similar maximum inhibition rate to the parent monoclonal antibody, although their corresponding $IC_{50}$ values were 4-5 times poorer than that of the parent monoclonal antibody.

**Table 3-22. Blocking the binding of CHO-K1/hPDL1 to PD1 protein**

| Antibody | $IC_{50}$ (nM) | Maximum inhibition rate (%) | Antibody | $IC_{50}$ (nM) | Maximum inhibition rate (%) | Antibody | $IC_{50}$ (nM) | Maximum inhibition rate (%) |
|---|---|---|---|---|---|---|---|---|
| PR000070 | 0.725 | 96.8 | PR001573 | 0.5 | 82.3 | PR001609 | 1.12 | 81.6 |
| PR000300 | 0.677 | 96.61 | PR001574 | 0.88 | 81.1 | PR001610 | 1.23 | 80.2 |
| PR000301 | 0.882 | 96.18 | PR001577 | 0.61 | 81 | PR000403 | 0.42 | 81.4 |
| PR000302 | 0.64 | 96.72 | PR001572 | 1.4 | 80.7 | PR000404 | 0.6 | 81.1 |
| PR000303 | 0.695 | 95.88 | PR001575 | 1.74 | 81.7 | PR000416 | 0.27 | 79.8 |
| | | | PR001578 | 1.69 | 81.4 | | | |
| **Experiment 1** | | | **Experiment 2** | | | | | |

**Example 3.9. Superantigen SEB stimulation assay**

This example is intended to investigate the activation effect of PD-L1 $\times$ CTLA4 bispecific antibody molecules on peripheral blood mononuclear cells (PBMCs). In the first step, the isolated human PBMC cells (MT-Bio) were firstly added to a 96-well plate (Corning, #3799) at 100 $\mu$L/well ($1\times10^5$ cells/well). Then, the antibody molecules at different concentrations were added at 100 $\mu$L/well, wherein the antibody concentrations may be the final concentration of (100 nM, 10 nM, 1 nM, 0.1 nM, 0.01 nM), or a concentration obtained by mixing two antibodies in a certain ratio, and two duplicate wells were set for each concentration. hIgG1 iso (CrownBio, #C0001) was used as an isotype control. The mixture was incubated at 37 °C for 30 min. Thereafter, the superantigen staphylocccal enterotoxin B (SEB) was added at 50 $\mu$L/well to make a final concentration of 100 ng/mL or 10 ng/mL. The cells were incubated in an incubator at 37 °C with 5% $CO_2$ for 96 h or 120 h, and the supernatant was collected. In the second step, the concentration of IL-2 in the collected supernatant was determined using an IL-2 ELISA kit (Biolegend, #431805), and the procedures were as described in the specification of the kit. The data were processed and analyzed by plotting using a GraphPad Prism 8 software.

In this example, the anti-CTLA4 HCAb monoclonal antibody PR000184 and anti-PD-L1 IgG monoclonal antibody PR000416 were used as positive control molecules.

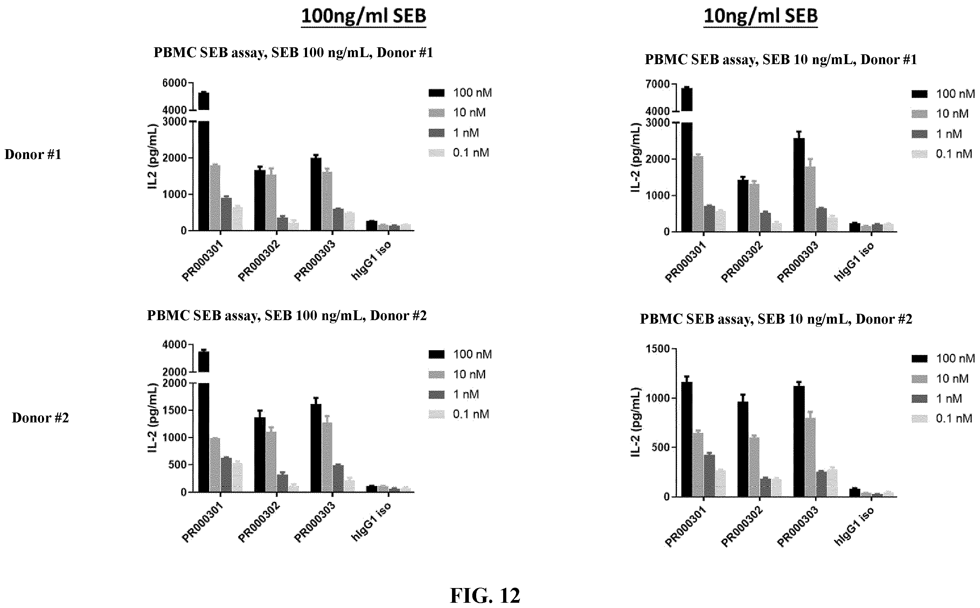

As shown in FIG. 12 to FIG. 15, when stimulated with SEB, the anti-CTLA4 monoclonal antibody molecules, anti-PD-L1 monoclonal antibody molecules and PD-L1$\times$CTLA4 bispecific antibody molecules were all able to promote T cells to product IL-2 to different degrees, and their T cell activation ability was related to their structures.

As shown in FIG. 12, the bispecific antibody molecules of IgG-VH tetravalent symmetric structures were able to activate T cells to produce IL-2. Moreover, the bispecific antibody molecule with a structure in which VH of anti-CTLA4 was at the N-terminus of IgG (PR000301) had stronger T cell activation ability than the bispecific antibody molecules with structures in which VH of anti-CTLA4 was at the C-terminus of IgG (PR000302 and PR000303).

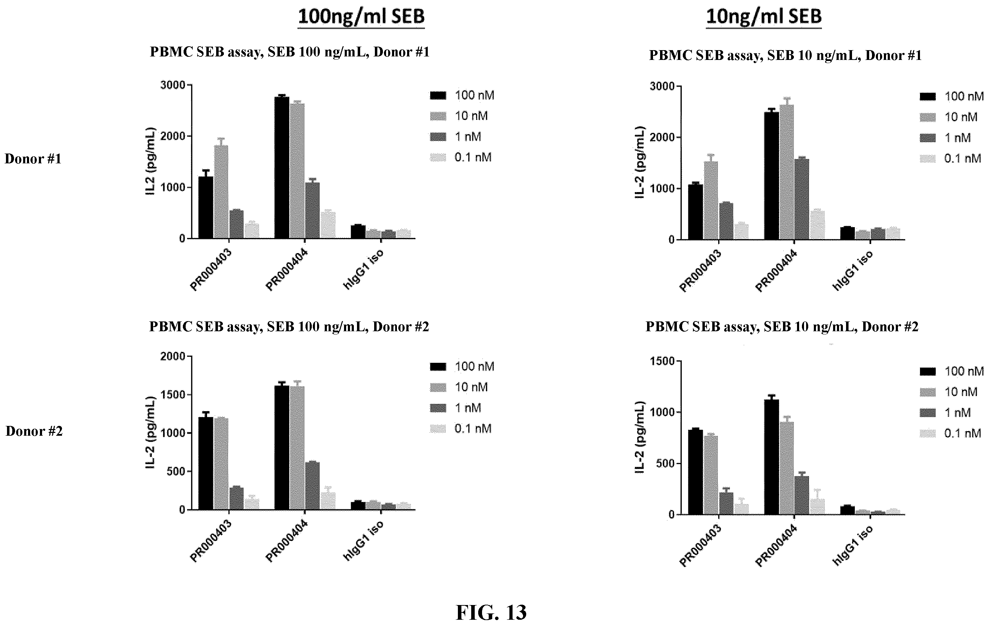

As shown in FIG. 13, the bispecific antibody molecules of Fab-HCAb symmetric structures were able to activate T cells to produce IL-2. Moreover, PR000404 had stronger T cell activation ability than PR000403. PR000404 and PR000403 had similar structures, the only difference between which was that PR000404 had a normal Fc region of human IgG1, while PR000403 had three point mutations (L234A, L235A, and P329G) in the Fc region to reduce Fc-related effector functions. It is speculated that PR000404 can clear Treg cells by CTLA4-mediated ADCC, thereby further enhancing the functions of T cells.

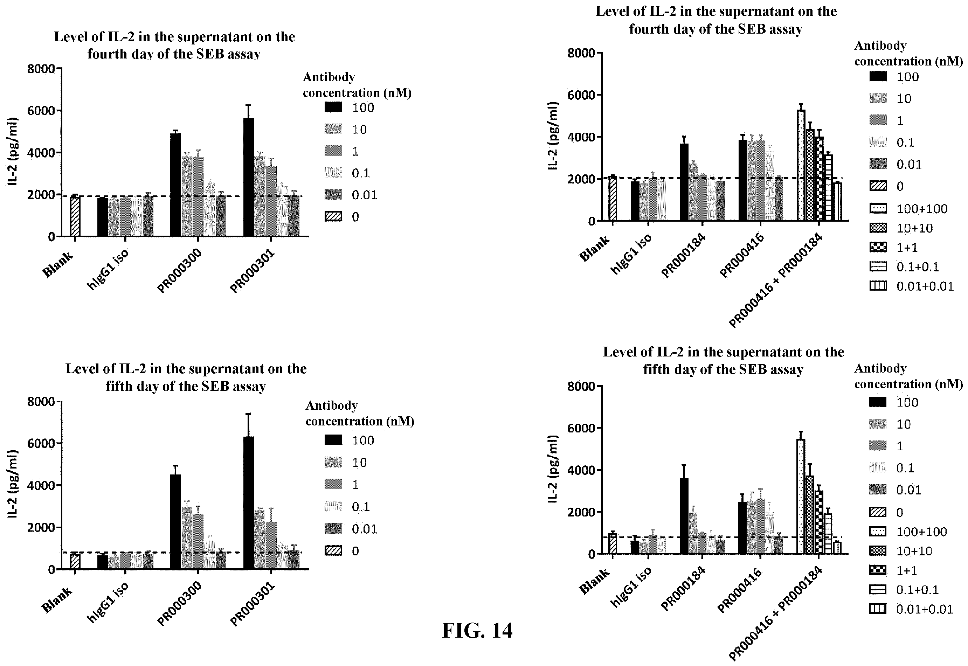

As shown in FIG. 14, the bispecific antibody molecules PR000300 and PR000301 had comparable or even stronger T cell activation ability than the anti-CTLA4 monoclonal antibody, the anti-PD-L1 monoclonal antibody and a 1:1 concentration combination thereof (using the same donor PBMC in the same experiment allows for comparison of the absolute values of IL-2 levels).

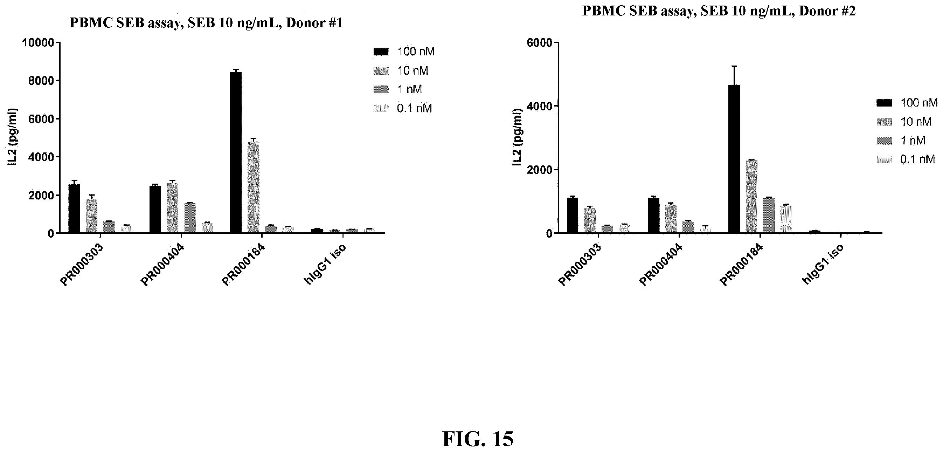

As shown in FIG. 15, the bispecific antibody molecules PR000303 (of an IgG_HC-VH structure, i.e., VH at the C-terminus of the heavy chain of IgG) and PR000404 (of a Fab-HCAb structure) had weaker T cell activation ability than the anti-CTLA4 HCAb monoclonal antibody PR000184. This indicates that the CTLA4 end will have weakened activity in those two structures as compared to its presence in normal IgG or HCAb bivalent structures, so that its dose-related toxicity can be reduced for suitable use in current combination drug doses in the clinic.

### Example 3.10. Mixed Lymphocyte Reaction (MLR)

This example is intended to investigate the T cell activation effect of PD-L1 $\times$ CTLA4 bispecific antibody molecules by the mixed lymphocyte reaction (MLR). In the first step, the recombinant human interleukin 4 (IL-4, R&D Systems, #204-GMP) and the recombinant human GM-CSF (R&D Systems, #215-GM) were added to the first donor PBMC cells (MT-Bio). After 6 days of induction, immature human CD14$^+$ dendritic cells (iDC cells) were obtained. 1 $\mu$g/mL lipopolysaccharide (LPS, Sigma, #L2630) was then added, and after 24 h of induction, mature dendritic cells (mDC cells) were obtained. In the second step, T lymphocytes were isolated from the second donor PBMC cells (MT-Bio) using a T cell isolation kit (StemCell, #17951). In the third step, the obtained T cells and mDC cells were seeded in a 96-well plate (T cells at $1\times10^5$/well and mDC cells at $1\times10^4$/well) at a ratio of 10:1. Then, antibody molecules at different concentrations were added at 100 $\mu$L/well, wherein the antibody concentrations may be the final concentration of (100 nM, 10 nM, 1 nM, 0.1 nM, 0 nM), or a concentration obtained by mixing two antibodies in a certain ratio, and two duplicate wells were set for each concentration. hIgG1 iso (CrownBio, # C0001) was used as an isotype control. The cells were incubated in an incubator at 37 °C with 5% $CO_2$ for 5 days. In the fourth step, supernatants on day 3 and on day 5 were each collected. The IL-2 concentration in the 3-day supernatant was determined using an IL-2 ELISA kit (Thermo, #88-7025-88), and the IFN-$\gamma$ concentration in the 5-day supernatant was determined using an IFN-$\gamma$ ELISA kit (Thermo, #88-7316-88).

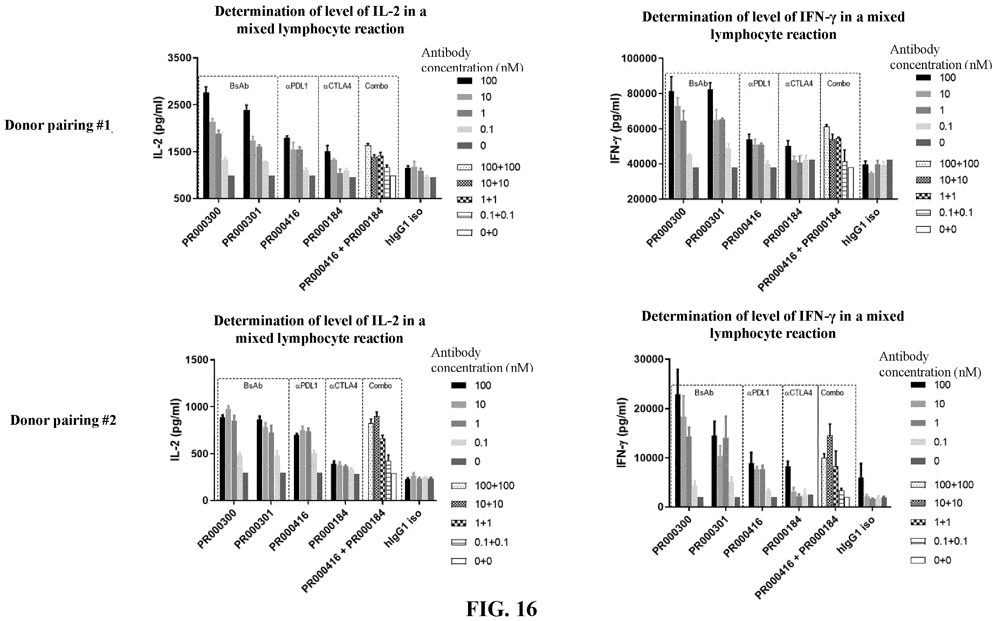

As shown in FIG. 16, in two independent MLR experiments (with different donor pairings), the bispecific antibody molecules PR000300 and PR000301 (VH at the N-terminus of the heavy or light chain of IgG) both showed stronger T cell activation than the anti-CTLA4 monoclonal antibody, anti-PD-L1 monoclonal antibody and a 1:1 concentration combination thereof, and were able to better promote the production of IL-2 and IFN-$\gamma$.

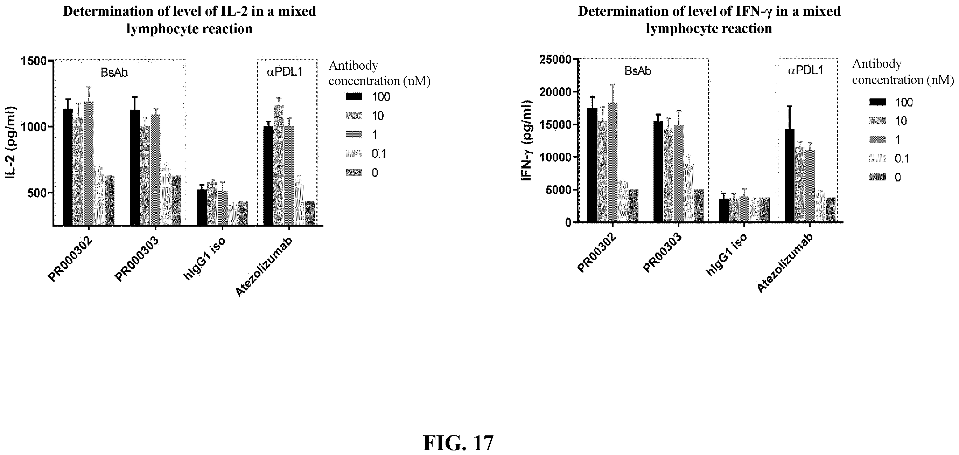

As shown in FIG. 17, when VH of anti-CTLA4 was at the C-terminus of the heavy or light chain of IgG, the bispecific antibody molecules PR000302 and PR000303 showed stronger T cell activation ability than the anti-PD-L1 monoclonal antibody atezolizumab in the MLR experiment.

### Example 3.11. Anti-tumor efficacy of bispecific antibody molecules

In this study, in order to evaluate the *in vivo* anti-tumor effect of PD-L1 $\times$ CTLA4 antibody, female B6-PD1/CTLA4 transgenic mice aged 6-8 weeks were subcutaneously inoculated with MC38-hPDL1 cells (human PD-L1 was overexpressed on MC38 cells). When the mean tumor value reached about 100 mm$^3$, tumor-bearing mice were grouped according to tumor volume, and then the antibody drug diluted with PBS at a specific concentration was administered intraperitoneally (i.p.) at a specific dose and frequency. The dosing regimen used in this example: twice a week for a total of 8 doses (BIW$\times$4 weeks). PBS was used as a blank control, 3 mg/kg PR001573 was used as a bispecific antibody administration group, and 3 mg/kg ipilimumab plus 3 mg/kg atezolizumab were used as a combination administration group. Tumor volume and body weight of mice were measured on days 6, 9, 12, 15, 19, 22, 26, 29 and 33 after tumor inoculation.

The tumor volume was calculated as follows: tumor volume (mm$^3$) = 0.5 $\times$ (long diameter of tumor $\times$ short diameter of tumor$^2$).

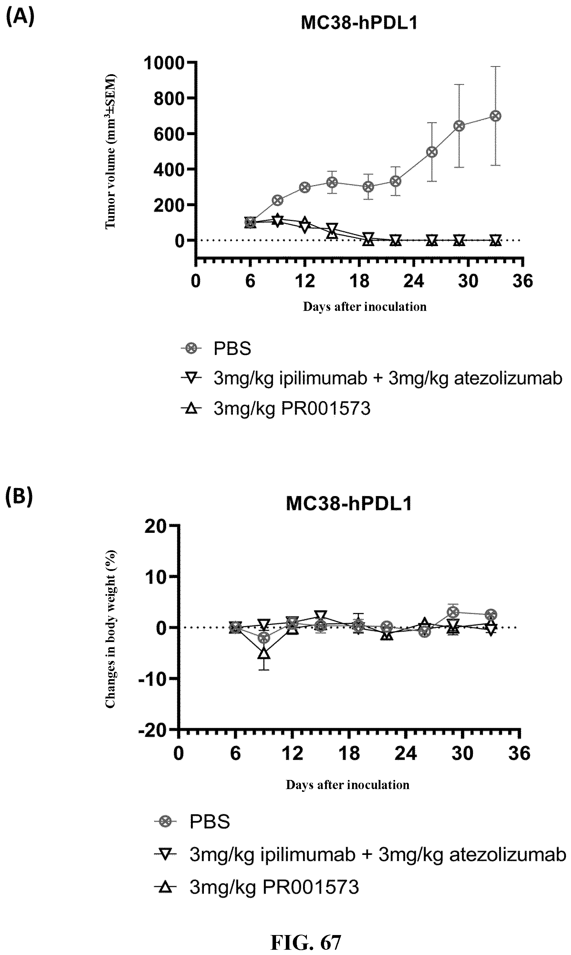

As shown in FIG. 67, the control group had a tumor volume of about 700 mm$^3$ at day 33 after tumor inoculation. The tumors of the combination administration group (3 mg/kg ipilimumab + 3mg/kg atezolizumab) completely regressed with a tumor growth inhibition (TGI) of 100%, which was significantly different from that of the control group (P = 0.03). The tumors in the bispecific antibody administration group (3 mg/kg PR001573) completely regressed with a TGI of 100%, which was significantly different from that of the control group (P = 0.03). Mice in the administration group were well tolerated throughout the experiment with no significant change in body weight. This indicates that the efficacy of 3mg/kg PR001573 is comparable to that of the combination of ipilimumab and atezolizumab at an equal dose.

### Example 3.12. Summary

In this example, PD-L1$\times$CTLA4 bispecific antibody molecules of a variety of structures were constructed using the antigen-binding domain Fab of the anti-PD-L1 IgG antibody and the antigen-binding domain VH of anti-CTLA4 HCAb antibody. This shows the flexibility of constructing a bispecific antibody molecular structure based on HCAb. By regulating the functional activity of PD-L1 and CTLA4 ends through different structure types, relative positions, binding valence and other parameters, different activity combinations are designed to meet the requirements of different dose combinations of combination drugs in the clinic.

For example, when VH of anti-CTLA4 antibody is at N-terminus of anti-PD-L1 IgG antibody, both PD-L1 end and CTLA4 end of the bispecific antibody molecules PR000300 and PR000301 can almost completely retain activity comparable to their parent monoclonal antibody, and those bispecific antibody molecules show comparable or even stronger T cell activation ability than the 1:1 concentration combination of the bispecific antibody molecule and parent monoclonal antibody in *in vitro* functional experiments such as mixed lymphocyte reaction and superantigen stimulation experiments. Therefore, this structure can be used to achieve a 1:1 dose combination of combination drugs. The mouse tumor efficacy model also proves that the bispecific antibody PR001573 of that structure has the same efficacy as the combination drugs.

For another example, when VH of anti-CTLA4 is at the C-terminus of anti-PD-L1 IgG or in Fab-HCAb structure, the PD-L1 ends of bispecific antibody molecules PR000302, PR000303 and PR000404 almost completely retain activity comparable to their parent monoclonal antibody, but the CTLA4 ends of those bispecific antibody molecules have weakened activity to different degrees. Therefore, this structure can be used to achieve a 3:1 or even 10:1 dose combination of combination drugs in the clinic.

## Example 4. HER2×CTLA4 bispecific antibodies

### Example 4.1. Background

Cytotoxic T lymphocyte-associated antigen 4 (CTLA4) is a negative regulator expressed on T cells. After binding to CD80 or CD86 on antigen presenting cells, the CTLA4 blocks the co-stimulatory signal of CD28 and simultaneously down-regulates the activity of T cells, thereby playing a role in immunosuppression. CTLA4-mediated inhibition mechanisms are often responsible for the escape of tumor cells from the immune system. By blocking the interaction between CTLA4 and its ligand, the activity of T cells can be restored, and the anti-tumor ability can be enhanced. Ipilimumab (trade name: Yervoy®) is the first anti-CTLA4 monoclonal antibody drugs approved for marketing, and is also the first product in t the era of tumor immunotherapy. Ipilimumab has a better therapeutic effect on the treatment of advanced melanoma, but also brings high immune-related side effects, with an incidence rate of the related grade 3-5 adverse reactions of up to 50%, which seriously influences its clinical application. The toxic and side effects of ipilimumab are mostly related to the CTLA4 targets, and in the current combination regimens of the PD-1/PD-L1 inhibitor and the CTLA4 inhibitor, the CTLA4 inhibitor, whether ipilimumab or tremelimumab, is usually selected at a lower dose.

In order to reduce the toxic and side effects of CTLA4 inhibitors, one of the methods worth trying is the targeted delivery of CTLA4 inhibitors into tumor tissues, so that the relevant T cell-mediated responses are limited to the tumor microenvironment, thereby reducing the risk of cytokine release syndrome. This targeted delivery can be achieved by intratumoral injection of CTLA4 inhibitors, but the intratumoral injection method involves both the risk of surgical operation and is limited to some superficial accessible tumor tissues. This example provides another targeted delivery method, in which antibodies that recognize tumor-associated antigens were used to redirect CTLA4 inhibitors into a specific tumor microenvironment where they can relieve T cell immunosuppressive signals and restore T cell function.

In this example, we constructed bispecific antibodies targeting both HER2 and CTLA4 to improve anti-tumor efficacy and safety through one or more mechanisms of action. Firstly, the HER2×CTLA4 bispecific antibody activates T cells by blocking the CTLA4 signaling pathway, while retaining the original mechanism of action of HER2 inhibitors (preventing dimerization of HER2, promoting internalization and degradation of HER2, and inhibiting downstream phosphorylation signals). Secondly, the HER2×CTLA4 bispecific antibody is enriched in tumor tissue highly expressing HER2 and can specifically relieve CTLA4 inhibition signals in a tumor microenvironment to activate T cells, so that toxic and side effects caused by non-specific activation of CTLA4 monoclonal antibodies in a peripheral system are reduced. Thirdly, the HER2×CTLA4 bispecific antibody can selectively retain Fc effector function (e.g., ADCC), thereby specifically killing inhibitory T cells highly expressing CTLA4 such as Treg cells by CTLA4 or specifically killing tumor cells highly expressing HER2 by HER2 in a tumor microenvironment. In addition, the bispecific antibody, as a drug product, is more advantageous in terms of economy and convenience of administration than the combination of two drug products.

### Example 4.2. Acquisition of anti-HER2 IgG antibodies and anti-CTLA4 HCAb antibodies

In this example, anti-HER2 IgG antibodies trastuzumab and pertuzumab were used, the corresponding amino acid sequences of which were derived from the IMGT database, see Table 4-11.

The fully human anti-CTLA4 HCAb antibody PR000184 (Table 4-11) used in this example was derived from Harbour HCAb mice, and was found as described in Example 3.2.2.

**Example 4.3. Construction of bispecific antibody molecules using anti-HER2 IgG antibodies and anti-CTLA4 HCAb antibodies**

In this example, anti-HER2×CTLA4 bispecific antibody molecules of a variety of structures were constructed using the antigen-binding domain Fab of the anti-HER2 IgG antibody PR000210 (trastuzumab analog) or PR000672 (pertuzumab analog) and the antigen-binding domain VH of the anti-CTLA4 HCAb antibody PR000184.
In this and subsequent examples, the anti-HER2 IgG monoclonal antibody PR000210 (trastuzumab analog) or PR000672 (pertuzumab analog) was used as a positive control molecule, and was also the parent monoclonal antibody of the HER2 end of the HER2×CTLA4 bispecific antibody molecule.
In this and subsequent examples, the anti-CTLA4 HCAb monoclonal antibody PR000184 was used as a positive control molecule, and was also the parent monoclonal antibody of the CTLA4 end of the HER2×CTLA4 bispecific antibody molecule.

**Example 4.3.1. Construction of molecules of Fab-HCAb symmetric structures**

HER2×CTLA4 bispecific antibody molecules of Fab-HCAb symmetric structures were designed according to the structures described in Example 1.1 using anti-HER2 IgG antibodies and anti-CTLA4 heavy-chain antibodies, with the results summarized in Table 4-1. The antibody molecule samples were prepared and analyzed according to the method described in Example 2, with the results summarized in Table 4-2.

**Table 4-1. HER2×CTLA4 bispecific antibody molecules of Fab-HCAb symmetric structures**

| Structure No. | Bispecific antibody molecules | HER2 antibody (Fab) | CTLA4 antibody (VH_B) | First linker peptide (between Fab and VH_B) | Second linker peptide (between VH_B and CH2) | Fc type (mutation) |
|---|---|---|---|---|---|---|
| 1 | PR000305 | PR000210 | PR000184 | None | Human IgG1 hinge | Human IgG1 |
| 1 | PR000653 | PR000210 | PR000184 | GS_7 | Human IgG1 hinge | Human IgG1 |
| 1 | PR000654 | PR000210 | PR000184 | GS_15 | Human IgG1 hinge | Human IgG1 |
| 1 | PR000655 | PR000210 | PR000184 | H1_15 | Human IgG1 hinge | Human IgG1 |
| 1 | PR000656 | PR000210 | PR000184 | None | Human IgG1 hinge (C220S) | Human IgG1 |
| 1 | PR000658 | PR000210 | PR000184 | GS_15 | Human IgG1 hinge (C220S) | Human IgG1 |
| 1 | PR000659 | PR000210 | PR000184 | H1_15 | Human IgG1 hinge (C220S) | Human IgG1 |
| 1 | PR000706 | PR000210 | PR000184 | GS_7 | G5-LH | Human IgG1 |
| 1 | PR000716 | PR000210 | PR000184 | GS_15 | Human IgG1 hinge (C220S) | Human IgG1 (L234A, L235A, P329G) |
| 1 | PR000717 | PR000210 | PR000184 | GS_15 | Human IgG1 hinge (C220S) | Human IgG1 (S239D, I332E) |

**Table 4-2. Expression of HER2×CTLA4 bispecific antibody molecule proteins of Fab-HCAb symmetric structures**

| Structure No. | Bispecific antibody molecules | Expression system and volume | Plasmid transfection ratio (short chain:long chain) | Yield (mg/L) after first purification | SEC-HPLC purity (%) |
|---|---|---|---|---|---|
| 1 | PR000305 | HEK293-F (30ml) | 2:3 | 70.00 | 87.17 |

(continued)

| Structure No. | Bispecific antibody molecules | Expression system and volume | Plasmid transfection ratio (short chain:long chain) | Yield (mg/L) after first purification | SEC-HPLC purity (%) |
|---|---|---|---|---|---|
| 1 | PR000653 | HEK293-F (30ml) | 3 : 2 | 69.85 | 100.00 |
| 1 | PR000654 | HEK293-F (30ml) | 3 : 2 | 62.87 | 100.00 |
| 1 | PR000655 | HEK293-F (30ml) | 3 : 2 | 102.14 | 97.82 |
| 1 | PR000656 | HEK293-F (30ml) | 3 : 2 | 79.67 | 99.52 |
| 1 | PR000658 | HEK293-F (30ml) | 3 : 2 | 77.33 | 97.10 |
| 1 | PR000659 | HEK293-F (30ml) | 3 : 2 | 86.88 | 95.18 |
| 1 | PR000706 | HEK293-F (30ml) | 3 : 2 | 40.87 | 100.00 |
| 1 | PR000716 | HEK293-F (30ml) | 3 : 2 | 54.39 | 100.00 |
| 1 | PR000717 | HEK293-F (30ml) | 3 : 2 | 38.33 | 100.00 |

**Example 4.3.2. Construction of molecules of IgG-VH tetravalent symmetric structure**

HER2×CTLA4 bispecific antibody molecules of IgG-VH tetravalent symmetric structures were designed according to the structures described in Example 1.2 using anti-HER2 IgG antibodies and anti-CTLA4 heavy-chain antibodies, with the results summarized in Table 4-3. The antibody molecule samples were prepared and analyzed according to the method described in Example 2, with the results summarized in Table 4-4.

**Table 4-3. HER2×CTLA4 bispecific antibody molecules of IgG-VH tetravalent symmetric structures**

| Structure No. | Bispecific antibody molecules | HER2 antibody (IgG) | CTLA4 antibody (VH_B) | VH_B position relative to IgG | Linker peptide | Fc type (mutation) |
|---|---|---|---|---|---|---|
| 5 | PR000539 | PR000210 | PR000184 | C-terminus of heavy chain | L-GS_15-RT | Human IgG1 |
| 5 | PR000540 | PR000210 | PR000184 | C-terminus of heavy chain | L-H1_15-RT | Human IgG1 |
| 5 | PR000714 | PR000210 | PR000184 | C-terminus of heavy chain | L-GS_15-RT | Human IgG1 (L234A, L235A, P329G) |
| 5 | PR000715 | PR000210 | PR000184 | C-terminus of heavy chain | L-GS_15-RT | Human IgG1 (S239D, I332E) |
| 3 | PR001583 | PR000210 | PR000184 | N-terminus of heavy chain | KL-H1_15-RT | Human IgG1 |
| 4 | PR001584 | PR000210 | PR000184 | N-terminus of light chain | KL-H1_15-AS | Human IgG1 |
| 3 | PR001586 | PR000210 | PR000184 | N-terminus of heavy chain | KL-H1_15-RT | Human IgG1 (S239D, I332E) |
| 4 | PR001974 | PR000210 | PR000184 | N-terminus of light chain | KL-H1_15-AS | Human IgG1 (S239D, I332E) |
| 3 | PR001588 | PR000672 | PR000184 | N-terminus of heavy chain | KL-H1_15-RT | Human IgG1 |
| 4 | PR001589 | PR000672 | PR000184 | N-terminus of light chain | KL-H1_15-AS | Human IgG1 |

(continued)

| Structure No. | Bispecific antibody molecules | HER2 antibody (IgG) | CTLA4 antibody (VH_B) | VH_B position relative to IgG | Linker peptide | Fc type (mutation) |
|---|---|---|---|---|---|---|
| 3 | PR001591 | PR000672 | PR000184 | N-terminus of heavy chain | KL-H1_15-RT | Human IgG1 (S239D, I332E) |

**Table 4-4. Expression of HER2×CTLA4 bispecific antibody molecule proteins of IgG-VH tetravalent symmetric structures**

| Structure No. | Bispecific antibody molecules | Expression system and volume | Yield (mg/L) after first purification | SEC-HPLC purity (%) |
|---|---|---|---|---|
| 5 | PR000539 | HEK293-F (30ml) | 188.33 | 100.00 |
| 5 | PR000540 | HEK293-F (30ml) | 261.33 | 99.94 |
| 5 | PR000714 | HEK293-F (30ml) | 34.33 | 98.21 |
| 5 | PR000715 | HEK293-F (30ml) | 32.00 | 99.05 |
| 3 | PR001583 | HEK293-F (40ml) | 153.25 | 96.07 |
| 4 | PR001584 | HEK293-F (40ml) | 147.25 | 95.15 |
| 3 | PR001586 | HEK293-F (40ml) | 112.50 | 96.26 |
| 4 | PR001974 | HEK293-F (40ml) | 75.00 | 100 |
| 3 | PR001588 | HEK293-F (50ml) | 35.21 | 100 |
| 4 | PR001589 | HEK293-F (50ml) | 39.45 | 100 |
| 3 | PR001591 | HEK293-F (50ml) | 25.94 | 100 |

**Example 4.3.3. Construction of molecules of IgG-VH(2) hexavalent symmetric structures**

HER2×CTLA4 bispecific antibody molecules of IgG-VH(2) hexavalent symmetric structures were designed according to the structures described in Example 1.3 using anti-HER2 IgG antibodies and anti-CTLA4 heavy-chain antibodies, with the results summarized in Table 4-5. The antibody molecule samples were prepared and analyzed according to the method described in Example 2, with the results summarized in Table 4-6.

**Table 4-5. HER2×CTLA4 bispecific antibody molecules of IgG-VH(2) hexavalent symmetric structures**

| Structure No. | Bispecific antibody molecules | HER2 antibody (IgG) | CTLA4 antibody (VH_B) | First linker peptide (between Fc and VH_B) | Second linker peptide (between VH_B and VH_B) | Fc type (mutation) |
|---|---|---|---|---|---|---|
| 7 | PR000541 | PR000210 | PR000184 | L-GS_15-RT | GS_5 | Human IgG1 |
| 7 | PR000542 | PR000210 | PR000184 | L-H1_15-RT | GS_5 | Human IgG1 |
| 7 | PR001579 | PR000210 | PR000184 | L-H1_15-RT | GS_15 | Human IgG1 |
| 7 | PR001580 | PR000210 | PR000184 | L-H1_15-RT | GS_20 | Human IgG1 |
| 7 | PR001581 | PR000210 | PR000184 | L-H1_15-RT | H1_15 | Human IgG1 |

(continued)

| Structure No. | Bispecific antibody molecules | HER2 antibody (IgG) | CTLA4 antibody (VH_B) | First linker peptide (between Fc and VH_B) | Second linker peptide (between VH_B and VH_B) | Fc type (mutation) |
|---|---|---|---|---|---|---|
| 7 | PR001582 | PR000210 | PR000184 | GS_15 | GS_5 | Human IgG1 (S239D, I332E) |

**Table 4-6. Expression of HER2×CTLA4 bispecific antibody molecule proteins of IgG-VH(2) hexavalent symmetric structures**

| Structure No. | Bispecific antibody molecules | Expression system and volume | Yield (mg/L) after first purification | SEC-HPLC purity (%) |
|---|---|---|---|---|
| 7 | PR000541 | HEK293-F (30ml) | 66 | 98.75 |
| 7 | PR000542 | HEK293-F (30ml) | 125 | 99.83 |
| 7 | PR001579 | HEK293-F (40ml) | 127 | 98.23 |
| 7 | PR001580 | HEK293-F (40ml) | 120 | 98.04 |
| 7 | PR001581 | HEK293-F (40ml) | 140 | 98.18 |
| 7 | PR001582 | HEK293-F (40ml) | 39.5 | 98.19 |

**Example 4.3.4. Construction of molecules of 2×VH-IgG hexavalent symmetric structures**

HER2×CTLA4 bispecific antibody molecules of 2×VH-IgG hexavalent symmetric structures were designed according to the structures described in Example 1.4 using anti-HER2 IgG antibodies and anti-CTLA4 heavy-chain antibodies, with the results summarized in Table 4-7. The antibody molecule samples were prepared and analyzed according to the method described in Example 2, with the results summarized in Table 4-8.

**Table 4-7. HER2×CTLA4 bispecific antibody molecules of 2×VH-IgG hexavalent symmetric structures**

| Structure No. | Bispecific antibody molecules | HER2 antibody (IgG) | CTLA4 antibody (VH_B) | First linker peptide (between VH_B and VL_A) | Second linker peptide (between VH_B and VH_A) | Fc type (mutation) |
|---|---|---|---|---|---|---|
| 9 | PR001585 | PR000210 | PR000184 | KL-H1_15-AS | KL-H1_15-RT | Human IgG1 |
| 9 | PR001587 | PR000210 | PR000184 | KL-H1_15-AS | KL-H1_15-RT | Human IgG1 (S239D, I332E) |
| 9 | PR001590 | PR000672 | PR000184 | KL-H1_15-AS | KL-H1_15-RT | Human IgG1 |
| 9 | PR001592 | PR000672 | PR000184 | KL-H1_15-AS | KL-H1_15-RT | Human IgG1 (S239D, I332E) |

**Table 4-8. Expression of HER2×CTLA4 bispecific antibody molecule proteins of 2×VH-IgG hexavalent symmetric structures**

| Structure No. | Bispecific antibody molecules | Expression system and volume | Yield (mg/L) after first purification | SEC-HPLC purity (%) |
|---|---|---|---|---|
| 9 | PR001585 | HEK293-F (50ml) | 27.6 | 100 |
| 9 | PR001587 | HEK293-F (50ml) | 19.8 | 100 |
| 9 | PR001590 | HEK293-F (50ml) | 8.2 | n.d. |
| 9 | PR001592 | HEK293-F (50ml) | 6.4 | n.d. |

**Example 4.3.5. Construction of molecules of Fab-Fc-VH(n) bivalent or trivalent asymmetric structures**

HER2×CTLA4 bispecific antibody molecules of Fab-Fc-VH(n, n = {1, 2}) asymmetric structures were designed according to the structures described in Example 1.5 and Example 1.6 using anti-HER2 IgG antibodies and anti-CTLA4 heavy-chain antibodies, with the results summarized in Table 4-9. The antibody molecule samples were prepared and analyzed according to the method described in Example 2, with the results summarized in Table 4-10.

**Table 4-9. HER2×CTLA4 bispecific antibody molecules of Fab-Fc-VH(n) asymmetric structures**

| Structure No. | Bispecific antibody molecules | HER2 antibody (Fab) | CTLA4 antibody (VH_B) | Structure of Fab end | Number of repetition for VH_B | Linker peptide | Fc type of Fab end (mutation) | Fc type of VH_B end (mutation) |
|---|---|---|---|---|---|---|---|---|
| 11 | PR000916 | PR000210 | PR000184 | Normal | 1 | None | Human IgG1 (knob) | Human IgG1 (hole) |
| 11 | PR000917 | PR000210 | PR000184 | Normal | 1 | None | Human IgG1 (knob, DE) | Human IgG1 (hole, DE) |
| 14 | PR002666 | PR000210 | PR000184 | Normal | 2 | GS_5 | Human IgG1 (knob) | Human IgG1 (hole) |
| 14 | PR002667 | PR000210 | PR000184 | Normal | 2 | GS_15 | Human IgG1 (knob) | Human IgG1 (hole) |
| 14 | PR002668 | PR000210 | PR000184 | Normal | 2 | GS_5 | Human IgG1 (knob, DE) | Human IgG1 (hole, DE) |
| 14 | PR002669 | PR000210 | PR000184 | Normal | 2 | GS_15 | Human IgG1 (knob, DE) | Human IgG1 (hole, DE) |
| 18 | PR002670 | PR000210 | PR000184 | cross Fd/LC | 2 | GS_5 | Human IgG1 (knob) | Human IgG1 (hole) |
| 18 | PR002671 | PR000210 | PR000184 | cross Fd/LC | 2 | GS_15 | Human IgG1 (knob) | Human IgG1 (hole) |
| 18 | PR002672 | PR000210 | PR000184 | cross Fd/LC | 2 | GS_5 | Human IgG1 (knob, DE) | Human IgG1 (hole, DE) |
| 18 | PR002673 | PR000210 | PR000184 | cross Fd/LC | 2 | GS_15 | Human IgG1 (knob, DE) | Human IgG1 (hole, DE) |

(mutation code: **Knob:** S354C, T366W; **Hole:** Y349C, T366S, L368A, Y407V; **DE:** S239D, I332E.)

**Table 4-10. Expression of HER2×CTLA4 bispecific antibody molecule proteins of Fab-Fc-VH(n) asymmetric structures**

| Structure No. | Bispecific antibody molecules | Expression system and volume | Plasmid transfection ratio (chain 2:3:1) | Yield (mg/L) after first purification | SEC-HPLC purity (%) |
|---|---|---|---|---|---|
| 11 | PR000916 | HEK293-F (50ml) | 1 : 1 : 1 | 30.4 | 98.54 |
| 11 | PR000917 | HEK293-F (50ml) | 1 : 1 : 1 | 13.9 | 97.62 |
| 14 | PR002666 | HEK293-F (40ml) | 3 : 2 : 5 | 147.8 | 79.59 |
| 14 | PR002667 | HEK293-F (40ml) | 3 : 2 : 5 | 196.5 | 79.31 |
| 14 | PR002668 | HEK293-F (40ml) | 3 : 2 : 5 | 10.8 | 82.76 |
| 14 | PR002669 | HEK293-F (40ml) | 3 : 2 : 5 | 35.5 | 70 |
| 18 | PR002670 | HEK293-F (40ml) | 3 : 2 : 5 | 94.8 | 74.19 |
| 18 | PR002671 | HEK293-F (40ml) | 3 : 2 : 5 | 150 | 76.19 |
| 18 | PR002672 | HEK293-F (40ml) | 3 : 2 : 5 | 3 | 84.9 |
| 18 | PR002673 | HEK293-F (40ml) | 3 : 2 : 5 | 8 | 81.31 |

**Example 4.3.6. Sequences of HER2×CTLA4 bispecific antibody molecules and control molecules**

The sequence numbers corresponding to the sequences of the HER2×CTLA4 bispecific antibody molecules constructed in this example, the corresponding parent monoclonal antibody molecules such as the HER2 monoclonal antibody and the CTLA4 monoclonal antibody, and the control molecules are listed in Table 4-11, Table 4-12 and Table 4-13. The structure numbers in Table 4-13 correspond to those in Table 1-1 and FIG. 1.The sequence numbers of the corresponding CDR sequences of the first and second antigen-binding domains of the bispecific antibody molecules are listed in Table 4-14.

**Table 4-11. Control molecules and parent monoclonal antibodies**

| Antibody No. | Antibody |
|---|---|
| PR000210 | Anti-HER2 monoclonal antibody trastuzumab analog, hIgG1 |
| PR000672 | Anti-HER2 monoclonal antibody pertuzumab analog, hIgG1 |
| PR000184 | Anti-CTLA4 heavy-chain antibody CL5v3, hIgG1 |
| PR000218 | Anti-CTLA4 heavy-chain antibody CL5v3, hIgG1(S239D, I332E) |

**Table 4-12. Sequence numbers of sequences and CDR sequences of control molecules and parent monoclonal antibodies**

| Antibody No. | Light chain | Heavy chain | VL | VH | LCDR1 | LCDR2 | LCDR3 | HCDR1 | HCDR2 | HCDR3 |
|---|---|---|---|---|---|---|---|---|---|---|
| PR000210 | 351 | 305 | 286 | 238 | 171 | 190 | 215 | 19 | 67 | 127 |
| PR000672 | 359 | 315 | 293 | 247 | 176 | 196 | 220 | 23 | 74 | 132 |
| PR000184 | None | 303 | None | 236 | None | None | None | 17 | 65 | 125 |
| PR000218 | None | 306 | None | 236 | None | None | None | 17 | 65 | 125 |

**Table 4-13. Sequence numbers of HER2×CTLA4 bispecific antibody molecules of this example**

| Structure No. | Antibody No. | Polypeptide chain 1 | Polypeptide chain 2 | Polypeptide chain 3 |
|---|---|---|---|---|
| 1 | PR000305 | 367 | 368 | None |
| 5 | PR000539 | 351 | 374 | None |
| 5 | PR000540 | 351 | 375 | None |
| 7 | PR000541 | 351 | 376 | None |
| 7 | PR000542 | 351 | 377 | None |
| 1 | PR000653 | 367 | 378 | None |
| 1 | PR000654 | 367 | 379 | None |
| 1 | PR000655 | 367 | 380 | None |
| 1 | PR000656 | 367 | 381 | None |
| 1 | PR000658 | 367 | 382 | None |
| 1 | PR000659 | 367 | 383 | None |
| 1 | PR000706 | 367 | 385 | None |
| 5 | PR000714 | 351 | 386 | None |
| 5 | PR000715 | 351 | 387 | None |
| 1 | PR000716 | 367 | 388 | None |
| 1 | PR000717 | 367 | 389 | None |
| 11 | PR000916 | 351 | 391 | 390 |
| 11 | PR000917 | 351 | 393 | 392 |
| 7 | PR001579 | 351 | 397 | None |
| 7 | PR001580 | 351 | 398 | None |
| 7 | PR001581 | 351 | 399 | None |
| 7 | PR001582 | 351 | 400 | None |
| 3 | PR001583 | 351 | 401 | None |
| 4 | PR001584 | 402 | 305 | None |
| 9 | PR001585 | 402 | 401 | None |
| 3 | PR001586 | 351 | 403 | None |
| 9 | PR001587 | 402 | 403 | None |
| 3 | PR001588 | 359 | 404 | None |
| 4 | PR001589 | 405 | 315 | None |
| 9 | PR001590 | 405 | 404 | None |
| 3 | PR001591 | 359 | 406 | None |
| 9 | PR001592 | 405 | 406 | None |
| 4 | PR001974 | 402 | 409 | None |
| 14 | PR002666 | 351 | 391 | 434 |
| 14 | PR002667 | 351 | 391 | 435 |
| 14 | PR002668 | 351 | 393 | 436 |
| 14 | PR002669 | 351 | 393 | 437 |
| 18 | PR002670 | 367 | 438 | 434 |
| 18 | PR002671 | 367 | 438 | 435 |

(continued)

| Structure No. | Antibody No. | Polypeptide chain 1 | Polypeptide chain 2 | Polypeptide chain 3 |
|---|---|---|---|---|
| 18 | PR002672 | 367 | 439 | 436 |
| 18 | PR002673 | 367 | 439 | 437 |

**Table 4-14. Sequence numbers of CDRs of antigen-binding domains of HER2×CTLA4 bispecific antibody molecules**

| Structure No. | Antibody No. | Antigen-binding domain No. | LCDR1 | LCDR2 | LCDR3 | HCDR1 | HCDR2 | HCDR3 |
|---|---|---|---|---|---|---|---|---|
| 1 | PR000305, PR000653, PR000654, PR000655, PR000656, PR000658, PR000659, PR000706, PR000716, PR000717 | #1 | 171 | 190 | 215 | 19 | 67 | 127 |
| | | #2 | None | None | None | 17 | 65 | 125 |
| 3 | PR001583, PR001586 | #1 | 171 | 190 | 215 | 19 | 67 | 127 |
| | | #2 | None | None | None | 17 | 65 | 125 |
| 3 | PR001588, PR001591 | #1 | 176 | 196 | 220 | 23 | 74 | 132 |
| | | #2 | None | None | None | 17 | 65 | 125 |
| 4 | PR001584, PR001974 | #1 | 171 | 190 | 215 | 19 | 67 | 127 |
| | | #2 | None | None | None | 17 | 65 | 125 |
| 4 | PR001589 | #1 | 176 | 196 | 220 | 23 | 74 | 132 |
| | | #2 | None | None | None | 17 | 65 | 125 |
| 5 | PR000539, PR000540, PR000714, PR000715 | #1 | 171 | 190 | 215 | 19 | 67 | 127 |
| | | #2 | None | None | None | 17 | 65 | 125 |
| 7 | PR000541, PR000542, PR001579, PR001580, PR001581, PR001582 | #1 | 171 | 190 | 215 | 19 | 67 | 127 |
| | | #2 | None | None | None | 17 | 65 | 125 |
| 9 | PR001585, PR001587 | #1 | 171 | 190 | 215 | 19 | 67 | 127 |
| | | #2 | None | None | None | 17 | 65 | 125 |
| 9 | PR001590, PR001592 | #1 | 176 | 196 | 220 | 23 | 74 | 132 |
| | | #2 | None | None | None | 17 | 65 | 125 |
| 11 | PR000916, PR000917 | #1 | 171 | 190 | 215 | 19 | 67 | 127 |
| | | #2 | None | None | None | 17 | 65 | 125 |

(continued)

| Structure No. | Antibody No. | Antigen-binding domain No. | LCDR1 | LCDR2 | LCDR3 | HCDR1 | HCDR2 | HCDR3 |
|---|---|---|---|---|---|---|---|---|
| 14 | PR002666, PR002667, PR002668, PR002669 | #1 | 171 | 190 | 215 | 19 | 67 | 127 |
| | | #2 | None | None | None | 17 | 65 | 125 |
| 18 | PR002670, PR002671, PR002672, PR002673 | #1 | 171 | 190 | 215 | 19 | 67 | 127 |
| | | #2 | None | None | None | 17 | 65 | 125 |

**Example 4.4. Binding to CTLA4**

This example is intended to investigate the binding activity of the HER2×CTLA4 bispecific antibody molecules to CTLA4.

**Example 4.4.1. Binding to CHO-K1 cells CHO-K1/hCTLA4 highly expressing human CTLA4**

The binding ability of the antibody molecules to a CHO-K1 cell line CHO-K1/hCTLA4 cells (ChemPartner) and other cells highly expressing human CTLA4 was determined by flow cytometry FACS. Specifically, CHO-K1/hCTLA4 cells were digested and resuspended in an F12K medium, with the cell density adjusted to $2\times10^6$ cells/mL. Thereafter, the CHO-K1/hCTLA4 cells were seeded in a 96-well V-bottom plate (Corning, #3894) at 100 μL/well and centrifuged at 4 °C for 5 min, and the supernatant was discarded. Then, the antibody molecules at a total of 8 concentrations obtained by a 5-fold gradient dilution from the highest final concentration of 300 nM, or a total of 12 concentrations obtained by a 4-fold gradient dilution from the highest final concentration of 300 nM were added to the 96-well plate at 100 μL/well, and the mixture was mixed well. hIgG1 iso (CrownBio, #C0001) was used as an isotype control. The cells were incubated at 4 °C for 1 h away from light. Then, the cells in each well were rinsed twice with 100 μL of pre-cooled FACS buffer (PBS buffer containing 0.5% BSA) and centrifuged at 500 g at 4 °C for 5 min, and then the supernatant was discarded. Thereafter, a fluorescent secondary antibody (Goat human IgG (H + L) Alexa Fluor 488 conjugation, Thermo, #A11013, 1:1000 dilution) was added at 100 μL/well, and the plate was incubated at 4 °C for 1 h away from light. Then, the cells in each well were then rinsed twice with 200 μL of pre-cooled FACS buffer and centrifuged at 500 g at 4 °C for 5 min, and then the supernatant was discarded. Finally, a pre-cooled FACS buffer was added at 200 μL/well to resuspend the cells. Fluorescence signal values were read using a BD FACS CANTOII flow cytometer or an ACEA NovoCyte flow cytometer, and the data were processed and analyzed using FlowJo v10 (FlowJo, LLC) software. The data were processed and analyzed by plotting using GraphPad Prism 8 software, and binding curves of the antibodies to target cells, $EC_{50}$ values and other parameters were obtained through four-parameter nonlinear fitting.

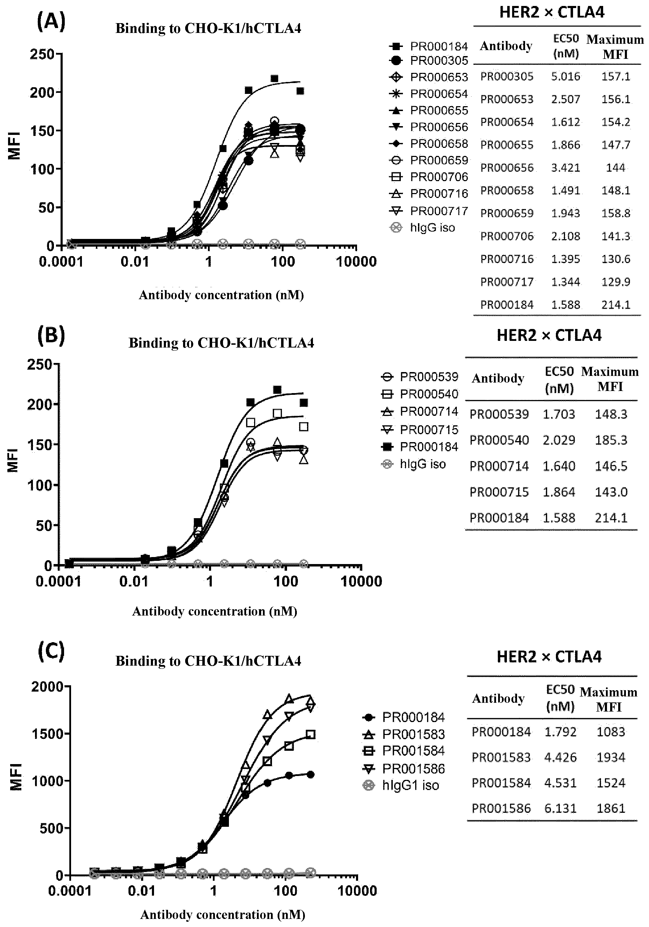

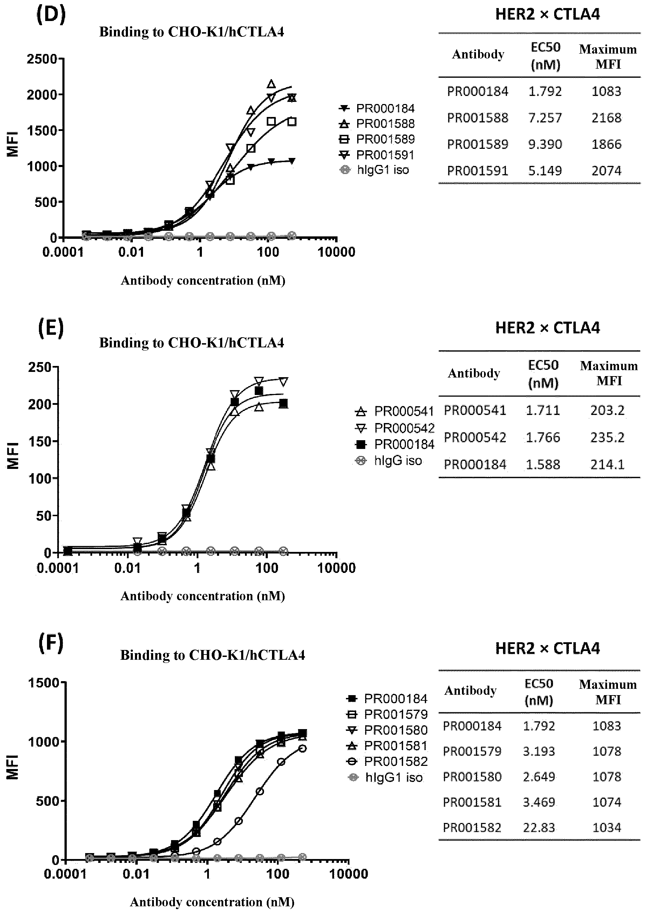

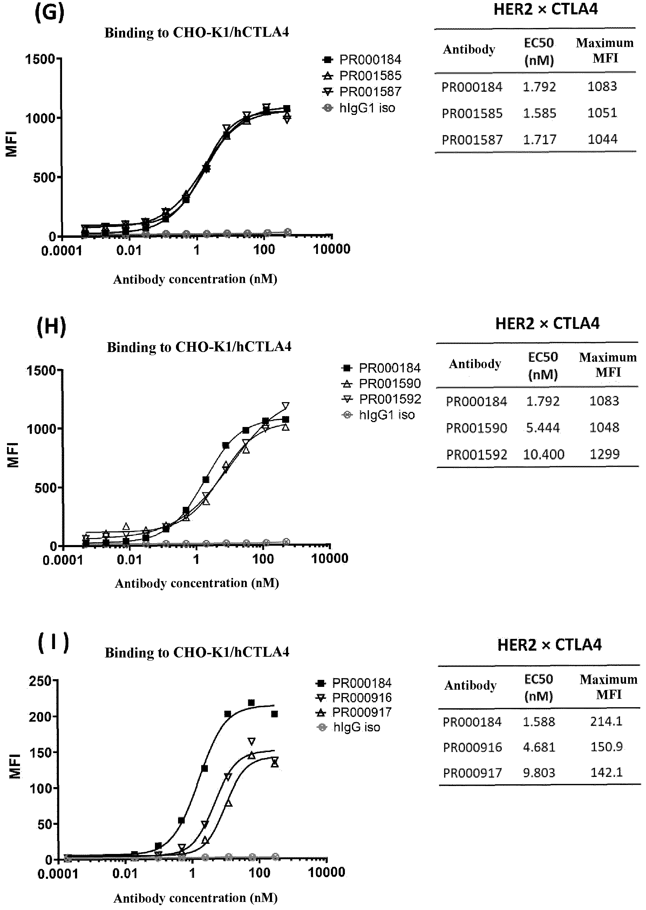

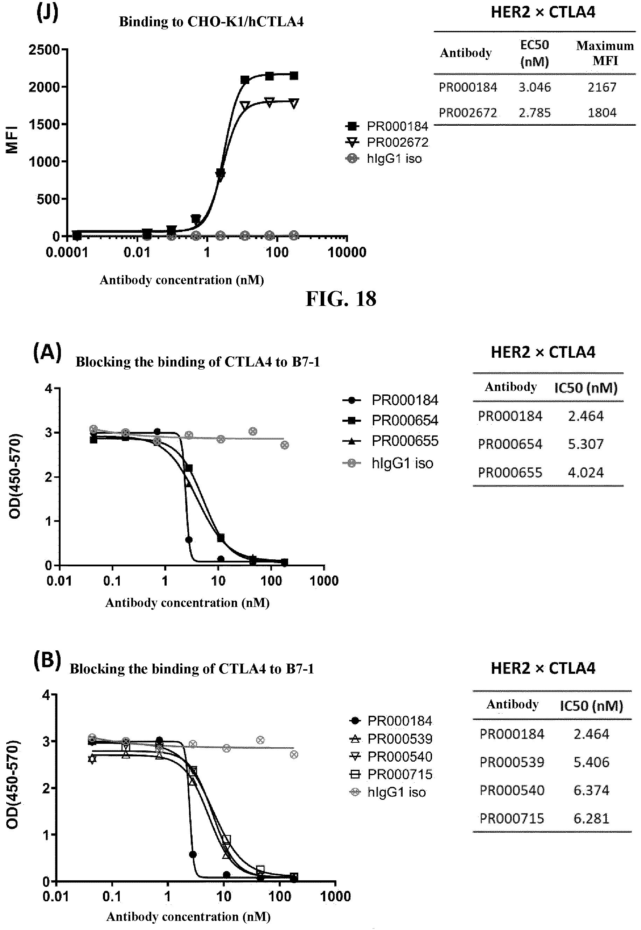

In this example, the anti-CTLA4 HCAb monoclonal antibody PR000184 was used as a positive control molecule, and was also the parent monoclonal antibody of the CTLA4 end of the HER2×CTLA4 bispecific antibody molecule. The results are shown in FIG. 18 and Table 4-15.

As shown in FIG. 18 (A), the bispecific antibody molecules of Fab-HCAb symmetric structures were all able to bind to CTLA4. Those molecules had similar structures and were all composed of the Fab domain of anti-HER2 monoclonal antibody PR000210 and the VH domain of anti-CTLA4 HCAb monoclonal antibody PR000184, with minor differences in the different first linker peptides and hinge regions linking Fc, and different Fc types or mutations. Thus, those bispecific antibody molecules had similar binding ability to CTLA4. They had $EC_{50}$ values for binding to CTLA4 that were similar to or 1.5-3 times poorer than the parent monoclonal antibody PR000184, but had lower maximum binding signals (maximum MFIs) on FACS than the parent monoclonal antibody PR000184. This may suggest that in a Fab-HCAb structure, the Fab domain may have a "masking" effect on the VH domain of HCAb, so that the VH domain in this structure is able to bind to less CTLA4 molecules than it would be at the free end, but its binding potency is comparable to that of the parental monoclonal antibody PR000184 ($EC_{50}$ value) for the same number of available CTLA4 molecules.

As shown in FIG. 18 (B-D), the bispecific antibody molecules of IgG-VH tetravalent symmetric structures had slightly different binding ability to CTLA4 according to their specific molecular structures. The bispecific antibody molecules of such structures were further classified into three classes according to the position of the VH end of anti-CTLA4 relative to anti-HER2 IgG in the bispecific antibody structure: VH at the C-terminus of the heavy chain of IgG (PR000539, PR000540, PR000714, and PR000715); VH at the N-terminus of the heavy chain of IgG (PR001583, PR001586, PR001588, and PR001591); and VH at the N-terminus of the light chain of IgG (PR001584 and PR001589). When VH was at the C-terminus of the heavy chain of IgG, those bispecific antibody molecules had similar $EC_{50}$ values for binding to CTLA4 to the parent monoclonal antibody PR000184, but had slightly lower maximum MFIs on FACS than the parent monoclonal antibody PR000184. When VH was at the N-terminus of either the heavy or light chain of IgG, those bispecific antibody molecules had slightly poorer $EC_{50}$ values for binding to CTLA4 than the parent monoclonal antibody PR000184, but had superior maximum MFIs on FACS than the parent monoclonal antibody PR000184. This indicates that the number and binding potency of VH-binding target molecules can be modulated by adjusting the relative position of VH on IgG.

As shown in FIG. 18 (E-F), the bispecific antibody molecules of IgG-VH-VH hexavalent symmetric structures were all able to bind to CTLA4. Those molecules have similar structures and were all composed of anti-HER2 monoclonal antibody PR000210 and the VH domain of anti-CTLA4 HCAb monoclonal antibody PR000184, with minor differences in the different first linker peptides and second linker peptides, and different Fc types or mutations. With the exception of PR001582, those bispecific antibody molecules had similar binding ability to CTLA4 to the parental monoclonal antibody PR000184 (similar $EC_{50}$ values and similar maximum MFIs).

As shown in FIG. 18 (G-H), the bispecific antibody molecules of $2\times$VH-IgG hexavalent symmetric structures were all able to bind to CTLA4. Those molecules had similar structures and were all composed of anti-HER2 monoclonal antibody PR000210 or PR000672 and the VH domain of anti-CTLA4 HCAb monoclonal antibody PR000184. The bispecific antibody molecules (PR001585 and PR001587) constructed based on the parent monoclonal antibody PR000210 had similar binding ability to CTLA4 to the parent monoclonal antibody PR000184; and the bispecific antibody molecules (PR001590 and PR001592) constructed based on the parent monoclonal antibody PR000672 had slightly weaker binding ability to CTLA4 than the parent monoclonal antibody PR000184.

As shown in FIG. 18 (I-J), the binding ability of the bispecific antibody molecules of Fab-Fc-VH (n, n = 1, 2) asymmetric structures to CTLA4 was related to the number of VH domains of the parent monoclonal antibody PR000184 contained in the structure. The bispecific antibody molecules PR000916 and PR000917 both contained only one CTLA4-binding domain, so that their binding ability to CTLA4 was significantly weaker than that of the bivalent parent monoclonal antibody PR000184. The bispecific antibody molecule PR002672 contained two CTLA4-binding domains formed in tandem, and its binding ability to CTLA4 was similar to that of the parent monoclonal antibody PR000184. This structure shows that bispecific antibody molecules containing the VH domain derived from HCAb have such flexibility that: the binding ability to the target is modulated by adjusting the number of VH domains in tandem.

**Table 4-15. Binding to CHO-K1/hCTLA4**

| Antibody | $EC_{50}$ (nM) | Maximum MFI | Antibody | $EC_{50}$ (nM) | Maximum MFI | Antibody | $EC_{50}$ (nM) | Maximum MFI |
|---|---|---|---|---|---|---|---|---|
| PR000184 | 1.588 | 214.1 | PR000184 | 1.792 | 1083 | PR000184 | 3.046 | 2167 |
| PR000305 | 5.016 | 157.1 | PR001583 | 4.426 | 1934 | PR002672 | 2.785 | 1804 |
| PR000653 | 2.507 | 156.1 | PR001584 | 4.531 | 1524 | | | |
| PR000654 | 1.612 | 154.2 | PR001586 | 6.131 | 1861 | | | |
| PR000655 | 1.866 | 147.7 | PR001588 | 7.257 | 2168 | | | |
| PR000656 | 3.421 | 144 | PR001589 | 939 | 1866 | | | |
| PR000658 | 1.491 | 148.1 | PR001591 | 5.149 | 2074 | | | |
| PR000659 | 1.943 | 158.8 | PR001579 | 3.193 | 1078 | | | |
| PR000706 | 2.108 | 141.3 | PR001580 | 2.649 | 1078 | | | |
| PR000716 | 1.395 | 130.6 | PR001581 | 3.469 | 1074 | | | |
| PR000717 | 1.344 | 129.9 | PR001582 | 22.83 | 1034 | | | |
| PR000539 | 1.703 | 148.3 | PR001585 | 1.585 | 1051 | | | |
| PR000540 | 2.029 | 185.3 | PR001587 | 1.717 | 1044 | | | |
| PR000714 | 1.64 | 146.5 | PR001590 | 5.444 | 1048 | | | |
| PR000715 | 1.864 | 143 | PR001592 | 10.4 | 1299 | | | |
| PR000541 | 1.711 | 203.2 | | | | | | |
| PR000542 | 1.766 | 235.2 | | | | | | |

(continued)

| Antibody | EC$_{50}$ (nM) | Maximum MFI | Antibody | EC$_{50}$ (nM) | Maximum MFI | Antibody | EC$_{50}$ (nM) | Maximum MFI |
|---|---|---|---|---|---|---|---|---|
| PR000916 | 4.681 | 150.9 | | | | | | |
| PR000917 | 9.803 | 142.1 | | | | | | |
| Experiment 1 | | | Experiment 2 | | | Experiment 3 | | |

## Example 4.5. Blocking the binding of CTLA4 to its ligand

This example is intended to investigate the inhibitory activity of the HER2×CTLA4 bispecific antibody molecules for the binding of CTLA4 to its ligand B7-1/CD80.

## Example 4.5.1. Blocking the binding of human CTLA4 protein to its ligand protein

The inhibitory activity of the antibody molecules for the binding of human CTLA4 protein to its ligand B7-1/CD80 protein was determined by enzyme-linked immunosorbent assay (ELISA). Specifically, a 96-well plate was firstly coated with 2 μg/mL protein human B7-1-Fc (ACRO Biosystems, #B71-H5259) at 100 μL/well and placed at 4 °C overnight. The plate was then rinsed 3 times with a PBST buffer (a PBS buffer containing 0.05% Tween-20), added with a blocking buffer (a PBS buffer containing 5% skim milk powder), and incubated at 37 °C for 1 h. Then, the antibody molecules at a total of 7 concentrations obtained by a 3-fold gradient dilution with the highest final concentration of 200 nM were added at 90 μL/well. The mixture was mixed well and incubated at 37 °C for 20 min. hIgG1 iso (CrownBio, #C0001) was used as an isotype control. Then, biotinylated human CTLA4-Fc protein (ACRO Biosystems, # CT4-H82F3) was added at 10 μL/well to make a final concentration of 0.25 μg/mL, and the mixture was incubated at 37 °C for 1 h. The plate was then rinsed 3 times with a PBST buffer. Then, a label Precision Protein™ StrepTactin-HRP conjugate (Bio-RAD, #1610380, diluted in a 1:4000 ratio) was added at 100 μL/well, and the mixture was incubated at 37 °C for 0.5 h. The plate was then rinsed 3 times with a PBST buffer. Then, TMB chromogenic solution was added at 100 μL/well for reaction for 15 min. Finally, a stop buffer was added to stop the reaction. The absorbance value (OD value) was read at 490 nM using an Enspire™ multifunctional microplate reader (Perkin Elmer, Inc.). The data were processed and analyzed by plotting using GraphPad Prism 8 software, and inhibition curves, IC$_{50}$ values and other parameters were obtained through four-parameter nonlinear fitting.

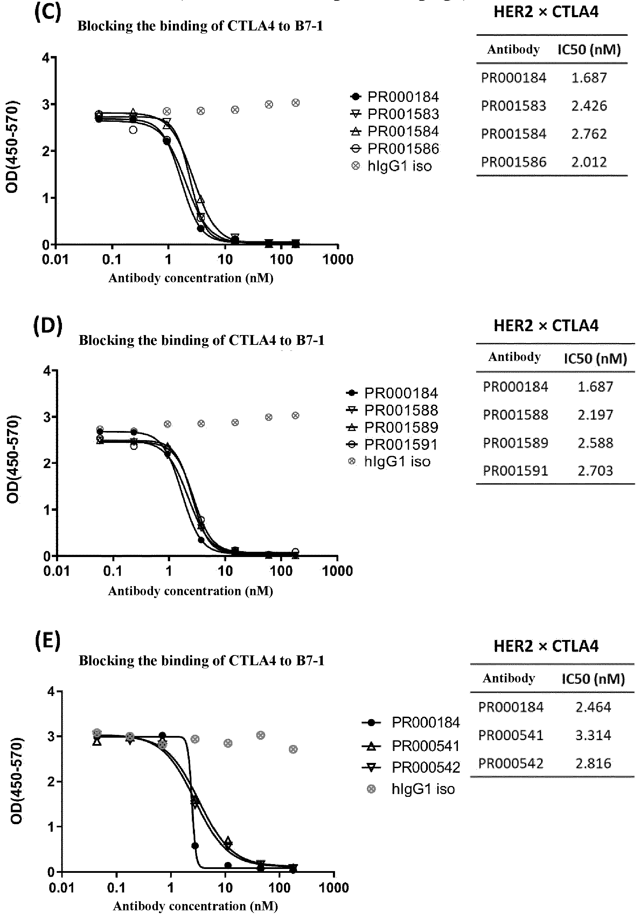

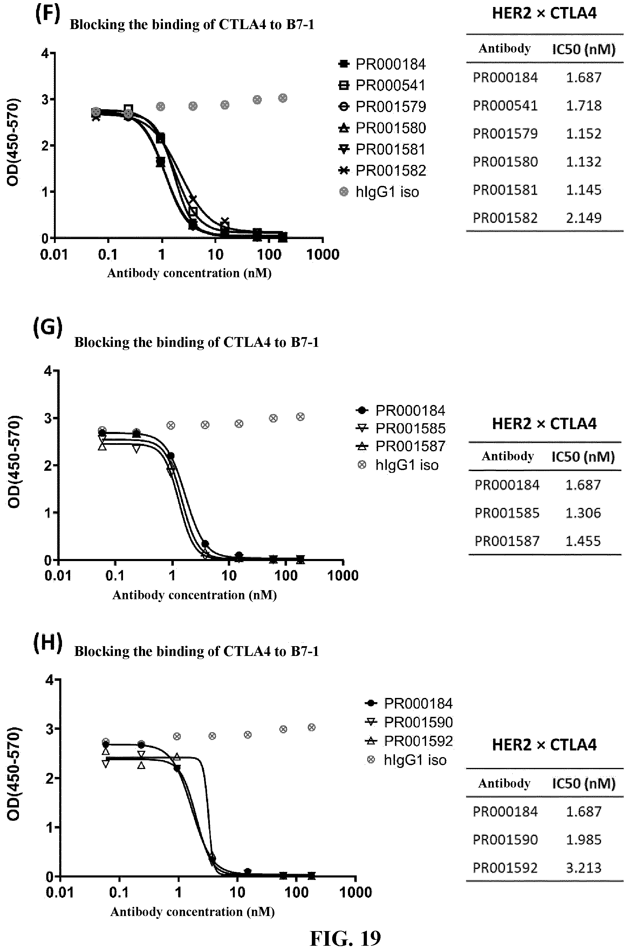

In this example, the anti-CTLA4 HCAb monoclonal antibody PR000184 was used as a positive control molecule, and was also the parent monoclonal antibody of the CTLA4 end of the HER2×CTLA4 bispecific antibody molecule. The results are shown in FIG. 19 and Table 4-16.

As shown in FIG. 19 (A), the bispecific antibody molecules of Fab-HCAb symmetric structures all had ability (inhibitory activity) to block the binding of CTLA4 protein to its ligand protein, which was similar to or slightly weaker than that of the parent monoclonal antibody PR000184, as evidenced by the similar maximum OD difference values, but the IC$_{50}$ values that were slightly poorer by only 1.5-2.1 times.

As shown in FIG. 19 (B-D), the bispecific antibody molecules of IgG-VH tetravalent symmetric structures had slightly different inhibitory activity according to their specific molecular structures. The bispecific antibody molecules of such structures were further classified into three classes according to the position of the VH end of anti-CTLA4 relative to anti-HER2 IgG in the bispecific antibody structure: VH at the C-terminus of the heavy chain of IgG (PR000539, PR000540, and PR000715); VH at the N-terminus of the heavy chain of IgG (PR001583, PR001586, PR001588, and PR001591); and VH at the N-terminus of the light chain of IgG (PR001584 and PR001589). When VH was at the C-terminus of the heavy chain of IgG, the inhibitory activity of those bispecific antibody molecules was slightly weaker than that of the parent monoclonal antibody, as evidence by the similar maximum OD difference values, but the IC$_{50}$ values that were slightly poorer by only 1.5-2.5 times. When VH was at the N-terminus of either the heavy or light chain of IgG, those bispecific antibody molecules had similar inhibitory activity to the parent monoclonal antibody.

As shown in FIG. 19 (E-F), the bispecific antibody molecules of IgG-VH-VH hexavalent symmetric structures had similar or slightly superior inhibitory activity to the parent monoclonal antibody.

As shown in FIG. 19 (G-H), the bispecific antibody molecules of 2×VH-IgG hexavalent symmetric structures had similar to or slightly superior inhibitory activity to the parent monoclonal antibody.

**Table 4-16. Blocking the binding of human CTLA4 protein to B7-1 protein**

| Antibody | IC$_{50}$ (nM) | Antibody | IC$_{50}$ (nM) | Antibody | IC$_{50}$ (nM) |
|---|---|---|---|---|---|
| PR000184 | 2.464 | PR000184 | 1.687 | PR001579 | 1.152 |

(continued)

| Antibody | IC$_{50}$ (nM) | Antibody | IC$_{50}$ (nM) | Antibody | IC$_{50}$ (nM) |
|---|---|---|---|---|---|
| PR000654 | 5.307 | PR001583 | 2.426 | PR001580 | 1.132 |
| PR000655 | 4.024 | PR001584 | 2.762 | PR001581 | 1.145 |
| PR000539 | 5.406 | PR001586 | 2.012 | PR001582 | 2.149 |
| PR000540 | 6.374 | PR001588 | 2.197 | PR001585 | 1.306 |
| PR000715 | 6.281 | PR001589 | 2.588 | PR001587 | 1.455 |
| PR000541 | 3.314 | PR001591 | 2.703 | PR001590 | 1.985 |
| PR000542 | 2.816 | PR000541 | 1.718 | PR001592 | 3.213 |
| **Experiment 1** | | **Experiment 2** | | | |

## Example 4.5.2. Blocking the binding of human CTLA4 cell to its ligand protein

The inhibitory activity of the antibody molecules for the binding of cells expressing CTLA4 to the ligand B7-1/CD80 of CTLA4 was determined by flow cytometry FACS. Specifically, a CHO-K1 cell line CHO-K1/hCTLA4 (ChemPartner) highly expressing human CTLA4 was digested and resuspended in an FACS buffer (a PBS buffer containing 2% FBS), with the cell density adjusted to $3 \times 10^6$ cells/mL. The CHO-K1/hCTLA4 cells were seeded in a 96 well plate ($3 \times 10^5$ cells/well) at 100 $\mu$L/well and centrifuged at 500 g at 4 °C for 5 min, and the supernatant was discarded. Then, the serially diluted antibody molecules were added to the 96-well plate at 100 $\mu$L/well, and the mixtures were well mixed. The antibody molecules might serially diluted 3-fold from the highest final concentration 200 nM to obtain a total of 8 concentrations. hIgG1 iso (CrownBio, #C0001) was used as an isotype control. Then, 3 $\mu$g/mL biotinylated ligand protein human B7-1-Fc-biotin (ACRO Biosystems, #B71-H82F2) was added to the 96-well plate at 100 $\mu$L/well, and the mixture was mixed well. The 96-well plate was incubated at 4 °C for 1 h away from light. Then, the cells in each well were rinsed twice with 200 $\mu$L of pre-cooled FACS buffer and centrifuged at 500 g at 4 °C for 5 min, and then the supernatant was discarded. Thereafter, a fluorescent label (Streptavidin, Alexa Fluor™ 488 conjugate, Thermo, #S32354, diluted in a 1:1000 ratio) was added at 100 $\mu$L/well, and the plate was incubated at 4 °C for 1 h away from light. Then, the cells in each well were rinsed three times with 200 $\mu$L of pre-cooled FACS buffer and centrifuged at 500 g at 4 °C for 5 min, and then the supernatant was discarded. Finally, a pre-cooled FACS buffer was added at 200 $\mu$L/well to resuspend the cells. Fluorescence signal values were read using a BD FACS CANTOII flow cytometer or an ACEA NovoCyte flow cytometer, and the data were processed and analyzed using FlowJo v10 (FlowJo, LLC) software. The data were processed and analyzed by plotting using GraphPad Prism 8 software to convert the fluorescence signal (MFI) into the inhibition rate, and inhibition curves, IC$_{50}$ values, the maximum inhibition rates and other parameters were obtained through four-parameter nonlinear fitting.

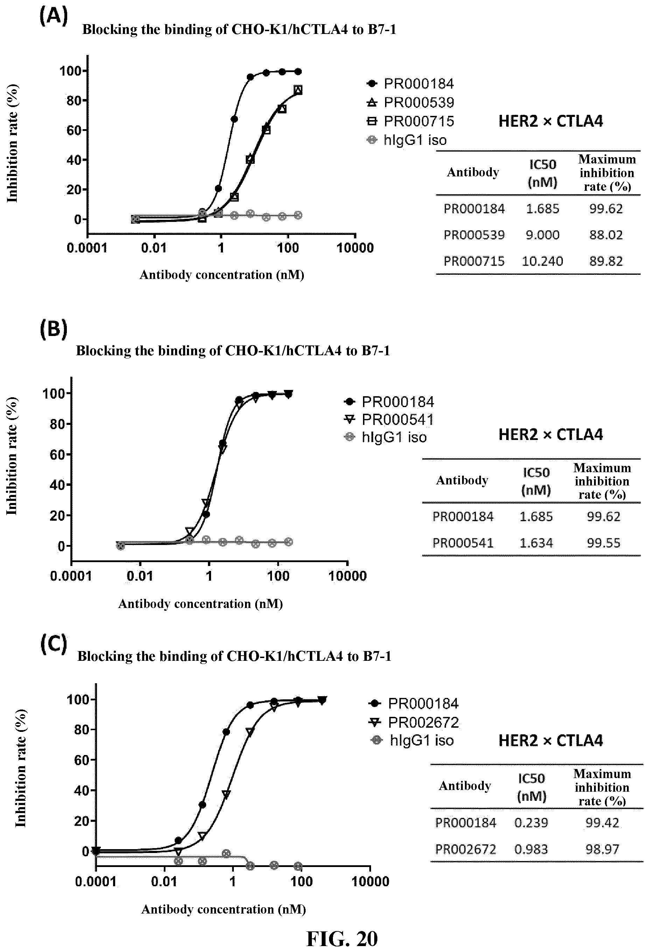

In this example, the anti-CTLA4 HCAb monoclonal antibody PR000184 was used as a positive control molecule, and was also the parent monoclonal antibody of the CTLA4 end of the HER2×CTLA4 bispecific antibody molecule. The results are shown in FIG. 20 and Table 4-17.

As shown in FIG. 20 (A-B), when VH of anti-CTLA4 was at the C-terminus of the heavy chain of anti-HER2 IgG, the bispecific antibody molecules of IgG-VH tetravalent symmetric structures had significantly reduced inhibitory activity, but the bispecific antibody molecules of IgG-VH-VH hexavalent symmetric structures (PR000541) had similar inhibitory activity to the parent monoclonal antibody.

As shown in FIG. 20 (C), the bispecific antibody molecule of a Fab-Fc-VH(2) asymmetric structure (PR002672) contained two CTLA4-binding domains formed in tandem, and in terms of inhibitory activity, it achieved nearly 100% inhibition as compared to the bivalent parent monoclonal antibody, although its IC$_{50}$ value was about 4 times poorer than that of the parent monoclonal antibody.

**Table 4-17. Blocking the binding of human CTLA4 cell to B7-1 protein**

| Antibody | IC$_{50}$ (nM) | Maximum inhibition rate (%) | Antibody | IC$_{50}$ (nM) | Maximum inhibition rate (%) |
|---|---|---|---|---|---|
| PR000184 | 1.685 | 99.62 | PR000184 | 0.239 | 99.42 |
| PR000539 | 9.0 | 88.02 | PR002672 | 0.983 | 98.97 |
| PR000715 | 10.24 | 89.82 | | | |
| PR000541 | 1.634 | 99.55 | | | |

(continued)

| Antibody | $IC_{50}$ (nM) | Maximum inhibition rate (%) | Antibody | $IC_{50}$ (nM) | Maximum inhibition rate (%) |
|---|---|---|---|---|---|
| Experiment 1 | | | Experiment 2 | | |

**Example 4.6. Binding to HER2**

This example is intended to investigate the binding activity of the HER2×CTLA4 bispecific antibody molecules to HER2.

**Example 4.6.1. Binding to SK-BR-3 cells highly expressing human HER2**

The binding ability of the antibodies to a tumor cell line SK-BR-3 (ATCC, HTB-30) highly expressing human HER2 was determined by flow cytometry FACS. Specifically, the SK-BR-3 cells were digested and resuspended in a DMEM complete medium, with the cell density adjusted to $1\times10^6$ cells/mL. Then, the cells were seeded in a 96-well V-bottom plate (Corning, #3894) at 100 μL/well and centrifuged at 4 °C for 5 min, and the supernatant was discarded. Then, the antibody molecules at a total of 8 concentrations obtained by a 5-fold gradient dilution with the highest final concentration of 100 nM were added at 100 μL/well. The mixture was mixed well. hIgG1 iso (CrownBio, #C0001) was used as an isotype control. The cells were incubated at 4 °C for 1 h away from light. Thereafter, the mixture was centrifuged at 4 °C for 5 min, and the supernatant was discarded. Then, the cells in each well were rinsed twice with 200 μL of pre-cooled FACS buffer (a PBS buffer containing 0.5% BSA), and centrifuged at 500 g at 4 °C for 5 min, and then the supernatant was discarded. Thereafter, a fluorescent secondary antibody (Goat human IgG (H + L) Alexa Fluor 488 conjugation, Thermo, #A11013, 1:1000 dilution) was added at 100 μL/well, and the plate was incubated at 4 °C for 1 h away from light. Then, the cells in each well were then rinsed twice with 200 μL of pre-cooled FACS buffer and centrifuged at 500 g at 4 °C for 5 min, and then the supernatant was discarded. Finally, a pre-cooled FACS buffer was added at 200 μL/well to resuspend the cells. Fluorescence signal values were read using a BD FACS CANTOII flow cytometer or an ACEA NovoCyte flow cytometer, and the data were processed and analyzed using FlowJo v10 (FlowJo, LLC) software. The data were processed and analyzed by plotting using GraphPad Prism 8 software, and binding curves of the antibodies to target cells, $EC_{50}$ values and other parameters were obtained through four-parameter nonlinear fitting.

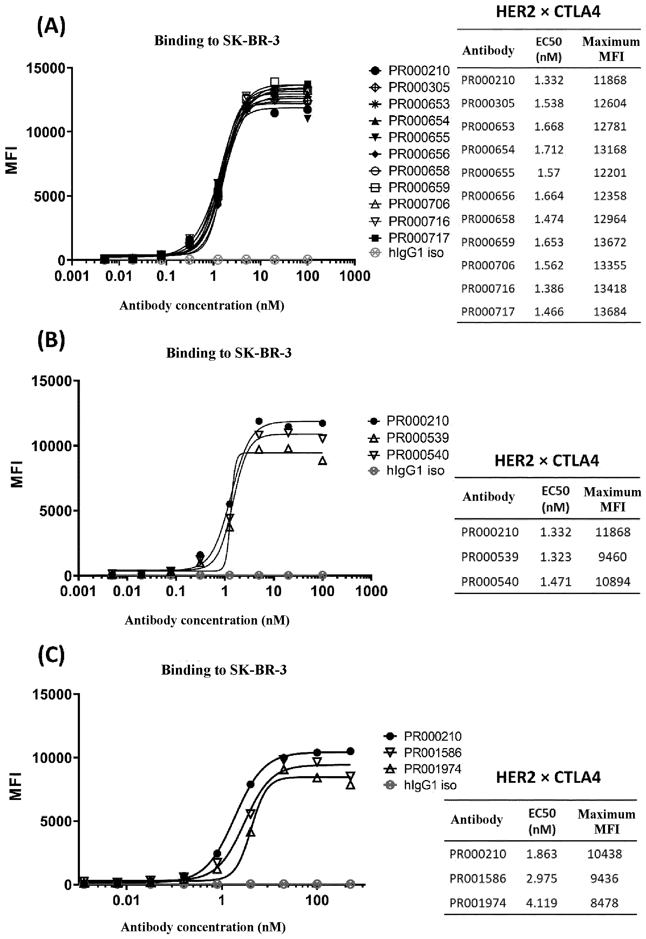

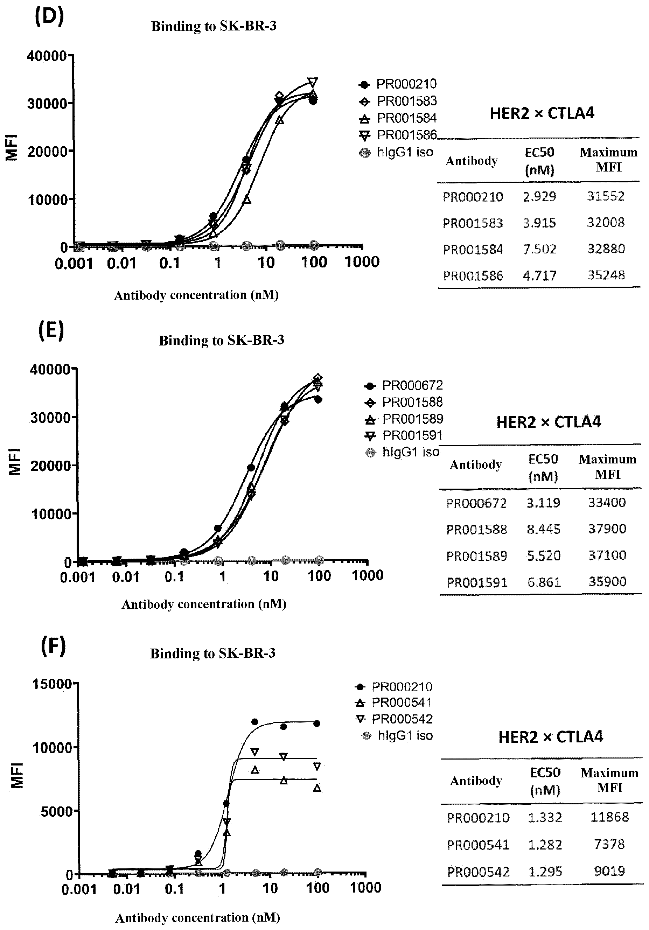

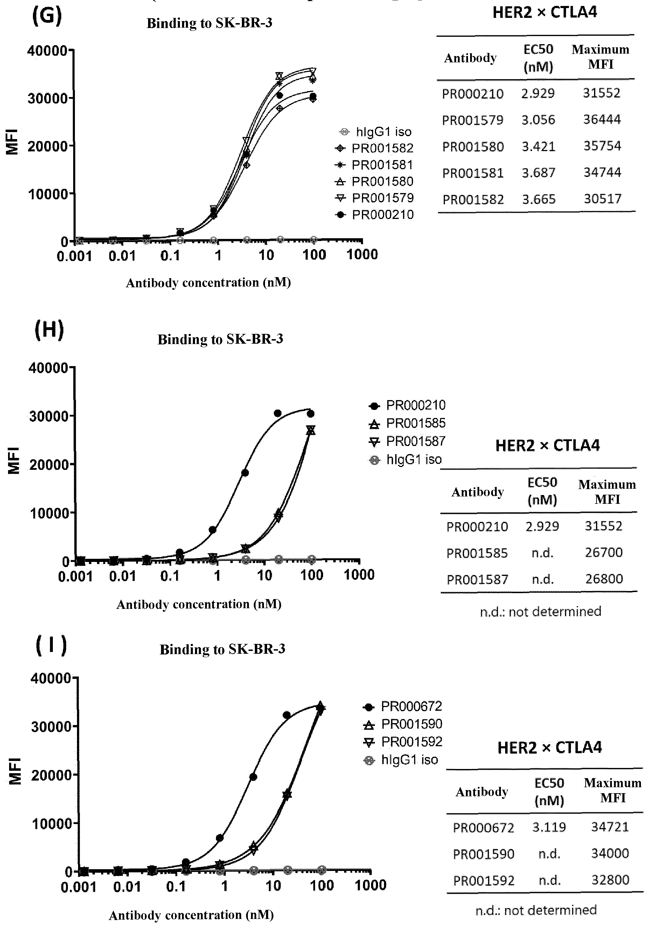

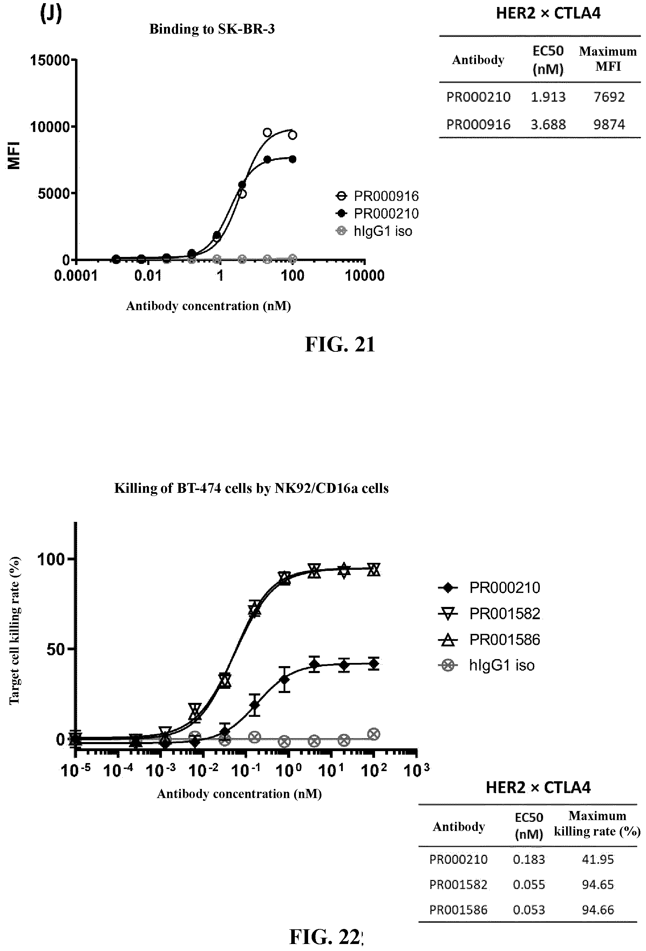

In this example, the anti-HER2 monoclonal antibody PR000210 (trastuzumab analog) or PR000672 (pertuzumab analog) was used as a positive control molecule, and was also the parent monoclonal antibody of the HER2×CTLA4 bispecific antibody molecule. The results are shown in FIG. 21 and Table 4-18.

As shown in FIG. 21 (A), the bispecific antibody molecules of Fab-HCAb symmetric structures had similar binding ability to HER2 to the parent monoclonal antibody PR000210, which was reflected in similar $EC_{50}$ values and maximum MFIs. This indicates that the Fab end structure of anti-HER2 of the bispecific antibody molecule of a Fab-HCAb symmetric structure can well retain the binding ability to HER2.

As shown in FIG. 21 (B-E), the bispecific antibody molecules of IgG-VH tetravalent symmetric structures had slightly different binding ability to HER2 according to their specific molecular structures. The bispecific antibody molecules of such structures were further classified into three classes according to the position of the VH end of anti-CTLA4 relative to anti-HER2 IgG in the bispecific antibody structure: VH at the C-terminus of the heavy chain of IgG (PR000539 and PR000540); VH at the N-terminus of the heavy chain of IgG (PR001583, PR001586, PR001588, and PR001591); and VH at the N-terminus of the light chain of IgG (PR001584, PR001974, and PR001589). In terms of the $EC_{50}$ values, when VH was at the C-terminus of the heavy chain of IgG, the structure well retained the binding ability to HER2, indicating that the VH end has no significant effect on the Fab end of IgG; when VH was at the N-terminus of either the heavy or light chain of IgG, the binding ability of the bispecific antibody molecule to HER2 was reduced by 1.5-2.5 times as compared to that of the corresponding parent monoclonal antibody.

As shown in FIG. 21 (F-G), the bispecific antibody molecules of IgG-VH-VH hexavalent symmetric structures had similar binding ability to HER2 to the corresponding parental monoclonal antibody.

As shown in FIG. 21 (H-I), the bispecific antibody molecules of 2×VH-IgG hexavalent symmetric structures had significantly reduced binding ability to HER2 as compared to its corresponding parent monoclonal antibody, indicating that when VH is linked to the N-terminus of both the heavy and light chains of IgG, the function of Fab end of IgG is significantly affected.

As shown in FIG. 21 (J), the HER2-binding domain of the bispecific antibody molecule of a Fab-Fc-VH asymmetric structure (PR000916) was a monovalent Fab structure, but it had very similar binding ability to HER2 to the bivalent parent monoclonal antibody, indicating that the binding domain of trastuzumab has no significant difference in monovalent binding and bivalent binding, and the bispecific antibody molecule of a Fab-Fc-VH asymmetric structure well retains the binding ability to HER2.

**Table 4-18. Binding to SK-BR-3**

| Antibody | EC$_{50}$ (nM) | Maximum MFI | Antibody | EC$_{50}$ (nM) | Maximum MFI |
|---|---|---|---|---|---|
| PR000210 | 1.332 | 11868 | PR000210 | 2.929 | 31552 |
| PR000305 | 1.538 | 12604 | PR001583 | 3.915 | 32008 |
| PR000653 | 1.668 | 12781 | PR001584 | 7.502 | 32880 |
| PR000654 | 1.712 | 13168 | PR001586 | 4.717 | 35248 |
| PR000655 | 1.57 | 12201 | PR001579 | 3.056 | 36444 |
| PR000656 | 1.664 | 12358 | PR001580 | 3.421 | 35754 |
| PR000658 | 1.474 | 12964 | PR001581 | 3.687 | 34744 |
| PR000659 | 1.653 | 13672 | PR001582 | 3.665 | 30517 |
| PR000706 | 1.562 | 13355 | PR001585 | n.d. | 26700 |
| PR000716 | 1.386 | 13418 | PR001587 | n.d. | 26800 |
| PR000717 | 1.466 | 13684 | | | |
| PR000539 | 1.323 | 9460 | | | |
| PR000540 | 1.471 | 10894 | | | |
| PR000541 | 1.282 | 7378 | | | |
| PR000542 | 1.295 | 9019 | | | |
| **Antibody** | **EC$_{50}$ (nM)** | **Maximum MFI** | **Antibody** | **EC$_{50}$ (nM)** | **Maximum MFI** |
| PR000210 | 1.913 | 7692 | PR000210 | 1.863 | 10438 |
| PR000916 | 3.688 | 9874 | PR001586 | 2.975 | 9436 |
| | | | PR001974 | 4.119 | 8478 |
| **Antibody** | **EC$_{50}$ (nM)** | **Maximum MFI** | | | |
| PR000672 | 3.119 | 33400 | | | |
| PR001588 | 8.445 | 37900 | | | |
| PR001589 | 5.52 | 37100 | | | |
| PR001591 | 6.861 | 35900 | | | |
| PR001590 | n.d. | 34000 | | | |
| PR001592 | n.d. | 32800 | | | |

## Example 4.7. Superantigen SEB stimulation assay

This example is intended to investigate the activation effect of HER2×CTLA4 bispecific antibody molecules on peripheral blood mononuclear cells (PBMCs). In the first step, the isolated human PBMCs (MT-Bio) were firstly adjusted to $5×10^6$ cells/mL. Thereafter, PBMC was added to a 96-well plate (Corning, # 3799) at 50 µL/well. Then, the antibody molecules at different concentrations were added at 100 µL/well, wherein the antibody concentrations may be the final concentration gradient of (150 nM, 30 nM, 1 nM) or (80 nM, 8 nM, 0.8 nM, 0.08 nM); and two duplicate wells were set for each concentration. hIgG1 iso (CrownBio, # C0001) was used as an isotype control. The mixture was incubated at 37 °C for 30 min. Thereafter, 400 ng/mL superantigen staphyloccal enterotoxin B (SEB) was added at 50 µL/well to make a final concentration of 100 ng/mL. The cells were incubated in an incubator at 37 °C with 5% $CO_2$ for 72 h or 96 h, and the supernatant was collected. In the second step, the concentration of IL-2 in the collected supernatant was determined using an IL-2 assay kit (Thermo, #88-7025-77). The data were processed and analyzed by plotting using a GraphPad Prism 8 software.

In this example, the anti-CTLA4 HCAb monoclonal antibody PR000184 or PR000218 was used as a positive control molecule, and was also the parent monoclonal antibody of the CTLA4 end of the HER2×CTLA4 bispecific antibody molecule. PR000218 was an ADCC-enhanced variant constructed by Fc mutation on the basis of PR000184.

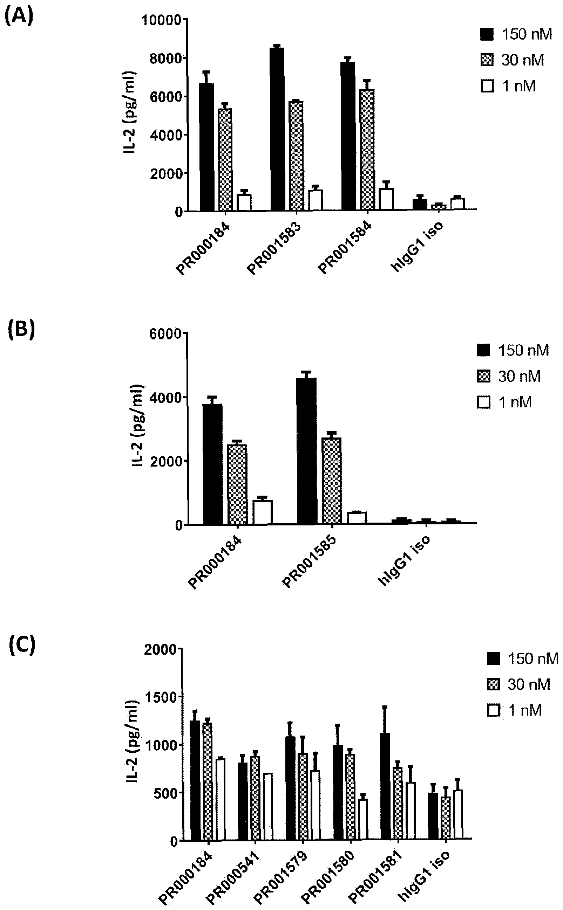

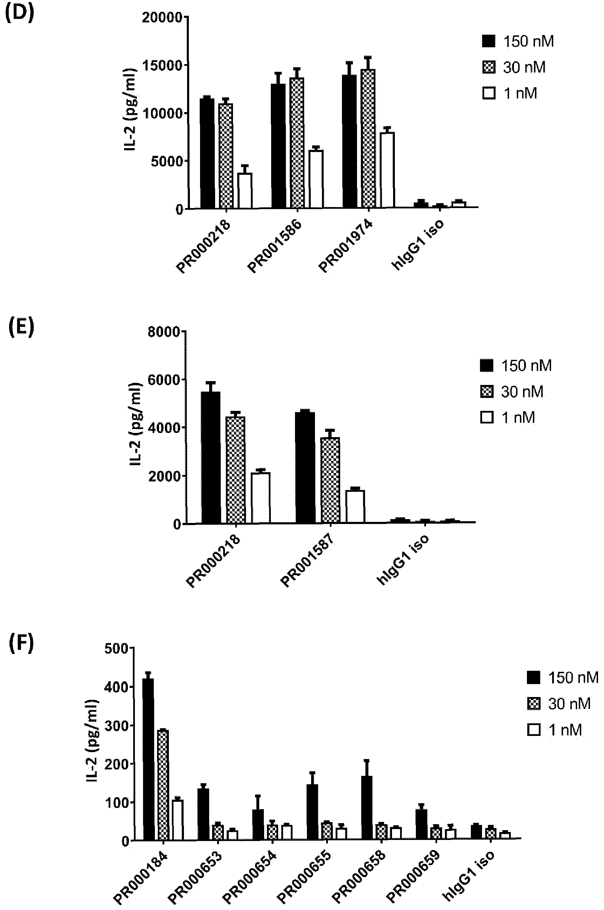

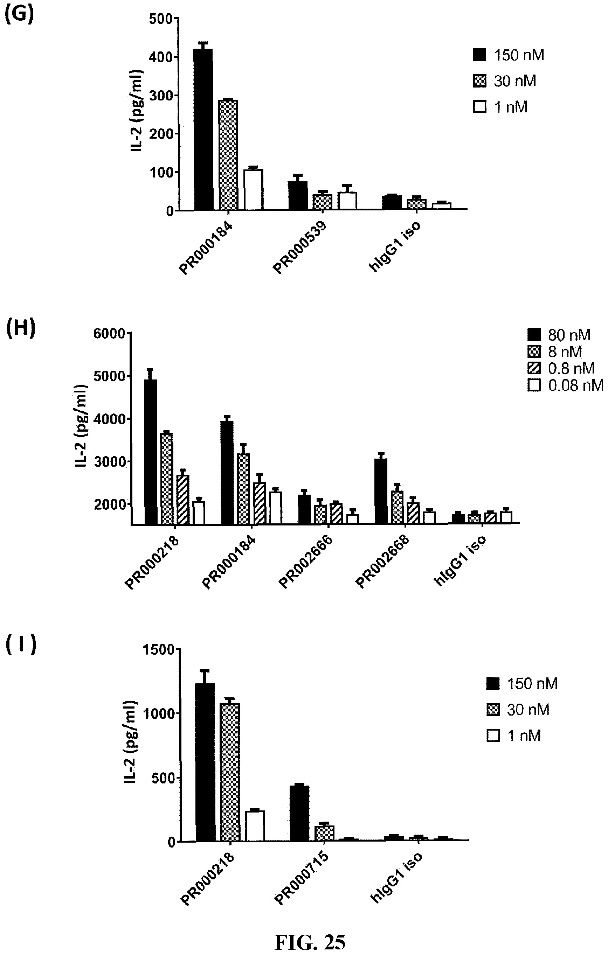

As shown in FIG. 25, when stimulated with SEB, the anti-CTLA4 monoclonal antibodies and the HER2×CTLA4 bispecific antibody molecules were all able to promote T cells to product IL-2 to different degrees, and their T cell activation ability was related to their structures.

As shown in FIG. 25 (F), the bispecific antibody molecules of Fab-HCAb symmetric structures had weaker T cell activation ability than the corresponding parent monoclonal antibody. The CTLA4 end had weakened activity in the Fab-HCAb structure as compared to its presence in normal IgG or HCAb bivalent structures, so that its dose-related toxicity could be reduced for suitable use in current combination drug doses in the clinic.

As shown in FIG. 25 (A, D, G, I), the bispecific antibody molecules of IgG-VH tetravalent symmetric structures had slightly different T cell activation ability according to their specific molecular structures. The bispecific antibody molecules of such structures were further classified into three classes according to the position of the VH end of anti-CTLA4 relative to anti-HER2 IgG in the bispecific antibody structure: VH at the C-terminus of the heavy chain of IgG (PR000539 and PR000715); VH at the N-terminus of the heavy chain of IgG (PR001583 and PR001586); and VH at the N-terminus of the light chain of IgG (PR001584 and PR001974). When VH was at the C-terminus of the heavy chain of IgG, the bispecific antibody molecule had significantly weakened T cell activation ability than the corresponding parent monoclonal antibody. When VH was at the N-terminus of either the heavy or light chain of IgG, the bispecific antibody molecule had similar or even slightly enhanced T cell activation ability as compared to the corresponding parent monoclonal antibody. This indicates that the activity of anti-CTLA4 at the VH end can be modulated by adjusting the relative position of VH on IgG to be suitable for different scenarios of combination drug doses in the clinic.

As shown in FIG. 25 (C), the bispecific antibody molecules of IgG-VH-VH hexavalent symmetric structures had similar T cell activation ability to the parent monoclonal antibody, and the activity of their CTLA4 ends can be modulated by using different linker peptides.

As shown in FIG. 25 (B, E), the bispecific antibody molecules of $2 \times$VH-IgG hexavalent symmetric structures had similar T cell activation ability to the parent monoclonal antibody, indicating that the CTLA4 ends of those bispecific antibody molecules almost completely retain the activity.

As shown in FIG. 25 (H), the bispecific antibody molecule of a Fab-Fc-VH(2) asymmetric structure contained two CTLA4-binding domains formed in tandem, and its T cell activation ability was weakened as compared to that of the corresponding parent monoclonal antibody. The CTLA4 end may have reduced dose-related toxicity for suitable use in current combination drug doses in the clinic.

## Example 4.8. Inhibition on proliferation of HER2$^+$ cells

This example is intended to investigate the inhibition of the HER2$\times$CTLA4 bispecific antibody molecules on the proliferation of the tumor cell line SK-BR-3 (ATCC, HTB-30) highly expressing human HER2. Specifically, SK-BR-3 cells were digested and resuspended in a DMEM complete medium. The cells were seeded in a 96-well plate (Perkin Elmer, #6005225) at 2000 cells/50 μL, and incubated in an incubator at 37 °C with 5% $CO_2$ overnight. The next day, the antibody molecules at a total of 6 concentrations obtained by a 5-fold gradient dilution with the highest final concentration of 100 nM were added at 50 μL/well. The mixture was mixed well. hIgG1 iso (CrownBio, #C0001) was used as an isotype control. The cells were incubated in an incubator at 37 °C with 5% $CO_2$ for 7 days. Thereafter, 100 μL of CellTiter-Glo (Promega, #G7573) was added to each well, and the mixture was mixed on a horizontal shaker for 10 min to induce cell lysis. Finally, chemiluminescence values were measured using an Enspire™ multifunctional microplate reader (Perkin Elmer, Inc.). The data were processed and analyzed by plotting using GraphPad Prism 8 software, and inhibition rates and other parameters were obtained through four-parameter nonlinear fitting.

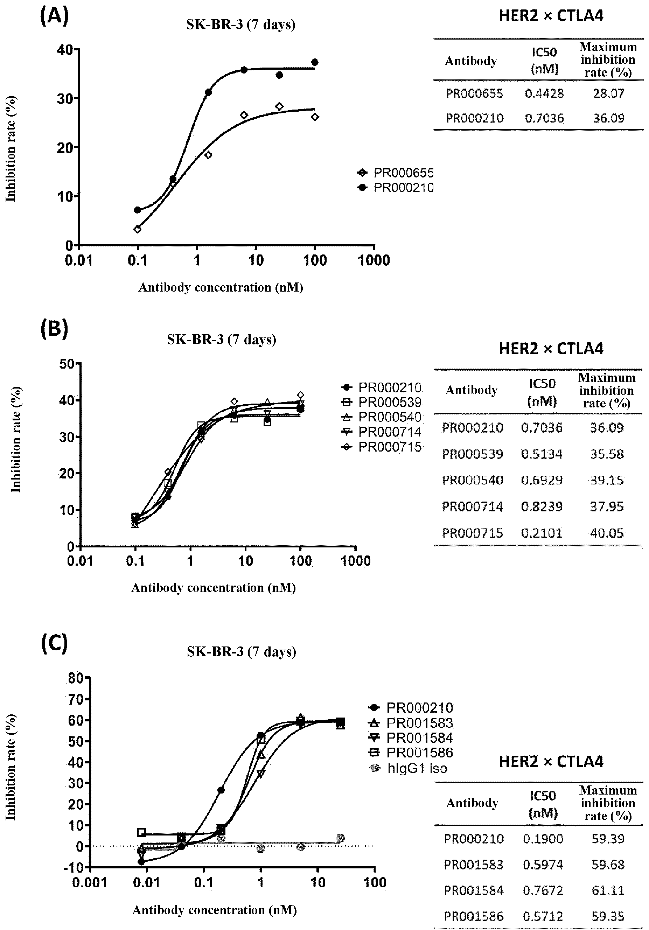

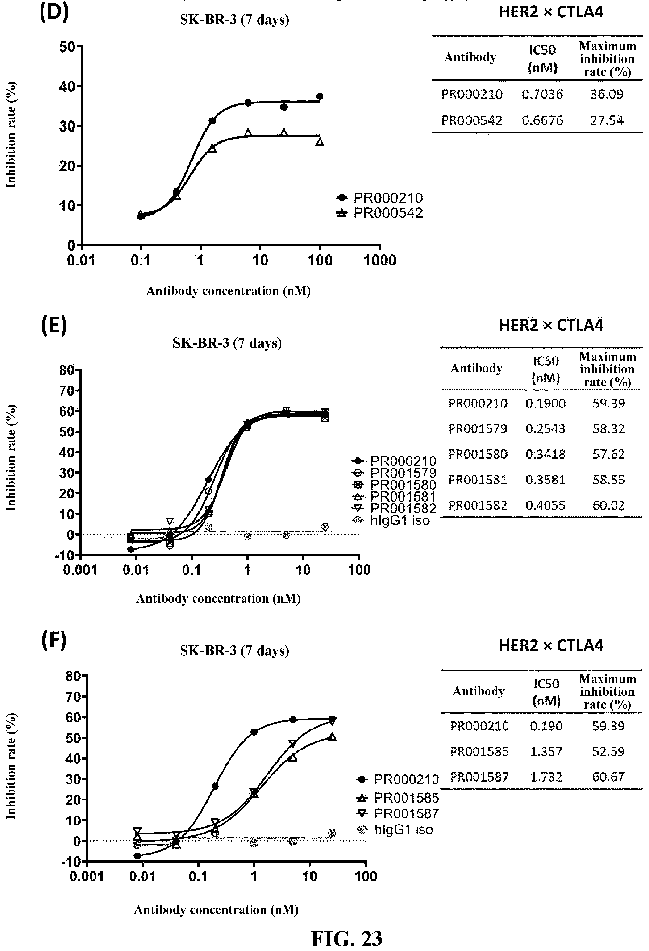

In this example, the anti-HER2 monoclonal antibody PR000210 (trastuzumab analog) was used as a positive control molecule, and was also the parent monoclonal antibody of the HER2$\times$CTLA4 bispecific antibody molecule. The results are shown in FIG. 23 and Table 4-19.

As shown in FIG. 23 (A), the bispecific antibody molecule of a Fab-HCAb symmetric structure (PR000655) had significantly reduced inhibition ability to the proliferation of SK-BR-3 cells as compared to the parent monoclonal antibody PR000210.

As shown in FIG. 23 (B-C), the bispecific antibody molecules of IgG-VH tetravalent symmetric structures had slightly different inhibitory effects on SK-BR-3 cell proliferation according to their specific molecular structures. The bispecific antibody molecules of such structures were further classified into three classes according to the position of the VH end of anti-CTLA4 relative to anti-HER2 IgG in the bispecific antibody structure: VH at the C-terminus of the heavy chain of IgG (PR000539, PR000540, PR000714, and PR000715); VH at the N-terminus of the heavy chain of IgG (PR001583 and PR001586); and VH at the N-terminus of the light chain of IgG (PR001584). When VH was at the C-terminus of the heavy chain of IgG, the structure well retained the inhibition ability to the proliferation of SK-BR-3 cells, which was reflected in similar or slightly superior $IC_{50}$ and maximum inhibition rate to those of the parent monoclonal antibody, indicating that the VH end has no significant effect on the Fab end of IgG. When VH was at the N-terminus of either the heavy or light chain of IgG, the bispecific antibody molecules had slightly reduced inhibition ability to the proliferation of SK-BR-3 cells as compared to the parent monoclonal antibody, which was reflected in the $IC_{50}$ values that

were poorer by 3-4 times, but still the same maximum inhibition rate.

As shown in FIG. 23 (D-E), most of the bispecific antibody molecules of IgG-VH-VH hexavalent symmetric structures had similar inhibition ability to the proliferation of SK-BR-3 cells to the parental monoclonal antibody, except for PR000542 which showed a reduced level of maximum inhibition rate. This result is consistent with the previous results regarding the binding ability to HER2.

As shown in FIG. 23 (F), the bispecific antibody molecules of 2×VH-IgG hexavalent symmetric structures had significantly reduced inhibition ability to the proliferation of SK-BR-3 cells as compared to the parent monoclonal antibody. This result is consistent with the previous results regarding the binding ability to HER2.

**Table 4-19. Inhibition on proliferation of SK-BR-3**

| Antibody | $IC_{50}$ (nM) | Maximum inhibition rate (%) | Antibody | $IC_{50}$ (nM) | Maximum inhibition rate (%) |
|---|---|---|---|---|---|
| PR000655 | 0.4428 | 28.07 | PR000210 | 0.19 | 59.39 |
| PR000210 | 0.7036 | 36.09 | PR001583 | 0.5974 | 59.68 |
| PR000539 | 0.5134 | 35.58 | PR001584 | 0.7672 | 61.11 |
| PR000540 | 0.6929 | 39.15 | PR001586 | 0.5712 | 59.35 |
| PR000714 | 0.8239 | 37.95 | PR001579 | 0.2543 | 58.32 |
| PR000715 | 0.2101 | 40.05 | PR001580 | 0.3418 | 57.62 |
| PR000542 | 0.6676 | 27.54 | PR001581 | 0.3581 | 58.55 |
| | | | PR001582 | 0.4055 | 60.02 |
| | | | PR001585 | 1.357 | 52.59 |
| | | | PR001587 | 1.732 | 60.67 |
| **Experiment 1** | | | **Experiment 2** | | |

## Example 4.9. ADCC effect mediated by HER2

This example is intended to investigate the antibody-dependent cell-mediated cytotoxicity (ADCC) of HER2×CTLA4 bispecific antibody molecules to the tumor cell line BT-474 (ATCC, HTB-20) highly expressing human HER2. In the first step, target cells were labeled with DELFIA BATDA (Perkin Elmer, #C136-100). The specific labeling method was as follows. $1×10^6$ target cells were labeled with 2 μL of DELFIA BATDA reagent; the cells were incubated in an incubator at 37 °C with $CO_2$ for 20 min, washed 4 times with PBS, and centrifuged at 1000 rpm for 5 min; after the last wash, the precipitate was resuspended in an RPMI-1640 medium containing 20% FBS (Thermo, #A 10491), with the cell density adjusted to $1×10^5$ /mL. In the second step, the labeled target cells were seeded in a 96-well plate (Corning, #3599) at 100 μL/well. Then, the antibody molecules at a total of 10 concentrations obtained by a 5-fold gradient dilution with the highest final concentration of 100 nM were added at 50 μL/well. The mixture was mixed well. hIgG1 iso (CrownBio, #C0001) was used as an isotype control. The cells were incubated in an incubator at 37 °C with 5% $CO_2$ for 10 min. In the third step, the NK-92 MI/CD16a cells (ChemPartner) were collected as effector cells, and the cell density of NK-92 MI/CD16a was adjusted to $1.2×10^6$/mL. Then, the cells were added to the 96-well plate at 50 μL/well in an effect-to-target ratio of 6:1. Thereafter, the cells were incubated in an incubator at 37 °C with 5% $CO_2$ for 4 h. In the fourth step, the cells were centrifuged at 500 g for 5 min, and then 25 μL of supernatant from each well was added to a new 96-well assay plate. Then, a DELFIA® Europium solution (Perkin Elmer, #C135-100) was added at 200 μL/well, and the plate was shaken at 250 rpm at room temperature for 15 min. Finally, fluorescence values were measured using an EnVision® 2015 multifunctional microplate reader (Perkin Elmer, Inc.).

The killing rate was calculated according to the following formula:

$$\text{Kill rate } (\%) = (\text{ER - ETSR})/(\text{TMR - ETSR}) \times 100\%$$

wherein:

ER = experimental well (antibody + effector cell + target cell); ETSR = spontaneous release well for mixed effector cell and target cell (effector cell + target cell); TMR = maximum release well for target cell (target cell + lysis buffer)

The data were processed and analyzed by plotting using GraphPad Prism 8 software, and killing rate curves of the antibodies to target cells, $EC_{50}$ values, maximum killing rates and other parameters were obtained through four-parameter nonlinear fitting.

In this example, the anti-HER2 monoclonal antibody PR000210 (trastuzumab analog) was used as a positive control molecule, and was also the parent monoclonal antibody of the HER2×CTLA4 bispecific antibody molecule.

As shown in FIG. 22 and Table 4-20, the bispecific antibody molecule of an IgG-VH-VH hexavalent symmetric structure (PR001582) and the bispecific antibody molecule of an IgG-VH tetravalent symmetric structure (PR001586) were both able to cause a strong ADCC effect.

**Table 4-20. Killing target cell BT-474 by ADCC effect**

| Antibody | $EC_{50}$ (nM) | Maximum killing rate (%) |
|---|---|---|
| PR000210 | 0.183 | 41.95 |
| PR001582 | 0.055 | 94.65 |
| PR001586 | 0.053 | 94.66 |

**Example 4.10. Determination of the binding ability of bispecific antibody molecules to two cells simultaneously by flow cytometry**

This example is intended to investigate the binding ability of the HER2×CTLA4 bispecific antibody molecule to SK-BR-3 cells (ATCC, HTB-30) expressing human HER2 and CHO-K1/hCTLA4 cells (ChemPartner) expressing human CTLA4 simultaneously. In the first step, CHO-K1/hCTLA4 cells and SK-BR-3 cells were fluorescently labeled with CFSE (Thermo, #C34554) and Far red (Thermo, #C34564) dyes, respectively. The specific procedures were as follows. CHO-K1/hCTLA4 cells and SK-BR-3 cells were separately resuspended in PBS and adjusted to $2 \times 10^6$/mL. 5 μM CFSE and 1 μM Far red were added to CHO-K1/hCTLA4 cells and SK-BR-3 cells, respectively. The cells were incubated at 37 °C for 10 min with occasional shaking. 5-fold amount of a complete medium was added to stop staining. Thereafter, the mixture was centrifuged at 4 °C for 5 min, and the supernatant was discarded, followed by washing twice with PBS. The densities of the labeled CHO-K1/hCTLA4 cells and SK-BR-3 cells were adjusted to $4 \times 10^6$/mL and $2 \times 10^6$/mL, respectively. In the second step, 25 μL each of the labeled CHO-K1/hCTLA4 cells and SK-BR-3 cells were taken, and mixed well with 50 μL of antibody molecules at a total of 6 concentrations obtained by a four-fold gradient dilution with the highest final concentration of 31.25 nM. The mixture was added to a 96-well plate. hIgG1 iso (CrownBio, #C0001) was used as an isotype control. The plate was incubated at 4 °C for 1 h away from light. Thereafter, 100 μL of 4% paraformaldehyde (PFA) was added to each well to fix the cells for 10 min. Finally, fluorescence signal values were read using a BD FACS CANTOII flow cytometer, and the data were processed and analyzed using FlowJo v10 (FlowJo, LLC) software. The cell numbers in different quadrants were counted according to different fluorescent labels, respectively: Q1 is Far-red labeled SK-BR-3 cells (SK-BR-3+); Q3 is CFSE-labeled CHO-K1/hCTLA4 cells (CTLA4+); Q2 contains two labeled cell populations (CTLA4+ & SK-BR-3+) formed by the simultaneous binding of bispecific antibody molecules to two cells. The percentage of the double-labeled positive cell population (Q2) relative to all labeled SK-BR-3 cells (Q1 + Q2) was calculated according to the following equation, which reflected the binding ability of the bispecific antibody molecules to two cells simultaneously.

$$\text{Cell ratio of Q2: } (Q2 / (Q1 + Q2)) \times 100\%$$

The data were processed and analyzed by plotting using GraphPad Prism 8 software, and curves of the antibodies simultaneously binding to two target cells were obtained through four-parameter nonlinear fitting.

In this example, the anti-HER2 monoclonal antibody PR000210 (trastuzumab analog) and the anti-CTLA4 monoclonal antibody PR000184 were used as control molecules, and were also parent monoclonal antibodies of the HER2×CTLA4 bispecific antibody molecule.

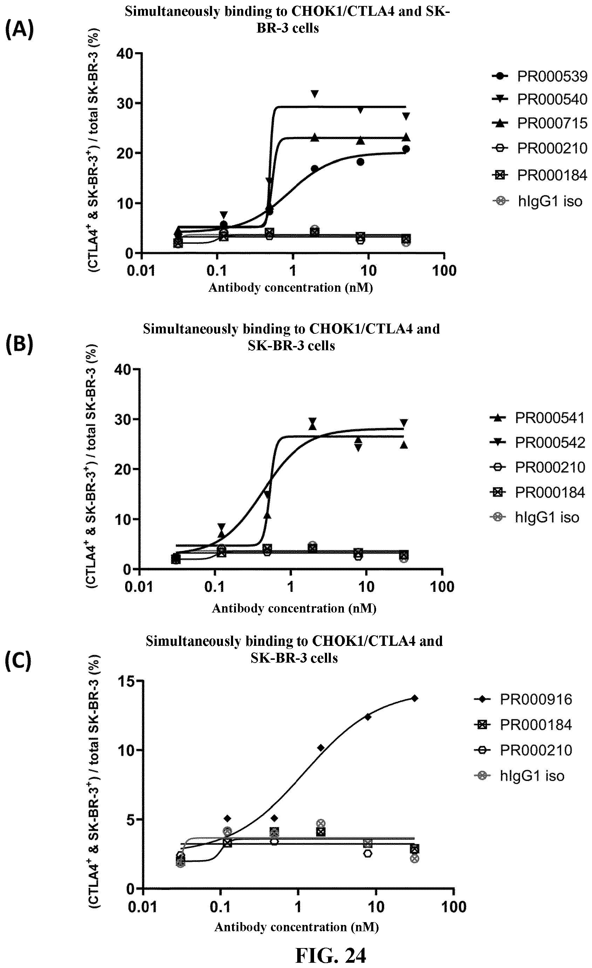

The panels A, B and C of FIG. 24 separately showed the ability of the bispecific antibodies of IgG-VH tetravalent symmetric structures (PR000539, PR000540, and PR000715), the bispecific antibodies of IgG-VH-VH hexavalent symmetric structures (PR000541 and PR000542), and the bispecific antibody of a Fab-Fc-VH asymmetric structure (PR000916) to simultaneously bind to CHOK1/CTLA4 and SK-BR-3 cells; wherein PR000210, PR000184 and hIgG1 iso were used as controls. The control molecules HER2 monoclonal antibody PR000210 and CTLA4 monoclonal antibody PR000184 were both unable to simultaneously bind to the two cells. The HER2×CTLA4 bispecific antibody molecules were able to simultaneously bind to two cells expressing different targets in a symmetric or asymmetric structure.

## Example 4.11. Pharmacokinetic study

In this example, the pharmacokinetic properties of antibody molecules in mice were investigated. The following antibodies were tested in this example: an anti-HER2 monoclonal antibody, PR000210 (trastuzumab analog); an HER2×CTLA4 bispecific antibody molecule of an IgG-VH tetravalent symmetric structure, PR000540; and an HER2×CTLA4 bispecific antibody molecule of an IgG-VH-VH hexavalent symmetric structure, PR000541.

The test was performed as follows: for each test antibody molecule, 3 female C57BL/6 mice weighing 18-22 g were selected and administered by intravenous injection at a dose of 5 mg/kg. The whole blood was collected before the administration and 0.5 h, 24 h (1 day), 2 days, 4 days, 7 days, 10 days and 14 days after the administration, left to stand for 30 min for coagulation, and centrifuged at 12,000 g at 4 °C for 5 min, and the isolated serum sample was cryopreserved at -80 °C until it was taken for analysis.

In this example, the drug concentration in the serum of mice was quantitatively determined by an Fc end ELISA method. The method was performed by capturing a fusion protein containing human Fc in the serum of mice using a goat anti-human Fc polyclonal antibody coating a 96-well plate, and then adding an HRP-labeled goat anti-human Fc secondary antibody. The plasma concentration data were analyzed using a non-compartmental analysis (NCA) model of Phoenix WinNonlin software (version 6.4) to evaluate the pharmacokinetics.

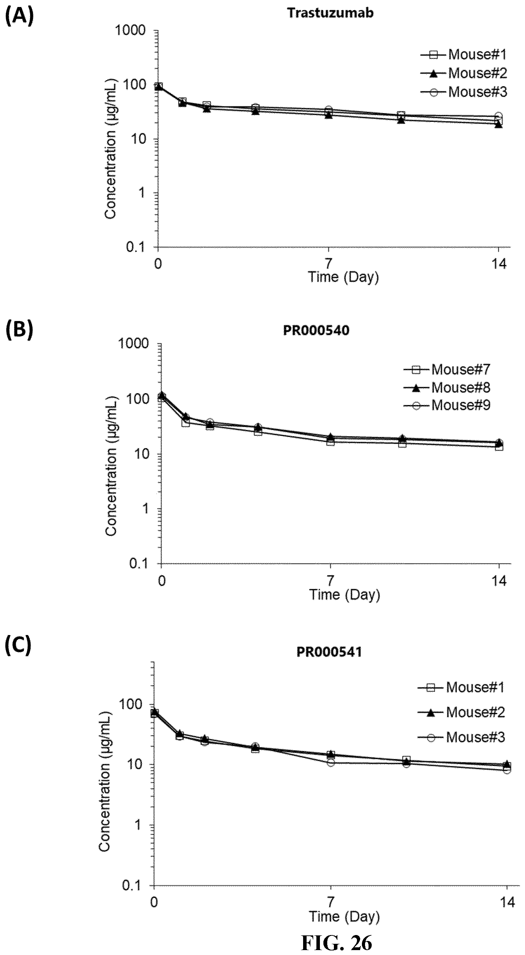

As shown in FIG. 26 and Table 4-21, the bispecific antibody PR000540 and the parent monoclonal antibody PR000210 had similar clearance rates, and PR000540 had a longer half-life $t_{1/2}$ value (more than 20 days). PR000541 showed a faster clearance rate, probably due to its complex structure.

**Table 4-21 Pharmacokinetics in mice**

| Structure | Antibody | CL (mL/day/kg) | Vss (mL/kg) | *Terminal t1/2 (day) | AUClast (day*ug/mL) | AUCINF (day*ug/mL) |
|---|---|---|---|---|---|---|
| IgG | PR000210 | 4.99 ± 0.93 | 96.2 ± 4.2 | 14.0 ± 1.9 | 470 ± 38 | 926 ± 177 |
| IgG HC-VH | PR000540 | 4.85 ± 0.59 | 142 ± 18 | 23.1 ± 3.2 | 374 ± 37 | 884 ± 109 |
| IgG_HC-VH-VH | PR000541 | 14.3 ± 1.41 | 152 ± 5.86 | 7.58 ± 0.58 | 251 ± 15.7 | 353 ± 33.4 |

## Example 4.12. Summary

In this example, **HER2×CTLA4** bispecific antibody molecules of a variety of structures are constructed using the antigen-binding domain Fab of the anti-HER2 IgG antibody and the antigen-binding domain VH of the anti-CTLA4 HCAb antibody. This shows the flexibility of constructing a bispecific antibody molecular structure based on HCAb. By regulating the functional activity of HER2 and CTLA4 ends through different structure types, relative positions, binding valence and other parameters, different activity combinations are designed to meet the requirements of different mechanisms of action. Currently, the recommended initial dose of the anti-HER2 monoclonal antibody trastuzumab is 4 mg/kg for the treatment of breast cancer and 8 mg/kg for the treatment of gastric cancer. It is slightly higher than the recommended dose of 3 mg/kg of the anti-CTLA4 monoclonal antibody Ipilimumab for the treatment of melanoma.

For example, when VH of anti-CTLA4 is at the N-terminus of anti-PD-L1 IgG, both the HER2 and CTLA4 ends of the bispecific antibody molecules (e.g., PR001583 and PR001584) can almost completely retain comparable activity to their parent monoclonal antibodies, and show similar ability to the corresponding parent monoclonal antibody in *in vitro* functional experiments such as a tumor cell proliferation inhibition experiment and a superantigen stimulation experiment. Therefore, this structure can be used to achieve a 1:1 dose combination of combination drugs.

For another example, both the HER2 and CTLA4 ends of bispecific antibody molecules of IgG-VH-VH hexavalent symmetric structures (e.g., PR001579, PR001580, and PR001581) can also almost completely retain comparable activity to their parent monoclonal antibodies. Therefore, this structure can be used to achieve a 1:1 dose combination of combination drugs.

For another example, when VH of anti-CTLA4 is at the C terminus of anti-HER2 IgG or in the Fab-HCAb structure, the activity of the HER2 ends of the bispecific antibody molecules (e.g., PR000539, PR000540, and PR000655) is almost comparable to their parent monoclonal antibodies, but the activity of the CTLA4 ends is weakened to different degrees. Therefore, this structure can be used to achieve the requirements for moderate or low doses of CTLA4 inhibitors in the clinic.

For another example, in the Fab-Fc-VH(2) asymmetric structure, the HER2-binding domain of the bispecific antibody molecule is a monovalent Fab structure, but its binding ability to HER2 is very close to that of the bivalent parent monoclonal antibody. This structure contains two CTLA4-binding domains formed in tandem, with similar binding ability to CTLA4 as the parent monoclonal antibody, but with reduced T cell activation ability as compared to the parent monoclonal antibody. Therefore, this structure can bring tumor target cells and T cells together to promote the formation of immune synapses, and can be used to achieve the requirements for moderate or low doses of CTLA4 inhibitors in the clinic, while also increasing the ability to specifically target tumors.

Example 5. PD-L1×4-1BB bispecific antibodies

Example 5.1. Background

Programmed death receptor 1 (PD-1) is mainly expressed in immune cells such as T cells. It has two ligands, i.e., programmed death ligand 1 (PD-L1) and PD-L2. PD-L1 is mainly expressed in antigen-presenting cells and a variety of tumor cells. The interaction between PD-L1 and PD-1 can down-regulate the activity of T cells, weaken the secretion of cytokines and play a role in immunosuppression. The expression of the PD-L1 protein can be detected in many human tumor tissues. The microenvironment at the tumor site can induce the expression of PD-L1 on tumor cells, and the expressed PD-L1 facilitates the occurrence and growth of tumors, induces the apoptosis of anti-tumor T cells and further protects the tumor cells from immune attack.

4-1BB (TNFRSF9, CD137) is a transmembrane protein belonging to the TNF receptor superfamily. 4-1BB is a co-stimulatory molecule expressed on a variety of immune cells. It is a multifunctional modulator of immune activity. Its expression is induced in activated T cells, NK cells and other immune cells. 4-1BB activates T cells through trimerization mediated by its ligand 4-1BBL, promoting cell proliferation and cytokine release. Anti-4-1BB agonistic antibodies have the function of inhibiting tumors. The first 4-1BB antibodies to be subjected to clinical trials were Utomilumab from Pfizer and Urelumab (BMS-663513) from Bristol-Myers Squibb. The initial clinical results of urelumab were published in 2008. Although encouraging efficacy was observed in some patients, the data showed Urelumab to cause target and dose-associated hepatotoxicity. Moreover, two patients in the clinical trial died from hepatotoxicity, resulting in the discontinuation of related clinical trials. Utomilumab has better safety enabling the dose to be increased to 10 mg/kg, but still has a poor therapeutic effect.

In this example, we constructed bispecific antibodies targeting both PD-L1 and 4-1BB to improve anti-tumor efficacy and safety through one or more mechanisms of action. Firstly, the PD-L1×4-1BB bispecific antibody can activate T cells by blocking the PD-1/PD-L1 signaling pathway. Secondly, the PD-L1 molecules highly expressed on the tumor cell surfaces can use the bispecific antibody molecules to promote the crosslinking and trimerization of the 4-1BB molecules on the T cell surfaces and activate the downstream signaling pathway, thereby promoting T cell activation and proliferation. Thirdly, the bispecific antibody molecule-mediated T cell activation is limited to the tumor microenvironment, so that the toxic and side effects caused by over-activation of T cells in normal tissues by monoclonal antibodies similar to Urelumab can be avoided.

**Example 5.2. Acquisition of anti-PD-L1 IgG antibodies and anti-4-1BB H2L2 or HCAb antibodies**

**Example 5.2.1. Acquisition of fully human anti-PD-L1 IgG antibodies**

The fully human anti-PD-L1 IgG antibody PR000265 (Table 5-8) used in this example was derived from Harbour H2L2 mice and was found as described in Example 3.2.1.

**Example 5.2.2. Acquisition of fully human anti-4-1BB H2L2 antibodies**

The Harbour H2L2 mouse (Harbour Antibodies BV) is a transgenic mouse carrying an immune repertoire of human immunoglobulins that produces antibodies with intact human antibody variable domains and rat constant domains.

Harbour H2L2 mice were subjected to multiple rounds of immunization with a soluble recombinant human 4-1BB-Fc fusion protein (GenScript Biotech). When the titer of the 4-1BB-specific antibody in the serum of mice was detected to reach a certain level, spleen cells of the mice were taken and fused with a myeloma cell line to obtain hybridoma cells. After multiple rounds of screening and cloning of the hybridoma cells, several monoclonal antibody molecules specifically recognizing 4-1BB were identified. The monoclonal antibodies were further identified, and several candidate antibody molecules were preferentially selected according to parameters such as the binding ability to human 4-1BB, the binding ability to cynomolgus monkey 4-1BB, and the T cell activation ability. The candidate antibody molecules were then subjected to sequence analysis and optimization to obtain several variant sequences. The VL and VH sequences of the antibody were fused to the corresponding human κ light chain constant region and IgG1 heavy chain constant region sequences and expressed to obtain recombinant fully human antibody molecules. The recombinant fully human anti-4-1BB IgG antibodies PR000197 and PR000448 are listed in Table 5-8.

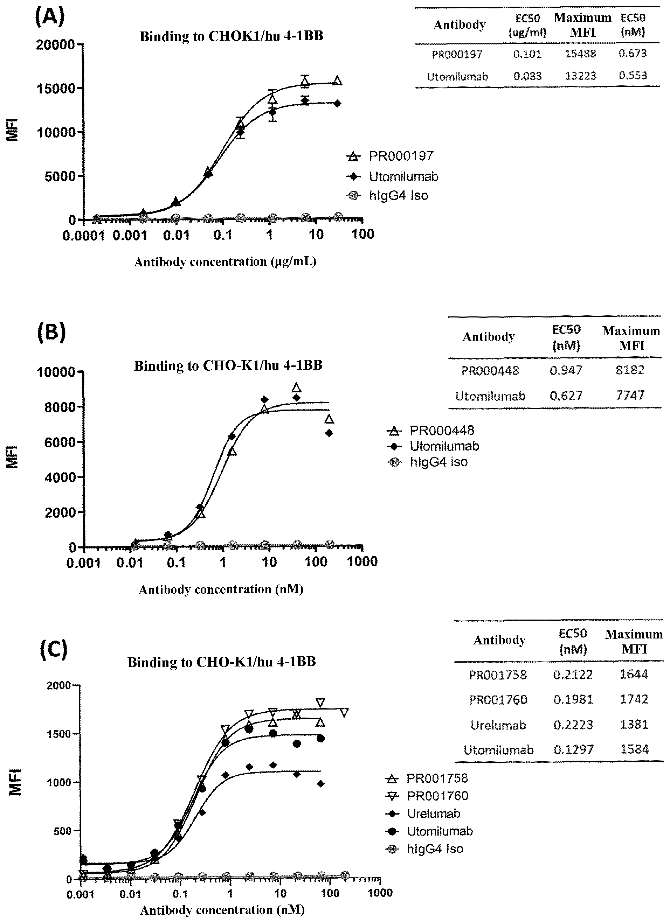

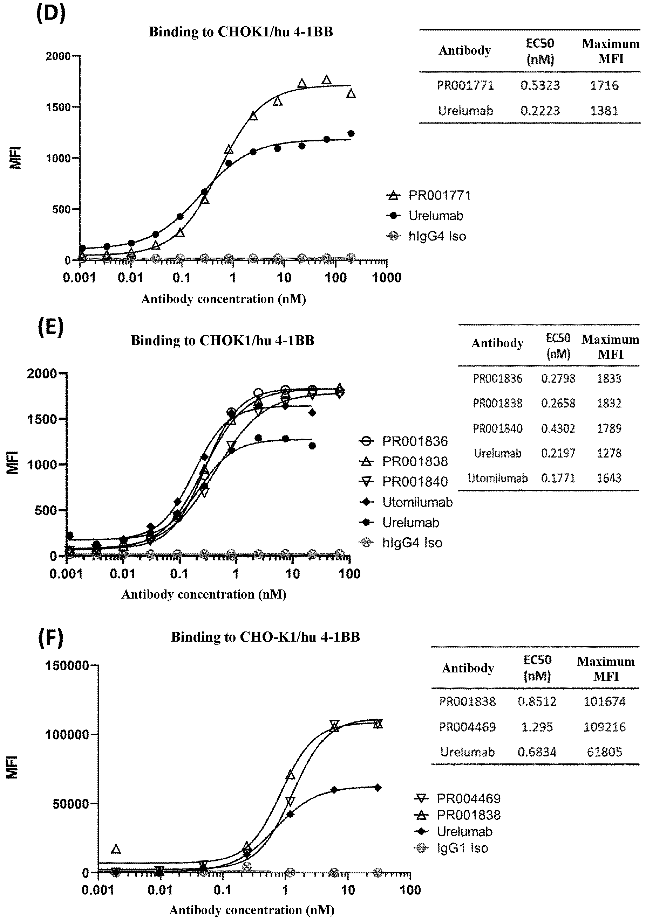

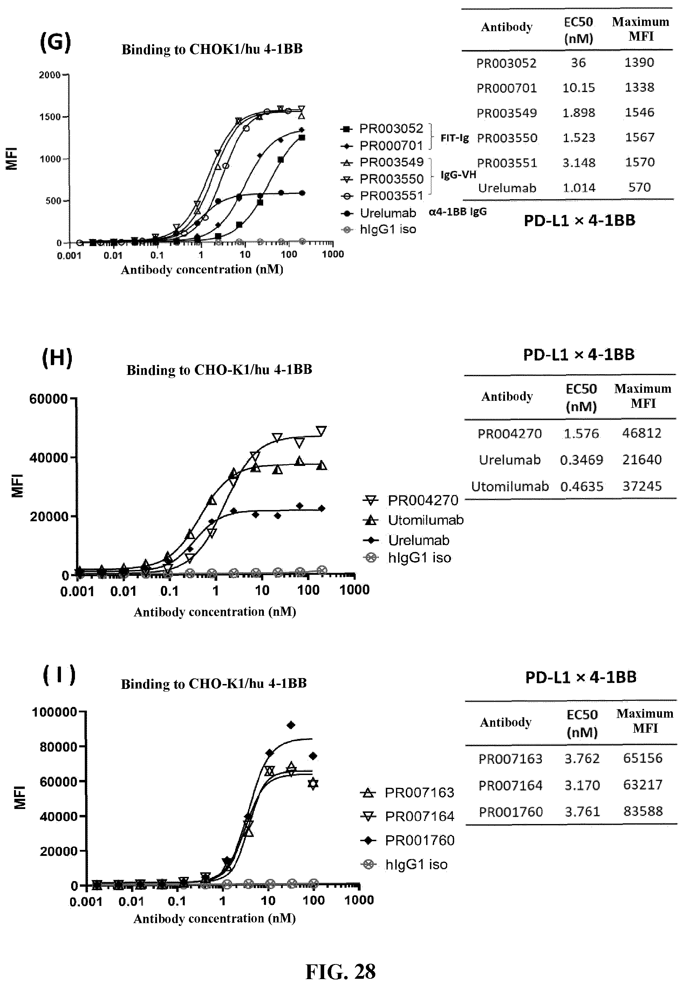

The binding ability of the 4-1BB antibodies PR000197 and PR000448 to a CHO-K1 cell line CHO-K1/hu 4-1BB (GenScript Biotech, M00538) highly expressing human 4-1BB was tested by flow cytometry FACS and the method described in Example 5.6. As shown in FIG. 28 (A-B), the anti-4-1BB antibodies had similar binding ability to Utomilumab.

### Example 5.2.3. Acquisition of fully human anti-4-1BB HCAb antibodies

The Harbour HCAb mouse (Harbour Antibodies BV, WO2010/109165A2) is a transgenic mouse carrying an immune repertoire of human immunoglobulins, capable of producing heavy chain-only antibodies that are only half the size of conventional IgG antibodies. The antibodies produced have only human antibody heavy chain variable domains and mouse Fc constant domains.

Harbour HCAb mice were subjected to multiple rounds of immunization with a soluble recombinant human 4-1BB-Fc fusion protein (ChemPartner) or human 4-1BB-overexpressing NIH-3T3 cells (ChemPartner). When the titer of the 4-1BB-specific antibody in the serum of mice was detected to reach a certain level, spleen cells of the mice were taken, from which B cells were isolated, and the CD138-positive plasma cells were sorted using a mouse plasma cell isolation kit (Miltenyi, #130-092-530). The human VH gene was amplified from plasma cells using conventional molecular biology techniques, and the amplified human VH gene fragments were constructed into mammalian cell expression plasmid pCAG vectors encoding the sequence of the heavy chain Fc region of the human IgG1 antibody. Mammal host cells (e.g., human embryonic kidney cell HEK293) were transfected with the plasmids and allowed to express antibodies to obtain a supernatant with fully human HCAb antibodies. Positive HCAb antibodies were identified by testing the supernatant with HCAb antibodies for binding to CHO-K1 cell CHO-K1/hu4-1BB highly expressing human 4-1BB by FACS. These HCAb antibodies were further identified, and several candidate HCAb antibody molecules were preferentially selected according to parameters such as the binding ability to human 4-1BB, the binding ability to cynomolgus monkey 4-1BB, and the T cell activation ability. The candidate HCAb antibody molecules were then subjected to sequence analysis and optimization to obtain several variant sequences. The VH sequence of the HCAb antibody and the Fc sequence of the heavy chain of human IgG1 were fused and expressed to obtain fully human recombinant HCAb antibody molecules. The recombinant fully human anti-4-1BB HCAb antibodies PR001758, PR001760 and PR001836 are listed in Table 5-8.

The binding ability of the 4-1BB heavy chain antibodies to CHO-K1/hu 4-1BB cells (GenScript Biotech, M00538) was tested by flow cytometry FACS and the method described in Example 5.6. As shown in FIG. 28 (C-F), the 4-1BB heavy chain antibodies had similar binding ability to utomilumab, and had superior binding ability to Urelumab.

### Example 5.3. Construction of bispecific antibody molecules of FIT-Ig structure using anti-PD-L1 IgG antibodies and anti-4-1BB IgG antibodies

In this example, anti-PD-L1 ×4-1BB bispecific antibody molecules of FIT-Ig structures were constructed using the antigen-binding domain Fab of the anti-PD-L1 IgG antibody PR000265 or PR000151 (atezolizumab analog) and the antigen-binding domain Fab of the anti-4-1BB IgG antibody PR000197 or PR000448. The FIT-Ig structures can be designed by referring to WO2015/103072A1, with the structures shown in FIG. 1 (28). The constructed molecules are summarized in Table 5-1. The antibody molecule samples were prepared and analyzed according to the method described in Example 2, with the results summarized in Table 5-2. The sequence numbers corresponding to the polypeptide chains of the bispecific antibody molecules of FIT-Ig structures are listed in Table 5-3.

### Table 5-1. PD-L1×4-1BB bispecific antibody molecules of FIT-Ig structures

| Structure No. | Bispecific antibody molecules | PD-L1 antibody (Fab A) | 4-1BB antibody (Fab B) | 4-1BB Fab position | Linker peptide | Fc type (mutation) |
|---|---|---|---|---|---|---|
| FIT-Ig | PR000701 | PR000265 | PR000197 | Close to Fc | None | Human IgG4 |
| FIT-Ig | PR003052 | PR000151 | PR000448 | Close to Fc | None | Human IgG4 |

### Table 5-2. Expression of PD-L1×4-1BB bispecific antibody molecule proteins of FIT-Ig structures

| Structure No. | Bispecific antibody molecules | Expression system and volume | Yield (mg/L) after first purification | SEC-HPLC purity (%) |
|---|---|---|---|---|
| FIT-Ig | PR000701 | HEK293-F (30ml) | 198.0 | 79.88 |
| FIT-Ig | PR003052 | HEK293-6E (40ml) | 47.5 | 87.76 |

**Table 5-3. Sequence numbers of PD-L1×4-1BB bispecific antibody molecules of FIT-Ig structures**

| Antibody No. | Polypeptide chain 1 | Polypeptide chain 2 | Polypeptide chain 3 |
|---|---|---|---|
| PR000701 | 384 | 371 | 355 |
| PR003052 | 452 | 451 | 355 |

### Example 5.4. Construction of bispecific antibody molecules using anti-PD-L1 IgG antibodies and anti-4-1BB HCAb antibodies

In this example, PD-L1 ×4-1BB bispecific antibody molecules of a variety of structures were constructed using the antigen-binding domain Fab of the anti-PD-L1 IgG antibody PR000265 and the antigen-binding domain VH of the anti-4-1BB HCAb antibody PR001758, PR001760, or PR001836.

In this and subsequent examples, the anti-PD-L1 IgG monoclonal antibody PR000265 was used as a positive control molecule, and was also the parent monoclonal antibody of the PD-L1 end of the PD-L1×4-1BB bispecific antibody molecules. The anti-4-1BB IgG monoclonal antibody Urelumab (IgG4) or Utomilumab (IgG2) was used as a positive control molecule. The PD-L1 ×4-1BB bispecific antibody molecule PR001289 was used as a positive control molecule, the sequences of which are derived from the sequences of the 4-1BB×PD-L1 single-domain antibody disclosed in the patent WO2017/123650A2.

### Example 5.4.1. Construction of molecules of Fab-HCAb symmetric structures

PD-L1×4-1BB bispecific antibody molecules of Fab-HCAb symmetric structures were designed according to the structures described in Example 1.1 using anti-PD-L1 IgG antibodies and anti-4-1BB heavy chain antibodies, with the results summarized in Table 5-4. The antibody molecule samples were prepared and analyzed according to the method described in Example 2, with the results summarized in Table 5-5.

**Table 5-4. PD-L1×4-1BB bispecific antibody molecules of Fab-HCAb symmetric structures**

| Structure No. | Bispecific antibody molecules | PD-L1 antibody (Fab) | 4-1BB antibody (VH_B) | First linker peptide (between Fab and VH_B) | Second linker peptide (between VH_B and CH2) | Fc type (mutation) |
|---|---|---|---|---|---|---|
| 1 | PR004270 | PR000265 | PR001760 | H1_15 | Human IgG1 hinge (C220S) | Human IgG 1 (L234A, L235A) |
| 2 | PR007163 | PR000265 | PR001760 | None | Human IgG1 hinge (C220S) | Human IgG 1 (L234A, L235A) |
| 1 | PR007164 | PR000265 | PR001760 | None | Human IgG1 hinge (C220S) | Human IgG 1 (L234A, L235A) |

**Table 5-5. Expression of PD-L1×4-1BB bispecific antibody molecule proteins of Fab-HCAb symmetric structures**

| Structure No. | Bispecific antibody molecules | Expression system and volume | Plasmid transfection ratio (short chain:long chain) | Yield (mg/L) after first purification | SEC-HPLC purity (%) |
|---|---|---|---|---|---|
| 1 | PR004270 | HEK293-6E (40ml) | 3 : 2 | 77.50 | 92.33 |
| 2 | PR007163 | HEK293 (100ml) | 3 : 1 | 13 | 97.84 |
| 1 | PR007164 | HEK293 (100ml) | 3 : 1 | 47 | 93.37 |

**Example 5.4.2. Construction of molecules of IgG-VH tetravalent symmetric structure**

PD-L1×4-1BB bispecific antibody molecules of IgG-VH tetravalent symmetric structures were designed according to the structures described in Example 1.2 using anti-PD-L1 IgG antibodies and anti-4-1BB heavy chain antibodies, with the results summarized in Table 5-6. The antibody molecule samples were prepared and analyzed according to the method described in Example 2, with the results summarized in Table 5-7.

**Table 5-6. PD-L1×4-1BB bispecific antibody molecules of IgG-VH tetravalent symmetric structures**

| Structure No. | Bispecific antibody molecules | PD-L1 antibody (IgG) | 4-1BB antibody (VH_B) | VH_B positionrelative to IgG | Linker peptide | Fc type (mutation) |
|---|---|---|---|---|---|---|
| 5 | PR003549 | PR000265 | PR001758 | C-terminus of heavy chain | H1_15-RT | Human IgG1 (L234A, L235A, P329G) |
| 5 | PR003550 | PR000265 | PR001760 | C-terminus of heavy chain | H1_15-RT | Human IgG1 (L234A, L235A, P329G) |
| 5 | PR003551 | PR000265 | PR001836 | C-terminus of heavy chain | H1_15-RT | Human IgG1 (L234A, L235A, P329G) |

**Table 5-7. Expression of PD-L1×4-1BB bispecific antibody molecule proteins of IgG-VH tetravalent symmetric structures**

| Structure No. | Bispecific antibody molecules | Expression system and volume | Yield (mg/L) after first purification | SEC-HPLC purity (%) |
|---|---|---|---|---|
| 5 | PR003549 | HEK293-F (30ml) | 24.51 | 98.31 |
| 5 | PR003550 | HEK293-F (30ml) | 42.16 | 95.41 |
| 5 | PR003551 | HEK293-F (30ml) | 2.28 | 99.39 |

**Example 5.4.3. Sequences of PD-L1×4-1BB bispecific antibody molecules and control molecules**

The sequence numbers corresponding to the sequences of the PD-L1 ×4-1BB bispecific antibody molecules constructed in this example, the corresponding parent monoclonal antibody molecules such as the PD-L1 monoclonal antibody and the 4-1BB monoclonal antibody, and the control molecules are listed in Table 5-8, Table 5-9 and Table 5-10. The structure numbers in Table 5-10 correspond to those in Table 1-1 and FIG. 1.The sequence numbers of the corresponding CDR sequences of the first and second antigen-binding domains of the bispecific antibody molecules are listed in Table 5-11.

**Table 5-8. Control molecules and parent monoclonal antibodies**

| Antibody No. | Antibody |
|---|---|
| PR000265 | Anti-PD-L1 91G3H5H3 (D54E), hIgG1 (N297A) |
| PR000151 | Anti-PD-L1 monoclonal antibody atezolizumab analog, hIgG1 (N297A) |
| PR000448 | Anti-4-1BB 79B10G8D4 (H: N52Q; L: F2I), hIgG4 (S228P) |
| PR000197 | Anti-4-1BB 79B10G8D4, hIgG4 (S228P) |
| PR001758 | Anti-4-1BB heavy-chain antibody 1016P0010B11, hIgG1 |
| PR001760 | Anti-4-1BB heavy-chain antibody 1016P0011G10, hIgG1 |
| PR001836 | Anti-4-1BB heavy-chain antibody 1016P0020G4, hIgG1 |
| PR000628 | Anti-4-1BB monoclonal antibody urelumab analog, hIgG4 (S228P) |

(continued)

| Antibody No. | Antibody |
|---|---|
| PR000483 | Anti-4-1BB monoclonal antibody utomilumab analog, hIgG2 |
| PR001289 | PD-L1×4-1BB bispecific antibody molecule, with only one different polypeptide chain, the sequences of which are derived from the sequence of the 4-1BB×PD-L1 single-domain antibody disclosed in the patent WO2017/123650A2, hIgG1(LALA) |

**Table 5-9. Sequence numbers of sequences and CDR sequences of control molecules and parent monoclonal antibodies**

| Antibody No. | Light chain | Heavy chain | VL | VH | LCDR1 | LCDR2 | LCDR3 | HCDR1 | HCDR2 | HCDR3 |
|---|---|---|---|---|---|---|---|---|---|---|
| PR000265 | 353 | 308 | 282 | 240 | 167 | 188 | 211 | 15 | 69 | 122 |
| PR000151 | 349 | 302 | 284 | 235 | 169 | 190 | 213 | 16 | 64 | 124 |
| PR000197 | 350 | 304 | 285 | 237 | 170 | 191 | 214 | 18 | 66 | 126 |
| PR000448 | 355 | 311 | 289 | 242 | 170 | 191 | 214 | 18 | 71 | 126 |
| PR001758 | None | 320 | None | 252 | None | None | None | 27 | 79 | 137 |
| PR001760 | None | 321 | None | 253 | None | None | None | 28 | 80 | 138 |
| PR001836 | None | 323 | None | 255 | None | None | None | 29 | 82 | 138 |
| PR000628 | 358 | 314 | 292 | 246 | 175 | 195 | 219 | 18 | 73 | 131 |
| PR000483 | 356 | 312 | 290 | 243 | 174 | 194 | 218 | 22 | 72 | 130 |
| PR001289 | None | 494 | None | None | None | None | None | None | None | None |

**Table 5-10. Sequence numbers of PD-L1×4-1BB bispecific antibody molecules of this example**

| Structure No. | Antibody No. | Polypeptide chain 1 (short chain) | Polypeptide chain 2 (long chain) |
|---|---|---|---|
| 5 | PR003549 | 353 | 460 |
| 5 | PR003550 | 353 | 461 |
| 5 | PR003551 | 353 | 462 |
| 1 | PR004270 | 371 | 486 |
| 2 | PR007163 | 353 | 521 |
| 1 | PR007164 | 371 | 522 |

**Table 5-11. Sequence numbers of CDRs of antigen-binding domains of PD-L1×4-1BB bispecific antibody molecules**

| Structure No. | Antibody No. | Antigen-binding domain No. | LCDR1 | LCDR2 | LCDR3 | HCDR1 | HCDR2 | HCDR3 |
|---|---|---|---|---|---|---|---|---|
| 1 | PR004270, PR007164 | #1 | 167 | 188 | 211 | 15 | 69 | 122 |
| | | #2 | None | None | None | 28 | 80 | 138 |
| 2 | PR007163 | #1 | 167 | 188 | 211 | 15 | 69 | 122 |
| | | #2 | None | None | None | 28 | 80 | 138 |
| 5 | PR003549 | #1 | 167 | 188 | 211 | 15 | 69 | 122 |
| | | #2 | None | None | None | 27 | 79 | 137 |

(continued)

| Structure No. | Antibody No. | Antigen-binding domain No. | LCDR1 | LCDR2 | LCDR3 | HCDR1 | HCDR2 | HCDR3 |
|---|---|---|---|---|---|---|---|---|
| 5 | PR003550 | #1 | 167 | 188 | 211 | 15 | 69 | 122 |
| | | #2 | None | None | None | 28 | 80 | 138 |
| 5 | PR003551 | #1 | 167 | 188 | 211 | 15 | 69 | 122 |
| | | #2 | None | None | None | 29 | 82 | 138 |
| FIT-Ig | PR000701 | #1 | 167 | 188 | 211 | 15 | 69 | 122 |
| | | #2 | 170 | 191 | 214 | 18 | 66 | 126 |
| FIT-Ig | PR003052 | #1 | 169 | 190 | 213 | 16 | 64 | 124 |
| | | #2 | 170 | 191 | 214 | 18 | 71 | 126 |

**Example 5.5. Binding to PD-L1**

This example is intended to investigate the binding activity of the PD-L1×4-1BB bispecific antibody molecules to PD-L1.

**Example 5.5.1. Binding to CHO-K1 cells CHO-K1/hPDL1 highly expressing human PD-L1**

The binding ability of the antibody molecules to a CHO-K1 cell line CHO-K1/hPDL1 (GenScript Biotech, M00543) highly expressing human PD-L1 was tested by flow cytometry FACS. Specifically, CHO-K1/hPDL1 cells were digested and resuspended in a complete medium, with the cell density adjusted to $1\times10^6$ cells/mL. Thereafter, the cells were seeded in a 96-well V-bottom plate (Corning, #3894) at 100 $\mu$L/well and centrifuged at 4 °C for 5 min, and the supernatant was discarded. Then, the serially diluted antibody molecules were added to the 96-well plate at 100 $\mu$L/well, and the mixtures were well mixed. The antibody molecules might serially diluted 3-fold from the highest final concentration 200 nM to obtain a total of 12 concentrations. hIgG1 iso (CrownBio, #C0001) was used as an isotype control. The cells were incubated at 4 °C for 1 h away from light. Then, the cells in each well were rinsed twice with 100 $\mu$L of pre-cooled FACS buffer (PBS buffer containing 0.5% BSA) and centrifuged at 500 g at 4 °C for 5 min, and then the supernatant was discarded. Thereafter, a fluorescent secondary antibody (Goat human IgG (H + L) Alexa Fluor 488 conjugation, Thermo, #A11013, 1: 1000 dilution) was added at 100 $\mu$L/well, and the plate was incubated at 4 °C for 1 h away from light. Then, the cells in each well were then rinsed twice with 200 $\mu$L of pre-cooled FACS buffer and centrifuged at 500 g at 4 °C for 5 min, and then the supernatant was discarded. Finally, a pre-cooled FACS buffer was added at 200 $\mu$L/well to resuspend the cells. Fluorescence signal values were read using a BD FACS CANTOII flow cytometer or an ACEA NovoCyte flow cytometer, and the data were processed and analyzed using FlowJo v10 (FlowJo, LLC) software. The data were processed and analyzed by plotting using GraphPad Prism 8 software, and binding curves of the antibodies to target cells, $EC_{50}$ values and other parameters were obtained through four-parameter nonlinear fitting.

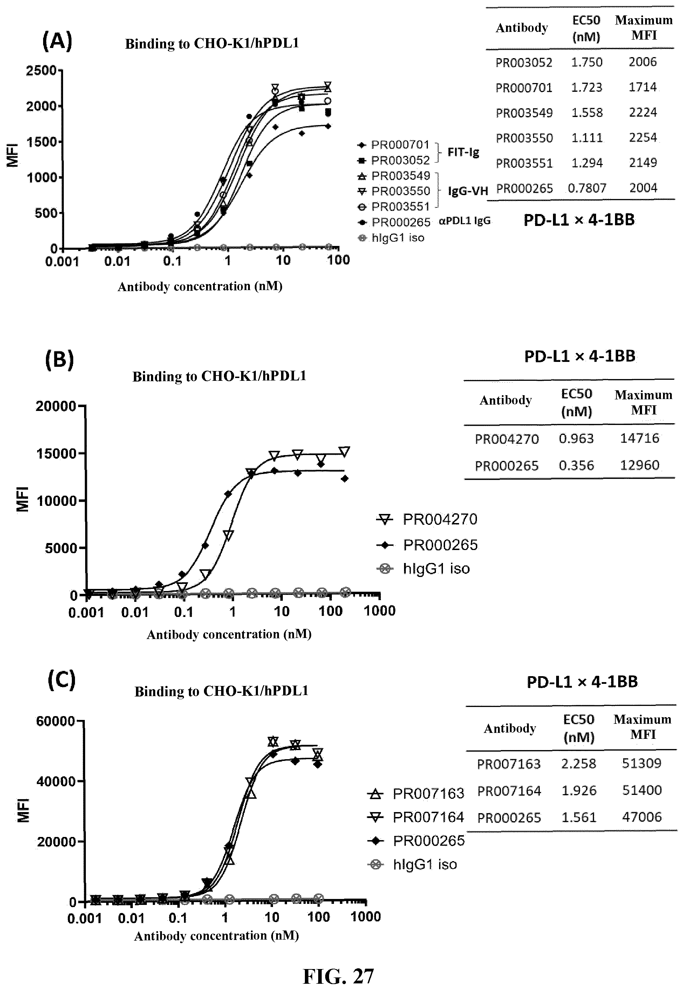

In this examples, the anti-PD-L1 monoclonal antibody PR000265 was used as a positive control molecule. It was also the parent monoclonal antibody of the PD-L1 end of the PD-L1 ×4-1BB bispecific antibody molecules. The results are shown in FIG. 27 and Table 5-12.

As shown in FIG. 27 (A), the bispecific antibody molecules of IgG-VH tetravalent symmetric structures (PR003549, PR003550, and PR003551) had similar binding ability to PD-L1 to the parent monoclonal antibody PR000265, and had slightly superior $EC_{50}$ values and maximum MFI values for binding to PD-L1 than the bispecific antibody molecules of FIT-Ig structures (PR000701 and PR003052).

As shown in FIG. 27 (B), the bispecific antibody molecule of a Fab-HCAb symmetric structure (PR004270) had similar binding ability to PD-L1 to the parent monoclonal antibody, and had a slightly weaker $EC_{50}$ value but a higher maximum MFI value for binding to PD-L1 than the parent monoclonal antibody.

As shown in FIG. 27 (C), the bispecific antibody molecules of Fab-HCAb symmetric structures, whether the molecule of the structure (1) (PR007164) or the molecule of the structure (2) (PR007163), had almost identical binding activity to PD-L1, indicating that the fusion of the CL of Fab to the heavy chain where VH_B is located does not affect the binding activity of the Fab end.

### Table 5-12. Binding to CHO-K1/hPDL1

| Antibody | $EC_{50}$ (nM) | Maximum MFI | Antibody | $EC_{50}$ (nM) | Maximum MFI | Antibody | $EC_{50}$ (nM) | Maximum MFI |
|---|---|---|---|---|---|---|---|---|
| PR003052 | 1.75 | 2006 | PR004270 | 0.963 | 14716 | PR007163 | 2.258 | 51309 |
| PR000701 | 1.723 | 1714 | PR000265 | 0.356 | 12960 | PR007164 | 1.926 | 51400 |
| PR003549 | 1.558 | 2224 | | | | PR000265 | 1.561 | 47006 |
| PR003550 | 1.111 | 2254 | | | | | | |
| PR003551 | 1.294 | 2149 | | | | | | |
| PR000265 | 0.7807 | 2004 | | | | | | |
| Experiment 1 | | | Experiment 2 | | | Experiment 3 | | |

### Example 5.6. Binding to 4-1BB

This example is intended to investigate the binding activity of the PD-L1×4-1BB bispecific antibody molecules to 4-1BB.

The binding abilities of the antibody molecules to a CHO-K1 cell strain highly expressing human 4-1BB (CHO-K1/hu4-1BB, GenScript, M00538) and a CHO-K1 cell strain highly expressing cynomolgus monkey 4-1BB (CHO-K1/cyno4-1BB, GenScript, M00569) were determined by flow cytometry FACS. Specifically, the cells were digested and resuspended in a complete medium, with the cell density adjusted to $2\times10^6$ cells/mL. Then, the cells were seeded in a 96-well V-bottom plate (Corning, #3894) at 100 μL/well ($2\times10^5$ cells/well) and centrifuged at 4 °C for 5 min, and the supernatant was discarded. Then, the serially diluted antibody molecules were added to the 96-well plate at 100 μL/well, and the mixtures were well mixed. The antibody molecules might serially diluted 3-fold from the highest final concentration 200 nM to obtain a total of 12 concentrations. hIgG1 iso (CrownBio, #C0001) was used as an isotype control. The cells were incubated at 4 °C for 1 h away from light. Then, the cells in each well were rinsed twice with 100 μL of pre-cooled FACS buffer (PBS buffer containing 0.5% BSA) and centrifuged at 500 g at 4 °C for 5 min, and then the supernatant was discarded. Thereafter, a fluorescent secondary antibody (Goat human IgG (H + L) Alexa Fluor 488 conjugation, Thermo, #A11013, 1: 1000 dilution) was added at 100 μL/well, and the plate was incubated at 4 °C for 1 h away from light. Then, the cells in each well were then rinsed twice with 200 μL of pre-cooled FACS buffer and centrifuged at 500 g at 4 °C for 5 min, and then the supernatant was discarded. Finally, a pre-cooled FACS buffer was added at 200 μL/well to resuspend the cells. Fluorescence signal values were read using a BD FACS CANTOII flow cytometer or an ACEA NovoCyte flow cytometer, and the data were processed and analyzed using FlowJo v10 (FlowJo, LLC) software. The data were processed and analyzed by plotting using GraphPad Prism 8 software, and binding curves of the antibodies to target cells, $EC_{50}$ values and other parameters were obtained through four-parameter nonlinear fitting.

In this example, the anti-4-1BB monoclonal antibody Urelumab or Utomilumab was used as a positive control molecule.

### Example 5.6.1. Binding to CHO-K1 cells CHO-K1/hu4-1BB highly expressing human 4-1BB

The results are shown in FIG. 28 and Table 5-13.

As shown in FIG. 28 (G), the bispecific antibody molecules of IgG-VH tetravalent symmetric structures (PR003549, PR003550, and PR003551) had superior binding ability to human 4-1BB to the bispecific antibody molecules of FIT-Ig structures (PR000701 and PR003052), and had superior maximum MFI values to the positive control Urelumab.

As shown in FIG. 28 (H), the bispecific antibody molecule of a Fab-HCAb symmetric structure (PR004270) had a superior binding ability to human 4-1BB in terms of the maximum MFI value to the positive controls Urelumab and Utomilumab.

As shown in FIG. 28 (I), the bispecific antibody molecules of Fab-HCAb symmetric structures, whether the molecule of the structure (1) (PR007164) or the molecule of the structure (2) (PR007163), had almost identical binding activity to 4-1BB, and were comparable to the parent monoclonal antibody PR001760.

### Table 5-13. Binding to CHO-K1/hu 4-1BB

| Antibody | $EC_{50}$ (nM) | Maximum MFI | Antibody | $EC_{50}$ (nM) | Maximum MFI | Antibody | $EC_{50}$ (nM) | Maximum MFI |
|---|---|---|---|---|---|---|---|---|
| PR000197 | 0.673 | 15488 | PR000448 | 0.947 | 8182 | PR001838 | 0.8512 | 101674 |

(continued)

| Antibody | EC$_{50}$ (nM) | Maximum MFI | Antibody | EC$_{50}$ (nM) | Maximum MFI | Antibody | EC$_{50}$ (nM) | Maximum MFI |
|---|---|---|---|---|---|---|---|---|
| Utomilumab | 0.553 | 13223 | Utomilumab | 0.627 | 7747 | PR004469 | 1.295 | 109216 |
|  |  |  |  |  |  | Urelumab | 0.6834 | 61805 |
| **Antibody** | **EC$_{50}$ (nM)** | **Maximum MFI** | **Antibody** | **EC$_{50}$ (nM)** | **Maximum MFI** |  |  |  |
| PR001758 | 0.2122 | 1644 | PR001836 | 0.2798 | 1833 |  |  |  |
| PR001760 | 0.1981 | 1742 | PR001838 | 0.2658 | 1832 |  |  |  |
| PR001771 | 0.5323 | 1716 | PR001840 | 0.4302 | 1789 |  |  |  |
| Urelumab | 0.2223 | 1381 | Urelumab | 0.2197 | 1278 |  |  |  |
| Utomilumab | 0.1297 | 1584 | Utomilumab | 0.1771 | 1643 |  |  |  |
| **Antibody** | **EC$_{50}$ (nM)** | **Maximum MFI** | **Antibody** | **EC$_{50}$ (nM)** | **Maximum MFI** | **Antibody** | **EC$_{50}$ (nM)** | **Maximum MFI** |
| PR003052 | 36 | 1390 | PR004270 | 1.576 | 46812 | PR007163 | 3.762 | 65156 |
| PR000701 | 10.15 | 1338 | Urelumab | 0.3469 | 21640 | PR007164 | 3.17 | 63217 |
| PR003549 | 1.898 | 1546 | Utomilumab | 0.4635 | 37245 | PR001760 | 3.761 | 83588 |
| PR003550 | 1.523 | 1567 |  |  |  |  |  |  |
| PR003551 | 3.148 | 1570 |  |  |  |  |  |  |
| Urelumab | 1.014 | 570 |  |  |  |  |  |  |

## Example 5.6.2. Binding to CHO-K1 cell CHO-K1/cyno 4-1BB highly expressing cynomolgus monkey 4-1BB

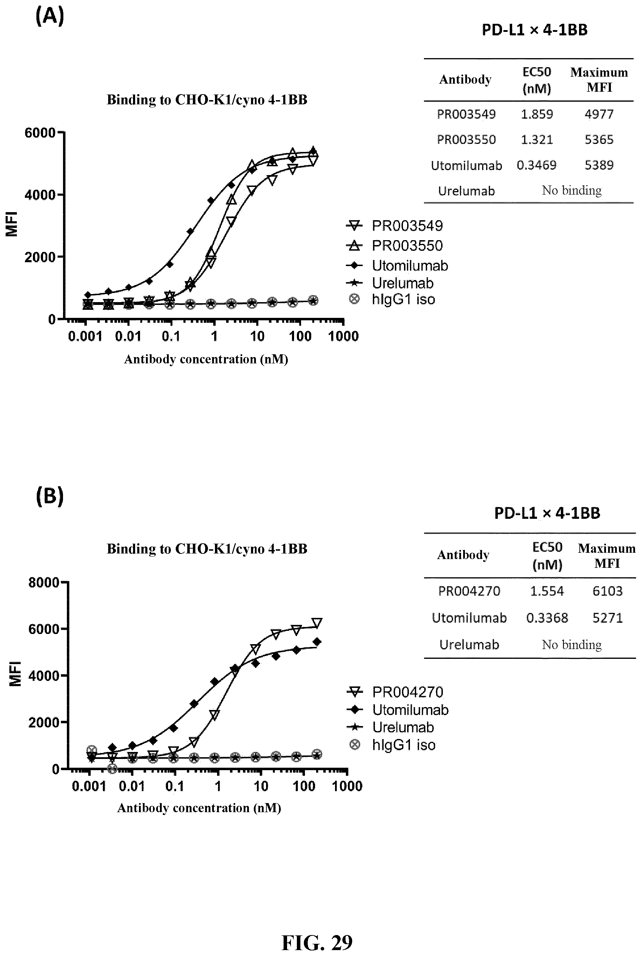

As shown in FIG. 29 and Table 5-14, the bispecific antibody molecules of this example were able to bind to cynomolgus monkey 4-1BB, whereas Urelumab was unable to bind to cynomolgus monkey 4-1BB. The binding ability of the bispecific antibody molecules to cynomolgus monkey 4-1BB (FIG. 29, A-B) was comparable to their binding ability to human 4-1BB (FIG. 28, G-H), with similar binding EC$_{50}$.

**Table 5-14. Binding to CHO-K1/cyno 4-1BB**

| Antibody | EC$_{50}$ (nM) | Maximum MFI | Antibody | EC$_{50}$ (nM) | Maximum MFI |
|---|---|---|---|---|---|
| PR003549 | 1.859 | 4977 | PR004270 | 1.554 | 6103 |
| PR003550 | 1.321 | 5365 | Utomilumab | 0.3368 | 5271 |
| Utomilumab | 0.3469 | 5389 | Urelumab | No binding | |
| Urelumab | No binding | | | | |
| **Experiment 1** | | | **Experiment 2** | | |

## Example 5.7. T cell specific activation mediated by target cells highly expressing PD-L1

This example is intended to investigate the activity of the PD-L1 ×4-1BB bispecific antibody molecules to activate T cells by binding to 4-1BB in the presence of target cells. The target cells may be cells expressing PD-L1 at different levels, e.g., CHO-K1/hPDL1 (GenScript, M00543) highly expressing human PD-L1 or MDA-MB-231 (ATCC, HTB-26) highly expressing human PD-L1. The effector cells may be isolated human PBMCs or T cells.

Specifically, a 96-well plate (Corning, #3599) was coated first with 0.3 μg/mL anti-CD3 antibody OKT3 (Thermo, #16-0037-81) at 100 μL/well. Then, the density of human T cells (isolated from human PBMCs with a T cell isolation kit (Miltenyi, #130-096-535)) was adjusted to $2\times10^6$ cells/mL, and the density of target cells was adjusted to $3\times10^5$ cells/mL. The two cell suspensions were each seeded into a 96-well plate at 50 μL/well, with a final effector-to-target ratio of 20:3. Then, antibody molecules at different concentrations were added at 100 μL/well, wherein the antibody concentrations may be the final concentration of (10 nM, 1 nM) or 20 nM, or a total of 8 concentrations obtained by a 5-fold gradient dilution from the highest final concentration of 20 nM; and two duplicate wells were set for each concentration. hIgG1 iso (CrownBio, #C0001) and hIgG4 iso (CrownBio, #C0045) were used as controls. The 96-well plate was incubated in an incubator at 37 °C with 5% $CO_2$ for 3 days. Supernatants after 48 h and 72 h of culture were each collected. The IL-2 concentration in the 48-hour supernatant was determined using an IL-2 ELISA kit (Thermo, #88-7025-88), and the IFN-γ concentration in the 72-hour supernatant was determined using an IFN-γ ELISA kit (Thermo, #88-7316-77). The ELISA assay was performed by referring to the instructions of relevant kit. The data were processed and analyzed by plotting using a GraphPad Prism 8 software.

In this example, the anti-4-1BB monoclonal antibody Urelumab was used as a positive control molecule.

## Example 5.7.1. CHO-K1/hPDL1-mediated T cell specific activation

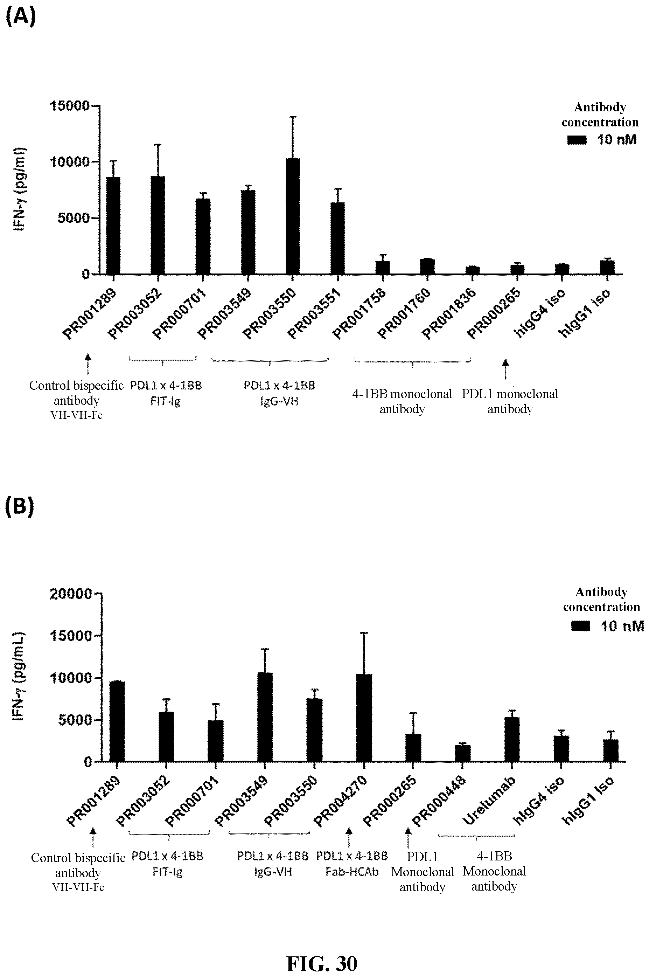

As shown in FIG. 30, in a system where a target cell CHO-K1/hPDL1 was mixed with a T cell, crosslinking-independent anti-4-1BB monoclonal antibody Urelumab was able to activate the T cell to release IFN-γ, while crosslinking-dependent anti-4-1BB monoclonal antibodies (PR000448, PR001758, PR001760, and PR001836) were hardly able to activate the T cell. The bispecific antibody molecules of FIT-Ig structures (PR003052 and PR000701), the bispecific antibody molecules of IgG-VH tetravalent symmetric structures (PR003549, PR003550, and PR003551) and the bispecific antibody molecule of a Fab-HCAb structure (PR004270) were all able to activate the T cell to release cytokines. This indicates that the activation of T cells by the bispecific antibody molecules is dependent on the specific activation of target cells. Moreover, the bispecific antibody molecules of IgG-VH tetravalent symmetric structures (PR003549 and PR003550) and the bispecific antibody molecule of a Fab-HCAb structure (PR004270) were able to cause higher release levels of cytokines and showed stronger T cell activation ability than the bispecific antibody molecules of FIT-Ig structures (PR003052 and PR000701); and those bispecific antibody molecules were superior to Urelumab. PR003549 and PR004270 had comparable or even stronger T cell activation ability than the control bispecific antibody molecule (PR001289).

## Example 5.7.2. MDA-MB-231-mediated T cell specific activation

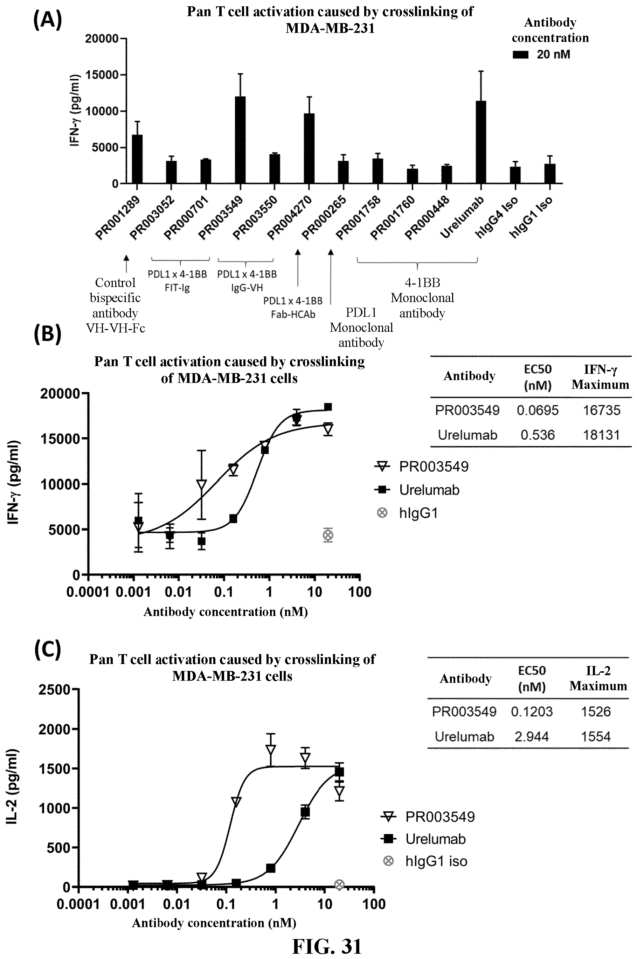

As shown in FIG. 31 (A), in a system where a target cell MDA-MB-231 was mixed with a T cell, the bispecific antibody of an IgG-VH tetravalent symmetric structure (PR003549) and the bispecific antibody of a Fab-HCAb structure (PR004270) had similar T cell activation ability; and those bispecific antibodies were superior to the bispecific antibodies of FIT-Ig structures (PR003052 and PR000701) and a control bispecific antibody molecule (PR001289).

As shown in FIG. 31 (B-C) and Table 5-15, in a system where a target cell MDA-MB-231 was mixed with a T cell, the bispecific antibody molecule of an IgG-VH tetravalent symmetric structure (PR003549) had stronger T cell activation ability than Urelumab, and was able to better promote the production of IFN-γ (B) and IL-2 (C).

### Table 5-15. Levels of cytokines

| Antibody | IFN-γ | | IL-2 | |
|---|---|---|---|---|
| | $EC_{50}$ (nM) | Maximum | $EC_{50}$ (nM) | Maximum |
| PR003549 | 0.0695 | 16735 | 0.1203 | 1526 |
| Urelumab | 0.536 | 18131 | 2.944 | 1554 |

## Example 5.8. Mixed Lymphocyte Reaction (MLR)

This example is intended to investigate the T cell activation effect of PD-L1×4-1BB bispecific antibody molecules by the mixed lymphocyte reaction (MLR).

In the first step, monocytes were isolated from PBMCs (MT-Bio) of a first donor using CD14 magnetic beads (Meltenyi, #130-050-201) according to the instructions of the relevant kit. Then, 50 ng/mL of recombinant human IL-4 (PeproTech, #200-02-A) and 100 ng/mL of recombinant human GM-CSF (PeproTech, #300-03-A) were added, and after 7 days of induction at 37 °C, immature dendritic cells (iDC cells) were obtained. 1 μg/mL lipopolysaccharide (LPS, Sigma, #L6529) was then added, and after 24 h of induction, mature dendritic cells (mDC cells) were obtained. In the second step, T lymphocytes were isolated from PBMCs (MT-Bio) of a second donor using a T cell isolation kit (Meltenyi, #130-096-535). In the third step, the obtained T cells and mDC cells were seeded in a 96-well plate (T cells at $1\times10^5$/well and mDC cells at $2\times10^4$/well) at a ratio of 5:1. Then, antibody molecules at different concentrations were added at 50 μL/well, wherein the antibody concentration may be the final concentration of (10 nM, 1 nM), or a total of 8 concentrations obtained by a 3-fold gradient dilution from the highest final concentration of 50 nM; and two duplicate wells were set for each concentration.

hIgG1 iso (CrownBio, #C0001) or a blank well was used as a control. The cells were incubated in an incubator at 37 °C with 5% $CO_2$ for 5 days. In the fourth step, supernatants on day 3 and on day 5 were each collected. The IL-2 concentration in the 3-day supernatant was determined using an IL-2 ELISA kit (Thermo, #88-7025-77), and the IFN-γ concentration in the 5-day supernatant was determined using an IFN-γ ELISA kit (Thermo, #88-7316-88). The ELISA assay was performed by referring to the instructions of relevant kit.

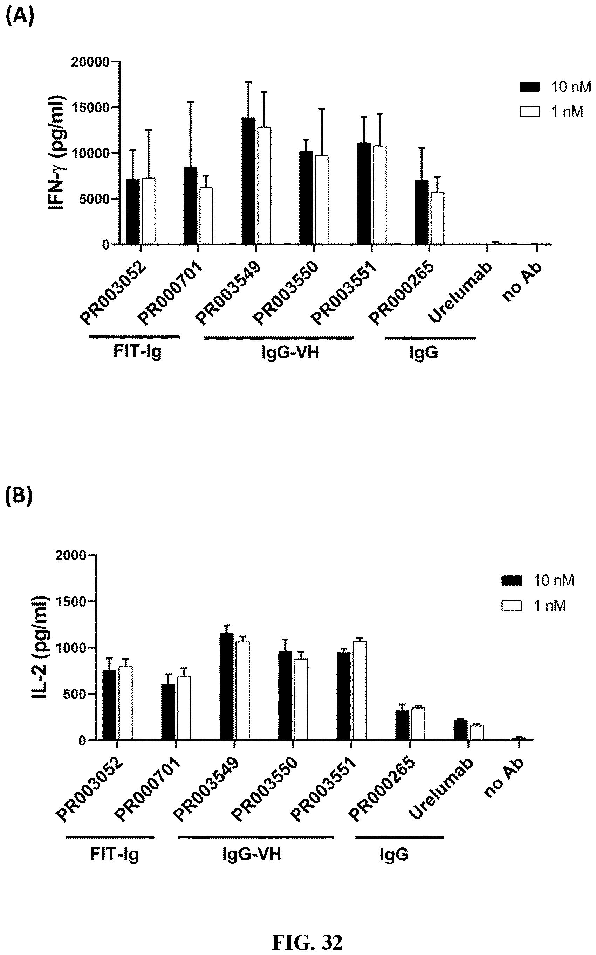

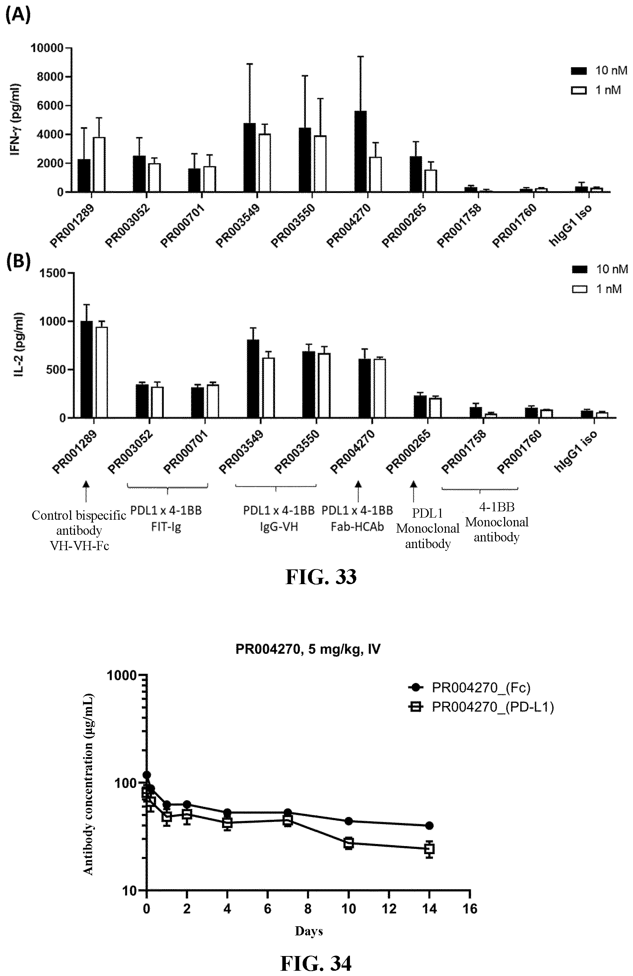

As shown in FIG. 32 and FIG. 33, in two independent MLR experiments (with different donor pairings), the anti-4-1BB monoclonal antibodies had a limited T cell activation effect and weak cytokine production ability; while the anti-PD-L1 monoclonal antibodies had a relatively significant activation effect. The bispecific antibody molecules were able to further improve the function of T cells and were superior to the anti-PD-L1 monoclonal antibodies.

As shown in FIG. 32, the bispecific antibody molecules of IgG-VH tetravalent symmetric structures (PR003549, PR003550, and PR003551) were able to cause higher release levels of cytokines and showed stronger T cell activation ability than the antibody molecules of FIT-Ig structures (PR003052 and PR000701); and those bispecific antibody molecules were superior to the anti-PD-L1 monoclonal antibodies.

As shown in FIG. 33, the bispecific antibody molecules of IgG-VH tetravalent symmetric structures (PR003549 and PR003550) and the bispecific antibody molecule of a Fab-HCAb structure (PR004270) were able to cause higher release levels of cytokines and showed stronger T cell activation ability than the bispecific antibody molecules of FIT-Ig structures (PR003052 and PR000701). Moreover, the bispecific antibody molecules of IgG-VH structures or Fab-HCAb structures were able to stimulate the production of more IFN-γ as compared to the control bispecific antibody molecule (PR001289).

## Example 5.9. Pharmacokinetic study

In this example, the pharmacokinetic properties of the PD-L1×4-1BB bispecific antibody molecule of a Fab-HCAb symmetric structure (PR004270) in mice were investigated.

The test was performed as follows: for each test antibody molecule, 6 female BALB/c mice weighing 18-22 g were selected and were administered with the bispecific antibody by intravenous injection at a dose of 5 mg/kg. The whole blood of 3 mice in one group was collected before the administration and 15 min, 24 h (1 day), 4 days and 10 days after the administration, and the whole blood of 3 mice in the other group was collected before the administration and 5 h, 2 days, 7 days and 14 days after the administration. The whole blood was left to stand for 30 min for coagulation and centrifuged, and the isolated serum sample was cryopreserved at -80 °C until it was taken for analysis. In this example, the drug concentration in the serum of mice was quantitatively determined by two ELISA methods. The ELISA method I, namely the Fc end detection (overall detection) method, was performed by capturing the antibody containing human Fc in the serum of mice using a goat anti-human Fc polyclonal antibody coating a 96-well plate, and then adding an HRP-labeled goat anti-human Fc secondary antibody; and the ELISA method II, namely the PD-L1-end detection (functional domain detection) method, was performed by capturing an antibody specifically recognizing PD-L1 in the serum of mice using a human PD-L1 protein coating a 96-well plate, and then adding an HRP-labeled goat anti-human Fc secondary antibody. Pharmacokinetic parameters were analyzed using Phoenix WinNonlin software version 8.2 by non-compartmental analysis (NCA).

As shown in FIG. 34 and Table 5-16, the bispecific antibody molecule of a Fab-HCAb structure (PR004270) had a similar serum half-life $t_{1/2}$ value to a conventional IgG antibody, and the $t_{1/2}$ value was more than 10 days as measured by the functional domain detection method.

### Table 5-16. Pharmacokinetics of PR004270 in BALB/c mice

| Bispecific antibody molecules | PR004270 | |
|---|---|---|
| Animals (number) | BALB/c mice (n = 6) | |
| Antibody dosage | 5 mg/kg, I.V. | |
| PK parameters | Fc end detection | PD-L1 end detection |
| T1/2 (hour) | 465.6 | 256.5 |
| Vd (mL/kg) | 75.7 | 83.9 |
| AUC (μg*hour/mL) | 17,536 | 13,126 |
| Cl (mL/hour/kg) | 0.11 | 0.23 |
| C0 (μg/mL) | 119.7 | 81.7 |

## Example 5.10. Summary

In this example, PD-L1 ×4-1BB bispecific antibody molecules of a variety of structures are constructed using the antigen-binding domain Fab of the anti-PD-L1 IgG antibody and the antigen-binding domain VH of the 4-1BB HCAb antibody. This shows the flexibility of constructing the molecular structure of bispecific antibodies based on HCAbs, and the T cell function-activating activity is regulated through different structure types, relative positions, binding valences and other parameters.

The activation of T cells by Urelumab is not target-specific, which is one of the causes for its clinical toxic and side effects. The T cell activation effect of PD-L1×4-1BB bispecific antibodies is specifically dependent on the expression of PD-L1. Crosslinking-dependent anti-4-1BB monoclonal antibodies are unable to directly activate T cells, but the PD-L1×4-1BB bispecific antibodies constructed using those anti-4-1BB monoclonal antibodies are able to specifically activate T cells in the presence of cells highly expressing PD-L1.

In one aspect, HCAb-based bispecific antibody structures, especially bispecific antibody molecules of IgG-VH tetravalent symmetric structures or bispecific antibody molecules of Fab-HCAb structures, retain the activity of the PD-L1 end and exhibit better T cell activation ability than the corresponding anti-PD-L1 parent monoclonal antibody in MLR experiments; in another aspect, the PD-L1 molecules highly expressed on target cells can mediate crosslinking and trimerization of 4-1BB to transmit T cell activation signals, and their T cell activation ability was even superior to that of Urelumab. Moreover, the bispecific antibody molecules of IgG-VH tetravalent symmetric structures or Fab-HCAb symmetric structures demonstrate better T cell activation ability than bispecific antibody molecules of FIT-Ig structures.

In conclusion, the PD-L1 ×4-1BB bispecific antibody molecules with good safety, outstanding functional activity and good molecular stability are constructed in this example.

## Example 6. B7H4×4-1BB bispecific antibodies

## Example 6.1. Background

B7-H4 (VTCN1, B7h.5, B7S1, B7x) is a transmembrane protein belonging to the B7/CD28 superfamily. The B7-H4 protein is expressed in some immune cells such as monocytes and dendritic cells, and is possibly involved in the negative regulation of immune response of T cells. In addition, B7H4 is highly expressed on the surface of tumor cells of breast cancer, ovarian cancer, endometrial cancer, non-small cell lung cancer, kidney cancer, etc., while it is not expressed or is very little expressed in most normal tissues. As an emerging target for these tumors, B7-H4 has received attention in recent years. Anti-B7-H4 antibodies can act on tumor cells through multiple mechanisms, but its development is mainly focused on monoclonal antibodies, and no bispecific antibody therapy is available at present.

4-1BB (TNFRSF9, CD137) is a transmembrane protein belonging to the TNF receptor superfamily. 4-1BB is a co-stimulatory molecule expressed on a variety of immune cells. It is a multifunctional modulator of immune activity. Its expression is induced in activated T cells, NK cells and other immune cells. 4-1BB activates T cells through trimerization mediated by its ligand 4-1BBL, promoting cell proliferation and cytokine release. Anti-4-1BB agonistic antibodies have the function of inhibiting tumors. The first 4-1BB antibodies to be subjected to clinical trials were Utomilumab from Pfizer and Urelumab (BMS-663513) from Bristol-Myers Squibb. The initial clinical results of Urelumab were published in 2008. Although encouraging efficacy was observed in some patients, the data showed urelumab to cause target and dose-associated hepatotoxicity. Moreover, two patients in the clinical trial died from hepatotoxicity, resulting in the discontinuation of related clinical trials. Utomilumab has better safety enabling the dose to be increased to 10 mg/kg, but still has a poor therapeutic effect.

In this example, we constructed bispecific antibodies targeting both B7H4 and 4-1BB to improve anti-tumor efficacy and safety through one or more mechanisms of action. Firstly, B7H4×4-1BB bispecific antibodies can activate T cells by relieving the negative regulatory signals of B7H4. Secondly, B7H4×4-1BB bispecific antibodies are enriched in tumor tissues with high B7H4 expression, and immune cells and tumor cells are combined together through the bispecific antibody molecules in a tumor microenvironment to promote the formation of immune synapses; meanwhile, the B7H4 molecules highly expressed on the surface of the tumor cells can promote the crosslinking of 4-1BB molecules on the surface of the T cell through bispecific antibody molecules, activate a downstream signaling pathway and provide a co-stimulation signal, thereby promoting the activation and proliferation of T cells and improving the anti-tumor activity. Thirdly, the anti-4-1BB agonistic antibodies used in this example have the function dependent on molecular crosslinking, and they can only mediate T cell activation using target cells in a tumor microenvironment to avoid the toxic and side effects caused by over-activation of T cells in normal tissues by monoclonal antibodies similar to Urelumab.

## Example 6.2. Acquisition of anti-B7H4 IgG antibodies and anti-4-1BB HCAb antibodies

**Example 6.2.1. Acquisition of fully human anti-B7H4 H2L2 antibodies**

The Harbour H2L2 mouse (Harbour Antibodies BV) is a transgenic mouse carrying an immune repertoire of human immunoglobulins that produces antibodies with intact human antibody variable domains and rat constant domains.

Harbour H2L2 mice were subjected to multiple rounds of immunization with a soluble recombinant human B7H4-mFc fusion protein (Sino Biological Inc., #10738-H05H). When the titer of the B7H4-specific antibody in the serum of mice was detected to reach a certain level, spleen cells of the mice were taken and fused with a myeloma cell line to obtain hybridoma cells. After multiple rounds of screening and cloning of the hybridoma cells, several monoclonal antibody molecules specifically recognizing B7H4 were identified. The monoclonal antibodies were further identified, and several candidate antibody molecules were preferentially selected according to parameters such as the binding ability to human B7H4, the binding ability to cynomolgus monkey B7H4, and the internalization ability of target cell receptors. The candidate antibody molecules were then subjected to sequence analysis and optimization to obtain several variant sequences. The VL and VH sequences of the antibody were fused to the corresponding human κ light chain constant region and IgG1 heavy chain constant region sequences and expressed to obtain recombinant fully human antibody molecules. The recombinant fully human anti-B7H4 IgG antibodies PR002408 and PR002410 are listed in Table 6-9.

**Example 6.2.2. Acquisition of fully human anti-4-1BB HCAb antibodies**

The fully human anti-4-1BB HCAb antibodies PR001758, PR001760, PR001771, PR001840 and PR004469 (Table 6-9) used in this example were derived from Harbour HCAb mice, and were found as described in Example 5.2.3.

The binding ability of the 4-1BB heavy chain antibodies to CHO-K1/hu 4-1BB cells (GenScript Biotech, M00538) was tested by flow cytometry FACS and the method described in Example 5.6. As shown in FIG. 28 (C-F), the 4-1BB heavy chain antibodies had similar binding ability to Utomilumab, and had superior binding ability to Urelumab.

**Example 6.3. Construction of bispecific antibody molecules using anti-B7H4 IgG antibodies and anti-4-1BB HCAb antibodies**

In this example, B7H4×4-1BB bispecific antibody molecules of a variety of structures were constructed using the antigen-binding domain Fab of the anti-B7H4 IgG antibody PR002408 or PR002410 and the antigen-binding domain VH of the anti-4-1BB HCAb antibody PR001758, PR001760, PR001771, PR001840 or PR004469.

In this and subsequent examples, the anti-B7H4 IgG monoclonal antibody PR002408 was used as a positive control molecule, and was also the parent monoclonal antibody of the B7H4 end of the B7H4×4-1BB bispecific antibody molecules.

In this and subsequent examples, the anti-4-1BB IgG monoclonal antibody urelumab was used as a positive control molecule.

**Example 6.3.1. Construction of molecules of Fab-HCAb symmetric structures**

B7H4×4-1BB bispecific antibody molecules of Fab-HCAb symmetric structures were designed according to the structures described in Example 1.1 using anti-B7H4 IgG antibodies and anti-4-1BB heavy chain antibodies, with the results summarized in Table 6-1. The antibody molecule samples were prepared and analyzed according to the method described in Example 2, with the results summarized in Table 6-2.

**Table 6-1. B7H4×4-1BB bispecific antibody molecules of Fab-HCAb tetravalent symmetric structures**

| Structure No. | Bispecific antibody molecules | B7H4 antibody (IgG) | 4-1BB antibody (VH_B) | First linker peptide (between Fab and VH_B) | Second linker peptide (between VH_B and CH2) | Fc type (mutation) |
|---|---|---|---|---|---|---|
| 1 | PR004279 | PR002408 | PR001760 | H1_15 | Human IgG1 hinge (C220S) | Human IgG1 (L234A, L235A) |
| 1 | PR005189 | PR002408 | PR001760 | None | Human IgG1 hinge (C220S) | Human IgG1 (L234A, L235A) |

(continued)

| Structure No. | Bispecific antibody molecules | B7H4 antibody (IgG) | 4-1BB antibody (VH_B) | First linker peptide (between Fab and VH_B) | Second linker peptide (between VH_B and CH2) | Fc type (mutation) |
|---|---|---|---|---|---|---|
| 2 | PR007165 | PR002408 | PR001760 | None | Human IgG1 hinge (C220S) | Human IgG1 (L234A, L235A) |

**Table 6-2. Expression of B7H4×4-1BB bispecific antibody molecule proteins of Fab-HCAb tetravalent symmetric structures**

| Structure No. | Bispecific antibody molecules | Expression system and volume | Plasmid transfection ratio (short chain:long chain) | Yield (mg/L) after first purification | SEC-HPLC purity (%) |
|---|---|---|---|---|---|
| 1 | PR004279 | HEK293-F (30ml) | 1 : 1 | 11.375 | 79.18 |

**Example 6.3.2. Construction of molecules of IgG-VH tetravalent symmetric structure**

B7H4×4-1BB bispecific antibody molecules of IgG-VH tetravalent symmetric structures were designed according to the structures described in Example 1.2 using anti-B7H4 IgG antibodies and anti-4-1BB heavy chain antibodies, with the results summarized in Table 6-3. The antibody molecule samples were prepared and analyzed according to the method described in Example 2, with the results summarized in Table 6-4.

**Table 6-3. B7H4×4-1BB bispecific antibody molecules of IgG-VH tetravalent symmetric structures**

| Structure No. | Bispecific antibody molecules | B7H4 antibody (IgG) | 4-1BB antibody (VH_B) | VH_B position relative to IgG | Linker peptide | Fc type (mutation) |
|---|---|---|---|---|---|---|
| 5 | PR003334 | PR002408 | PR001758 | C-terminus of heavy chain | H1_15-RT | Human IgG1 (L234A, L235A) |
| 5 | PR003335 | PR002408 | PR001760 | C-terminus of heavy chain | H1_15-RT | Human IgG1 (L234A, L235A) |
| 5 | PR003338 | PR002408 | PR004469 | C-terminus of heavy chain | H1_15-RT | Human IgG1 (L234A, L235A) |

**Table 6-4. B7H4×4-1BB bispecific antibody molecule proteins of IgG-VH tetravalent symmetric structures**

| Structure No. | Bispecific antibody molecules | Expression system and volume | Plasmid transfection ratio (short chain:long chain) | Yield (mg/L) after first purification | SEC-HPLC purity (%) | DSF Tm1 (°C) |
|---|---|---|---|---|---|---|
| 5 | PR003334 | ExpiCHO (200ml) | 3:2 | 38.2 | 97.98 | 60.32 |
| 5 | PR003335 | ExpiCHO (200ml) | 3:2 | 68.9 | 99.29 | 65.59 |
| 5 | PR003338 | HEK293-F (10ml) | 3:2 | 74.6 | 98.02 | 62.17 |

**Example 6.3.3. Construction of molecules of IgG-VH(2) hexavalent symmetric structures**

B7H4×4-1BB bispecific antibody molecules of IgG-VH(2) hexavalent symmetric structures were designed according to the structures described in Example 1.3 using anti-B7H4 IgG antibodies and anti-4-1BB heavy chain antibodies, with the results summarized in Table 6-5. The antibody molecule samples were prepared and analyzed according to the method described in Example 2, with the results summarized in Table 6-6.

**Table 6-5. B7H4×4-1BB bispecific antibody molecules of IgG-VH(2) hexavalent symmetric structures**

| Structure No. | Bispecific antibody molecules | B7H4 antibody (IgG) | 4-1BB antibody (VH_B) | First linker peptide (between Fc and VH_B) | Second linker peptide (between VH_B and VH_B) | Fc type (mutation) |
|---|---|---|---|---|---|---|
| 7 | PR003487 | PR002408 | PR001760 | H1_15-RT | None | Human IgG1 (L234A, L235A) |
| 7 | PR003488 | PR002408 | PR001760 | H1_15-RT | GS_5 | Human IgG1 (L234A, L235A) |

**Table 6-6. Expression of B7H4×4-1BB bispecific antibody molecule proteins of IgG-VH(2) hexavalent symmetric structures**

| Structure No. | Bispecific antibody molecules | Expression system and volume | Yield (mg/L) after first purification | SEC-HPLC purity (%) |
|---|---|---|---|---|
| 7 | PR003487 | HEK293-F (30ml) | 19 | 84 |
| 7 | PR003488 | HEK293-F (30ml) | 17.39 | 85 |

**Example 6.3.4. Construction of molecules of Fab-Fc-VH(n) asymmetric structures**

B7H4×4-1BB bispecific antibody molecules of Fab-Fc-VH(n, n = {2,3}) asymmetric structures were designed according to the structures described in Example 1.6 and Example 1.9 using anti-B7H4 IgG antibodies and anti-4-1BB heavy chain antibodies, with the results summarized in Table 6-7. The antibody molecule samples were prepared and analyzed according to the method described in Example 2, with the results summarized in Table 6-8.The mutation based on the "knob-into-hole" structure was used and the L234A and L235A double mutation was introduced in the Fc region of the molecules.

**Table 6-7. B7H4×4-1BB bispecific antibody molecules of Fab-Fc-VH(n) asymmetric structures**

| Structure No. | Bispecific antibody molecules | B7H4 antibody (Fab) | 4-1BB antibody (VH_B) | Structure of Fab end | Number of repetition n for VH_B | First linker peptide | Second linker peptide |
|---|---|---|---|---|---|---|---|
| 14 | PR004160 | PR002410 | PR001760 | Normal | 2 | GS_15 | None |
| 26 | PR004161 | PR002410 | PR001760 | Normal | 3 | GS_15 | AS-GS_15 |
| 26 | PR004181 | PR002410 | PR001771 | Normal | 3 | GS_15 | GS_15 |
| 26 | PR004182 | PR002410 | PR001840 | Normal | 3 | GS_15 | GS_15 |

**Table 6-8. Expression of B7H4×4-1BB bispecific antibody molecule proteins of Fab-Fc-VH(n) asymmetric structures**

| Structure No. | Bispecific antibody molecules | Expression system and volume | Plasmid transfection ratio | Yield (mg/L) after first purification | SEC-HPLC purity (%) |
|---|---|---|---|---|---|
| 14 | PR004160 | HEK293-F (30ml) | 1 : 1 : 1 | 29.36 | 89.41 |
| 26 | PR004161 | HEK293-F (30ml) | 1 : 1 : 1 | 31.62 | 85.47 |
| 26 | PR004181 | HEK293-F (30ml) | 1 : 1 : 1 | 87.25 | 87.19 |
| 26 | PR004182 | HEK293-F (30ml) | 1 : 1 : 1 | 24.75 | 79.14 |

**Example 6.3.5. Sequences of B7H4×4-1BB bispecific antibody molecules and control molecules**

The sequence numbers corresponding to the sequences of the B7H4×4-1BB bispecific antibody molecules constructed in this example, the corresponding parent monoclonal antibody molecules such as the B7H4 monoclonal antibody and the 4-1BB monoclonal antibody, and the control molecules are listed in Table 6-9, Table 6-10 and Table 6-11. The structure numbers in Table 6-11 correspond to those in Table 1-1 and FIG. 1.The sequence numbers of the corresponding CDR sequences of the first and second antigen-binding domains of the bispecific antibody molecules are listed in Table 6-12.

**Table 6-9. Control molecules and parent monoclonal antibodies**

| Antibody No. | Antibody |
|---|---|
| PR002408 | Anti-B7H4 monoclonal antibody 80C8-2E9 (H: G55A; L: N92Q), hIgG1 |
| PR002410 | Anti-B7H4 monoclonal antibody 1025_B-1H11 (L: C87Y), hIgG1 |
| PR001758 | Anti-4-1BB heavy-chain antibody 1016P0010B11, hIgG1 |
| PR001760 | Anti-4-1BB heavy-chain antibody 1016P0011G10, hIgG1 |
| PR001771 | Anti-4-1BB heavy-chain antibody 1016P0042C5, hIgG1 |
| PR001840 | Anti-4-1BB heavy-chain antibody 1016P0037D2, hIgG1 |
| PR004469 | Anti-4-1BB heavy-chain antibody PR001838_G53A, hIgG1 |
| PR000628 | Anti-4-1BB monoclonal antibody urelumab analog, hIgG4 (S228P) |

**Table 6-10. Sequence numbers of sequences and CDR sequences of control molecules and parent monoclonal antibodies**

| Antibody No. | Light chain | Heavy chain | VL | VH | LCDR1 | LCDR2 | LCDR3 | HCDR1 | HCDR2 | HCDR3 |
|---|---|---|---|---|---|---|---|---|---|---|
| PR002408 | 360 | 326 | 298 | 261 | 180 | 191 | 225 | 32 | 87 | 143 |
| PR002410 | 361 | 327 | 299 | 262 | 177 | 191 | 226 | 33 | 88 | 144 |
| PR001758 | None | 320 | None | 252 | None | None | None | 27 | 79 | 137 |
| PR001760 | None | 321 | None | 253 | None | None | None | 28 | 80 | 138 |
| PR001771 | None | 322 | None | 254 | None | None | None | 28 | 81 | 139 |
| PR001840 | None | 324 | None | 256 | None | None | None | 15 | 83 | 140 |
| PR004469 | None | 329 | None | 264 | None | None | None | 28 | 89 | 145 |
| PR000628 | 358 | 314 | 292 | 246 | 175 | 195 | 219 | 18 | 73 | 131 |

**Table 6-11. Sequence numbers of B7H4×4-1BB bispecific antibody molecules of this example**

| Structure No. | Antibody No. | Polypeptide chain 1 (short chain) | Polypeptide chain 2 (long chain) |
|---|---|---|---|
| 5 | PR003334 | 360 | 455 |

(continued)

| Structure No. | Antibody No. | Polypeptide chain 1 (short chain) | Polypeptide chain 2 (long chain) |
|---|---|---|---|
| 5 | PR003335 | 360 | 456 |
| 5 | PR003338 | 360 | 457 |
| 7 | PR003487 | 360 | 458 |
| 7 | PR003488 | 360 | 459 |
| 1 | PR004279 | 487 | 488 |
| 1 | PR005189 | 487 | 520 |
| 2 | PR007165 | 360 | 523 |
| 14 | PR004160 | 361 | 481 |
| 26 | PR004161 | 361 | 481 |
| 26 | PR004181 | 361 | 481 |
| 26 | PR004182 | 361 | 481 |

**Table 6-12. Sequence numbers of CDRs of antigen-binding domains of B7H4×4-1BB bispecific antibody molecules**

| Structure No. | Antibody No. | Antigen-binding domain No. | LCDR1 | LCDR2 | LCDR3 | HCDR1 | HCDR2 | HCDR3 |
|---|---|---|---|---|---|---|---|---|
| 1 | PR004279, PR005189 | #1 | 180 | 191 | 225 | 32 | 87 | 143 |
| | | #2 | None | None | None | 28 | 80 | 138 |
| 2 | PR007165 | #1 | 180 | 191 | 225 | 32 | 87 | 143 |
| | | #2 | None | None | None | 28 | 80 | 138 |
| 5 | PR003334 | #1 | 180 | 191 | 225 | 32 | 87 | 143 |
| | | #2 | None | None | None | 27 | 79 | 137 |
| 5 | PR003335 | #1 | 180 | 191 | 225 | 32 | 87 | 143 |
| | | #2 | None | None | None | 28 | 80 | 138 |
| 5 | PR003338 | #1 | 180 | 191 | 225 | 32 | 87 | 143 |
| | | #2 | None | None | None | 28 | 89 | 145 |
| 7 | PR003487, PR003488 | #1 | 180 | 191 | 225 | 32 | 87 | 143 |
| | | #2 | None | None | None | 28 | 80 | 138 |
| 14 | PR004160 | #1 | 177 | 191 | 226 | 33 | 88 | 144 |
| | | #2 | None | None | None | 28 | 80 | 138 |
| 26 | PR004161 | #1 | 177 | 191 | 226 | 33 | 88 | 144 |
| | | #2 | None | None | None | 28 | 80 | 138 |
| 26 | PR004181 | #1 | 177 | 191 | 226 | 33 | 88 | 144 |
| | | #2 | None | None | None | 28 | 81 | 139 |
| 26 | PR004182 | #1 | 177 | 191 | 226 | 33 | 88 | 144 |
| | | #2 | None | None | None | 15 | 83 | 140 |

### Example 6.4. Binding to B7H4

This example is intended to investigate the binding activity of the B7H4×4-1BB bispecific antibody molecules to B7H4.

### Example 6.4.1. Binding to SK-BR-3 cells highly expressing human B7H4

The binding ability of the antibodies to a tumor cell line SK-BR-3 (ATCC, HTB-30) highly expressing human B7H4 was tested by flow cytometry FACS. Specifically, SK-BR-3 cells were digested and resuspended in a complete medium, with the cell density adjusted to $2\times10^6$ cells/mL. Then, the cells were seeded in a 96-well V-bottom plate (Corning, #3894) at 50 µL/well. Then, antibody molecules at a total of 8 concentrations obtained by a 5-fold gradient dilution were added at 50 µL/well, and the mixture was mixed well. hIgG1 iso (CrownBio, #C0001) was used as an isotype control. The cells were incubated at 4 °C for 2 h away from light. Then, the cells in each well were then rinsed twice with 100 µL of pre-cooled PBS buffer and centrifuged at 500 g at 4 °C for 5 min, and then the supernatant was discarded. Thereafter, a fluorescent secondary antibody (Alexa Fluor 647-conjugated AffiniPure Goat Anti-Human IgG, Fcγ Fragment Specific, Jackson ImmunoResearch, #109-605-098, diluted in a 1:1000 ratio) was added at 100 µL/well, and the plate was incubated at 4 °C for 1 h away from light. Then, the cells in each well were then rinsed twice with 100 µL of pre-cooled PBS buffer and centrifuged at 500 g at 4 °C for 5 min, and then the supernatant was discarded. Finally, a pre-cooled FACS buffer (PBS buffer containing 0.5% BSA) was added at 200 µL/well to resuspend the cells. Fluorescence signal values were read using a BD FACS CANTOII flow cytometer or an ACEA NovoCyte flow cytometer (ACEA Biosciences Inc.), and the data were processed and analyzed using FlowJo v10 (FlowJo, LLC) software. The data were processed and analyzed by plotting using GraphPad Prism 8 software, and binding curves of the antibodies to target cells, $EC_{50}$ values and other parameters were obtained through four-parameter nonlinear fitting.

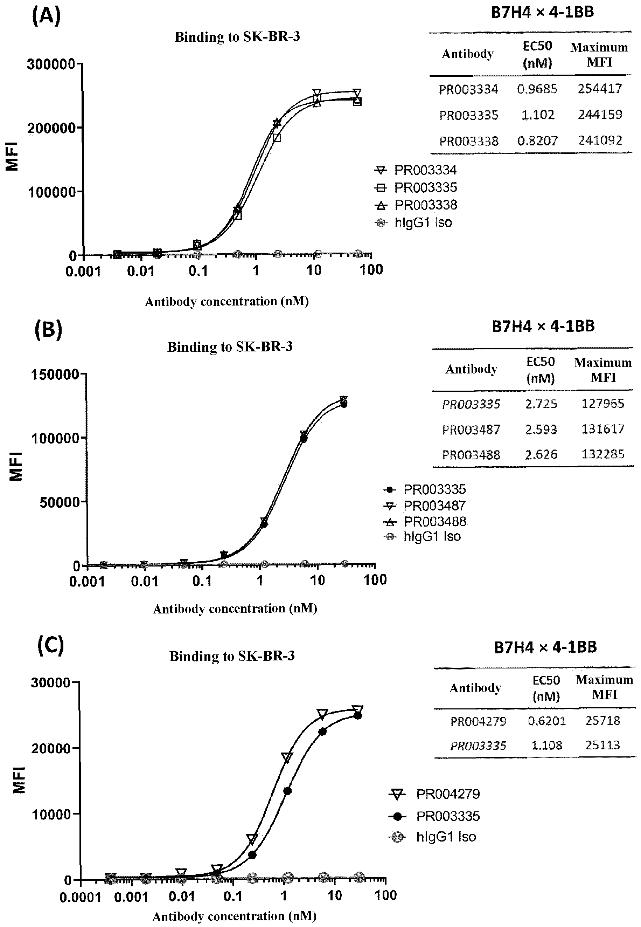

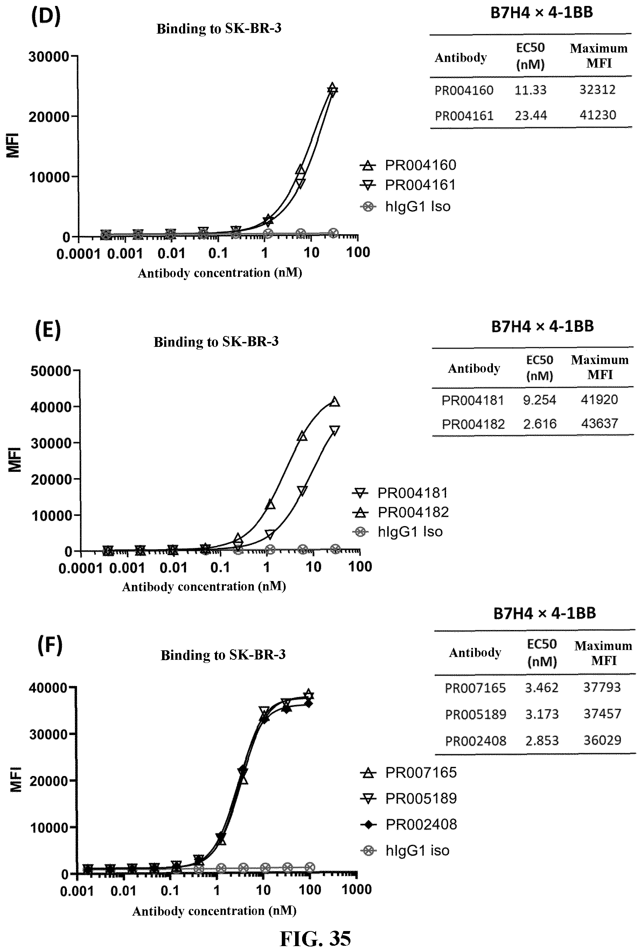

As shown in FIG. 35 and Table 6-13, the bispecific antibody molecules were all able to bind to SK-BR-3 cells. As shown in FIG. 35 (A-C), the bispecific antibody molecules of symmetric structures (PR003334, PR003335, PR003338, PR003487, PR003488, and PR004279) had identical bivalent B7H4-binding domain (derived from the anti-B7H4 monoclonal antibody PR002408), and had very close $EC_{50}$ for binding to B7H4. The bispecific antibody molecules of IgG-VH-VH hexavalent symmetric structures (PR003487 and PR003488) had almost identical binding ability to B7H4 to the bispecific antibody molecule of an IgG-VH tetravalent symmetric structure (PR003335). The bispecific antibody molecule of a Fab-HCAb symmetric structure (PR004279) had slightly stronger binding activity to B7H4 than the bispecific antibody molecule of an IgG-VH structure (PR003335). As shown in FIG. 35 (D-E), the bispecific antibody molecules of asymmetric structures (PR004160, PR004161, PR004181, and PR004182) had only one B7H4-binding domain and thus had weaker binding ability to B7H4 than the bispecific antibody molecules of symmetric structures, but PR004182 still had similar binding $EC_{50}$ to the bispecific antibody molecules of symmetric structures. As shown in FIG. 35 (F), the bispecific antibody molecules of Fab-HCAb symmetric structures, whether the molecule of the structure (1) (PR005189) or the molecule of the structure (2) (PR007165), had almost identical binding activity, indicating that the fusion of the CL of Fab to the heavy chain where VH_B is located does not affect the binding activity of the Fab end.

### Table 6-13. Binding to SK-BR-3

| Antibody | $EC_{50}$ (nM) | Maximum MFI | Antibody | $EC_{50}$ (nM) | Maximum MFI | Antibody | $EC_{50}$ (nM) | Maximum MFI |
|---|---|---|---|---|---|---|---|---|
| PR003334 | 0.9685 | 254417 | PR003335 | 2.725 | 127965 | PR004279 | 0.6201 | 25718 |
| PR003335 | 1.102 | 244159 | PR003487 | 2.593 | 131617 | PR003335 | 1.108 | 25113 |
| PR003338 | 0.8207 | 241092 | PR003488 | 2.626 | 132285 | | | |
| Antibody | $EC_{50}$ (nM) | Maximum MFI | Antibody | $EC_{50}$ (nM) | Maximum MFI | Antibody | $EC_{50}$ (nM) | Maximum MFI |
| PR007165 | 3.462 | 37793 | PR004160 | 11.33 | 32312 | PR004181 | 9.254 | 41920 |
| PR005189 | 3.173 | 37457 | PR004161 | 23.44 | 41230 | PR004182 | 2.616 | 43637 |
| PR002408 | 2.853 | 36029 | | | | | | |

### Example 6.5. Binding to 4-1BB

This example is intended to investigate the binding activity of the B7H4×4-1BB bispecific antibody molecules to 4-1BB.

**Example 6.5.1. Binding to CHO-K1 cells CHO-K1/hu4-1BB highly expressing human 4-1BB**

The binding ability of the antibody molecules to a CHO-K1 cell line CHO-K1/hu 4-1BB (GenScript Biotech, M00538) highly expressing human 4-1BB was tested by flow cytometry FACS. Specifically, the cells were digested and resuspended in a complete medium, with the cell density adjusted to $2\times10^6$ cells/mL. Then, the cells were seeded in a 96-well V-bottom plate (Corning, #3894) at 100 $\mu$L/well ($2\times10^5$ cells/well) and centrifuged at 4 °C for 5 min, and the supernatant was discarded. Then, the antibody molecules at a total of 8 concentrations obtained by a 5-fold gradient dilution were added into a 96-well plate at 100 $\mu$L/well, and the mixture was mixed well. hIgG1 iso (CrownBio, #C0001) was used as an isotype control. The cells were incubated at 4 °C for 1 h away from light. Then, the cells in each well were rinsed twice with 100 $\mu$L of pre-cooled PBS buffer, and centrifuged at 500 g at 4 °C for 5 min, and then the supernatant was discarded. Thereafter, a fluorescent secondary antibody (Alexa Fluor® 488, Goat Anti-Human IgG, Fc$\gamma$ fragment specific, Jackson ImmunoResearch, #109-545-098, diluted in a 1: 1000 ratio) was added at 100 $\mu$L/well, and the plate was incubated at 4 °C for 1 h away from light. Then, the cells in each well were then rinsed twice with 200 $\mu$L of pre-cooled PBS buffer and centrifuged at 500 g at 4 °C for 5 min, and then the supernatant was discarded. Finally, a pre-cooled FACS buffer (PBS buffer containing 0.5% BSA) was added at 200 $\mu$L/well to resuspend the cells. Fluorescence signal values were read using a BD FACS CANTOII flow cytometer or an ACEA NovoCyte flow cytometer, and the data were processed and analyzed using FlowJo v10 (FlowJo, LLC) software. The data were processed and analyzed by plotting using GraphPad Prism 8 software, and binding curves of the antibodies to target cells, $EC_{50}$ values and other parameters were obtained through four-parameter nonlinear fitting.

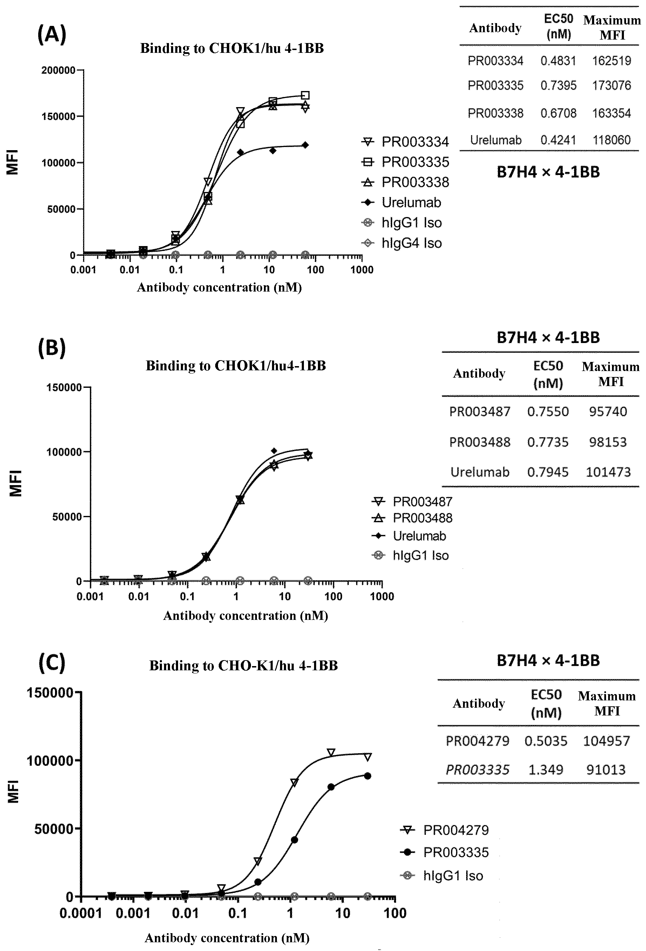

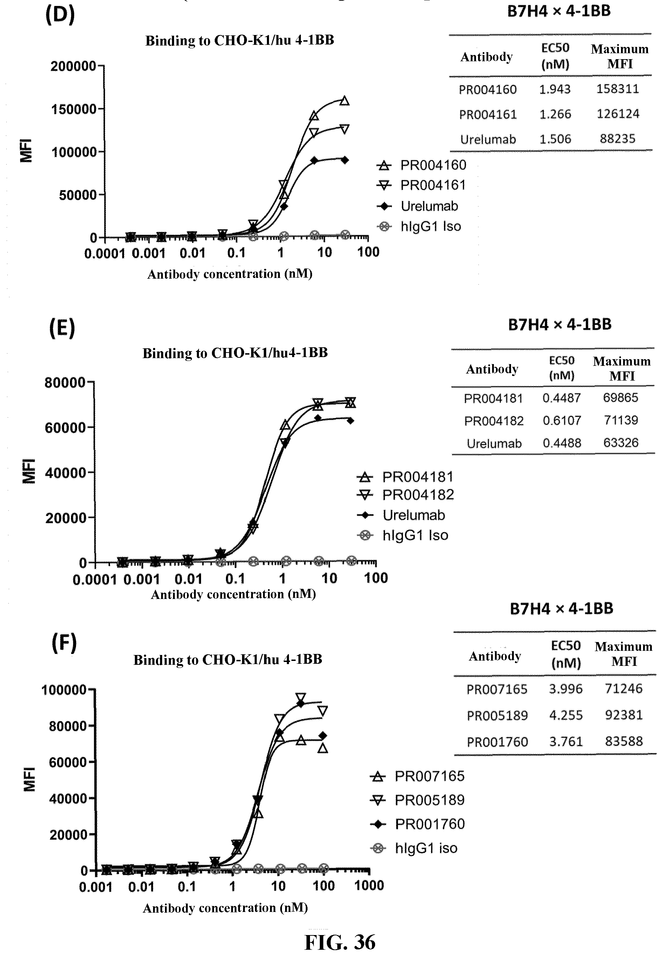

In this example, the anti-4-1BB monoclonal antibody Urelumab was used as a positive control molecule. The results are shown in FIG. 36 and Table 6-14.

As shown in FIG. 36, the bispecific antibody molecules were all able to bind to CHO-K1/hu 4-1BB cells, and had very similar $EC_{50}$ in binding curves. As shown in FIG. 36 (A), the bispecific antibody molecules of IgG-VH tetravalent symmetric structures had greater maximum MFI values for binding to 4-1BB than Urelumab; as shown in FIG. 36 (B), the bispecific antibody molecules of IgG-VH-VH hexavalent symmetric structures had similar binding activity to 4-1BB to Urelumab; as shown in FIG. 36 (C), the bispecific antibody molecule of a Fab-HCAb symmetric structure (PR004279) had slightly stronger binding activity to 4-1BB than the bispecific antibody molecule of an IgG-VH structure (PR003335); as shown in FIG. 36 (D-E), the bispecific antibody molecules of Fab-Fc-VH(n) asymmetric structures had a similar $EC_{50}$ value but a higher maximum MFI value for binding to 4-1BB as compared to Urelumab;and as shown in FIG. 36 (F), the bispecific antibody molecules of Fab-HCAb symmetric structures, whether the molecule of the structure (1) (PR005189) or the molecule of the structure (2) (PR007165), had similar binding activity to 4-1BB, and were comparable to the parent monoclonal antibody PR001760.

**Table 6-14. Binding to CHO-K1/hu 4-1BB**

| Antibody | $EC_{50}$ (nM) | Maximum MFI | Antibody | $EC_{50}$ (nM) | Maximum MFI | Antibody | $EC_{50}$ (nM) | Maximum MFI |
|---|---|---|---|---|---|---|---|---|
| PR003334 | 0.4831 | 162519 | PR003487 | 0.755 | 95740 | PR004279 | 0.5035 | 104957 |
| PR003335 | 0.7395 | 173076 | PR003488 | 0.7735 | 98153 | PR003335 | 1.349 | 91013 |
| PR003338 | 0.6708 | 163354 | Urelumab | 0.7945 | 101473 | | | |
| Urelumab | 0.4241 | 118060 | | | | | | |
| Antibody | $EC_{50}$ (nM) | Maximum MFI | Antibody | $EC_{50}$ (nM) | Maximum MFI | Antibody | $EC_{50}$ (nM) | Maximum MFI |
| PR007165 | 3.996 | 71246 | PR004160 | 1.943 | 158311 | PR004181 | 0.4487 | 69865 |
| PR005189 | 4.255 | 92381 | PR004161 | 1.266 | 126124 | PR004182 | 0.6107 | 71139 |
| PR001760 | 3.761 | 83588 | Urelumab | 1.506 | 88235 | Urelumab | 0.4488 | 63326 |

**Example 6.6. T cell specific activation mediated by target cells highly expressing B7H4**

This example is intended to investigate the activity of the B7H4×4-1BB bispecific antibody molecules to activate T cells by binding to 4-1BB in the presence of target cells. The target cells may be cells expressing B7H4 to different degrees, such as SK-BR-3 (ATCC, HTB-30) highly expressing B7H4 or JIMT-1 (DSMZ, ACC 589) not expressing B7H4. The effector cells may be isolated human PBMCs or T cells.

Specifically, anti-CD3 antibody OKT3 (Thermo, #16-0037-81) was firstly used for coating a 96-well plate (Corning, #3799). Then, the density of human T cells was adjusted to $3 \times 10^6$ cells/mL, and the density of target cells was adjusted to $3 \times 10^5$ cells/mL. The two cell suspensions were each seeded into a 96-well plate at 50 μL/well, with a final effector-to-target ratio of 10:1. Then, antibody molecules at different concentrations were added at 50 μL/well, wherein the concentrations are a total of 6 concentrations obtained by a 5-fold gradient dilution, and two duplicate wells were set for each concentration. hIgG1 iso (CrownBio, #C0001) and hIgG4 iso (CrownBio, #C0045) were used as controls. The 96-well plate was incubated in an incubator at 37 °C with 5% $CO_2$ for 2 days. After the incubation was completed, 100 μL of the supernatant was taken and added into another 96-well plate (Corning, #3599), and the mixture was centrifuged at 500 g at 4 °C for 5 min. 50 μL of the supernatant was taken for the measurement of the level of cytokines. The concentration of IL-2 in the supernatant was determined using an IL-2 ELISA kit (Thermo, #88-7025-88), and the concentration of IFN-γ in the supernatant was determined using an IFN-γ ELISA kit (Thermo, #88-7316-88). The ELISA assay was performed by referring to the instructions of relevant kit. The data were processed and analyzed by plotting using a GraphPad Prism 8 software.

In this example, the anti-4-1BB monoclonal antibody Urelumab was used as a positive control molecule.

### Example 6.6.1. SK-BR-3-mediated T cell specific activation

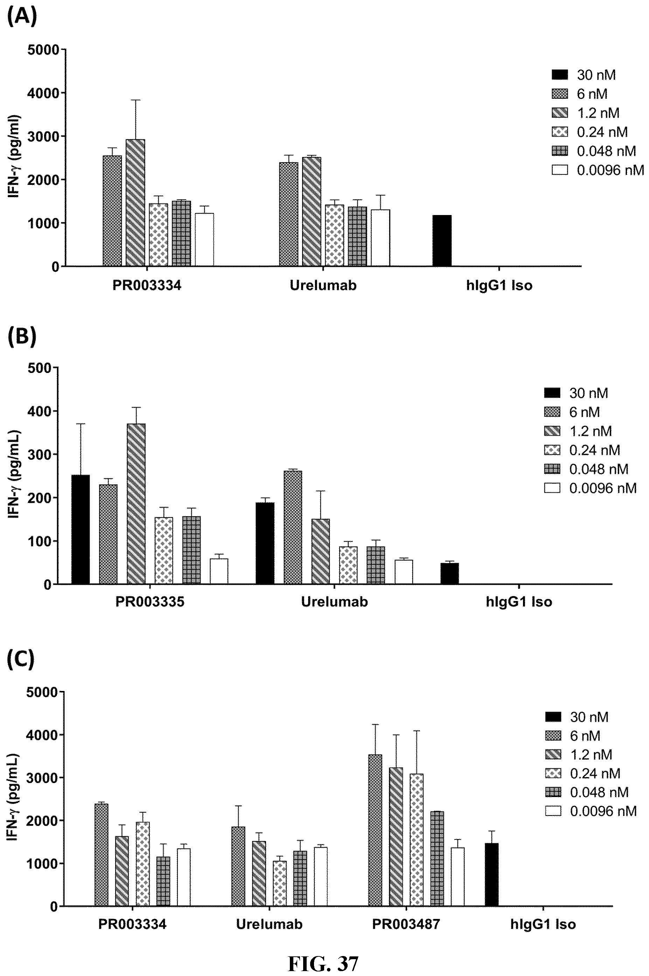

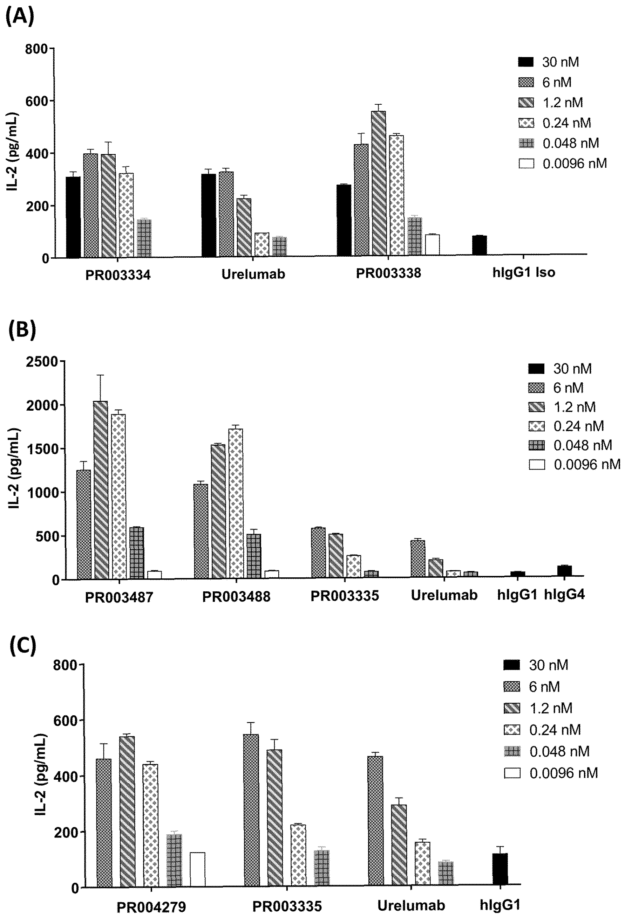

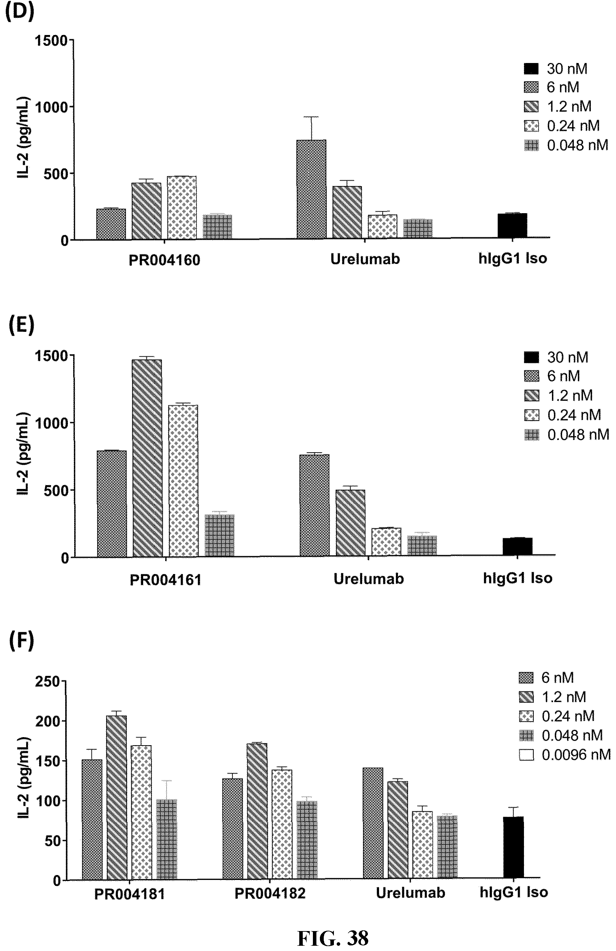

As shown in FIG. 37 and FIG. 38, in the presence of SK-BR-3 cells highly expressing B7-H4, the B7-H4×4-1BB bispecific antibody molecules had superior T cell activation ability to positive control molecules, as evidenced by the higher release of the cytokines IFN-γ (FIG. 37) or IL-2 (FIG. 38) produced in this system than that produced in the positive control molecules.

As shown in FIG. 37 (A-B) and FIG. 38 (A), the bispecific antibodies of IgG-VH tetravalent symmetric structures (PR003334, PR003335, and PR003338) had slightly stronger T cell activation ability than Urelumab, and PR003335 and PR003338 had slightly stronger T cell activation ability than PR003334.

As shown in FIG. 37 (C) and FIG. 38 (B), the bispecific antibodies of IgG-VH-VH hexavalent symmetric structures (PR003487 and PR003488) had significantly stronger T cell activation ability than the bispecific antibodies of IgG-VH tetravalent structures (PR003334 and PR003335) and Urelumab.

As shown in FIG. 38 (C), the bispecific antibody of a Fab-HCAb symmetric structure (PR004279) had slightly stronger T cell activation ability than Urelumab.

As shown in FIG. 38 (D-F), the bispecific antibodies of Fab-Fc-VH(n) asymmetric structures (PR004160, PR004161, PR004181, and PR004182) were able to stimulate the IL-2 production by T cells. PR004160 and PR004161 had identical sequences of the 4-1BB-binding domain (VH), but PR004160 had two VH domains in tandem (i.e., the Fab-Fc-VH(2) structure), while PR004161 had three VH domains in tandem (i.e., the Fab-Fc-VH(3) structure). PR004161 had significantly stronger T cell activation ability than PR004160 and Urelumab. PR004181 and PR004182 of similar Fab-Fc-VH(3) structures also showed superior T cell activation ability to Urelumab. Although those bispecific antibodies of asymmetric structures had only one B7H4-binding domain and had weaker binding ability to B7H4 than the bispecific antibodies of symmetric structures, they were still able to mediate the T cell activation using cells highly expressing B7-H4, and had superior activation ability to Urelumab.

### Example 6.6.2. Influence of the expression of B7H4 on the T cell activation

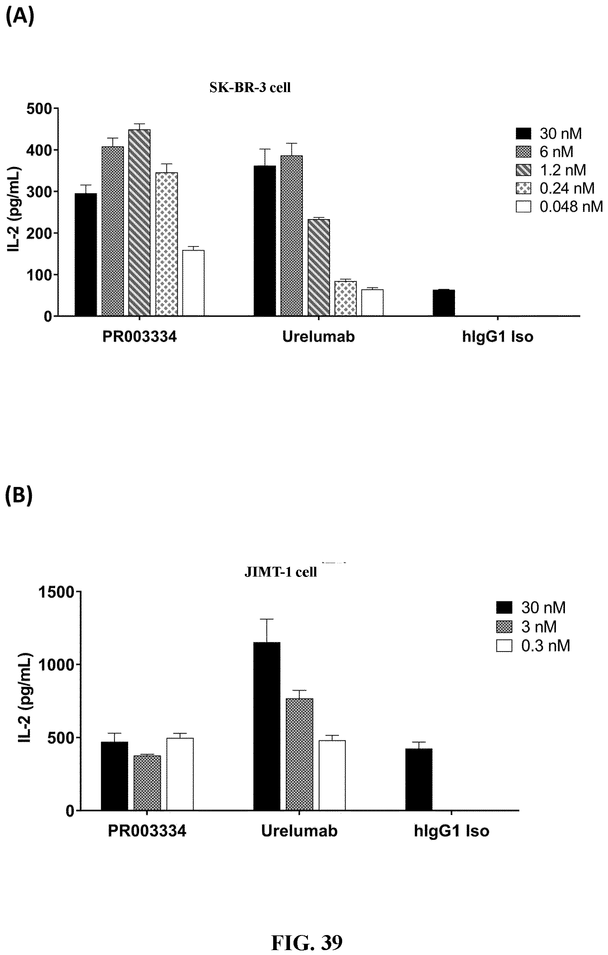

As shown in FIG. 39, the T cell activation by the B7H4×4-1BB bispecific antibody was specifically dependent on the expression of B7H4. (A) The bispecific antibody PR003334 was able to specifically activate T cells to release IL-2 in the presence of SK-BR-3 cells highly expressing B7H4; and (B) PR003334 was unable to activate T cells in the presence of JIMT-1 cells not expressing B7H4. Urelumab had no target specificity, so it was able to activate T cells even without expression of B7H4.

### Example 6.7. Serum stability

In this example, the stability of B7H4×4-1BB bispecific antibody molecules of IgG-VH symmetric structures (PR003334 and PR003335) in high concentrations of human sera was investigated. Specifically, the bispecific antibody molecules were diluted in a 3-fold gradient with 90% human serum at an initial concentration of 100 nM for a total of 8 concentration points. The samples at each concentration were divided into 6 aliquots, incubated at 37 °C for 0 day, 1 day, 2 days, 4 days, 7 days and 14 days, quickly frozen with liquid nitrogen, and stored at -40 °C. Then, samples were collected after incubation in serum for different periods of time, and tested for the binding activity of the bispecific antibody molecules to SK-BR-3 cells and CHOK1/hu 4-1BB cells according to the methods described in Example 6.4.1 and Example 6.5.1, to investigate the change in the binding activity of the bispecific antibody molecules after incubation in serum for different periods of time.

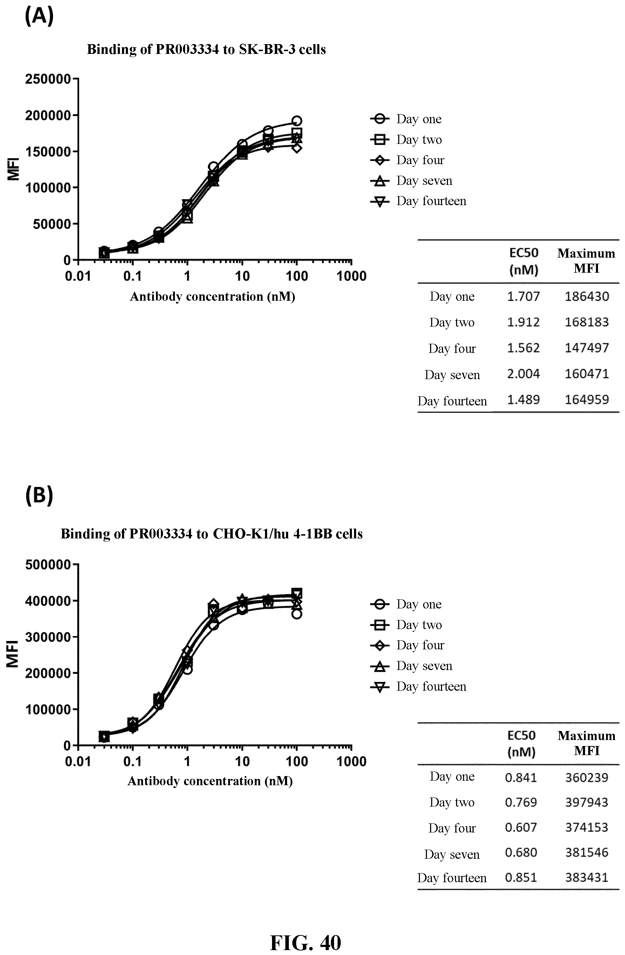

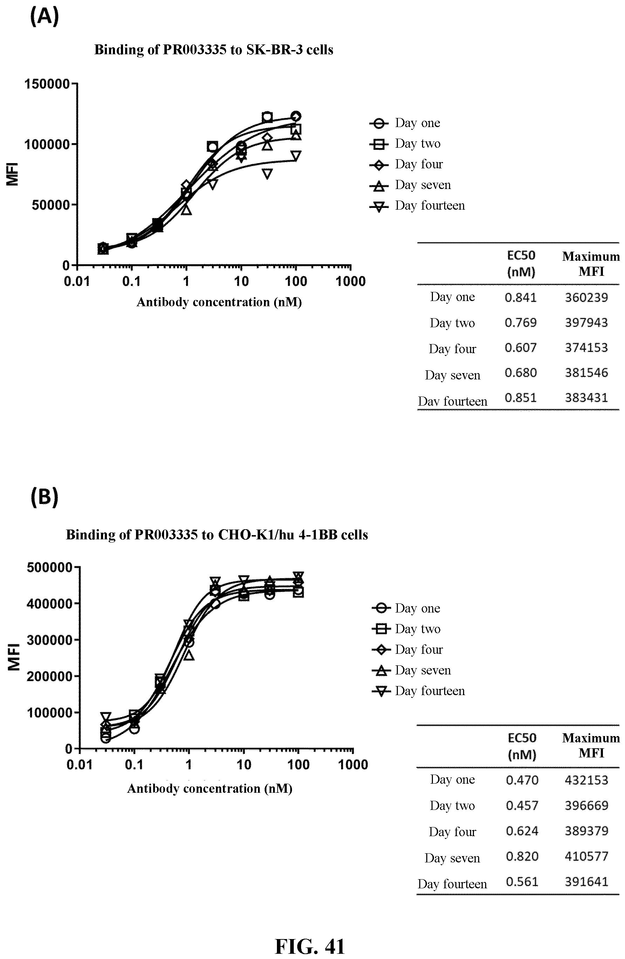

The bispecific antibody molecules PR003334 (FIG. 40, Table 6-15) and PR003335 (FIG. 41, Table 6-16) were stable in human serum, and showed no significant change in the binding activity of both the B7H4 and 4-1BB ends even after 14 days of incubation.

**Table 6-15. Binding activity of PR003334 to target cells after incubation in serum for different periods of time**

| PR003334 | Binding to SK-BR-3 | | Binding to CHO-K1/hu 4-1BB | |
|---|---|---|---|---|
| | $EC_{50}$ (nM) | Maximum MFI | $EC_{50}$ (nM) | Maximum MFI |
| Day one | 1.707 | 186430 | 0.841 | 360239 |
| Day two | 1.912 | 168183 | 0.769 | 397943 |
| Day four | 1.562 | 147497 | 0.607 | 374153 |
| Day seven | 2.004 | 160471 | 0.68 | 381546 |
| Day fourteen | 1.489 | 164959 | 0.851 | 383431 |

**Table 6-16. Binding activity of PR003335 to target cells after incubation in serum for different periods of time**

| PR003335 | Binding to SK-BR-3 | | Binding to CHO-K1/hu 4-1BB | |
|---|---|---|---|---|
| | $EC_{50}$ (nM) | Maximum MFI | $EC_{50}$ (nM) | Maximum MFI |
| Day one | 1.285 | 112575 | 0.470 | 432153 |
| Day two | 1.039 | 102557 | 0.457 | 396669 |
| Day four | 1.072 | 120474 | 0.624 | 389379 |
| Day seven | 1.41 | 93520 | 0.820 | 410577 |
| Day fourteen | 0.6645 | 78631 | 0.561 | 391641 |

### Example 6.8. Pharmacokinetic study

In this example, the pharmacokinetic properties of the B7H4×4-1BB bispecific antibody molecules of IgG-VH symmetric structures (PR003334 and PR003335) in mice were investigated.

The test was performed as follows: for each test antibody molecule, 6 female C57BL/6 mice weighing 18-22 g were selected and were administered with the bispecific antibody by intravenous injection at a dose of 5 mg/kg. The whole blood of 3 mice in one group was collected before the administration and 15 min, 24 h (1 day), 4 days and 10 days after the administration, and the whole blood of 3 mice in the other group was collected before the administration and 5 h, 2 days, 7 days and 14 days after the administration. The whole blood was left to stand for 30 min for coagulation and centrifuged at 2,000 rpm at 4 °C for 5 min, and the isolated serum sample was cryopreserved at -80 °C until it was taken for analysis. In this example, the drug concentration in the serum of mice was quantitatively determined by two ELISA methods. The ELISA method I, namely the Fc end detection (overall detection) method, was performed by capturing the protein containing human Fc in the serum of mice using a goat anti-human Fc polyclonal antibody coating a 96-well plate, and then adding an HRP-labeled goat anti-human Fc secondary antibody; and the ELISA method II, namely the 4-1BB end detection (functional domain detection) method, was performed by capturing the antigen-binding protein specifically recognizing human 4-1BB in the serum of mice using a human 4-1BB protein (Acro biosystems, #41B-H5227) coating a 96-well plate, and then adding an HRP-labeled goat anti-human Fc secondary antibody. Pharmacokinetic parameters were analyzed using Phoenix WinNonlin software version 8.2 by non-compartmental analysis (NCA).

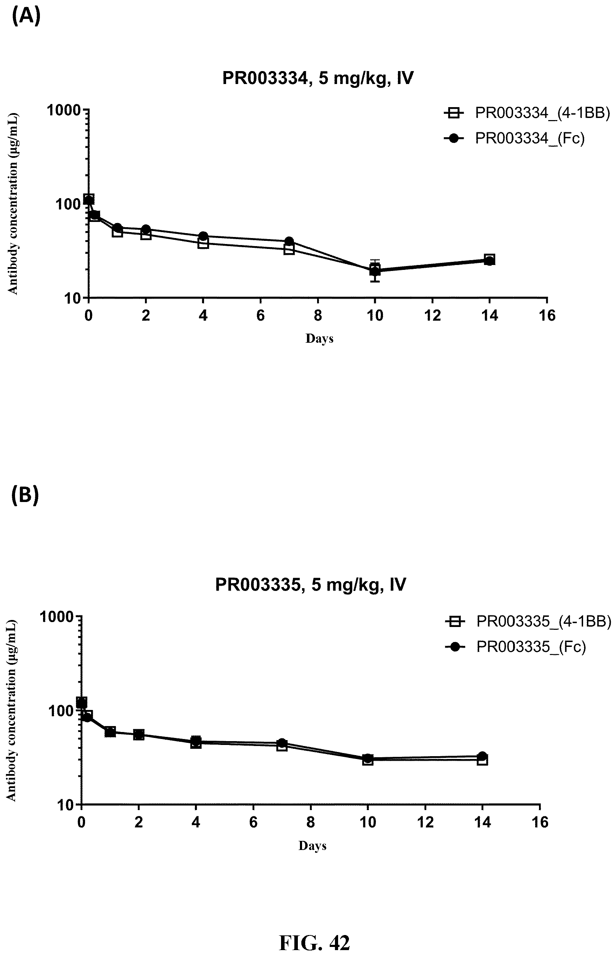

As shown in FIG. 42 and Table 6-17, the bispecific antibody molecules of IgG-VH structures (PR003334 and PR003335) had similar pharmacokinetics to conventional IgG antibodies: under the overall detection method, the serum half-life $t_{1/2}$ values of PR003334 and PR003335 in mice were about 8 days and 14 days, respectively; and under the functional domain detection method, the serum half-life $t_{1/2}$ values of PR003334 and PR003335 in mice were about 9 days and 12 days, respectively.

**Table 6-17. Pharmacokinetics of B7H4×4-1BB bispecific antibody molecules in C57BL/6 mice**

| Bispecific antibody molecules | PR003334 | PR003335 |
|---|---|---|
| Animals (number) | C57BL/6 mice (n = 6) | C57BL/6 mice (n = 6) |
| Antibody dosage | 5 mg/kg, I.V. | 5 mg/kg, I.V. |

(continued)

| PK parameters | Fc end detection | 4-1BB end detection | Fc end detection | 4-1BB end detection |
|---|---|---|---|---|
| T1/2 (hour) | 193 | 218 | 336 | 295 |
| Vd (mL/kg) | 76 | 89 | 81 | 81 |
| AUC ($\mu$g*hour/mL) | 12,680 | 11,471 | 14,813 | 14,286 |
| Cl (mL/hour/kg) | 0.28 | 0.28 | 0.17 | 0.19 |
| C0 ($\mu$g/mL) | 111 | 114 | 123 | 124 |

**Example 6.9. Anti-tumor efficacy of bispecific antibody molecules**

In this example, the anti-tumor efficacy of B7H4×4-1BB bispecific antibody molecule PR003334 in a BALB/c-hCD137/CT26-hB7H4 mouse tumor model was investigated.

The test was performed as follows: female BALB/c-hCD137 mice (BALB/c mice introduced with exogenous human 4-1BB transgene, GemPharmatech) aged 6-8 weeks were selected, and were inoculated subcutaneously with CT26-hB7H4 tumor cells (mouse colon cancer cells CT26 introduced with exogenous human B7H4 transgene, Harbour BioMed) in the logarithmic growth phase on the right dorsal side of the experimental mice at a dose of $5\times10^5$ cells/mouse. When the mean tumor volume reached 80 mm$^3$, the mice were randomized into groups of 6. On the day of grouping, the drug diluted with PBS at a specific concentration was administered by intraperitoneal injection (i.p.), twice a week for a total of 6 doses (BIW × 3), with PBS as a blank control group. Tumor volume and body weight of mice were measured on the day of the first administration and on days 4, 7, 11, 14 and 18 thereafter. The tumor volume was calculated as follows: tumor volume (mm$^3$) = 0.5 × (long diameter of tumor × short diameter of tumor$^2$). After the experiment was completed, tumor-bearing mice were euthanized, stripped of tumors and weighed. The tumor volume, body weight, and the like of mice in each group were calculated and the results were expressed as mean ± standard error (Mean ± SEM). The different treatment groups were separately compared with the control group for any significant difference by one-way analysis of variance (one-way ANOVA).

In this example, the anti-4-1BB monoclonal antibody Urelumab was used as a positive control molecule. The dose of 6 mpk (mg/kg) for the bispecific antibody molecule PR003334 and the dose of 5 mpk for Urelumab were equivalent doses at the same molar concentration (converted according to molecular weight); and the dose of 18 mpk for the bispecific antibody molecule PR003334 and the dose of 15 mpk for Urelumab were equivalent doses at the same molar concentration. An anti-mouse PD-1 antibody (Bio X Cell, clone RMP1-14, #BE0146) at a dose of 10 mpk was also used as a control molecule. PBS was used as a blank control group.

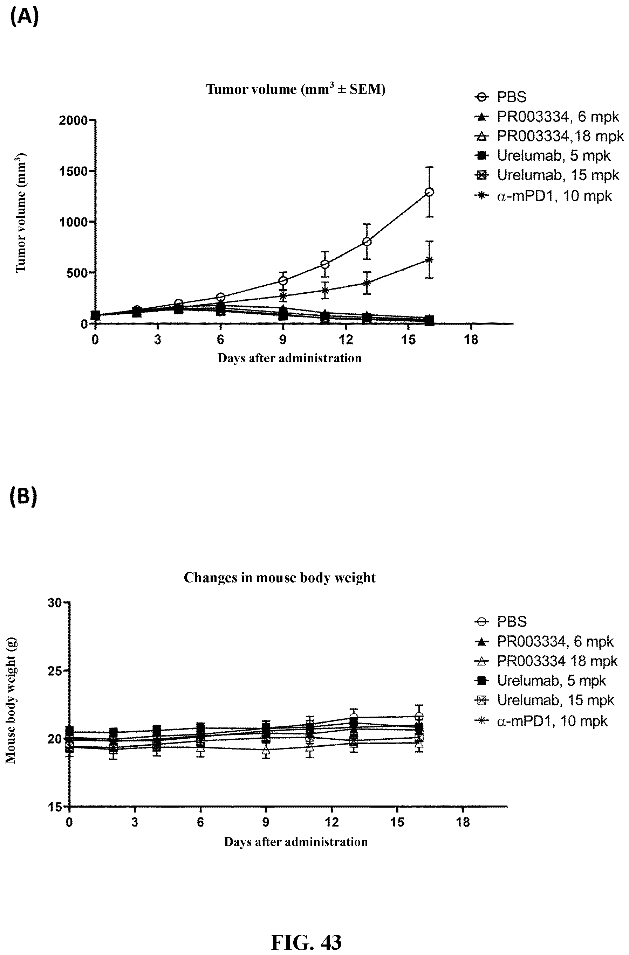

As shown in FIG. 43, the bispecific antibody molecule PR003334 and Urelumab both had the efficacy of inhibiting the tumor growth in each dose group, and were superior to the PD-1 antibody (A). The change in body weight of mice in each group was within the normal range (B).

**Example 6.10. Determination of stability and physicochemical properties of bispecific antibody molecules by UNcle**

In this example, the physicochemical properties such as molecular stability of the B7H4×4-1BB bispecific antibody molecules of IgG-VH symmetric structures (PR003334, PR003335, and PR003338) were determined by the method described in Example 2.5.As shown in Table 6-18, those bispecific antibody molecules had good stability.

**Table 6-18. Determination parameters for bispecific antibody molecules in UNcle**

| | PR003334 | PR003335 | PR003338 |
|---|---|---|---|
| Tm (°C) | 60.32 | 65.59 | 62.17 |
| Tagg (°C) at 266 nm | 52.00 | 50.39 | 54.14 |
| Tagg (°C) at 473 nm | / | / | / |
| Initial SLS at 266 nm (counts x104) | 0.26 | 0.22 | 0.20 |
| Max SLS at 266 nm (counts x104) | 1.00 | 0.40 | 0.34 |
| Initial SLS at 473 nm (counts) | < 0 | < 0 | < 0 |
| Max SLS at 473 nm (counts) | < 0 | < 0 | < 0 |

(continued)

|  | PR003334 | PR003335 | PR003338 |
|---|---|---|---|
| Initial diameter (nm) (25°C) | 16.22 | 16.38 | 11.74 |
| Final diameter (nm) (95°C) | 23.85 | 17.51 | 16.52 |
| Initial PDI | 0.267 | 0.229 | 0.043 |
| Final PDI | 0.089 | 0.194 | 0.185 |

## Example 6.11. Summary

In this example, B7H4×4-1BB bispecific antibody molecules of a variety of structures are constructed using the antigen-binding domain Fab of the anti-B7H4 IgG antibody and the antigen-binding domain VH of the anti-4-1BB HCAb antibody. This shows the flexibility of constructing the molecular structure of bispecific antibodies based on HCAbs, and the T cell function-activating activity is regulated through different structure types, relative positions, binding valences and other parameters.

The activation of T cells by Urelumab is not target-specific, which is one of the causes for its clinical toxic and side effects. However, the T cell activation effect of B7H4×4-1BB bispecific antibodies is specifically dependent on the expression of B7H4. In the presence of cells highly expressing B7H4, the bispecific antibodies are able to specifically activate T cells; whereas in the absence of B7H4 expression, the bispecific antibodies are unable to activate T cells. Therefore, the B7H4×4-1BB bispecific antibodies have better safety than 4-1BB monoclonal antibodies such as Urelumab.

In another aspect, the B7H4×4-1BB bispecific antibodies are able to mediate the crosslinking and trimerization of 4-1BB in the presence of B7H4 to transmit T cell activation signals, and their T cell activation ability is even better than that of Urelumab. In the Fab-HCAb and IgG-VH tetravalent symmetric structures, VH of the 4-1BB end is bivalent, and its T cell activation ability is slightly superior to that of Urelumab; in the IgG-VH-VH hexavalent symmetric structures, VH of the 4-1BB end is multivalent, and its T cell activation ability is significantly enhanced and is significantly superior to that of Urelumab.

In conclusion, the B7H4×4-1BB bispecific antibody molecules with good safety, outstanding functional activity and good molecular stability are constructed in this example.

## Example 7. BCMA×CD3 bispecific antibodies

## Example 7.1. Background

BCMA (B-cell maturation antigen, TNFRSF 17, CD269) is a transmembrane protein belonging to the TNF receptor superfamily, and is involved in the maturation, growth and survival of B cells. BCMA has two main ligands: the high-affinity ligand APRIL (proliferation-inducing ligand) and the low-affinity ligand BAFF (B-cell activating factor). BCMA is a highly differentiated plasma cell-selective protein whose expression is restricted to the B cell lineage and is found predominantly on plasma cells and plasmablasts, and to some extent on memory B cells, but not on peripheral B cells. BCMA is expressed in malignant plasma cells of multiple myeloma (MM) patients and supports the growth and survival of multiple myeloma cells. Multiple myeloma is the second most common hematologic malignancy following non-Hodgkin's lymphoma, accounting for about 13% of hematological malignant tumors. As an emerging target for multiple myeloma, BCMA antibodies can act on MM cells through a variety of mechanisms.

Currently, the antibodies against BCMA with the fastest development in the clinical stage include CA8-J6M0 hIgG1 (hereinafter also referred to as positive control 1, and numbered PR000274 in the examples) from GlaxoSmithKline (GSK) Plcand an antibody-drug conjugate prepared on the basis of CA8-J6M0, belantamab mafodotin (CA8-J6M0-mcMMAF, GSK2857916). GSK2857916 has been used in multiple clinical trials to treat patients with different types of MM. Data from a small clinical study showed that objective response rate (ORR) reached 60% and median progression-free survival (PFS) was 12 months in 35 patients with relapsed or refractory MM who were over-pretreated (most patients had failed at least 5 therapies). In terms of safety, the most common side effects include corneal events, thrombocytopenia and anemia, which are all associated with cytotoxic agents conjugated to ADCs.

Currently, bispecific antibodies under clinical development include AMG-420 from Amgen, REGN-5458 from Regeneron, CC-93269 from Celgene, JNJ-64007957 from Johnson, and TNB383B from Abbvie. However, those bispecific antibodies also have problems such as short half-life and cytokine release syndrome (CRS). The bispecific antibody TNB383B was subjected to the clinical phase I in 2019 for MM, which is characterized by low cytokine release levels. However, TNB383B bind to neither BCMA nor CD3 of cynomolgus monkeys and cannot be evaluated toxicologically in cynomolgus monkeys.

Therefore, there is an urgent need to develop safer and more effective bispecific antibodies that target both human BCMA and CD3 and can bind to cynomolgus monkey BCMA and CD3.

**Example 7.2. Discovery of anti-BCMA HCAb antibodies**

The BCMA antigen can be used to immunize laboratory animals such as mice, rats, rabbits, sheep, and camels, to obtain an antibody molecule specifically binding to BCMA. Typically, the resulting antibody molecules are non-human antibodies. After obtaining non-human antibodies, these molecules need to be humanized by antibody engineering technology to reduce immunogenicity and improve druggability. However, the humanization of antibodies is complex in terms of the technology, and the humanized molecules tend to have reduced affinity for antigens. On the other hand, advances in transgenic technology have made it possible to develop genetically engineered mice that carry a human immunoglobulin immune repertoire and have the endogenous murine immune repertoire deleted. The antibodies produced by the transgenic mice have fully human sequences, so that further humanization is not needed, and the efficiency of developing therapeutic antibodies is greatly improved. The Harbour HCAb mouse (Harbour Antibodies BV, WO2010/109165A2) is a transgenic mice carrying an immune repertoire of human immunoglobulins, capable of producing novel "heavy chain"-only antibodies that are only half the size of conventional IgG antibodies. The antibodies produced have only human antibody "heavy chain" variable domains and mouse Fc constant domains. Due to the absence of light chain, this antibody almost solves the problems of light chain mismatch and heterodimerization, allowing the technical platform to develop products that are difficult to realize by the conventional antibody platform.

**Example 7.2.1. Immunization of mice with BCMA antigen**

Harbour HCAb mice were subjected to multiple rounds of immunization with a soluble recombinant human BCMA-ECD-Fc fusion protein. The antigenic protein was mixed with an immunoadjuvant to form an immunogenic reagent, which was then injected subcutaneously via the groin or intraperitoneally. In each round of immunization, each mouse received a total injection dose of 100 $\mu$L. In the first round of immunization, each mouse received an immunization with an immunogenic reagent prepared by mixing 50 $\mu$g of antigenic protein (recombinant human BCMA-ECD-Fc, ACRO Biosystems, #BC7-H82F0) with complete Freund's adjuvant (Sigma, #F5881) in a 1:1 volume ratio. In each subsequent round of booster immunization, each mouse received an immunization with an immunogenic reagent prepared by mixing 25 $\mu$g of antigenic protein with Sigma Adjuvant System adjuvant (Sigma, #S6322). The interval between rounds of booster immunization was at least two weeks. In general, there are no more than five rounds of booster immunizations. The immunization was performed at days 0, 14, 28, 42, 56 and 70; and the antibody titer in serum of mice was measured at days 49 and 77. The last round of booster immunization was performed at a dose of 25 $\mu$g of antigenic protein per mouse 5 days before the isolation of HCAb mouse splenic B cells.

**Example 7.2.2. Acquisition of HCAb monoclonal antibodies and antibody sequences**

When the titer of the BCMA-specific antibody in the serum of mice was detected to reach a certain level, spleen cells of the mice were taken, from which B cells were isolated, and the CD138-positive plasma cells and BCMA antigen-positive B cell populations were sorted using a BD FACSAria™ III cell sorter. RNA was extracted and reversely transcribed into cDNA, and human VH gene was amplified by PCR. The amplified VH gene fragment was constructed into a mammalian cell expression plasmid pCAG vector encoding the sequence of the heavy chain Fc domain of the human IgG1 antibody. Mammalian host cells (such as human embryonic kidney cell HEK293) were transfected with the plasmid for expression, and the expressed HCAb antibody supernatant was screened with recombinant human BCMA-Fc, Avitag recombinant protein (ACRO Biosystems, #BC7-H82F0) through Mirrorball (SPT Labtech, mirrorball® fluorescence cytometer). The obtained positive monoclonal antibody supernatant was further identified by flow cytometry FACS. The binding ability of the antibody supernatant to cells such as an HEK293T cell line HEK293T/hBCMA (KYinno, KC-0233) highly expressing human BCMA, an HEK293T cell line HEK293T/cyno BCMA (KYinno, KC-0979) highly expressing cynomolgus monkey BCMA, and a cell line NCI-H929 (ATCC, CRL-9068) highly expressing human BCMA was tested by FACS. Four positive monoclonal antibodies were obtained by multiple rounds of screening. The nucleotide sequences encoding the variable domains of the antibody molecules and the corresponding amino acid sequences were obtained through conventional sequencing means. In this example, the sequences of the variable domains of the anti-BCMA monoclonal antibody molecules obtained from immunized Harbour HCAb mice were human antibody sequences.

**Example 7.2.3. Preparation of fully human recombinant anti-BCMA antibodies**

Purified recombinant anti-BCMA heavy chain antibodies were prepared according to the method described in Example 2.2 using plasmids encoding HCAb antibodies by conventional recombinant protein expression and purification techniques.

The sequence numbers (SEQ ID NOs) corresponding to the amino acid sequence of the heavy chain variable domain, the amino acid sequences of the full-length heavy chain, and the amino acid sequences of CDRs defined according to the Chothia scheme of the anti-BCMA HCAb antibodies in this example are listed in Table 7-1. The sequence numbers (SEQ ID NOs) corresponding to the light and heavy chain sequences of the positive control 1, CA8-J6M0 (antibody No. PR000274) are listed in Table 7-2.

**Table 7-1. Sequence numbers of anti-BCMA HCAb antibodies**

| Clone No. | Antibody No. | Heavy chain | VH | HCDR1 | HCDR2 | HCDR3 |
|---|---|---|---|---|---|---|
| 1005P10H8 | PR000940 | 316 | 248 | 15 | 75 | 133 |
| 1005P16A10 | PR000943 | 317 | 249 | 24 | 76 | 134 |
| 1005P36F3 | PR001035 | 318 | 250 | 25 | 77 | 135 |
| 1005P63B7 | PR001046 | 319 | 251 | 26 | 78 | 136 |

**Table 7-2. Sequence number of a control anti-BCMA antibody**

| Antibodies alias | Antibody No. | Heavy chain | Light chain |
|---|---|---|---|
| Positive control 1 | PR000274 | 309 | 354 |

### Example 7.3. Binding of HCAb antibodies to BCMA

This example is intended to investigate the binding activity of anti-BCMA HCAb antibodies to human and cynomolgus monkey BCMAs. The binding ability of the recombinant anti-BCMA antibodies to cells such as an HEK293T cell line HEK293T/hBCMA (KYinno, KC-0233) highly expressing human BCMA, an HEK293T cell line HEK293T/cyno BCMA (KYinno, KC-0979) highly expressing cynomolgus monkey BCMA, and a cell line NCI-H929 (ATCC, CRL-9068) highly expressing human BCMA was tested by flow cytometry FACS. Specifically, HEK293T/hBCMA cells and HEK293T/-cyno BCMA cells were digested and resuspended in a DMEM complete medium. In addition, NCI-H929 cell suspension was collected. The density of each of the three cells was adjusted to $1 \times 10^6$ cells/mL. The cells were seeded in a 96-well V-bottom plate (Corning, #3894) at 100 μL/well, followed by the addition of test antibodies diluted in a 3-fold gradient at a concentration that was 2 times the final concentration, each at 100 μL/well. The cells were incubated at 4 °C for 1 h away from light. Thereafter, the cells in each well were rinsed twice with 100 μL of pre-cooled PBS, and centrifuged at 500 g at 4 °C for 5 min, and then the supernatant was discarded. Thereafter, a fluorescent secondary antibody (Alexa Fluor 488-conjugated AffiniPure Goat Anti-Human IgG, Fcγ Fragment Specific, Jackson, #109-545-06, diluted in a 1:500 ratio) was added at 100 μL/well, and the plate was incubated at 4 °C for 30 min away from light. The cells in each well were rinsed twice with 100 μL of pre-cooled PBS, and centrifuged at 500 g at 4 °C for 5 min, and then the supernatant was discarded. Finally, the cells in each well were resuspended in 200 μL of pre-cooled PBS. Fluorescence signal values were read using a BD FACS CANTOII flow cytometer or an ACEA NovoCyte flow cytometer, and the data were processed and analyzed using FlowJo v10 (FlowJo, LLC) software. The data were processed and analyzed by plotting using GraphPad Prism 8 software, and binding curves of the antibodies to target cells, $EC_{50}$ values and other parameters were obtained through four-parameter nonlinear fitting.

In this example, the anti-BCMA monoclonal antibody PR000274 was used as a positive control 1 molecule.

### Example 7.3.1. Binding to HEK293T/hBCMA cells highly expressing human BCMA

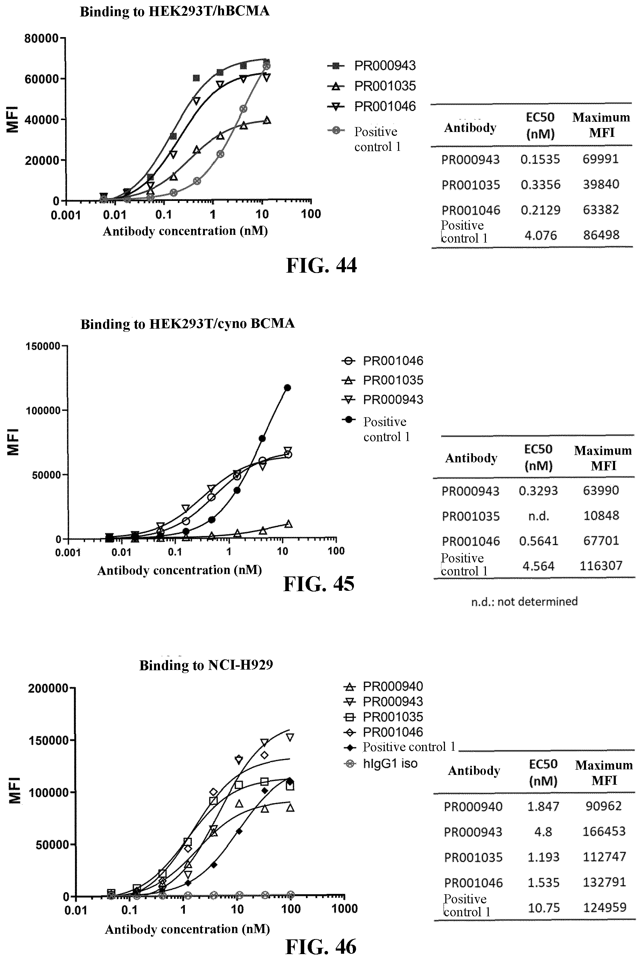

As shown in FIG. 44 and Table 7-3, the anti-human BCMA HCAb monoclonal antibodies PR000943, PR001035 and PR001046 were able to bind to HEK293T/hBCMA, and all had superior $EC_{50}$ in the binding curves to the positive control 1.

**Table 7-3. Binding to HEK293T/hBCMA**

| Antibody | $EC_{50}$ (nM) | Maximum MFI |
|---|---|---|
| PR000943 | 0.1535 | 69991 |
| PR001035 | 0.3356 | 39840 |

(continued)

| Antibody | EC$_{50}$ (nM) | Maximum MFI |
|---|---|---|
| PR001046 | 0.2129 | 63382 |
| Positive control 1 | 4.076 | 86498 |

### Example 7.3.2. Binding to NCI-H929 cells highly expressing human BCMA

As shown in FIG. 46 and Table 7-4, the anti-human BCMA HCAb monoclonal antibodies PR000940, PR000943, PR001035 and PR001046 were able to bind to NCI-H929, and all had superior EC$_{50}$ in the binding curves to the positive control 1.

### Table 7-4. Binding to NCI-H929

| Antibody | EC$_{50}$ (nM) | Maximum MFI |
|---|---|---|
| PR000940 | 1.847 | 90962 |
| PR000943 | 4.8 | 166453 |
| PR001035 | 1.193 | 112747 |
| PR001046 | 1.535 | 132791 |
| Positive control 1 | 10.75 | 124959 |

### Example 7.3.3. Binding to HEK293T/cyno BCMA cells highly expressing cynomolgus monkey BCMA

As shown in FIG. 45 and Table 7-5, the anti-human BCMA HCAb monoclonal antibodies PR000943 and PR001046 were able to bind to HEK293T//cyno BCMA, and both had superior EC$_{50}$ in the binding curves to the positive control 1.PR001035 had a weaker binding ability to cynomolgus monkey BCMA.

### Table 7-5. Binding to HEK293T/cyno BCMA

| Antibody | EC$_{50}$ (nM) | Maximum MFI |
|---|---|---|
| PR000943 | 0.3293 | 63990 |
| PR001035 | n.d. | 10848 |
| PR001046 | 0.5641 | 67701 |
| Positive control 1 | 4.564 | 116307 |

### Example 7.4. Killing of target cells by internalization of HCAb antibodies

This example is intended to investigate the killing of cells expressing human BCMA mediated by internalization of anti-human BCMA HCAb antibodies. Specifically, an HEK293T cell line HEK293T/hBCMA (KYinno, KC-0233) highly expressing human BCMA was digested, and the cells were resuspended in a DMEM complete medium, counted and inoculated to a black-wall and transparent-bottom 96-well plate (Perkin Elmer, #6005225) at 5000 or 10000 cells/well. The recombinant antibody was serially diluted to 9 different concentrations and added, with the final concentration starting at 100 nM. $\alpha$-hFc-MMAF (Moradec, #AH-102-AF) was added to make a final concentration of 1 $\mu$g/mL. The plate was incubated at 37 °C, 5% CO$_2$ for 72 h, and the cells were lysed with a CTG kit (Promega, #G7573). The luminescence was detected using an Enspire™ multi-functional microplate reader (PerkinElmer, Inc.). The data were processed and analyzed by plotting using GraphPad Prism 8 software, and binding curves of the antibodies to target cells, EC$_{50}$ values, maximum killing rate and other parameters were obtained through four-parameter nonlinear fitting.

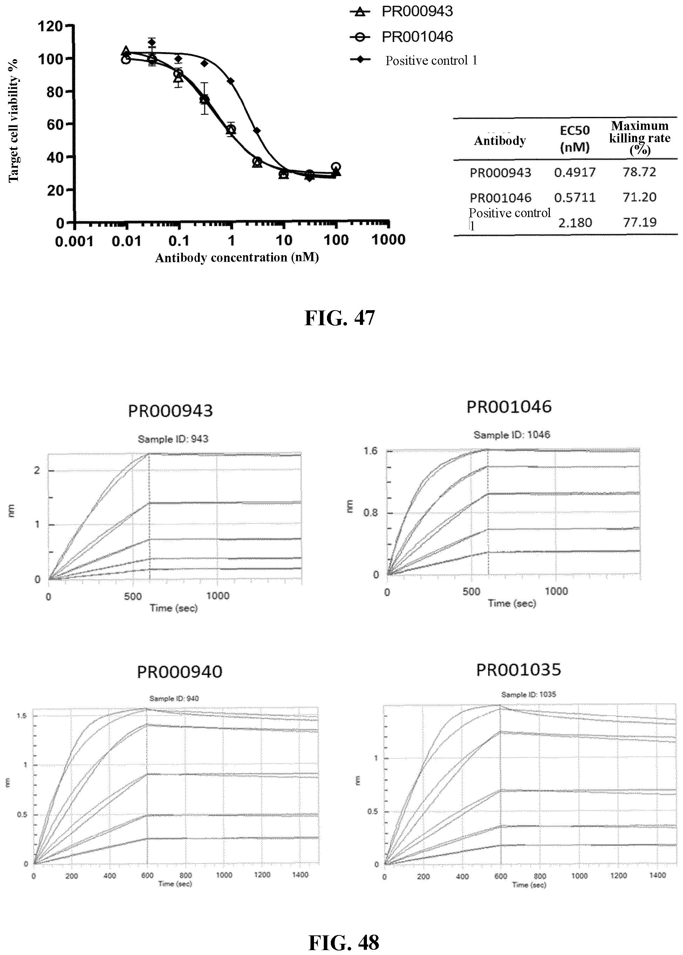

As shown in FIG. 47 and Table 7-6, the anti-human BCMA HCAb antibodies PR000943 and PR001046 had superior internalization ability to the positive control 1 molecule.

**Table 7-6. Target cell killing mediated by antibody internalization**

| Antibody | EC$_{50}$ (nM) | Maximum killing rate (%) |
|---|---|---|
| PR000943 | 0.4917 | 78.72 |
| PR001046 | 0.5711 | 71.20 |
| Positive control 1 | 2.180 | 77.19 |

**Example 7.5. Binding affinity for BCMA determined by a BLI method**

In this example, the binding kinetics between the anti-BCMA antibody and the BCMA antigenic protein were analyzed by the Biolayer Interferometry (BLI) technique using an Octet Red96e molecular interaction analyzer (ForteBio, Octet Red96e). In this example, the BCMA protein was biotinylated human BCMA-Fc, Avitag recombinant protein (ACRO Biosystems, #BC7-H82F0), and the assay buffer was $1\times$ kinetics buffer (diluted from $10\times$ kinetics buffer (ForteBio, #18-1105)).

When the affinity between the antigen and the antibody (at multiple concentrations) was determined, the rotation speed of sensor was set at 1000 rpm/min. Two columns of SA sensors (8 sensors in each column; with the sensors in the first column referred to as the reference SA sensor and the sensors in the second column referred to as the test SA sensor) were firstly equilibrated in the assay buffer for 10 min. Then, the biotinylated human BCMA-Fc, Avitag recombinant protein was captured by test SA sensors with a capture height of 0.9-1.1 nm, and the reference SA sensors were immersed in the buffer for 30 s. Then, the SA sensors were equilibrated in the assay buffer for 2 min. The two columns of SA sensors were then associated with the anti-BCMA antibody diluted in a gradient (e.g., the antibody concentration may be 6 concentrations obtained by a two-fold gradient dilution from 50 nM, and 0 nM) for 5 min, and then dissociated for 10 min.

When data analysis was performed using Octet Data Analysis software (Fortebio, version 11.0), the reference signals were subtracted by a double reference mode; the data were fitted by a "1:1 Global fitting" method, and the kinetic parameters of the binding of the antigen to the antibody were calculated to obtain kon (1/Ms) values, kdis (1/s) values and KD (M) values.

As shown in FIG. 48 and Table 7-7, the anti-human BCMA HCAb antibodies had stronger binding affinity for the human BCMA protein.

**Table 7-7. Binding affinity of anti-BCMA HCAb antibodies for human BCMA protein**

| Antibody No. | KD (M) | kon (1/Ms) | kdis (1/s) | Full R^2 |
|---|---|---|---|---|
| PR000940 | 1.52E-10 | 4.01E+05 | 6.09E-05 | 0.995 |
| PR000943 | 1.62E-10 | 1.19E+05 | 1.93E-05 | 0.9986 |
| PR001035 | 2.76E-10 | 3.25E+05 | 8.98E-05 | 0.9926 |
| PR001046 | 2.53E-11 | 4.23E+05 | 1.07E-05 | 0.9983 |

**Example 7.6. Optimization of HCAb antibody sequences to improve binding affinity for BCMA**

In this example, the binding affinity of the HCAb antibody PR001046 for BCMA was improved by an antibody engineering method.

**Example 7.6.1. Acquisition of variants of PR001046**

Mutants with improved binding affinity for BCMA were obtained by two rounds of site-directed mutagenesis of the CDR regions of the variable region VH of the HCAb antibody PR001046.

In the first round, 35 sites in the three CDR regions (specified by the Kabat CDR scheme) of PR001046 VH were scanned site-by-site for amino acids to create single-site saturation mutagenesis libraries for 35 different sites. The 35 saturation mutagenesis libraries were screened by BCMA binding ELISA, positive clones with a signal of 2-fold over PR001046 VH were picked out for sequencing and further tested for their binding ability to BCMA by BCMA (at multiple concentrations obtained by a gradient dilution) combined with ELISA. Several mutation hot sites were preferentially selected.

In the second round, mutation hot sites preferentially selected in the first round were randomly combined to establish a library containing all mutation combinations. The combination library was then screened. Positive clones were subjected to sequencing and sequence analysis, and further tested for their binding ability to BCMA. Several mutants were selected.

The variant molecules of PR001046 VH were represented by the corresponding clone numbers, e.g., PR001046-R1-12G7, PR001046-R2-7E7 and the like. The VH regions of the variant molecules were constructed into a prokaryotic expression vector with Flag and His tags, and then the variant molecules were prepared by the conventional recombinant protein expression and purification techniques, with the corresponding sequence numbers listed in Table 7-8.Finally, the binding ability of the recombinant mutant molecules was determined by FACS, BLI and other methods.

**Table 7-8. Sequence numbers of PR001046-derived variant molecules**

| Variant clone No. | Antibody No. | Heavy chain | VH | HCDR1 | HCDR2 | HCDR3 |
|---|---|---|---|---|---|---|
| | PR001046 | 319 | 251 | 26 | 78 | 136 |
| 1046-R1-12G7 | PR004559 | 330 | 265 | 26 | 90 | 136 |
| 1046-R2-10F1 | PR004560 | 331 | 266 | 34 | 78 | 146 |
| 1046-R2-10H1 | PR004561 | 332 | 267 | 35 | 78 | 147 |
| 1046-R2-10H5 | PR004562 | 333 | 268 | 35 | 90 | 147 |
| 1046-R2-1A1 | PR004563 | 334 | 269 | 36 | 90 | 146 |
| 1046-R2-2D4 | PR004564 | 335 | 270 | 35 | 76 | 147 |
| 1046-R2-2G4 | PR004565 | 336 | 271 | 34 | 78 | 148 |
| 1046-R2-4G10 | PR004566 | 337 | 272 | 35 | 76 | 136 |
| 1046-R2-5E1 | PR004567 | 338 | 273 | 35 | 90 | 146 |
| 1046-R2-5H1 | PR004568 | 339 | 274 | 34 | 76 | 136 |
| 1046-R2-5H2 | PR004569 | 340 | 275 | 35 | 76 | 146 |
| 1046-R2-6D6 | PR004570 | 341 | 276 | 34 | 76 | 146 |
| 1046-R2-6F1 | PR004571 | 342 | 277 | 35 | 90 | 148 |
| 1046-R2-7E7 | PR004572 | 343 | 278 | 35 | 90 | 136 |
| 1046-R2-8B1 | PR004573 | 344 | 279 | 35 | 90 | 136 |
| 1046-R2-9E5 | PR004574 | 345 | 280 | 34 | 76 | 146 |

**Example 7.6.2. Analysis of dissociation rate order of variant molecules binding to BCMA protein by the BLI method**

The binding ability of PR001046 VH-derived single-domain variant molecules to BCMA was determined by the BLI technique by the method described in Example 7.5. In this example, the supernatant containing PR001046 VH-derived variant molecules was directly used, and the binding kinetics between the variant molecules and biotinylated human BCMA-Fc, Avitag recombinant protein were analyzed using an Octet Red96e molecular interaction analyzer. The variant molecules were ranked by the dissociation rate in the binding kinetics, and those with slower dissociation rate (stronger affinity) were selected. In this example, only the dissociation rate in the binding kinetics of each variant molecule to BCMA was analyzed and its relative value (Kdis Fold) to the original molecule (PR001046 VH) was calculated; a "Kdis Fold" of less than 1 indicates that the variant molecule has a slower dissociation rate than the original molecule.

In this example, multi-concentration binding kinetics analysis was not used for calculating the antigen-antibody binding affinity, and gradient dilution of the antibody to be analyzed was not needed, therefore, only one SA sensor was needed for each antibody, allowing multiple samples to be analyzed in each analysis cycle, thereby increasing the analysis throughput. By this method, the dissociation rate analysis can be performed on supernatant samples of a large number of variant molecules, and the molecules with superior dissociation rate can be selected.

As shown in Table 7-9, 16 PR001046 VH-derived variant molecules showed a slower dissociation rate (i.e., stronger affinity for BCMA) than the original molecule.

**Table 7-9. Dissociation rate Kdis of PR001046-derived variant molecules binding to BCMA protein**

| Variant clone | Antibody No. | Conc. (nM) | Response | Kdis (1/s) | Kdis fold |
|---|---|---|---|---|---|
| | PR001046 | 100 | 0.2606 | 6.96E-04 | 1.00 |

(continued)

| Variant clone | Antibody No. | Conc. (nM) | Response | Kdis (1/s) | Kdis fold |
|---|---|---|---|---|---|
| 1046-R1-12G7 | PR004559 | 100 | 0.2614 | 3.79E-04 | 0.54 |
| 1046-R2-10F1 | PR004560 | 100 | 0.5732 | 3.01E-04 | 0.43 |
| 1046-R2-10H1 | PR004561 | 100 | 0.2694 | 3.00E-04 | 0.43 |
| 1046-R2-10H5 | PR004562 | 100 | 0.5352 | 2.17E-04 | 0.31 |
| 1046-R2-1A1 | PR004563 | 100 | 0.4933 | 2.59E-04 | 0.37 |
| 1046-R2-2D4 | PR004564 | 100 | 0.5701 | 2.28E-04 | 0.33 |
| 1046-R2-2G4 | PR004565 | 100 | 0.5572 | 2.36E-04 | 0.34 |
| 1046-R2-4G10 | PR004566 | 100 | 0.4245 | 2.86E-04 | 0.41 |
| 1046-R2-5E1 | PR004567 | 100 | 0.5781 | 2.01E-04 | 0.29 |
| 1046-R2-5H1 | PR004568 | 100 | 0.7325 | 2.30E-04 | 0.33 |
| 1046-R2-5H2 | PR004569 | 100 | 0.7303 | 2.54E-04 | 0.36 |
| 1046-R2-6D6 | PR004570 | 100 | 0.4373 | 2.34E-04 | 0.34 |
| 1046-R2-6F1 | PR004571 | 100 | 0.7035 | 2.19E-04 | 0.31 |
| 1046-R2-7E7 | PR004572 | 100 | 0.2896 | 2.36E-04 | 0.34 |
| 1046-R2-8B1 | PR004573 | 100 | 0.2680 | 2.82E-04 | 0.41 |
| 1046-R2-9E5 | PR004574 | 100 | 0.2901 | 3.14E-04 | 0.45 |

### Example 7.6.3. Binding of variant molecules to NCI-H929 cells highly expressing human BCMA

The binding ability of PR001046 VH-derived single-domain variant molecules to NCI-H929 cells highly expressing human BCMA was determined by the method described in Example 7.3. It is specifically noted that the recombinant variant molecules are VH single-domain antibody molecules with a Flag tag, and a fluorescent secondary antibody against the tag is required in the FACS assay.

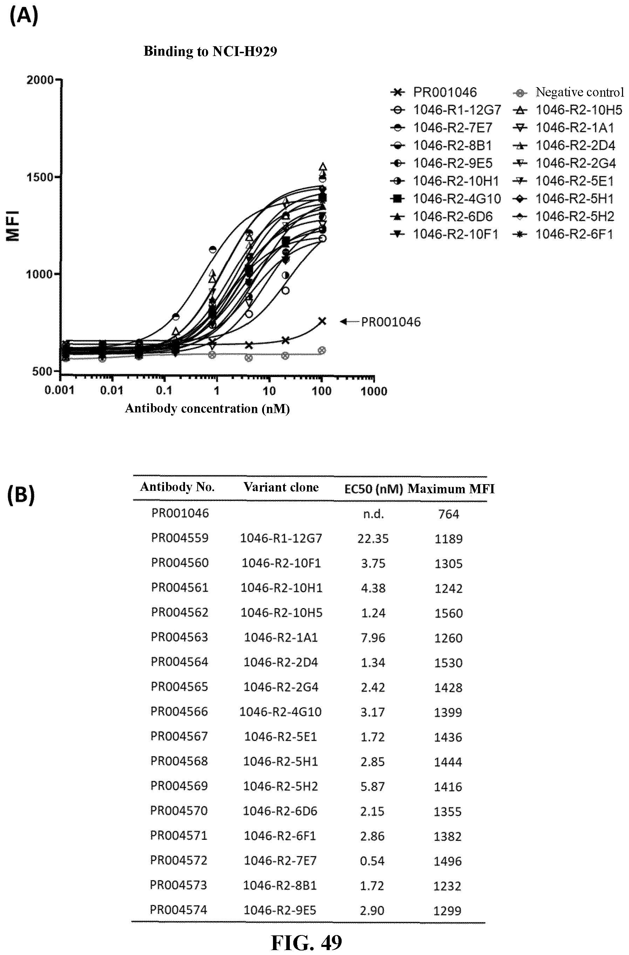

As shown in FIG. 49 and Table 7-10, 16 PR001046 VH-derived single-domain variant molecules were able to better bind to NCI-H929 cells, with superior binding ability to the original molecule.

### Table 7-10. Binding to NCI-H929

| Antibody No. | Variant clone | EC$_{50}$ (nM) | Maximum MFI |
|---|---|---|---|
| PR001046 | | n.d. | 764 |
| PR004559 | 1046-R1-12G7 | 22.35 | 1189 |
| PR004560 | 1046-R2-10F1 | 3.75 | 1305 |
| PR004561 | 1046-R2-10H1 | 4.38 | 1242 |
| PR004562 | 1046-R2-10H5 | 1.24 | 1560 |
| PR004563 | 1046-R2-1A1 | 7.96 | 1260 |
| PR004564 | 1046-R2-2D4 | 1.34 | 1530 |
| PR004565 | 1046-R2-2G4 | 2.42 | 1428 |
| PR004566 | 1046-R2-4G10 | 3.17 | 1399 |
| PR004567 | 1046-R2-5E1 | 1.72 | 1436 |
| PR004568 | 1046-R2-5H1 | 2.85 | 1444 |
| PR004569 | 1046-R2-5H2 | 5.87 | 1416 |
| PR004570 | 1046-R2-6D6 | 2.15 | 1355 |
| PR004571 | 1046-R2-6F1 | 2.86 | 1382 |

(continued)

| Antibody No. | Variant clone | EC$_{50}$ (nM) | Maximum MFI |
|---|---|---|---|
| PR004572 | 1046-R2-7E7 | 0.54 | 1496 |
| PR004573 | 1046-R2-8B1 | 1.72 | 1232 |
| PR004574 | 1046-R2-9E5 | 2.90 | 1299 |

## Example 7.7. Discovery of anti-CD3 IgG antibodies

The anti-CD3 antibody is an important component in the construction of T-Cell engager bispecific antibodies. In this example, the discovery and preparation of anti-CD3 antibodies by different methods were introduced.

## Example 7.7.1. Discovery of fully human CD3 antibodies

The Harbour H2L2 mouse (Harbour Antibodies BV) is a transgenic mouse carrying an immune repertoire of human immunoglobulins that produces antibodies with intact human antibody variable domains and rat constant domains. Harbour H2L2 mice were subjected to multiple rounds of immunization with a CD3 antigen. The antigen may be a recombinant human CD3 fusion protein, or a short peptide containing the first 27 amino acid residues at the N terminus of human CD3E, or a human T cell. When the titer of the CD3-specific antibody in the serum of mice was detected to reach a certain level, spleen cells of the mice were taken, from which B cells were isolated. Then, RNA was extracted and cDNA was prepared by RT-PCR. VH and Vκ fragments were amplified by PCR using cDNA as a template, and scFv fragments were prepared by overlap PCR and ligated to a pre-linearized vector pHBM-scFv-ST. The ligation product obtained were transformed into MC1061F' electroporation competent cells (Lucigen, #60512-1), and a phage library was prepared. Multiple rounds of biopanning were performed with biotinylated antigenic protein, and screening was performed with CHO-K1 cells CHO-K1/hCD3 (Harbour BioMed) highly expressing a human CD3/TCR complex. Positive clones were identified and sequenced. Single-chain variable region fusion proteins (scFv-Fc structure) binding to CD3 obtained by screening by the phage display technique are listed in Table 7-11.

**Table 7-11. Sequence numbers of fully human anti-human CD3 antibodies**

| Clone No. | Antibody No. | Polypeptide chain | VL | VH | LCDR1 | LCDR2 | LCDR3 | HCDR1 | HCDR2 | HCDR3 |
|---|---|---|---|---|---|---|---|---|---|---|
| M15-C10 | PR002161 | 490 | 294 | 258 | 177 | 191 | 221 | 15 | 84 | 141 |
| M15-H2 | PR002163 | 491 | 295 | 259 | 178 | 197 | 222 | 31 | 85 | 142 |
| M23-FII | PR002337 | 492 | 296 | 260 | 177 | 191 | 223 | 31 | 86 | 141 |
| M27-BII | PR002340 | 493 | 297 | 259 | 179 | 198 | 224 | 31 | 85 | 142 |

## Example 7.7.2. Humanization of CD3 antibodies

SP34 is a mouse-derived anti-human CD3 antibody and has a function of activating T cells. SP34 is one of the very few antibodies that can bind to multiple primate CD3 antibodies (e.g., human and cynomolgus monkey CD3 antibodies) (see Salmeron, A. et al., J Immunol 147 (1991) 3047-3052; Conrad M.L., et al., Cytometry A 71 (2007) 925-933). The variable region sequences VH and VL are derived from WO2016071004A1.

In this example, the sequences of the variable regions of the mouse antibody SP34 were humanized by the method of "CDR grafting". Specifically, the CDRs of the VH of SP34 were grafted to the framework region of the VH of a human antibody, and the CDRs of the VL of SP34 were grafted to the framework region of the VL of a human antibody. The sequences of the framework regions of the VH or VL of a human antibody may be derived from human germline gene sequences or from antibody sequences following V(D)J rearrangement or from consensus sequences of specific VH or VL gene family of a human antibody. In this example, the sequences of the framework regions provided by the human germline gene sequences were used as a humanization template sequence. Specifically, the sequences of the framework regions FR1, FR2, and FR3 were provided by the human germline V gene segment and the sequences of the framework region FR4 was provided by the human germline J gene segment. Finally, humanized variable region (VH or VL) sequences were constructed in the arrangement of (human) FR1-(mouse) CDR1-(human) FR2-(mouse) CDR2-(human) FR3-(mouse) CDR3-(human) FR4.

In this example, the sequence of the human germline V gene segment IGHV3-73*01 or the human germline V gene segment IGHV3-23*01 in combination with the human germline J gene segment IGHJ1*01 was used as a humanization template to provide the sequences of the framework regions. Moreover, amino acid mutations were introduced in one or more of positions 30, 73, 76, 78, 93 and 94 (according to the Chothia numbering scheme) to obtain a plurality of different VH variant sequences. In this example, the sequence of the human germline V gene segment IGLV7-46*02 in combination with the human germline J gene segment IGLJ2*01 or the sequence of the human germline V gene segment IGKV1-39*01 in combination with the human germline J gene segment IGKJ4*01 was also used as a humanization template to provide the framework region sequences. Moreover, amino acid mutations were introduced in zero or one or more of positions 2, 36, 46, 49, 66, 69, 71 and 87 (according to the Chothia numbering scheme) to obtain a plurality of different VL variant sequences.

The VH variant sequences and VL variant sequences derived from SP34 were combined in pairs, and recombinant antibodies were prepared by the method described in Example 2.2. Recombinant antibodies and corresponding sequence numbers of SP34 and its derived variants are listed in Table 7-12 and Table 7-13.

**Table 7-12. SP34-derived humanized recombinant antibodies**

| Antibody No. | Variable region variants | Fc type (mutation) |
|---|---|---|
| PR000260 | Initial sequences of SP34 mouse antibody | Human IgG1 (LALA) |
| PR000511 | VH: VH3231; VL: VL7461 | Human IgG1 (LALA) |
| PR001848 | VH: VH3232; VL: VL7461 | Human IgG1 (AAG) |
| PR003886 | VH: VH3730_N30S; VL: VL7461 | Human IgG1 (LALA) |
| PR004616 | VH: VH3233; VL: VL7461 | Human IgG1 (LALA) |
| PR000510 | VH: VH3731; VL: VL7461, constructed as scFv | Human IgG1 (LALA) |

(AAG: L234A, L235A, P329G; LALA: L234A, L235A.)

**Table 7-13. Sequence numbers of sequences and CDR sequences of SP34-derived humanized recombinant antibodies**

| Antibody No. | Light chain | Heavy chain | VL | VH | LCDR1 | LCDR2 | LCDR3 | HCDR1 | HCDR2 | HCDR3 |
|---|---|---|---|---|---|---|---|---|---|---|
| PR000260 | 352 | 307 | 287 | 239 | 172 | 192 | 216 | 20 | 68 | 128 |
| PR000511 | 357 | 313 | 291 | 245 | 172 | 192 | 216 | 20 | 68 | 128 |
| PR001848 | 357 | 325 | 291 | 257 | 172 | 192 | 216 | 30 | 68 | 128 |
| PR003886 | 357 | 328 | 291 | 263 | 172 | 192 | 216 | 30 | 68 | 128 |
| PR004616 | 357 | 346 | 291 | 281 | 172 | 192 | 216 | 30 | 68 | 128 |
| PR000510 | None | 489 | 291 | 244 | 172 | 192 | 216 | 20 | 68 | 128 |

**Example 7.7.3. Binding of CD3 antibodies to T cells**

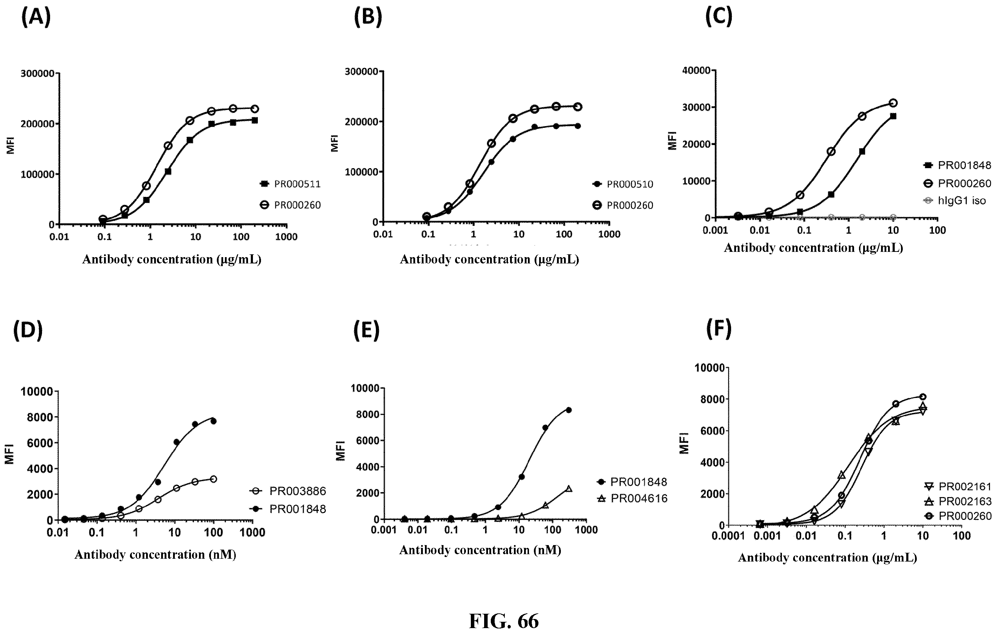

The binding ability of the CD3 antibodies to human T cells was tested by flow cytometry FACS and the method described in Example 7.10. As shown in FIG. 66, the CD3 antibodies were all able to bind to human T cells; in particular, the fully human CD3 antibodies (PR002161 and PR002163) had similar binding ability to T cells to SP34 chimeric antibody (PR000260) (FIG. 66 (F)); PR000510 and PR000511 (SP34-derived humanized antibodies) had close binding ability to T cells to PR000260 (FIG. 66 (A-B)); PR001848, PR003886, and PR004616 (SP34-derived humanized antibodies) had weakened binding ability to T cells to different degrees after sequence optimization (FIG. 66 (C-E)), to selectively modulate T cell activation function in subsequent design of bispecific antibodies.

**Example 7.8. Construction of bispecific antibody molecules using anti-CD3 IgG antibodies and anti-BCMA HCAb antibodies**

In this example, BCMA×CD3 bispecific antibody molecules of a variety of structures were constructed using the antigen-binding domain Fab of the anti-CD3 IgG antibody and the antigen-binding domain VH of the anti-BCMA HCAb antibody. The sequence of the antigen-binding domain Fab of the anti-CD3 antibodies was derived from the sequences described in Table 7-11 and Table 7-13; and the sequence of the antigen-binding domain VH of the anti-BCMA antibodies was derived from the sequences described in Table 7-1 and Table 7-8.

In this and subsequent examples, the control molecules include: anti-BCMA monoclonal antibody CA8-J6M0 (antibody No. PR000274, sequence source patent US9273141B2) used as the positive control 1 molecule; the BCMA×CD3 bispecific antibody molecule (antibody No. PR002199, sequence source patent WO2019133761A1) used as the positive control 2 molecule; the BCMA×CD3 bispecific antibody (antibody No. PR003106, sequence source patent WO2017134134A1) used as the positive control 3 molecule; and the hIgG1 chimeric antibody derived from the anti-CD3 monoclonal antibody SP34 (antibody No. PR000260, sequence source patent WO2016071004A1). The sequences of the control molecules are shown in Table 7-19.

**Example 7.8.1. Construction of molecules of Fab-Fc-VH(n) asymmetric structures**

BCMA×CD3 bispecific antibody molecules of Fab-Fc-VH(n, n = {1,2}) asymmetric structures were designed according to the structures described in Example 1.5 and Example 1.6 using anti-CD3 IgG antibodies and anti-BCMA heavy chain antibodies, with the results summarized in Table 7-14; and molecules of trivalent asymmetric structures containing two different anti-BCMA VH domain sequences were designed according to the structures described in Example 1.6.2, with the results summarized in Table 7-15. The antibody molecule samples were prepared and analyzed according to the method described in Example 2, with the results summarized in Table 7-16.

**Table 7-14. BCMA×CD3 bispecific antibody molecules of Fab-Fc-VH(n) asymmetric structures**

| Structure No. | Bispecific antibody molecules | CD3 Antibody (Fab) | BCMA antibody (VH_B) | Structure of Fab end | Number of repetition n for VH_B | Linker peptide | Fc type of Fab end (mutation) | Fc type of VH_B end (mutation) |
|---|---|---|---|---|---|---|---|---|
| 11 | PR001987 | PR000511 | PR000940 | Normal | 1 | None | Human IgG1 (knob, AAG) | Human IgG1 (hole, AAG) |
| 11 | PR001988 | PR000511 | PR000943 | Normal | 1 | None | Human IgG1 (knob, AAG) | Human IgG1 (hole, AAG) |
| 11 | PR001989 | PR000511 | PR001035 | Normal | 1 | None | Human IgG1 (knob, AAG) | Human IgG1 (hole, AAG) |
| 11 | PR001990 | PR000511 | PR001046 | Normal | 1 | None | Human IgG1 (knob, AAG) | Human IgG1 (hole, AAG) |
| 12 | PR001991 | PR000511 | PR000940 | cross VH/VL | 1 | None | Human IgG1 (knob, AAG) | Human IgG1 (hole, AAG) |
| 12 | PR001992 | PR000511 | PR000943 | cross VH/VL | 1 | None | Human IgG1 (knob, AAG) | Human IgG1 (hole, AAG) |
| 12 | PR001993 | PR000511 | PR001035 | cross VH/VL | 1 | None | Human IgG1 (knob, AAG) | Human IgG1 (hole, AAG) |
| 12 | PR001994 | PR000511 | PR001046 | cross VH/VL | 1 | None | Human IgG1 (knob, AAG) | Human IgG1 (hole, AAG) |
| 13 | PR001995 | PR000511 | PR000940 | cross Fd/LC | 1 | None | Human IgG1 (knob, AAG) | Human IgG1 (hole, AAG) |
| 13 | PR001996 | PR000511 | PR000943 | cross Fd/LC | 1 | None | Human IgG1 (knob, AAG) | Human IgG1 (hole, AAG) |
| 13 | PR001997 | PR000511 | PR001035 | cross Fd/LC | 1 | None | Human IgG1 (knob, AAG) | Human IgG1 (hole, AAG) |
| 13 | PR001998 | PR000511 | PR001046 | cross Fd/LC | 1 | None | Human IgG1 (knob, AAG) | Human IgG1 (hole, AAG) |
| 14 | PR002299 | PR001848 | PR000940 | Normal | 2 | RT-GS_5-KL | Human IgG1 (knob, AAG) | Human IgG1 (hole, AAG) |
| 14 | PR002300 | PR001848 | PR000940 | Normal | 2 | RT-GS_15-KL | Human IgG1 (knob, AAG) | Human IgG1 (hole, AAG) |

(continued)

| Structure No. | Bispecific antibody molecules | CD3 Antibody (Fab) | BCMA antibody (VH_B) | Structure of Fab end | Number of repetition n for VH_B | Linker peptide | Fc type of Fab end (mutation) | Fc type of VH_B end (mutation) |
|---|---|---|---|---|---|---|---|---|
| 14 | PR002301 | PR001848 | PR000940 | Normal | 2 | RT-GS_25-KL | Human IgG1 (knob, AAG) | Human IgG1 (hole, AAG) |
| 14 | PR002308 | PR001848 | PR001046 | Normal | 2 | RT-GS_5-KL | Human IgG1 (knob, AAG) | Human IgG1 (hole, AAG) |
| 14 | PR002309 | PR001848 | PR001046 | Normal | 2 | RT-GS-15-KL | Human IgG1 (knob, AAG) | Human IgG1 (hole, AAG) |
| 14 | PR002310 | PR001848 | PR001046 | Normal | 2 | RT-GS-25-KL | Human IgG1 (knob, AAG) | Human IgG1 (hole, AAG) |
| 11 | PR002929 | PR001848 | PR001046 | Normal | 1 | None | Human IgG1 (knob, LALA) | Human IgG1 (hole, LALA) |
| 11 | PR002935 | PR004616 | PR001046 | Normal | 1 | None | Human IgG1 (knob, LALA) | Human IgG1 (hole, LALA) |
| 14 | PR002892 | PR001848 | PR001046 | Normal | 2 | GS_5 | Human IgG1 (knob, LALA) | Human IgG1 (hole, LALA) |
| 14 | PR002895 | PR001848 | PR001046 | Normal | 2 | GS_15 | Human IgG1 (knob, LALA) | Human IgG1 (hole, LALA) |
| 14 | PR002950 | PR004616 | PR001046 | Normal | 2 | GS_5 | Human IgG1 (knob, LALA) | Human IgG1 (hole, LALA) |
| 14 | PR002953 | PR004616 | PR001046 | Normal | 2 | GS_15 | Human IgG1 (knob, LALA) | Human IgG1 (hole, LALA) |
| 14 | PR002898 | PR001848 | PR001046 | Normal | 2 | GS_2 | Human IgG1 (knob, LALA) | Human IgG1 (hole, LALA) |
| 14 | PR002901 | PR001848 | PR001046 | Normal | 2 | GS_4 | Human IgG1 (knob, LALA) | Human IgG1 (hole, LALA) |
| 14 | PR002956 | PR004616 | PR001046 | Normal | 2 | GS_2 | Human IgG1 (knob, LALA) | Human IgG1 (hole, LALA) |
| 14 | PR002959 | PR004616 | PR001046 | Normal | 2 | GS_4 | Human IgG1 (knob, LALA) | Human IgG1 (hole, LALA) |

| Structure No. | Bispecific antibody molecules | CD3 Antibody (Fab) | BCMA antibody (VH_B) | Structure of Fab end | Number of repetition n for VH_B | Linker peptide | Fc type of Fab end (mutation) | Fc type of VH_B end (mutation) |
|---|---|---|---|---|---|---|---|---|
| 14 | PR003177 | PR003886 | PR001046 | Normal | 2 | GS_5 | Human IgG1 (knob, LALA) | Human IgG1 (hole, LALA) |
| 14 | PR003178 | PR003886 | PR001046 | Normal | 2 | GS_15 | Human IgG1 (knob, LALA) | Human IgG1 (hole, LALA) |
| 14 | PR003690 | PR003886 | PR001046 | Normal | 2 | GS_15 | Human IgG1 (knob, LALA, YTE) | Human IgG1 (hole, LALA, YTE) |
| 11 | PR003867 | PR003886 | PR004559 | Normal | 1 | None | Human IgG1 (knob, LALA) | Human IgG1 (hole, LALA) |
| 11 | PR003868 | PR003886 | PR004563 | Normal | 1 | None | Human IgG1 (knob, LALA) | Human IgG1 (hole, LALA) |
| 11 | PR003869 | PR003886 | PR004564 | Normal | 1 | None | Human IgG1 (knob, LALA) | Human IgG1 (hole, LALA) |
| 11 | PR003870 | PR003886 | PR004565 | Normal | 1 | None | Human IgG1 (knob, LALA) | Human IgG1 (hole, LALA) |
| 11 | PR003871 | PR003886 | PR004566 | Normal | 1 | None | Human IgG1 (knob, LALA) | Human IgG1 (hole, LALA) |
| 11 | PR003872 | PR003886 | PR004567 | Normal | 1 | None | Human IgG1 (knob, LALA) | Human IgG1 (hole, LALA) |
| 11 | PR003873 | PR003886 | PR004568 | Normal | 1 | None | Human IgG1 (knob, LALA) | Human IgG1 (hole, LALA) |
| 11 | PR003874 | PR003886 | PR004569 | Normal | 1 | None | Human IgG1 (knob, LALA) | Human IgG1 (hole, LALA) |
| 11 | PR003875 | PR003886 | PR004570 | Normal | 1 | None | Human IgG1 (knob, LALA) | Human IgG1 (hole, LALA) |
| 11 | PR003876 | PR003886 | PR004571 | Normal | 1 | None | Human IgG1 (knob, LALA) | Human IgG1 (hole, LALA) |
| 11 | PR003877 | PR003886 | PR004572 | Normal | 1 | None | Human IgG1 (knob, LALA) | Human IgG1 (hole, LALA) |

(continued)

| Structure No. | Bispecific antibody molecules | CD3 Antibody (Fab) | BCMA antibody (VH_B) | Structure of Fab end | Number of repetition n for VH_B | Linker peptide | Fc type of Fab end (mutation) | Fc type of VH_B end (mutation) |
|---|---|---|---|---|---|---|---|---|
| 11 | PR003878 | PR003886 | PR004573 | Normal | 1 | None | Human IgG1 (knob, LALA) | Human IgG1 (hole, LALA) |
| 11 | PR003879 | PR003886 | PR004574 | Normal | 1 | None | Human IgG1 (knob, LALA) | Human IgG1 (hole, LALA) |
| 11 | PR003880 | PR003886 | PR004560 | Normal | 1 | None | Human IgG1 (knob, LALA) | Human IgG1 (hole, LALA) |
| 11 | PR003881 | PR003886 | PR004561 | Normal | 1 | None | Human IgG1 (knob, LALA) | Human IgG1 (hole, LALA) |
| 11 | PR003882 | PR003886 | PR004562 | Normal | 1 | None | Human IgG1 (knob, LALA) | Human IgG1 (hole, LALA) |

(mutation code: **Knob:** S354C, T366W; **Hole:** Y349C, T366S, L368A, Y407V; **AAG:** L234A, L235A, P329G; **LALA:** L234A, L235A; **YTE:** M252Y, S254T, T256E.)

Table 7-15. BCMA×CD3 multispecific antibody molecules of Fab-Fc-VH(2) trivalent asymmetric structures containing two different anti-BCMA VH domain sequences

| Structure No. | Bispecific antibody molecules | CD3 antibody (Fab) | BCMA antibody (VH_C) | BCMA antibody (VH_B) | Structure of Fab end | Linker peptide (between VH_B and VH_C) | Fc type of Fab end (mutation) | Fc type of VH_B end (mutation) |
|---|---|---|---|---|---|---|---|---|
| 15 | PR002313 | PR001848 | PR000940 | PR000943 | Normal | RT-GS_15-KL | Human IgG1 (knob, AAG) | Human IgG1 (hole, AAG) |
| 15 | PR002326 | PR001848 | PR001035 | PR001046 | Normal | RT-GS_15-KL | Human IgG1 (knob, AAG) | Human IgG1 (hole, AAG) |
| 15 | PR002328 | PR001848 | PR001035 | PR001046 | Normal | RT-GS_25-KL | Human IgG1 (knob, AAG) | Human IgG1 (hole, AAG) |
| 15 | PR002332 | PR001848 | PR001046 | PR001035 | Normal | RT-GS_15-KL | Human IgG1 (knob, AAG) | Human IgG1 (hole, AAG) |

(mutation code: **Knob:** S354C, T366W; **Hole:** Y349C, T366S, L368A, Y407V; **AAG:** L234A, L235A, P329G.)

**Table 7-16. Expression of BCMA×CD3 bispecific antibody molecule proteins of Fab-Fc-VH(n) asymmetric structures**

| Structure No. | Bispecific antibody molecules | Expression system and volume | Plasmid transfection ratio | Yield (mg/L) after first purification | SEC-HPLC purity (%) |
|---|---|---|---|---|---|
| 11 | PR001987 | HEK293-F (40ml) | 3 : 2 : 4 | 61 | 67.88 |
| 11 | PR001988 | HEK293-F (40ml) | 3 : 2 : 4 | 50.7 | 66.65 |
| 11 | PR001989 | HEK293-F (40ml) | 3 : 2 : 4 | 76 | 86.06 |
| 11 | PR001990 | HEK293-F (40ml) | 3 : 2 : 4 | 68.3 | 76.97 |
| 12 | PR001991 | HEK293-F (40ml) | 3 : 2 : 4 | 20.8 | 71.97 |
| 12 | PR001992 | HEK293-F (40ml) | 3 : 2 : 4 | 8.7 | 77.76 |
| 12 | PR001993 | HEK293-F (40ml) | 3 : 2 : 4 | 12.6 | 77.71 |
| 12 | PR001994 | HEK293-F (40ml) | 3 : 2 : 4 | 17.1 | 80.01 |
| 13 | PR001995 | HEK293-F (40ml) | 3 : 2 : 4 | 118.3 | 70.29 |
| 13 | PR001996 | HEK293-F (40ml) | 3 : 2 : 4 | 71.3 | 71.98 |
| 13 | PR001997 | HEK293-F (40ml) | 3 : 2 : 4 | 60.7 | 79.66 |
| 13 | PR001998 | HEK293-F (40ml) | 3 : 2 : 4 | 62 | 39.96 |
| 14 | PR002299 | HEK293-F (40ml) | 1 : 1 : 1 | 20.5 | 46.03 |
| 14 | PR002300 | HEK293-F (40ml) | 1 : 1 : 1 | 31 | 80.48 |
| 14 | PR002301 | HEK293-F (40ml) | 1 : 1 : 1 | 14.4 | 75.42 |
| 14 | PR002308 | HEK293-F (40ml) | 1 : 1 : 1 | 14.7 | 64.92 |
| 14 | PR002309 | HEK293-F (40ml) | 1 : 1 : 1 | 31 | 75.96 |
| 14 | PR002310 | HEK293-F (40ml) | 1 : 1 : 1 | 26.8 | 71.46 |
| 15 | PR002313 | HEK293-F (40ml) | 1 : 1 : 1 | 24.8 | 70.13 |
| 15 | PR002326 | HEK293-F (40ml) | 1 : 1 : 1 | 14 | 84.62 |
| 15 | PR002328 | HEK293-F (40ml) | 1 : 1 : 1 | 27.5 | 79.04 |
| 15 | PR002332 | HEK293-F (40ml) | 1 : 1 : 1 | 23.1 | 82.13 |
| 11 | PR002929 | HEK293-F (40ml) | 1 : 1 : 1 | 13.5 | 94.45 |
| 11 | PR002935 | HEK293-F (40ml) | 1 : 1 : 1 | 4.55 | 94.26 |
| 14 | PR002892 | HEK293-F (40ml) | 1 : 1 : 1 | 6.5 | 76.9 |
| 14 | PR002895 | HEK293-F (40ml) | 1 : 1 : 1 | 16.9 | 84.34 |
| 14 | PR002950 | HEK293-F (40ml) | 1 : 1 : 1 | 3.3 | 75.26 |
| 14 | PR002953 | HEK293-F (40ml) | 1 : 1 : 1 | 7.4 | 89.17 |
| 14 | PR002898 | HEK293-F (40ml) | 1 : 1 : 1 | 5.6 | 81.72 |
| 14 | PR002901 | HEK293-F (40ml) | 1 : 1 : 1 | 5.8 | 83.72 |
| 14 | PR002956 | HEK293-F (40ml) | 1 : 1 : 1 | 5.4 | 78.14 |
| 14 | PR002959 | HEK293-F (40ml) | 1 : 1 : 1 | 6 | 73.9 |
| 14 | PR003177 | Expi-CHO (300ml) | 1 : 1 : 1 | 196 | 83.29 |
| 14 | PR003178 | Expi-CHO (300ml) | 1 : 1 : 1 | 188 | 83.04 |

(continued)

| Structure No. | Bispecific antibody molecules | Expression system and volume | Plasmid transfection ratio | Yield (mg/L) after first purification | SEC-HPLC purity (%) |
|---|---|---|---|---|---|
| 14 | PR003690 | Expi-CHO (300ml) | 1 : 1 : 1 | 200 | 83.81 |
| 11 | PR003867 | HEK293-F (30ml) | 1 : 1 : 1 | 11.46 | 100 |
| 11 | PR003868 | HEK293-F (30ml) | 1 : 1 : 1 | 0.12 | n.d. |
| 11 | PR003869 | HEK293-F (30ml) | 1 : 1 : 1 | 5.63 | 100 |
| 11 | PR003870 | HEK293-F (30ml) | 1 : 1 : 1 | 2.4 | 100 |
| 11 | PR003871 | HEK293-F (30ml) | 1 : 1 : 1 | 6.7 | 100 |
| 11 | PR003872 | HEK293-F (30ml) | 1 : 1 : 1 | 8.92 | 100 |
| 11 | PR003873 | HEK293-F (30ml) | 1 : 1 : 1 | 10.9 | 97.4 |
| 11 | PR003874 | HEK293-F (30ml) | 1 : 1 : 1 | 11.31 | 100 |
| 11 | PR003875 | HEK293-F (30ml) | 1 : 1 : 1 | 26.19 | 100 |
| 11 | PR003876 | HEK293-F (30ml) | 1 : 1 : 1 | 11.88 | 100 |
| 11 | PR003877 | HEK293-F (30ml) | 1 : 1 : 1 | 10.87 | 100 |
| 11 | PR003878 | HEK293-F (30ml) | 1 : 1 : 1 | 12.5 | 96.2 |
| 11 | PR003879 | HEK293-F (30ml) | 1 : 1 : 1 | 24.05 | 100 |
| 11 | PR003880 | HEK293-F (30ml) | 1 : 1 : 1 | 4.49 | 100 |
| 11 | PR003881 | HEK293-F (30ml) | 1 : 1 : 1 | 19.29 | 100 |
| 11 | PR003882 | HEK293-F (30ml) | 1 : 1 : 1 | 11.61 | 100 |

**Example 7.8.2. Construction of molecules of scFv-Fc-VH(n) asymmetric structures**

BCMA×CD3 bispecific antibody molecules of scFv-Fc-VH(n, n = {1,2}) asymmetric structures were designed according to the structures described in Example 1.7 and Example 1.8 using anti-CD3 IgG antibodies and anti-BCMA heavy chain antibodies, with the results summarized in Table 7-17. The antibody molecule samples were prepared and analyzed according to the method described in Example 2, with the results summarized in Table 7-18.

**Table 7-17. BCMA×CD3 bispecific antibody molecules of scFv-Fc-VH(n) asymmetric structures**

| Structure No. | Bispecific antibody molecules | CD3 Antibody (scFv) | BCMA Antibody (VH) | Structure of scFv end | Number of repetition n for VH_B | Linker peptide 2 (between VH_B and VH_B) | Fc type of Fab end (mutation) | Fc type of VH_B end (mutation) |
|---|---|---|---|---|---|---|---|---|
| 20 | PR001999 | PR000510 | PR000940 | VL-linker peptide-VH | 1 | None | Human IgG1 (knob, AAG) | Human IgG1 (hole, AAG) |
| 20 | PR002000 | PR000510 | PR000943 | VL-linker peptide-VH | 1 | None | Human IgG1 (knob, AAG) | Human IgG1 (hole, AAG) |
| 20 | PR002001 | PR000510 | PR001035 | VL-linker peptide-VH | 1 | None | Human IgG1 (knob, AAG) | Human IgG1 (hole, AAG) |
| 20 | PR002002 | PR000510 | PR001046 | VL-linker peptide-VH | 1 | None | Human IgG1 (knob, AAG) | Human IgG1 (hole, AAG) |
| 23 | PR002867 | PR002161 | PR001046 | VH-linker peptide-VL | 2 | RT-GS_15-KL | Human IgG1 (knob, AAG) | Human IgG1 (hole, AAG) |
| 23 | PR002868 | PR002163 | PR001046 | VH-linker peptide-VL | 2 | RT-GS_15-KL | Human IgG1 (knob, AAG) | Human IgG1 (hole, AAG) |
| 23 | PR002869 | PR002337 | PR001046 | VH-linker peptide-VL | 2 | RT-GS_15-KL | Human IgG1 (knob, AAG) | Human IgG1 (hole, AAG) |
| 23 | PR002870 | PR002340 | PR001046 | VH-linker peptide-VL | 2 | RT-GS_15-KL | Human IgG1 (knob, AAG) | Human IgG1 (hole, AAG) |

(mutation code: **Knob:** S354C, T366W; **Hole:** Y349C, T366S, L368A, Y407V; **AAG:** L234A, L235A, P329G.)

Table 7-18. Expression of BCMA×CD3 bispecific antibody molecule proteins of scFv-Fc-VH(n) asymmetric structures

| Structure No. | Bispecific antibody molecules | Expression system and volume | Yield (mg/L) after first purification | SEC-HPLC purity (%) |
|---|---|---|---|---|
| 20 | PR001999 | HEK293-F (40ml) | 18.4 | 74.35 |
| 20 | PR002000 | HEK293-F (40ml) | 13.6 | 77.84 |
| 20 | PR002001 | HEK293-F (40ml) | 1.0 | 60.21 |
| 20 | PR002002 | HEK293-F (40ml) | 14.0 | 79.47 |
| 23 | PR002867 | HEK293-F (40ml) | 7.4 | 66.68 |
| 23 | PR002868 | HEK293-F (40ml) | 8.8 | 67.14 |
| 23 | PR002869 | HEK293-F (40ml) | 17.4 | 83.62 |
| 23 | PR002870 | HEK293-F (40ml) | 6.5 | 74.84 |

**Example 7.8.3. Sequences of BCMA×CD3 bispecific antibody molecules and control molecules**

The sequence numbers corresponding to the sequences of the BCMA×CD3 bispecific antibody molecules constructed in this example and the control molecules are listed in Table 7-20 and Table 7-19. In the bispecific antibody molecules, the sequence of the antigen-binding domain Fab of the anti-CD3 antibodies was derived from the sequences described in Table 7-11 and Table 7-13; and the sequence of the antigen-binding domain VH of the anti-BCMA antibodies was derived from the sequences described in Table 7-1 and Table 7-8. The structure numbers in Table 7-20 correspond to those in Table 1-1 and FIG. 1. The sequence numbers of the corresponding CDR sequences of the different antigen-binding domains of the BCMA×CD3 bispecific or multispecific antibody molecules are listed in Table 7-21.

Table 7-19. Sequence numbers of control molecules

| Alias | Antibody No. | Description | Polypeptide chain 1 | Polypeptide chain 2 | Polypeptide chain 3 |
|---|---|---|---|---|---|
| Positive control 1 | PR000274 | Anti-BCMA monoclonal antibody CA8-J6M0 | 309 | 354 | None |
| Positive control 2 | PR002199 | BCMA×CD3 bispecific antibody, derived from WO2019133761A1 | 420 | 421 | 422 |
| Positive control 3 | PR003106 | BCMA×CD3 bispecific antibody, derived from WO2017134134A1 | 453 | None | None |
| SP34 | PR000260 | Anti-CD3 monoclonal antibody SP34 | 352 | 307 | None |

Table 7-20. Sequence numbers of BCMA×CD3 bispecific antibody molecules of this example

| Structure No. | Antibody No. | Polypeptide chain 1 | Polypeptide chain 2 | Polypeptide chain 3 |
|---|---|---|---|---|
| 11 | PR001987 | 357 | 411 | 410 |
| 11 | PR001988 | 357 | 411 | 412 |
| 11 | PR001989 | 357 | 411 | 413 |
| 11 | PR001990 | 357 | 411 | 414 |
| 11 | PR002929 | 357 | 444 | 449 |

(continued)

| Structure No. | Antibody No. | Polypeptide chain 1 | Polypeptide chain 2 | Polypeptide chain 3 |
|---|---|---|---|---|
| 11 | PR002935 | 357 | 450 | 449 |
| 11 | PR003867 | 357 | 454 | 465 |
| 11 | PR003868 | 357 | 454 | 466 |
| 11 | PR003869 | 357 | 454 | 467 |
| 11 | PR003870 | 357 | 454 | 468 |
| 11 | PR003871 | 357 | 454 | 469 |
| 11 | PR003872 | 357 | 454 | 470 |
| 11 | PR003873 | 357 | 454 | 471 |
| 11 | PR003874 | 357 | 454 | 472 |
| 11 | PR003875 | 357 | 454 | 473 |
| 11 | PR003876 | 357 | 454 | 474 |
| 11 | PR003877 | 357 | 454 | 475 |
| 11 | PR003878 | 357 | 454 | 476 |
| 11 | PR003879 | 357 | 454 | 477 |
| 11 | PR003880 | 357 | 454 | 478 |
| 11 | PR003881 | 357 | 454 | 479 |
| 11 | PR003882 | 357 | 454 | 480 |
| 12 | PR001991 | 415 | 416 | 410 |
| 12 | PR001992 | 415 | 416 | 412 |
| 12 | PR001993 | 415 | 416 | 413 |
| 12 | PR001994 | 415 | 416 | 414 |
| 13 | PR001995 | 417 | 418 | 410 |
| 13 | PR001996 | 417 | 418 | 412 |
| 13 | PR001997 | 417 | 418 | 413 |
| 13 | PR001998 | 417 | 418 | 414 |
| 14 | PR002299 | 357 | 423 | 424 |
| 14 | PR002300 | 357 | 423 | 425 |
| 14 | PR002301 | 357 | 423 | 426 |
| 14 | PR002308 | 357 | 423 | 427 |
| 14 | PR002309 | 357 | 423 | 428 |
| 14 | PR002310 | 357 | 423 | 429 |
| 14 | PR002892 | 357 | 444 | 445 |
| 14 | PR002895 | 357 | 444 | 446 |
| 14 | PR002898 | 357 | 444 | 447 |
| 14 | PR002901 | 357 | 444 | 448 |
| 14 | PR002950 | 357 | 450 | 445 |
| 14 | PR002953 | 357 | 450 | 446 |
| 14 | PR002956 | 357 | 450 | 447 |
| 14 | PR002959 | 357 | 450 | 448 |

(continued)

| Structure No. | Antibody No. | Polypeptide chain 1 | Polypeptide chain 2 | Polypeptide chain 3 |
|---|---|---|---|---|
| 14 | PR003177 | 357 | 454 | 445 |
| 14 | PR003178 | 357 | 454 | 446 |
| 14 | PR003690 | 357 | 463 | 464 |
| 15 | PR002313 | 357 | 423 | 430 |
| 15 | PR002326 | 357 | 423 | 431 |
| 15 | PR002328 | 357 | 423 | 432 |
| 15 | PR002332 | 357 | 423 | 433 |
| 20 | PR001999 | 419 | 410 | None |
| 20 | PR002000 | 419 | 412 | None |
| 20 | PR002001 | 419 | 413 | None |
| 20 | PR002002 | 419 | 414 | None |
| 23 | PR002867 | 440 | 428 | None |
| 23 | PR002868 | 441 | 428 | None |
| 23 | PR002869 | 442 | 428 | None |
| 23 | PR002870 | 443 | 428 | None |

**Table 7-21. Sequence numbers of CDRs of antigen-binding domains of BCMA×CD3 bispecific or multispecific antibody molecules**

| Structure No. | Antibody No. | Antigen-binding domain No. | LCDR1 | LCDR2 | LCDR3 | HCDR1 | HCDR2 | HCDR3 |
|---|---|---|---|---|---|---|---|---|
| 11 | PR001987 | #1 | 172 | 192 | 216 | 20 | 68 | 128 |
| 11 | PR001987 | #2 | None | None | None | 15 | 75 | 133 |
| 11 | PR001988 | #1 | 172 | 192 | 216 | 20 | 68 | 128 |
| 11 | PR001988 | #2 | None | None | None | 24 | 76 | 134 |
| 11 | PR001989 | #1 | 172 | 192 | 216 | 20 | 68 | 128 |
| 11 | PR001989 | #2 | None | None | None | 25 | 77 | 135 |
| 11 | PR001990 | #1 | 172 | 192 | 216 | 20 | 68 | 128 |
| 11 | PR001990 | #2 | None | None | None | 26 | 78 | 136 |
| 11 | PR002929 | #1 | 172 | 192 | 216 | 30 | 68 | 128 |
| 11 | PR002929 | #2 | None | None | None | 26 | 78 | 136 |
| 11 | PR002935 | #1 | 172 | 192 | 216 | 30 | 68 | 128 |
| 11 | PR002935 | #2 | None | None | None | 26 | 78 | 136 |
| 11 | PR003867 | #1 | 172 | 192 | 216 | 30 | 68 | 128 |
| 11 | PR003867 | #2 | None | None | None | 26 | 90 | 136 |
| 11 | PR003868 | #1 | 172 | 192 | 216 | 30 | 68 | 128 |
| 11 | PR003868 | #2 | None | None | None | 36 | 90 | 146 |
| 11 | PR003869 | #1 | 172 | 192 | 216 | 30 | 68 | 128 |
| 11 | PR003869 | #2 | None | None | None | 35 | 76 | 147 |

(continued)

| Structure No. | Antibody No. | Antigen-binding domain No. | LCDR1 | LCDR2 | LCDR3 | HCDR1 | HCDR2 | HCDR3 |
|---|---|---|---|---|---|---|---|---|
| 11 | PR003870 | #1 | 172 | 192 | 216 | 30 | 68 | 128 |
| | | #2 | None | None | None | 34 | 78 | 148 |
| 11 | PR003871 | #1 | 172 | 192 | 216 | 30 | 68 | 128 |
| | | #2 | None | None | None | 35 | 76 | 136 |
| 11 | PR003872 | #1 | 172 | 192 | 216 | 30 | 68 | 128 |
| | | #2 | None | None | None | 35 | 90 | 146 |
| 11 | PR003873 | #1 | 172 | 192 | 216 | 30 | 68 | 128 |
| | | #2 | None | None | None | 34 | 76 | 136 |
| 11 | PR003874 | #1 | 172 | 192 | 216 | 30 | 68 | 128 |
| | | #2 | None | None | None | 35 | 76 | 146 |
| 11 | PR003875 | #1 | 172 | 192 | 216 | 30 | 68 | 128 |
| | | #2 | None | None | None | 34 | 76 | 146 |
| 11 | PR003876 | #1 | 172 | 192 | 216 | 30 | 68 | 128 |
| | | #2 | None | None | None | 35 | 90 | 148 |
| 11 | PR003877 | #1 | 172 | 192 | 216 | 30 | 68 | 128 |
| | | #2 | None | None | None | 35 | 90 | 136 |
| 11 | PR003878 | #1 | 172 | 192 | 216 | 30 | 68 | 128 |
| | | #2 | None | None | None | 35 | 90 | 136 |
| 11 | PR003879 | #1 | 172 | 192 | 216 | 30 | 68 | 128 |
| | | #2 | None | None | None | 34 | 76 | 146 |
| 11 | PR003880 | #1 | 172 | 192 | 216 | 30 | 68 | 128 |
| | | #2 | None | None | None | 34 | 78 | 146 |
| 11 | PR003881 | #1 | 172 | 192 | 216 | 30 | 68 | 128 |
| | | #2 | None | None | None | 35 | 78 | 147 |
| 11 | PR003882 | #1 | 172 | 192 | 216 | 30 | 68 | 128 |
| | | #2 | None | None | None | 35 | 90 | 147 |
| 12 | PR001991 | #1 | 172 | 192 | 216 | 20 | 68 | 128 |
| | | #2 | None | None | None | 15 | 75 | 133 |
| 12 | PR001992 | #1 | 172 | 192 | 216 | 20 | 68 | 128 |
| | | #2 | None | None | None | 24 | 76 | 134 |
| 12 | PR001993 | #1 | 172 | 192 | 216 | 20 | 68 | 128 |
| | | #2 | None | None | None | 25 | 77 | 135 |
| 12 | PR001994 | #1 | 172 | 192 | 216 | 20 | 68 | 128 |
| | | #2 | None | None | None | 26 | 78 | 136 |
| 13 | PR001995 | #1 | 172 | 192 | 216 | 20 | 68 | 128 |
| | | #2 | None | None | None | 15 | 75 | 133 |

(continued)

| Structure No. | Antibody No. | Antigen-binding domain No. | LCDR1 | LCDR2 | LCDR3 | HCDR1 | HCDR2 | HCDR3 |
|---|---|---|---|---|---|---|---|---|
| 13 | PR001996 | #1 | 172 | 192 | 216 | 20 | 68 | 128 |
| 13 | PR001996 | #2 | None | None | None | 24 | 76 | 134 |
| 13 | PR001997 | #1 | 172 | 192 | 216 | 20 | 68 | 128 |
| 13 | PR001997 | #2 | None | None | None | 25 | 77 | 135 |
| 13 | PR001998 | #1 | 172 | 192 | 216 | 20 | 68 | 128 |
| 13 | PR001998 | #2 | None | None | None | 26 | 78 | 136 |
| 14 | PR002299, PR002300, PR002301 | #1 | 172 | 192 | 216 | 30 | 68 | 128 |
| 14 | PR002299, PR002300, PR002301 | #2 | None | None | None | 15 | 75 | 133 |
| 14 | PR002308, PR002309, PR002310, PR002892, PR002895, PR002898, PR002901 | #1 | 172 | 192 | 216 | 30 | 68 | 128 |
| 14 | PR002308, PR002309, PR002310, PR002892, PR002895, PR002898, PR002901 | #2 | None | None | None | 26 | 78 | 136 |
| 14 | PR002950, PR002953, PR002956, PR002959 | #1 | 172 | 192 | 216 | 30 | 68 | 128 |
| 14 | PR002950, PR002953, PR002956, PR002959 | #2 | None | None | None | 26 | 78 | 136 |
| 14 | PR003177, PR003178, PR003690 | #1 | 172 | 192 | 216 | 30 | 68 | 128 |
| 14 | PR003177, PR003178, PR003690 | #2 | None | None | None | 26 | 78 | 136 |
| 15 | PR002313 | #1 | 172 | 192 | 216 | 30 | 68 | 128 |
| 15 | PR002313 | #2 | None | None | None | 15 | 75 | 133 |
| 15 | PR002313 | #3 | None | None | None | 24 | 76 | 134 |
| 15 | PR002326, PR002328, PR002332 | #1 | 172 | 192 | 216 | 30 | 68 | 128 |
| 15 | PR002326, PR002328, PR002332 | #2 | None | None | None | 25 | 77 | 135 |
| 15 | PR002326, PR002328, PR002332 | #3 | None | None | None | 26 | 78 | 136 |
| 20 | PR001999 | #1 | 172 | 192 | 216 | 20 | 68 | 128 |
| 20 | PR001999 | #2 | None | None | None | 15 | 75 | 133 |
| 20 | PR002000 | #1 | 172 | 192 | 216 | 20 | 68 | 128 |
| 20 | PR002000 | #2 | None | None | None | 24 | 76 | 134 |
| 20 | PR002001 | #1 | 172 | 192 | 216 | 20 | 68 | 128 |
| 20 | PR002001 | #2 | None | None | None | 25 | 77 | 135 |
| 20 | PR002002 | #1 | 172 | 192 | 216 | 20 | 68 | 128 |
| 20 | PR002002 | #2 | None | None | None | 26 | 78 | 136 |
| 23 | PR002867 | #1 | 177 | 191 | 221 | 15 | 84 | 141 |
| 23 | PR002867 | #2 | None | None | None | 26 | 78 | 136 |
| 23 | PR002868 | #1 | 178 | 197 | 222 | 31 | 85 | 142 |
| 23 | PR002868 | #2 | None | None | None | 26 | 78 | 136 |

(continued)

| Structure No. | Antibody No. | Antigen-binding domain No. | LCDR1 | LCDR2 | LCDR3 | HCDR1 | HCDR2 | HCDR3 |
|---|---|---|---|---|---|---|---|---|
| 23 | PR002869 | #1 | 177 | 191 | 223 | 31 | 86 | 141 |
| | | #2 | None | None | None | 26 | 78 | 136 |
| 23 | PR002870 | #1 | 179 | 198 | 224 | 31 | 85 | 142 |
| | | #2 | None | None | None | 26 | 78 | 136 |

## Example 7.9. Binding of bispecific antibody molecules to BCMA

This example is intended to investigate the binding ability of the BCMA×CD3 bispecific antibodies to BCMA.

The binding ability of the bispecific antibody molecules to cells such as an HEK293T cell line HEK293T/hBCMA (KYinno, KC-0233) highly expressing human BCMA, an HEK293T cell line HEK293T/cyno BCMA (KYinno, KC-0979) highly expressing cynomolgus monkey BCMA, and a cell line NCI-H929 (ATCC, CRL-9068) highly expressing human BCMA was tested according to the method described in Example 7.3 by flow cytometry FACS.

In this example, the anti-BCMA monoclonal antibody PR000274 was used as a positive control 1 molecule. The BCMA×CD3 bispecific antibody molecule PR002199 constructed using the sequences in the patent WO2019133761A1 was used as the positive control 2 molecule. The BCMA×CD3 bispecific antibody molecule PR003106 constructed using the sequences in the patent WO2017134134A1 was used as the positive control 3 molecule.

## Example 7.9.1. Binding to HEK293T/hBCMA cells highly expressing human BCMA

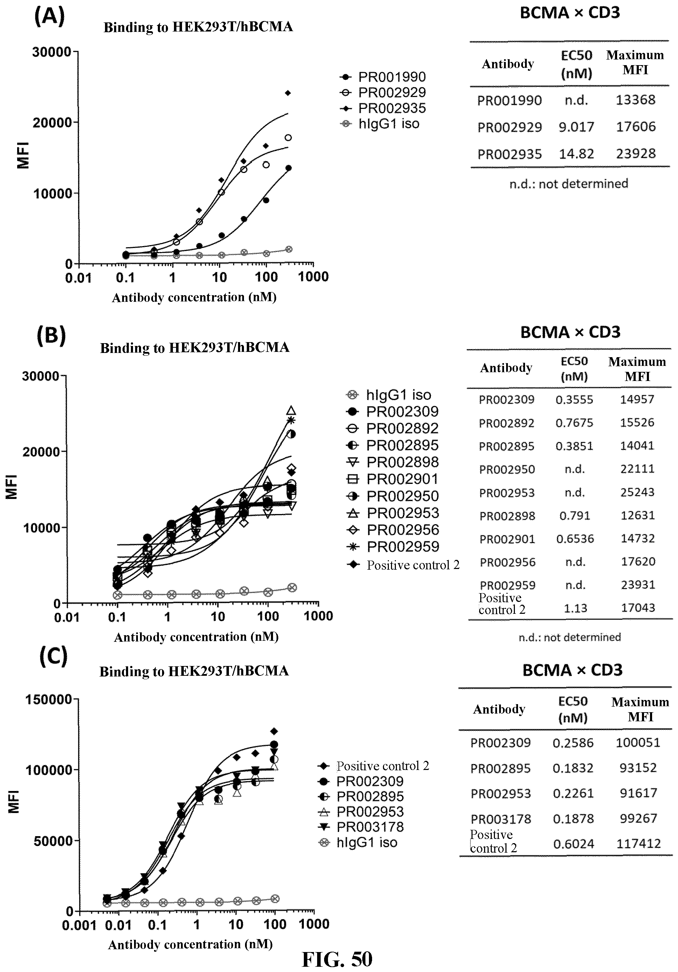

As shown in FIG. 50 and Table 7-22, the bispecific antibody molecules were both able to bind to human BCMA, and the bispecific antibody molecule of Fab-Fc-VH(2) asymmetric structures containing two BCMA-binding domains formed in tandem had stronger binding ability to BCMA than the bispecific antibody molecule of Fab-Fc-VH asymmetric structures containing only one BCMA-binding domain.

### Table 7-22. Binding to HEK293T/hBCMA

| Antibody | $EC_{50}$ (nM) | Maximum MFI | Antibody | $EC_{50}$ (nM) | Maximum MFI | Antibody | $EC_{50}$ (nM) | Maximum MFI |
|---|---|---|---|---|---|---|---|---|
| PR001990 | n.d. | 13368 | PR002309 | 0.3555 | 14957 | PR002309 | 0.2586 | 100051 |
| PR002929 | 9.017 | 17606 | PR002892 | 0.7675 | 15526 | PR002895 | 0.1832 | 93152 |
| PR002935 | 14.82 | 23928 | PR002895 | 0.3851 | 14041 | PR002953 | 0.2261 | 91617 |
| | | | PR002950 | n.d. | 22111 | PR003178 | 0.1878 | 99267 |
| | | | PR002953 | n.d. | 25243 | Positive control 2 | 0.6024 | 117412 |
| | | | PR002898 | 0.791 | 12631 | | | |
| | | | PR002901 | 0.6536 | 14732 | | | |
| | | | PR002956 | n.d. | 17620 | | | |
| | | | PR002959 | n.d. | 23931 | | | |
| | | | Positive control 2 | 1.13 | 17043 | | | |
| Experiment 1 | | | Experiment 2 | | | Experiment 3 | | |

## Example 7.9.2. Binding to NCI-H929 tumor cells highly expressing human BCMA

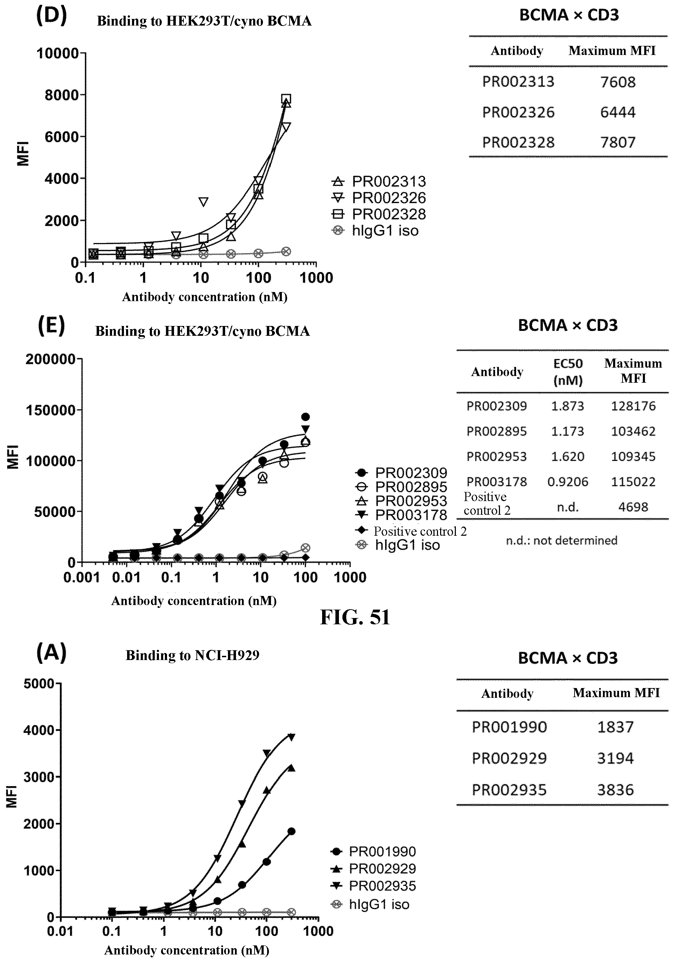

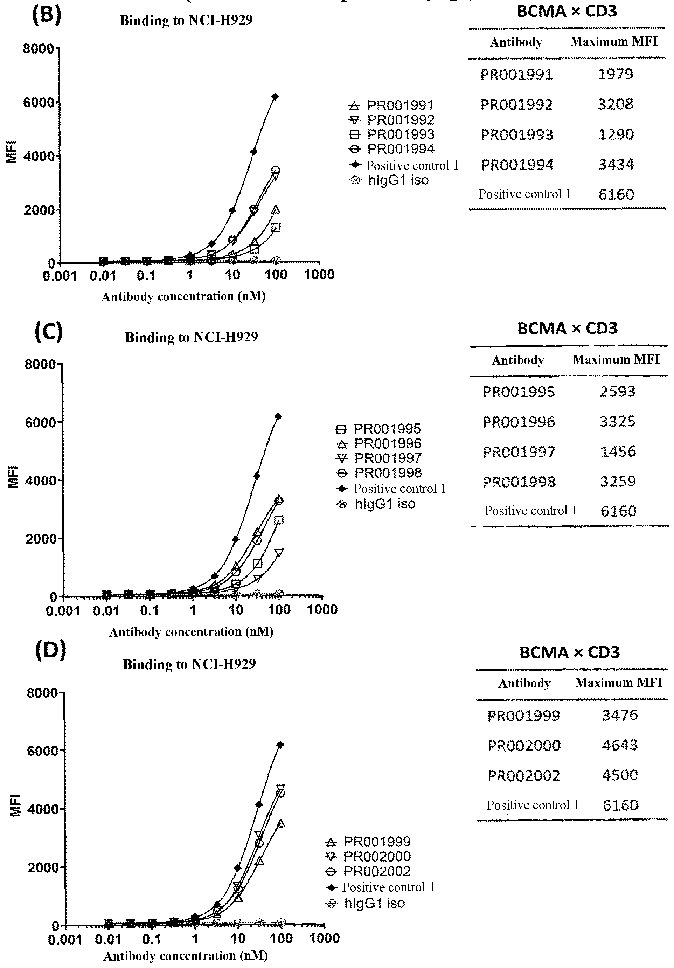

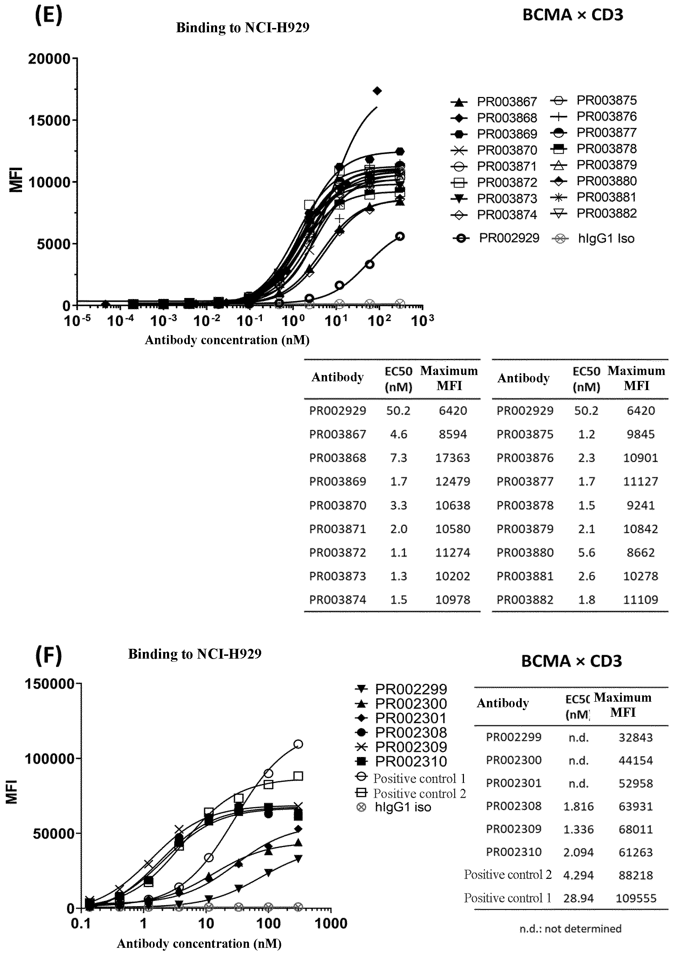

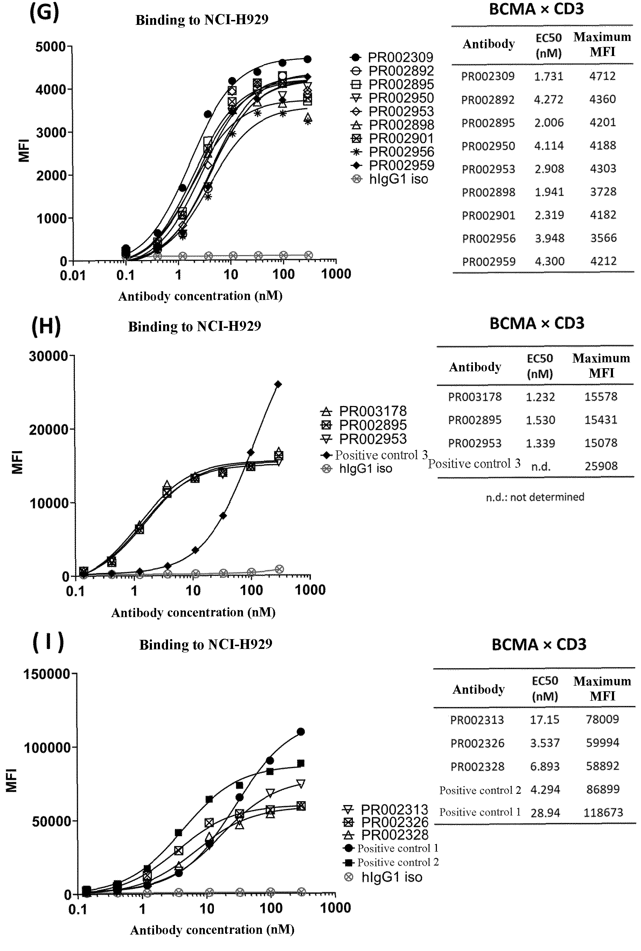

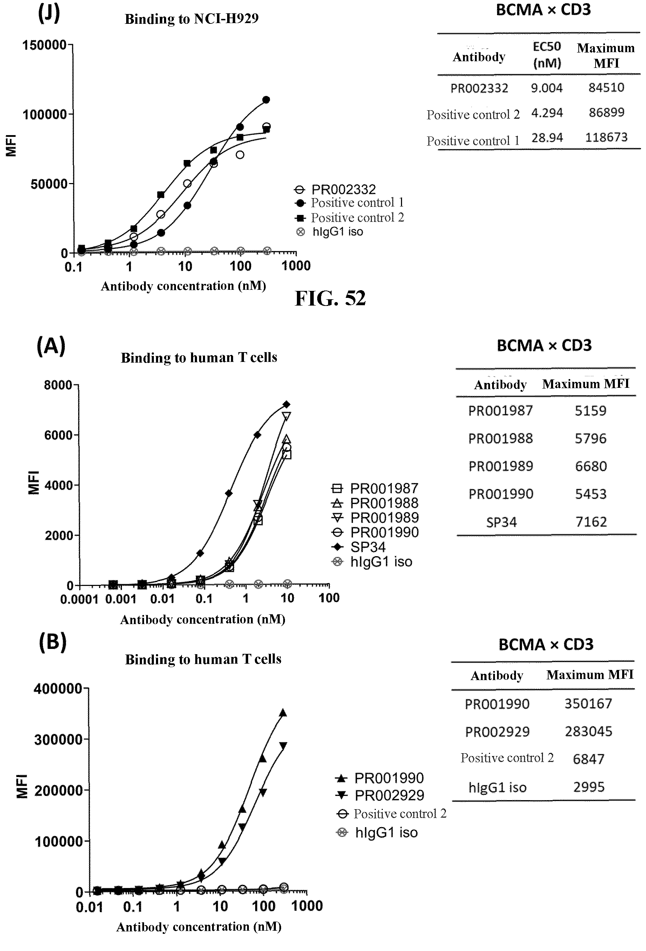

As shown in FIG. 52 and Table 7-23, the bispecific antibody molecules were all able to bind to human BCMA. Overall, the bispecific antibody molecules with only one BCMA-binding domain (e.g., in the Fab-Fc-VH structure and the scFv-Fc-VH structure) had weaker binding ability to BCMA than the molecules with a bivalent BCMA-binding domain (e.g., IgG monoclonal antibody, or bispecific antibody molecules of Fab-Fc-VH(2) asymmetric structures). As shown in FIG. 52 (E), a series of bispecific antibody molecules of Fab-Fc-VH structures (PR003867, ..., PR003882) were constructed based on PR001046-derived HCAb variants with improved affinity, and had stronger binding ability to BCMA than PR002929 (with a Fab-Fc-VH structure constructed with PR001046 HCAb). The bispecific antibody molecules of Fab-Fc-VH(2) asymmetric structures had superior binding ability to BCMA to positive controls 1 and 3.

**Table 7-23. Binding to NCI-H929**

| Antibody | $EC_{50}$ (nM) | Maximum MFI | Antibody | $EC_{50}$ (nM) | Maximum MFI | Antibody | $EC_{50}$ (nM) | Maximum MFI |
|---|---|---|---|---|---|---|---|---|
| PR001990 | n.d. | 1837 | PR001991 | n.d. | 1979 | PR002929 | 50.2 | 6420 |
| PR002929 | n.d. | 3194 | PR001992 | n.d. | 3208 | PR003867 | 4.6 | 8594 |
| PR002935 | n.d. | 3836 | PR001993 | n.d. | 1290 | PR003868 | 7.3 | 17363 |
| | | | PR001994 | n.d. | 3434 | PR003869 | 1.7 | 12479 |
| | | | PR001995 | n.d. | 2593 | PR003870 | 3.3 | 10638 |
| | | | PR001996 | n.d. | 3325 | PR003871 | 2 | 10580 |
| | | | PR001997 | n.d. | 1456 | PR003872 | 1.1 | 11274 |
| | | | PR001998 | n.d. | 3259 | PR003873 | 1.3 | 10202 |
| | | | PR001999 | n.d. | 3476 | PR003874 | 1.5 | 10978 |
| | | | PR002000 | n.d. | 4643 | PR003875 | 1.2 | 9845 |
| | | | PR002002 | n.d. | 4500 | PR003876 | 2.3 | 10901 |
| | | | Positive control 1 | n.d. | 6160 | PR003877 | 1.7 | 11127 |
| | | | | | | PR003878 | 1.5 | 9241 |
| | | | | | | PR003879 | 2.1 | 10842 |
| | | | | | | PR003880 | 5.6 | 8662 |
| | | | | | | PR003881 | 2.6 | 10278 |
| | | | | | | PR003882 | 1.8 | 11109 |
| Antibody | $EC_{50}$ (nM) | Maximum MFI | Antibody | $EC_{50}$ (nM) | Maximum MFI | Antibody | $EC_{50}$ (nM) | Maximum MFI |
| PR002299 | n.d. | 32843 | PR002309 | 1.731 | 4712 | PR003178 | 1.232 | 15578 |
| PR002300 | n.d. | 44154 | PR002892 | 4.272 | 4360 | PR002895 | 1.53 | 15431 |
| PR002301 | n.d. | 52958 | PR002895 | 2.006 | 4201 | PR002953 | 1.339 | 15078 |
| PR002308 | 1.816 | 63931 | PR002950 | 4.114 | 4188 | Positive control 3 | n.d. | 25908 |
| PR002309 | 1.336 | 68011 | PR002953 | 2.908 | 4303 | | | |
| PR002310 | 2.094 | 61263 | PR002898 | 1.941 | 3728 | | | |
| PR002313 | 17.15 | 78009 | PR002901 | 2.319 | 4182 | | | |
| PR002326 | 3.537 | 59994 | PR002956 | 3.948 | 3566 | | | |
| PR002328 | 6.893 | 58892 | PR002959 | 4.3 | 4212 | | | |
| PR002332 | 9.004 | 84510 | | | | | | |
| Positive control 2 | 4.294 | 88218 | | | | | | |
| Positive control 1 | 28.94 | 109555 | | | | | | |

**Example 7.9.3. Binding to HEK293T/cyno BCMA cells highly expressing cynomolgus monkey BCMA**

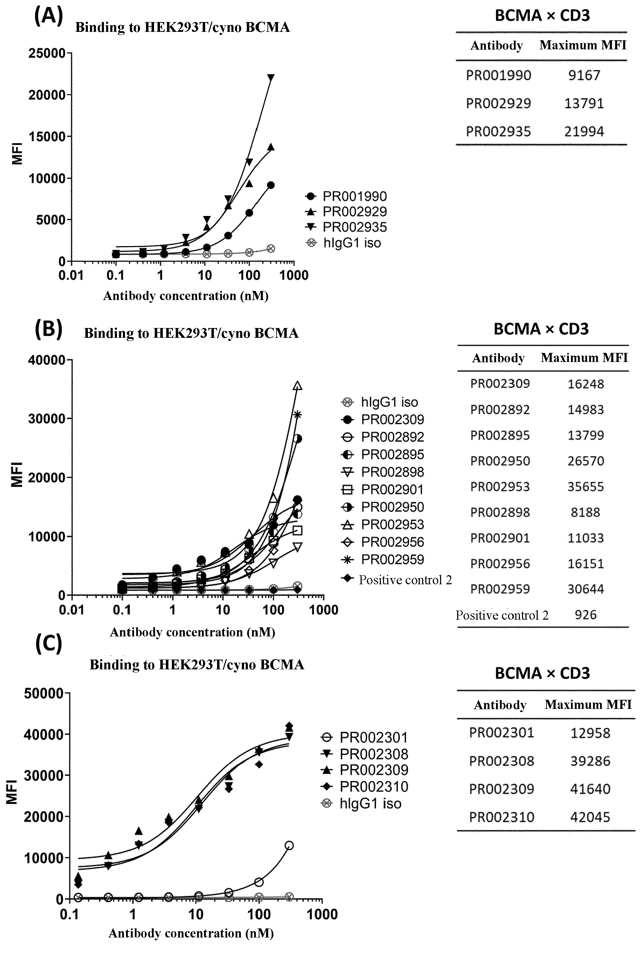

As shown in FIG. 51 and Table 7-24, the bispecific antibody molecules were all able to bind to cynomolgus monkey BCMA, whereas the positive control 2 bispecific antibody molecule was unable to bind to cynomolgus monkey BCMA.Moreover, the bispecific antibody molecules of Fab-Fc-VH(2) structures containing two VH domains of PR001046 in tandem had stronger binding ability to cynomolgus monkey BCMA.

**Table 7-24. Binding to HEK293T/cyno BCMA**

| Antibody | Maximum MFI | Antibody | Maximum MFI | Antibody | Maximum MFI |
|---|---|---|---|---|---|
| PR001990 | 9167 | PR002309 | 16248 | PR002301 | 12958 |
| PR002929 | 13791 | PR002892 | 14983 | PR002308 | 39286 |
| PR002935 | 21994 | PR002895 | 13799 | PR002309 | 41640 |
| | | PR002950 | 26570 | PR002310 | 42045 |
| | | PR002953 | 35655 | | |
| | | PR002898 | 8188 | | |
| | | PR002901 | 11033 | | |
| | | PR002956 | 16151 | | |
| | | PR002959 | 30644 | | |
| | | Positive control 2 | 926 | | |
| **Antibody** | **Maximum MFI** | **Antibody** | **Maximum MFI** | | |
| PR002313 | 7608 | PR002309 | 128176 | | |
| PR002326 | 6444 | PR002895 | 103462 | | |
| PR002328 | 7807 | PR002953 | 109345 | | |
| | | PR003178 | 115022 | | |
| | | Positive control 2 | 4698 | | |

**Example 7.10. Binding of bispecific antibody molecules to T cells**

This example is intended to investigate the binding ability of the BCMA×CD3 bispecific antibodies to T cells.

The binding ability of the antibody molecules to human or cynomolgus monkey T cells was tested by flow cytometry FACS. Specifically, T cells were isolated from human or cynomolgus monkey PBMC cells using a T cell isolation kit (Meltenyi, #130-096-535). The T cell density was adjusted to $1\times10^6$ cells/mL. The cells were seeded in a 96-well plate (Corning, #3894) at 100 μL/well, followed by the addition of test antibodies diluted in a 3-fold gradient at a concentration that was 2 times the final concentration, each at 100 μL/well. The cells were incubated at 4 °C for 1 h away from light. Then, the cells in each well were rinsed twice with 100 μL of pre-cooled PBS, and centrifuged at 500 g at 4 °C for 5 min, and then the supernatant was discarded. Thereafter, a fluorescent secondary antibody (Alexa Fluor 488-conjugated AffiniPure Goat Anti-Human IgG, Fcγ Fragment Specific, Jackson, #109-545-06, diluted in a 1:500 ratio) was added at 100 μL/well, and the plate was incubated at 4 °C for 30 min away from light. The cells in each well were rinsed twice with 100 μL of pre-cooled PBS, and centrifuged at 500 g at 4 °C for 5 min, and then the supernatant was discarded. Finally, the cells in each well were resuspended in 200 μL of pre-cooled PBS. Fluorescence signal values were read using a BD FACS CANTOII flow cytometer or an ACEA NovoCyte flow cytometer, and the data were processed and analyzed using FlowJo v10 (FlowJo, LLC) software. The data were processed and analyzed by plotting using GraphPad Prism 8 software, and binding curves of the antibodies to target cells, $EC_{50}$ values and other parameters were obtained through four-parameter nonlinear fitting.

In this example, the anti-CD3 monoclonal antibody SP34 (antibody No. PR000260) was used as a positive control; and the positive control 2 bispecific antibody molecule (antibody No. PR002199) was used as another control molecule.

### Example 7.10.1. Binding to human T cells

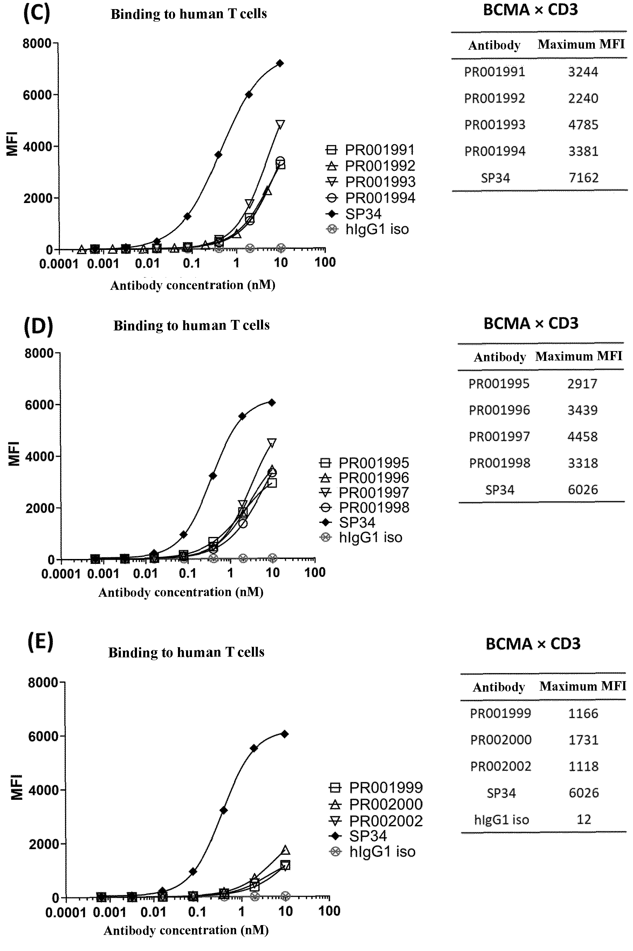

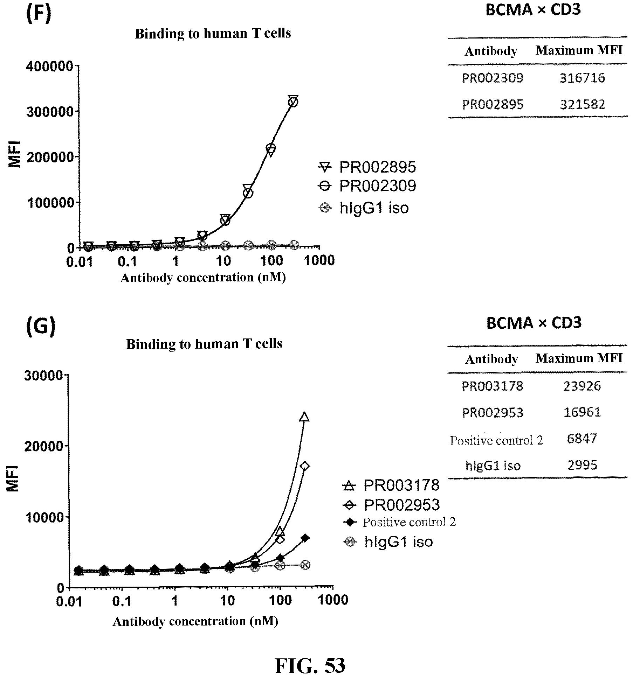

As shown in FIG. 53 and Table 7-25, the bispecific antibody molecules were all able to bind to human T cells. The positive control molecule SP34 was of a bivalent IgG structure, and had very strong binding ability to T cells. The positive control 2 bispecific antibody molecule had a very weak binding ability to T cells. The CD3 end of the bispecific antibody molecule was of a monovalent Fab structure, and the Fab sequence of which was a variant sequence derived from the anti-CD3 monoclonal antibody SP34. The binding ability of those variant sequences to T cells was reduced to different degrees through sequence design and mutation, so that the T cell activation ability of the CD3 end was reduced, and the side effects caused by excessive release of cytokines were reduced. The CD3 ends of different bispecific antibody molecules have different structures or variant sequences, and also differ in their binding ability to T cells. The different requirements for balancing activity and safety in the clinic lead to different requirements for the binding ability of T cells.

#### Table 7-25. Binding to human T cells

| Antibody | Maximum MFI | Antibody | Maximum MFI | Antibody | Maximum MFI |
|---|---|---|---|---|---|
| PR001987 | 5159 | PR001995 | 2917 | PR001990 | 350167 |
| PR001988 | 5796 | PR001996 | 3439 | PR002929 | 283045 |
| PR001989 | 6680 | PR001997 | 4458 | PR002309 | 316716 |
| PR001990 | 5453 | PR001998 | 3318 | PR002895 | 321582 |
| PR001991 | 3244 | PR001999 | 1166 | PR003178 | 23926 |
| PR001992 | 2240 | PR002000 | 1731 | PR002953 | 16961 |
| PR001993 | 4785 | PR002002 | 1118 | Positive control 2 | 6847 |
| PR001994 | 3381 | SP34 | 6026 | hIgG1 iso | 2995 |
| SP34 | 7162 | hIgG1 iso | 12 | | |
| **Experiment 1** | | **Experiment 2** | | **Experiment 3** | |

### Example 7.10.2. Binding to cynomolgus monkey T cells

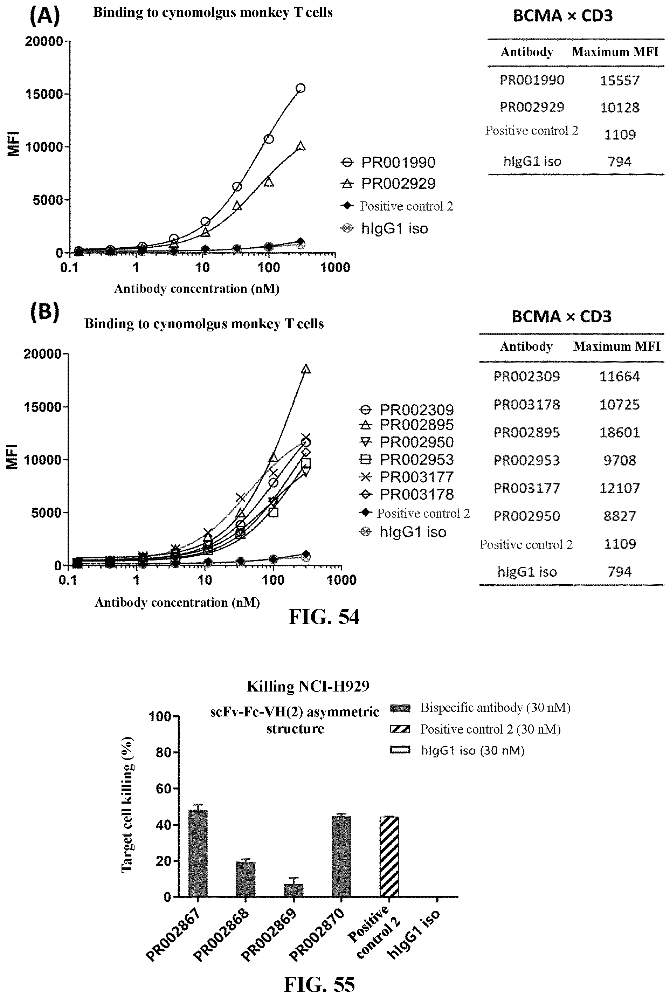

As shown in FIG. 54 and Table 7-26, the bispecific antibody molecules of this example were able to bind to cynomolgus monkey T cells because their Fab sequences at CD3 ends were variant sequences derived from the anti-CD3 monoclonal antibody SP34, whereas the positive control 2 bispecific antibody molecule was unable to bind to cynomolgus monkey T cells.

#### Table 7-26. Binding to cynomolgus monkey T cells

| Antibody | Maximum MFI |
|---|---|
| PR001990 | 15557 |
| PR002929 | 10128 |
| PR002309 | 11664 |
| PR003178 | 10725 |
| PR002895 | 18601 |
| PR002953 | 9708 |
| PR003177 | 12107 |
| PR002950 | 8827 |
| Positive control 2 | 1109 |
| hIgG1 iso | 794 |

### Example 7.11. Binding affinity of bispecific antibody molecules for BCMA determined by a BLI method

In this example, the binding kinetics between the BCMA×CD3 bispecific antibody molecule and the BCMA antigenic protein were analyzed by the method described in Example 7.5 using an Octet Red96e molecular interaction analyzer (ForteBio, Octet Red96e).

The bispecific antibody molecules were all able to bind to human and cynomolgus monkey BCMAs (Table 7-27 and Table 7-28), whereas the positive control 2 bispecific antibody molecule was unable to bind to human and cynomolgus monkey BCMAs.Moreover, the ratio of the binding affinity (KD) values of the bispecific antibody molecules of this example to human BCMA (ACRO Biosystems, #BC7-H82F0) and to cynomolgus monkey BCMA (ACRO Biosystems, #BCA-C82F4) (Table 7-29) was within 5-fold (0.5-5), indicating that there is a good correlation.

**Table 7-27. Binding affinity of BCMA×CD3 bispecific antibody molecules for human BCMA protein**

| Antibody | Antigen | KD (M) | kon(1/Ms) | kdis(1/s) | Full R^2 |
|---|---|---|---|---|---|
| Positive control 1 | huBCMA.Fc | 6.55E-10 | 2.06E+05 | 1.35E-04 | 0.9994 |
| PR001046 | huBCMA.Fc | 7.75E-11 | 4.59E+05 | 3.56E-05 | 0.9952 |
| PR001990 | huBCMA.Fc | 1.95E-09 | 3.22E+05 | 6.29E-04 | 0.995 |
| Positive control 2 | huBCMA.Fc | 4.52E-11 | 5.45E+05 | 2.46E-05 | 0.9969 |
| PR002309 | huBCMA.Fc | 4.48E-11 | 4.22E+05 | 1.89E-05 | 0.9974 |
| PR002895 | huBCMA.Fc | 1.97E-10 | 4.12E+05 | 8.11E-05 | 0.9974 |
| PR002929 | huBCMA.Fc | 5.95E-09 | 2.49E+05 | 1.48E-03 | 0.9933 |
| PR002953 | huBCMA.Fc | 2.25E-10 | 4.74E+05 | 1.07E-04 | 0.998 |
| PR003178 | huBCMA.Fc | 2.36E-10 | 4.94E+05 | 1.17E-04 | 0.9978 |

**Table 7-28. Binding affinity of BCMA×CD3 bispecific antibody molecules for cynomolgus monkey BCMA protein**

| Antibody | Antigen | KD (M) | kon(1/Ms) | kdis(1/s) | Full R^2 |
|---|---|---|---|---|---|
| Positive control 1 | cyBCMA.Fc | 3.88E-11 | 2.00E+05 | 7.76E-06 | 0.9456 |
| PR001046 | cyBCMA.Fc | 1.19E-10 | 6.33E+05 | 7.54E-05 | 0.9965 |
| PR001990 | cyBCMA.Fc | 3.35E-09 | 7.46E+05 | 2.50E-03 | 0.9689 |
| Positive control 2 | cyBCMA.Fc | \ | | | |
| PR002309 | cyBCMA.Fc | 1.41E-10 | 8.23E+05 | 1.16E-04 | 0.9973 |
| PR002895 | cyBCMA.Fc | 2.72E-10 | 8.90E+05 | 2.42E-04 | 0.9983 |
| PR002929 | cyBCMA.Fc | 6.62E-09 | 8.79E+05 | 5.82E-03 | 0.9437 |
| PR002953 | cyBCMA.Fc | 1.95E-10 | 3.65E+05 | 7.12E-05 | 0.9989 |
| PR003178 | cyBCMA.Fc | 3.19E-10 | 8.22E+05 | 2.62E-04 | 0.9981 |

**Table 7-29. Ratio of binding affinity values for BCMA**

| Antibody | KD ratio (cy BCMA/hu BCMA) |
|---|---|
| Positive control 1 | 0.06 |
| PR001046 | 1.54 |
| PR001990 | 1.72 |
| Positive control 2 | N/A |
| PR002309 | 3.16 |
| PR002895 | 1.38 |
| PR002929 | 1.11 |
| PR002953 | 0.87 |
| PR003178 | 1.35 |

**Example 7.12. Killing assay on target cells by bispecific antibody molecules**

In this example, T cell activation ability and specific target cell killing ability mediated by BCMA×CD3 bispecific antibody molecules were investigated.

**Example 7.12.1. *In vitro* killing assay on NCI-H929 cells highly expressing BCMA and release of cytokines**

In this example, the ability of bispecific antibody molecules to activate T cells, release cytokines and kill tumor cells in the presence of NCI-H929 tumor cells (ATCC, CRL-9068) highly expressing BCMA was investigated. The effector cells may be human peripheral blood mononuclear cells PBMCs or T cells isolated from PBMCs.

Specifically, the density of effector cells (PBMCs or T cells) was adjusted to $1.1\times10^6$ cells/mL and the density of NCI-H929 was adjusted to $0.11\times10^6$ cells/mL in a culture medium (RPMI1640 medium containing 5% FBS). The two cell suspensions were each seeded into a 96-well plate (Corning, #3799) at 90 μL cells/well. Then, the test antibodies at different concentrations (gradient dilution to a concentration that was 10 times the final concentration) were added at 20 μL/well, wherein the antibody concentration may be the final concentration of 30 nM, or a total of 3 concentrations obtained by a 20-fold gradient dilution from the highest final concentration of 1 nM, or a total of 10 concentrations obtained by a 4-fold gradient dilution from the highest final concentration of 30 nM. hIgG1 iso (CrownBio, #C0001) was used as an isotype control. The final effector-to-target ratio was 10:1, and two duplicate wells were set for each concentration. The well to which the test antibody was added was called ER (experimental well, containing the test antibody sample and the effector and target cells); meanwhile, different control groups were set in the plate according to the parameter composition in the following formula: ESR (natural release well for effector cell, containing only effector cell and culture medium); TSR (natural release well for target cell, containing only target cell and culture medium); CMB (medium reference well, containing only medium); TMR (maximum release well for target cell, containing only target cell and culture medium); and VCC (volume reference well, containing only medium). The 96 well plate was incubated in a carbon dioxide incubator at 37 °C for 24 h. Then, 10 μL of lysis buffer was added into the TMR and VCC wells, and the plate was incubated for 30 min. After incubation was completed, the supernatant was added into a 96-well plate (Corning, #3599) at 50 μL/well, followed by the addition of a cytotoxicity assay reagent (CytoTox 96® nonradioactive cytotoxicity assay kit, Promega, #G1780) at 50 μL/well. After incubation for 30 min at room temperature, 50 μL of reaction stop buffer was added to stop the reaction. Finally, the absorbance value was read at 490 nM using an Enspire™ multifunctional microplate reader (Perkin Elmer, Inc.), and the target cell killing rate was calculated according to the following calculation formula:

$$\text{killing rate} = ((\text{ER} - \text{CMB}) - (\text{ESR} - \text{CMB}) - (\text{TSR} - \text{CMB}))/(\text{TMR} - \text{VCC}) \times 100\%$$

wherein:

ER = experimental well, sample + effector cells + target cells
ESR = natural release well for effector cell, effector cell + culture medium
TSR = natural release well for target cell, target cell + culture medium
TMR = maximum release well for target cell, target cell + medium + lysis buffer
VCC = volume reference well, medium + lysis buffer
CMB = medium reference well, medium

In addition to cytotoxicity, cytokine release levels in the cell supernatant can also be determined. Briefly, 50 μL of supernatant was removed for determination of cytokine levels. The concentration of TNF-α in the supernatant was determined using a TNF-α ELISA kit (Thermo, #88-7346-88); the concentration of IL-6 in the supernatant was determined using an IL-6 ELISA kit (Thermo, #88-7066-88); the concentration of IL-2 in the supernatant was determined using an IL-2 ELISA kit (Thermo, #88-7025-88); the concentration of IFN-γ in the supernatant was determined using an IFN-γ ELISA kit (Thermo, #88-7316-88); and the concentration of IL-10 in the supernatant was determined using an IL-10 ELISA kit (Thermo, #88-7106-88). The ELISA assay was performed by referring to the instructions of relevant kit. The data was processed and analyzed by plotting using GraphPad Prism 8 software.

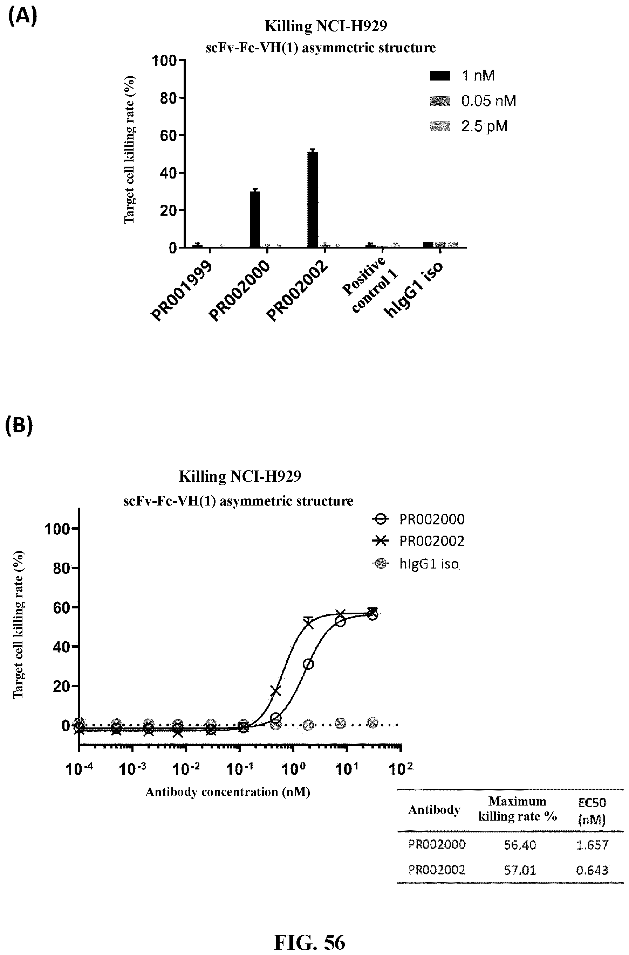

As shown in FIG. 56, the bispecific antibody molecules of scFv-Fc-VH(1) asymmetric structures (PR002000 and PR002002) were able to effectively kill target cells. The bispecific antibody molecule PR001999 constructed based on the anti-BCMA HCAb monoclonal antibody PR000940 had no killing function on target cells. Those molecules were structurally similar and contained identical sequence of the CD3-binding domain (scFv), with the difference being that the sequences of the BCMA-binding domain (VH) were different. The differences in the binding affinity or epitope of different VHs to the BCMA antigen result in differences in the killing activity of the bispecific antibody molecules against target cells. The anti-BCMA IgG monoclonal antibody positive control 1 molecule also had no killing function.

As shown in FIG. 55, the bispecific antibody molecules of scFv-Fc-VH(2) asymmetric structures (PR002867, PR002868, PR002869, and PR002870) were able to kill target cells to different degrees. Those molecules were structurally similar and contained two identical BCMA-binding domains (VHs) in tandem, with the only difference being that the sequence of the CD3-binding domain (scFv) was derived from different anti-CD3 monoclonal antibodies. PR002867 and PR002870 had similar target cell killing ability to the positive control 2 bispecific antibody molecule, whereas PR002868 and PR002869 had weaker target cell killing ability.

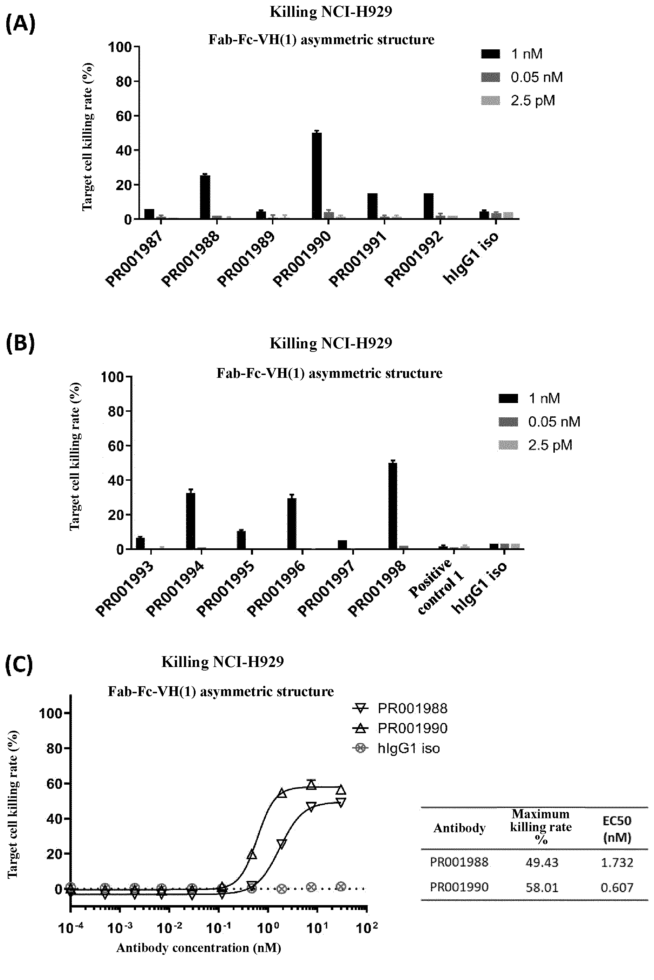

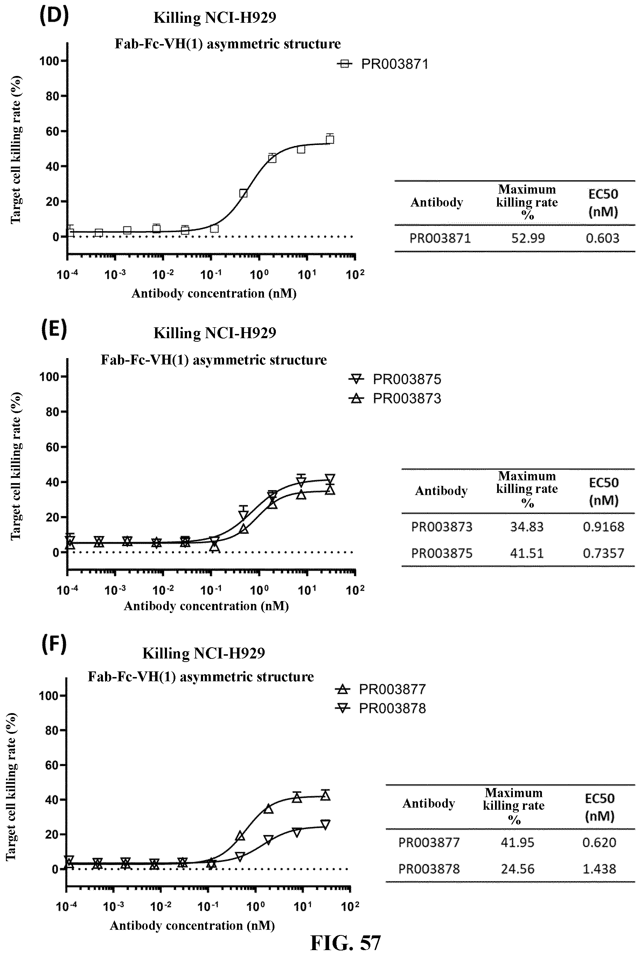

As shown in FIG. 57 (A-B), 12 bispecific antibody molecules of Fab-Fc-VH(1) asymmetric structures (PR001987, ..., PR001998) had similar molecular structures but different sequences of the BCMA-binding domain and CD3-binding domain; and thus had different target cell killing ability. Among them, PR001988, PR001990, PR001994, PR001996 and PR001998 had stronger target cell killing ability. As shown in FIG. 57 (C), PR001990 had stronger target cell killing ability than PR001988. As shown in FIG. 57 (D-F), the bispecific antibody molecules of Fab-Fc-VH structures constructed based on PR001046-derived HCAb variants with improved affinity had target cell killing ability.

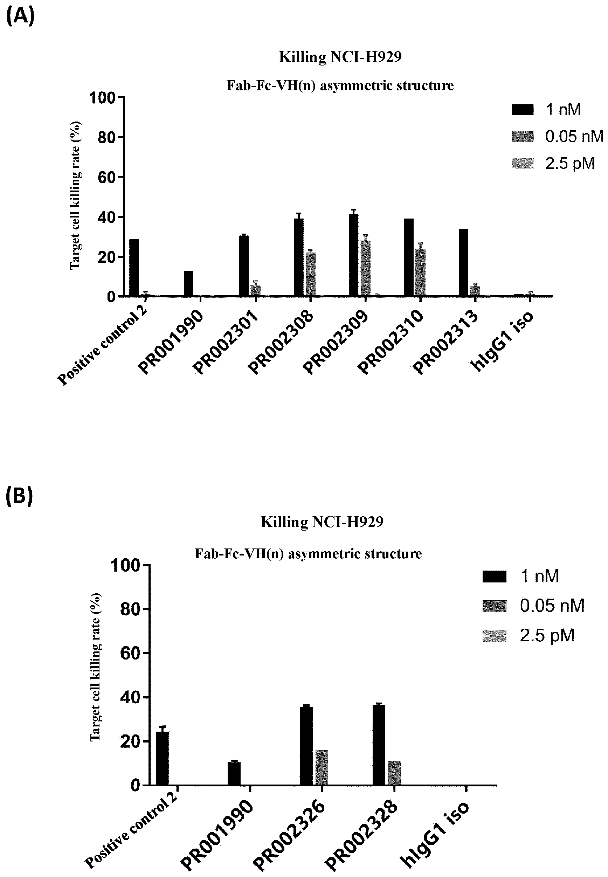

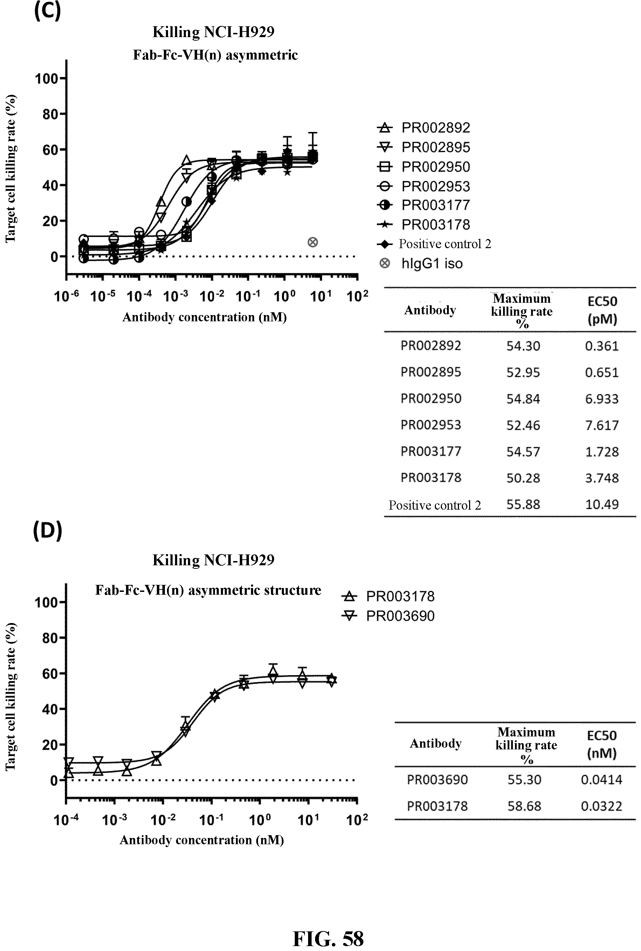

As shown in FIG. 58, the bispecific antibody molecules of Fab-Fc-VH(2) asymmetric structures were able to effectively kill target cells. Those molecules all contained two BCMA-binding domains (VHs) in tandem. They had significantly superior target cell killing ability to PR001990 of a Fab-Fc-VH(1) structure and the positive control 2 bispecific antibody molecule. As shown in FIG. 58 (A-B), the bispecific antibody molecules PR002301, PR002308, PR002309, PR002310, PR002313, PR002326 and PR002328 had identical sequences of CD3-binding domain. Among them, the BCMA-binding domains of PR002301, PR002308, PR002309 and PR002310 were formed by two identical VH sequences in tandem; the BCMA-binding domains of PR002313, PR002326 and PR002328 were formed by two different VH sequences in tandem; in particular, the two different VHs used by the BCMA-binding domains of PR002326 and PR002328 may bind to different epitopes of BCMA. As shown in FIG. 58 (C), the BCMA-binding domains of the bispecific antibody molecules PR002892, PR002895, PR002950, PR002953, PR003177 and PR003178 were formed by two identical VH sequences derived from PR001046 connected in tandem via linker peptide sequences of different lengths. However, those bispecific antibody molecules had different sequences of the CD3-binding domains and different binding ability to T cells, and thus exhibited different target cell killing efficiency ($EC_{50}$). As shown in FIG. 58 (D), the bispecific antibody molecule PR003690 had consistent target cell killing ability with PR003178, because the target-binding domain of PR003690 was not changed, although extra amino acid mutations were introduced into the Fc region of PR003690 to further improve the serum half-life on the basis of PR003178.

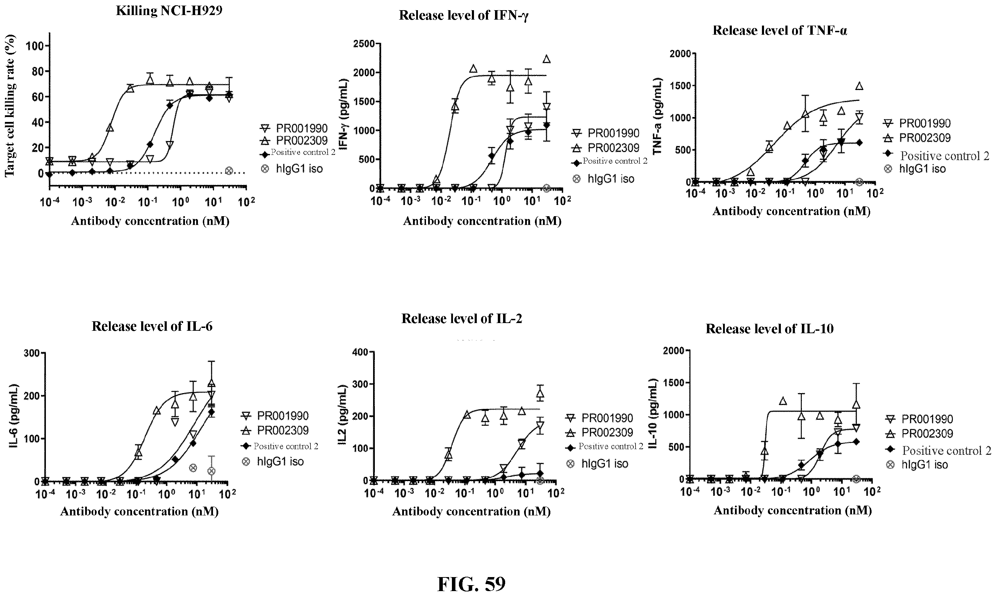

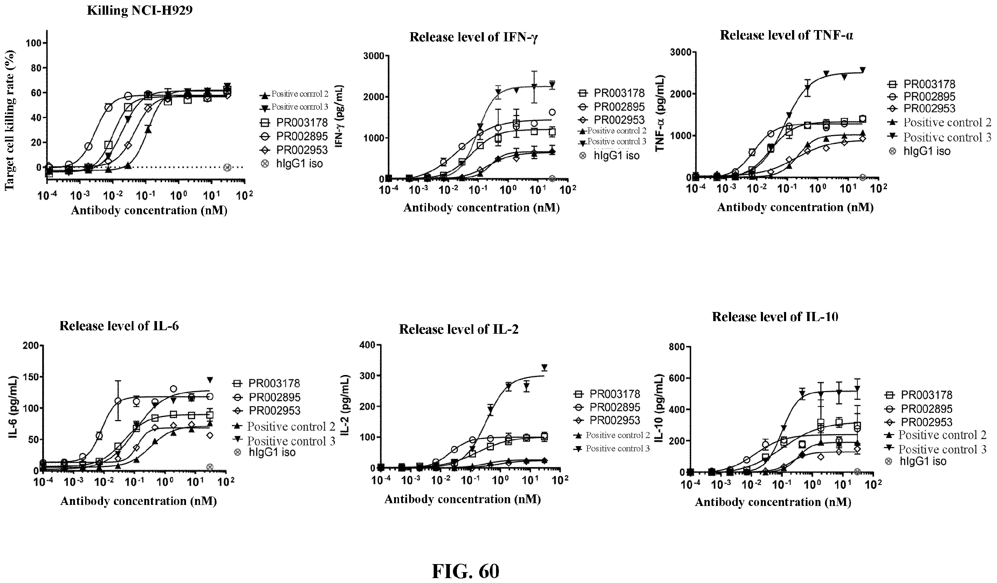

As shown in FIG. 59 and FIG. 60, the target cell killing ability and the release levels of various cytokines of the bispecific antibody molecules PR001990 (Fab-Fc-VH(1) structure, strong CD3 binding), PR002309 (Fab-Fc-VH(2) structure, strong CD3 binding), PR002895 (Fab-Fc-VH(2) structure, strong CD3 binding), PR002953 (Fab-Fc-VH(2) structure, weak CD3 binding) and PR003178 (Fab-Fc-VH(2) structure, moderate CD3 binding) as well as the positive control 2 and positive control 3 were investigated more systematically (Table 7-30 and Table 7-31). Those bispecific antibody molecules were able to achieve similar maximum killing rates, but with different killing efficiency $EC_{50}$ and different release levels of cytokines induced. PR002309, PR002895, PR002953 and PR003178 had superior target cell killing $EC_{50}$ to the positive control 2; moreover, PR002895, PR002953 and PR003178 caused lower release levels of various cytokines than the positive control 3. In particular, PR003178 had superior target cell killing ability ($EC_{50}$ was 10-fold higher) to the positive control 2; PR003178 had comparable killing ability to the positive control 3, but a lower maximum cytokine release than the positive control 3. This indicates that PR003178 can control the release level of cytokines while maintaining effective tumor killing efficiency to reduce the risk of cytokine release syndrome, potentially better balancing efficacy and safety in the clinic and leading to a better therapeutic window.

**Table 7-30. *In vitro* killing of NCI-H929 cells by effector cells and release of cytokines mediated by BCMA×CD3 bispecific antibody molecules**

| | Antibody | PR001990 | PR002309 | Positive control 2 |
|---|---|---|---|---|
| NCI-H929 killing | Maximum killing rate % | 61.38 | 69.52 | 61.2 |
| | $EC_{50}$ (nM) | 0.5901 | 0.007717 | 0.1603 |
| IFN-$\gamma$ | Maximum (pg/ml) | 1232 | 1950 | 1017 |
| | $EC_{50}$ (nM) | 1.358 | 0.01945 | 0.483 |
| TNF-$\alpha$ | Maximum (pg/mL) | 1198 | 1298 | 608.1 |
| | $EC_{50}$ (nM) | 5.486 | 0.0445 | 0.4638 |
| IL-2 | Maximum (pg/mL) | 185.4 | 222 | 21.51 |
| | $EC_{50}$ (nM) | 5.292 | 0.03746 | 2.272 |

(continued)

| | Antibody | PR001990 | PR002309 | Positive control 2 |
|---|---|---|---|---|
| IL-6 | Maximum (pg/mL) | 272.9 | 208.9 | 245.1 |
| | $EC_{50}$ (nM) | 8.204 | 0.1945 | 13.13 |
| IL-10 | Maximum (pg/mL) | 776.8 | 1054 | 582.8 |
| | $EC_{50}$ (nM) | 1.956 | 0.02982 | 0.8611 |

**Table 7-31. *In vitro* killing of NCI-H929 cells by effector cells and release of cytokines mediated by BCMA×CD3 bispecific antibody molecules**

| | Antibody | PR002895 | PR002953 | PR003178 | Positive control 2 | Positive control 3 |
|---|---|---|---|---|---|---|
| NCI-H929 killing | Maximum killing rate % | 57.93 | 57.51 | 56.78 | 62.03 | 61.5 |
| | $EC_{50}$ (nM) | 0.002932 | 0.04699 | 0.01018 | 0.1154 | 0.01919 |
| IFN-$\gamma$ | Maximum (pg/mL) | 1441 | 639.8 | 1207 | 658.4 | 2247 |
| | $EC_{50}$ (nM) | 0.02232 | 0.2124 | 0.0595 | 0.2607 | 0.09596 |
| TNF-$\alpha$ | Maximum (pg/mL) | 1282 | 894.2 | 1330 | 1021 | 2506 |
| | $EC_{50}$ (nM) | 0.008184 | 0.1965 | 0.02814 | 0.2243 | 0.08062 |
| IL-2 | Maximum (pg/mL) | 100.1 | 23.57 | 101.1 | 25.79 | 300.2 |
| | $EC_{50}$ (nM) | 0.02112 | 0.7954 | 0.1748 | 0.3118 | 0.3374 |
| IL-6 | Maximum (pg/mL) | 118.1 | 68.45 | 89.66 | 71.43 | 128.4 |
| | $EC_{50}$ (nM) | 0.008391 | 0.1278 | 0.04478 | 0.3411 | 0.1101 |
| IL-10 | Maximum (pg/mL) | 239.6 | 129.2 | 321.5 | 190.5 | 516.9 |
| | $EC_{50}$ (nM) | 0.01341 | 0.2081 | 0.1044 | 0.335 | 0.1007 |

**Example 7.12.2. Influence of soluble APRIL and soluble BAFF on the target cell killing effect of bispecific antibody molecules**

In this example, the influence of BCMA ligands (APRIL and BAFF) on the target cell killing effect of bispecific antibody molecules was further investigated on the basis of the method described in Example 7.12.1, wherein the concentrations of APRIL and BAFF were about 100 ng/mL in the plasma of multiple myeloma patients (Blood 2004; 103: 3148-3157).Specifically, in the method described in Example 7.12.1, when the test antibodies at different concentrations were added, each of the BCMA ligand proteins were also added at 20 $\mu$L/well, so that the final concentrations of the soluble ligand proteins were 100 ng/mL and 10 ng/mL, respectively. The soluble APRIL protein (sAPRIL) was a recombinant human APRIL protein (Novoprotein, #CU89); and the soluble BAFF protein (sBAFF) was a recombinant human BAFF protein (ACRO Biosystems, #BAF-H5248). The effector cells were human PBMCs or T cells and the target cells were NCI-H929. Target cell killing rates were calculated by the method described in Example 7.12.1.

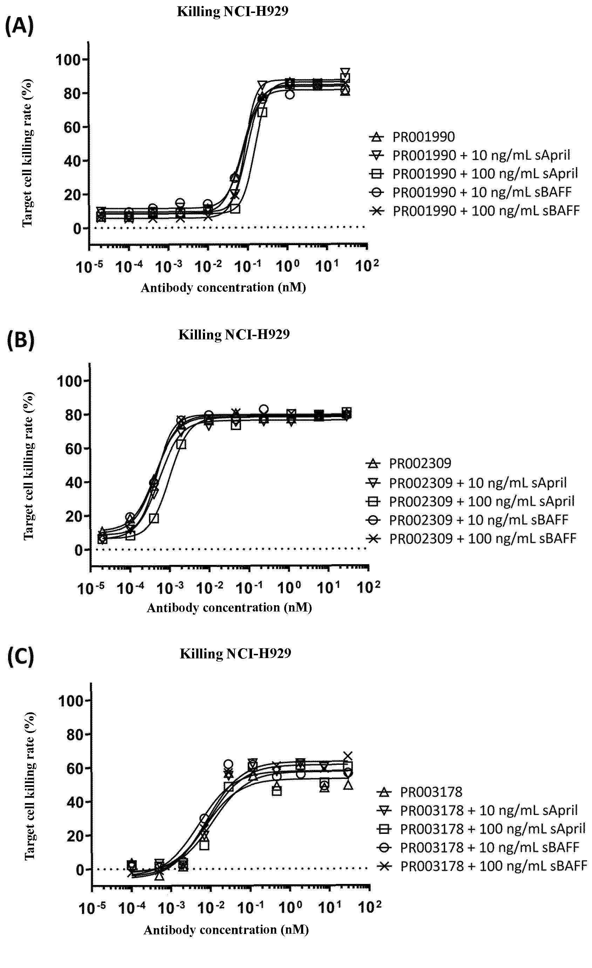

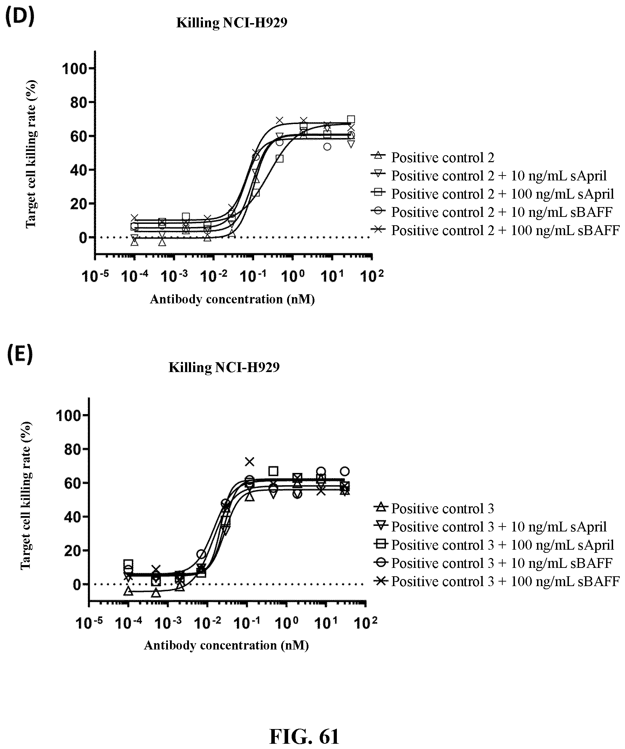

As shown in FIG. 61 and Table 7-32, soluble APRIL and BAFF have little influence on the target cell killing ability of the bispecific antibody molecules.

**Table 7-32. Killing of NCI-H929 by BCMA×CD3 bispecific antibody molecules in the presence of soluble APRILs or BAFFs at various concentrations**

| T cell + NCI-H929 | PR001990 | PR001990 + 10 ng/mL sBAFF | PR001990 + 100 ng/mL sBAFF | PR001990 + 10 ng/mL sAPRIL | PR001990 + 100 ng/mL sAPRIL |
|---|---|---|---|---|---|
| Maximum killing rate % | 83.57 | 81.46 | 84.47 | 87.3 | 86.13 |
| $EC_{50}$ (nM) | 0.07327 | 0.07866 | 0.09395 | 0.08791 | 0.1545 |

(continued)

| T cell + NCI-H929 | PR002309 | PR002309 + 10 ng/mL sBAFF | PR002309 + 100 ng/mL sBAFF | PR002309 + 10 ng/mL sAPRIL | PR002309 + 100 ng/mL sAPRIL |
|---|---|---|---|---|---|
| Maximum killing rate % | 78.49 | 79.37 | 79.63 | 76.24 | 78.13 |
| $EC_{50}$ (nM) | 0.0004388 | 0.0004473 | 0.0004741 | 0.0005381 | 0.0009908 |
| **PBMC + NCI-H929** | **PR003178** | **PR003178 + 10 ng/mL sBAFF** | **PR003178 + 100 ng/mL sBAFF** | **PR003178 + 10 ng/mL sAPRIL** | **PR003178 + 100 ng/mL sAPRIL** |
| Maximum killing rate % | 53.3 | 58 | 63.5 | 61.6 | 57.6 |
| $EC_{50}$ (nM) | 0.007 | 0.005 | 0.008 | 0.009 | 0.011 |
| **PBMC + NCI-H929** | **Positive control 2** | **Positive control 2 + 10 ng/mL sBAFF** | **Positive control 2 + 100 ng/mL sBAFF** | **Positive control 2 + 10 ng/mL sAPRIL** | **Positive control 2 + 100 ng/mL sAPRIL** |
| Maximum killing rate % | 60.5 | 58.3 | 67.7 | 61 | 67.2 |
| $EC_{50}$ (nM) | 0.103 | 0.063 | 0.076 | 0.096 | 0.253 |
| **PBMC + NCI-H929** | **Positive control 3** | **Positive control 3 + 10 ng/mL sBAFF** | **Positive control 3 + 100 ng/mL sBAFF** | **Positive control 3 + 10 ng/mL sAPRIL** | **Positive control 3 + 100 ng/mL sAPRIL** |
| Maximum killing rate % | 58.2 | 61.4 | 61.7 | 55.9 | 62.3 |
| $EC_{50}$ (nM) | 0.014 | 0.015 | 0.022 | 0.027 | 0.026 |

**Example 7.12.3. Influence of soluble BCMA on the target cell killing effect of bispecific antibody molecules**

BCMA is a transmembrane protein, but its extracellular region can be shed from the cell membrane to form soluble BCMA (sBCMA). The level of sBCMA is observed to increase to different degrees in the plasma of multiple myeloma patients, with the median concentration of 176.0 ng/mL (Leuk Res. 2019 Jun; 81: 62-66).

In this example, optimization was performed on the basis of the method described in Example 7.12.1 to investigate the influence of soluble BCMA (sBCMA) on the target cell killing effect of bispecific antibody molecules. Specifically, in the method described in Example 7.12.1, when the test antibodies at different concentrations were added, the recombinant human BCMA protein (ACRO Biosystems, #BCA-H522y) was also added at 20 μL/well to reach final concentrations of 500 ng/mL, 250 ng/mL, 125 ng/mL and 62.5 ng/mL. The effector cells were human PBMCs and the target cells were NCI-H929. Target cell killing rates were calculated by the method described in Example 7.12.1.

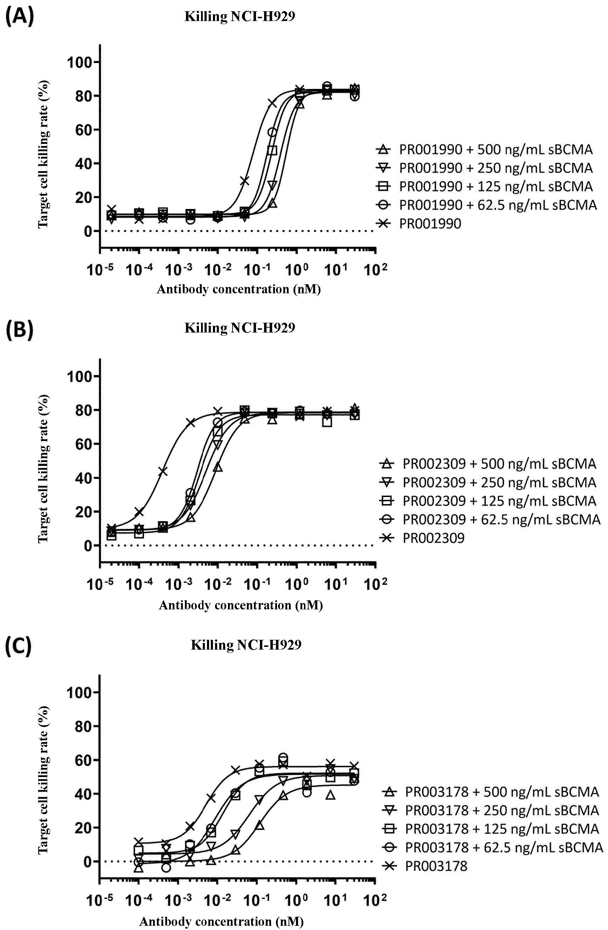

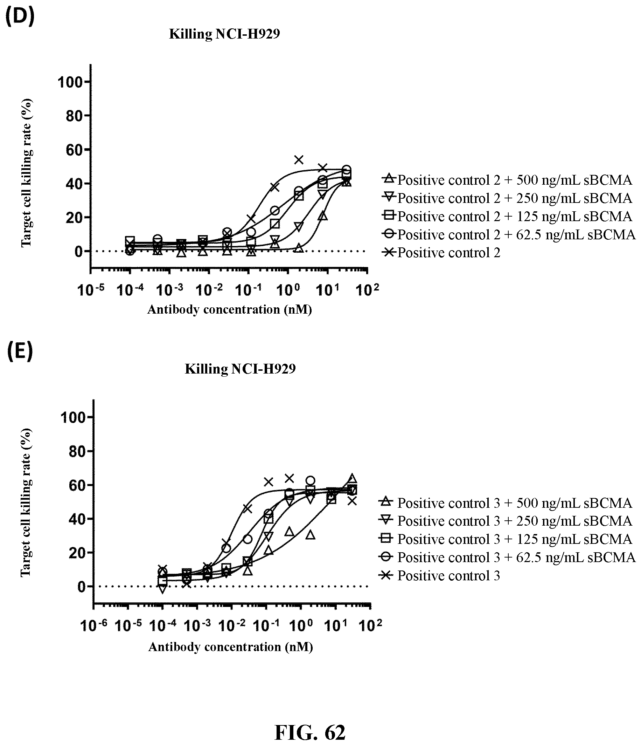

As shown in FIG. 62 and Table 7-33, the soluble BCMA had dose-dependent influence on the target cell killing rates ($EC_{50}$) of the bispecific antibody molecules, but did not affect the maximum killing rate. When the concentration of soluble BCMA was 176.0 ng/ml, the target cell killing rates ($EC_{50}$) of the bispecific antibody molecules PR002309 and PR003178 were superior to the positive control 2 and the positive control 3. In other words, the influence of soluble BCMA on the target cell killing rates of the bispecific antibody molecules PR002309 and PR003178 was less than that on the positive control 2 and positive control 3.

**Table 7-33. Killing of NCI-H929 by BCMA×CD3 bispecific antibody molecules in the presence of soluble BCMA at various concentrations**

| T cell + NCI-H929 | PR001990 | PR001990 + 62.5 ng/mL sBCMA | PR001990 + 125 ng/mL sBCMA | PR001990 + 250 ng/mL sBCMA | PR001990 + 500 ng/mL sBCMA |
|---|---|---|---|---|---|
| Maximum killing rate % | 83.77 | 82.11 | 83.23 | 82.17 | 82.71 |
| $EC_{50}$ (nM) | 0.07867 | 0.1763 | 0.2341 | 0.387 | 0.5458 |

(continued)

| T cell + NCI-H929 | PR002309 | PR002309 + 62.5 ng/mL sBCMA | PR002309 + 125 ng/mL sBCMA | PR002309 + 250 ng/mL sBCMA | PR002309 + 500 ng/mL sBCMA |
|---|---|---|---|---|---|
| Maximum killing rate % | 78.68 | 78.68 | 77.21 | 78.39 | 78.47 |
| $EC_{50}$ (nM) | 0.000393 | 0.002961 | 0.00347 | 0.005002 | 0.008797 |
| **PBMC + NCI-H929** | **PR003178** | **PR003178 + 62.5 ng/mL sBCMA** | **PR003178 + 125 ng/mL sBCMA** | **PR003178 + 250 ng/mL sBCMA** | **PR003178 + 500 ng/mL sBCMA** |
| Maximum killing rate % | 56.1 | 51.7 | 52.2 | 50.9 | 45.2 |
| $EC_{50}$ (nM) | 0.005 | 0.009 | 0.014 | 0.065 | 0.12 |
| **PBMC + NCI-H929** | **Positive control 2** | **Positive control 2 + 62.5 ng/mL sBCMA** | **Positive control 2 + 125 ng/mL sBCMA** | **Positive control 2 + 250 ng/mL sBCMA** | **Positive control 2 + 500 ng/mL sBCMA** |
| Maximum killing rate % | 43.3 | 48.9 | 39.6 | 40.9 | 41.8 |
| $EC_{50}$ (nM) | 0.2 | 0.8 | 1 | 3.5 | 7.7 |
| **PBMC + NCI-H929** | **Positive control 3** | **Positive control 3 + 62.5 ng/mL sBCMA** | **Positive control 3 + 125 ng/mL sBCMA** | **Positive control 3 + 250 ng/mL sBCMA** | **Positive control 3 + 500 ng/mL sBCMA** |
| Maximum killing rate % | 57.3 | 58.6 | 55.5 | 56.8 | 68.3 |
| $EC_{50}$ (nM) | 0.01 | 0.033 | 0.074 | 0.107 | 0.829 |

## Example 7.13. Pharmacokinetic study

In this example, the pharmacokinetic properties of the BCMA×CD3 bispecific antibody molecule of a Fab-Fc-VH asymmetric structure (PR002929) and the BCMA×CD3 bispecific antibody molecule of a Fab-Fc-VH(2) asymmetric structure (PR003178) at a single dose in mice or rats were investigated.

The test was performed as follows: 6 female BALB/c mice or 3 SD rats with appropriate body weight were selected and administered with the fusion protein by intravenous injection at a dose of 5 mg/kg. The whole blood was collected before the administration and 0.25 h, 5 h, 24 h (1 day), 2 days, 4 days, 7 days, 10 days and 14 days after the administration, left to stand for 30 min for coagulation, and centrifuged at 2,000 g at 4 °C for 5 min, and the isolated serum sample was cryopreserved at -80 °C until it was taken for analysis. In this example, the drug concentration in the serum was quantitatively determined by two methods. The method I, namely the Fc end detection ELISA method, for quantitatively determining the drug concentration in the serum was performed by capturing BCMA×CD3 bispecific antibody molecules containing a human Fc end in the serum using a goat anti-human Fc polyclonal antibody coating a 96-well plate, and then adding an HRP-labeled goat anti-human Fc secondary antibody. The method II, namely the BCMA end detection ELISA method, for quantitatively determining the drug concentration in the serum was performed by capturing intact BCMA×CD3 bispecific antibody molecules containing an anti-BCMA VH end in the serum using a recombinant BCMA protein (Human BCMA/TNFRSF17 Protein His Tag, ACRO Biosystems, #BCA-H522y) coating a 96-well plate, and then adding an HRP-labeled goat anti-human Fc secondary antibody. Pharmacokinetic parameters were analyzed using Phoenix WinNonlin software version 8.2 by non-compartmental analysis (NCA).

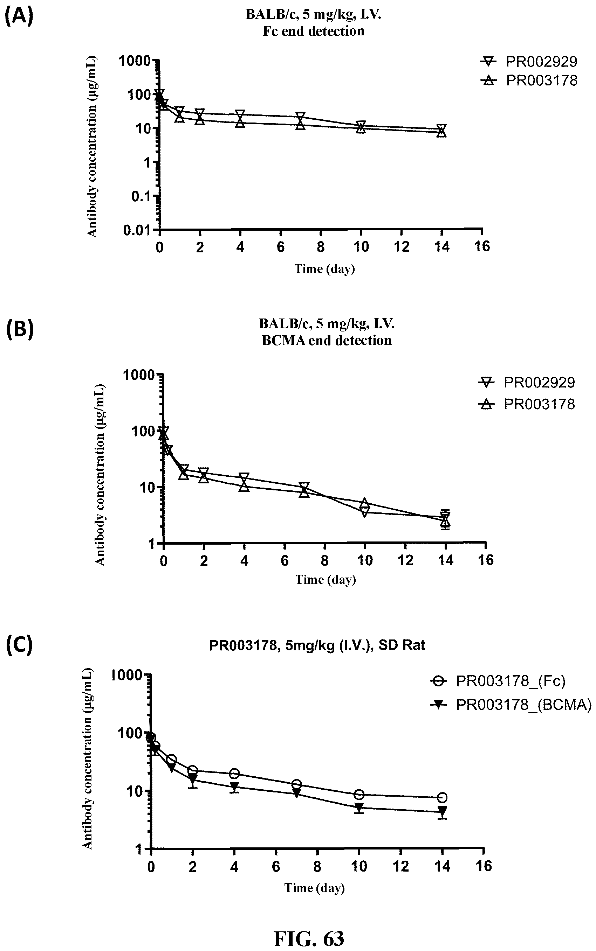

As shown in FIG. 63 (A-B), the bispecific antibody molecules PR002929 and PR003178 had similar pharmacokinetics: under the Fc end detection method, the serum half-life $t_{1/2}$ values of PR002929 and PR003178 in BALB/c mice were about 7 days and 9.6 days, respectively; under the BCMA end detection method, the serum half-life $t_{1/2}$ values of PR002929 and PR003178 in mice were both about 4 days.

As shown in FIG. 63 (C), the serum half-life $t_{1/2}$ values of PR003178 in rats under the Fc end detection method and the BCMA end detection method were about 5.6 days and 4.6 days, respectively.

The pharmacokinetic parameters of BCMA×CD3 bispecific antibody molecules in mice or rats were summarized in Table 7-34.

138

**Table 7-34. Pharmacokinetic parameters of BCMA×CD3 bispecific antibody molecules in mice or rats**

| Bispecific antibody molecules | PR003178 | | PR002929 | | PR003178 | |
|---|---|---|---|---|---|---|
| Animals (number) | BALB/c mice (n = 6) | | BALB/c mice (n = 6) | | SD rats (n = 3) | |
| Antibody dosage | 5 mg/kg, I.V. | | 5 mg/kg, I.V. | | 5 mg/kg, I.V. | |
| PK parameters | Fc end detection | BCMA end detection | Fc end detection | BCMA end detection | Fc end detection | BCMA end detection |
| T1/2 (hr) | 230.5 | 98.0 | 167.5 | 100.8 | 135.1 | 109.8 |
| Vd (mL/kg) | 218.4 | 189.4 | 126.7 | 171.2 | 137.2 | 181.2 |
| AUCall ($\mu$g*hour/mL) | 4,650 | 3,381 | 6,886 | 3,878 | 5,593 | 3,799 |
| Cl (mL/hr/kg) | 0.72 | 1.34 | 0.56 | 1.18 | 0.76 | 1.21 |
| C0 ($\mu$g/mL) | 93.4 | 87.4 | 106.4 | 100.1 | 83.3 | 76.3 |

## Example 7.14. Evaluation of anti-tumor efficacy in NCI-H929/human PBMC mouse model

This example is intended to evaluate the anti-tumor efficacy of BCMA×CD3 bispecific antibodies *in vivo.* Female NCG mice aged 6-8 week were intraperitoneally injected (i.p.) with $3\times10^6$ human PBMCs 3 days before tumor cell inoculation, and then each was subcutaneously inoculated with $5\times10^6$ NCI-H929 cells resuspended in a mixed solution of PBS and Matrigel (1:1) (0.1 mL/mice) on the day of tumor cell inoculation. When the tumor volume reached 90 mm$^3$, the mice were randomized into groups of 6. After grouping, specific concentrations of antibody drugs diluted in PBS were administered intravenously (i.v.) at a specific dose and frequency. In this example, multiple dosing regimens were employed: 1) a 10 $\mu$g/mouse dose group, single dose; and 2) 10 $\mu$g/mouse and 3 $\mu$g/mouse dose groups, 1 dose per week for a total of 2 doses (QW$\times$2). PBS was used as a blank for each experiment. Tumor volume and body weight of mice were measured on days 3, 7, 10 and 14 after the first administration. The tumor volume was calculated as follows: tumor volume (mm$^3$) = 0.5 $\times$ (long diameter of tumor $\times$ short diameter of tumor$^2$).

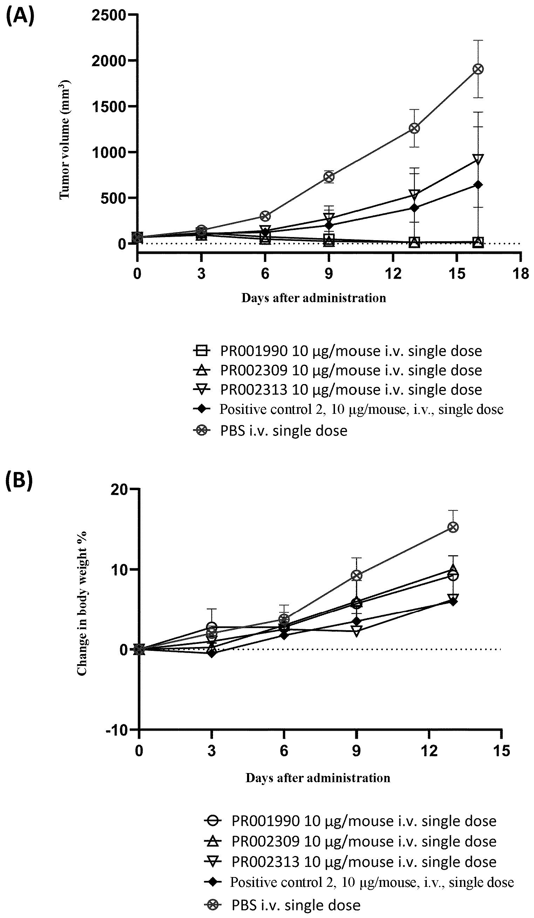

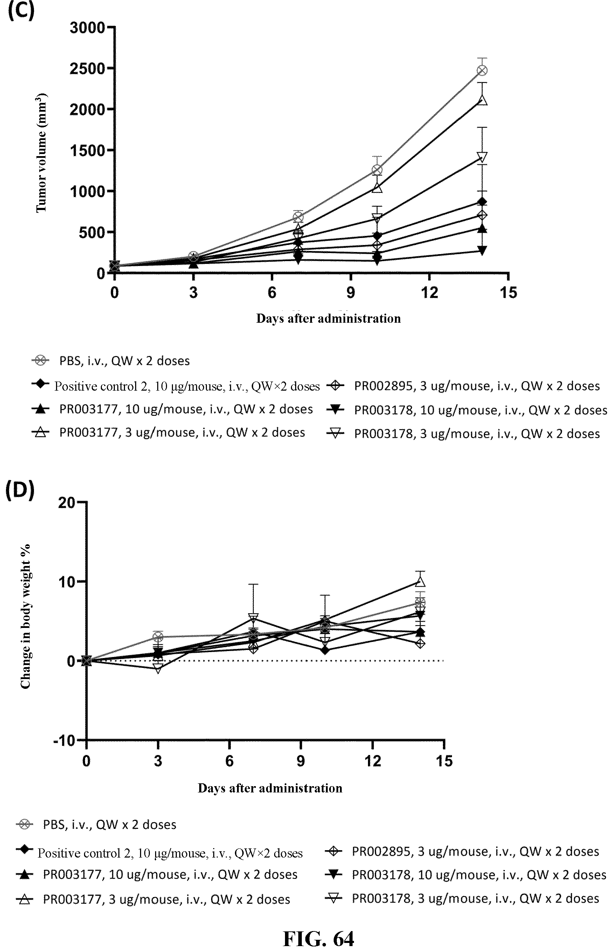

As shown in FIG. 64 (A-B), the bispecific antibody molecules such as PR001990, PR002309, PR002313 and the positive control 2 in the 10 $\mu$g/mouse single-dose groups showed efficacy in inhibiting tumor growth, and the PR001990 and PR002309 had significant superior efficacy to the positive control 2 molecule at an equal dose. The change in body weight of mice in each group was within the normal range.

As shown in FIG. 64 (C-D), all bispecific antibody molecules in different dose groups showed different degrees of efficacy in inhibiting tumor growth. Overall, the efficacy of the bispecific antibody molecules in the 10 $\mu$g/mouse group is superior to that in the 3 $\mu$g/mouse group. Moreover, at the dose of 10 $\mu$g/mouse, the efficacy of PR003177 and PR003178 was superior to that of the positive control 2 molecule QW$\times$2 at an equal dose; and the efficacy of PR002895 in the 3 $\mu$g/mouse dose group was even slightly superior to that of the positive control 2 molecule in the 10 $\mu$g/mouse dose group. The change in body weight of mice in each group was within the normal range.

## Example 7.15. Summary

In this example, BCMA×CD3 bispecific antibody molecules of a variety of asymmetric structures are constructed using the antigen-binding domain Fab (or scFv form) of the anti-CD3 IgG antibody and the antigen-binding domain VH of the anti-BCMA HCAb antibody. This shows the flexibility of constructing a bispecific antibody molecular structure based on HCAb. The bispecific antibody molecules of this example can simultaneously bind to human BCMA and human T cells, and also to cynomolgus monkey BCMA and cynomolgus monkey T cells. Therefore, cynomolgus monkeys can be used for non-primate toxicological risk assessment, which is very important for clinical development. However, the positive control 2 bispecific antibody molecule does not have this property of binding to the cynomolgus monkey target.

In this example, a series of BCMA×CD3 bispecific antibody molecules are constructed. During their constructions, the binding ability to T cells is modified by regulating the sequence and the fine structure of the CD3-binding domain, and the binding ability to target cells expressing BCMA is modified by regulating the sequence and the number of the BCMA-binding domain, so that the T cell activation ability and specific target cell killing ability of the bispecific antibody molecules are further regulated. In an *in vitro* target cell killing assay, the bispecific antibody molecules of Fab-Fc-VH(2) asymmetric structures (PR002309, PR002895, PR002953 and PR003178) have superior target cell killing EC$_{50}$ to the positive control 2; moreover, PR002895, PR002953 and PR003178 caused lower release levels of various cytokines than the positive control 3. In an *in vivo* anti-tumor efficacy mouse model, the efficacy of PR002309 and PR003178 is significantly superior to that of the positive control 2 molecule at an equal dose; and the efficacy of PR002895 in the low dose group is even slightly superior to that of the positive control 2 molecule in the high dose group.

In particular, PR003178 has significantly superior target cell killing ability to the positive control 2 and has comparable target cell killing ability to the positive control 3. However, PR003178 has lower release levels of cytokines than the positive control 3. This indicates that PR003178 can control the release level of cytokines while maintaining effective tumor killing efficiency to reduce the risk of cytokine release syndrome, potentially better balancing efficacy and safety in the clinic and leading to a better therapeutic window.

Some embodiments of the present invention relate to:

1. A binding protein comprising at least two protein functional regions, wherein the binding protein comprises a protein functional region A and a protein functional region B, wherein the protein functional region A and the protein functional region B target different antigens or antigenic epitopes, the protein functional region A is of a Fab or scFv structure, the protein functional region B is of a VH structure, and the number of each of the protein functional region A and the protein functional region B is one or more; preferably, the binding protein is of a symmetric or asymmetric structure, and/or the binding protein further comprises Fc, wherein the number of Fc is two, thereby forming an Fc dimer, preferably as a homodimer or a heterodimer.

2. The binding protein according to item 1, wherein,

(I) the binding protein is of a symmetric structure, and the symmetric structure is a left-right symmetric IgG-like structure, wherein the number of the protein functional region A is two, and the number of the protein functional region B is two or four;

preferably, when the number of the protein functional region B is two, the binding protein is a tetravalent binding protein comprising a structure described as follows: (a) a protein functional region A, a protein functional region B and Fc are sequentially arranged from the N-terminus to the C-terminus of the binding protein, wherein the protein functional region A is linked to the protein functional region B via a first linker peptide (L1), and the protein functional region B is linked to the Fc via a second linker peptide (L2); or, (b) a protein functional region B, a protein functional region A and Fc are sequentially arranged from the N-terminus to the C-terminus of the binding protein, wherein the protein functional region B is linked to the protein functional region A via a linker peptide, and the protein functional region A is linked to the Fc via a hinge region; or, (c) a protein functional region A, Fc and a protein functional region B are sequentially arranged from the N-terminus to the C-terminus of the binding region, wherein the protein functional region A is linked to the Fc via a hinge region, and the Fc is linked to the protein functional region B via a linker peptide;

when the number of the protein functional regions B is four, the binding protein is a hexavalent binding protein comprising a structure described as follows: two additional protein functional regions B are further linked to the N-terminus or C-terminus of the binding protein described in the (a), (b) or (c); preferably, linked to the C-terminus of the Fc or the C-terminus of the original protein functional region B of the binding protein described in the (c), or linked to the N-terminus of the protein functional region A together with the original protein functional region B of the binding protein described in the (b);

more preferably, the binding protein further comprises a protein functional region C, wherein the protein functional region C targets different antigens or antigenic epitopes with the protein functional region A and the protein functional region B; and the binding protein is a hexavalent or octavalent, trispecific binding protein comprising a structure described as follows: the protein functional region C is linked to the N-terminus or C-terminus of the above binding protein; preferably, the number of the protein functional region C is two, and the protein functional region C is linked to the C-terminus of the binding protein described in the (c), or linked to the N-terminus of the protein functional region A as the protein functional region B of the binding protein described in the (b); or,

(II) the binding protein is of an asymmetric structure, and the asymmetric structure is a left-right asymmetric IgG-like structure, wherein a left arm is a protein functional region A of a Fab or scFv structure, a right arm is a protein functional region B of a VH structure, and the protein functional region A and the protein functional region B are each linked to one Fc; preferably, the number of the protein functional region A is one, and the number of the protein functional region B is one or two or three;

preferably, when the number of the protein functional region B is one, the binding protein is a bivalent binding protein comprising a structure described as follows: the protein functional region A is of (d) a conventional Fab structure, or (e) a Fab (cross VH/VL) structure, or (f) a Fab (cross Fd/LC) structure; when the number of the protein functional region B is two, the binding protein is a trivalent binding protein comprising a structure described as follows: the protein functional region A of the left arm is of the (d) or (e) or (f) structure, and a second protein functional region B is linked to the N-terminus or C-terminus of the protein functional region A of the left arm, or the N-terminus of a first protein functional region B of the right arm, or the C-terminus of the Fc, preferably, the second protein functional region B is linked to the N-terminus of the first protein functional region B of the right arm; when the number of the protein functional regions B is three, the binding protein is a tetravalent binding protein comprising a structure as follows: the protein functional region A of the left arm is of the (d) or (e) or (f) structure, and a third protein functional region B is further linked to the N-terminus or C-terminus of the protein functional region A of the left arm, or the N-terminus or C-terminus of a second protein functional region B of the left arm, or the N-terminus of a first or second protein functional region B of the right arm, or the C-terminus of the Fc, preferably the second protein functional region B is linked to the N-terminus of the first protein functional region B of the right arm, and the third protein functional region B is linked to the N-terminus of the second protein functional region B; or,

when the number of the protein functional region B is one, the binding protein is a bivalent binding protein comprising a structure described as follows: the protein functional region A of the left arm is of (g) an scFv structure, wherein the scFv is linked to Fc via the terminus of VH or the terminus of VL; when the number of the protein functional region B is two, the binding protein is a trivalent binding protein comprising a structure described as follows: the protein functional region A of the left arm is of the (g) structure, and a second protein functional region B is linked to the N-terminus of the protein functional region A of the left arm, or the N-terminus of a first protein functional region B of the right arm, or the C-terminus of the Fc, preferably, the second protein functional region B is linked to the N-terminus of the first protein functional region B of the right arm; when the number of the protein functional regions B is three, the binding protein is a tetravalent binding protein comprising a structure described as follows: the protein functional region A of the left arm is of the (g) structure, and a third protein functional region B is further linked to the N-terminus of the protein functional region A of the left arm, or the N-terminus or C-terminus of a second protein functional region B, or the C-terminus of the Fc, preferably, the second protein functional region B is linked to the N-terminus of a first protein functional region B of the right arm, and the third protein functional region B is linked to the N-terminus of the second protein functional region B;

more preferably, the binding protein further comprises a protein functional region C, and the binding protein is a trivalent or multivalent multispecific binding protein, wherein the protein functional region C targets different antigens or antigenic epitopes with the protein functional region A and the protein functional region B, and the protein functional region C is linked to the N-terminus or C-terminus of the above binding protein; preferably, the protein functional region C is linked to the N-terminus of the protein functional region B of the above binding protein described in the (d), (e), (f) or (g); and

even more preferably, the binding protein further comprises a protein functional region D, and the binding protein is a tetravalent or multivalent multispecific binding protein, wherein: the protein functional region D targets different antigens or antigenic epitopes with the protein functional region A, the protein functional region B and the protein functional region C, and the protein functional region D is linked to the N-terminus or C-terminus of the above binding protein; preferably, the protein functional region D is linked to the N-terminus of the protein functional region B of the above binding protein described in the (d), (e), (f) or (g).

3. The binding protein according to item 1 or 2, wherein (A) the binding protein has four polypeptide chains, including two identical short chains and two identical long chains, wherein (1) the short chain comprises VH_A-CH1 sequentially from the N terminus to the C terminus, and the long chain comprises VL_A-CL-L1-VH_B-L2-CH2-CH3 sequentially from the N terminus to the C terminus; or (2) the short chain comprises VL_A-CL sequentially from the N terminus to the C terminus, and the long chain comprises VH_A-CH1-L1-VH_B-L2-CH2-CH3 sequentially from the N terminus to the C terminus; or (3) the short chain comprises VL_A-CL sequentially from the N terminus to the C terminus, and the long chain comprises VH_B-L-VH_A-CH1-h-CH2-CH3 sequentially from the N terminus to the C terminus; or (4) the short chain comprises VH_B-L-VL_A-CL sequentially from the N terminus to the C terminus, and the long chain comprises VH_A-CH1-h-CH2-CH3 sequentially from the N terminus to the C terminus; or (5) the short chain comprises VL_A-CL sequentially from the N terminus to the C terminus, and the long chain comprises VH_A-CH1-h-CH2-CH3-L-VH_B sequentially from the N terminus to the C terminus; or (6) the short chain comprises VL_A-CL-L-VH_B sequentially from the N terminus to the C terminus, and the long chain comprises VH_A-CH1-h-CH2-CH3 sequentially from the N terminus to the C terminus; or (7) the short chain comprises VL_A-CL sequentially from the N

terminus to the C terminus, and the long chain comprises VH_A-CH1-h-CH2-CH3-L1-VH_B-L2-VH_B sequentially from the N terminus to the C terminus; or (8) the short chain comprises VL_A-CL sequentially from the N terminus to the C terminus, and the long chain comprises VH_A-CH1-h-CH2-CH3-L1-VH_B-L2-VH_C sequentially from the N terminus to the C terminus; or (9) the short chain comprises VH_B-L1-VL_A-CL sequentially from the N terminus to the C terminus, and the long chain comprises VH_B-L2-VH_A-CH1-h-CH2-CH3 sequentially from the N terminus to the C terminus; or (10) the short chain comprises VH_B-L1-VL_A-CL sequentially from the N terminus to the C terminus, and the long chain comprises VH_C-L2-VH_A-CH1-h-CH2-CH3 sequentially from the N terminus to the C terminus; wherein the L, L1 and L2 are linker peptides, and the h is a hinge region or linker peptide, such as "-", GS or an amino acid sequence as set forth in any one of SEQ ID NOs: 495-519; or,

(B) the binding protein has three polypeptide chains, including one polypeptide chain 1, one polypeptide chain 2 and one polypeptide chain 3, wherein (11) the polypeptide chain 1 comprises VL_A-CL sequentially from the N-terminus to the C-terminus, the polypeptide chain 2 comprises VH_A-CH1-h-CH2-CH3 sequentially from the N-terminus to the C-terminus, and the polypeptide chain 3 comprises VH_B-h-CH2-CH3 sequentially from the N-terminus to the C-terminus; or (12) the polypeptide chain 1 comprises VH_A-CL sequentially from the N-terminus to the C-terminus, the polypeptide chain 2 comprises VL_A-CH1-h-CH2-CH3 sequentially from the N-terminus to the C-terminus, and the polypeptide chain 3 comprises VH_B-h-CH2-CH3 sequentially from the N-terminus to the C-terminus; or (13) the polypeptide chain 1 comprises VH_A-CH1 sequentially from the N-terminus to the C-terminus, the polypeptide chain 2 comprises VL_A-CL-h-CH2-CH3 sequentially from the N-terminus to the C-terminus, and the polypeptide chain 3 comprises VH_B-h-CH2-CH3 sequentially from the N-terminus to the C-terminus; or (14) the polypeptide chain 1 comprises VL_A-CL sequentially from the N-terminus to the C-terminus, the polypeptide chain 2 comprises VH_A-CH1-h-CH2-CH3 sequentially from the N-terminus to the C-terminus, and the polypeptide chain 3 comprises VH_B-L-VH_B-h-CH2-CH3 sequentially from the N-terminus to the C-terminus; or (15) the polypeptide chain 1 comprises VL_A-CL sequentially from the N-terminus to the C-terminus, the polypeptide chain 2 comprises VH_A-CH1-h-CH2-CH3 sequentially from the N-terminus to the C-terminus, and the polypeptide chain 3 comprises VH_C-L-VH_B-h-CH2-CH3 sequentially from the N-terminus to the C-terminus; or (16) the polypeptide chain 1 comprises VH_A-CL sequentially from the N-terminus to the C-terminus, the polypeptide chain 2 comprises VL_A-CH1-h-CH2-CH3 sequentially from the N-terminus to the C-terminus, and the polypeptide chain 3 comprises VH_B-L-VH_B-h-CH2-CH3 sequentially from the N-terminus to the C-terminus; or (17) the polypeptide chain 1 comprises VH_A-CL sequentially from the N-terminus to the C-terminus, the polypeptide chain 2 comprises VL_A-CH1-h-CH2-CH3 sequentially from the N-terminus to the C-terminus, and the polypeptide chain 3 comprises VH_C-L-VH_B-h-CH2-CH3 sequentially from the N-terminus to the C-terminus; or (18) the polypeptide chain 1 comprises VH_A-CH1 sequentially from the N-terminus to the C-terminus, the polypeptide chain 2 comprises VL_A-CL-h-CH2-CH3 sequentially from the N-terminus to the C-terminus, and the polypeptide chain 3 comprises VH_B-L-VH_B-h-CH2-CH3 sequentially from the N-terminus to the C-terminus; or (19) the polypeptide chain 1 comprises VH_A-CH1 sequentially from the N-terminus to the C-terminus, the polypeptide chain 2 comprises VL_A-CL-h-CH2-CH3 sequentially from the N-terminus to the C-terminus, and the polypeptide chain 3 comprises VH_C-L-VH_B-h-CH2-CH3 sequentially from the N-terminus to the C-terminus; or (26) the polypeptide chain 1 comprises VL_A-CL sequentially from the N-terminus to the C-terminus, the polypeptide chain 2 comprises VH_A-CH1-h-CH2-CH3 sequentially from the N-terminus to the C-terminus, and the polypeptide chain 3 comprises VH_B-L1-VH_B-L2-VH_B-h-CH2-CH3 sequentially from the N-terminus to the C-terminus; or (27) the polypeptide chain 1 comprises VL_A-CL sequentially from the N-terminus to the C-terminus, the polypeptide chain 2 comprises VH_A-CH1-h-CH2-CH3 sequentially from the N-terminus to the C-terminus, and the polypeptide chain 3 comprises VH_D-L1-VH_C-L2-VH_B-h-CH2-CH3 sequentially from the N-terminus to the C-terminus; wherein the L, L1 and L2 are linker peptides, and the h is a hinge region or linker peptide such as "-", GS or an amino acid sequence as set forth in any one of SEQ ID NOs: 495-519; or,

(C) the binding protein has two polypeptide chains, including one polypeptide chain 1 and one polypeptide chain 2, wherein (20) the polypeptide chain 1 comprises VL_A-L-VH_A-h-CH2-CH3 sequentially from the N-terminus to the C-terminus, and the polypeptide chain 2 comprises VH_B-h-CH2-CH3 sequentially from the N-terminus to the C-terminus; or (21) the polypeptide chain 1 comprises VH_A-L-VL_A-h-CH2-CH3 sequentially from the N-terminus to the C-terminus, and the polypeptide chain 2 comprises VH_B-h-CH2-CH3 sequentially from the N-terminus to the C-terminus; or (22) the polypeptide chain 1 comprises VL_A-L1-VH_A-h-CH2-CH3 sequentially from the N-terminus to the C-terminus, and the polypeptide chain 2 comprises VH_B-L2-VH_B-h-CH2-CH3 sequentially from the N-terminus to the C-terminus; or (23) the polypeptide chain 1 comprises VH_A-L1-VL_A-h-CH2-CH3 sequentially from the N-terminus to the C-terminus, and the polypeptide chain 2 comprises VH_B-L2-VH_B-h-CH2-CH3 sequentially from the N-terminus to the C-terminus; or (24) the polypeptide chain 1 comprises VL_A-L1-VH_A-h-CH2-CH3 sequentially from the N-terminus to the C-terminus, and the polypeptide chain 2 comprises VH_C-L2-VH_B-CH2-CH3 sequentially from the N-terminus to the C-terminus; or (25) the polypeptide chain 1 comprises VH_A-L1-VL_A-h-CH2-CH3 sequentially from the N-terminus to the C-terminus, and the polypeptide chain 2 comprises VH_C-L2-VH_B-h-CH2-CH3 sequentially from the N-terminus to the C-terminus; wherein the L, L1 and L2 are linker peptides, and the h is a hinge region or linker peptide such as "-", GS or an amino acid sequence as set forth in any one of SEQ ID NOs: 495-519.

4. The binding protein according to any one of items 1 to 3, wherein the protein functional region A or the protein functional region B is selected from one of a PD-L1 antibody, an HER2 antibody, a B7H4 antibody, a CD3 antibody, a CTLA4 antibody, a 4-1BB antibody and a BCMA antibody; preferably, the protein functional region A is a PD-L1 antibody, an HER2 antibody, a B7H4 antibody or a CD3 antibody, and the protein functional region B is a CTLA4 antibody, a 4-1BB antibody or a BCMA antibody;
more preferably, the protein functional region A is a PD-L1 antibody, and the protein functional region B is a CTLA4 antibody; or, the protein functional region A is a PD-L1 antibody, and the protein functional region B is a 4-1BB antibody; or, the protein functional region A is an HER2 antibody, and the protein functional region B is a CTLA4 antibody; or, the protein functional region A is a B7H4 antibody, and the protein functional region B is a 4-1BB antibody; or, the protein functional region A is a CD3 antibody, and the protein functional region B is a BCMA antibody.

5. The binding protein according to item 4, wherein the PD-L1 antibody comprises a light chain variable region and a heavy chain variable region, wherein the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 62 and 122, respectively; or, the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 69 and 122, respectively; preferably, the light chain variable region VL comprises an amino acid sequence as set forth in SEQ ID NO: 282; and the heavy chain variable region VH comprises an amino acid sequence as set forth in SEQ ID NO: 233; or, the light chain variable region VL comprises an amino acid sequence as set forth in SEQ ID NO: 282; and the heavy chain variable region VH comprises an amino acid sequence as set forth in SEQ ID NO: 240; and,

the PD-L1 antibody comprises a light chain variable region and a heavy chain variable region, wherein the light chain variable region VL comprises LCDR1, LCDR2 and LCDR3 with amino acid sequences as set forth in SEQ ID NOs: 169, 190 and 213, respectively; and the heavy chain variable region VH comprises HCDR1, HCDR2 and HCDR3 with amino acid sequences as set forth in SEQ ID NOs: 16, 64 and 124, respectively; preferably, the light chain variable region VL comprises an amino acid sequence as set forth in SEQ ID NO: 284; and the heavy chain variable region VH comprises an amino acid sequence as set forth in SEQ ID NO: 235; and,

the HER2 antibody comprises a light chain variable region and a heavy chain variable region, wherein the light chain variable region VL comprises LCDR1, LCDR2 and LCDR3 with amino acid sequences as set forth in SEQ ID NO: 171, 190 and 215, respectively; and the heavy chain variable region VH comprises HCDR1, HCDR2 and HCDR3 with amino acid sequences as set forth in SEQ ID NOs: 19, 67 and 127, respectively; or, the light chain variable region VL comprises LCDR1, LCDR2 and LCDR3 with amino acid sequences as set forth in SEQ ID NOs: 176, 196 and 220, respectively; and the heavy chain variable region VH comprises HCDR1, HCDR2 and HCDR3 with amino acid sequences as set forth in SEQ ID NOs: 23, 74 and 132, respectively; preferably, the light chain variable region VL comprises an amino acid sequence as set forth in SEQ ID NO: 286; and the heavy chain variable region VH comprises an amino acid sequence as set forth in SEQ ID NO: 238; or, the light chain variable region VL comprises an amino acid sequence as set forth in SEQ ID NO: 293; and the heavy chain variable region VH comprises an amino acid sequence as set forth in SEQ ID NO: 247; and,

the B7H4 antibody comprises a light chain variable region and a heavy chain variable region, wherein the light chain variable region VL comprises LCDR1, LCDR2 and LCDR3 with amino acid sequences as set forth in SEQ ID NOs: 180, 191 and 225, respectively; and the heavy chain variable region VH comprises HCDR1, HCDR2 and HCDR3 with amino acid sequences as set forth in SEQ ID NOs: 32, 87 and 143, respectively; or, the light chain variable region VL comprises LCDR1, LCDR2 and LCDR3 with amino acid sequences as set forth in SEQ ID NOs: 177, 191 and 226, respectively; and the heavy chain variable region VH comprises HCDR1, HCDR2 and HCDR3 with amino acid sequences as set forth in SEQ ID NOs: 33, 88 and 144, respectively; preferably, the light chain variable region VL comprises an amino acid sequence as set forth in SEQ ID NO: 298; and the heavy chain variable region VH comprises an amino acid sequence as set forth in SEQ ID NO: 261; or, the light chain variable region VL comprises an amino acid sequence as set forth in SEQ ID NO: 299; and the heavy chain variable region VH comprises an amino acid sequence as set forth in SEQ ID NO: 262; and,

the CTLA4 antibody comprises a heavy chain variable region, wherein the heavy chain variable region VH comprises HCDR1, HCDR2 and HCDR3 with amino acid sequences as set forth in SEQ ID NOs: 17, 65 and 125, respectively; preferably, the heavy chain variable region VH comprises an amino acid sequence as set forth in SEQ ID NO: 236; and,

the 4-1BB antibody comprises a light chain variable region and a heavy chain variable region, wherein the light chain variable region VL comprises LCDR1, LCDR2 and LCDR3 with amino acid sequences as set forth in SEQ ID NO: 170, 191 and 214, respectively; and the heavy chain variable region VH comprises HCDR1, HCDR2 and HCDR3 with amino acid sequences as set forth in SEQ ID NOs: 18, 66 and 126, respectively; or, the light chain variable region VL comprises LCDR1, LCDR2 and LCDR3 with amino acid sequences as set forth in SEQ ID NOs: 170, 191 and 214, respectively; and the heavy chain variable region VH comprises HCDR1, HCDR2 and HCDR3 with amino acid sequences as set forth in SEQ ID NOs: 18, 71 and 126, respectively; preferably, the light chain variable region VL comprises an amino acid sequence as set forth in SEQ ID NO: 285; and the heavy chain variable region VH comprises an amino acid sequence as set forth in SEQ ID NO: 237; or, the light chain variable region VL comprises an amino acid sequence as set forth in SEQ ID NO: 289; and the heavy chain variable region VH comprises an amino acid sequence as set forth in SEQ ID NO: 242; and,

the 4-1BB antibody comprises a heavy chain variable region, wherein the heavy chain variable region VH comprises HCDR1, HCDR2 and HCDR3 with amino acid sequences as set forth in SEQ ID NOs: 27, 79 and 137, respectively; or, the heavy chain variable region VH comprises HCDR1, HCDR2 and HCDR3 with amino acid sequences as set forth in SEQ ID NOs: 28, 80 and 138, respectively; or, the heavy chain variable region VH comprises HCDR1, HCDR2 and HCDR3 with amino acid sequences as set forth in SEQ ID NOs: 29, 82 and 138, respectively; or, the heavy chain variable region VH comprises HCDR1, HCDR2 and HCDR3 with amino acid sequences as set forth in SEQ ID NOs: 28, 89 and 145, respectively; or, the heavy chain variable region VH comprises HCDR1, HCDR2 and HCDR3 with amino acid sequences as set forth in SEQ ID NOs: 28, 81 and 139, respectively; or, the heavy chain variable region VH comprises HCDR1, HCDR2 and HCDR3 with amino acid sequences as set forth in SEQ ID NOs: 15, 83 and 140, respectively; preferably, the heavy chain variable region VH comprises an amino acid sequence as set forth in SEQ ID NO: 252; or, the heavy chain variable region VH comprises an amino acid sequence as set forth in SEQ ID NO: 253; or, the heavy chain variable region VH comprises an amino acid sequence as set forth in SEQ ID NO: 255; or, the heavy chain variable region VH comprises an amino acid sequence as set forth in SEQ ID NO: 264; or, the heavy chain variable region VH comprises an amino acid sequence as set forth in SEQ ID NO: 254; or, the heavy chain variable region VH comprises an amino acid sequence as set forth in SEQ ID NO: 256; and,

the CD3 antibody comprises a light chain variable region and a heavy chain variable region, wherein the light chain variable region VL comprises LCDR1, LCDR2 and LCDR3 with amino acid sequences as set forth in SEQ ID NOs: 177, 191 and 221, respectively; and the heavy chain variable region VH comprises HCDR1, HCDR2 and HCDR3 with amino acid sequences as set forth in SEQ ID NOs: 15, 84 and 141, respectively; or, the light chain variable region VL comprises LCDR1, LCDR2 and LCDR3 with amino acid sequences as set forth in SEQ ID NOs: 178, 197 and 222; and the heavy chain variable region VH comprises HCDR1, HCDR2 and HCDR3 with amino acid sequences as set forth in SEQ ID NOs: 31, 85 and 142, respectively; or, the light chain variable region VL comprises LCDR1, LCDR2 and LCDR3 with amino acid sequences as set forth in SEQ ID NOs: 177, 191 and 223, respectively; and the heavy chain variable region VH comprises HCDR1, HCDR2 and HCDR3 with amino acid sequences as set forth in SEQ ID NOs: 31, 86 and 141, respectively; or, the light chain variable region VL comprises LCDR1, LCDR2 and LCDR3 with amino acid sequences as set forth in SEQ ID NOs: 179, 198 and 224, respectively; and the heavy chain variable region VH comprises HCDR1, HCDR2 and HCDR3 with amino acid sequences as set forth in SEQ ID NOs: 31, 85 and 142, respectively; preferably, the light chain variable region VL comprises an amino acid sequence as set forth in SEQ ID NO: 294; and the heavy chain variable region VH comprises an amino acid sequence as set forth in SEQ ID NO: 258; or, the light chain variable region VL comprises an amino acid sequence as set forth in SEQ ID NO: 295; and the heavy chain variable region VH comprises an amino acid sequence as set forth in SEQ ID NO: 259; or, the light chain variable region VL comprises an amino acid sequence as set forth in SEQ ID NO: 296; and the heavy chain variable region VH comprises an amino acid sequence as set forth in SEQ ID NO: 260; or, the light chain variable region VL comprises an amino acid sequence as set forth in SEQ ID NO: 297; and the heavy chain variable region VH comprises an amino acid sequence as set forth in SEQ ID NO: 259; and,

the CD3 antibody comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 20, 68 and 128, respectively; or, the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; preferably, the light chain variable region VL comprises an amino acid sequence as set forth in SEQ ID NO: 291; and the heavy chain variable region VH comprises an amino acid sequence as set forth in SEQ ID NO: 245, respectively; or, the light chain variable region VL comprises an amino acid sequence as set forth in SEQ ID NO: 291; and the heavy chain variable region VH comprises an amino acid sequence as set forth in SEQ ID NO: 257; or, the light chain variable region VL comprises an amino acid sequence as set forth in SEQ ID NO: 291; and the heavy chain variable region VH comprises an amino acid sequence as set forth in SEQ ID NO: 263; or, the light chain variable region VL comprises an amino acid sequence as set forth in SEQ ID NO: 291; and the heavy chain variable region VH comprises an amino acid sequence as set forth in SEQ ID NO: 281; or, the light chain variable region VL comprises an amino acid sequence as set forth in SEQ ID NO: 291; and the heavy chain variable region VH comprises an amino acid sequence as set forth in SEQ ID NO: 244; and,

the BCMA antibody comprises a heavy chain variable region, wherein the heavy chain variable region VH comprises HCDR1, HCDR2 and HCDR3 with amino acid sequences as set forth in SEQ ID NOs: 15, 75 and 133, respectively; or, the heavy chain variable region VH comprises HCDR1, HCDR2 and HCDR3 with amino acid sequences set forth in SEQ ID NOs: 24, 76 and 134, respectively; or, the heavy chain variable region VH comprises HCDR1, HCDR2 and HCDR3 with amino acid sequences set forth in SEQ ID NOs: 25, 77 and 135, respectively; or, the heavy chain variable region VH comprises HCDR1, HCDR2 and HCDR3 with amino acid sequences as set forth in SEQ ID NOs: 26, 78 and 136, respectively; preferably, the heavy chain variable region VH comprises an amino acid sequence as set forth in SEQ ID NO: 248; or, the heavy chain variable region VH comprises an amino acid sequence as set forth in SEQ ID NO: 249; or, the heavy chain variable region VH comprises an amino acid sequence as set forth in SEQ ID NO: 250; or, the heavy chain variable region VH comprises an amino acid sequence as set forth in SEQ ID NO: 251; and,

the BCMA antibody comprises a heavy chain variable region, wherein the heavy chain variable region VH comprises HCDR1, HCDR2 and HCDR3 with amino acid sequences as set forth in SEQ ID NOs: 26, 90 and 136; or, the heavy chain variable region VH comprises HCDR1, HCDR2 and HCDR3 with amino acid sequences as set forth in SEQ ID NOs: 34, 78 and 146, respectively; or, the heavy chain variable region VH comprises HCDR1, HCDR2 and HCDR3 with amino acid sequences as set forth in SEQ ID NOs: 35, 78 and 147; or, the heavy chain variable region VH comprises HCDR1, HCDR2 and HCDR3 with amino acid sequences as set forth in SEQ ID NOs: 35, 90 and 147, respectively; or, the heavy chain variable region VH comprises HCDR1, HCDR2 and HCDR3 with amino acid sequences as set forth in SEQ ID NOs: 36, 90 and 146, respectively; or, the heavy chain variable region VH comprises HCDR1, HCDR2 and HCDR3 with amino acid sequences as set forth in SEQ ID NOs: 35, 76 and 147, respectively; or, the heavy chain variable region VH comprises HCDR1, HCDR2 and HCDR3 with amino acid sequences as set forth in SEQ ID NOs: 34, 78 and 148, respectively; or, the heavy chain variable region VH comprises HCDR1, HCDR2 and HCDR3 with amino acid sequences as set forth in SEQ ID NOs: 35, 76 and 136, respectively; or, the heavy chain variable region VH comprises HCDR1, HCDR2 and HCDR3 with amino acid sequences as set forth in SEQ ID NOs: 35, 90 and 146, respectively; or, the heavy chain variable region VH comprises HCDR1, HCDR2 and HCDR3 with amino acid sequences as set forth in SEQ ID NOs: 34, 76 and 136, respectively; or, the heavy chain variable region VH comprises HCDR1, HCDR2 and HCDR3 with amino acid sequences as set forth in SEQ ID NO 35, 76 and 146, respectively; or, the heavy chain variable region VH comprises HCDR1, HCDR2 and HCDR3 with amino acid sequences as set forth in SEQ ID NOs: 34, 76 and 146, respectively; or, the heavy chain variable region VH comprises HCDR1, HCDR2 and HCDR3 with amino acid sequences as set forth in SEQ ID NOs: 35, 90 and 148, respectively; or, the heavy chain variable region VH comprises HCDR1, HCDR2 and HCDR3 with amino acid sequences as set forth in SEQ ID NOs: 35, 90 and 136, respectively; preferably, the heavy chain variable region VH comprises an amino acid sequence as set forth in SEQ ID NO: 265; or, the heavy chain variable region VH comprises an amino acid sequence as set forth in SEQ ID NO: 266; or, the heavy chain variable region VH comprises an amino acid sequence as set forth in SEQ ID NO: 267; or, the heavy chain variable region VH comprises an amino acid sequence as set forth in SEQ ID NO: 268; or, the heavy chain variable region VH comprises an amino acid sequence as set forth in SEQ ID NO: 269; or, the heavy chain variable region VH comprises an amino acid sequence as set forth in SEQ ID NO: 270; or, the heavy chain variable region VH comprises an amino acid sequence as set forth in SEQ ID NO: 271; or, the heavy chain variable region VH comprises an amino acid sequence as set forth in SEQ ID NO: 272; or, the heavy chain variable region VH comprises an amino acid sequence as set forth in SEQ ID NO: 273; or, the heavy chain variable region VH comprises an amino acid sequence as set forth in SEQ ID NO: 274; or, the heavy chain variable region VH comprises an amino acid sequence as set forth in SEQ ID NO: 275; or, the heavy chain variable region VH comprises an amino acid sequence as set forth in SEQ ID NO: 276; or, the heavy chain variable region VH comprises an amino acid sequence as set forth in SEQ ID NO: 277; or, the heavy chain variable region VH comprises an amino acid sequence as set forth in SEQ ID NO: 278; or, the heavy chain variable region VH comprises an amino acid sequence as set forth in SEQ ID NO: 279; or, the heavy chain variable region VH comprises an amino acid sequence as set forth in SEQ ID NO: 280.

6. The binding protein according to any one of items 1 to 4,

(A) comprising a protein functional region A and a protein functional region B, wherein:

the protein functional region A comprises a light chain variable region and a heavy chain variable region, wherein the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 69 and 122, respectively; and the protein functional region B comprises a heavy chain variable region, wherein the heavy chain variable region VH comprises HCDR1, HCDR2 and HCDR3 with amino acid sequences as set forth in SEQ ID NOs: 17, 65 and 125, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 62 and 122, respectively; and the protein functional region B comprises a heavy chain variable region, wherein the heavy chain variable region VH comprises HCDR1, HCDR2 and HCDR3 with amino acid sequences as set forth in SEQ ID NOs: 17, 65 and 125, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 171, 190 and 215, respectively; and the heavy chain variable region VH comprises HCDR1, HCDR2 and HCDR3 with amino acid sequences as set forth in SEQ ID NOs: 19, 67 and 127, respectively; and the protein functional region B comprises a heavy chain variable region, wherein the heavy chain variable region VH comprises HCDR1, HCDR2 and HCDR3 with amino acid sequences as set forth in SEQ ID NOs: 17, 65 and 125, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 176, 196 and 220, respectively; and the heavy chain variable region VH comprises HCDR1, HCDR2 and HCDR3 with amino acid sequences as set forth in SEQ ID NOs: 23, 74 and 132, respectively; and the protein functional region B comprises a heavy chain variable region, wherein the heavy chain variable region VH comprises HCDR1, HCDR2 and HCDR3 with amino acid sequences as set forth in SEQ ID NOs: 17, 65 and 125, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 69 and 122, respectively; and the protein functional region B comprises a heavy chain variable region, wherein the heavy chain variable region VH comprises HCDR1, HCDR2 and HCDR3 with amino acid sequences as set forth in SEQ ID NOs: 28, 80 and 138, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 69 and 122, respectively; and the protein functional region B comprises a heavy chain variable region, wherein the heavy chain variable region VH comprises HCDR1, HCDR2 and HCDR3 with amino acid sequences as set forth in SEQ ID NOs: 27, 79 and 137, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 167, 188 and 211, respectively; and the heavy chain variable region VH comprises HCDR1, HCDR2 and HCDR3 with amino acid sequences as set forth in SEQ ID NOs: 15, 69 and 122, respectively; and the protein functional region B comprises a heavy chain variable region, wherein the heavy chain variable region VH comprises HCDR1, HCDR2 and HCDR3 with amino acid sequences as set forth in SEQ ID NOs: 29, 82 and 138, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 180, 191 and 225, respectively; and the heavy chain variable region VH comprises HCDR1, HCDR2 and HCDR3 with amino acid sequences as set forth in SEQ ID NOs: 32, 87 and 143, respectively; and the protein functional region B comprises a heavy chain variable region, wherein the heavy chain variable region VH comprises HCDR1, HCDR2 and HCDR3 with amino acid sequences as set forth in SEQ ID NOs: 28, 80 and 138, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 180, 191 and 225, respectively; and the heavy chain variable region VH comprises HCDR1, HCDR2 and HCDR3 with amino acid sequences as set forth in SEQ ID NOs: 32, 87 and 143, respectively; and the protein functional region B comprises a heavy chain variable region, wherein the heavy chain variable region VH comprises HCDR1, HCDR2 and HCDR3 with amino acid sequences as set forth in SEQ ID NOs: 27, 79 and 137, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 180, 191 and 225, respectively; and the heavy chain variable region VH comprises HCDR1, HCDR2 and HCDR3 with amino acid sequences as set forth in SEQ ID NOs: 32, 87 and 143, respectively; and the protein functional region B comprises a heavy chain variable region, wherein the heavy chain variable region VH comprises HCDR1, HCDR2 and HCDR3 with amino acid sequences as set forth in SEQ ID NOs: 28, 89 and 145, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 177, 191 and 226, respectively; and the heavy chain variable region VH comprises HCDR1, HCDR2 and HCDR3 with amino acid sequences as set forth in SEQ ID NOs: 33, 88 and 144, respectively; and the protein functional region B comprises a heavy chain variable region, wherein the heavy chain variable region VH comprises HCDR1, HCDR2 and HCDR3 with amino acid sequences as set forth in SEQ ID NOs: 28, 80 and 138, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 177, 191 and 226, respectively; and the heavy chain variable region VH comprises HCDR1, HCDR2 and HCDR3 with amino acid sequences as set forth in SEQ ID NOs: 33, 88 and 144, respectively; and the protein functional region B comprises a heavy chain variable region, wherein the heavy chain variable region VH comprises HCDR1, HCDR2 and HCDR3 with amino acid sequences as set forth in SEQ ID NOs: 28, 81 and 139, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 177, 191 and 226, respectively; and the heavy chain variable region VH comprises HCDR1, HCDR2 and HCDR3 with amino acid sequences as set forth in SEQ ID NOs: 33, 88 and 144, respectively; and the protein functional region B comprises a heavy chain variable region, wherein the heavy chain variable region VH comprises HCDR1, HCDR2 and HCDR3 with amino acid sequences as set forth in SEQ ID NOs: 15, 83 and 140, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 20, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 24, 76 and 134, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 20, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 20, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 15, 75 and 133, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 15, 75 and 133, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 26, 90 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 36, 90 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 76 and 147, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 34, 78 and 148, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 76 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 90 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 34, 76 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 76 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 34, 76 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 90 and 148, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 90 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 34, 78 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 78 and 147, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 90 and 147, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 177, 191 and 221, respectively; and the heavy chain variable region VH comprises HCDR1, HCDR2 and HCDR3 with amino acid sequences as set forth in SEQ ID NOs: 15, 84 and 141, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 178, 197 and 222, respectively; and the heavy chain variable region VH comprises HCDR1, HCDR2 and HCDR3 with amino acid sequences as set forth in SEQ ID NOs: 31, 85 and 142, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 177, 191 and 223, respectively; and the heavy chain variable region VH comprises HCDR1, HCDR2 and HCDR3 with amino acid sequences as set forth in SEQ ID NOs: 31, 86 and 141, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 179, 198 and 224, respectively; and the heavy chain variable region VH comprises HCDR1, HCDR2 and HCDR3 with amino acid sequences as set forth in SEQ ID NOs: 31, 85 and 142, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

(B) comprising a protein functional region A, a protein functional region B and a protein functional region C, wherein:

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; the protein functional region B comprises a heavy chain variable region, wherein the heavy chain variable region VH comprises HCDR1, HCDR2 and HCDR3 with amino acid sequences as set forth in SEQ ID NOs: 15, 75 and 133, respectively; and the protein functional region C comprises a heavy chain variable region, wherein the heavy chain variable region VH comprises HCDR1, HCDR2 and HCDR3 with amino acid sequences as set forth in SEQ ID NOs: 24, 76 and 134, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; the protein functional region B comprises a heavy chain variable region, wherein the heavy chain variable region VH comprises HCDR1, HCDR2 and HCDR3 with amino acid sequences as set forth in SEQ ID NOs: 25, 77 and 135, respectively; and the protein functional region C comprises a heavy chain variable region, wherein the heavy chain variable region VH comprises HCDR1, HCDR2 and HCDR3 with amino acid sequences as set forth in SEQ ID NOs: 26, 78 and 136, respectively.

7. The binding protein according to any one of items 1 to 6,

(A) comprising a protein functional region A and a protein functional region B, wherein:

the protein functional region A comprises a light chain variable region and a heavy chain variable region, wherein the light chain variable region VL comprises an amino acid sequence as set forth in SEQ ID NO: 282; and the heavy chain variable region VH comprises an amino acid sequence as set forth in SEQ ID NO: 240; and the protein functional region B comprises a heavy chain variable region, wherein the heavy chain variable region VH comprises an amino acid sequence as set forth in SEQ ID NO: 236; or,

the protein functional region A comprises a light chain variable region and a heavy chain variable region, wherein the light chain variable region VL comprises an amino acid sequence as set forth in SEQ ID NO: 282; and the heavy chain variable region VH comprises an amino acid sequence as set forth in SEQ ID NO: 233; and the protein functional region B comprises a heavy chain variable region, wherein the heavy chain variable region VH comprises an amino acid sequence as set forth in SEQ ID NO: 236; or,

the protein functional region A comprises a light chain variable region and a heavy chain variable region, wherein the light chain variable region VL comprises an amino acid sequence as set forth in SEQ ID NO: 286; and the heavy chain variable region VH comprises an amino acid sequence as set forth in SEQ ID NO: 238; and the protein functional region B comprises a heavy chain variable region, wherein the heavy chain variable region VH comprises an amino acid sequence as set forth in SEQ ID NO: 236; or,

the protein functional region A comprises a light chain variable region and a heavy chain variable region, wherein the light chain variable region VL comprises an amino acid sequence as set forth in SEQ ID NO: 293; and the heavy chain variable region VH comprises an amino acid sequence as set forth in SEQ ID NO: 247; and the protein functional region B comprises a heavy chain variable region, wherein the heavy chain variable region VH comprises an amino acid sequence as set forth in SEQ ID NO: 236; or,

the protein functional region A comprises a light chain variable region and a heavy chain variable region, wherein the light chain variable region VL comprises an amino acid sequence as set forth in SEQ ID NO: 282; and the heavy chain variable region VH comprises an amino acid sequence as set forth in SEQ ID NO: 240; and the protein functional region B comprises a heavy chain variable region, wherein the heavy chain variable region VH comprises an amino acid sequence as set forth in SEQ ID NO: 253; or,

the protein functional region A comprises a light chain variable region and a heavy chain variable region, wherein the light chain variable region VL comprises an amino acid sequence as set forth in SEQ ID NO: 282; and the heavy chain variable region VH comprises an amino acid sequence as set forth in SEQ ID NO: 240; and the protein functional region B comprises a heavy chain variable region, wherein the heavy chain variable region VH comprises an amino acid sequence as set forth in SEQ ID NO: 252; or,

the protein functional region A comprises a light chain variable region and a heavy chain variable region, wherein the light chain variable region VL comprises an amino acid sequence as set forth in SEQ ID NO: 282; and the heavy chain variable region VH comprises an amino acid sequence as set forth in SEQ ID NO: 240; and the protein functional region B comprises a heavy chain variable region, wherein the heavy chain variable region VH comprises an amino acid sequence as set forth in SEQ ID NO: 255; or,

the protein functional region A comprises a light chain variable region and a heavy chain variable region, wherein the light chain variable region VL comprises an amino acid sequence as set forth in SEQ ID NO: 298; and the heavy chain variable region VH comprises an amino acid sequence as set forth in SEQ ID NO: 261; and the protein functional region B comprises a heavy chain variable region, wherein the heavy chain variable region VH comprises an amino acid sequence as set forth in SEQ ID NO: 253; or,

the protein functional region A comprises a light chain variable region and a heavy chain variable region, wherein the light chain variable region VL comprises an amino acid sequence as set forth in SEQ ID NO: 298; and the heavy chain variable region VH comprises an amino acid sequence as set forth in SEQ ID NO: 261; and the protein functional region B comprises a heavy chain variable region, wherein the heavy chain variable region VH comprises an amino acid sequence as set forth in SEQ ID NO: 252; or,

the protein functional region A comprises a light chain variable region and a heavy chain variable region, wherein the light chain variable region VL comprises an amino acid sequence as set forth in SEQ ID NO: 298; and the heavy chain variable region VH comprises an amino acid sequence as set forth in SEQ ID NO: 261; and the protein functional region B comprises a heavy chain variable region, wherein the heavy chain variable region VH comprises an amino acid sequence as set forth in SEQ ID NO: 264; or,

the protein functional region A comprises a light chain variable region and a heavy chain variable region, wherein the light chain variable region VL comprises an amino acid sequence as set forth in SEQ ID NO: 299; and the heavy chain variable region VH comprises an amino acid sequence as set forth in SEQ ID NO: 262; and the protein functional region B comprises a heavy chain variable region, wherein the heavy chain variable region VH comprises an amino acid sequence as set forth in SEQ ID NO: 253; or,

the protein functional region A comprises a light chain variable region and a heavy chain variable region, wherein the light chain variable region VL comprises an amino acid sequence as set forth in SEQ ID NO: 299; and the heavy chain variable region VH comprises an amino acid sequence as set forth in SEQ ID NO: 262; and the protein functional region B comprises a heavy chain variable region, wherein the heavy chain variable region VH comprises an amino acid sequence as set forth in SEQ ID NO: 254; or,

the protein functional region A comprises a light chain variable region and a heavy chain variable region, wherein the light chain variable region VL comprises an amino acid sequence as set forth in SEQ ID NO: 299; and the heavy chain variable region VH comprises an amino acid sequence as set forth in SEQ ID NO: 262; and the protein functional region B comprises a heavy chain variable region, wherein the heavy chain variable region VH comprises an amino acid sequence as set forth in SEQ ID NO: 256; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 245; and the protein functional region B comprises a heavy chain variable region, wherein the heavy chain variable region VH comprises an amino acid sequence as set forth in SEQ ID NO: 249; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 245; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 245; and the protein functional region B comprises a heavy chain variable region, wherein the heavy chain variable region VH comprises an amino acid sequence as set forth in SEQ ID NO: 248; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 257; and the protein functional region B comprises a heavy chain variable region, wherein the heavy chain variable region VH comprises an amino acid sequence as set forth in SEQ ID NO: 248; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 257; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 281; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 265; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 269; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 270; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 271; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 272; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 273; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 274; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 275; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 276; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 277; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 278; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 279; or,

the protein functional region A comprises a light chain variable region and a heavy chain variable region, wherein the light chain variable region VL comprises an amino acid sequence as set forth in SEQ ID NO: 291;

and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 280; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 266; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 267; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 268; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 244; and the protein functional region B comprises a heavy chain variable region, wherein the heavy chain variable region VH comprises an amino acid sequence as set forth in SEQ ID NO: 249; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 244; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 294; and the heavy chain variable region VH comprises an amino acid sequence as set forth in SEQ ID NO: 258; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 295; and the heavy chain variable region VH comprises an amino acid sequence as set forth in SEQ ID NO: 259; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 296; and the heavy chain variable region VH comprises an amino acid sequence as set forth in SEQ ID NO: 260; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 297; and the heavy chain variable region VH comprises an amino acid sequence as set forth in SEQ ID NO: 259; and the protein functional region B comprises a heavy chain variable region, wherein the heavy chain variable region VH comprises an amino acid sequence as set forth in SEQ ID NO: 251; or,

(B) comprising a protein functional region A, a protein functional region B and a protein functional region C, wherein:

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 257; the protein functional region B comprises a heavy chain variable region, wherein the heavy chain variable region VH comprises an amino acid sequence as set forth in SEQ ID NO: 248; and the protein functional region C comprises a heavy chain variable region, wherein the heavy chain variable region VH comprises an amino acid sequence as set forth in SEQ ID NO: 249; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 257; the protein functional region B comprises a heavy chain variable region, wherein the heavy chain variable region VH comprises an amino acid sequence as set forth in SEQ ID NO: 250; and the protein functional region C comprises a heavy chain variable region, wherein the heavy chain variable region VH comprises an amino acid sequence as set forth in SEQ ID NO: 251.

8. The binding protein according to any one of items 1 to 7,

(1) comprising two polypeptide chains, wherein,

a first polypeptide chain comprises an amino acid sequence as set forth in SEQ ID NO: 371; and a second polypeptide chain comprises an amino acid sequence as set forth in SEQ ID NO: 372; or,

a first polypeptide chain comprises an amino acid sequence as set forth in SEQ ID NO: 371; and a second polypeptide chain comprises an amino acid sequence as set forth in SEQ ID NO: 373; or,

a first polypeptide chain comprises an amino acid sequence as set forth in SEQ ID NO: 353; and a second polypeptide chain comprises an amino acid sequence as set forth in SEQ ID NO: 362; or,

a first polypeptide chain comprises an amino acid sequence as set forth in SEQ ID NO: 363; and a second polypeptide chain comprises an amino acid sequence as set forth in SEQ ID NO: 364; or,

a first polypeptide chain comprises an amino acid sequence as set forth in SEQ ID NO: 365; and a second polypeptide chain comprises an amino acid sequence as set forth in SEQ ID NO: 364; or,

a first polypeptide chain comprises an amino acid sequence as set forth in SEQ ID NO: 353; and a second polypeptide chain comprises an amino acid sequence as set forth in SEQ ID NO: 366; or,

a first polypeptide chain comprises an amino acid sequence as set forth in SEQ ID NO: 353; and a second polypeptide chain comprises an amino acid sequence as set forth in SEQ ID NO: 369; or,

a first polypeptide chain comprises an amino acid sequence as set forth in SEQ ID NO: 353; and a second polypeptide chain comprises an amino acid sequence as set forth in SEQ ID NO: 370; or,

a first polypeptide chain comprises an amino acid sequence as set forth in SEQ ID NO: 353; and a second polypeptide chain comprises an amino acid sequence as set forth in SEQ ID NO: 394; or,

a first polypeptide chain comprises an amino acid sequence as set forth in SEQ ID NO: 353; and a second polypeptide chain comprises an amino acid sequence as set forth in SEQ ID NO: 395; or,

a first polypeptide chain comprises an amino acid sequence as set forth in SEQ ID NO: 363; and a second polypeptide chain comprises an amino acid sequence as set forth in SEQ ID NO: 310; or,

a first polypeptide chain comprises an amino acid sequence as set forth in SEQ ID NO: 363; and a second polypeptide chain comprises an amino acid sequence as set forth in SEQ ID NO: 396; or,

a first polypeptide chain comprises an amino acid sequence as set forth in SEQ ID NO: 363; and a second polypeptide chain comprises an amino acid sequence as set forth in SEQ ID NO: 362; or,

a first polypeptide chain comprises an amino acid sequence as set forth in SEQ ID NO: 363; and a second polypeptide chain comprises an amino acid sequence as set forth in SEQ ID NO: 394; or,

a first polypeptide chain comprises an amino acid sequence as set forth in SEQ ID NO: 363; and a second polypeptide chain comprises an amino acid sequence as set forth in SEQ ID NO: 395; or,

a first polypeptide chain comprises an amino acid sequence as set forth in SEQ ID NO: 367; and a second polypeptide chain comprises an amino acid sequence as set forth in SEQ ID NO: 368; or,

a first polypeptide chain comprises an amino acid sequence as set forth in SEQ ID NO: 367; and a second polypeptide chain comprises an amino acid sequence as set forth in SEQ ID NO: 378; or,

a first polypeptide chain comprises an amino acid sequence as set forth in SEQ ID NO: 367; and a second polypeptide chain comprises an amino acid sequence as set forth in SEQ ID NO: 379; or,

a first polypeptide chain comprises an amino acid sequence as set forth in SEQ ID NO: 367; and a second polypeptide chain comprises an amino acid sequence as set forth in SEQ ID NO: 380; or,

a first polypeptide chain comprises an amino acid sequence as set forth in SEQ ID NO: 367; and a second polypeptide chain comprises an amino acid sequence as set forth in SEQ ID NO: 381; or,

a first polypeptide chain comprises an amino acid sequence as set forth in SEQ ID NO: 367; and a second polypeptide chain comprises an amino acid sequence as set forth in SEQ ID NO: 382; or,

a first polypeptide chain comprises an amino acid sequence as set forth in SEQ ID NO: 367; and a second polypeptide chain comprises an amino acid sequence as set forth in SEQ ID NO: 383; or,

a first polypeptide chain comprises an amino acid sequence as set forth in SEQ ID NO: 367; and a second polypeptide chain comprises an amino acid sequence as set forth in SEQ ID NO: 385; or,

a first polypeptide chain comprises an amino acid sequence as set forth in SEQ ID NO: 367; and a second polypeptide chain comprises an amino acid sequence as set forth in SEQ ID NO: 388; or,

a first polypeptide chain comprises an amino acid sequence as set forth in SEQ ID NO: 367; and a second polypeptide chain comprises an amino acid sequence as set forth in SEQ ID NO: 389; or,

a first polypeptide chain comprises an amino acid sequence as set forth in SEQ ID NO: 351; and a second polypeptide chain comprises an amino acid sequence as set forth in SEQ ID NO: 374; or,

a first polypeptide chain comprises an amino acid sequence as set forth in SEQ ID NO: 351; and a second polypeptide chain comprises an amino acid sequence as set forth in SEQ ID NO: 375; or,

a first polypeptide chain comprises an amino acid sequence as set forth in SEQ ID NO: 351; and a second polypeptide chain comprises an amino acid sequence as set forth in SEQ ID NO: 386; or,

a first polypeptide chain comprises an amino acid sequence as set forth in SEQ ID NO: 351; and a second polypeptide chain comprises an amino acid sequence as set forth in SEQ ID NO: 387; or,

a first polypeptide chain comprises an amino acid sequence as set forth in SEQ ID NO: 351; and a second polypeptide chain comprises an amino acid sequence as set forth in SEQ ID NO: 401; or,

a first polypeptide chain comprises an amino acid sequence as set forth in SEQ ID NO: 402; and a second polypeptide chain comprises an amino acid sequence as set forth in SEQ ID NO: 305; or,

a first polypeptide chain comprises an amino acid sequence as set forth in SEQ ID NO: 351; and a second polypeptide chain comprises an amino acid sequence as set forth in SEQ ID NO: 403; or,

a first polypeptide chain comprises an amino acid sequence as set forth in SEQ ID NO: 402; and a second polypeptide chain comprises an amino acid sequence as set forth in SEQ ID NO: 409; or,

a first polypeptide chain comprises an amino acid sequence as set forth in SEQ ID NO: 359; and a second polypeptide chain comprises an amino acid sequence as set forth in SEQ ID NO: 404; or,

a first polypeptide chain comprises an amino acid sequence as set forth in SEQ ID NO: 405; and a second polypeptide chain comprises an amino acid sequence as set forth in SEQ ID NO: 315; or,

a first polypeptide chain comprises an amino acid sequence as set forth in SEQ ID NO: 359; and a second polypeptide chain comprises an amino acid sequence as set forth in SEQ ID NO: 406; or,

a first polypeptide chain comprises an amino acid sequence as set forth in SEQ ID NO: 351; and a second polypeptide chain comprises an amino acid sequence as set forth in SEQ ID NO: 376; or,

a first polypeptide chain comprises an amino acid sequence as set forth in SEQ ID NO: 351; and a second polypeptide chain comprises an amino acid sequence as set forth in SEQ ID NO: 377; or,

a first polypeptide chain comprises an amino acid sequence as set forth in SEQ ID NO: 351; and a second polypeptide chain comprises an amino acid sequence as set forth in SEQ ID NO: 397; or,

a first polypeptide chain comprises an amino acid sequence as set forth in SEQ ID NO: 351; and a second polypeptide chain comprises an amino acid sequence as set forth in SEQ ID NO: 398; or,

a first polypeptide chain comprises an amino acid sequence as set forth in SEQ ID NO: 351; and a second polypeptide chain comprises an amino acid sequence as set forth in SEQ ID NO: 399; or,

a first polypeptide chain comprises an amino acid sequence as set forth in SEQ ID NO: 351; and a second polypeptide chain comprises an amino acid sequence as set forth in SEQ ID NO: 400; or,

a first polypeptide chain comprises an amino acid sequence as set forth in SEQ ID NO: 402; and a second polypeptide chain comprises an amino acid sequence as set forth in SEQ ID NO: 401; or,

a first polypeptide chain comprises an amino acid sequence as set forth in SEQ ID NO: 402; and a second polypeptide chain comprises an amino acid sequence as set forth in SEQ ID NO: 403; or,

a first polypeptide chain comprises an amino acid sequence as set forth in SEQ ID NO: 405; and a second polypeptide chain comprises an amino acid sequence as set forth in SEQ ID NO: 404; or,

a first polypeptide chain comprises an amino acid sequence as set forth in SEQ ID NO: 405; and a second polypeptide chain comprises an amino acid sequence as set forth in SEQ ID NO: 406; or,

a first polypeptide chain comprises an amino acid sequence as set forth in SEQ ID NO: 371; and a second polypeptide chain comprises an amino acid sequence as set forth in SEQ ID NO: 486; or,

a first polypeptide chain comprises an amino acid sequence as set forth in SEQ ID NO: 353; and a second polypeptide chain comprises an amino acid sequence as set forth in SEQ ID NO: 460; or,

a first polypeptide chain comprises an amino acid sequence as set forth in SEQ ID NO: 353; and a second polypeptide chain comprises an amino acid sequence as set forth in SEQ ID NO: 461; or,

a first polypeptide chain comprises an amino acid sequence as set forth in SEQ ID NO: 353; and a second polypeptide chain comprises an amino acid sequence as set forth in SEQ ID NO: 462; or,

a first polypeptide chain comprises an amino acid sequence as set forth in SEQ ID NO: 487; and a second polypeptide chain comprises an amino acid sequence as set forth in SEQ ID NO: 488; or,

a first polypeptide chain comprises an amino acid sequence as set forth in SEQ ID NO: 360; and a second polypeptide chain comprises an amino acid sequence as set forth in SEQ ID NO: 455; or,

a first polypeptide chain comprises an amino acid sequence as set forth in SEQ ID NO: 360; and a second polypeptide chain comprises an amino acid sequence as set forth in SEQ ID NO: 456; or,

a first polypeptide chain comprises an amino acid sequence as set forth in SEQ ID NO: 360; and a second polypeptide chain comprises an amino acid sequence as set forth in SEQ ID NO: 457; or,

a first polypeptide chain comprises an amino acid sequence as set forth in SEQ ID NO: 360; and a second polypeptide chain comprises an amino acid sequence as set forth in SEQ ID NO: 458; or,

a first polypeptide chain comprises an amino acid sequence as set forth in SEQ ID NO: 360; and a second polypeptide chain comprises an amino acid sequence as set forth in SEQ ID NO: 459; or,

a first polypeptide chain comprises an amino acid sequence as set forth in SEQ ID NO: 419; and a second polypeptide chain comprises an amino acid sequence as set forth in SEQ ID NO: 412; or,

a first polypeptide chain comprises an amino acid sequence as set forth in SEQ ID NO: 419; and a second polypeptide chain comprises an amino acid sequence as set forth in SEQ ID NO: 414; or,

a first polypeptide chain comprises an amino acid sequence as set forth in SEQ ID NO: 440; and a second polypeptide chain comprises an amino acid sequence as set forth in SEQ ID NO: 428; or,

a first polypeptide chain comprises an amino acid sequence as set forth in SEQ ID NO: 441; and a second polypeptide chain comprises an amino acid sequence as set forth in SEQ ID NO: 428; or,

a first polypeptide chain comprises an amino acid sequence as set forth in SEQ ID NO: 442; and a second polypeptide chain comprises an amino acid sequence as set forth in SEQ ID NO: 428; or,

a first polypeptide chain comprises an amino acid sequence as set forth in SEQ ID NO: 443; and a second polypeptide chain comprises an amino acid sequence as set forth in SEQ ID NO: 428; or,

a first polypeptide chain comprises an amino acid sequence as set forth in SEQ ID NO: 487; and a second polypeptide chain comprises an amino acid sequence as set forth in SEQ ID NO: 520; or,

a first polypeptide chain comprises an amino acid sequence as set forth in SEQ ID NO: 353; and a second polypeptide chain comprises an amino acid sequence as set forth in SEQ ID NO: 521; or,

a first polypeptide chain comprises an amino acid sequence as set forth in SEQ ID NO: 371; and a second polypeptide chain comprises an amino acid sequence as set forth in SEQ ID NO: 522; or,

a first polypeptide chain comprises an amino acid sequence as set forth in SEQ ID NO: 360; and a second polypeptide chain comprises an amino acid sequence as set forth in SEQ ID NO: 523; or,

(2) comprising three polypeptide chains, wherein,

a first polypeptide chain comprises an amino acid sequence as set forth in SEQ ID NO: 353; a second polypeptide chain comprises an amino acid sequence as set forth in SEQ ID NO: 407; and a third polypeptide chain comprises an amino acid sequence as set forth in SEQ ID NO: 390; or,

a first polypeptide chain comprises an amino acid sequence as set forth in SEQ ID NO: 353; a second polypeptide chain comprises an amino acid sequence as set forth in SEQ ID NO: 408; and a third polypeptide chain comprises an amino acid sequence as set forth in SEQ ID NO: 392; or,

a first polypeptide chain comprises an amino acid sequence as set forth in SEQ ID NO: 351; a second polypeptide chain comprises an amino acid sequence as set forth in SEQ ID NO: 391; and a third polypeptide chain comprises an amino acid sequence as set forth in SEQ ID NO: 390; or,

a first polypeptide chain comprises an amino acid sequence as set forth in SEQ ID NO: 351; a second polypeptide chain comprises an amino acid sequence as set forth in SEQ ID NO: 393; and a third polypeptide chain comprises an amino acid sequence as set forth in SEQ ID NO: 392; or,

a first polypeptide chain comprises an amino acid sequence as set forth in SEQ ID NO: 351; a second polypeptide chain comprises an amino acid sequence as set forth in SEQ ID NO: 391; and a third polypeptide chain comprises an amino acid sequence as set forth in SEQ ID NO: 434; or,

a first polypeptide chain comprises an amino acid sequence as set forth in SEQ ID NO: 351; a second polypeptide chain comprises an amino acid sequence as set forth in SEQ ID NO: 391; and a third polypeptide chain comprises an amino acid sequence as set forth in SEQ ID NO: 435; or,

a first polypeptide chain comprises an amino acid sequence as set forth in SEQ ID NO: 351; a second polypeptide chain comprises an amino acid sequence as set forth in SEQ ID NO: 393; and a third polypeptide chain comprises an amino acid sequence as set forth in SEQ ID NO: 436; or,

a first polypeptide chain comprises an amino acid sequence as set forth in SEQ ID NO: 351; a second polypeptide chain comprises an amino acid sequence as set forth in SEQ ID NO: 393; and a third polypeptide chain comprises an amino acid sequence as set forth in SEQ ID NO: 437; or,

a first polypeptide chain comprises an amino acid sequence as set forth in SEQ ID NO: 367; a second polypeptide chain comprises an amino acid sequence as set forth in SEQ ID NO: 438; and a third polypeptide chain comprises an amino acid sequence as set forth in SEQ ID NO: 434; or,

a first polypeptide chain comprises an amino acid sequence as set forth in SEQ ID NO: 367; a second polypeptide chain comprises an amino acid sequence as set forth in SEQ ID NO: 438; and a third polypeptide chain comprises an amino acid sequence as set forth in SEQ ID NO: 435; or,

a first polypeptide chain comprises an amino acid sequence as set forth in SEQ ID NO: 367; a second polypeptide chain comprises an amino acid sequence as set forth in SEQ ID NO: 439; and a third polypeptide chain comprises an amino acid sequence as set forth in SEQ ID NO: 436; or,

a first polypeptide chain comprises an amino acid sequence as set forth in SEQ ID NO: 367; a second polypeptide chain comprises an amino acid sequence as set forth in SEQ ID NO: 439; and a third polypeptide chain comprises an amino acid sequence as set forth in SEQ ID NO: 437; or,

a first polypeptide chain comprises an amino acid sequence as set forth in SEQ ID NO: 361; a second polypeptide chain comprises an amino acid sequence as set forth in SEQ ID NO: 481; and a third polypeptide chain comprises an amino acid sequence as set forth in SEQ ID NO: 482; or,

a first polypeptide chain comprises an amino acid sequence as set forth in SEQ ID NO: 361; a second polypeptide chain comprises an amino acid sequence as set forth in SEQ ID NO: 481; and a third polypeptide chain comprises an amino acid sequence as set forth in SEQ ID NO: 483; or,

a first polypeptide chain comprises an amino acid sequence as set forth in SEQ ID NO: 361; a second polypeptide chain comprises an amino acid sequence as set forth in SEQ ID NO: 481; and a third polypeptide chain comprises an amino acid sequence as set forth in SEQ ID NO: 484; or,

a first polypeptide chain comprises an amino acid sequence as set forth in SEQ ID NO: 361; a second polypeptide chain comprises an amino acid sequence as set forth in SEQ ID NO: 481; and a third polypeptide chain comprises an amino acid sequence as set forth in SEQ ID NO: 485; or,

a first polypeptide chain comprises an amino acid sequence as set forth in SEQ ID NO: 357; a second polypeptide chain comprises an amino acid sequence as set forth in SEQ ID NO: 411; and a third polypeptide chain comprises an amino acid sequence as set forth in SEQ ID NO: 412; or,

a first polypeptide chain comprises an amino acid sequence as set forth in SEQ ID NO: 357; a second polypeptide chain comprises an amino acid sequence as set forth in SEQ ID NO: 411; and a third polypeptide chain comprises an amino acid sequence as set forth in SEQ ID NO: 414; or,

a first polypeptide chain comprises an amino acid sequence as set forth in SEQ ID NO: 415; a second polypeptide chain comprises an amino acid sequence as set forth in SEQ ID NO: 416; and a third polypeptide chain comprises an amino acid sequence as set forth in SEQ ID NO: 410; or,

a first polypeptide chain comprises an amino acid sequence as set forth in SEQ ID NO: 415; a second polypeptide chain comprises an amino acid sequence as set forth in SEQ ID NO: 416; and a third polypeptide chain comprises an amino acid sequence as set forth in SEQ ID NO: 412; or,

a first polypeptide chain comprises an amino acid sequence as set forth in SEQ ID NO: 415; a second polypeptide chain comprises an amino acid sequence as set forth in SEQ ID NO: 416; and a third polypeptide chain comprises an amino acid sequence as set forth in SEQ ID NO: 414; or,

a first polypeptide chain comprises an amino acid sequence as set forth in SEQ ID NO: 417; a second polypeptide chain comprises an amino acid sequence as set forth in SEQ ID NO: 418; and a third polypeptide chain comprises an amino acid sequence as set forth in SEQ ID NO: 412; or,

a first polypeptide chain comprises an amino acid sequence as set forth in SEQ ID NO: 417; a second polypeptide chain comprises an amino acid sequence as set forth in SEQ ID NO: 418; and a third polypeptide chain comprises an amino acid sequence as set forth in SEQ ID NO: 414; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 426; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 427; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 428; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 429; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 449; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 449; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 445; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 446; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 445; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 446; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 447; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 448; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 447; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 448; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 445; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 446; or,

a first polypeptide chain comprises an amino acid sequence as set forth in SEQ ID NO: 357; a second polypeptide chain comprises an amino acid sequence as set forth in SEQ ID NO: 463; and a third polypeptide chain comprises an amino acid sequence as set forth in SEQ ID NO: 464; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 465; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 466; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 467; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 468; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 469; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 470; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 471; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 472; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 473; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 474; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 475; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 476; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 477; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 478; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 479; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 480; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 430; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 431; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 432; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 433.

9. The binding protein according to any one of items 1 to 8, wherein the binding protein can further comprise a light chain constant region, preferably a human light chain constant region, and more preferably a human light chain constant region Cκ or Cλ; and the binding protein can further comprise CH1, CH2 and/or CH3 of a heavy chain constant region, preferably a human heavy chain constant region, and more preferably a human IgG1, IgG2, IgG3 or IgG4 heavy chain constant region, wherein the IgG1 heavy chain constant region can further have one or more of the mutations of C220S, N297A, L234A, L235A, P329G, S239D, I332E, S354C, T366W, Y349C, T366S, L368A, Y407V, M252Y, S254T, T256E and others on Fc, wherein the positions of the mutations are numbered according to an EU numbering scheme.

10. An isolated nucleic acid encoding the binding protein according to any one of items 1 to 9.

11. An expression vector comprising the isolated nucleic acid according to item 10.

12. A host cell comprising the expression vector according to item 11, wherein the host cell is a prokaryotic cell or a eukaryotic cell.

13. A method for preparing the binding protein according to any one of items 1 to 9, comprising the steps of: culturing the host cell as described above and obtaining the binding protein from a culture.

14. A pharmaceutical composition comprising the binding protein according to any one of items 1 to 9.

15. A combination of kits comprising a kit I comprising the binding protein according to any one of items 1 to 9 or the pharmaceutical composition according to item 14, and a kit II comprising an additional antibody or pharmaceutical composition for treating cancer.

16. Use of the binding protein according to any one of items 1 to 9 or the pharmaceutical composition according to item 14 in the preparation of a medicament for treating and/or preventing cancer, wherein preferably, the cancer is breast cancer, ovarian cancer, endometrial cancer, renal cancer, melanoma, lung cancer, gastric cancer, liver cancer, esophageal cancer, cervical cancer, head and neck tumor, cholangiocarcinoma, gallbladder cancer, bladder cancer, sarcoma, colorectal cancer, lymphoma or multiple myeloma.

17. A method for treating cancer, comprising administering the binding protein according to any one of items 1 to 9, or the pharmaceutical composition according to item 14, or the combination of kits according to item 15 to a subject in need thereof; preferably, further comprising administering an additional antibody for treating cancer, such as an immune checkpoint antibody and/or a chemotherapeutic agent.

18. A CD3 antibody, wherein the CD3 antibody comprises a light chain variable region and a heavy chain variable region;

the light chain variable region VL comprises LCDR1, LCDR2 and LCDR3 with amino acid sequences as set forth in SEQ ID NOs: 177, 191 and 221, respectively; the heavy chain variable region VH comprises HCDR1, HCDR2 and HCDR3 with amino acid sequences as set forth in SEQ ID NOs: 15, 84 and 141, respectively;

or, the light chain variable region VL comprises LCDR1, LCDR2 and LCDR3 with amino acid sequences as set forth in SEQ ID NOs: 178, 197 and 222, respectively; the heavy chain variable region VH comprises HCDR1, HCDR2 and HCDR3 with amino acid sequences as set forth in SEQ ID NOs: 31, 85 and 142, respectively;

or, the light chain variable region VL comprises LCDR1, LCDR2 and LCDR3 with amino acid sequences as set forth in SEQ ID NOs: 177, 191 and 223, respectively; the heavy chain variable region VH comprises HCDR1, HCDR2 and HCDR3 with amino acid sequences as set forth in SEQ ID NOs: 31, 86 and 141, respectively;

or, the light chain variable region VL comprises LCDR1, LCDR2 and LCDR3 with amino acid sequences as set forth in SEQ ID NOs: 179, 198 and 224, respectively; the heavy chain variable region VH comprises HCDR1, HCDR2 and HCDR3 with amino acid sequences as set forth in SEQ ID NOs: 31, 85 and 142, respectively;

or, the light chain variable region VL comprises LCDR1, LCDR2 and LCDR3 with amino acid sequences as set forth in SEQ ID NOs: 172, 192 and 216, respectively; the heavy chain variable region VH comprises HCDR1, HCDR2 and HCDR3 with amino acid sequences as set forth in SEQ ID NOs: 20, 68 and 128, respectively;

or, the light chain variable region VL comprises LCDR1, LCDR2 and LCDR3 with amino acid sequences as set forth in SEQ ID NOs: 172, 192 and 216, respectively; the heavy chain variable region VH comprises HCDR1, HCDR2 and HCDR3 with amino acid sequences as set forth in SEQ ID NOs: 30, 68 and 128, respectively;

preferably, the light chain variable region VL comprises the amino acid sequences as set forth in SEQ ID NOs: 294; the heavy chain variable region VH comprises the amino acid sequences as set forth in SEQ ID NOs: 258;

or, the light chain variable region VL comprises the amino acid sequences as set forth in SEQ ID NOs: 295; the heavy chain variable region VH comprises the amino acid sequences as set forth in SEQ ID NOs: 259;

or, the light chain variable region VL comprises the amino acid sequences as set forth in SEQ ID NOs: 296; the heavy chain variable region VH comprises the amino acid sequences as set forth in SEQ ID NOs: 260;

or, the light chain variable region VL comprises the amino acid sequences as set forth in SEQ ID NOs: 297; the heavy chain variable region VH comprises the amino acid sequences as set forth in SEQ ID NOs: 259;

or, the light chain variable region VL comprises the amino acid sequences as set forth in SEQ ID NOs: 291; the heavy chain variable region VH comprises the amino acid sequences as set forth in SEQ ID NOs: 245;

or, the light chain variable region VL comprises the amino acid sequences as set forth in SEQ ID NOs: 291; the heavy chain variable region VH comprises the amino acid sequences as set forth in SEQ ID NOs: 257;

or, the light chain variable region VL comprises the amino acid sequences as set forth in SEQ ID NOs: 291; the heavy chain variable region VH comprises the amino acid sequences as set forth in SEQ ID NOs: 263;

or, the light chain variable region VL comprises the amino acid sequences as set forth in SEQ ID NOs: 291; the heavy chain variable region VH comprises the amino acid sequences as set forth in SEQ ID NOs: 281;

or, the light chain variable region VL comprises the amino acid sequences as set forth in SEQ ID NOs: 291; the heavy chain variable region VH comprises the amino acid sequences as set forth in SEQ ID NOs: 244;

more preferably, the CD3 antibody comprises two polypeptide chains, wherein, a first polypeptide chain comprises an amino acid sequence as set forth in SEQ ID NO: 357; and a second polypeptide chain comprises an amino acid sequence as set forth in SEQ ID NO: 313;

or, a first polypeptide chain comprises an amino acid sequence as set forth in SEQ ID NO: 357; and a second polypeptide chain comprises an amino acid sequence as set forth in SEQ ID NO: 325;

or, a first polypeptide chain comprises an amino acid sequence as set forth in SEQ ID NO: 357; and a second polypeptide chain comprises an amino acid sequence as set forth in SEQ ID NO: 328;

or, a first polypeptide chain comprises an amino acid sequence as set forth in SEQ ID NO: 357; and a second polypeptide chain comprises an amino acid sequence as set forth in SEQ ID NO: 346;

or, the CD3 antibody comprises one polypeptide chain, wherein, the polypeptide chain comprises an amino acid sequence as set forth in SEQ ID NO: 489, an amino acid sequence as set forth in SEQ ID NO: 490, an amino acid sequence as set forth in SEQ ID NO: 491, an amino acid sequence as set forth in SEQ ID NO: 492, or, an amino acid sequence as set forth in SEQ ID NO: 493.

19. A BCMA antibody,wherein the BCMA antibody comprises a heavy chain variable region; the heavy chain variable region VH comprises HCDR1, HCDR2 and HCDR3 with amino acid sequences as set forth in SEQ ID NOs: 15, 75 and 133, respectively;or, the heavy chain variable region VH comprises HCDR1, HCDR2 and HCDR3 with amino acid sequences as set forth in SEQ ID NOs: 24, 76 and 134, respectively; or, the heavy chain variable region VH comprises HCDR1, HCDR2 and HCDR3 with amino acid sequences as set forth in SEQ ID NOs: 25, 77 and 135, respectively; or, the heavy chain variable region VH comprises HCDR1, HCDR2 and HCDR3 with amino acid sequences as set forth in SEQ ID NOs: 26, 78 and 136, respectively;

or, the BCMA antibody comprises a heavy chain variable region; the heavy chain variable region VH comprises HCDR1, HCDR2 and HCDR3 with amino acid sequences as set forth in SEQ ID NOs: 26, 90 and 136, respectively;or, the heavy chain variable region VH comprises HCDR1, HCDR2 and HCDR3 with amino acid sequences as set forth in SEQ ID NOs: 34, 78 and 146, respectively; or, the heavy chain variable region VH comprises HCDR1, HCDR2 and HCDR3 with amino acid sequences as set forth in SEQ ID NOs: 35, 78 and 147, respectively; or, the heavy chain variable region VH comprises HCDR1, HCDR2 and HCDR3 with amino acid sequences as set forth in SEQ ID NOs: 35, 90 and 147, respectively; or, the heavy chain variable region VH comprises HCDR1, HCDR2 and HCDR3 with amino acid sequences as set forth in SEQ ID NOs: 36, 90 and 146, respectively; or, the heavy chain variable region VH comprises HCDR1, HCDR2 and HCDR3 with amino acid sequences as set forth in SEQ ID NOs: 35, 76 and 147, respectively; or, the heavy chain variable region VH comprises HCDR1, HCDR2 and HCDR3 with amino acid sequences as set forth in SEQ ID NOs: 34, 78 and 148, respectively; or, the heavy chain variable region VH comprises HCDR1, HCDR2 and HCDR3 with amino acid sequences as set forth in SEQ ID NOs: 35, 76 and 136, respectively; or, the heavy chain variable region VH comprises HCDR1, HCDR2 and HCDR3 with amino acid sequences as set forth in SEQ ID NOs: 35, 90 and 146, respectively; or, the heavy chain variable region VH comprises HCDR1, HCDR2 and HCDR3 with amino acid sequences as set forth in SEQ ID NOs: 34, 76 and 136, respectively; or, the heavy chain variable region VH comprises HCDR1, HCDR2 and HCDR3 with amino acid sequences as set forth in SEQ ID NOs: 35, 76 and 146, respectively; or, the heavy chain variable region VH comprises HCDR1, HCDR2 and HCDR3 with amino acid sequences as set forth in SEQ ID NOs: 34, 76 and 146, respectively; or, the heavy chain variable region VH comprises HCDR1, HCDR2 and HCDR3 with amino acid sequences as set forth in SEQ ID NOs: 35, 90 and 148, respectively; or, the heavy chain variable region VH comprises HCDR1, HCDR2 and HCDR3 with amino acid sequences as set forth in SEQ ID NOs: 35, 90 and 136, respectively;

preferably, the heavy chain variable region VH comprises the amino acid sequences as set forth in SEQ ID NOs: 248; or the heavy chain variable region VH comprises the amino acid sequences as set forth in SEQ ID NOs: 249; or, the heavy chain variable region VH comprises the amino acid sequences as set forth in SEQ ID NOs: 250; or, the heavy chain variable region VH comprises the amino acid sequences as set forth in SEQ ID NOs: 251;

or, the heavy chain variable region VH comprises the amino acid sequences as set forth in SEQ ID NOs: 265; or, the heavy chain variable region VH comprises the amino acid sequences as set forth in SEQ ID NOs: 266; or, the heavy chain variable region VH comprises the amino acid sequences as set forth in SEQ ID NOs: 267; or, the heavy chain variable region VH comprises the amino acid sequences as set forth in SEQ ID NOs: 268; or, the heavy chain variable region VH comprises the amino acid sequences as set forth in SEQ ID NOs: 269; or, the heavy chain variable region VH comprises the amino acid sequences as set forth in SEQ ID NOs: 270; or, the heavy chain variable region VH comprises the amino acid sequences as set forth in SEQ ID NOs: 271; or, the heavy chain variable region VH comprises the amino acid sequences as set forth in SEQ ID NOs: 272; or, the heavy chain variable region VH comprises the amino acid sequences as set forth in SEQ ID NOs: 273; or, the heavy chain variable region VH comprises the amino acid sequences as set forth in SEQ ID NOs: 274; or, the heavy chain variable region VH comprises the amino acid sequences as set forth in SEQ ID NOs: 275; or, the heavy chain variable region VH comprises the amino acid sequences as set forth in SEQ ID NOs: 276; or, the heavy chain variable region VH comprises the amino acid sequences as set forth in SEQ ID NOs: 277; or, the heavy chain variable region VH comprises the amino acid sequences as set forth in SEQ ID NOs: 278; or, the heavy chain variable region VH comprises the amino acid sequences as set forth in SEQ ID NOs: 279; or, the heavy chain variable region VH comprises the amino acid sequences as set forth in SEQ ID NOs: 280;

more preferably, the BCMA antibody comprises one polypeptide chain, wherein, the polypeptide chain comprises an amino acid sequence as set forth in SEQ ID NO: 316, an amino acid sequence as set forth in SEQ ID NO: 317, an amino acid sequence as set forth in SEQ ID NO: 318, or an amino acid sequence as set forth in SEQ ID NO: 319;

or, the BCMA antibody comprises one polypeptide chain,wherein,the polypeptide chain comprises an amino acid sequence as set forth in SEQ ID NO: 330,or, an amino acid sequence as set forth in SEQ ID NO: 331, or, an amino acid sequence as set forth in SEQ ID NO: 332, or, an amino acid sequence as set forth in SEQ ID NO: 333, or, an amino acid sequence as set forth in SEQ ID NO: 334, or, an amino acid sequence as set forth in SEQ ID NO: 335, or, an amino acid sequence as set forth in SEQ ID NO: 336, or, an amino acid sequence as set forth in SEQ ID NO: 337, or, an amino acid sequence as set forth in SEQ ID NO: 338, or, an amino acid sequence as set forth in SEQ ID NO: 339, or, an amino acid sequence as set forth in SEQ ID NO: 340, or, an amino acid sequence as set forth in SEQ ID NO: 341, or, an amino acid sequence as set forth in SEQ ID NO: 342, or, an amino acid sequence as set forth in SEQ ID NO: 343, or, an amino acid sequence as set forth in SEQ ID NO: 344, or, an amino acid sequence as set forth in SEQ ID NO: 345.

**Claims**

1. A bispecific antibody comprising at least two protein functional regions, wherein the bispecific antibody comprises a protein functional region A and a protein functional region B, wherein the protein functional region A is a CD3-binding domain of a Fab or scFv structure, the protein functional region B is a BCMA-binding domain of a VH structure, and the number of each of the protein functional region A and the protein functional region B is one or more, wherein: the BCMA-binding domain comprises a heavy chain variable region, wherein the heavy chain variable region VH comprises HCDR1, HCDR2 and HCDR3 with amino acid sequences as set forth in SEQ ID NOs: 26, 78 and 136, respectively; or the heavy chain variable region VH comprises HCDR1, HCDR2 and HCDR3 with amino acid sequences as set forth in SEQ ID NOs: 26, 90 and 136; or, the heavy chain variable region VH comprises HCDR1, HCDR2 and HCDR3 with amino acid sequences as set forth in SEQ ID NOs: 36, 90 and 146, respectively; or, the heavy chain variable region VH comprises HCDR1, HCDR2 and HCDR3 with amino acid sequences as set forth in SEQ ID NOs: 35, 76 and 147, respectively; or, the heavy chain variable region VH comprises HCDR1, HCDR2 and HCDR3 with amino acid sequences as set forth in SEQ ID NOs: 34, 78 and 148, respectively; or, the heavy chain variable region VH comprises HCDR1, HCDR2 and HCDR3 with amino acid sequences as set forth in SEQ ID NOs: 35, 76 and 136, respectively; or, the heavy chain variable region VH comprises HCDR1, HCDR2 and HCDR3 with amino acid sequences as set forth in SEQ ID NOs: 35, 90 and 146, respectively; or, the heavy chain variable region VH comprises HCDR1, HCDR2 and HCDR3 with amino acid sequences as set forth in SEQ ID NOs: 34, 76 and 136, respectively; or, the heavy chain variable region VH comprises HCDR1, HCDR2 and HCDR3 with amino acid sequences as set forth in SEQ ID NO 35, 76 and 146, respectively; or, the heavy chain variable region VH comprises HCDR1, HCDR2 and HCDR3 with amino acid sequences as set forth in SEQ ID NOs: 34, 76 and 146, respectively; or, the heavy chain variable region VH comprises HCDR1, HCDR2 and HCDR3 with amino acid sequences as set forth in SEQ ID NOs: 35, 90 and 148, respectively; or, the heavy chain variable region VH comprises HCDR1, HCDR2 and HCDR3 with amino acid sequences as set forth in SEQ ID NOs: 35, 90 and 136, respectively; or, the heavy chain variable region VH comprises HCDR1, HCDR2 and HCDR3 with amino acid sequences as set forth in SEQ ID NOs: 34, 78 and 146, respectively; or, the heavy chain variable region VH comprises HCDR1, HCDR2 and HCDR3 with amino acid sequences as set forth in SEQ ID NOs: 35, 78 and 147; or, the heavy chain variable region VH comprises HCDR1, HCDR2 and HCDR3 with amino acid sequences as set forth in SEQ ID NOs: 35, 90 and 147, respectively; or, the heavy chain variable region VH comprises HCDR1, HCDR2 and HCDR3 with amino acid sequences as set forth in SEQ ID NOs: 15, 75 and 133, respectively; or, the heavy chain variable region VH comprises HCDR1, HCDR2 and HCDR3 with amino acid sequences set forth in SEQ ID NOs: 24, 76 and 134, respectively; or, the heavy chain variable region VH comprises HCDR1, HCDR2 and HCDR3 with amino acid sequences set forth in SEQ ID NOs: 25, 77 and 135, respectively.

2. The bispecific antibody according to claim 1, wherein the CD3-binding domain comprises a light chain variable region and a heavy chain variable region, wherein: the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; or the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 20, 68 and 128, respectively; or the light chain variable region VL comprises LCDR1, LCDR2 and LCDR3 with amino acid sequences as set forth in SEQ ID NOs: 177, 191 and 221, respectively; and the heavy chain variable region VH comprises HCDR1, HCDR2 and HCDR3 with amino acid sequences as set forth in SEQ ID NOs: 15, 84 and 141, respectively; or, the light chain variable region VL comprises LCDR1, LCDR2 and LCDR3 with amino acid sequences as set forth in SEQ ID NOs: 178, 197 and 222; and the heavy chain variable region VH comprises HCDR1, HCDR2 and HCDR3 with amino acid sequences as set forth in SEQ ID NOs: 31, 85 and 142, respectively; or, the light chain variable region VL comprises LCDR1, LCDR2 and LCDR3 with amino acid sequences as set forth in SEQ ID NOs: 177, 191 and 223, respectively; and the heavy chain variable region VH comprises HCDR1, HCDR2 and HCDR3 with amino acid sequences as set forth in SEQ ID NOs: 31, 86 and 141, respectively; or, the light chain variable region VL comprises LCDR1, LCDR2 and LCDR3 with amino acid sequences as set forth in SEQ ID NOs: 179, 198 and 224, respectively; and the heavy chain variable region VH comprises HCDR1, HCDR2 and HCDR3 with amino acid sequences as set forth in SEQ ID NOs: 31, 85 and 142, respectively.

3. The bispecific antibody according to claim 1 or 2, wherein the BCMA-binding domain comprises a heavy chain variable region, wherein the heavy chain variable region VH comprises an amino acid sequence as set forth in SEQ ID NO: 251; or the heavy chain variable region VH comprises an amino acid sequence as set forth in SEQ ID NO: 265; or, the heavy chain variable region VH comprises an amino acid sequence as set forth in SEQ ID NO: 269; or, the heavy chain variable region VH comprises an amino acid sequence as set forth in SEQ ID NO: 270; or, the heavy chain variable region VH comprises an amino acid sequence as set forth in SEQ ID NO: 271; or, the heavy chain variable region VH comprises an amino acid sequence as set forth in SEQ ID NO: 272; or, the heavy chain variable region VH comprises an amino acid sequence as set forth in SEQ ID NO: 273; or, the heavy chain variable region VH comprises an amino acid sequence as set forth in SEQ ID NO: 274; or, the heavy chain variable region VH comprises an amino acid sequence as set forth in SEQ ID NO: 275; or, the heavy chain variable region VH comprises an amino acid sequence as set forth in SEQ ID NO: 276; or, the heavy chain variable region VH comprises an amino acid sequence as set forth in SEQ ID NO: 277; or, the heavy chain variable region VH comprises an amino acid sequence as set forth in SEQ ID NO: 278; or, the heavy chain variable region VH comprises an amino acid sequence as set forth in SEQ ID NO: 279; or, the heavy chain variable region VH comprises an amino acid sequence as set forth in SEQ ID NO: 280; or, the heavy chain variable region VH comprises an amino acid sequence as set forth in SEQ ID NO: 266; or, the heavy chain variable region VH comprises an amino acid sequence as set forth in SEQ ID NO: 267; or, the heavy chain variable region VH comprises an amino acid sequence as set forth in SEQ ID NO: 268; or the heavy chain variable region VH comprises an amino acid sequence as set forth in SEQ ID NO: 248; or, the heavy chain variable region VH comprises an amino acid sequence as set forth in SEQ ID NO: 249; or, the heavy chain variable region VH comprises an amino acid sequence as set forth in SEQ ID NO: 250.

4. The bispecific antibody according to any one of claims 1-3, wherein the CD3-domain comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; or, the light chain variable region VL comprises an amino acid sequence as set forth in SEQ ID NO: 291; and the heavy chain variable region VH comprises an amino acid sequence as set forth in SEQ ID NO: 257; or, the light chain variable region VL comprises an amino acid sequence as set forth in SEQ ID NO: 291; and the heavy chain variable region VH comprises an amino acid sequence as set forth in SEQ ID NO: 281; or the light chain variable region VL comprises an amino acid sequence as set forth in SEQ ID NO: 291; and the heavy chain variable region VH comprises an amino acid sequence as set forth in SEQ ID NO: 245, respectively; or, the light chain variable region VL comprises an amino acid sequence as set forth in SEQ ID NO: 291; and the heavy chain variable region VH comprises an amino acid sequence as set forth in SEQ ID NO: 244; or,
the light chain variable region VL comprises an amino acid sequence as set forth in SEQ ID NO: 294; and the heavy chain variable region VH comprises an amino acid sequence as set forth in SEQ ID NO: 258; or, the light chain variable region VL comprises an amino acid sequence as set forth in SEQ ID NO: 295; and the heavy chain variable region VH comprises an amino acid sequence as set forth in SEQ ID NO: 259; or, the light chain variable region VL comprises an amino acid sequence as set forth in SEQ ID NO: 296; and the heavy chain variable region VH comprises an amino acid sequence as set forth in SEQ ID NO: 260; or, the light chain variable region VL comprises an amino acid sequence as set forth in SEQ ID NO: 297; and the heavy chain variable region VH comprises an amino acid sequence as set forth in SEQ ID NO: 259.

5. The bispecific antibody according to any one of claims 1 to 4, wherein:

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,
the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 26, 90 and 136, respectively; or,
the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 36, 90 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 76 and 147, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 34, 78 and 148, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 76 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 90 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 34, 76 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 76 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 34, 76 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 90 and 148, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 90 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 34, 78 and 146, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 78 and 147, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 35, 90 and 147, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 20, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 15, 75 and 133, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 20, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 24, 76 and 134, respectively; or,the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 20, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 25, 77 and 135, respectively,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 20, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises a heavy chain variable region, wherein the heavy chain variable region VH comprises HCDR1, HCDR2 and HCDR3 with amino acid sequences as set forth in SEQ ID NOs: 15, 75 and 133, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B comprises comprises a heavy chain variable region, wherein the heavy chain variable region VH comprises HCDR1, HCDR2 and HCDR3 with amino acid sequences as set forth in SEQ ID NOs: 25, 77 and 135, respectively, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 177, 191 and 221, respectively; and the heavy chain variable region VH comprises HCDR1, HCDR2 and HCDR3 with amino acid sequences as set forth in SEQ ID NOs: 15, 84 and 141, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 178, 197 and 222, respectively; and the heavy chain variable region VH comprises HCDR1, HCDR2 and HCDR3 with amino acid sequences as set forth in SEQ ID NOs: 31, 85 and 142, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 177, 191 and 223, respectively; and the heavy chain variable region VH comprises HCDR1, HCDR2 and HCDR3 with amino acid sequences as set forth in SEQ ID NOs: 31, 86 and 141, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or,

the protein functional region A comprises a light chain variable region and a heavy chain variable region, wherein the light chain variable region VL comprises LCDR1, LCDR2 and LCDR3 with amino acid sequences as set forth in SEQ ID NOs: 179, 198 and 224, respectively; and the heavy chain variable region VH comprises HCDR1, HCDR2 and HCDR3 with amino acid sequences as set forth in SEQ ID NOs: 31, 85 and 142, respectively; and the protein functional region B comprises a heavy chain variable region, wherein the heavy chain variable region VH comprises HCDR1, HCDR2 and HCDR3 with amino acid sequences as set forth in SEQ ID NOs: 26, 78 and 136, respectively; or

optionally, the bispecific antibody further comprises a protein functional region C, wherein the protein functional region C is a BCMA-binding domain of a VH structure, wherein:

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B and the protein functional region C independently comprise a heavy chain variable region, wherein the heavy chain variable region VH is selected from: (1) a VH that comprises HCDR1, HCDR2 and HCDR3 with amino acid sequences as set forth in SEQ ID NOs: 15, 75 and 133, respectively; and (2) a VH that comprises HCDR1, HCDR2 and HCDR3 with amino acid sequences as set forth in SEQ ID NOs: 24, 76 and 134, respectively; or

the protein functional region A comprises a light chain variable region and a heavy chain variable region, wherein the light chain variable region VL comprises LCDR1, LCDR2 and LCDR3 with amino acid sequences as set forth in SEQ ID NOs: 172, 192 and 216, respectively; and the heavy chain variable region VH comprises HCDR1, HCDR2 and HCDR3 with amino acid sequences as set forth in SEQ ID NOs: 30, 68 and 128, respectively; and the protein functional region B and the protein functional region C independently comprise a heavy chain variable region, wherein the heavy chain variable region VH is selected from: (1) a VH that comprises HCDR1, HCDR2 and HCDR3 with amino acid sequences as set forth in SEQ ID NOs: 25, 77 and 135, respectively; and (2) a VH that comprises HCDR1, HCDR2 and HCDR3 with amino acid sequences as set forth in SEQ ID NOs: 26, 78 and 136, respectively.

6. The bispecific antibody according to any one of claims 1 to 5, wherein:

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 265; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 269; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 270; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 271; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 272; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 273; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 274; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 275; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 276; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 277; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 278; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 279; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 280; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 266; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 267; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 263; and the protein functional region B comprises a heavy chain variable region, wherein the heavy chain variable region VH comprises an amino acid sequence as set forth in SEQ ID NO: 268; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 257; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 281; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 245; and the protein functional region B comprises a heavy chain variable region, wherein the heavy chain variable region VH comprises an amino acid sequence as set forth in SEQ ID NO: 248; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 245; and the protein functional region B comprises a heavy chain variable region, wherein the heavy chain variable region VH comprises an amino acid sequence as set forth in SEQ ID NO: 249; or

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 245; and the protein functional region B comprises a heavy chain variable region, wherein the heavy chain variable region VH comprises an amino acid sequence as set forth in SEQ ID NO: 250; or

the protein functional region A comprises a light chain variable region and a heavy chain variable region, wherein

the light chain variable region VL comprises an amino acid sequence as set forth in SEQ ID NO: 291; and the heavy chain variable region VH comprises an amino acid sequence as set forth in SEQ ID NO: 245; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 257; and the protein functional region B comprises a heavy chain variable region, wherein the heavy chain variable region VH comprises an amino acid sequence as set forth in SEQ ID NO: 248; or,

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 257; and the protein functional region B comprises a heavy chain variable region, wherein the heavy chain variable region VH comprises an amino acid sequence as set forth in SEQ ID NO: 250; or

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 244; and the protein functional region B comprises a heavy chain variable region, wherein the heavy chain variable region VH comprises an amino acid sequence as set forth in SEQ ID NO: 248; or

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 244; and the protein functional region B comprises a heavy chain variable region, wherein the heavy chain variable region VH comprises an amino acid sequence as set forth in SEQ ID NO: 249; or

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 244; and the protein functional region B comprises a heavy chain variable region, wherein the heavy chain variable region VH comprises an amino acid sequence as set forth in SEQ ID NO: 250; or

the protein functional region A comprises a light chain variable region and a heavy chain variable region, wherein the light chain variable region VL comprises an amino acid sequence as set forth in SEQ ID NO: 291; and the heavy chain variable region VH comprises an amino acid sequence as set forth in SEQ ID NO: 244; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 294; and the heavy chain variable region VH comprises an amino acid sequence as set forth in SEQ ID NO: 258; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 295; and the heavy chain variable region VH comprises an amino acid sequence as set forth in SEQ ID NO: 259; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 296; and the heavy chain variable region VH comprises an amino acid sequence as set forth in SEQ ID NO: 260; and the protein functional region B comprises a heavy chain variable region, wherein the heavy chain variable region VH comprises an amino acid sequence as set forth in SEQ ID NO: 251; or,

the protein functional region A comprises a light chain variable region and a heavy chain variable region, wherein the light chain variable region VL comprises an amino acid sequence as set forth in SEQ ID NO: 297; and the heavy chain variable region VH comprises an amino acid sequence as set forth in SEQ ID NO: 259; and the protein functional region B comprises a heavy chain variable region, wherein the heavy chain variable region VH comprises an amino acid sequence as set forth in SEQ ID NO: 251,

optionally, the bispecific antibody further comprises a protein functional region C, wherein the protein functional region C is a BCMA-binding domain of a VH structure, wherein:

the protein functional region A comprises a light chain variable region and a heavy chain variable region, wherein the light chain variable region VL comprises an amino acid sequence as set forth in SEQ ID NO: 291, and the heavy chain variable region VH comprises an amino acid sequence as set forth in SEQ ID NO: 257; and the protein functional region B and the protein functional region C independently comprises a heavy chain variable region, wherein the heavy chain variable region VH is selected from: (1) a VH that comprises an amino acid sequence as set forth in SEQ ID NO: 248; and (2) a VH that comprises an amino acid sequence as set forth in SEQ ID NO: 249; or

the protein functional region A comprises a light chain variable region and a heavy chain variable region, wherein the light chain variable region VL comprises an amino acid sequence as set forth in SEQ ID NO: 291, and the heavy chain variable region VH comprises an amino acid sequence as set forth in SEQ ID NO: 257; and the protein functional region B and the protein functional region C independently comprises a heavy chain variable region, wherein the heavy chain variable region VH is selected from: (1) a VH that comprises an amino acid sequence as set forth in SEQ ID NO: 250; and (2) a VH that comprises an amino acid sequence as set forth in SEQ ID NO: 251.

7. The bispecific antibody according to any one of claims 1 to 6, wherein:

(A) the bispecific antibody has three polypeptide chains, including one polypeptide chain 1, one polypeptide chain 2 and one polypeptide chain 3, wherein (14) the polypeptide chain 1 comprises VL_A-CL sequentially from the N-terminus to the C-terminus, the polypeptide chain 2 comprises VH_A-CH1-h-CH2-CH3 sequentially from the N-terminus to the C-terminus, and the polypeptide chain 3 comprises VH_B-L-VH_B-h-CH2-CH3 sequentially from the N-terminus to the C-terminus; or (11) the polypeptide chain 1 comprises VL_A-CL sequentially from the N-terminus to the C-terminus, the polypeptide chain 2 comprises VH_A-CH1-h-CH2-CH3 sequentially from the N-terminus to the C-terminus, and the polypeptide chain 3 comprises VH_B-h-CH2-CH3 sequentially from the N-terminus to the C-terminus; or (12) the polypeptide chain 1 comprises VH_A-CL sequentially from the N-terminus to the C-terminus, the polypeptide chain 2 comprises VL_A-CH1-h-CH2-CH3 sequentially from the N-terminus to the C-terminus, and the polypeptide chain 3 comprises VH_B-h-CH2-CH3 sequentially from the N-terminus to the C-terminus; or (13) the polypeptide chain 1 comprises VH_A-CH1 sequentially from the N-terminus to the C-terminus, the polypeptide chain 2 comprises VL_A-CL-h-CH2-CH3 sequentially from the N-terminus to the C-terminus, and the polypeptide chain 3 comprises VH_B-h-CH2-CH3 sequentially from the N-terminus to the C-terminus; or (15) the polypeptide chain 1 comprises VL_A-CL sequentially from the N-terminus to the C-terminus, the polypeptide chain 2 comprises VH_A-CH1-h-CH2-CH3 sequentially from the N-terminus to the C-terminus, and the polypeptide chain 3 comprises VH_C-L-VH_B-h-CH2-CH3 sequentially from the N-terminus to the C-terminus;

(B) the bispecific antibody has two polypeptide chains, including one polypeptide chain 1 and one polypeptide chain 2, wherein (23) the polypeptide chain 1 comprises VH_A-L1-VL_Ah-CH2-CH3 sequentially from the N-terminus to the C-terminus, and the polypeptide chain 2 comprises VH_B-L2-VH_B-h-CH2-CH3 sequentially from the N-terminus to the C-terminus; or (20) the polypeptide chain 1 comprises VL_A-L-VH_A-h-CH2-CH3 sequentially from the N-terminus to the C-terminus, and the polypeptide chain 2 comprises VH_B-h-CH2-CH3 sequentially from the N-terminus to the C-terminus; wherein the L, L1 and L2 are linker peptides, and the h is a hinge region or linker peptide such as "-", GS or an amino acid sequence as set forth in any one of SEQ ID NOs: 495-519; or,

wherein the "L", and "h" are independently selected from"-", GS or an amino acid sequence as set forth in any one of SEQ ID NOs: 495-519, the "VH_A" is the heavy chain variable region of the protein functional region A, the "VL_A" is the light chain variable region of the the protein functional region A, the "VH_B" is the heavy chain variable region of the protein functional region B, and the "VH_C" is the heavy chain variable region of the protein functional region C.

8. The bispecific antibody according to any one of claims 1 to 7, wherein the bispecific antibody comprises:

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 445; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 446; or,

a first polypeptide chain comprises an amino acid sequence as set forth in SEQ ID NO: 357; a second polypeptide chain comprises an amino acid sequence as set forth in SEQ ID NO: 463; and a third polypeptide chain comprises an amino acid sequence as set forth in SEQ ID NO: 464; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 465; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 466; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 467; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 468; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 469; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 470; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 471; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 472; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 473; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 474; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 475; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 476; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 477; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 478; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 479; or,

a first polypeptide chain comprises an amino acid sequence as set forth in SEQ ID NO: 357; a second polypeptide chain comprises an amino acid sequence as set forth in SEQ ID NO: 454; and a third polypeptide chain comprises an amino acid sequence as set forth in SEQ ID NO: 480; or

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 427; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 428; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 429; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 445; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 446; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 447; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 448; or,

a first polypeptide chain comprises an amino acid sequence as set forth in SEQ ID NO: 357; a second polypeptide chain comprises an amino acid sequence as set forth in SEQ ID NO: 444; and a third polypeptide chain comprises an amino acid sequence as set forth in SEQ ID NO: 449; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 445; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 446; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 447; or,

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 448; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 433; or

a first polypeptide chain comprises an amino acid sequence as set forth in SEQ ID NO: 357; a second polypeptide chain comprises an amino acid sequence as set forth in SEQ ID NO: 450; and a third polypeptide chain comprises an amino acid sequence as set forth in SEQ ID NO: 449; or,

a first polypeptide chain comprises an amino acid sequence as set forth in SEQ ID NO: 357; a second polypeptide chain comprises an amino acid sequence as set forth in SEQ ID NO: 411; and a third polypeptide chain comprises an amino acid sequence as set forth in SEQ ID NO: 410; or

a first polypeptide chain comprises an amino acid sequence as set forth in SEQ ID NO: 415; a second polypeptide chain comprises an amino acid sequence as set forth in SEQ ID NO: 416; and a third polypeptide chain comprises an amino acid sequence as set forth in SEQ ID NO: 410; or,

a first polypeptide chain comprises an amino acid sequence as set forth in SEQ ID NO: 417; a second polypeptide chain comprises an amino acid sequence as set forth in SEQ ID NO: 418; and a third polypeptide chain comprises an amino acid sequence as set forth in SEQ ID NO: 410; or

a first polypeptide chain comprises an amino acid sequence as set forth in SEQ ID NO: 357; a second polypeptide chain comprises an amino acid sequence as set forth in SEQ ID NO: 411; and a third polypeptide chain comprises an amino acid sequence as set forth in SEQ ID NO: 412; or,

a first polypeptide chain comprises an amino acid sequence as set forth in SEQ ID NO: 415; a second polypeptide chain comprises an amino acid sequence as set forth in SEQ ID NO: 416; and a third polypeptide chain comprises an amino acid sequence as set forth in SEQ ID NO: 412; or,

a first polypeptide chain comprises an amino acid sequence as set forth in SEQ ID NO: 417; a second polypeptide chain comprises an amino acid sequence as set forth in SEQ ID NO: 418; and a third polypeptide chain comprises an amino acid sequence as set forth in SEQ ID NO: 412; or,

a first polypeptide chain comprises an amino acid sequence as set forth in SEQ ID NO: 357; a second polypeptide chain comprises an amino acid sequence as set forth in SEQ ID NO: 411; and a third polypeptide chain comprises an amino acid sequence as set forth in SEQ ID NO: 413; or

a first polypeptide chain comprises an amino acid sequence as set forth in SEQ ID NO: 415; a second polypeptide chain comprises an amino acid sequence as set forth in SEQ ID NO: 416; and a third polypeptide chain comprises an amino acid sequence as set forth in SEQ ID NO: 413; or

a first polypeptide chain comprises an amino acid sequence as set forth in SEQ ID NO: 417; a second polypeptide chain comprises an amino acid sequence as set forth in SEQ ID NO: 418; and a third polypeptide chain comprises an amino acid sequence as set forth in SEQ ID NO: 413; or

a first polypeptide chain comprises an amino acid sequence as set forth in SEQ ID NO: 357; a second polypeptide chain comprises an amino acid sequence as set forth in SEQ ID NO: 411; and a third polypeptide chain comprises an amino acid sequence as set forth in SEQ ID NO: 414; or,

a first polypeptide chain comprises an amino acid sequence as set forth in SEQ ID NO: 415; a second polypeptide chain comprises an amino acid sequence as set forth in SEQ ID NO: 416; and a third polypeptide chain comprises an amino acid sequence as set forth in SEQ ID NO: 414; or,

a first polypeptide chain comprises an amino acid sequence as set forth in SEQ ID NO: 417; a second polypeptide chain comprises an amino acid sequence as set forth in SEQ ID NO: 418; and a third polypeptide chain comprises an amino acid sequence as set forth in SEQ ID NO: 414; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 424; or

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 425; or

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 426; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 430; or,

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 431; or

a first polypeptide chain comprises an amino acid sequence as set forth in SEQ ID NO: 357; a second polypeptide chain comprises an amino acid sequence as set forth in SEQ ID NO: 423; and a third polypeptide chain comprises an amino acid sequence as set forth in SEQ ID NO: 432; or

a first polypeptide chain comprising an amino acid sequence as set forth in SEQ ID NO: 419; and a second polypeptide chain comprising an amino acid sequence as set forth in SEQ ID NO: 410; or

a first polypeptide chain comprises an amino acid sequence as set forth in SEQ ID NO: 419; and a second polypeptide chain comprises an amino acid sequence as set forth in SEQ ID NO: 412; or

a first polypeptide chain comprises an amino acid sequence as set forth in SEQ ID NO: 419; and a second polypeptide chain comprises an amino acid sequence as set forth in SEQ ID NO: 413; or

a first polypeptide chain comprises an amino acid sequence as set forth in SEQ ID NO: 419; and a second polypeptide chain comprises an amino acid sequence as set forth in SEQ ID NO: 414; or

a first polypeptide chain comprises an amino acid sequence as set forth in SEQ ID NO: 440; and a second polypeptide chain comprises an amino acid sequence as set forth in SEQ ID NO: 428; or

a first polypeptide chain comprises an amino acid sequence as set forth in SEQ ID NO: 441; and a second polypeptide chain comprises an amino acid sequence as set forth in SEQ ID NO: 428; or

a first polypeptide chain comprises an amino acid sequence as set forth in SEQ ID NO: 442; and a second polypeptide chain comprises an amino acid sequence as set forth in SEQ ID NO: 428; or

a first polypeptide chain comprises an amino acid sequence as set forth in SEQ ID NO: 443; and a second polypeptide chain comprises an amino acid sequence as set forth in SEQ ID NO: 428.

9. The bispecific antibody according to any one of claims 1 to 7, wherein the bispecific antibody further comprises a light chain constant region, preferably a human light chain constant region, and more preferably a human light chain constant region Cκ or Cλ; and the bispecific antibody further comprises CH1, CH2 and/or CH3 of a heavy chain constant region, preferably a human heavy chain constant region, and more preferably a human IgG1, IgG2, IgG3 or IgG4 heavy chain constant region, wherein the IgG1 heavy chain constant region preferably further has one or more of the mutations of C220S, N297A, L234A, L235A, P329G, S239D, I332E, S354C, T366W, Y349C, T366S, L368A, Y407V, M252Y, S254T, T256E and others on Fc, wherein the positions of the mutations are numbered according to an EU numbering scheme.

10. An isolated nucleic acid encoding the bispecific antibody according to any one of claims 1 to 9.

11. An expression vector comprising the isolated nucleic acid according to claim 10.

12. A host cell comprising the expression vector according to claim 11, wherein the host cell is a prokaryotic cell or a eukaryotic cell.

13. A pharmaceutical composition comprising the bispecific antibody according to any one of claims 1 to 9.

14. The bispecific antibody according to any one of claims 1 to 9 or the composition according to claim 13 for use in treating and/or preventing cancer.

15. The bispecific antibody or composition for use according to claim 14, wherein the cancer is multiple myeloma, breast cancer, ovarian cancer, endometrial cancer, renal cancer, melanoma, lung cancer, gastric cancer, liver cancer, esophageal cancer, cervical cancer, head and neck tumor, cholangiocarcinoma, gallbladder cancer, bladder cancer, sarcoma, colorectal cancer, or lymphoma.

**(1)**

**Structure (1)**  Fab(CL)-VH-Fc

Short chain   N′ — [ VH_A | CH1 ] — C′

Long chain    N′ — [ VL_A | CL | L1 | VH_B | L2 | CH2 | CH3 ] — C′

First linker peptide    Second linker peptide

**(2)**

**Structure (2)**  Fab(CH1)-VH-Fc

Short chain   N′ — [ VL_A | CL ] — C′

Long chain    N′ — [ VH_A | CH1 | L1 | VH_B | L2 | CH2 | CH3 ] — C′

First linker peptide    Second linker peptide

**(3)**

## Structure (3)    VH-IgG_HC

Short chain    N' — | VL_A | CL | — C'

Long chain    N' — | VH_B | L | VH_A | CH1 | h | CH2 | CH3 | — C'

Linker peptide          Hinge region

**(4)**

## Structure (4)    VH-IgG_LC

Linker peptide

Short chain    N' — | VH_B | L | VL_A | CL | — C'

Long chain    N' — | VH_A | CH1 | h | CH2 | CH3 | — C'

Hinge region

(5)

# Structure (5) IgG_HC-VH

Short chain   N' ─┤ VL_A │ CL ├─ C'

Long chain   N' ─┤ VH_A │ CH1 │ h │ CH2 │ CH3 │ L │ VH_B ├─ C'

Hinge region          Linker peptide

(6)

# Structure (6) IgG_LC-VH

Linker peptide

Short chain   N' ─┤ VL_A │ CL │ L │ VH_B ├─ C'

Long chain   N' ─┤ VH_A │ CH1 │ h │ CH2 │ CH3 ├─ C'

Hinge region

**(7)**

**Structure (7)** IgG_HC-VH-VH

Short chain  N′ — | VL_A | CL | — C′

Long chain  N′ — | VH_A | CH1 | h | CH2 | CH3 | L1 | VH_B | L2 | VH_B | — C′

Hinge region    First linker peptide    Second linker peptide

**(8)**

**Structure (8)** IgG_HC-VH′-VH″

Short chain  N′ — | VL_A | CL | — C′

Long chain  N′ — | VH_A | CH1 | h | CH2 | CH3 | L1 | VH_B | L2 | VH_C | — C′

Hinge region    First linker peptide    Second linker peptide

**(9)**

## Structure (9)   2xVH-IgG(HC+LC)

First linker
peptide

Short chain   N′—| VH_B | L1 | VL_A | CL |— C′

Long chain   N′—| VH_B | L2 | VH_A | CH1 | h | CH2 | CH3 |— C′

Second linker          Hinge region
peptide

**(10)**

## Structure (10)   (VH′+VH″)-IgG(HC+LC)

First linker
peptide

Short chain   N′—| VH_B | L1 | VL_A | CL |— C′

Long chain   N′—| VH_C | L2 | VH_A | CH1 | h | CH2 | CH3 |— C′

Second linker          Hinge region
peptide

**(11)**

## Structure (11)   Fab-Fc-VH

Polypeptide chain-1    N' — | VL_A | CL | — C'

Polypeptide chain-2    N' — | VH_A | CH1 | h | CH2 | CH3 | — C'

Polypeptide chain-3    N' — | VH_B | h | CH2 | CH3 | — C'

Hinge region
(or linker peptide)

**(12)**

## Structure (12)   Fab(cross VH/VL)-Fc-VH

Polypeptide chain-1    N' — | VH_A | CL | — C'

Polypeptide chain-2    N' — | VL_A | CH1 | h | CH2 | CH3 | — C'

Polypeptide chain-3    N' — | VH_B | h | CH2 | CH3 | — C'

Hinge region
(or linker peptide)

**(13)**

## Structure (13)  Fab(cross Fd/LC)-Fc-VH

Polypeptide chain-1    N'—[ VH_A | CH1 ]—C'

Polypeptide chain-2    N'—[ VL_A | CL | h | CH2 | CH3 ]—C'

Polypeptide chain-3    N'—[ VH_B | h | CH2 | CH3 ]—C'

Hinge region
(or linker peptide)

**(14)**

## Structure (14)  Fab-Fc-VH-VH

Polypeptide chain-1    N'—[ VL_A | CL ]—C'

Polypeptide chain-2    N'—[ VH_A | CH1 | h | CH2 | CH3 ]—C'

Polypeptide chain-3    N'—[ VH_B | L | VH_B | h | CH2 | CH3 ]—C'

Linker peptide      Hinge region
(or linker peptide)

**(15)**

### Structure (15)   Fab-Fc-VH'-VH"

Polypeptide chain-1   N' — | VL_A | CL | — C'

Polypeptide chain-2   N' — | VH_A | CH1 | h | CH2 | CH3 | — C'

Polypeptide chain-3   N' — | VH_C | L | VH_B | h | CH2 | CH3 | — C'

Linker peptide       Hinge region
                     (or linker peptide)

**(16)**

### Structure (16)   Fab(cross VH/VL)-Fc-VH-VH

Polypeptide chain-1   N' — | VH_A | CL | — C'

Polypeptide chain-2   N' — | VL_A | CH1 | h | CH2 | CH3 | — C'

Polypeptide chain-3   N' — | VH_B | L | VH_B | h | CH2 | CH3 | — C'

Linker peptide       Hinge region
                     (or linker peptide)

**(17)**

**Structure (17)**   Fab(cross VH/VL)-Fc-VH′-VH″

Polypeptide chain-1   N′ — | VH_A | CL | — C′

Polypeptide chain-2   N′ — | VL_A | CH1 | h | CH2 | CH3 | — C′

Polypeptide chain-3   N′ — | VH_C | L | VH_B | h | CH2 | CH3 | — C′

Linker peptide       Hinge region
                     (or linker peptide)

**(18)**

**Structure (18)**   Fab(cross Fd/LC)-Fc-VH-VH

Polypeptide chain-1   N′ — | VH_A | CH1 | — C′

Polypeptide chain-2   N′ — | VL_A | CL | h | CH2 | CH3 | — C′

Polypeptide chain-3   N′ — | VH_B | L | VH_B | h | CH2 | CH3 | — C′

Linker peptide       Hinge region
                     (or linker peptide)

**(19)**

## Structure (19) Fab(cross Fd/LC)-Fc-VH'-VH"

| | | |
|---|---|---|
| Polypeptide chain-1 | N' —| VH_A | CH1 |— C' |

| | | |
|---|---|---|
| Polypeptide chain-2 | N' —| VL_A | CL | h | CH2 | CH3 |— C' |

| | | |
|---|---|---|
| Polypeptide chain-3 | N' —| VH_C | L | VH_B | h | CH2 | CH3 |— C' |

Linker peptide    Hinge region
(or linker peptide)

**(20)**

## Structure (20)    scFv(VL-VH)-Fc-VH

Linker peptide    Hinge region
(or linker peptide)

| | | |
|---|---|---|
| Polypeptide chain-1 | N' —| VL_A | L | VH_A | h | CH2 | CH3 |— C' |

| | | |
|---|---|---|
| Polypeptide chain-2 | N' —| VH_B | h | CH2 | CH3 |— C' |

Hinge region
(or linker peptide)

**(21)**

**Structure (21)**    scFv(VH-VL)-Fc-VH

Hinge region
Linker peptide (or linker peptide)

Polypeptide chain-1    N' — | VH_A | L | VL_A | h | CH2 | CH3 | — C'

Polypeptide chain-2    N' — | VH_B | h | CH2 | CH3 | — C'

Hinge region
(or linker peptide)

**(22)**

**Structure (22)**    scFv(VL-VH)-Fc-VH-VH

Hinge region
Linker peptide 1    (or linker peptide)

Polypeptide chain-1    N' — | VL_A | L1 | VH_A | h | CH2 | CH3 | — C'

Polypeptide chain-2    N' — | VH_B | L2 | VH_B | h | CH2 | CH3 | — C'

Linker peptide 2    Hinge region
(or linker peptide)

**(23)**

**Structure (23)**   scFv(VH-VL)-Fc-VH-VH

Hinge region
Linker peptide 1 (or linker peptide)

Polypeptide chain-1   N' — | VH_A | L1 | VL_A | h | CH2 | CH3 | — C'

Polypeptide chain-2   N' — | VH_B | L2 | VH_B | h | CH2 | CH3 | — C'

Linker peptide 2    Hinge region
(or linker peptide)

**(24)**

**Structure (24)**   scFv(VL-VH)-Fc-VH'-VH"

Hinge region
Linker peptide 1 (or linker peptide)

Polypeptide chain-1   N' — | VL_A | L1 | VH_A | h | CH2 | CH3 | — C'

Polypeptide chain-2   N' — | VH_C | L2 | VH_B | h | CH2 | CH3 | — C'

Linker peptide 2    Hinge region
(or linker peptide)

**(25)**

## Structure (25)   scFv(VH-VL)-Fc-VH'-VH"

Linker peptide 1    Hinge region (or linker peptide)

Polypeptide chain-1    N' — | VH_A | L1 | VL_A | h | CH2 | CH3 | — C'

Polypeptide chain-2    N' — | VH_C | L2 | VH_B | h | CH2 | CH3 | — C'

Linker peptide 2    Hinge region (or linker peptide)

**(26)**

## Structure (26)   Fab-Fc-VH-VH-VH

Polypeptide chain-1    N' — | VL_A | CL | — C'

Polypeptide chain-2    N' — | VH_A | CH1 | h | CH2 | CH3 | — C'

Polypeptide chain-3    N' — | VH_B | L1 | VH_B | L2 | VH_B | h | CH2 | CH3 | — C'

First linker peptide    Second linker peptide    Hinge region (or linker peptide)

**(27)**

## Structure (27) Fab-Fc-VH′-VH″-VH‴

Polypeptide chain-1    N′ —[ VL_A | CL ]— C′

Polypeptide chain-2    N′ —[ VH_A | CH1 | h | CH2 | CH3 ]— C′

Polypeptide chain-3    N′ —[ VH_D | L1 | VH_C | L2 | VH_B | h | CH2 | CH3 ]— C′

First linker   Second linker   Hinge region
peptide        peptide         (or linker peptide)

**(28)**

## FIT-Ig (WO2015103072A1)

Linker peptide                    Hinge region

Polypeptide chain-1    N′ —[ VL_A | CL | L | VH_B | CH1 | h | CH2 | CH3 ]— C′

Polypeptide chain-2    N′ —[ VH_A | CH1 ]— C′

Polypeptide chain-3                       N′ —[ VL_B | CL ]— C′

## FIG. 1

**Binding to human CTLA4 protein hCTLA4-His**

Legend:
- ◆ PR000184
- ○ PR000300
- ⊟ PR000301
- ▽ PR000302
- △ PR000303
- ⊗ hIgG1 iso

**PD-L1 × CTLA4**

| Antibody | EC50 (nM) | OD450 Maximum |
|----------|-----------|---------------|
| PR000184 | 0.599 | 3.01 |
| PR000300 | 0.567 | 2.95 |
| PR000301 | 0.350 | 2.92 |
| PR000302 | 10.60 | 3.55 |
| PR000303 | 1.566 | 2.72 |

## FIG. 2

**(A)**

**Binding to CHO-K1/hCTLA4**

Legend:
- ◆ PR000184
- ○ PR000300
- ⊟ PR000301
- ▽ PR000302
- △ PR000303
- ⊗ hIgG1 iso

**PD-L1 × CTLA4**

| Antibody | EC50 (nM) | Maximum MFI |
|----------|-----------|-------------|
| PR000184 | 1.688 | 1668 |
| PR000300 | 2.914 | 2621 |
| PR000301 | 1.935 | 2360 |
| PR000302 | 26.15 | 1566 |
| PR000303 | 4.362 | 2040 |

**(B)**

**Binding to CHO-K1/hCTLA4**

Legend:
- ○ PR001573
- ⊟ PR001574
- ◇ PR001577
- ◆ PR000184
- ⊗ hIgG1 iso
- ● Ipilimumab

**PD-L1 × CTLA4**

| Antibody | EC50 (nM) | Maximum MFI |
|----------|-----------|-------------|
| PR001573 | 9.905 | 780.8 |
| PR001574 | 11.980 | 677.2 |
| PR001577 | 8.516 | 584.9 |
| PR000184 | 4.642 | 722.0 |
| Ipilimumab | 3.412 | 420.3 |

**(C)**

**Binding to CHO-K1/hCTLA4**

Legend:
- ⊖ PR001572
- ▽ PR001575
- △ PR001578
- ◆ PR000184
- ⊛ hIgG1 iso
- ● Ipilimumab

**PD-L1 × CTLA4**

| Antibody | EC50 (nM) | Maximum MFI |
|---|---|---|
| PR001572 | 2.911 | 433.7 |
| PR001575 | 5.075 | 415.3 |
| PR001578 | 4.646 | 401.3 |
| PR000184 | 4.642 | 722.0 |
| Ipilimumab | 3.412 | 420.3 |

**(D)**

**Binding to CHO-K1/hCTLA4**

Legend:
- ▽ PR001609
- △ PR001610
- ◆ PR000184
- ⊛ hIgG1 iso
- ● Ipilimumab

**PD-L1 × CTLA4**

| Antibody | EC50 (nM) | Maximum MFI |
|---|---|---|
| PR001609 | n.d. | 355.4 |
| PR001610 | n.d. | 467.9 |
| PR000184 | 4.642 | 722.0 |
| Ipilimumab | 3.412 | 420.3 |

**(E)**

**Binding to CHO-K1/hCTLA4**

Legend:
- ▽ PR000403
- △ PR000404
- ◆ PR000184
- ⊛ hIgG1 iso
- ● Ipilimumab

**PD-L1 × CTLA4**

| Antibody | EC50 (nM) | Maximum MFI |
|---|---|---|
| PR000403 | 20.54 | 343.2 |
| PR000404 | 26.92 | 338.6 |
| PR000184 | 4.642 | 722.0 |
| Ipilimumab | 3.412 | 420.3 |

**FIG. 3**

**(A)**

Binding to HEK293-hCTLA4

PD-L1 × CTLA4

| Antibody | EC50 (nM) | Maximum MFI |
|---|---|---|
| PR000401 | 5.173 | 1911 |
| PR000402 | 2.400 | 1764 |
| PR000184 | 1.147 | 2261 |

**(B)**

Binding to HEK293-hCTLA4

PD-L1 × CTLA4

| Antibody | EC50 (nM) | Maximum MFI |
|---|---|---|
| PR000403 | 16.300 | 1408 |
| PR000404 | 37.220 | 1615 |
| PR000184 | 1.147 | 2261 |

**FIG. 4**

Blocking the binding of CTLA4 to B7-1

PD-L1 × CTLA4

| Antibody | IC50 (nM) | Maximum inhibition rate (%) |
|---|---|---|
| PR000184 | 2.673 | 97.20 |
| PR000300 | 2.091 | 98.17 |
| PR000301 | 2.511 | 98.31 |
| PR000302 | 11.57 | 98.91 |
| PR000303 | 4.121 | 97.26 |

**FIG. 5**

**(A)** Blocking the binding of CHO-K1/hCTLA4 to B7-1

### PD-L1 × CTLA4

| Antibody | IC50 (nM) | Maximum inhibition rate (%) |
|---|---|---|
| PR000184 | 4.132 | 99.71 |
| PR000300 | 6.417 | 100.8 |
| PR000301 | 3.210 | 99.88 |
| PR000302 | 71.23 | 48.98 |
| PR000303 | 9.712 | 83.30 |

**(B)** Blocking the binding of CHO-K1/hCTLA4 to B7-1

### PD-L1 × CTLA4

| Antibody | IC50 (nM) | Maximum inhibition rate (%) |
|---|---|---|
| PR000184 | 3.009 | 100.0 |
| PR001573 | 6.222 | 99.2 |
| PR001574 | 4.488 | 99.8 |
| PR001577 | 4.883 | 99.7 |

**(C)** Blocking the binding of CHO-K1/hCTLA4 to B7-1

### PD-L1 × CTLA4

| Antibody | IC50 (nM) | Maximum inhibition rate (%) |
|---|---|---|
| PR000184 | 3.009 | 100.0 |
| PR001572 | 2.982 | 100.0 |
| PR001575 | 3.795 | 100.0 |
| PR001578 | 2.966 | 100.0 |

**(D)**

PD-L1 × CTLA4

Blocking the binding of CHO-K1/hCTLA4 to B7-1

- ▽ PR001609
- △ PR001610
- ◆ PR000184
- ⊗ hIgG1 iso

| Antibody | IC50 (nM) | Maximum inhibition rate (%) |
|---|---|---|
| PR000184 | 3.009 | 100.0 |
| PR001609 | 98.330 | 95.0 |
| PR001610 | 96.56 | 61.3 |

FIG. 6

Binding to human PD-L1 protein hPDL1-His

PD-L1 × CTLA4

- ◆ PR000070
- ⊖ PR000300
- ⊟ PR000301
- ▽ PR000302
- △ PR000303
- ⊗ hIgG1 iso

| Antibody | EC50 (nM) | OD450 Maximum |
|---|---|---|
| PR000070 | 0.146 | 3.454 |
| PR000300 | 0.108 | 3.519 |
| PR000301 | 0.131 | 3.528 |
| PR000302 | 0.152 | 3.542 |
| PR000303 | 0.241 | 3.523 |

FIG. 7

**(A)**

**Binding to CHO-K1/hPDL1**

- ◆ PR000070
- ◯ PR000300
- ⊟ PR000301
- ▽ PR000302
- △ PR000303
- ⊗ hIgG1 iso

**PD-L1 × CTLA4**

| Antibody | EC50 (nM) | Maximum MFI |
|----------|-----------|-------------|
| PR000070 | 0.747 | 22681 |
| PR000300 | 0.884 | 21029 |
| PR000301 | 0.918 | 16556 |
| PR000302 | 0.737 | 18580 |
| PR000303 | 0.848 | 17992 |

**(B)**

**Binding to CHO-K1/hPDL1**

- ⊟ PR001573
- △ PR001574
- ▽ PR001577
- ◆ PR000416
- ⊗ hIgG1 iso

**PD-L1 × CTLA4**

| Antibody | EC50 (nM) | Maximum MFI |
|----------|-----------|-------------|
| PR001573 | 0.83 | 8308 |
| PR001574 | 0.93 | 6712 |
| PR001577 | 0.96 | 6609 |
| PR000416 | 0.50 | 8379 |

**(C)**

**Binding to CHO-K1/hPDL1**

- ▽ PR001572
- △ PR001575
- ⊟ PR001578
- ◆ PR000416
- ⊗ hIgG1 iso

**PD-L1 × CTLA4**

| Antibody | EC50 (nM) | Maximum MFI |
|----------|-----------|-------------|
| PR001572 | 1.07 | 5828 |
| PR001575 | 2.41 | 5879 |
| PR001578 | 1.94 | 5658 |
| PR000416 | 0.50 | 8379 |

**(D)**

**Binding to CHO-K1/hPDL1**

- ▽ PR000403
- △ PR000404
- ◆ PR000416
- ⊗ hIgG1 iso

**PD-L1 × CTLA4**

| Antibody | EC50 (nM) | Maximum MFI |
|---|---|---|
| PR000403 | 0.73 | 8094 |
| PR000404 | 0.78 | 8102 |
| PR000416 | 0.50 | 8379 |

**(E)**

**Binding to CHO-K1/hPDL1**

- ▽ PR001609
- △ PR001610
- ◆ PR000416
- ⊗ hIgG1 iso

**PD-L1 × CTLA4**

| Antibody | EC50 (nM) | Maximum MFI |
|---|---|---|
| PR001609 | 1.51 | 11168 |
| PR001610 | 1.37 | 11146 |
| PR000416 | 0.50 | 8379 |

**FIG. 8**

**Binding to MDA-MB-231 cells expressing human PDL1**

- ▽ PR000401
- △ PR000402
- ■ PR000265
- ⊗ hIgG1 iso

**PD-L1 × CTLA4**

| Antibody | EC50 (nM) | Maximum MFI |
|---|---|---|
| PR000401 | 0.288 | 994 |
| PR000402 | 0.259 | 841 |
| PR000265 | 0.163 | 958 |

**FIG. 9**

**(A)**

**PD-L1 × CTLA4**

**Blocking the binding of CHO-K1/hPDL1 to PD1**

| Antibody | IC50 (nM) | Maximum inhibition rate (%) |
|---|---|---|
| PR000070 | 0.725 | 96.80 |
| PR000300 | 0.677 | 96.61 |
| PR000301 | 0.882 | 96.18 |
| PR000302 | 0.640 | 96.72 |
| PR000303 | 0.695 | 95.88 |

**(B)**

**PD-L1 × CTLA4**

**Blocking the binding of CHO-K1/hPDL1 to PD1**

| Antibody | IC50 (nM) | Maximum inhibition rate (%) |
|---|---|---|
| PR001573 | 0.50 | 82.3 |
| PR001574 | 0.88 | 81.1 |
| PR001577 | 0.61 | 81.0 |
| PR000416 | 0.27 | 79.8 |

**(C)**

**PD-L1 × CTLA4**

**Blocking the binding of CHO-K1/hPDL1 to PD1**

| Antibody | IC50 (nM) | Maximum inhibition rate (%) |
|---|---|---|
| PR001572 | 1.40 | 80.7 |
| PR001575 | 1.74 | 81.7 |
| PR001578 | 1.69 | 81.4 |
| PR000416 | 0.27 | 79.8 |

**(D)**

Blocking the binding of CHO-K1/hPDL1 to PD1

**PD-L1 × CTLA4**

| Antibody | IC50 (nM) | Maximum inhibition rate (%) |
|---|---|---|
| PR000403 | 0.42 | 81.4 |
| PR000404 | 0.60 | 81.1 |
| PR000416 | 0.27 | 79.8 |

**(E)**

Blocking the binding of CHO-K1/hPDL1 to PD1

**PD-L1 × CTLA4**

| Antibody | IC50 (nM) | Maximum inhibition rate (%) |
|---|---|---|
| PR001609 | 1.12 | 81.6 |
| PR001610 | 1.23 | 80.2 |
| PR000416 | 0.27 | 79.8 |

**FIG. 10**

Killing of 293F/hCTLA4 cells by NK92/CD16a cells

**PD-L1 × CTLA4**

| Antibody | EC50 (nM) | Maximum killing rate (%) |
|---|---|---|
| PR000300 | 0.008 | 23.81 |
| PR000301 | 0.031 | 36.62 |
| PR000184 | 0.015 | 30.20 |

**FIG. 11**

FIG. 12

FIG. 13

FIG. 14

FIG. 15

EP 4 545 568 A2

Determination of level of IL-2 in a mixed lymphocyte reaction

Determination of level of IFN-γ in a mixed lymphocyte reaction

Donor pairing #1

Donor pairing #2

FIG. 16

FIG. 17

**(A)**

Binding to CHO-K1/hCTLA4

Legend: PR000184, PR000305, PR000653, PR000654, PR000655, PR000656, PR000658, PR000659, PR000706, PR000716, PR000717, hIgG iso

### HER2 × CTLA4

| Antibody | EC50 (nM) | Maximum MFI |
|---|---|---|
| PR000305 | 5.016 | 157.1 |
| PR000653 | 2.507 | 156.1 |
| PR000654 | 1.612 | 154.2 |
| PR000655 | 1.866 | 147.7 |
| PR000656 | 3.421 | 144 |
| PR000658 | 1.491 | 148.1 |
| PR000659 | 1.943 | 158.8 |
| PR000706 | 2.108 | 141.3 |
| PR000716 | 1.395 | 130.6 |
| PR000717 | 1.344 | 129.9 |
| PR000184 | 1.588 | 214.1 |

**(B)**

Binding to CHO-K1/hCTLA4

Legend: PR000539, PR000540, PR000714, PR000715, PR000184, hIgG iso

### HER2 × CTLA4

| Antibody | EC50 (nM) | Maximum MFI |
|---|---|---|
| PR000539 | 1.703 | 148.3 |
| PR000540 | 2.029 | 185.3 |
| PR000714 | 1.640 | 146.5 |
| PR000715 | 1.864 | 143.0 |
| PR000184 | 1.588 | 214.1 |

**(C)**

Binding to CHO-K1/hCTLA4

Legend: PR000184, PR001583, PR001584, PR001586, hIgG1 iso

### HER2 × CTLA4

| Antibody | EC50 (nM) | Maximum MFI |
|---|---|---|
| PR000184 | 1.792 | 1083 |
| PR001583 | 4.426 | 1934 |
| PR001584 | 4.531 | 1524 |
| PR001586 | 6.131 | 1861 |

**(D)**

**Binding to CHO-K1/hCTLA4**

**HER2 × CTLA4**

| Antibody | EC50 (nM) | Maximum MFI |
|---|---|---|
| PR000184 | 1.792 | 1083 |
| PR001588 | 7.257 | 2168 |
| PR001589 | 9.390 | 1866 |
| PR001591 | 5.149 | 2074 |

**(E)**

**Binding to CHO-K1/hCTLA4**

**HER2 × CTLA4**

| Antibody | EC50 (nM) | Maximum MFI |
|---|---|---|
| PR000541 | 1.711 | 203.2 |
| PR000542 | 1.766 | 235.2 |
| PR000184 | 1.588 | 214.1 |

**(F)**

**Binding to CHO-K1/hCTLA4**

**HER2 × CTLA4**

| Antibody | EC50 (nM) | Maximum MFI |
|---|---|---|
| PR000184 | 1.792 | 1083 |
| PR001579 | 3.193 | 1078 |
| PR001580 | 2.649 | 1078 |
| PR001581 | 3.469 | 1074 |
| PR001582 | 22.83 | 1034 |

**(G)**

**Binding to CHO-K1/hCTLA4**

Legend:
- ■ PR000184
- △ PR001585
- ▽ PR001587
- ◉ hIgG1 iso

### HER2 × CTLA4

| Antibody | EC50 (nM) | Maximum MFI |
|---|---|---|
| PR000184 | 1.792 | 1083 |
| PR001585 | 1.585 | 1051 |
| PR001587 | 1.717 | 1044 |

**(H)**

**Binding to CHO-K1/hCTLA4**

Legend:
- ■ PR000184
- △ PR001590
- ▽ PR001592
- ◉ hIgG1 iso

### HER2 × CTLA4

| Antibody | EC50 (nM) | Maximum MFI |
|---|---|---|
| PR000184 | 1.792 | 1083 |
| PR001590 | 5.444 | 1048 |
| PR001592 | 10.400 | 1299 |

**( I )**

**Binding to CHO-K1/hCTLA4**

Legend:
- ■ PR000184
- ▽ PR000916
- △ PR000917
- ◉ hIgG iso

### HER2 × CTLA4

| Antibody | EC50 (nM) | Maximum MFI |
|---|---|---|
| PR000184 | 1.588 | 214.1 |
| PR000916 | 4.681 | 150.9 |
| PR000917 | 9.803 | 142.1 |

**(J)**

**Binding to CHO-K1/hCTLA4**

**HER2 × CTLA4**

| Antibody | EC50 (nM) | Maximum MFI |
|----------|-----------|-------------|
| PR000184 | 3.046 | 2167 |
| PR002672 | 2.785 | 1804 |

**FIG. 18**

**(A)**

**Blocking the binding of CTLA4 to B7-1**

**HER2 × CTLA4**

| Antibody | IC50 (nM) |
|----------|-----------|
| PR000184 | 2.464 |
| PR000654 | 5.307 |
| PR000655 | 4.024 |

**(B)**

**Blocking the binding of CTLA4 to B7-1**

**HER2 × CTLA4**

| Antibody | IC50 (nM) |
|----------|-----------|
| PR000184 | 2.464 |
| PR000539 | 5.406 |
| PR000540 | 6.374 |
| PR000715 | 6.281 |

**(C)**

**Blocking the binding of CTLA4 to B7-1**

Legend:
- ● PR000184
- ▽ PR001583
- △ PR001584
- ⊖ PR001586
- ⊗ hIgG1 iso

**HER2 × CTLA4**

| Antibody | IC50 (nM) |
|---|---|
| PR000184 | 1.687 |
| PR001583 | 2.426 |
| PR001584 | 2.762 |
| PR001586 | 2.012 |

**(D)**

**Blocking the binding of CTLA4 to B7-1**

Legend:
- ● PR000184
- ▽ PR001588
- △ PR001589
- ⊖ PR001591
- ⊗ hIgG1 iso

**HER2 × CTLA4**

| Antibody | IC50 (nM) |
|---|---|
| PR000184 | 1.687 |
| PR001588 | 2.197 |
| PR001589 | 2.588 |
| PR001591 | 2.703 |

**(E)**

**Blocking the binding of CTLA4 to B7-1**

Legend:
- ● PR000184
- △ PR000541
- ▽ PR000542
- ⊗ hIgG1 iso

**HER2 × CTLA4**

| Antibody | IC50 (nM) |
|---|---|
| PR000184 | 2.464 |
| PR000541 | 3.314 |
| PR000542 | 2.816 |

**(F)** Blocking the binding of CTLA4 to B7-1

- PR000184
- PR000541
- PR001579
- PR001580
- PR001581
- PR001582
- hIgG1 iso

**HER2 × CTLA4**

| Antibody | IC50 (nM) |
| --- | --- |
| PR000184 | 1.687 |
| PR000541 | 1.718 |
| PR001579 | 1.152 |
| PR001580 | 1.132 |
| PR001581 | 1.145 |
| PR001582 | 2.149 |

**(G)** Blocking the binding of CTLA4 to B7-1

- PR000184
- PR001585
- PR001587
- hIgG1 iso

**HER2 × CTLA4**

| Antibody | IC50 (nM) |
| --- | --- |
| PR000184 | 1.687 |
| PR001585 | 1.306 |
| PR001587 | 1.455 |

**(H)** Blocking the binding of CTLA4 to B7-1

- PR000184
- PR001590
- PR001592
- hIgG1 iso

**HER2 × CTLA4**

| Antibody | IC50 (nM) |
| --- | --- |
| PR000184 | 1.687 |
| PR001590 | 1.985 |
| PR001592 | 3.213 |

**FIG. 19**

**(A)**

Blocking the binding of CHO-K1/hCTLA4 to B7-1

● PR000184
△ PR000539
⊟ PR000715
◉ hIgG1 iso

**HER2 × CTLA4**

| Antibody | IC50 (nM) | Maximum inhibition rate (%) |
|---|---|---|
| PR000184 | 1.685 | 99.62 |
| PR000539 | 9.000 | 88.02 |
| PR000715 | 10.240 | 89.82 |

**(B)**

Blocking the binding of CHO-K1/hCTLA4 to B7-1

● PR000184
▽ PR000541
◉ hIgG1 iso

**HER2 × CTLA4**

| Antibody | IC50 (nM) | Maximum inhibition rate (%) |
|---|---|---|
| PR000184 | 1.685 | 99.62 |
| PR000541 | 1.634 | 99.55 |

**(C)**

Blocking the binding of CHO-K1/hCTLA4 to B7-1

● PR000184
▽ PR002672
◉ hIgG1 iso

**HER2 × CTLA4**

| Antibody | IC50 (nM) | Maximum inhibition rate (%) |
|---|---|---|
| PR000184 | 0.239 | 99.42 |
| PR002672 | 0.983 | 98.97 |

**FIG. 20**

**(A)**

**Binding to SK-BR-3**

Legend:
- ● PR000210
- ◈ PR000305
- ✳ PR000653
- ▲ PR000654
- ▼ PR000655
- ◆ PR000656
- ⊖ PR000658
- ⊟ PR000659
- △ PR000706
- ▽ PR000716
- ■ PR000717
- ⊛ hIgG1 iso

**HER2 × CTLA4**

| Antibody | EC50 (nM) | Maximum MFI |
|---|---|---|
| PR000210 | 1.332 | 11868 |
| PR000305 | 1.538 | 12604 |
| PR000653 | 1.668 | 12781 |
| PR000654 | 1.712 | 13168 |
| PR000655 | 1.57 | 12201 |
| PR000656 | 1.664 | 12358 |
| PR000658 | 1.474 | 12964 |
| PR000659 | 1.653 | 13672 |
| PR000706 | 1.562 | 13355 |
| PR000716 | 1.386 | 13418 |
| PR000717 | 1.466 | 13684 |

**(B)**

**Binding to SK-BR-3**

Legend:
- ● PR000210
- △ PR000539
- ▽ PR000540
- ⊛ hIgG1 iso

**HER2 × CTLA4**

| Antibody | EC50 (nM) | Maximum MFI |
|---|---|---|
| PR000210 | 1.332 | 11868 |
| PR000539 | 1.323 | 9460 |
| PR000540 | 1.471 | 10894 |

**(C)**

**Binding to SK-BR-3**

Legend:
- ● PR000210
- ▽ PR001586
- △ PR001974
- ⊛ hIgG1 iso

**HER2 × CTLA4**

| Antibody | EC50 (nM) | Maximum MFI |
|---|---|---|
| PR000210 | 1.863 | 10438 |
| PR001586 | 2.975 | 9436 |
| PR001974 | 4.119 | 8478 |

**(D)**

**Binding to SK-BR-3**

| | PR000210 |
| | PR001583 |
| | PR001584 |
| | PR001586 |
| | hIgG1 iso |

**HER2 × CTLA4**

| Antibody | EC50 (nM) | Maximum MFI |
|---|---|---|
| PR000210 | 2.929 | 31552 |
| PR001583 | 3.915 | 32008 |
| PR001584 | 7.502 | 32880 |
| PR001586 | 4.717 | 35248 |

**(E)**

**Binding to SK-BR-3**

| | PR000672 |
| | PR001588 |
| | PR001589 |
| | PR001591 |
| | hIgG1 iso |

**HER2 × CTLA4**

| Antibody | EC50 (nM) | Maximum MFI |
|---|---|---|
| PR000672 | 3.119 | 33400 |
| PR001588 | 8.445 | 37900 |
| PR001589 | 5.520 | 37100 |
| PR001591 | 6.861 | 35900 |

**(F)**

**Binding to SK-BR-3**

| | PR000210 |
| | PR000541 |
| | PR000542 |
| | hIgG1 iso |

**HER2 × CTLA4**

| Antibody | EC50 (nM) | Maximum MFI |
|---|---|---|
| PR000210 | 1.332 | 11868 |
| PR000541 | 1.282 | 7378 |
| PR000542 | 1.295 | 9019 |

**(G)**

**Binding to SK-BR-3**

**HER2 × CTLA4**

| Antibody | EC50 (nM) | Maximum MFI |
|---|---|---|
| PR000210 | 2.929 | 31552 |
| PR001579 | 3.056 | 36444 |
| PR001580 | 3.421 | 35754 |
| PR001581 | 3.687 | 34744 |
| PR001582 | 3.665 | 30517 |

**(H)**

**Binding to SK-BR-3**

**HER2 × CTLA4**

| Antibody | EC50 (nM) | Maximum MFI |
|---|---|---|
| PR000210 | 2.929 | 31552 |
| PR001585 | n.d. | 26700 |
| PR001587 | n.d. | 26800 |

n.d.: not determined

**( I )**

**Binding to SK-BR-3**

**HER2 × CTLA4**

| Antibody | EC50 (nM) | Maximum MFI |
|---|---|---|
| PR000672 | 3.119 | 34721 |
| PR001590 | n.d. | 34000 |
| PR001592 | n.d. | 32800 |

n.d.: not determined

**(J)**

**Binding to SK-BR-3**

| HER2 × CTLA4 | | |
|---|---|---|
| Antibody | EC50 (nM) | Maximum MFI |
| PR000210 | 1.913 | 7692 |
| PR000916 | 3.688 | 9874 |

**FIG. 21**

**Killing of BT-474 cells by NK92/CD16a cells**

| HER2 × CTLA4 | | |
|---|---|---|
| Antibody | EC50 (nM) | Maximum killing rate (%) |
| PR000210 | 0.183 | 41.95 |
| PR001582 | 0.055 | 94.65 |
| PR001586 | 0.053 | 94.66 |

**FIG. 22**

**(A)**

SK-BR-3 (7 days)

HER2 × CTLA4

| Antibody | IC50 (nM) | Maximum inhibition rate (%) |
|----------|-----------|------------------------------|
| PR000655 | 0.4428 | 28.07 |
| PR000210 | 0.7036 | 36.09 |

◇ PR000655
● PR000210

**(B)**

SK-BR-3 (7 days)

● PR000210
⊟ PR000539
△ PR000540
▽ PR000714
◇ PR000715

HER2 × CTLA4

| Antibody | IC50 (nM) | Maximum inhibition rate (%) |
|----------|-----------|------------------------------|
| PR000210 | 0.7036 | 36.09 |
| PR000539 | 0.5134 | 35.58 |
| PR000540 | 0.6929 | 39.15 |
| PR000714 | 0.8239 | 37.95 |
| PR000715 | 0.2101 | 40.05 |

**(C)**

SK-BR-3 (7 days)

● PR000210
△ PR001583
▽ PR001584
⊟ PR001586
◉ hIgG1 iso

HER2 × CTLA4

| Antibody | IC50 (nM) | Maximum inhibition rate (%) |
|----------|-----------|------------------------------|
| PR000210 | 0.1900 | 59.39 |
| PR001583 | 0.5974 | 59.68 |
| PR001584 | 0.7672 | 61.11 |
| PR001586 | 0.5712 | 59.35 |

220

**(D)**

**SK-BR-3 (7 days)**

**HER2 × CTLA4**

| Antibody | IC50 (nM) | Maximum inhibition rate (%) |
|---|---|---|
| PR000210 | 0.7036 | 36.09 |
| PR000542 | 0.6676 | 27.54 |

**(E)**

**SK-BR-3 (7 days)**

**HER2 × CTLA4**

| Antibody | IC50 (nM) | Maximum inhibition rate (%) |
|---|---|---|
| PR000210 | 0.1900 | 59.39 |
| PR001579 | 0.2543 | 58.32 |
| PR001580 | 0.3418 | 57.62 |
| PR001581 | 0.3581 | 58.55 |
| PR001582 | 0.4055 | 60.02 |

**(F)**

**SK-BR-3 (7 days)**

**HER2 × CTLA4**

| Antibody | IC50 (nM) | Maximum inhibition rate (%) |
|---|---|---|
| PR000210 | 0.190 | 59.39 |
| PR001585 | 1.357 | 52.59 |
| PR001587 | 1.732 | 60.67 |

**FIG. 23**

**(A)**

Simultaneously binding to CHOK1/CTLA4 and SK-BR-3 cells

- PR000539
- PR000540
- PR000715
- PR000210
- PR000184
- hIgG1 iso

**(B)**

Simultaneously binding to CHOK1/CTLA4 and SK-BR-3 cells

- PR000541
- PR000542
- PR000210
- PR000184
- hIgG1 iso

**(C)**

Simultaneously binding to CHOK1/CTLA4 and SK-BR-3 cells

- PR000916
- PR000184
- PR000210
- hIgG1 iso

FIG. 24

**(A)**

**(B)**

**(C)**

**(D)**

**(E)**

**(F)**

**(G)**

**(H)**

**( I )**

**FIG. 25**

FIG. 26

**(A)**

**Binding to CHO-K1/hPDL1**

| Antibody | EC50 (nM) | Maximum MFI |
|---|---|---|
| PR003052 | 1.750 | 2006 |
| PR000701 | 1.723 | 1714 |
| PR003549 | 1.558 | 2224 |
| PR003550 | 1.111 | 2254 |
| PR003551 | 1.294 | 2149 |
| PR000265 | 0.7807 | 2004 |

**PD-L1 × 4-1BB**

**(B)**

**Binding to CHO-K1/hPDL1**

**PD-L1 × 4-1BB**

| Antibody | EC50 (nM) | Maximum MFI |
|---|---|---|
| PR004270 | 0.963 | 14716 |
| PR000265 | 0.356 | 12960 |

**(C)**

**Binding to CHO-K1/hPDL1**

**PD-L1 × 4-1BB**

| Antibody | EC50 (nM) | Maximum MFI |
|---|---|---|
| PR007163 | 2.258 | 51309 |
| PR007164 | 1.926 | 51400 |
| PR000265 | 1.561 | 47006 |

**FIG. 27**

**(A)**

**Binding to CHOK1/hu 4-1BB**

| Antibody | EC50 (ug/ml) | Maximum MFI | EC50 (nM) |
|---|---|---|---|
| PR000197 | 0.101 | 15488 | 0.673 |
| Utomilumab | 0.083 | 13223 | 0.553 |

**(B)**

**Binding to CHO-K1/hu 4-1BB**

| Antibody | EC50 (nM) | Maximum MFI |
|---|---|---|
| PR000448 | 0.947 | 8182 |
| Utomilumab | 0.627 | 7747 |

**(C)**

**Binding to CHO-K1/hu 4-1BB**

| Antibody | EC50 (nM) | Maximum MFI |
|---|---|---|
| PR001758 | 0.2122 | 1644 |
| PR001760 | 0.1981 | 1742 |
| Urelumab | 0.2223 | 1381 |
| Utomilumab | 0.1297 | 1584 |

**(D)**

### Binding to CHOK1/hu 4-1BB

| Antibody | EC50 (nM) | Maximum MFI |
|---|---|---|
| PR001771 | 0.5323 | 1716 |
| Urelumab | 0.2223 | 1381 |

**(E)**

### Binding to CHOK1/hu 4-1BB

| Antibody | EC50 (nM) | Maximum MFI |
|---|---|---|
| PR001836 | 0.2798 | 1833 |
| PR001838 | 0.2658 | 1832 |
| PR001840 | 0.4302 | 1789 |
| Urelumab | 0.2197 | 1278 |
| Utomilumab | 0.1771 | 1643 |

**(F)**

### Binding to CHO-K1/hu 4-1BB

| Antibody | EC50 (nM) | Maximum MFI |
|---|---|---|
| PR001838 | 0.8512 | 101674 |
| PR004469 | 1.295 | 109216 |
| Urelumab | 0.6834 | 61805 |

**(G)**

Binding to CHOK1/hu 4-1BB

| Antibody | EC50 (nM) | Maximum MFI |
|---|---|---|
| PR003052 | 36 | 1390 |
| PR000701 | 10.15 | 1338 |
| PR003549 | 1.898 | 1546 |
| PR003550 | 1.523 | 1567 |
| PR003551 | 3.148 | 1570 |
| Urelumab | 1.014 | 570 |

**PD-L1 × 4-1BB**

**(H)**

Binding to CHO-K1/hu 4-1BB

**PD-L1 × 4-1BB**

| Antibody | EC50 (nM) | Maximum MFI |
|---|---|---|
| PR004270 | 1.576 | 46812 |
| Urelumab | 0.3469 | 21640 |
| Utomilumab | 0.4635 | 37245 |

**( I )**

Binding to CHO-K1/hu 4-1BB

**PD-L1 × 4-1BB**

| Antibody | EC50 (nM) | Maximum MFI |
|---|---|---|
| PR007163 | 3.762 | 65156 |
| PR007164 | 3.170 | 63217 |
| PR001760 | 3.761 | 83588 |

**FIG. 28**

**(A)**

**PD-L1 × 4-1BB**

Binding to CHO-K1/cyno 4-1BB

| Antibody | EC50 (nM) | Maximum MFI |
|---|---|---|
| PR003549 | 1.859 | 4977 |
| PR003550 | 1.321 | 5365 |
| Utomilumab | 0.3469 | 5389 |
| Urelumab | No binding | |

**(B)**

**PD-L1 × 4-1BB**

Binding to CHO-K1/cyno 4-1BB

| Antibody | EC50 (nM) | Maximum MFI |
|---|---|---|
| PR004270 | 1.554 | 6103 |
| Utomilumab | 0.3368 | 5271 |
| Urelumab | No binding | |

**FIG. 29**

**(A)**

**(B)**

**FIG. 30**

**FIG. 31**

FIG. 32

**(A)**

**(B)**

**FIG. 33**

PR004270, 5 mg/kg, IV

**FIG. 34**

**(A)**

**Binding to SK-BR-3**

**B7H4 × 4-1BB**

| Antibody | EC50 (nM) | Maximum MFI |
|----------|-----------|-------------|
| PR003334 | 0.9685 | 254417 |
| PR003335 | 1.102 | 244159 |
| PR003338 | 0.8207 | 241092 |

▽ PR003334
⊟ PR003335
△ PR003338
⊛ hIgG1 Iso

**(B)**

**Binding to SK-BR-3**

**B7H4 × 4-1BB**

| Antibody | EC50 (nM) | Maximum MFI |
|----------|-----------|-------------|
| PR003335 | 2.725 | 127965 |
| PR003487 | 2.593 | 131617 |
| PR003488 | 2.626 | 132285 |

● PR003335
▽ PR003487
△ PR003488
⊛ hIgG1 Iso

**(C)**

**Binding to SK-BR-3**

**B7H4 × 4-1BB**

| Antibody | EC50 (nM) | Maximum MFI |
|----------|-----------|-------------|
| PR004279 | 0.6201 | 25718 |
| PR003335 | 1.108 | 25113 |

▽ PR004279
● PR003335
⊛ hIgG1 Iso

**(D)**

Binding to SK-BR-3

B7H4 × 4-1BB

| Antibody | EC50 (nM) | Maximum MFI |
|---|---|---|
| PR004160 | 11.33 | 32312 |
| PR004161 | 23.44 | 41230 |

**(E)**

Binding to SK-BR-3

B7H4 × 4-1BB

| Antibody | EC50 (nM) | Maximum MFI |
|---|---|---|
| PR004181 | 9.254 | 41920 |
| PR004182 | 2.616 | 43637 |

**(F)**

Binding to SK-BR-3

B7H4 × 4-1BB

| Antibody | EC50 (nM) | Maximum MFI |
|---|---|---|
| PR007165 | 3.462 | 37793 |
| PR005189 | 3.173 | 37457 |
| PR002408 | 2.853 | 36029 |

FIG. 35

**(A)**

**Binding to CHOK1/hu 4-1BB**

| Antibody | EC50 (nM) | Maximum MFI |
|----------|-----------|-------------|
| PR003334 | 0.4831 | 162519 |
| PR003335 | 0.7395 | 173076 |
| PR003338 | 0.6708 | 163354 |
| Urelumab | 0.4241 | 118060 |

**B7H4 × 4-1BB**

**(B)**

**Binding to CHOK1/hu4-1BB**

**B7H4 × 4-1BB**

| Antibody | EC50 (nM) | Maximum MFI |
|----------|-----------|-------------|
| PR003487 | 0.7550 | 95740 |
| PR003488 | 0.7735 | 98153 |
| Urelumab | 0.7945 | 101473 |

**(C)**

**Binding to CHO-K1/hu 4-1BB**

**B7H4 × 4-1BB**

| Antibody | EC50 (nM) | Maximum MFI |
|----------|-----------|-------------|
| PR004279 | 0.5035 | 104957 |
| *PR003335* | 1.349 | 91013 |

**(D)**

Binding to CHO-K1/hu 4-1BB

**B7H4 × 4-1BB**

| Antibody | EC50 (nM) | Maximum MFI |
|---|---|---|
| PR004160 | 1.943 | 158311 |
| PR004161 | 1.266 | 126124 |
| Urelumab | 1.506 | 88235 |

**(E)**

Binding to CHO-K1/hu4-1BB

**B7H4 × 4-1BB**

| Antibody | EC50 (nM) | Maximum MFI |
|---|---|---|
| PR004181 | 0.4487 | 69865 |
| PR004182 | 0.6107 | 71139 |
| Urelumab | 0.4488 | 63326 |

**(F)**

Binding to CHO-K1/hu 4-1BB

**B7H4 × 4-1BB**

| Antibody | EC50 (nM) | Maximum MFI |
|---|---|---|
| PR007165 | 3.996 | 71246 |
| PR005189 | 4.255 | 92381 |
| PR001760 | 3.761 | 83588 |

**FIG. 36**

239

FIG. 37

FIG. 38

**(A)**

**(B)**

**FIG. 39**

**(A)**

**Binding of PR003334 to SK-BR-3 cells**

|  | EC50 (nM) | Maximum MFI |
|---|---|---|
| Day one | 1.707 | 186430 |
| Day two | 1.912 | 168183 |
| Day four | 1.562 | 147497 |
| Day seven | 2.004 | 160471 |
| Day fourteen | 1.489 | 164959 |

**(B)**

**Binding of PR003334 to CHO-K1/hu 4-1BB cells**

|  | EC50 (nM) | Maximum MFI |
|---|---|---|
| Day one | 0.841 | 360239 |
| Day two | 0.769 | 397943 |
| Day four | 0.607 | 374153 |
| Day seven | 0.680 | 381546 |
| Day fourteen | 0.851 | 383431 |

**FIG. 40**

**(A)**

**Binding of PR003335 to SK-BR-3 cells**

|            | EC50 (nM) | Maximum MFI |
|------------|-----------|-------------|
| Day one    | 0.841     | 360239      |
| Day two    | 0.769     | 397943      |
| Day four   | 0.607     | 374153      |
| Day seven  | 0.680     | 381546      |
| Day fourteen | 0.851   | 383431      |

**(B)**

**Binding of PR003335 to CHO-K1/hu 4-1BB cells**

|            | EC50 (nM) | Maximum MFI |
|------------|-----------|-------------|
| Day one    | 0.470     | 432153      |
| Day two    | 0.457     | 396669      |
| Day four   | 0.624     | 389379      |
| Day seven  | 0.820     | 410577      |
| Day fourteen | 0.561   | 391641      |

**FIG. 41**

**(A)**

### PR003334, 5 mg/kg, IV

**(B)**

### PR003335, 5 mg/kg, IV

**FIG. 42**

**(A)**

**(B)**

**FIG. 43**

**Binding to HEK293T/hBCMA**

| Antibody | EC50 (nM) | Maximum MFI |
|---|---|---|
| PR000943 | 0.1535 | 69991 |
| PR001035 | 0.3356 | 39840 |
| PR001046 | 0.2129 | 63382 |
| Positive control 1 | 4.076 | 86498 |

**FIG. 44**

**Binding to HEK293T/cyno BCMA**

| Antibody | EC50 (nM) | Maximum MFI |
|---|---|---|
| PR000943 | 0.3293 | 63990 |
| PR001035 | n.d. | 10848 |
| PR001046 | 0.5641 | 67701 |
| Positive control 1 | 4.564 | 116307 |

n.d.: not determined

**FIG. 45**

**Binding to NCI-H929**

| Antibody | EC50 (nM) | Maximum MFI |
|---|---|---|
| PR000940 | 1.847 | 90962 |
| PR000943 | 4.8 | 166453 |
| PR001035 | 1.193 | 112747 |
| PR001046 | 1.535 | 132791 |
| Positive control 1 | 10.75 | 124959 |

**FIG. 46**

FIG. 47

FIG. 48

**(A)**

**Binding to NCI-H929**

**(B)**

| Antibody No. | Variant clone | EC50 (nM) | Maximum MFI |
|---|---|---|---|
| PR001046 | | n.d. | 764 |
| PR004559 | 1046-R1-12G7 | 22.35 | 1189 |
| PR004560 | 1046-R2-10F1 | 3.75 | 1305 |
| PR004561 | 1046-R2-10H1 | 4.38 | 1242 |
| PR004562 | 1046-R2-10H5 | 1.24 | 1560 |
| PR004563 | 1046-R2-1A1 | 7.96 | 1260 |
| PR004564 | 1046-R2-2D4 | 1.34 | 1530 |
| PR004565 | 1046-R2-2G4 | 2.42 | 1428 |
| PR004566 | 1046-R2-4G10 | 3.17 | 1399 |
| PR004567 | 1046-R2-5E1 | 1.72 | 1436 |
| PR004568 | 1046-R2-5H1 | 2.85 | 1444 |
| PR004569 | 1046-R2-5H2 | 5.87 | 1416 |
| PR004570 | 1046-R2-6D6 | 2.15 | 1355 |
| PR004571 | 1046-R2-6F1 | 2.86 | 1382 |
| PR004572 | 1046-R2-7E7 | 0.54 | 1496 |
| PR004573 | 1046-R2-8B1 | 1.72 | 1232 |
| PR004574 | 1046-R2-9E5 | 2.90 | 1299 |

**FIG. 49**

**(A)**

**Binding to HEK293T/hBCMA**

- ● PR001990
- ○ PR002929
- ◆ PR002935
- ● hIgG1 iso

**BCMA × CD3**

| Antibody | EC50 (nM) | Maximum MFI |
|---|---|---|
| PR001990 | n.d. | 13368 |
| PR002929 | 9.017 | 17606 |
| PR002935 | 14.82 | 23928 |

n.d.: not determined

**(B)**

**Binding to HEK293T/hBCMA**

- ⊗ hIgG1 iso
- ● PR002309
- ⊖ PR002892
- ⊕ PR002895
- ▽ PR002898
- ⊟ PR002901
- ⊕ PR002950
- △ PR002953
- ◇ PR002956
- ＊ PR002959
- ◆ Positive control 2

**BCMA × CD3**

| Antibody | EC50 (nM) | Maximum MFI |
|---|---|---|
| PR002309 | 0.3555 | 14957 |
| PR002892 | 0.7675 | 15526 |
| PR002895 | 0.3851 | 14041 |
| PR002950 | n.d. | 22111 |
| PR002953 | n.d. | 25243 |
| PR002898 | 0.791 | 12631 |
| PR002901 | 0.6536 | 14732 |
| PR002956 | n.d. | 17620 |
| PR002959 | n.d. | 23931 |
| Positive control 2 | 1.13 | 17043 |

n.d.: not determined

**(C)**

**Binding to HEK293T/hBCMA**

- ◆ Positive control 2
- ● PR002309
- ⊕ PR002895
- △ PR002953
- ▼ PR003178
- ⊗ hIgG1 iso

**BCMA × CD3**

| Antibody | EC50 (nM) | Maximum MFI |
|---|---|---|
| PR002309 | 0.2586 | 100051 |
| PR002895 | 0.1832 | 93152 |
| PR002953 | 0.2261 | 91617 |
| PR003178 | 0.1878 | 99267 |
| Positive control 2 | 0.6024 | 117412 |

**FIG. 50**

**(A)** Binding to HEK293T/cyno BCMA

| BCMA × CD3 | |
| --- | --- |
| Antibody | Maximum MFI |
| PR001990 | 9167 |
| PR002929 | 13791 |
| PR002935 | 21994 |

**(B)** Binding to HEK293T/cyno BCMA

| BCMA × CD3 | |
| --- | --- |
| Antibody | Maximum MFI |
| PR002309 | 16248 |
| PR002892 | 14983 |
| PR002895 | 13799 |
| PR002950 | 26570 |
| PR002953 | 35655 |
| PR002898 | 8188 |
| PR002901 | 11033 |
| PR002956 | 16151 |
| PR002959 | 30644 |
| Positive control 2 | 926 |

**(C)** Binding to HEK293T/cyno BCMA

| BCMA × CD3 | |
| --- | --- |
| Antibody | Maximum MFI |
| PR002301 | 12958 |
| PR002308 | 39286 |
| PR002309 | 41640 |
| PR002310 | 42045 |

**(D)** Binding to HEK293T/cyno BCMA

BCMA × CD3

| Antibody | Maximum MFI |
|---|---|
| PR002313 | 7608 |
| PR002326 | 6444 |
| PR002328 | 7807 |

**(E)** Binding to HEK293T/cyno BCMA

BCMA × CD3

| Antibody | EC50 (nM) | Maximum MFI |
|---|---|---|
| PR002309 | 1.873 | 128176 |
| PR002895 | 1.173 | 103462 |
| PR002953 | 1.620 | 109345 |
| PR003178 | 0.9206 | 115022 |
| Positive control 2 | n.d. | 4698 |

n.d.: not determined

**FIG. 51**

**(A)** Binding to NCI-H929

BCMA × CD3

| Antibody | Maximum MFI |
|---|---|
| PR001990 | 1837 |
| PR002929 | 3194 |
| PR002935 | 3836 |

**(B)** Binding to NCI-H929

| BCMA × CD3 | |
|---|---|
| **Antibody** | **Maximum MFI** |
| PR001991 | 1979 |
| PR001992 | 3208 |
| PR001993 | 1290 |
| PR001994 | 3434 |
| Positive control 1 | 6160 |

**(C)** Binding to NCI-H929

| BCMA × CD3 | |
|---|---|
| **Antibody** | **Maximum MFI** |
| PR001995 | 2593 |
| PR001996 | 3325 |
| PR001997 | 1456 |
| PR001998 | 3259 |
| Positive control 1 | 6160 |

**(D)** Binding to NCI-H929

| BCMA × CD3 | |
|---|---|
| **Antibody** | **Maximum MFI** |
| PR001999 | 3476 |
| PR002000 | 4643 |
| PR002002 | 4500 |
| Positive control 1 | 6160 |

254

**(E)**

**Binding to NCI-H929**

**BCMA × CD3**

| Antibody | EC50 (nM) | Maximum MFI | Antibody | EC50 (nM) | Maximum MFI |
|----------|-----------|-------------|----------|-----------|-------------|
| PR002929 | 50.2 | 6420 | PR002929 | 50.2 | 6420 |
| PR003867 | 4.6 | 8594 | PR003875 | 1.2 | 9845 |
| PR003868 | 7.3 | 17363 | PR003876 | 2.3 | 10901 |
| PR003869 | 1.7 | 12479 | PR003877 | 1.7 | 11127 |
| PR003870 | 3.3 | 10638 | PR003878 | 1.5 | 9241 |
| PR003871 | 2.0 | 10580 | PR003879 | 2.1 | 10842 |
| PR003872 | 1.1 | 11274 | PR003880 | 5.6 | 8662 |
| PR003873 | 1.3 | 10202 | PR003881 | 2.6 | 10278 |
| PR003874 | 1.5 | 10978 | PR003882 | 1.8 | 11109 |

**(F)**

**Binding to NCI-H929**

**BCMA × CD3**

| Antibody | EC50 (nM) | Maximum MFI |
|----------|-----------|-------------|
| PR002299 | n.d. | 32843 |
| PR002300 | n.d. | 44154 |
| PR002301 | n.d. | 52958 |
| PR002308 | 1.816 | 63931 |
| PR002309 | 1.336 | 68011 |
| PR002310 | 2.094 | 61263 |
| Positive control 2 | 4.294 | 88218 |
| Positive control 1 | 28.94 | 109555 |

n.d.: not determined

**(G)** **Binding to NCI-H929**

**BCMA × CD3**

| Antibody | EC50 (nM) | Maximum MFI |
|---|---|---|
| PR002309 | 1.731 | 4712 |
| PR002892 | 4.272 | 4360 |
| PR002895 | 2.006 | 4201 |
| PR002950 | 4.114 | 4188 |
| PR002953 | 2.908 | 4303 |
| PR002898 | 1.941 | 3728 |
| PR002901 | 2.319 | 4182 |
| PR002956 | 3.948 | 3566 |
| PR002959 | 4.300 | 4212 |

Legend: PR002309, PR002892, PR002895, PR002950, PR002953, PR002898, PR002901, PR002956, PR002959, hIgG1 iso

**(H)** **Binding to NCI-H929**

**BCMA × CD3**

| Antibody | EC50 (nM) | Maximum MFI |
|---|---|---|
| PR003178 | 1.232 | 15578 |
| PR002895 | 1.530 | 15431 |
| PR002953 | 1.339 | 15078 |
| Positive control 3 | n.d. | 25908 |

n.d.: not determined

Legend: PR003178, PR002895, PR002953, Positive control 3, hIgG1 iso

**(I)** **Binding to NCI-H929**

**BCMA × CD3**

| Antibody | EC50 (nM) | Maximum MFI |
|---|---|---|
| PR002313 | 17.15 | 78009 |
| PR002326 | 3.537 | 59994 |
| PR002328 | 6.893 | 58892 |
| Positive control 2 | 4.294 | 86899 |
| Positive control 1 | 28.94 | 118673 |

Legend: PR002313, PR002326, PR002328, Positive control 1, Positive control 2, hIgG1 iso

256

**(J)**

**Binding to NCI-H929**

**BCMA × CD3**

| Antibody | EC50 (nM) | Maximum MFI |
|---|---|---|
| PR002332 | 9.004 | 84510 |
| Positive control 2 | 4.294 | 86899 |
| Positive control 1 | 28.94 | 118673 |

Legend:
⊖ PR002332
● Positive control 1
■ Positive control 2
⊗ hIgG1 iso

**FIG. 52**

**(A)**

**Binding to human T cells**

**BCMA × CD3**

| Antibody | Maximum MFI |
|---|---|
| PR001987 | 5159 |
| PR001988 | 5796 |
| PR001989 | 6680 |
| PR001990 | 5453 |
| SP34 | 7162 |

Legend:
⊟ PR001987
△ PR001988
▽ PR001989
⊖ PR001990
◆ SP34
⊗ hIgG1 iso

**(B)**

**Binding to human T cells**

**BCMA × CD3**

| Antibody | Maximum MFI |
|---|---|
| PR001990 | 350167 |
| PR002929 | 283045 |
| Positive control 2 | 6847 |
| hIgG1 iso | 2995 |

Legend:
▲ PR001990
▼ PR002929
⊖ Positive control 2
⊗ hIgG1 iso

**(C)** Binding to human T cells

BCMA × CD3

| Antibody | Maximum MFI |
|----------|-------------|
| PR001991 | 3244 |
| PR001992 | 2240 |
| PR001993 | 4785 |
| PR001994 | 3381 |
| SP34 | 7162 |

**(D)** Binding to human T cells

BCMA × CD3

| Antibody | Maximum MFI |
|----------|-------------|
| PR001995 | 2917 |
| PR001996 | 3439 |
| PR001997 | 4458 |
| PR001998 | 3318 |
| SP34 | 6026 |

**(E)** Binding to human T cells

BCMA × CD3

| Antibody | Maximum MFI |
|----------|-------------|
| PR001999 | 1166 |
| PR002000 | 1731 |
| PR002002 | 1118 |
| SP34 | 6026 |
| hIgG1 iso | 12 |

**(F)**

**Binding to human T cells**

**BCMA × CD3**

| Antibody | Maximum MFI |
|----------|-------------|
| PR002309 | 316716 |
| PR002895 | 321582 |

**(G)**

**Binding to human T cells**

**BCMA × CD3**

| Antibody | Maximum MFI |
|----------|-------------|
| PR003178 | 23926 |
| PR002953 | 16961 |
| Positive control 2 | 6847 |
| hIgG1 iso | 2995 |

**FIG. 53**

**(A)** Binding to cynomolgus monkey T cells

BCMA × CD3

| Antibody | Maximum MFI |
|---|---|
| PR001990 | 15557 |
| PR002929 | 10128 |
| Positive control 2 | 1109 |
| hIgG1 iso | 794 |

**(B)** Binding to cynomolgus monkey T cells

BCMA × CD3

| Antibody | Maximum MFI |
|---|---|
| PR002309 | 11664 |
| PR003178 | 10725 |
| PR002895 | 18601 |
| PR002953 | 9708 |
| PR003177 | 12107 |
| PR002950 | 8827 |
| Positive control 2 | 1109 |
| hIgG1 iso | 794 |

**FIG. 54**

Killing NCI-H929

scFv-Fc-VH(2) asymmetric structure

■ Bispecific antibody (30 nM)
▨ Positive control 2 (30 nM)
▢ hIgG1 iso (30 nM)

**FIG. 55**

**(A)**

Killing NCI-H929
scFv-Fc-VH(1) asymmetric structure

**(B)**

Killing NCI-H929
scFv-Fc-VH(1) asymmetric structure

| Antibody | Maximum killing rate % | EC50 (nM) |
|---|---|---|
| PR002000 | 56.40 | 1.657 |
| PR002002 | 57.01 | 0.643 |

**FIG. 56**

**(A)**

**Killing NCI-H929**

**Fab-Fc-VH(1) asymmetric structure**

**(B)**

**Killing NCI-H929**

**Fab-Fc-VH(1) asymmetric structure**

**(C)**

**Killing NCI-H929**

**Fab-Fc-VH(1) asymmetric structure**

| Antibody | Maximum killing rate % | EC50 (nM) |
|---|---|---|
| PR001988 | 49.43 | 1.732 |
| PR001990 | 58.01 | 0.607 |

**FIG. 57**

**(A)**

Killing NCI-H929

Fab-Fc-VH(n) asymmetric structure

**(B)**

Killing NCI-H929

Fab-Fc-VH(n) asymmetric structure

**(C)**

**Killing NCI-H929**

**Fab-Fc-VH(n) asymmetric**

| Antibody | Maximum killing rate % | EC50 (pM) |
|---|---|---|
| PR002892 | 54.30 | 0.361 |
| PR002895 | 52.95 | 0.651 |
| PR002950 | 54.84 | 6.933 |
| PR002953 | 52.46 | 7.617 |
| PR003177 | 54.57 | 1.728 |
| PR003178 | 50.28 | 3.748 |
| Positive control 2 | 55.88 | 10.49 |

**(D)**

**Killing NCI-H929**

**Fab-Fc-VH(n) asymmetric structure**

| Antibody | Maximum killing rate % | EC50 (nM) |
|---|---|---|
| PR003690 | 55.30 | 0.0414 |
| PR003178 | 58.68 | 0.0322 |

**FIG. 58**

FIG. 59

EP 4 545 568 A2

FIG. 60

**(A)**

**Killing NCI-H929**

PR001990
PR001990 + 10 ng/mL sApril
PR001990 + 100 ng/mL sApril
PR001990 + 10 ng/mL sBAFF
PR001990 + 100 ng/mL sBAFF

**(B)**

**Killing NCI-H929**

PR002309
PR002309 + 10 ng/mL sApril
PR002309 + 100 ng/mL sApril
PR002309 + 10 ng/mL sBAFF
PR002309 + 100 ng/mL sBAFF

**(C)**

**Killing NCI-H929**

PR003178
PR003178 + 10 ng/mL sApril
PR003178 + 100 ng/mL sApril
PR003178 + 10 ng/mL sBAFF
PR003178 + 100 ng/mL sBAFF

**(D)**

Killing NCI-H929

-△- Positive control 2
-▽- Positive control 2 + 10 ng/mL sApril
-⊟- Positive control 2 + 100 ng/mL sApril
-⊖- Positive control 2 + 10 ng/mL sBAFF
-✳- Positive control 2 + 100 ng/mL sBAFF

**(E)**

Killing NCI-H929

-△- Positive control 3
-▽- Positive control 3 + 10 ng/mL sApril
-⊟- Positive control 3 + 100 ng/mL sApril
-⊖- Positive control 3 + 10 ng/mL sBAFF
-✳- Positive control 3 + 100 ng/mL sBAFF

**FIG. 61**

**(A)**

Killing NCI-H929

**(B)**

Killing NCI-H929

**(C)**

Killing NCI-H929

**(D)**

Killing NCI-H929

△ Positive control 2 + 500 ng/mL sBCMA
▽ Positive control 2 + 250 ng/mL sBCMA
⊟ Positive control 2 + 125 ng/mL sBCMA
⊖ Positive control 2 + 62.5 ng/mL sBCMA
✕ Positive control 2

**(E)**

Killing NCI-H929

△ Positive control 3 + 500 ng/mL sBCMA
▽ Positive control 3 + 250 ng/mL sBCMA
⊟ Positive control 3 + 125 ng/mL sBCMA
⊖ Positive control 3 + 62.5 ng/mL sBCMA
✳ Positive control 3

**FIG. 62**

**(A)**

BALB/c, 5 mg/kg, I.V.
Fc end detection

**(B)**

BALB/c, 5 mg/kg, I.V.
BCMA end detection

**(C)**

PR003178, 5mg/kg (I.V.), SD Rat

**FIG. 63**

**(A)**

PR001990 10 μg/mouse i.v. single dose
PR002309 10 μg/mouse i.v. single dose
PR002313 10 μg/mouse i.v. single dose
Positive control 2, 10 μg/mouse, i.v., single dose
PBS i.v. single dose

**(B)**

PR001990 10 μg/mouse i.v. single dose
PR002309 10 μg/mouse i.v. single dose
PR002313 10 μg/mouse i.v. single dose
Positive control 2, 10 μg/mouse, i.v., single dose
PBS i.v. single dose

**(C)**

Tumor volume (mm³) vs Days after administration

-⊗- PBS, i.v., QW x 2 doses

-◆- Positive control 2, 10 µg/mouse, i.v., QW×2 doses  -◇- PR002895, 3 ug/mouse, i.v., QW x 2 doses

-▲- PR003177, 10 ug/mouse, i.v., QW x 2 doses  -▼- PR003178, 10 ug/mouse, i.v., QW x 2 doses

-△- PR003177, 3 ug/mouse, i.v., QW x 2 doses  -▽- PR003178, 3 ug/mouse, i.v., QW x 2 doses

**(D)**

Change in body weight % vs Days after administration

-⊗- PBS, i.v., QW x 2 doses

-◆- Positive control 2, 10 µg/mouse, i.v., QW×2 doses  -◇- PR002895, 3 ug/mouse, i.v., QW x 2 doses

-▲- PR003177, 10 ug/mouse, i.v., QW x 2 doses  -▼- PR003178, 10 ug/mouse, i.v., QW x 2 doses

-△- PR003177, 3 ug/mouse, i.v., QW x 2 doses  -▽- PR003178, 3 ug/mouse, i.v., QW x 2 doses

**FIG. 64**

**(A)** PD-L1×4-1BB bispecific antibody molecule of a Fab-HCAb symmetric structure, PR004270

**PR004270 SDS-PAGE**

1: PR004270 reduced
2: PR004270 non-reduced

**PR004270 SEC-HPLC**

| Column Name: | TSKgel G3000SWxl | | Column Size: | 7.8 mm × 300 mm |
| Flow Rate: | 0.7 ml/min | | Particle Size: | 5 µm |
| Mobbile Phase: | 0.1 mol/L Na2SO4 in 0.1 mol/L Phosphate Buffer(pH 6.7) | | | |

Signal: VWD1 A, Wavelength=280 nm

| RT [min] | Height | Width [min] | Area | Area% |
|---|---|---|---|---|
| 8.465 | 0.3033 | 0.1909 | 4.1311 | 0.0532 |
| 8.785 | 0.9529 | 0.2859 | 18.7758 | 0.2416 |
| 9.511 | 9.7702 | 0.6088 | 395.9571 | 5.0947 |
| 11.013 | 224.4043 | 0.4851 | 7175.8320 | 92.3308 |
| 12.200 | 4.4294 | 0.3348 | 114.9631 | 1.4792 |
| 14.699 | 0.5468 | 1.6073 | 62.2095 | 0.8004 |
| | | | Sum | 7771.8687 |

**(B)** PD-L1×CTLA4 bispecific antibody molecule of an IgG-VH tetravalent symmetric structure, PR000300

**PR000300 SDS-PAGE**

1: IgG1 reduced
2: PR000300 reduced
8: IgG1 non-reduced
9: PR000300 non-reduced

**PR000300 SEC-HPLC**

<Chromatogram>

<Peak Table>

Detector A 280nm

| Peak# | Ret. Time | Area | Area% | Height | Height% |
|---|---|---|---|---|---|
| 1 | 6.480 | 3634 | 0.171 | 200 | 0.210 |
| 2 | 7.746 | 2115237 | 99.829 | 95299 | 99.790 |
| Total | | 2118871 | 100.000 | 95499 | 100.000 |

**(C)** **HER2×CTLA4 bispecific antibody molecule of an IgG-VH(2) hexavalent symmetric structure, PR001582**

PR001582 SDS-PAGE

1: PR001582 reduced
2: PR001582 non-reduced

PR001582 SEC-HPLC

| Column Name: | TSKgel G3000SWxl | Column Size: | 7.8 mm × 300 mm |
| Flow Rate: | 0.7 ml/min | Particle Size: | 5 µm |
| Mobile Phase: | 0.1 mol/L Na2SO4 in 0.1 mol/L Phosphate Buffer(pH 6.7) | | |

Signal: VWD1A,Wavelength=280 nm

| RT [min] | Height | Width [min] | Area | Area% |
|---|---|---|---|---|
| 8.152 | 0.65 | 1.10 | 21.42 | 0.65 |
| 9.278 | 0.40 | 1.01 | 18.67 | 0.56 |
| 10.942 | 87.37 | 4.41 | 3278.12 | 98.79 |
| | | Sum | 3318.21 | |

**(D)** **HER2×CTLA4 bispecific antibody molecule of a 2×VH-IgG hexavalent symmetric structure, PR001585**

PR001585 SDS-PAGE

(non-reduced)          (reduced)

1: *PR001573*
8: **PR001585**

PR001585 SEC-HPLC

Signal 1: DAD1 A, Sig=280,4 Ref=off

```
Peak  RetTime Type  Width     Area        Height     Area
  #    [min]        [min]     [mAU*s]     [mAU]      %
----|-------|----|-------|----------|----------|--------|
  1  13.280  BB   0.8803  2224.76392  29.55051  100.0000

Totals :                    2224.76392  29.55051
```

**(E)** HER2×CTLA4 bispecific antibody molecules of Fab-Fc-VH asymmetric structures, PR000916 and PR000917

## PR000916 SDS-PAGE

1: PR000916 non-reduced
2: PR000917 non-reduced

## PR000916 SEC-HPLC

## PR000917 HPLC-SEC

**(F)** HER2×CTLA4 bispecific antibody molecule of a Fab-Fc-VH(2) asymmetric structure, PR002668

## PR002668 SDS-PAGE

1: PR002668 reduced
2: PR002668 non-reduced

## PR002668 SEC-HPLC

Column Name: TSKgel G3000SWxl   Column Size: 7.8 mm × 300 mm
Flow Rate: 0.7 ml/min   Particle Size: 5 µm
Mobile Phase: 0.1 mol/L Na2SO4 in 0.1 mol/L Phosphate Buffer(pH 6.7)

Signal: VWD1A,Wavelength=280 nm

| RT [min] | Height | Width [min] | Area | Area% |
|---|---|---|---|---|
| 7.774 | 0.77 | 1.12 | 27.54 | 2.70 |
| 8.385 | 1.45 | 1.47 | 72.10 | 7.06 |
| 10.271 | 1.25 | 1.39 | 64.36 | 6.30 |
| 11.499 | 0.55 | 0.36 | 12.00 | 1.18 |
| 12.076 | 25.33 | 0.50 | 845.08 | 82.76 |
| | | Sum | 1021.07 | |

## (G) BCMA×CD3 bispecific antibody molecule of an scFv-Fc-VH asymmetric structure, PR002002

### PR002002 SDS-PAGE

1: PR002002 reduced
2: PR002002 non-reduced

### PR002002 SEC-HPLC

| Column Name: | TSKgel G3000SWxl | Column Size: | 7.8 mm × 300 mm |
| Flow Rate: | 0.7 ml/min | Particle Size: | 5 µm |
| Mobile Phase: | 0.1 mol/L Na2SO4 in 0.1 mol/L Phosphate Buffer(pH 6.7) | | |

Signal: VWD1A,Wavelength=280 nm

| RT [min] | Height | Width [min] | Area | Area% |
|---|---|---|---|---|
| 8.249 | 3.18 | 1.19 | 94.38 | 4.86 |
| 9.059 | 1.18 | 1.69 | 100.47 | 5.17 |
| 11.205 | 3.56 | 1.42 | 161.68 | 8.32 |
| 12.824 | 49.01 | 2.58 | 1543.79 | 79.47 |
| 14.698 | 0.55 | 2.05 | 42.20 | 2.17 |
| | | Sum | 1942.52 | |

## (H) BCMA×CD3 bispecific antibody molecule of an scFv-Fc-VH(2) asymmetric structure, PR002869

### PR002869 SDS-PAGE

1: PR002869 reduced
2: PR002869 non-reduced

### PR002869 SEC-HPLC

| Column Name: | TSKgel G3000SWxl | Column Size: | 7.8 mm × 300 mm |
| Flow Rate: | 0.7 ml/min | Particle Size: | 5 µm |
| Mobile Phase: | 0.1 mol/L Na2SO4 in 0.1 mol/L Phosphate Buffer(pH 6.7) | | |

Signal: VWD1A,Wavelength=280 nm

| RT [min] | Height | Width [min] | Area | Area% |
|---|---|---|---|---|
| 8.405 | 3.60 | 2.44 | 155.44 | 4.98 |
| 10.333 | 0.57 | 0.60 | 19.11 | 0.61 |
| 10.947 | 0.84 | 0.69 | 33.27 | 1.06 |
| 12.307 | 75.10 | 1.89 | 2612.45 | 83.62 |
| 13.187 | 8.71 | 0.50 | 259.84 | 8.32 |
| 14.295 | 0.89 | 0.83 | 44.26 | 1.42 |
| | | Sum | 3124.37 | |

## FIG. 65

**FIG. 66**

**(A)**

MC38-hPDL1

-⊗- PBS
-▽- 3mg/kg ipilimumab + 3mg/kg atezolizumab
-△- 3mg/kg PR001573

**(B)**

MC38-hPDL1

-⊗- PBS
-▽- 3mg/kg ipilimumab + 3mg/kg atezolizumab
-△- 3mg/kg PR001573

**FIG. 67**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007096779 A
- WO 2010109165 A2
- WO 2015103072 A1
- WO 2017123650 A2
- WO 2016071004 A1
- US 9273141 B2
- WO 2019133761 A1
- WO 2017134134 A1

**Non-patent literature cited in the description**

- **KABAT et al.** Sequences of Proteins of Immunological Interest. National Institutes of Health (U.S.), 1991
- *J Mol Biol*, 1997, vol. 273, 927-48
- *Rev Assoc Med Bras*, 2017, vol. 63 (9), 814-823
- **ZHAO et al.** *Immunity*, 2019, vol. 51, 1059-1073
- **SALMERON, A. et al.** *J Immunol*, 1991, vol. 147, 3047-3052
- **CONRAD M.L. et al.** *Cytometry A*, 2007, vol. 71, 925-933
- *Blood*, 2004, vol. 103, 3148-3157
- *Leuk Res.*, June 2019, vol. 81, 62-66